# EUROPEAN PATENT APPLICATION

(11) **EP 2 468 768 A2**
(43) Date of publication of application: **27.06.2012**
(21) Application number: 12154514.9
(22) Date of filing: 21.07.2006
(51) Int. Cl.: C07K 16/00, C12P 21/02, C12P 21/06, C07H 21/02, C12N 15/00, C12N 1/18, C07H 21/04

(54) **Multiple gene expression including sorf contructs and methods with polyproteins, pro-proteins, and proteolysis**

(30) Priority: 21.07.2005 US 701855 P
(62) Divisional of application: 06788320.7
(71) Applicant: Abbott Laboratories, Abbott Park, Illinois 60064-6008 (US)
(72) Inventor: Carson, Gerald R., Belmont, MA Massachusetts 02178 (US); Gion, Wendy, Charlton, MA Massachusetts 01507 (US); Salfeld, Jochen G., North Grafton, MA Massachusetts 01536 (US); Gu, Jijie, Shrewsbury, MA Massachusetts 01545 (US); Regier, Dean A., Upton, MA Massachusetts 01568 (US); Kunes, Yune, Winchester, MA Massachusetts 01890 (US)
(74) Representative: Modiano, Micaela Nadia

(57) **Abstract**

Disclosed is an expression vector for expressing a recombinant protein, comprising a coding sequence for a polyprotein, wherein the polyprotein comprises at least a first and a second protein segment, wherein said protein segments are separated by a protein cleavage site therebetween, wherein the protein cleavage site comprises a self processing peptide cleavage sequence, a signal peptide cleavage sequence, or a protease cleavage sequence; and wherein said coding sequence is expressible in a host cell and is cleaved within the host cell.

SEQUENCE LISTING

<110>  (APPLICANT) Abbott Laboratories
       (AGENT) Greenlee, Winner and Sullivan, P.C.
       CARSON, Gerald R.
       GION, Wendy
       SALFELD, Jochen G.
       GU, Jijie
       REGIER, Dean A.
       KUNES, Yune

<120>  MULTIPLE GENE EXPRESSION INCLUDING SORF CONSTRUCTS AND METHODS
       WITH POLYPROTEINS, PRO-PROTEINS, AND PROTEOLYSIS

<130>  74-05_WO

<140>  PCT/US 06/_______________(to be assigned)
<141>  2006-07-21

<150>  US 60/701,855
<151>  2005-07-21

<160>  158

<170>  PatentIn version 3.3

<210>  1
<211>  4
<212>  PRT
<213>  Artificial

<220>
<223>  Synthetic:  cleavage recognition site for furin.


<220>
<221>  MISC_FEATURE
<222>  (2)..(3)
<223>  At position 2, Xaa can be any amino acid and at position 3, Xaa
       can be Arg or Lys.

<400>  1

Arg Xaa Xaa Arg
1


<210>  2
<211>  5
<212>  PRT
<213>  Artificial

<220>
<223>  Recognition sequence for VP4 of IPNV.


<220>
<221>  MISC_FEATURE
<222>  (1)..(4)
<223>  At position 1, Xaa can be Ser or Thr and at position 4, Xaa can
       be Ser or Ala.

<400>  2

Xaa Xaa Ala Xaa Gly
1               5

<210>  3
<211>  7
<212>  PRT
<213>  Artificial

<220>
<223>  Recognition sequence for TEV protease.


<220>
<221>  misc_feature
<222>  (2)..(3)
<223>  Xaa can be any naturally occurring amino acid

<220>
<221>  misc_feature
<222>  (5)..(5)
<223>  Xaa can be any naturally occurring amino acid

<400>  3

Glu Xaa Xaa Tyr Xaa Gln Gly
1               5


<210>  4
<211>  8
<212>  PRT
<213>  Artificial

<220>
<223>  recognition site for rhinovirus 3C protease

<400>  4

Leu Glu Val Leu Phe Gln Gly Pro
1               5


<210>  5
<211>  6
<212>  PRT
<213>  Artificial

<220>
<223>  Recongition sequence of PC5/6 protease, LPC/PC7 protese and
       enterokinase.


<220>
<221>  MISC_FEATURE
<222>  (6)..(6)
<223>  Xaa can be any amino acid.

<400>  5

Asp Asp Asp Asp Lys Xaa
1               5


<210>  6
<211>  5
<212>  PRT
<213>  Artificial

<220>
<223>  Recognition seuqence for Factor Xa protease.

<220>
<221>  MISC_FEATURE
<222>  (2)..(5)
<223>  At position 2 Xaa is Glu or Asp, and at position 5 Xaa can be any
       amino acid.

<400>  6

Ile Xaa Gly Arg Xaa
1               5


<210>  7
<211>  7
<212>  PRT
<213>  Artificial

<220>
<223>  Recognition sequence for thrombin.

<400>  7

Leu Val Gly Pro Arg Gly Ser
1               5


<210>  8
<211>  6
<212>  PRT
<213>  Artificial

<220>
<223>  Recognition sequence for genenase I.

<400>  8

Pro Gly Ala Ala His Tyr
1               5


<210>  9
<211>  7
<212>  PRT
<213>  Artificial

<220>
<223>  Recognition sequence for MMP protease, N1a of turnip mosaic
       potyvirus and KEX2 protease.

<400>  9

Met Tyr Lys Arg Glu Ala Asp
1               5


<210>  10
<211>  4
<212>  PRT
<213>  Artificial

<220>
<223>  Amino acod sequence of furin which targets protein to Trans Golgi
       Network.

<400>  10

Glu Glu Asp Glu

1

<210>  11
<211>  24
<212>  PRT
<213>  Artificial

<220>
<223>  Internally cleavable signal peptide of influenza virus C.

<400>  11

Met Gly Arg Met Ala Met Lys Trp Leu Val Val Ile Ile Cys Phe Ser
1               5                   10                  15

Ile Thr Ser Gln Pro Ala Ser Ala
            20

<210>  12
<211>  19
<212>  PRT
<213>  Artificial

<220>
<223>  FMDV 2A sequence

<400>  12

Leu Leu Asn Phe Asp Leu Leu Lys Leu Ala Gly Asp Val Glu Ser Asn
1               5                   10                  15

Pro Gly Pro

<210>  13
<211>  19
<212>  PRT
<213>  Artificial

<220>
<223>  FMDV 2A sequence.

<400>  13

Thr Leu Asn Phe Asp Leu Leu Lys Leu Ala Gly Asp Val Glu Ser Asn
1               5                   10                  15

Pro Gly Pro

<210>  14
<211>  14
<212>  PRT
<213>  Artificial

<220>
<223>  FDMV 2A sequence.

<400>  14

Leu Leu Lys Leu Ala Gly Asp Val Glu Ser Asn Pro Gly Pro

1                   5                       10


<210>  15
<211>  20
<212>  PRT
<213>  Artificial

<220>
<223>  Variant of 2A sequence.

<400>  15

Gln Leu Leu Asn Phe Asp Leu Leu Lys Leu Ala Gly Asp Val Glu Ser
1                   5                       10                      15


Asn Pro Gly Pro
            20


<210>  16
<211>  19
<212>  PRT
<213>  Artificial

<220>
<223>  Variant of 2A sequence.

<400>  16

Asn Phe Asp Leu Leu Lys Leu Ala Gly Asp Val Glu Ser Asn Pro Gly
1                   5                       10                      15


Pro Phe Phe



<210>  17
<211>  14
<212>  PRT
<213>  Artificial

<220>
<223>  Variant of 2A sequence.

<400>  17

Leu Leu Lys Leu Ala Gly Asp Val Glu Ser Asn Pro Gly Pro
1                   5                       10


<210>  18
<211>  17
<212>  PRT
<213>  Artificial

<220>
<223>  Variant of 2A sequence.

<400>  18

Asn Phe Asp Leu Leu Lys Leu Ala Gly Asp Val Glu Ser Asn Pro Gly
1                   5                       10                      15


Pro

<210>  19
<211>  24
<212>  PRT
<213>  Artificial

<220>
<223>  Variant of 2A sequence.

<400>  19

Ala Pro Val Lys Gln Thr Leu Asn Phe Asp Leu Leu Lys Leu Ala Gly
1               5                   10                  15

Asp Val Glu Ser Asn Pro Gly Pro
            20

<210>  20
<211>  58
<212>  PRT
<213>  Artificial

<220>
<223>  Variant of 2A sequence.

<400>  20

Val Thr Glu Leu Leu Tyr Arg Met Lys Arg Ala Glu Thr Tyr Cys Pro
1               5                   10                  15

Arg Pro Leu Leu Ala Ile His Pro Thr Glu Ala Arg His Lys Gln Lys
            20                  25                  30

Ile Val Ala Pro Val Lys Gln Thr Leu Asn Phe Asp Leu Leu Lys Leu
        35                  40                  45

Ala Gly Asp Val Glu Ser Asn Pro Gly Pro
    50                  55

<210>  21
<211>  10
<212>  PRT
<213>  Artificial

<220>
<223>  N-terminal sequence of D2E7 immunoglobulin heavy chain.

<400>  21

Glu Val Gln Leu Val Glu Ser Gly Gly Gly
1               5                   10

<210>  22
<211>  10
<212>  PRT
<213>  Artificial

<220>
<223>  N-terminal sequence of D2E7 immunoglobulin light chain.

<400>  22

Asp Ile Gln Met Thr Gln Ser Pro Ser Ser
1               5                   10


<210>  23
<211>  22
<212>  PRT
<213>  Artificial

<220>
<223>  D2E7 light chain signal sequence.

<400>  23

Met Asp Met Arg Val Pro Ala Gln Leu Leu Gly Leu Leu Leu Leu Trp
1               5                   10                  15


Phe Pro Gly Ser Arg Cys
            20


<210>  24
<211>  20
<212>  PRT
<213>  Artificial

<220>
<223>  D2E7 signal peptide sequence in Construct H.

<400>  24

Met Asp Met Arg Val Pro Ala Gln Leu Leu Gly Asp Glu Trp Phe Pro
1               5                   10                  15


Gly Ser Arg Cys
            20


<210>  25
<211>  15
<212>  PRT
<213>  Artificial

<220>
<223>  Amino acid sequence at end of intein and in start of light chain
       protein in Construct J.

<400>  25

Met Asp Met Arg Val Pro Ala Gln Trp Phe Pro Gly Ser Arg Cys
1               5                   10                  15


<210>  26
<211>  10
<212>  PRT
<213>  Artificial

<220>
<223>  N-terminal sequence of light chain in Construct H.

<400>  26

Met Asp Met Arg Val Pro Ala Gln Leu Leu
1               5                   10

<210>  27
<211>  22
<212>  PRT
<213>  Artificial

<220>
<223>  Amino acid sequence following intein in Construct L.

<400>  27

Met Asp Met Arg Val Pro Ala Gln Leu Leu Gly Leu Leu Leu Leu Trp
1               5                   10                  15

Phe Pro Gly Ser Gly Gly
            20

<210>  28
<211>  10
<212>  PRT
<213>  Artificial

<220>
<223>  Signal peptidase cleavage site sequence.

<400>  28

Leu Ala Gly Phe Ala Thr Val Ala Gln Ala
1               5                   10

<210>  29
<211>  2925
<212>  DNA
<213>  Artificial

<220>
<223>  Synthetic construct, D2E7 LC-LC-HC Polyprotein  coding sequence.

<220>
<221>  CDS
<222>  (1)..(2922)

<400>  29
atg gac atg cgc gtg ccc gcc cag ctg ctg ggc ctg ctg ctg ctg tgg       48
ttc ccc ggc tcg cga tgc gac atc cag atg acc cag tct cca tcc tcc       96
ctg tct gca tct gta ggg gac aga gtc acc atc act tgt cgg gca agt      144
cag ggc atc aga aat tac tta gcc tgg tat cag caa aaa cca ggg aaa      192
gcc cct aag ctc ctg atc tat gct gca tcc act ttg caa tca ggg gtc      240
cca tct cgg ttc agt ggc agt gga tct ggg aca gat ttc act ctc acc      288
atc agc agc cta cag cct gaa gat gtt gca act tat tac tgt caa agg      336
tat aac cgt gca ccg tat act ttt ggc cag ggg acc aag gtg gaa atc      384
aaa cgt acg gtg gct gca cca tct gtc ttc atc ttc ccg cca tct gat      432
gag cag ttg aaa tct gga act gcc tct gtt gtg tgc ctg ctg aat aac      480
ttc tat ccc aga gag gcc aaa gta cag tgg aag gtg gat aac gcc ctc      528
caa tcg ggt aac tcc cag gag agt gtc aca gag cag gac agc aag gac      576
agc acc tac agc ctc agc agc acc ctg acg ctg agc aaa gca gac tac      624
gag aaa cac aaa gtc tac gcc tgc gaa gtc acc cat cag ggc ctg agc      672
tcg ccc gtc aca aag agc ttc aac agg gga agg tgt aag aga ctt ctc      720
aag ttg gca gga gac gtt gag tcc aac cct ggg ccc atg gac atg cgc      768
gtg ccc gcc cag ctg ctg ggc ctg ctg ctg ctg tgg ttc ccc ggc tcg      816
cga tgc gac atc cag atg acc cag tct cca tcc tcc ctg tct gca tct      864
gta ggg gac aga gtc acc atc act tgt cgg gca agt cag ggc atc aga      912
aat tac tta gcc tgg tat cag caa aaa cca ggg aaa gcc cct aag ctc      960
ctg atc tat gct gca tcc act ttg caa tca ggg gtc cca tct cgg ttc     1008
agt ggc agt gga tct ggg aca gat ttc act ctc acc atc agc agc cta     1056
cag cct gaa gat gtt gca act tat tac tgt caa agg tat aac cgt gca      1104
ccg tat act ttt ggc cag ggg acc aag gtg gaa atc aaa cgt acg gtg      1152
gct gca cca tct gtc ttc atc ttc ccg cca tct gat gag cag ttg aaa      1200
tct gga act gcc tct gtt gtg tgc ctg ctg aat aac ttc tat ccc aga      1248
gag gcc aaa gta cag tgg aag gtg gat aac gcc ctc caa tcg ggt aac      1296
tcc cag gag agt gtc aca gag cag gac agc aag gac agc acc tac agc      1344
ctc agc agc acc ctg acg ctg agc aaa gca gac tac gag aaa cac aaa      1392
gtc tac gcc tgc gaa gtc acc cat cag ggc ctg agc tcg ccc gtc aca      1440
aag agc ttc aac agg gga agg tgt aag aga ctt ctc aag ttg gca gga      1488
gac gtt gag tcc aac cct ggg ccc atg gag ttt ggg ctg agc tgg ctt      1536
ttt ctt gtc gcg att tta aaa ggt gtc cag tgt gag gtg cag ctg gtg      1584
gag tct ggg gga ggc ttg gta cag ccc ggc agg tcc ctg aga ctc tcc      1632
tgt gcg gcc tct gga ttc acc ttt gat gat tat gcc atg cac tgg gtc      1680
cgg caa gct cca ggg aag ggc ctg gaa tgg gtc tca gct atc act tgg      1728
aat agt ggt cac ata gac tat gcg gac tct gtg gag ggc cga ttc acc      1776
atc tcc aga gac aac gcc aag aac tcc ctg tat ctg caa atg aac agt      1824
ctg aga gct gag gat acg gcc gta tat tac tgt gcg aaa gtc tcg tac      1872
ctt agc acc gcg tcc tcc ctt gac tat tgg ggc caa ggt acc ctg gtc      1920
acc gtc tcg agt gcg tcg acc aag ggc cca tcg gtc ttc ccc ctg gca      1968
ccc tcc tcc aag agc acc tct ggg ggc aca gcg gcc ctg ggc tgc ctg      2016
gtc aag gac tac ttc ccc gaa ccg gtg acg gtg tcg tgg aac tca ggc      2064
gcc ctg acc agc ggc gtg cac acc ttc ccg gct gtc cta cag tcc tca      2112
gga ctc tac tcc ctc agc agc gtg gtg acc gtg ccc tcc agc agc ttg      2160
ggc acc cag acc tac atc tgc aac gtg aat cac aag ccc agc aac acc      2208
aag gtg gac aag aaa gtt gag ccc aaa tct tgt gac aaa act cac aca      2256
tgc cca ccg tgc cca gca cct gaa ctc ctg ggg gga ccg tca gtc ttc      2304
ctc ttc ccc cca aaa ccc aag gac acc ctc atg atc tcc cgg acc cct      2352
gag gtc aca tgc gtg gtg gtg gac gtg agc cac gaa gac cct gag gtc      2400
aag ttc aac tgg tac gtg gac ggc gtg gag gtg cat aat gcc aag aca      2448
aag ccg cgg gag gag cag tac aac agc acg tac cgt gtg gtc agc gtc      2496
ctc acc gtc ctg cac cag gac tgg ctg aat ggc aag gag tac aag tgc      2544
aag gtc tcc aac aaa gcc ctc cca gcc ccc atc gag aaa acc atc tcc      2592
aaa gcc aaa ggg cag ccc cga gaa cca cag gtg tac acc ctg ccc cca      2640
tcc cgg gat gag ctg acc aag aac cag gtc agc ctg acc tgc ctg gtc      2688
aaa ggc ttc tat ccc agc gac atc gcc gtg gag tgg gag agc aat ggg        2736
cag ccg gag aac aac tac aag acc acg cct ccc gtg ctg gac tcc gac        2784
ggc tcc ttc ttc ctc tac agc aag ctc acc gtg gac aag agc agg tgg        2832
cag cag ggg aac gtc ttc tca tgc tcc gtg atg cat gag gct ctg cac        2880
aac cac tac acg cag aag agc ctc tcc ctg tct ccg ggt aaa tga            2925


<210>  30
<211>  974
<212>  PRT
<213>  Artificial

<220>
<223>  Synthetic Construct

<400>  30

<210>  31
<211>  10323
<212>  DNA
<213>  Artificial

<220>
<223>  Synthetic construct, D2E7 LC-LC-HC Polyprotein Expression Vector.

<400>  31
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga     240

cggtaaatgg cccgcctggc attatgccca gtacatgacc ttatgggact ttcctacttg     300

gcagtacatc tacgtattag tcatcgctat taccatggtg atgcggtttt ggcagtacat     360

caatgggcgt ggatagcggt ttgactcacg gggatttcca agtctccacc ccattgacgt     420

caatgggagt ttgttttggc accaaaatca acgggacttt ccaaaatgtc gtaacaactc     480

cgccccattg acgcaaatgg gcagggaatt cgagctcggt actcgagcgg tgttccgcgg     540

tcctcctcgt atagaaactc ggaccactct gagacgaagg ctcgcgtcca ggccagcacg     600

aaggaggcta agtgggaggg gtagcggtcg ttgtccacta gggggtccac tcgctccagg     660

gtgtgaagac acatgtcgcc ctcttcggca tcaaggaagg tgattggttt ataggtgtag     720

gccacgtgac cgggtgttcc tgaagggggg ctataaaagg gggtgggggc gcgttcgtcc     780

tcactctctt ccgcatcgct gtctgcgagg gccagctgtt gggctcgcgg ttgaggacaa     840

actcttcgcg gtctttccag tactcttgga tcggaaaccc gtcggcctcc gaacggtact     900

ccgccaccga gggacctgag cgagtccgca tcgaccggat cggaaaacct ctcgactgtt     960

ggggtgagta ctccctctca aaagcgggca tgacttctgc gctaagattg tcagtttcca    1020

aaaacgagga ggatttgata ttcacctggc ccgcggtgat gcctttgagg gtggccgcgt    1080

ccatctggtc agaaaagaca atctttttgt tgtcaagctt gaggtgtggc aggcttgaga    1140

tctggccata cacttgagtg acaatgacat ccactttgcc tttctctcca caggtgtcca    1200

ctcccaggtc caaccggaat tgtacccgcg gccagagctt gcccgggcgc caccatggac    1260

atgcgcgtgc ccgcccagct gctgggcctg ctgctgctgt ggttccccgg ctcgcgatgc    1320

gacatccaga tgacccagtc tccatcctcc ctgtctgcat ctgtagggga cagagtcacc    1380

atcacttgtc gggcaagtca gggcatcaga aattacttag cctggtatca gcaaaaacca    1440

gggaaagccc ctaagctcct gatctatgct gcatccactt tgcaatcagg ggtcccatct    1500

cggttcagtg gcagtggatc tgggacagat ttcactctca ccatcagcag cctacagcct    1560

gaagatgttg caacttatta ctgtcaaagg tataaccgtg caccgtatac ttttggccag    1620

gggaccaagg tggaaatcaa acgtacggtg gctgcaccat ctgtcttcat cttcccgcca 1680
tctgatgagc agttgaaatc tggaactgcc tctgttgtgt gcctgctgaa taacttctat 1740
cccagagagg ccaaagtaca gtggaaggtg gataacgccc tccaatcggg taactcccag 1800
gagagtgtca cagagcagga cagcaaggac agcacctaca gcctcagcag caccctgacg 1860
ctgagcaaag cagactacga gaaacacaaa gtctacgcct gcgaagtcac ccatcagggc 1920
ctgagctcgc ccgtcacaaa gagcttcaac aggggaaggt gtaagagact tctcaagttg 1980
gcaggagacg ttgagtccaa ccctgggccc atggacatgc gcgtgcccgc ccagctgctg 2040
ggcctgctgc tgctgtggtt ccccggctcg cgatgcgaca tccagatgac ccagtctcca 2100
tcctccctgt ctgcatctgt aggggacaga gtcaccatca cttgtcgggc aagtcagggc 2160
atcagaaatt acttagcctg gtatcagcaa aaaccaggga aagcccctaa gctcctgatc 2220
tatgctgcat ccactttgca atcaggggtc ccatctcggt tcagtggcag tggatctggg 2280
acagatttca ctctcaccat cagcagccta cagcctgaag atgttgcaac ttattactgt 2340
caaaggtata accgtgcacc gtatactttt ggccagggga ccaaggtgga aatcaaacgt 2400
acggtggctg caccatctgt cttcatcttc ccgccatctg atgagcagtt gaaatctgga 2460
actgcctctg ttgtgtgcct gctgaataac ttctatccca gagaggccaa agtacagtgg 2520
aaggtggata acgccctcca atcgggtaac tcccaggaga gtgtcacaga gcaggacagc 2580
aaggacagca cctacagcct cagcagcacc ctgacgctga gcaaagcaga ctacgagaaa 2640
cacaaagtct acgcctgcga agtcacccat cagggcctga gctcgcccgt cacaaagagc 2700
ttcaacaggg gaaggtgtaa gagacttctc aagttggcag gagacgttga gtccaaccct 2760
gggcccatgg agtttgggct gagctggctt tttcttgtcg cgattttaaa aggtgtccag 2820
tgtgaggtgc agctggtgga gtctggggga ggcttggtac agcccggcag gtccctgaga 2880
ctctcctgtg cggcctctgg attcaccttt gatgattatg ccatgcactg ggtccggcaa 2940
gctccaggga agggcctgga atgggtctca gctatcactt ggaatagtgg tcacatagac 3000
tatgcggact ctgtggaggg ccgattcacc atctccagag acaacgccaa gaactccctg 3060
tatctgcaaa tgaacagtct gagagctgag gatacggccg tatattactg tgcgaaagtc 3120
tcgtacctta gcaccgcgtc ctcccttgac tattggggcc aaggtaccct ggtcaccgtc 3180
tcgagtgcgt cgaccaaggg cccatcggtc ttccccctgg caccctcctc caagagcacc 3240
tctgggggca cagcggccct gggctgcctg gtcaaggact acttccccga accggtgacg 3300
gtgtcgtgga actcaggcgc cctgaccagc ggcgtgcaca ccttcccggc tgtcctacag 3360
tcctcaggac tctactccct cagcagcgtg gtgaccgtgc cctccagcag cttgggcacc 3420
cagacctaca tctgcaacgt gaatcacaag cccagcaaca ccaaggtgga caagaaagtt 3480
gagcccaaat cttgtgacaa aactcacaca tgcccaccgt gcccagcacc tgaactcctg 3540
gggggaccgt cagtcttcct cttcccccca aaacccaagg acaccctcat gatctcccgg 3600
acccctgagg tcacatgcgt ggtggtggac gtgagccacg aagaccctga ggtcaagttc 3660

aactggtacg tggacggcgt ggaggtgcat aatgccaaga caaagccgcg ggaggagcag 3720
tacaacagca cgtaccgtgt ggtcagcgtc ctcaccgtcc tgcaccagga ctggctgaat 3780
ggcaaggagt acaagtgcaa ggtctccaac aaagccctcc cagcccccat cgagaaaacc 3840
atctccaaag ccaaagggca gccccgagaa ccacaggtgt acaccctgcc cccatcccgg 3900
gatgagctga ccaagaacca ggtcagcctg acctgcctgg tcaaaggctt ctatcccagc 3960
gacatcgccg tggagtggga gagcaatggg cagccggaga acaactacaa gaccacgcct 4020
cccgtgctgg actccgacgg ctccttcttc ctctacagca agctcaccgt ggacaagagc 4080
aggtggcagc aggggaacgt cttctcatgc tccgtgatgc atgaggctct gcacaaccac 4140
tacacgcaga agagcctctc cctgtctccg ggtaaatgag aattagtcta ctcgcaaggg 4200
gcggccgcgt ttaaactgaa tgagcgcgtc catccagaca tgataagata cattgatgag 4260
tttggacaaa ccacaactag aatgcagtga aaaaaatgct ttatttgtga aatttgtgat 4320
gctattgctt tatttgtaac cattataagc tgcaataaac aagttaacaa caacaattgc 4380
attcatttta tgtttcaggt tcagggggag gtgtgggagg ttttttaaag caagtaaaac 4440
ctctacaaat gtggtatggc tgattatgat ccggctgcct cgcgcgtttc ggtgatgacg 4500
gtgaaaacct ctgacacatg cagctcccgg agacggtcac agcttgtctg taagcggatg 4560
ccgggagcag acaagcccgt cagggcgcgt cagcgggtgt tggcgggtgt cggggcgcag 4620
ccatgaccgg tcgacggcgc gccttttttt ttaattttta ttttatttta tttttgacgc 4680
gccgaaggcg cgatctgagc tcggtacagc ttggctgtgg aatgtgtgtc agttagggtg 4740
tggaaagtcc ccaggctccc cagcaggcag aagtatgcaa agcatgcatc tcaattagtc 4800
agcaaccagg tgtggaaagt ccccaggctc cccagcaggc agaagtatgc aaagcatgca 4860
tctcaattag tcagcaacca tagtcccgcc cctaactccg cccatcccgc ccctaactcc 4920
gcccagttcc gcccattctc cgccccatgg ctgactaatt ttttttattt atgcagaggc 4980
cgaggccgcc tcggcctctg agctattcca gaagtagtga ggaggctttt ttggaggcct 5040
aggcttttgc aaaaagctcc tcgaggaact gaaaaaccag aaagttaact ggtaagttta 5100
gtctttttgt cttttatttc aggtcccgga tccggtggtg gtgcaaatca aagaactgct 5160
cctcagtgga tgttgccttt acttctaggc ctgtacggaa gtgttacttc tgctctaaaa 5220
gctgcggaat tgtacccgcg gcctaatacg actcactata gggactagta tggttcgacc 5280
attgaactgc atcgtcgccg tgtcccaaaa tatggggatt ggcaagaacg gagacctacc 5340
ctggcctccg ctcaggaacg agttcaagta cttccaaaga atgaccacaa cctcttcagt 5400
ggaaggtaaa cagaatctgg tgattatggg taggaaaacc tggttctcca ttcctgagaa 5460
gaatcgacct ttaaaggaca gaattaatat agttctcagt agagaactca aagaaccacc 5520
acgaggagct cattttcttg ccaaaagttt agatgatgcc ttaagactta ttgaacaacc 5580
ggaattggca agtaaagtag acatggtttg gatagtcgga ggcagttctg tttaccagga 5640
agccatgaat caaccaggcc acctcagact ctttgtgaca aggatcatgc aggaatttga 5700

aagtgacacg tttttcccag aaattgattt ggggaaatat aaacttctcc cagaataccc 5760
aggcgtcctc tctgaggtcc aggaggaaaa aggcatcaag tataagtttg aagtctacga 5820
gaagaaagac taagcggccg agcgcgcgga tctggaaacg ggagatgggg gaggctaact 5880
gaagcacgga aggagacaat accggaagga acccgcgcta tgacggcaat aaaaagacag 5940
aataaaacgc acgggtgttg ggtcgtttgt tcataaacgc ggggttcggt cccagggctg 6000
gcactctgtc gataccccac cgagacccca ttggggccaa tacgcccgcg tttcttcctt 6060
ttccccaccc caccccccaa gttcgggtga aggcccaggg ctcgcagcca acgtcggggc 6120
ggcaggccct gccatagcca ctggccccgt gggttaggga cggggtcccc catggggaat 6180
ggtttatggt tcgtgggggt tattattttg ggcgttgcgt ggggtctgga gatcccccgg 6240
gctgcaggaa ttccgttaca ttacttacgg taaatggccc gcctggctga ccgcccaacg 6300
acccccgccc attgacgtca ataatgacgt atgttcccat agtaacgcca atagggactt 6360
tccattgacg tcaatgggtg gagtatttac ggtaaactgc ccacttggca gtacatcaag 6420
tgtatcatat gccaagtacg ccccctattg acgtcaatga cggtaaatgg cccgcctggc 6480
attatgccca gtacatgacc ttatgggact ttcctacttg gcagtacatc tacgtattag 6540
tcatcgctat taccatggtg atgcggtttt ggcagtacat caatgggcgt ggatagcggt 6600
ttgactcacg gggatttcca agtctccacc ccattgacgt caatgggagt ttgttttggc 6660
accaaaatca acgggacttt ccaaaatgtc gtaacaactc cgccccattg acgcaaatgg 6720
gcgggaattc gagctcggta ctcgagcggt gttccgcggt cctcctcgta tagaaactcg 6780
gaccactctg agacgaaggc tcgcgtccag gccagcacga aggaggctaa gtgggagggg 6840
tagcggtcgt tgtccactag ggggtccact cgctccaggg tgtgaagaca catgtcgccc 6900
tcttcggcat caaggaaggt gattggttta taggtgtagg ccacgtgacc gggtgttcct 6960
gaaggggggc tataaaaggg ggtgggggcg cgttcgtcct cactctcttc cgcatcgctg 7020
tctgcgaggg ccagctgttg ggctcgcggt tgaggacaaa ctcttcgcgg tctttccagt 7080
actcttggat cggaaacccg tcggcctccg aacggtactc cgccaccgag ggacctgagc 7140
gagtccgcat cgaccggatc ggaaaacctc tcgactgttg gggtgagtac tccctctcaa 7200
aagcgggcat gacttctgcg ctaagattgt cagtttccaa aaacgaggag gatttgatat 7260
tcacctggcc cgcggtgatg cctttgaggg tggccgcgtc catctggtca gaaaagacaa 7320
tctttttgtt gtcaagcttg aggtgtggca ggcttgagat ctggccatac acttgagtga 7380
caatgacatc cactttgcct ttctctccac aggtgtccac tcccaggtcc aaccggaatt 7440
gtacccgcgg ccagagcttg cgggcgccac cgcggccgcg ggatccagac catgataaga 7500
tacattgatg agtttggaca aaccacaact agaatgcagt gaaaaaaatg ctttatttgt 7560
gaaatttgtg atgctattgc tttatttgta accattataa gctgcaataa acaagttaac 7620
aacaacaatt gcattcattt tatgtttcag gttcaggggg aggtgtggga ggttttttcg 7680
gatcctcttg gcgtaatcat ggtcatagct gtttcctgtg tgaaattgtt atccgctcac 7740

aattccacac aacatacgag ccggaagcat aaagtgtaaa gcctggggtg cctaatgagt 7800
gagctaactc acattaattg cgttgcgctc actgcccgct ttccagtcgg gaaacctgtc 7860
gtgccagctg cattaatgaa tcggccaacg cgcggggaga ggcggtttgc gtattgggcg 7920
ctcttccgct tcctcgctca ctgactcgct gcgctcggtc gttcggctgc ggcgagcggt 7980
atcagctcac tcaaaggcgg taatacggtt atccacagaa tcaggggata acgcaggaaa 8040
gaacatgtga gcaaaaggcc agcaaaaggc caggaaccgt aaaaaggccg cgttgctggc 8100
gtttttccat aggctccgcc cccctgacga gcatcacaaa aatcgacgct caagtcagag 8160
gtggcgaaac ccgacaggac tataaagata ccaggcgttt ccccctggaa gctccctcgt 8220
gcgctctcct gttccgaccc tgccgcttac cggatacctg tccgcctttc tcccttcggg 8280
aagcgtggcg ctttctcata gctcacgctg taggtatctc agttcggtgt aggtcgttcg 8340
ctccaagctg ggctgtgtgc acgaaccccc cgttcagccc gaccgctgcg ccttatccgg 8400
taactatcgt cttgagtcca acccggtaag acacgactta tcgccactgg cagcagccac 8460
tggtaacagg attagcagag cgaggtatgt aggcggtgct acagagttct tgaagtggtg 8520
gcctaactac ggctacacta gaagaacagt atttggtatc tgcgctctgc tgaagccagt 8580
taccttcgga aaaagagttg gtagctcttg atccggcaaa caaaccaccg ctggtagcgg 8640
tggttttttt gtttgcaagc agcagattac gcgcagaaaa aaaggatctc aagaagatcc 8700
tttgatcttt tctacggggt ctgacgctca gtggaacgaa aactcacgtt aagggatttt 8760
ggtcatgaga ttatcaaaaa ggatcttcac ctagatcctt ttaaattaaa aatgaagttt 8820
taaatcaatc taaagtatat atgagtaaac ttggtctgac agttaccaat gcttaatcag 8880
tgaggcacct atctcagcga tctgtctatt tcgttcatcc atagttgcct gactccccgt 8940
cgtgtagata actacgatac gggagggctt accatctggc cccagtgctg caatgatacc 9000
gcgagaccca cgctcaccgg ctccagattt atcagcaata aaccagccag ccggaagggc 9060
cgagcgcaga agtggtcctg caactttatc cgcctccatc cagtctatta attgttgccg 9120
ggaagctaga gtaagtagtt cgccagttaa tagtttgcgc aacgttgttg ccattgctac 9180
aggcatcgtg gtgtcacgct cgtcgtttgg tatggcttca ttcagctccg gttcccaacg 9240
atcaaggcga gttacatgat cccccatgtt gtgcaaaaaa gcggttagct ccttcggtcc 9300
tccgatcgtt gtcagaagta agttggccgc agtgttatca ctcatggtta tggcagcact 9360
gcataattct cttactgtca tgccatccgt aagatgcttt tctgtgactg gtgagtactc 9420
aaccaagtca ttctgagaat agtgtatgcg gcgaccgagt tgctcttgcc cggcgtcaat 9480
acgggataat accgcgccac atagcagaac tttaaaagtg ctcatcattg gaaaacgttc 9540
ttcggggcga aaactctcaa ggatcttacc gctgttgaga tccagttcga tgtaacccac 9600
tcgtgcaccc aactgatctt cagcatcttt tactttcacc agcgtttctg ggtgagcaaa 9660
aacaggaagg caaaatgccg caaaaaaggg aataagggcg acacggaaat gttgaatact 9720
catactcttc ctttttcaat attattgaag catttatcag ggttattgtc tcatgagcgg 9780

atacatattt gaatgtattt agaaaaataa acaaataggg gttccgcgca catttccccg 9840

aaaagtgcca cctgacgtct aagaaaccat tattatcatg acattaacct ataaaaatag 9900

gcgtatcacg aggccctttc gtctcgcgcg tttcggtgat gacggtgaaa acctctgaca 9960

catgcagctc ccggagacgg tcacagcttg tctgtaagcg gatgccggga gcagacaagc 10020

ccgtcagggc gcgtcagcgg gtgttggcgg gtgtcggggc tggcttaact atgcggcatc 10080

agagcagatt gtactgagag tgcaccatat gcggtgtgaa ataccgcaca gatgcgtaag 10140

gagaaaatac cgcatcaggc gccattcgcc attcaggctg cgcaactgtt gggaagggcg 10200

atcggtgcgg gcctcttcgc tattacgcca gctggcgaaa gggggatgtg ctgcaaggcg 10260

attaagttgg gtaacgccag ggttttccca gttacgacgt tgtaaaacga cggccagtga 10320

att 10323

<210> 32
<211> 2835
<212> DNA
<213> Artificial

<220>
<223> Synthetic construct, coding seuqence for ABT-007 polyprotein.

<220>
<221> CDS
<222> (1)..(2832)

<400> 32
atg gag ttt ggg ctg agc tgg ctt ttt ctt gtc gcg att tta aaa ggt 48
gtc cag tgt cag gtg cag ctg cag gag tcg ggc cca gga ctg gtg aag 96
cct tcg gag acc ctg tcc ctc acc tgc act gtc tct ggt gcc tcc atc 144
agt agt tac tac tgg agc tgg atc cgg cag ccc cca ggg aag gga ctg 192
gag tgg att ggg tat atc ggg ggg gag ggg agc acc aac tac aac ccc 240
tcc ctc aag agt cga gtc acc ata tca gta gac acg tcc aag aac cag 288
ttc tcc ctg aag ctg agg tct gtg acc gct gcg gac acg gcc gtg tat 336
tac tgt gcg aga gag cga ctg ggg atc ggg gac tac tgg ggc cag gga 384
acc ctg gtc acc gtc tcc tca gcg tcg acc aag ggc cca tcg gtc ttc 432
ccc ctg gcg ccc tgc tct aga agc acc tcc gag agc aca gcg gcc ctg      480
ggc tgc ctg gtc aag gac tac ttc ccc gaa ccg gtg acg gtg tcg tgg      528
aac tca ggc gct ctg acc agc ggc gtg cac acc ttc cca gct gtc ctg      576
cag tcc tca gga ctc tac tcc ctc agc agc gtg gtg acc gtg ccc tcc      624
agc aac ttc ggc acc cag acc tac aca tgc aac gta gat cac aag ccc      672
agc aac acc aag gtg gac aag aca gtt gag cgc aaa tgt tgt gtc gag      720
tgc cca ccg tgc cca gca cca cct gtg gca gga ccg tca gtc ttc ctc      768
ttc ccc cca aaa ccc aag gac acc ctc atg atc tcc cgg acc cct gag      816
gtc acg tgc gtg gtg gtg gac gtg agc cac gaa gac ccc gag gtc cag      864
ttc aac tgg tac gtg gac ggc gtg gag gtg cat aat gcc aag aca aag      912
cca cgg gag gag cag ttc aac agc acg ttc cgt gtg gtc agc gtc ctc      960
acc gtt gtg cac cag gac tgg ctg aac ggc aag gag tac aag tgc aag     1008
gtc tcc aac aaa ggc ctc cca gcc ccc atc gag aaa acc atc tcc aaa     1056
acc aaa ggg cag ccc cga gaa cca cag gtg tac acc ctg ccc cca tcc     1104
cgg gag gag atg acc aag aac cag gtc agc ctg acc tgc ctg gtc aaa     1152
ggc ttc tac ccc agc gac atc gcc gtg gag tgg gag agc aat ggg cag     1200
ccg gag aac aac tac aag acc aca cct ccc atg ctg gac tcc gac ggc     1248
tcc ttc ttc ctc tac agc aag ctc acc gtg gac aag agc agg tgg cag 1296
cag ggg aac gtc ttc tca tgc tcc gtg atg cat gag gct ctg cac aac 1344
cac tac acg cag aag agc ctc tcc ctg tct agg ggt aaa cgc gaa cca 1392
gtt tat ttc cag ggg agc ttg ttt aag ggg ccg cgt gat tat aac cca 1440
ata tcg agt gcc att tgt cat cta acg aat gaa tct gat ggg cac aca 1488
aca tcg ttg tat ggt att ggt ttt ggc cct ttc atc atc aca aac aag 1536
cat ttg ttt aga aga aat aat ggt aca ctg tta gtt caa tca cta cat 1584
ggt gtg ttc aag gta aag aat acc aca act ttg caa caa cac ctc att 1632
gat ggg agg gac atg atg ctc att cgc atg cct aag gat ttc cca cca 1680
ttt cct caa aag ctg aaa ttc aga gag cca caa agg gaa gag cgc ata 1728
tgt ctt gtg aca acc aac ttc caa act aag agc atg tct agc atg gtt 1776
tca gat act agt tgc aca ttc cct tca tct gat ggt ata ttc tgg aaa 1824
cat tgg att cag acc aag gat ggg cac tgt ggt agc ccg ttg gtg tca 1872
act aga gat ggg ttt att gtt ggt ata cac tca gca tca aat ttc acc 1920
aac aca aac aat tat ttt aca agt gtg ccg aaa gac ttc atg gat tta 1968
ttg aca aat caa gag gcg cag caa tgg gtt agt ggt tgg cga ttg aat 2016
gct gac tca gtg tta tgg gga ggc cac aaa gtt ttc atg agc aaa cct 2064
gaa gaa ccc ttt cag cca gtc aaa gaa gca act caa ctc atg agt gaa      2112
tta gtc tac tcg caa ggg atg cgc gtg ccc gcc cag ctg ctg ggc ctg      2160
ctg ctg ctg tgg ttc ccc ggc tcg cga tgc gac atc cag ctg acc caa      2208
tct cca tcc tcc ctg tct gca tct gta gga gac aga gtc acc atc act      2256
tgc cgg gca agt cag ggc att aga aat gat tta ggc tgg tat cag cag      2304
aaa cca ggg aaa gcc cct aag cgc ctg atc tat gct gca tcc agt ttg      2352
caa agt ggg gtc cca tca agg ttc agc ggc agt gga tct ggg aca gaa      2400
ttc act ctc aca atc agc agc ctg cag cct gaa gat ttt gca act tat      2448
tac tgt cta cag cat aat act tac cct ccg acg ttc ggc caa ggg acc      2496
aag gtg gaa atc aaa cgt acg gtg gct gca cca tct gtc ttc atc ttc      2544
ccg cca tct gat gag cag ttg aaa tct gga act gcc tct gtt gtg tgc      2592
ctg ctg aat aac ttc tat ccc aga gag gcc aaa gta cag tgg aag gtg      2640
gat aac gcc ctc caa tcg ggt aac tcc cag gag agt gtc aca gag cag      2688
gac agc aag gac agc acc tac agc ctc agc agc acc ctg acg ctg agc      2736
aaa gca gac tac gag aaa cac aaa gtc tac gcc tgc gaa gtc acc cat      2784
cag ggc ctg agc tcg ccc gtc aca aag agc ttc aac agg gga gag tgt      2832
tga                                                                  2835

<210>  33
<211>  944
<212>  PRT
<213>  Artificial

<220>
<223>  Synthetic Construct

<400>  33

<210>  34
<211>  10212
<212>  DNA
<213>  Artificial

<220>
<223>  Synthetic construct, ABT-007 polyprotein expression vector.

<400>  34
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga     240

cggtaaatgg cccgcctggc attatgccca gtacatgacc ttatgggact ttcctacttg     300

gcagtacatc tacgtattag tcatcgctat taccatggtg atgcggtttt ggcagtacat     360

caatgggcgt ggatagcggt ttgactcacg gggatttcca agtctccacc ccattgacgt     420

caatgggagt ttgttttggc accaaaatca acgggacttt ccaaaatgtc gtaacaactc     480

cgccccaatg acgcaaatgg gcagggaatt cgagctcggt actcgagcgg tgttccgcgg 540
tcctcctcgt atagaaactc ggaccactct gagacgaagg ctcgcgtcca ggccagcacg 600
aaggaggcta agtgggaggg gtagcggtcg ttgtccacta gggggtccac tcgctccagg 660
gtgtgaagac acatgtcgcc ctcttcggca tcaaggaagg tgattggttt ataggtgtag 720
gccacgtgac cgggtgttcc tgaagggggg ctataaaagg gggtgggggc gcgttcgtcc 780
tcactctctt ccgcatcgct gtctgcgagg gccagctgtt gggctcgcgg ttgaggacaa 840
actcttcgcg gtctttccag tactcttgga tcggaaaccc gtcggcctcc gaacggtact 900
ccgccaccga gggacctgag cgagtccgca tcgaccggat cggaaaacct ctcgactgtt 960
ggggtgagta ctccctctca aaagcgggca tgacttctgc gctaagattg tcagtttcca 1020
aaaacgagga ggatttgata ttcacctggc ccgcggtgat gcctttgagg gtggccgcgt 1080
ccatctggtc agaaaagaca atctttttgt tgtcaagctt gaggtgtggc aggcttgaga 1140
tctggccata cacttgagtg acaatgacat ccactttgcc tttctctcca caggtgtcca 1200
ctcccaggtc caaccggaat tgtacccgcg gccagagctt gcccgggcgc caccatggag 1260
tttgggctga gctggctttt tcttgtcgcg attttaaaag gtgtccagtg tcaggtgcag 1320
ctgcaggagt cgggcccagg actggtgaag ccttcggaga ccctgtccct cacctgcact 1380
gtctctggtg cctccatcag tagttactac tggagctgga tccggcagcc cccagggaag 1440
ggactggagt ggattgggta tatcgggggg gaggggagca ccaactacaa cccctccctc 1500
aagagtcgag tcaccatatc agtagacacg tccaagaacc agttctccct gaagctgagg 1560
tctgtgaccg ctgcggacac ggccgtgtat tactgtgcga gagagcgact ggggatcggg 1620
gactactggg gccagggaac cctggtcacc gtctcctcag cgtcgaccaa gggcccatcg 1680
gtcttccccc tggcgccctg ctctagaagc acctccgaga gcacagcggc cctgggctgc 1740
ctggtcaagg actacttccc cgaaccggtg acggtgtcgt ggaactcagg cgctctgacc 1800
agcggcgtgc acaccttccc agctgtcctg cagtcctcag gactctactc cctcagcagc 1860
gtggtgaccg tgccctccag caacttcggc acccagacct acacatgcaa cgtagatcac 1920
aagcccagca acaccaaggt ggacaagaca gttgagcgca aatgttgtgt cgagtgccca 1980
ccgtgcccag caccacctgt ggcaggaccg tcagtcttcc tcttcccccc aaaacccaag 2040
gacaccctca tgatctcccg gacccctgag gtcacgtgcg tggtggtgga cgtgagccac 2100
gaagaccccg aggtccagtt caactggtac gtggacggcg tggaggtgca taatgccaag 2160
acaaagccac gggaggagca gttcaacagc acgttccgtg tggtcagcgt cctcaccgtt 2220
gtgcaccagg actggctgaa cggcaaggag tacaagtgca aggtctccaa caaaggcctc 2280
ccagccccca tcgagaaaac catctccaaa accaaagggc agccccgaga accacaggtg 2340
tacaccctgc ccccatcccg ggaggagatg accaagaacc aggtcagcct gacctgcctg 2400
gtcaaaggct tctaccccag cgacatcgcc gtggagtggg agagcaatgg gcagccggag 2460
aacaactaca agaccacacc tcccatgctg gactccgacg gctccttctt cctctacagc 2520

aagctcaccg tggacaagag caggtggcag caggggaacg tcttctcatg ctccgtgatg 2580
catgaggctc tgcacaacca ctacacgcag aagagcctct ccctgtctag gggtaaacgc 2640
gaaccagttt atttccaggg gagcttgttt aaggggccgc gtgattataa cccaatatcg 2700
agtgccattt gtcatctaac gaatgaatct gatgggcaca caacatcgtt gtatggtatt 2760
ggttttggcc ctttcatcat cacaaacaag catttgttta gaagaaataa tggtacactg 2820
ttagttcaat cactacatgg tgtgttcaag gtaaagaata ccacaacttt gcaacaacac 2880
ctcattgatg ggagggacat gatgctcatt cgcatgccta aggatttccc accatttcct 2940
caaaagctga aattcagaga gccacaaagg gaagagcgca tatgtcttgt gacaaccaac 3000
ttccaaacta agagcatgtc tagcatggtt tcagatacta gttgcacatt cccttcatct 3060
gatggtatat tctggaaaca ttggattcag accaaggatg ggcactgtgg tagcccgttg 3120
gtgtcaacta gagatgggtt tattgttggt atacactcag catcaaattt caccaacaca 3180
aacaattatt ttacaagtgt gccgaaagac ttcatggatt tattgacaaa tcaagaggcg 3240
cagcaatggg ttagtggttg gcgattgaat gctgactcag tgttatgggg aggccacaaa 3300
gttttcatga gcaaacctga agaacccttt cagccagtca aagaagcaac tcaactcatg 3360
agtgaattag tctactcgca agggatgcgc gtgcccgccc agctgctggg cctgctgctg 3420
ctgtggttcc ccggctcgcg atgcgacatc cagctgaccc aatctccatc ctccctgtct 3480
gcatctgtag gagacagagt caccatcact tgccgggcaa gtcagggcat tagaaatgat 3540
ttaggctggt atcagcagaa accagggaaa gcccctaagc gcctgatcta tgctgcatcc 3600
agtttgcaaa gtggggtccc atcaaggttc agcggcagtg gatctgggac agaattcact 3660
ctcacaatca gcagcctgca gcctgaagat tttgcaactt attactgtct acagcataat 3720
acttaccctc cgacgttcgg ccaagggacc aaggtggaaa tcaaacgtac ggtggctgca 3780
ccatctgtct tcatcttccc gccatctgat gagcagttga aatctggaac tgcctctgtt 3840
gtgtgcctgc tgaataactt ctatcccaga gaggccaaag tacagtggaa ggtggataac 3900
gccctccaat cgggtaactc ccaggagagt gtcacagagc aggacagcaa ggacagcacc 3960
tacagcctca gcagcaccct gacgctgagc aaagcagact acgagaaaca caaagtctac 4020
gcctgcgaag tcacccatca gggcctgagc tcgcccgtca caaagagctt caacagggga 4080
gagtgttgag cggccgcgtt taaactgaat gagcgcgtcc atccagacat gataagatac 4140
attgatgagt ttggacaaac cacaactaga atgcagtgaa aaaaatgctt tatttgtgaa 4200
atttgtgatg ctattgcttt atttgtaacc attataagct gcaataaaca agttaacaac 4260
aacaattgca ttcattttat gtttcaggtt cagggggagg tgtgggaggt tttttaaagc 4320
aagtaaaacc tctacaaatg tggtatggct gattatgatc cggctgcctc gcgcgtttcg 4380
gtgatgacgg tgaaaacctc tgacacatgc agctcccgga gacggtcaca gcttgtctgt 4440
aagcggatgc cgggagcaga caagcccgtc agggcgcgtc agcgggtgtt ggcgggtgtc 4500
ggggcgcagc catgaccggt cgacggcgcg cctttttttt taatttttat tttattttat 4560

ttttgacgcg ccgaaggcgc gatctgagct cggtacagct tggctgtgga atgtgtgtca 4620
gttagggtgt ggaaagtccc caggctcccc agcaggcaga agtatgcaaa gcatgcatct 4680
caattagtca gcaaccaggt gtggaaagtc cccaggctcc ccagcaggca gaagtatgca 4740
aagcatgcat ctcaattagt cagcaaccat agtcccgccc ctaactccgc ccatcccgcc 4800
cctaactccg cccagttccg cccattctcc gccccatggc tgactaattt tttttattta 4860
tgcagaggcc gaggccgcct cggcctctga gctattccag aagtagtgag gaggcttttt 4920
tggaggccta ggcttttgca aaaagctcct cgaggaactg aaaaaccaga aagttaactg 4980
gtaagtttag tctttttgtc ttttatttca ggtcccggat ccggtggtgg tgcaaatcaa 5040
agaactgctc ctcagtggat gttgccttta cttctaggcc tgtacggaag tgttacttct 5100
gctctaaaag ctgcggaatt gtacccgcgg cctaatacga ctcactatag ggactagtat 5160
ggttcgacca ttgaactgca tcgtcgccgt gtcccaaaat atggggattg gcaagaacgg 5220
agacctaccc tggcctccgc tcaggaacga gttcaagtac ttccaaagaa tgaccacaac 5280
ctcttcagtg gaaggtaaac agaatctggt gattatgggt aggaaaacct ggttctccat 5340
tcctgagaag aatcgacctt taaaggacag aattaatata gttctcagta gagaactcaa 5400
agaaccacca cgaggagctc attttcttgc caaaagttta gatgatgcct taagacttat 5460
tgaacaaccg gaattggcaa gtaaagtaga catggtttgg atagtcggag gcagttctgt 5520
ttaccaggaa gccatgaatc aaccaggcca cctcagactc tttgtgacaa ggatcatgca 5580
ggaatttgaa agtgacacgt ttttcccaga aattgatttg gggaaatata aacttctccc 5640
agaataccca ggcgtcctct ctgaggtcca ggaggaaaaa ggcatcaagt ataagtttga 5700
agtctacgag aagaaagact aagcggccga gcgcgcggat ctggaaacgg gagatggggg 5760
aggctaactg aagcacggaa ggagacaata ccggaaggaa cccgcgctat gacggcaata 5820
aaaagacaga ataaaacgca cgggtgttgg gtcgtttgtt cataaacgcg gggttcggtc 5880
ccagggctgg cactctgtcg ataccccacc gagaccccat tggggccaat acgcccgcgt 5940
ttcttccttt tccccacccc accccccaag ttcgggtgaa ggcccagggc tcgcagccaa 6000
cgtcggggcg gcaggccctg ccatagccac tggccccgtg ggttagggac ggggtccccc 6060
atggggaatg gtttatggtt cgtgggggtt attattttgg gcgttgcgtg gggtctggag 6120
atcccccggg ctgcaggaat tccgttacat tacttacggt aaatggcccg cctggctgac 6180
cgcccaacga cccccgccca ttgacgtcaa taatgacgta tgttcccata gtaacgccaa 6240
tagggacttt ccattgacgt caatgggtgg agtatttacg gtaaactgcc cacttggcag 6300
tacatcaagt gtatcatatg ccaagtacgc cccctattga cgtcaatgac ggtaaatggc 6360
ccgcctggca ttatgcccag tacatgacct tatgggactt tcctacttgg cagtacatct 6420
acgtattagt catcgctatt accatggtga tgcggttttg gcagtacatc aatgggcgtg 6480
gatagcggtt tgactcacgg ggatttccaa gtctccaccc cattgacgtc aatgggagtt 6540
tgttttggca ccaaaatcaa cgggactttc caaaatgtcg taacaactcc gccccattga 6600

cgcaaaaggg cgggaattcg agctcggtac tcgagcggtg ttccgcggtc ctcctcgtat 6660
agaaactcgg accactctga gacgaaggct cgcgtccagg ccagcacgaa ggaggctaag 6720
tgggaggggt agcggtcgtt gtccactagg gggtccactc gctccagggt gtgaagacac 6780
atgtcgccct cttcggcatc aaggaaggtg attggtttat aggtgtaggc cacgtgaccg 6840
ggtgttcctg aagggggcct ataaaagggg gtgggggcgc gttcgtcctc actctcttcc 6900
gcatcgctgt ctgcgagggc cagctgttgg gctcgcggtt gaggacaaac tcttcgcggt 6960
ctttccagta ctcttggatc ggaaacccgt cggcctccga acggtactcc gccaccgagg 7020
gacctgagcg agtccgcatc gaccggatcg gaaaacctct cgactgttgg ggtgagtact 7080
ccctctcaaa agcgggcatg acttctgcgc taagattgtc agtttccaaa aacgaggagg 7140
atttgatatt cacctggccc gcggtgatgc ctttgagggt ggccgcgtcc atctggtcag 7200
aaaagacaat ctttttgttg tcaagcttga ggtgtggcag gcttgagatc tggccataca 7260
cttgagtgac aatgacatcc actttgcctt tctctccaca ggtgtccact cccaggtcca 7320
accggaattg tacccgcggc cagagcttgc gggcgccacc gcggccgcgg ggatccagac 7380
atgataagat acattgatga gtttggacaa accacaacta gaatgcagtg aaaaaaatgc 7440
tttatttgtg aaatttgtga tgctattgct ttatttgtaa ccattataag ctgcaataaa 7500
caagttaaca acaacaattg cattcatttt atgtttcagg ttcaggggga ggtgtgggag 7560
gttttttcgg atcctcttgg cgtaatcatg gtcatagctg tttcctgtgt gaaattgtta 7620
tccgctcaca attccacaca acatacgagc cggaagcata aagtgtaaag cctggggtgc 7680
ctaatgagtg agctaactca cattaattgc gttgcgctca ctgcccgctt tccagtcggg 7740
aaacctgtcg tgccagctgc attaatgaat cggccaacgc gcggggaaag gcggtttgcg 7800
tattgggcgc tcttccgctt cctcgctcac tgactcgctg cgctcggtcg ttcggctgcg 7860
gcgagcggta tcagctcact caaaggcggt aatacggtta tccacagaat caggggataa 7920
cgcaggaaag aacatgtgag caaaaggcca gcaaaaggcc aggaaccgta aaaaggccgc 7980
gttgctggcg tttttccata ggctccgccc ccctgacgag catcacaaaa atcgacgctc 8040
aagtcagagg tggcgaaacc cgacaggact ataaagatac caggcgtttc cccctggaag 8100
ctccctcgtg cgctctcctg ttccgaccct gccgcttacc ggatacctgt ccgcctttct 8160
cccttcggga agcgtggcgc tttctcatag ctcacgctgt aggtatctca gttcggtgta 8220
ggtcgttcgc tccaagctgg gctgtgtgca cgaacccccc gttcagcccg accgctgcgc 8280
cttatccggt aactatcgtc ttgagtccaa cccggtaaga cacgacttat cgccactggc 8340
agcagccact ggtaacagga ttagcagagc gaggtatgta ggcggtgcta cagagttctt 8400
gaagtggtgg cctaactacg gctacactag aagaacagta tttggtatct gcgctctgct 8460
gaagccagtt accttcggaa aaagagttgg tagctcttga tccggcaaac aaaccaccgc 8520
tggtagcggt ggtttttttg tttgcaagca gcagattacg cgcagaaaaa aaggatctca 8580
agaagatcct ttgatctttt ctacggggtc tgacgctcag tggaacgaaa actcacgtta 8640

agggattttg gtcatgagat tatcaaaaag gatcttcacc tagatccttt ttaattaaaa 8700
atgaagtttt aaatcaatct aaagtatata tgagtaaact tggtctgaca gttaccaatg 8760
cttaatcagt gaggcaccta tctcagcgat ctgtctattt cgttcatcca tagttgcctg 8820
actccccgtc gtgtagataa ctacgatacg ggagggctta ccatctggcc ccagtgctgc 8880
aatgataccg cgagacccac gctcaccggc tccagattta tcagcaataa accagccagc 8940
cggaagggcc gagcgcagaa gtggtcctgc aactttatcc gcctccatcc agtctattaa 9000
ttgttgccgg gaagctagag taagtagttc gccagttaat agtttgcgca acgttgttgc 9060
cattgctaca ggcatcgtgg tgtcacgctc gtcgtttggt atggcttcat tcagctccgg 9120
ttcccaacga tcaaggcgag ttacatgatc ccccatgttg tgcaaaaaag cggttagctc 9180
cttcggtcct ccgatcgttg tcagaagtaa gttggccgca gtgttatcac tcatggttat 9240
ggcagcactg cataattctc ttactgtcat gccatccgta agatgctttt ctgtgactgg 9300
tgagtactca accaagtcat tctgagaata gtgtatgcgg cgaccgagtt gctcttgccc 9360
ggcgtcaata cgggataata ccgcgccaca tagcagaact ttaaaagtgc tcatcattgg 9420
aaaacgttct tcggggcgaa aactctcaag gatcttaccg ctgttgagat ccagttcgat 9480
gtaacccact cgtgcaccca actgatcttc agcatctttt actttcacca gcgtttctgg 9540
gtgagcaaaa acaggaaggc aaaatgccgc aaaaaaggga ataagggcga cacggaaatg 9600
ttgaatactc atactcttcc tttttcaata ttattgaagc atttatcagg gttattgtct 9660
catgagcgga tacatatttg aatgtattta gaaaaataaa caaatagggg ttccgcgcac 9720
atttccccga aaagtgccac ctgacgtcta agaaaccatt attatcatga cattaaccta 9780
taaaaatagg cgtatcacga ggccctttcg tctcgcgcgt ttcggtgatg acggtgaaaa 9840
cctctgacac atgcagctcc cggagacggt cacagcttgt ctgtaagcgg atgccgggag 9900
cagacaagcc cgtcagggcg cgtcagcggg tgttggcggg tgtcggggct ggcttaacta 9960
tgcggcatca gagcagattg tactgagagt gcaccatatg cggtgtgaaa taccgcacag 10020
atgcgtaagg agaaaatacc gcatcaggcg ccattcgcca ttcaggctgc gcaactgttg 10080
ggaagggcga tcggtgcggg cctcttcgct attacgccag ctggcgaaag ggggatgtgc 10140
tgcaaggcga ttaagttggg taacgccagg gttttcccag ttacgacgtt gtaaaacgac 10200
ggccagtgaa tt 10212

<210> 35
<211> 2853
<212> DNA
<213> Artificial

<220>
<223> Synthetic construct, sequence encoding ABT-874 (J695) TEV
      Polyprotein.

<220>
<221> CDS

<222>  (1)..(2850)

<400>  35
atg gag ttt ggg ctg agc tgg ctt ttt ctt gtc gcg att tta aaa ggt      48
gtc cag tgt cag gtg cag ctg gtg gag tct ggg gga ggc gtg gtc cag      96
cct ggg agg tcc ctg aga ctc tcc tgt gca gcg tct gga ttc acc ttc      144
agt agc tat ggc atg cac tgg gtc cgc cag gct cca ggc aag ggg ctg      192
gag tgg gtg gca ttt ata cgg tat gat gga agt aat aaa tac tat gca      240
gac tcc gtg aag ggc cga ttc acc atc tcc aga gac aat tcc aag aac      288
acg ctg tat ctg cag atg aac agc ctg aga gct gag gac acg gct gtg      336
tat tac tgt aag acc cat ggt agc cat gac aac tgg ggc caa ggg aca      384
atg gtc acc gtc tct tca gcg tcg acc aag ggc cca tcg gtc ttc ccc      432
ctg gca ccc tcc tcc aag agc acc tct ggg ggc aca gcg gcc ctg ggc      480
tgc ctg gtc aag gac tac ttc ccc gaa ccg gtg acg gtg tcg tgg aac      528
tca ggc gcc ctg acc agc ggc gtg cac acc ttc ccg gct gtc cta cag      576
tcc tca gga ctc tac tcc ctc agc agc gtg gtg acc gtg ccc tcc agc      624
agc ttg ggc acc cag acc tac atc tgc aac gtg aat cac aag ccc agc      672
aac acc aag gtg gac aag aaa gtt gag ccc aaa tct tgt gac aaa act      720
cac aca tgc cca ccg tgc cca gca cct gaa ctc ctg ggg gga ccg tca      768
gtc ttc ctc ttc ccc cca aaa ccc aag gac acc ctc atg atc tcc cgg      816
acc cct gag gtc aca tgc gtg gtg gtg gac gtg agc cac gaa gac cct      864
gag gtc aag ttc aac tgg tac gtg gac ggc gtg gag gtg cat aat gcc      912
aag aca aag ccg cgg gag gag cag tac aac agc acg tac cgt gtg gtc      960
agc gtc ctc acc gtc ctg cac cag gac tgg ctg aat ggc aag gag tac     1008
aag tgc aag gtc tcc aac aaa gcc ctc cca gcc ccc atc gag aaa acc     1056
atc tcc aaa gcc aaa ggg cag ccc cga gaa cca cag gtg tac acc ctg     1104
ccc cca tcc cgc gag gag atg acc aag aac cag gtc agc ctg acc tgc     1152
ctg gtc aaa ggc ttc tat ccc agc gac atc gcc gtg gag tgg gag agc     1200
aat ggg cag ccg gag aac aac tac aag acc acg cct ccc gtg ctg gac     1248
tcc gac ggc tcc ttc ttc ctc tac agc aag ctc acc gtg gac aag agc     1296
agg tgg cag cag ggg aac gtc ttc tca tgc tcc gtg atg cat gag gct     1344
ctg cac aac cac tac acg cag aag agc ctc tcc ctg tct agg ggt aaa     1392
cgc gaa cca gtt tat ttc cag ggg agc ttg ttt aag ggg ccg cgt gat     1440
tat aac cca ata tcg agt gcc att tgt cat cta acg aat gaa tct gat     1488
ggg cac aca aca tcg ttg tat ggt att ggt ttt ggc cct ttc atc atc     1536
aca aac aag cat ttg ttt aga aga aat aat ggt aca ctg tta gtt caa     1584
tca cta cat ggt gtg ttc aag gta aag aat acc aca act ttg caa caa     1632
cac ctc att gat ggg agg gac atg atg ctc att cgc atg cct aag gat      1680
ttc cca cca ttt cct caa aag ctg aaa ttc aga gag cca caa agg gaa      1728
gag cgc ata tgt ctt gtg aca acc aac ttc caa act aag agc atg tct      1776
agc atg gtt tca gat act agt tgc aca ttc cct tca tct gat ggt ata      1824
ttc tgg aaa cat tgg att cag acc aag gat ggg cac tgt ggt agc ccg      1872
ttg gtg tca act aga gat ggg ttt att gtt ggt ata cac tca gca tca      1920
aat ttc acc aac aca aac aat tat ttt aca agt gtg ccg aaa gac ttc      1968
atg gat tta ttg aca aat caa gag gcg cag caa tgg gtt agt ggt tgg      2016
cga ttg aat gct gac tca gtg tta tgg gga ggc cac aaa gtt ttc atg      2064
agc aaa cct gaa gaa ccc ttt cag cca gtc aaa gaa gca act caa ctc      2112
atg agt gaa tta gtc tac tcg caa ggg atg act tgg acc cca ctc ctc      2160
ttc ctc acc ctc ctc ctc cac tgc aca gga agc tta tcc cag tct gtg      2208
ctg act cag ccc ccc tca gtg tct ggg gcc ccc ggg cag aga gtc acc      2256
atc tct tgt tct gga agc aga tcc aac atc ggc agt aat act gta aag      2304
tgg tat cag cag ctc cca gga acg gcc ccc aaa ctc ctc atc tat tac      2352
aat gat cag cgg ccc tca ggg gtc cct gac cga ttc tct gga tcc aag      2400
tct ggc acc tca gcc tcc ctc gcc atc act ggg ctc cag gct gaa gac      2448
gag gct gac tat tac tgc cag tca tat gac aga tac acc cac ccc gcc 2496
ctg ctc ttc gga act ggg acc aag gtc aca gta cta ggt cag ccc aag 2544
gct gcc ccc tcg gtc act ctg ttc ccg ccc tcc tct gag gag ctt caa 2592
gcc aac aag gcc aca ctg gtg tgt ctc ata agt gac ttc tac ccg gga 2640
gcc gtg aca gtg gcc tgg aag gca gat agc agc ccc gtc aag gcg gga 2688
gtg gag acc acc aca ccc tcc aaa caa agc aac aac aag tac gcg gcc 2736
agc agc tac ctg agc ctg acg cct gag cag tgg aag tcc cac aga agc 2784
tac agc tgc cag gtc acg cat gaa ggg agc acc gtg gag aag aca gtg 2832
gcc cct aca gaa tgt tca tga 2853

<210> 36
<211> 950
<212> PRT
<213> Artificial

<220>
<223> Synthetic Construct

<400> 36

<210>  37
<211>  10230
<212>  DNA
<213>  Artificial

<220>
<223>  Synthetic construct, ABT-874 TEV polyprotein expression vector.

<400>  37
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga     240

cggtaaatgg cccgcctggc attatgccca gtacatgacc ttatgggact ttcctacttg     300

gcagtacatc tacgtattag tcatcgctat taccatggtg atgcggtttt ggcagtacat     360

caatgggcgt ggatagcggt ttgactcacg gggatttcca agtctccacc ccattgacgt     420

caatgggagt ttgttttggc accaaaatca acgggacttt ccaaaatgtc gtaacaactc     480

cgccccaatg acgcaaatgg gcagggaatt cgagctcggt actcgagcgg tgttccgcgg     540

tcctcctcgt atagaaactc ggaccactct gagacgaagg ctcgcgtcca ggccagcacg     600

aaggaggcta agtgggaggg gtagcggtcg ttgtccacta gggggtccac tcgctccagg     660

gtgtgaagac acatgtcgcc ctcttcggca tcaaggaagg tgattggttt ataggtgtag     720

gccacgtgac cgggtgttcc tgaagggggg ctataaaagg gggtgggggc gcgttcgtcc     780

tcactctctt ccgcatcgct gtctgcgagg gccagctgtt gggctcgcgg ttgaggacaa     840

actcttcgcg gtctttccag tactcttgga tcggaaaccc gtcggcctcc gaacggtact     900

ccgccaccga gggacctgag cgagtccgca tcgaccggat cggaaaacct ctcgactgtt     960

ggggtgagta ctccctctca aaagcgggca tgacttctgc gctaagattg tcagtttcca    1020

aaaacgagga ggatttgata ttcacctggc ccgcggtgat gcctttgagg gtggccgcgt    1080

ccatctggtc agaaaagaca atctttttgt tgtcaagctt gaggtgtggc aggcttgaga    1140

tctggccata cacttgagtg acaatgacat ccactttgcc tttctctcca caggtgtcca    1200

ctcccaggtc caaccggaat tgtacccgcg gccagagctt gcccgggcgc caccatggag    1260

tttgggctga gctggctttt tcttgtcgcg attttaaaag gtgtccagtg tcaggtgcag 1320
ctggtggagt ctgggggagg cgtggtccag cctgggaggt ccctgagact ctcctgtgca 1380
gcgtctggat tcaccttcag tagctatggc atgcactggg tccgccaggc tccaggcaag 1440
gggctggagt gggtggcatt tatacggtat gatggaagta ataaatacta tgcagactcc 1500
gtgaagggcc gattcaccat ctccagagac aattccaaga acacgctgta tctgcagatg 1560
aacagcctga gagctgagga cacggctgtg tattactgta agacccatgg tagccatgac 1620
aactggggcc aaggaacaat ggtcaccgtc tcttcagcgt cgaccaaggg cccatcggtc 1680
ttccccctgg caccctcctc caagagcacc tctgggggca cagcggccct gggctgcctg 1740
gtcaaggact acttccccga accggtgacg gtgtcgtgga actcaggcgc cctgaccagc 1800
ggcgtgcaca ccttcccggc tgtcctacag tcctcaggac tctactccct cagcagcgtg 1860
gtgaccgtgc cctccagcag cttgggcacc cagacctaca tctgcaacgt gaatcacaag 1920
cccagcaaca ccaaggtgga caagaaagtt gagcccaaat cttgtgacaa aactcacaca 1980
tgcccaccgt gcccagcacc tgaactcctg gggggaccgt cagtcttcct cttcccccca 2040
aaacccaagg acaccctcat gatctcccgg acccctgagg tcacatgcgt ggtggtggac 2100
gtgagccacg aagaccctga ggtcaagttc aactggtacg tggacggcgt ggaggtgcat 2160
aatgccaaga caaagccgcg ggaggagcag tacaacagca cgtaccgtgt ggtcagcgtc 2220
ctcaccgtcc tgcaccagga ctggctgaat ggcaaggagt acaagtgcaa ggtctccaac 2280
aaagccctcc cagcccccat cgagaaaacc atctccaaag ccaaagggca gccccgagaa 2340
ccacaggtgt acaccctgcc cccatcccgc gaggagatga ccaagaacca ggtcagcctg 2400
acctgcctgg tcaaaggctt ctatcccagc gacatcgccg tggagtggga gagcaatggg 2460
cagccggaga acaactacaa gaccacgcct cccgtgctgg actccgacgg ctccttcttc 2520
ctctacagca agctcaccgt ggacaagagc aggtggcagc aggggaacgt cttctcatgc 2580
tccgtgatgc atgaggctct gcacaaccac tacacgcaga agagcctctc cctgtctagg 2640
ggtaaacgcg aaccagttta tttccagggg agcttgttta aggggccgcg tgattataac 2700
ccaatatcga gtgccatttg tcatctaacg aatgaatctg atgggcacac aacatcgttg 2760
tatggtattg gttttggccc tttcatcatc acaaacaagc atttgtttag aagaaataat 2820
ggtacactgt tagttcaatc actacatggt gtgttcaagg taaagaatac cacaactttg 2880
caacaacacc tcattgatgg gagggacatg atgctcattc gcatgcctaa ggatttccca 2940
ccatttcctc aaaagctgaa attcagagag ccacaaaggg aagagcgcat atgtcttgtg 3000
acaaccaact tccaaactaa gagcatgtct agcatggttt cagatactag ttgcacattc 3060
ccttcatctg atggtatatt ctggaaacat tggattcaga ccaaggatgg gcactgtggt 3120
agcccgttgg tgtcaactag agatgggttt attgttggta tacactcagc atcaaatttc 3180
accaacacaa acaattattt tacaagtgtg ccgaaagact tcatggattt attgacaaat 3240
caagaggcgc agcaatgggt tagtggttgg cgattgaatg ctgactcagt gttatgggga 3300

ggccacaaag ttttcatgag caaacctgaa gaaccctttc agccagtcaa agaagcaact 3360
caactcatga gtgaattagt ctactcgcaa gggatgactt ggaccccact cctcttcctc 3420
accctcctcc tccactgcac aggaagctta tcccagtctg tgctgactca gcccccctca 3480
gtgtctgggg cccccgggca gagagtcacc atctcttgtt ctggaagcag atccaacatc 3540
ggcagtaata ctgtaaagtg gtatcagcag ctcccaggaa cggcccccaa actcctcatc 3600
tattacaatg atcagcggcc ctcaggggtc cctgaccgat tctctggatc caagtctggc 3660
acctcagcct ccctcgccat cactgggctc caggctgaag acgaggctga ctattactgc 3720
cagtcatatg acagatacac ccaccccgcc ctgctcttcg gaactgggac caaggtcaca 3780
gtactaggtc agcccaaggc tgccccctcg gtcactctgt tcccgccctc ctctgaggag 3840
cttcaagcca acaaggccac actggtgtgt ctcataagtg acttctaccc gggagccgtg 3900
acagtggcct ggaaggcaga tagcagcccc gtcaaggcgg gagtggagac caccacaccc 3960
tccaaacaaa gcaacaacaa gtacgcggcc agcagctacc tgagcctgac gcctgagcag 4020
tggaagtccc acagaagcta cagctgccag gtcacgcatg aagggagcac cgtggagaag 4080
acagtggccc ctacagaatg ttcatgagcg gccgcgttta aactgaatga gcgcgtccat 4140
ccagacatga taagatacat tgatgagttt ggacaaacca caactagaat gcagtgaaaa 4200
aaatgcttta tttgtgaaat ttgtgatgct attgctttat ttgtaaccat tataagctgc 4260
aataaacaag ttaacaacaa caattgcatt cattttatgt ttcaggttca gggggaggtg 4320
tgggaggttt tttaaagcaa gtaaaacctc tacaaatgtg gtatggctga ttatgatccg 4380
gctgcctcgc gcgtttcggt gatgacggtg aaaacctctg acacatgcag ctcccggaga 4440
cggtcacagc ttgtctgtaa gcggatgccg ggagcagaca agcccgtcag ggcgcgtcag 4500
cgggtgttgg cgggtgtcgg ggcgcagcca tgaccggtcg acggcgcgcc ttttttttta 4560
atttttattt tattttattt ttgacgcgcc gaaggcgcga tctgagctcg gtacagcttg 4620
gctgtggaat gtgtgtcagt tagggtgtgg aaagtcccca ggctccccag caggcagaag 4680
tatgcaaagc atgcatctca attagtcagc aaccaggtgt ggaaagtccc caggctcccc 4740
agcaggcaga agtatgcaaa gcatgcatct caattagtca gcaaccatag tcccgcccct 4800
aactccgccc atcccgcccc taactccgcc cagttccgcc cattctccgc cccatggctg 4860
actaattttt tttatttatg cagaggccga ggccgcctcg gcctctgagc tattccagaa 4920
gtagtgagga ggcttttttg gaggcctagg cttttgcaaa aagctcctcg aggaactgaa 4980
aaaccagaaa gttaactggt aagtttagtc tttttgtctt ttatttcagg tcccggatcc 5040
ggtggtggtg caaatcaaag aactgctcct cagtggatgt tgcctttact tctaggcctg 5100
tacggaagtg ttacttctgc tctaaaagct gcggaattgt acccgcggcc taatacgact 5160
cactataggg actagtatgg ttcgaccatt gaactgcatc gtcgccgtgt cccaaaatat 5220
ggggattggc aagaacggag acctaccctg gcctccgctc aggaacgagt tcaagtactt 5280
ccaaagaatg accacaacct cttcagtgga aggtaaacag aatctggtga ttatgggtag 5340

gaaaacctgg ttctccattc ctgagaagaa tcgaccttta aaggacagaa ttaatatagt 5400
tctcagtaga gaactcaaag aaccaccacg aggagctcat tttcttgcca aaagtttaga 5460
tgatgcctta agacttattg aacaaccgga attggcaagt aaagtagaca tggtttggat 5520
agtcggaggc agttctgttt accaggaagc catgaatcaa ccaggccacc tcagactctt 5580
tgtgacaagg atcatgcagg aatttgaaag tgacacgttt ttcccagaaa ttgatttggg 5640
gaaatataaa cttctcccag aatacccagg cgtcctctct gaggtccagg aggaaaaagg 5700
catcaagtat aagtttgaag tctacgagaa gaaagactaa gcggccgagc gcgcggatct 5760
ggaaacggga gatgggggag gctaactgaa gcacggaagg agacaatacc ggaaggaacc 5820
cgcgctatga cggcaataaa aagacagaat aaaacgcacg ggtgttgggt cgtttgttca 5880
taaacgcggg gttcggtccc agggctggca ctctgtcgat accccaccga gaccccattg 5940
gggccaatac gcccgcgttt cttccttttc cccaccccac cccccaagtt cgggtgaagg 6000
cccagggctc gcagccaacg tcggggcggc aggccctgcc atagccactg gccccgtggg 6060
ttagggacgg ggtcccccat ggggaatggt ttatggttcg tgggggttat tattttgggc 6120
gttgcgtggg gtctggagat cccccgggct gcaggaattc cgttacatta cttacggtaa 6180
atggcccgcc tggctgaccg cccaacgacc cccgcccatt gacgtcaata atgacgtatg 6240
ttcccatagt aacgccaata gggactttcc attgacgtca atgggtggag tatttacggt 6300
aaactgccca cttggcagta catcaagtgt atcatatgcc aagtacgccc cctattgacg 6360
tcaatgacgg taaatggccc gcctggcatt atgcccagta catgacctta tgggactttc 6420
ctacttggca gtacatctac gtattagtca tcgctattac catggtgatg cggttttggc 6480
agtacatcaa tgggcgtgga tagcggtttg actcacgggg atttccaagt ctccacccca 6540
ttgacgtcaa tgggagtttg ttttggcacc aaaatcaacg ggactttcca aaatgtcgta 6600
acaactccgc cccattgacg caaaagggcg ggaattcgag ctcggtactc gagcggtgtt 6660
ccgcggtcct cctcgtatag aaactcggac cactctgaga cgaaggctcg cgtccaggcc 6720
agcacgaagg aggctaagtg ggaggggtag cggtcgttgt ccactagggg gtccactcgc 6780
tccagggtgt gaagacacat gtcgccctct tcggcatcaa ggaaggtgat tggtttatag 6840
gtgtaggcca cgtgaccggg tgttcctgaa ggggggctat aaaagggggt gggggcgcgt 6900
tcgtcctcac tctcttccgc atcgctgtct gcgagggcca gctgttgggc tcgcggttga 6960
ggacaaactc ttcgcggtct ttccagtact cttggatcgg aaacccgtcg gcctccgaac 7020
ggtactccgc caccgaggga cctgagcgag tccgcatcga ccggatcgga aaacctctcg 7080
actgttgggg tgagtactcc ctctcaaaag cgggcatgac ttctgcgcta agattgtcag 7140
tttccaaaaa cgaggaggat ttgatattca cctggcccgc ggtgatgcct ttgagggtgg 7200
ccgcgtccat ctggtcagaa aagacaatct ttttgttgtc aagcttgagg tgtggcaggc 7260
ttgagatctg gccatacact tgagtgacaa tgacatccac tttgcctttc tctccacagg 7320
tgtccactcc caggtccaac cggaattgta cccgcggcca gagcttgcgg gcgccaccgc 7380

ggccgcgggg atccagacat gataagatac attgatgagt ttggacaaac cacaactaga 7440
atgcagtgaa aaaaatgctt tatttgtgaa atttgtgatg ctattgcttt atttgtaacc 7500
attataagct gcaataaaca agttaacaac aacaattgca ttcattttat gtttcaggtt 7560
cagggggagg tgtgggaggt tttttcggat cctcttggcg taatcatggt catagctgtt 7620
tcctgtgtga aattgttatc cgctcacaat tccacacaac atacgagccg gaagcataaa 7680
gtgtaaagcc tggggtgcct aatgagtgag ctaactcaca ttaattgcgt tgcgctcact 7740
gcccgctttc cagtcgggaa acctgtcgtg ccagctgcat taatgaatcg gccaacgcgc 7800
ggggaaaggc ggtttgcgta ttgggcgctc ttccgcttcc tcgctcactg actcgctgcg 7860
ctcggtcgtt cggctgcggc gagcggtatc agctcactca aaggcggtaa tacggttatc 7920
cacagaatca ggggataacg caggaaagaa catgtgagca aaaggccagc aaaaggccag 7980
gaaccgtaaa aaggccgcgt tgctggcgtt tttccatagg ctccgccccc ctgacgagca 8040
tcacaaaaat cgacgctcaa gtcagaggtg gcgaaacccg acaggactat aaagatacca 8100
ggcgtttccc cctggaagct ccctcgtgcg ctctcctgtt ccgaccctgc cgcttaccgg 8160
atacctgtcc gcctttctcc cttcgggaag cgtggcgctt tctcatagct cacgctgtag 8220
gtatctcagt tcggtgtagg tcgttcgctc caagctgggc tgtgtgcacg aaccccccgt 8280
tcagcccgac cgctgcgcct tatccggtaa ctatcgtctt gagtccaacc cggtaagaca 8340
cgacttatcg ccactggcag cagccactgg taacaggatt agcagagcga ggtatgtagg 8400
cggtgctaca gagttcttga agtggtggcc taactacggc tacactagaa gaacagtatt 8460
tggtatctgc gctctgctga agccagttac cttcggaaaa agagttggta gctcttgatc 8520
cggcaaacaa accaccgctg gtagcggtgg tttttttgtt tgcaagcagc agattacgcg 8580
cagaaaaaaa ggatctcaag aagatccttt gatcttttct acggggtctg acgctcagtg 8640
gaacgaaaac tcacgttaag ggattttggt catgagatta tcaaaaagga tcttcaccta 8700
gatcctttta aattaaaaat gaagttttaa atcaatctaa agtatatatg agtaaacttg 8760
gtctgacagt taccaatgct taatcagtga ggcacctatc tcagcgatct gtctatttcg 8820
ttcatccata gttgcctgac tccccgtcgt gtagataact acgatacggg agggcttacc 8880
atctggcccc agtgctgcaa tgataccgcg agacccacgc tcaccggctc cagatttatc 8940
agcaataaac cagccagccg gaagggccga gcgcagaagt ggtcctgcaa ctttatccgc 9000
ctccatccag tctattaatt gttgccggga agctagagta agtagttcgc cagttaatag 9060
tttgcgcaac gttgttgcca ttgctacagg catcgtggtg tcacgctcgt cgtttggtat 9120
ggcttcattc agctccggtt cccaacgatc aaggcgagtt acatgatccc ccatgttgtg 9180
caaaaaagcg gttagctcct tcggtcctcc gatcgttgtc agaagtaagt tggccgcagt 9240
gttatcactc atggttatgg cagcactgca taattctctt actgtcatgc catccgtaag 9300
atgcttttct gtgactggtg agtactcaac caagtcattc tgagaatagt gtatgcggcg 9360
accgagttgc tcttgcccgg cgtcaatacg ggataatacc gcgccacata gcagaacttt 9420

aaaagtgctc atcattggaa aacgttcttc ggggcgaaaa ctctcaagga tcttaccgct 9480

gttgagatcc agttcgatgt aacccactcg tgcacccaac tgatcttcag catcttttac 9540

tttcaccagc gtttctgggt gagcaaaaac aggaaggcaa aatgccgcaa aaaagggaat 9600

aagggcgaca cggaaatgtt gaatactcat actcttcctt tttcaatatt attgaagcat 9660

ttatcagggt tattgtctca tgagcggata catatttgaa tgtatttaga aaaataaaca 9720

aataggggtt ccgcgcacat ttccccgaaa agtgccacct gacgtctaag aaaccattat 9780

tatcatgaca ttaacctata aaaataggcg tatcacgagg ccctttcgtc tcgcgcgttt 9840

cggtgatgac ggtgaaaacc tctgacacat gcagctcccg gagacggtca cagcttgtct 9900

gtaagcggat gccgggagca gacaagcccg tcagggcgcg tcagcgggtg ttggcgggtg 9960

tcggggctgg cttaactatg cggcatcaga gcagattgta ctgagagtgc accatatgcg 10020

gtgtgaaata ccgcacagat gcgtaaggag aaaataccgc atcaggcgcc attcgccatt 10080

caggctgcgc aactgttggg aagggcgatc ggtgcgggcc tcttcgctat tacgccagct 10140

ggcgaaaggg ggatgtgctg caaggcgatt aagttgggta acgccagggt tttcccagtt 10200

acgacgttgt aaaacgacgg ccagtgaatt 10230

<210> 38
<211> 2901
<212> DNA
<213> Artificial

<220>
<223> Synthetic construct, sequence encoding EL246 GG TEV polyprotein.

<220>
<221> CDS
<222> (1)..(2898)

<400> 38
atg gag ttt ggg ctg agc tgg ctt ttt ctt gtc gcg att tta aaa ggt 48
gtc cag tgc gag gtg cag ctg gtg cag tct gga gca gag gtg aaa aag 96
ccc ggg gag tct ctg aag atc tcc tgt aag ggg tcc gga tac gca ttc 144
agt agt tcc tgg atc ggc tgg gtg cgc cag atg ccc ggg aaa ggc ctg 192
gag tgg atg ggg cgg att tat cct gga gat gga gat act aac tac aat 240
ggg aag ttc aag ggc cag gtc acc atc tca gcc gac aag tcc atc agc 288
acc gcc tac ctg cag tgg agc agc ctg aag gct agc gac acc gcc atg 336
tat tac tgt gcg aga gcg cgc gtg gga tcc acg gtc tat gat ggt tac 384
ctc tat gca atg gac tac tgg ggt caa ggt acc tca gtc acc gtc tcc 432
tca gcg tcg acc aag ggc cca tcg gtc ttc ccc ctg gca ccc tcc tcc 480
aag agc acc tct ggg ggc aca gcg gcc ctg ggc tgc ctg gtc aag gac 528
tac ttc ccc gaa ccg gtg acg gtg tcg tgg aac tca ggc gcc ctg acc 576
agc ggc gtg cac acc ttc ccg gct gtc cta cag tcc tca gga ctc tac 624
tcc ctc agc agc gtg gtg acc gtg ccc tcc agc agc ttg ggc acc cag 672
acc tac atc tgc aac gtg aat cac aag ccc agc aac acc aag gtg gac 720
aag aaa gtt gag ccc aaa tct tgt gac aaa act cac aca tgc cca ccg 768
tgc cca gca cct gaa gcc gcg ggg gga ccg tca gtc ttc ctc ttc ccc 816
cca aaa ccc aag gac acc ctc atg atc tcc cgg acc cct gag gtc aca 864
tgc gtg gtg gtg gac gtg agc cac gaa gac cct gag gtc aag ttc aac 912
tgg tac gtg gac ggc gtg gag gtg cat aat gcc aag aca aag ccg cgg 960
gag gag cag tac aac agc acg tac cgt gtg gtc agc gtc ctc acc gtc 1008
ctg cac cag gac tgg ctg aat ggc aag gag tac aag tgc aag gtc tcc 1056
aac aaa gcc ctc cca gcc ccc atc gag aaa acc atc tcc aaa gcc aaa 1104
ggg cag ccc cga gaa cca cag gtg tac acc ctg ccc cca tcc cgc gag 1152
gag atg acc aag aac cag gtc agc ctg acc tgc ctg gtc aaa ggc ttc 1200
tat ccc agc gac atc gcc gtg gag tgg gag agc aat ggg cag ccg gag 1248
aac aac tac aag acc acg cct ccc gtg ctg gac tcc gac ggc tcc ttc 1296
ttc ctc tac agc aag ctc acc gtg gac aag agc agg tgg cag cag ggg 1344
aac gtc ttc tca tgc tcc gtg atg cat gag gct ctg cac aac cac tac 1392
acg cag aag agc ctc tcc ctg tct agg ggt aaa cgc gaa cca gtt tat 1440
ttc cag ggg agc ttg ttt aag ggg ccg cgt gat tat aac cca ata tcg 1488
agt gcc att tgt cat cta acg aat gaa tct gat ggg cac aca aca tcg 1536
ttg tat ggt att ggt ttt ggc cct ttc atc atc aca aac aag cat ttg 1584
ttt aga aga aat aat ggt aca ctg tta gtt caa tca cta cat ggt gtg 1632
ttc aag gta aag aat acc aca act ttg caa caa cac ctc att gat ggg 1680
agg gac atg atg ctc att cgc atg cct aag gat ttc cca cca ttt cct 1728
caa aag ctg aaa ttc aga gag cca caa agg gaa gag cgc ata tgt ctt 1776
gtg aca acc aac ttc caa act aag agc atg tct agc atg gtt tca gat 1824
act agt tgc aca ttc cct tca tct gat ggt ata ttc tgg aaa cat tgg 1872
att cag acc aag gat ggg cac tgt ggt agc ccg ttg gtg tca act aga 1920
gat ggg ttt att gtt ggt ata cac tca gca tca aat ttc acc aac aca     1968
aac aat tat ttt aca agt gtg ccg aaa gac ttc atg gat tta ttg aca     2016
aat caa gag gcg cag caa tgg gtt agt ggt tgg cga ttg aat gct gac     2064
tca gtg tta tgg gga ggc cac aaa gtt ttc atg agc aaa cct gaa gaa     2112
ccc ttt cag cca gtc aaa gaa gca act caa ctc atg agt gaa tta gtc     2160
tac tcg caa ggg atg gac atg cgc gtg ccc gcc cag ctg ctg ggc ctg     2208
ctg ctg ctg tgg ttc ccc ggc tcg cga tgc gac atc gtg atg acc cag     2256
tct cca gac tcc ctg gct gtg tct ctg ggc gag agg gcc acc atc aac     2304
tgc aag tcc agt cag agc ctt tca tat aga agc aat caa aag aac tcg     2352
ttg gcc tgg tac cag cag aaa cca gga cag cct cct aag ctg ctc att     2400
tac tgg gct agc act agg gaa tct ggg gtc cct gac cga ttc agt gga     2448
tcc ggg tct ggg aca gat ttc act ctc acc atc agc agc ctg cag gct     2496
gaa gat gtg gca gtt tat tac tgt cac caa tat tat agc tat ccg tac     2544
acg ttc gga ggg ggg acc aag gtg gaa att aaa cgt acg gtg gct gca     2592
cca tct gtc ttc atc ttc ccg cca tct gat gag cag ttg aaa tct gga     2640
act gcc tct gtt gtg tgc ctg ctg aat aac ttc tat ccc aga gag gcc     2688
aaa gta cag tgg aag gtg gat aac gcc ctc caa tcg ggt aac tcc cag     2736
gag agt gtc aca gag cag gac agc aag gac agc acc tac agc ctc agc     2784
agc acc ctg acg ctg agc aaa gca gac tac gag aaa cac aaa gtc tac     2832
gcc tgc gaa gtc acc cat cag ggc ctg agc tcg ccc gtc aca aag agc     2880
ttc aac agg gga gag tgt tga                                         2901

<210>  39
<211>  966
<212>  PRT
<213>  Artificial

<220>
<223>  Synthetic Construct

<400>  39

<210>  40

<211>  10278
<212>  DNA
<213>  Artificial

<220>
<223>  Synthetic construct, EL246 GG TEV Polyprotein expression vector.

<400>  40
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga     240

cggtaaatgg cccgcctggc attatgccca gtacatgacc ttatgggact ttcctacttg     300

gcagtacatc tacgtattag tcatcgctat taccatggtg atgcggtttt ggcagtacat     360

caatgggcgt ggatagcggt ttgactcacg gggatttcca agtctccacc ccattgacgt     420

caatgggagt ttgttttggc accaaaatca acgggacttt ccaaaatgtc gtaacaactc     480

cgccccattg acgcaaatgg gcagggaatt cgagctcggt actcgagcgg tgttccgcgg     540

tcctcctcgt atagaaactc ggaccactct gagacgaagg ctcgcgtcca ggccagcacg     600

aaggaggcta agtgggaggg gtagcggtcg ttgtccacta gggggtccac tcgctccagg     660

gtgtgaagac acatgtcgcc ctcttcggca tcaaggaagg tgattggttt ataggtgtag     720

gccacgtgac cgggtgttcc tgaagggggg ctataaaagg gggtgggggc gcgttcgtcc     780

tcactctctt ccgcatcgct gtctgcgagg gccagctgtt gggctcgcgg ttgaggacaa     840

actcttcgcg gtctttccag tactcttgga tcggaaaccc gtcggcctcc gaacggtact     900

ccgccaccga gggacctgag cgagtccgca tcgaccggat cggaaaacct ctcgactgtt     960

ggggtgagta ctccctctca aaagcgggca tgacttctgc gctaagattg tcagtttcca    1020

aaaacgagga ggatttgata ttcacctggc ccgcggtgat gcctttgagg gtggccgcgt    1080

ccatctggtc agaaaagaca atctttttgt tgtcaagctt gaggtgtggc aggcttgaga    1140

tctggccata cacttgagtg acaatgacat ccactttgcc tttctctcca caggtgtcca    1200

ctcccaggtc caaccggaat tgtacccgcg gccagagctt gcccgggcgc caccatggag    1260

tttgggctga gctggctttt tcttgtcgcg attttaaaag gtgtccagtg cgaggtgcag    1320

ctggtgcagt ctggagcaga ggtgaaaaag cccggggagt ctctgaagat ctcctgtaag    1380

gggtccggat acgcattcag tagttcctgg atcggctggg tgcgccagat gcccgggaaa    1440

ggcctggagt ggatggggcg gatttatcct ggagatggag atactaacta caatggaaag    1500

ttcaagggcc aggtcaccat ctcagccgac aagtccatca gcaccgccta cctgcagtgg    1560

agcagcctga aggctagcga caccgccatg tattactgtg cgagagcgcg cgtgggatcc    1620

acggtctatg atggttacct ctatgcaatg gactactggg gtcaaggtac ctcagtcacc    1680

gtctcctcag cgtcgaccaa gggcccatcg gtcttccccc tggcaccctc ctccaagagc    1740

acctctgggg gcacagcggc cctgggctgc ctggtcaagg actacttccc cgaaccggtg    1800

acggtgtcgt ggaactcagg cgccctgacc agcggcgtgc acaccttccc ggctgtccta 1860
cagtcctcag gactctactc cctcagcagc gtggtgaccg tgccctccag cagcttgggc 1920
acccagacct acatctgcaa cgtgaatcac aagcccagca acaccaaggt ggacaagaaa 1980
gttgagccca aatcttgtga caaaactcac acatgcccac cgtgcccagc acctgaagcc 2040
gcggggggac cgtcagtctt cctcttcccc ccaaaaccca aggacaccct catgatctcc 2100
cggacccctg aggtcacatg cgtggtggtg gacgtgagcc acgaagaccc tgaggtcaag 2160
ttcaactggt acgtggacgg cgtggaggtg cataatgcca agacaaagcc gcgggaggag 2220
cagtacaaca gcacgtaccg tgtggtcagc gtcctcaccg tcctgcacca ggactggctg 2280
aatggcaagg agtacaagtg caaggtctcc aacaaagccc tcccagcccc catcgagaaa 2340
accatctcca aagccaaagg gcagccccga gaaccacagg tgtacaccct gcccccatcc 2400
cgcgaggaga tgaccaagaa ccaggtcagc ctgacctgcc tggtcaaagg cttctatccc 2460
agcgacatcg ccgtggagtg ggagagcaat gggcagccgg agaacaacta caagaccacg 2520
cctcccgtgc tggactccga cggctccttc ttcctctaca gcaagctcac cgtggacaag 2580
agcaggtggc agcaggggaa cgtcttctca tgctccgtga tgcatgaggc tctgcacaac 2640
cactacacgc agaagagcct ctccctgtct aggggtaaac gcgaaccagt ttatttccag 2700
gggagcttgt ttaaggggcc gcgtgattat aacccaatat cgagtgccat ttgtcatcta 2760
acgaatgaat ctgatgggca cacaacatcg ttgtatggta ttggttttgg ccctttcatc 2820
atcacaaaca agcatttgtt tagaagaaat aatggtacac tgttagttca atcactacat 2880
ggtgtgttca aggtaaagaa taccacaact ttgcaacaac acctcattga tgggagggac 2940
atgatgctca ttcgcatgcc taaggatttc ccaccatttc ctcaaaagct gaaattcaga 3000
gagccacaaa gggaagagcg catatgtctt gtgacaacca acttccaaac taagagcatg 3060
tctagcatgg tttcagatac tagttgcaca ttcccttcat ctgatggtat attctggaaa 3120
cattggattc agaccaagga tgggcactgt ggtagcccgt tggtgtcaac tagagatggg 3180
tttattgttg gtatacactc agcatcaaat ttcaccaaca caaacaatta ttttacaagt 3240
gtgccgaaag acttcatgga tttattgaca aatcaagagg cgcagcaatg ggttagtggt 3300
tggcgattga atgctgactc agtgttatgg ggaggccaca aagttttcat gagcaaacct 3360
gaagaaccct ttcagccagt caaagaagca actcaactca tgagtgaatt agtctactcg 3420
caagggatgg acatgcgcgt gcccgcccag ctgctgggcc tgctgctgct gtggttcccc 3480
ggctcgcgat gcgacatcgt gatgacccag tctccagact ccctggctgt gtctctgggc 3540
gagagggcca ccatcaactg caagtccagt cagagccttt catatagaag caatcaaaag 3600
aactcgttgg cctggtacca gcagaaacca ggacagcctc ctaagctgct catttactgg 3660
gctagcacta gggaatctgg ggtccctgac cgattcagtg gatccgggtc tgggacagat 3720
ttcactctca ccatcagcag cctgcaggct gaagatgtgg cagtttatta ctgtcaccaa 3780
tattatagct atccgtacac gttcggaggg gggaccaagg tggaaattaa acgtacggtg 3840

gctgcaccat ctgtcttcat cttcccgcca tctgatgagc agttgaaatc tggaactgcc 3900
tctgttgtgt gcctgctgaa taacttctat cccagagagg ccaaagtaca gtggaaggtg 3960
gataacgccc tccaatcggg taactcccag gagagtgtca cagagcagga cagcaaggac 4020
agcacctaca gcctcagcag caccctgacg ctgagcaaag cagactacga gaaacacaaa 4080
gtctacgcct gcgaagtcac ccatcagggc ctgagctcgc ccgtcacaaa gagcttcaac 4140
aggggagagt gttgagcggc cgcgtttaaa ctgaatgagc gcgtccatcc agacatgata 4200
agatacattg atgagtttgg acaaaccaca actagaatgc agtgaaaaaa atgctttatt 4260
tgtgaaattt gtgatgctat tgctttattt gtaaccatta taagctgcaa taaacaagtt 4320
aacaacaaca attgcattca ttttatgttt caggttcagg gggaggtgtg ggaggttttt 4380
taaagcaagt aaaacctcta caaatgtggt atggctgatt atgatccggc tgcctcgcgc 4440
gtttcggtga tgacggtgaa aacctctgac acatgcagct cccggagacg gtcacagctt 4500
gtctgtaagc ggatgccggg agcagacaag cccgtcaggg cgcgtcagcg ggtgttggcg 4560
ggtgtcgggg cgcagccatg accggtcgac ggcgcgcctt tttttttaat ttttatttta 4620
ttttattttt gacgcgccga aggcgcgatc tgagctcggt acagcttggc tgtggaatgt 4680
gtgtcagtta gggtgtggaa agtccccagg ctccccagca ggcagaagta tgcaaagcat 4740
gcatctcaat tagtcagcaa ccaggtgtgg aaagtcccca ggctccccag caggcagaag 4800
tatgcaaagc atgcatctca attagtcagc aaccatagtc ccgcccctaa ctccgcccat 4860
cccgccccta actccgccca gttccgccca ttctccgccc catggctgac taattttttt 4920
tatttatgca gaggccgagg ccgcctcggc ctctgagcta ttccagaagt agtgaggagg 4980
cttttttgga ggcctaggct tttgcaaaaa gctcctcgag gaactgaaaa accagaaagt 5040
taactggtaa gtttagtctt tttgtctttt atttcaggtc ccggatccgg tggtggtgca 5100
aatcaaagaa ctgctcctca gtggatgttg cctttacttc taggcctgta cggaagtgtt 5160
acttctgctc taaaagctgc ggaattgtac ccgcggccta atacgactca ctatagggac 5220
tagtatggtt cgaccattga actgcatcgt cgccgtgtcc caaaatatgg ggattggcaa 5280
gaacggagac ctaccctggc ctccgctcag gaacgagttc aagtacttcc aaagaatgac 5340
cacaacctct tcagtggaag gtaaacagaa tctggtgatt atgggtagga aaacctggtt 5400
ctccattcct gagaagaatc gacctttaaa ggacagaatt aatatagttc tcagtagaga 5460
actcaaagaa ccaccacgag gagctcattt tcttgccaaa agtttagatg atgccttaag 5520
acttattgaa caaccggaat tggcaagtaa agtagacatg gtttggatag tcggaggcag 5580
ttctgtttac caggaagcca tgaatcaacc aggccacctc agactctttg tgacaaggat 5640
catgcaggaa tttgaaagtg acacgttttt cccagaaatt gatttgggga aatataaact 5700
tctcccagaa tacccaggcg tcctctctga ggtccaggag gaaaaaggca tcaagtataa 5760
gtttgaagtc tacgagaaga aagactaagc ggccgagcgc gcggatctgg aaacgggaga 5820
tgggggaggc taactgaagc acggaaggag acaataccgg aaggaacccg cgctatgacg 5880

gcaataaaaa gacagaataa aacgcacggg tgttgggtcg tttgttcata aacgcggggt 5940
tcggtcccag ggctggcact ctgtcgatac cccaccgaga ccccattggg gccaatacgc 6000
ccgcgtttct tccttttccc caccccaccc cccaagttcg ggtgaaggcc cagggctcgc 6060
agccaacgtc ggggcggcag gccctgccat agccactggc cccgtgggtt agggacgggg 6120
tcccccatgg ggaatggttt atggttcgtg ggggttatta ttttgggcgt tgcgtggggt 6180
ctggagatcc cccgggctgc aggaattccg ttacattact tacggtaaat ggcccgcctg 6240
gctgaccgcc caacgacccc cgcccattga cgtcaataat gacgtatgtt cccatagtaa 6300
cgccaatagg gactttccat tgacgtcaat gggtggagta tttacggtaa actgcccact 6360
tggcagtaca tcaagtgtat catatgccaa gtacgccccc tattgacgtc aatgacggta 6420
aatggcccgc ctggcattat gcccagtaca tgaccttatg ggactttcct acttggcagt 6480
acatctacgt attagtcatc gctattacca tggtgatgcg gttttggcag tacatcaatg 6540
ggcgtggata gcggtttgac tcacggggat ttccaagtct ccaccccatt gacgtcaatg 6600
ggagtttgtt ttggcaccaa aatcaacggg actttccaaa atgtcgtaac aactccgccc 6660
cattgacgca aatgggcggg aattcgagct cggtactcga gcggtgttcc gcggtcctcc 6720
tcgtatagaa actcggacca ctctgagacg aaggctcgcg tccaggccag cacgaaggag 6780
gctaagtggg aggggtagcg gtcgttgtcc actagggggt ccactcgctc cagggtgtga 6840
agacacatgt cgccctcttc ggcatcaagg aaggtgattg gtttataggt gtaggccacg 6900
tgaccgggtg ttcctgaagg ggggctataa aagggggtgg gggcgcgttc gtcctcactc 6960
tcttccgcat cgctgtctgc gagggccagc tgttgggctc gcggttgagg acaaactctt 7020
cgcggtcttt ccagtactct tggatcggaa acccgtcggc ctccgaacgg tactccgcca 7080
ccgagggacc tgagcgagtc cgcatcgacc ggatcggaaa acctctcgac tgttggggtg 7140
agtactccct ctcaaaagcg ggcatgactt ctgcgctaag attgtcagtt tccaaaaacg 7200
aggaggattt gatattcacc tggcccgcgg tgatgccttt gagggtggcc gcgtccatct 7260
ggtcagaaaa gacaatcttt ttgttgtcaa gcttgaggtg tggcaggctt gagatctggc 7320
catacacttg agtgacaatg acatccactt tgcctttctc tccacaggtg tccactccca 7380
ggtccaaccg gaattgtacc cgcggccaga gcttgcgggc gccaccgcgg ccgcggggat 7440
ccagacatga taagatacat tgatgagttt ggacaaacca caactagaat gcagtgaaaa 7500
aaatgcttta tttgtgaaat ttgtgatgct attgctttat ttgtaaccat tataagctgc 7560
aataaacaag ttaacaacaa caattgcatt cattttatgt ttcaggttca gggggaggtg 7620
tgggaggttt tttcggatcc tcttggcgta atcatggtca tagctgtttc ctgtgtgaaa 7680
ttgttatccg ctcacaattc cacacaacat acgagccgga agcataaagt gtaaagcctg 7740
gggtgcctaa tgagtgagct aactcacatt aattgcgttg cgctcactgc ccgctttcca 7800
gtcgggaaac ctgtcgtgcc agctgcatta atgaatcggc caacgcgcgg ggaaaggcgg 7860
tttgcgtatt gggcgctctt ccgcttcctc gctcactgac tcgctgcgct cggtcgttcg 7920

gctgcggcga gcggtatcag ctcactcaaa ggcggtaata cggttatcca cagaatcagg 7980
ggataacgca ggaaagaaca tgtgagcaaa aggccagcaa aaggccagga accgtaaaaa 8040
ggccgcgttg ctggcgtttt tccataggct ccgcccccct gacgagcatc acaaaaatcg 8100
acgctcaagt cagaggtggc gaaacccgac aggactataa agataccagg cgtttccccc 8160
tggaagctcc ctcgtgcgct ctcctgttcc gaccctgccg cttaccggat acctgtccgc 8220
ctttctccct tcgggaagcg tggcgctttc tcatagctca cgctgtaggt atctcagttc 8280
ggtgtaggtc gttcgctcca agctgggctg tgtgcacgaa ccccccgttc agcccgaccg 8340
ctgcgcctta tccggtaact atcgtcttga gtccaacccg gtaagacacg acttatcgcc 8400
actggcagca gccactggta acaggattag cagagcgagg tatgtaggcg gtgctacaga 8460
gttcttgaag tggtggccta actacggcta cactagaaga acagtatttg gtatctgcgc 8520
tctgctgaag ccagttacct tcggaaaaag agttggtagc tcttgatccg gcaaacaaac 8580
caccgctggt agcggtggtt tttttgtttg caagcagcag attacgcgca gaaaaaaagg 8640
atctcaagaa gatcctttga tcttttctac ggggtctgac gctcagtgga acgaaaactc 8700
acgttaaggg attttggtca tgagattatc aaaaaggatc ttcacctaga tccttttaaa 8760
ttaaaaatga agttttaaat caatctaaag tatatatgag taaacttggt ctgacagtta 8820
ccaatgctta atcagtgagg cacctatctc agcgatctgt ctatttcgtt catccatagt 8880
tgcctgactc cccgtcgtgt agataactac gatacgggag ggcttaccat ctggccccag 8940
tgctgcaatg ataccgcgag acccacgctc accggctcca gatttatcag caataaacca 9000
gccagccgga agggccgagc gcagaagtgg tcctgcaact ttatccgcct ccatccagtc 9060
tattaattgt tgccgggaag ctagagtaag tagttcgcca gttaatagtt tgcgcaacgt 9120
tgttgccatt gctacaggca tcgtggtgtc acgctcgtcg tttggtatgg cttcattcag 9180
ctccggttcc caacgatcaa ggcgagttac atgatccccc atgttgtgca aaaaagcggt 9240
tagctccttc ggtcctccga tcgttgtcag aagtaagttg gccgcagtgt tatcactcat 9300
ggttatggca gcactgcata attctcttac tgtcatgcca tccgtaagat gcttttctgt 9360
gactggtgag tactcaacca agtcattctg agaatagtgt atgcggcgac cgagttgctc 9420
ttgcccggcg tcaatacggg ataataccgc gccacatagc agaactttaa aagtgctcat 9480
cattggaaaa cgttcttcgg ggcgaaaact ctcaaggatc ttaccgctgt tgagatccag 9540
ttcgatgtaa cccactcgtg cacccaactg atcttcagca tcttttactt tcaccagcgt 9600
ttctgggtga gcaaaaacag gaaggcaaaa tgccgcaaaa aagggaataa gggcgacacg 9660
gaaatgttga atactcatac tcttcctttt tcaatattat tgaagcattt atcagggtta 9720
ttgtctcatg agcggataca tatttgaatg tatttagaaa aataaacaaa taggggttcc 9780
gcgcacattt ccccgaaaag tgccacctga cgtctaagaa accattatta tcatgacatt 9840
aacctataaa aataggcgta tcacgaggcc ctttcgtctc gcgcgtttcg gtgatgacgg 9900
tgaaaacctc tgacacatgc agctcccgga gacggtcaca gcttgtctgt aagcggatgc 9960

cgggagcaga caagcccgtc agggcgcgtc agcgggtgtt ggcgggtgtc ggggctggct 10020

taactatgcg gcatcagagc agattgtact gagagtgcac catatgcggt gtgaaatacc 10080

gcacagatgc gtaaggagaa aataccgcat caggcgccat tcgccattca ggctgcgcaa 10140

ctgttgggaa gggcgatcgg tgcgggcctc ttcgctatta cgccagctgg cgaaaggggg 10200

atgtgctgca aggcgattaa gttgggtaac gccagggttt tcccagttac gacgttgtaa 10260

aacgacggcc agtgaatt 10278

<210> 41
<211> 2865
<212> DNA
<213> Artificial

<220>
<223> Synthetic construct, ABT-325 TEV polyprotein coding sequence.

<220>
<221> CDS
<222> (1)..(2862)

<400> 41
atg gag ttt ggg ctg agc tgg ctt ttc ctt gtc gcg att tta aaa ggt 48
Met Glu Phe Gly Leu Ser Trp Leu Phe Leu Val Ala Ile Leu Lys Gly
1               5                   10                  15

gtc cag tgt gag gtg cag ctg gtg cag tct gga aca gag gtg aaa aaa 96
Val Gln Cys Glu Val Gln Leu Val Gln Ser Gly Thr Glu Val Lys Lys
            20                  25                  30

ccc ggg gag tct ctg aag atc tcc tgt aag ggt tct gga tac act gtt 144
Pro Gly Glu Ser Leu Lys Ile Ser Cys Lys Gly Ser Gly Tyr Thr Val
        35                  40                  45

acc agt tac tgg atc ggc tgg gtg cgc cag atg ccc ggg aaa ggc ctg 192
Thr Ser Tyr Trp Ile Gly Trp Val Arg Gln Met Pro Gly Lys Gly Leu
    50                  55                  60

gag tgg atg gga ttc atc tat cct ggt gac tct gaa acc aga tac agt 240
Glu Trp Met Gly Phe Ile Tyr Pro Gly Asp Ser Glu Thr Arg Tyr Ser
65                  70                  75                  80

ccg acc ttc caa ggc cag gtc acc atc tca gcc gac aag tcc ttc aat 288
Pro Thr Phe Gln Gly Gln Val Thr Ile Ser Ala Asp Lys Ser Phe Asn
                85                  90                  95

acc gcc ttc ctg cag tgg agc agt cta aag gcc tcg gac acc gcc atg 336
Thr Ala Phe Leu Gln Trp Ser Ser Leu Lys Ala Ser Asp Thr Ala Met
            100                 105                 110

tat tac tgt gcg cga gtc ggc agt ggc tgg tac cct tat act ttt gat 384
Tyr Tyr Cys Ala Arg Val Gly Ser Gly Trp Tyr Pro Tyr Thr Phe Asp
        115                 120                 125

atc tgg ggc caa ggg aca atg gtc acc gtc tct tca gcg tcg acc aag 432
Ile Trp Gly Gln Gly Thr Met Val Thr Val Ser Ser Ala Ser Thr Lys
    130                 135                 140

ggc cca tcg gtc ttc ccc ctg gca ccc tcc tcc aag agc acc tct ggg 480
Gly Pro Ser Val Phe Pro Leu Ala Pro Ser Ser Lys Ser Thr Ser Gly
145                 150                 155                 160

ggc aca gcg gcc ctg ggc tgc ctg gtc aag gac tac ttc ccc gaa ccg 528

Gly Thr Ala Ala Leu Gly Cys Leu Val Lys Asp Tyr Phe Pro Glu Pro
                165                 170                 175

gtg acg gtg tcg tgg aac tca ggc gcc ctg acc agc ggc gtg cac acc 576
Val Thr Val Ser Trp Asn Ser Gly Ala Leu Thr Ser Gly Val His Thr
            180                 185                 190

ttc ccg gct gtc cta cag tcc tca gga ctc tac tcc ctc agc agc gtg 624
Phe Pro Ala Val Leu Gln Ser Ser Gly Leu Tyr Ser Leu Ser Ser Val
        195                 200                 205

gtg acc gtg ccc tcc agc agc ttg ggc acc cag acc tac atc tgc aac 672
Val Thr Val Pro Ser Ser Ser Leu Gly Thr Gln Thr Tyr Ile Cys Asn
    210                 215                 220

gtg aat cac aag ccc agc aac acc aag gtg gac aag aaa gtt gag ccc 720
Val Asn His Lys Pro Ser Asn Thr Lys Val Asp Lys Lys Val Glu Pro
225                 230                 235                 240

aaa tct tgt gac aaa act cac aca tgc cca ccg tgc cca gca cct gaa 768
Lys Ser Cys Asp Lys Thr His Thr Cys Pro Pro Cys Pro Ala Pro Glu
                245                 250                 255

gcc gcg ggg gga ccg tca gtc ttc ctc ttc ccc cca aaa ccc aag gac 816
Ala Ala Gly Gly Pro Ser Val Phe Leu Phe Pro Pro Lys Pro Lys Asp
            260                 265                 270

acc ctc atg atc tcc cgg acc cct gag gtc aca tgc gtg gtg gtg gac 864
Thr Leu Met Ile Ser Arg Thr Pro Glu Val Thr Cys Val Val Val Asp
        275                 280                 285

gtg agc cac gaa gac cct gag gtc aag ttc aac tgg tac gtg gac ggc 912
Val Ser His Glu Asp Pro Glu Val Lys Phe Asn Trp Tyr Val Asp Gly
    290                 295                 300

gtg gag gtg cat aat gcc aag aca aag ccg cgg gag gag cag tac aac 960
Val Glu Val His Asn Ala Lys Thr Lys Pro Arg Glu Glu Gln Tyr Asn
305                 310                 315                 320

agc acg tac cgt gtg gtc agc gtc ctc acc gtc ctg cac cag gac tgg 1008
Ser Thr Tyr Arg Val Val Ser Val Leu Thr Val Leu His Gln Asp Trp
                325                 330                 335

ctg aat ggc aag gag tac aag tgc aag gtc tcc aac aaa gcc ctc cca 1056
Leu Asn Gly Lys Glu Tyr Lys Cys Lys Val Ser Asn Lys Ala Leu Pro
            340                 345                 350

gcc ccc atc gag aaa acc atc tcc aaa gcc aaa ggg cag ccc cga gaa 1104
Ala Pro Ile Glu Lys Thr Ile Ser Lys Ala Lys Gly Gln Pro Arg Glu
        355                 360                 365

cca cag gtg tac acc ctg ccc cca tcc cgc gag gag atg acc aag aac 1152
Pro Gln Val Tyr Thr Leu Pro Pro Ser Arg Glu Glu Met Thr Lys Asn
    370                 375                 380

cag gtc agc ctg acc tgc ctg gtc aaa ggc ttc tat ccc agc gac atc 1200
Gln Val Ser Leu Thr Cys Leu Val Lys Gly Phe Tyr Pro Ser Asp Ile
385                 390                 395                 400

gcc gtg gag tgg gag agc aat ggg cag ccg gag aac aac tac aag acc 1248
Ala Val Glu Trp Glu Ser Asn Gly Gln Pro Glu Asn Asn Tyr Lys Thr
                405                 410                 415

acg cct ccc gtg ctg gac tcc gac ggc tcc ttc ttc ctc tac agc aag 1296
Thr Pro Pro Val Leu Asp Ser Asp Gly Ser Phe Phe Leu Tyr Ser Lys
            420                 425                 430

ctc acc gtg gac aag agc agg tgg cag cag ggg aac gtc ttc tca tgc 1344

Leu Thr Val Asp Lys Ser Arg Trp Gln Gln Gly Asn Val Phe Ser Cys
        435                 440                 445

tcc gtg atg cat gag gct ctg cac aac cac tac acg cag aag agc ctc      1392
Ser Val Met His Glu Ala Leu His Asn His Tyr Thr Gln Lys Ser Leu
    450                 455                 460

tcc ctg tct agg ggt aaa cgc gaa cca gtt tat ttc cag ggg agc ttg      1440
Ser Leu Ser Arg Gly Lys Arg Glu Pro Val Tyr Phe Gln Gly Ser Leu
465                 470                 475                 480

ttt aag ggg ccg cgt gat tat aac cca ata tcg agt gcc att tgt cat      1488
Phe Lys Gly Pro Arg Asp Tyr Asn Pro Ile Ser Ser Ala Ile Cys His
                485                 490                 495

cta acg aat gaa tct gat ggg cac aca aca tcg ttg tat ggt att ggt      1536
Leu Thr Asn Glu Ser Asp Gly His Thr Thr Ser Leu Tyr Gly Ile Gly
            500                 505                 510

ttt ggc cct ttc atc atc aca aac aag cat ttg ttt aga aga aat aat      1584
Phe Gly Pro Phe Ile Ile Thr Asn Lys His Leu Phe Arg Arg Asn Asn
        515                 520                 525

ggt aca ctg tta gtt caa tca cta cat ggt gtg ttc aag gta aag aat      1632
Gly Thr Leu Leu Val Gln Ser Leu His Gly Val Phe Lys Val Lys Asn
    530                 535                 540

acc aca act ttg caa caa cac ctc att gat ggg agg gac atg atg ctc      1680
Thr Thr Thr Leu Gln Gln His Leu Ile Asp Gly Arg Asp Met Met Leu
545                 550                 555                 560

att cgc atg cct aag gat ttc cca cca ttt cct caa aag ctg aaa ttc      1728
Ile Arg Met Pro Lys Asp Phe Pro Pro Phe Pro Gln Lys Leu Lys Phe
                565                 570                 575

aga gag cca caa agg gaa gag cgc ata tgt ctt gtg aca acc aac ttc      1776
Arg Glu Pro Gln Arg Glu Glu Arg Ile Cys Leu Val Thr Thr Asn Phe
            580                 585                 590

caa act aag agc atg tct agc atg gtt tca gat act agt tgc aca ttc      1824
Gln Thr Lys Ser Met Ser Ser Met Val Ser Asp Thr Ser Cys Thr Phe
        595                 600                 605

cct tca tct gat ggt ata ttc tgg aaa cat tgg att cag acc aag gat      1872
Pro Ser Ser Asp Gly Ile Phe Trp Lys His Trp Ile Gln Thr Lys Asp
    610                 615                 620

ggg cac tgt ggt agc ccg ttg gtg tca act aga gat ggg ttt att gtt      1920
Gly His Cys Gly Ser Pro Leu Val Ser Thr Arg Asp Gly Phe Ile Val
625                 630                 635                 640

ggt ata cac tca gca tca aat ttc acc aac aca aac aat tat ttt aca      1968
Gly Ile His Ser Ala Ser Asn Phe Thr Asn Thr Asn Asn Tyr Phe Thr
                645                 650                 655

agt gtg ccg aaa gac ttc atg gat tta ttg aca aat caa gag gcg cag      2016
Ser Val Pro Lys Asp Phe Met Asp Leu Leu Thr Asn Gln Glu Ala Gln
            660                 665                 670

caa tgg gtt agt ggt tgg cga ttg aat gct gac tca gtg tta tgg gga      2064
Gln Trp Val Ser Gly Trp Arg Leu Asn Ala Asp Ser Val Leu Trp Gly
        675                 680                 685

ggc cac aaa gtt ttc atg agc aaa cct gaa gaa ccc ttt cag cca gtc      2112
Gly His Lys Val Phe Met Ser Lys Pro Glu Glu Pro Phe Gln Pro Val
    690                 695                 700

aaa gaa gca act caa ctc atg agt gaa tta gtc tac tcg caa ggg atg      2160

Lys Glu Ala Thr Gln Leu Met Ser Glu Leu Val Tyr Ser Gln Gly Met
705                 710                 715                 720

gaa gcc cca gcg cag ctt ctc ttc ctc ctg cta ctc tgg ctc cca gat     2208
Glu Ala Pro Ala Gln Leu Leu Phe Leu Leu Leu Leu Trp Leu Pro Asp
                725                 730                 735

acc act gga gaa ata gtg atg acg cag tct cca gcc acc ctg tct gtg     2256
Thr Thr Gly Glu Ile Val Met Thr Gln Ser Pro Ala Thr Leu Ser Val
            740                 745                 750

tct cca ggg gaa aga gcc acc ctc tcc tgc agg gcc agt gag agt att     2304
Ser Pro Gly Glu Arg Ala Thr Leu Ser Cys Arg Ala Ser Glu Ser Ile
        755                 760                 765

agc agc aac tta gcc tgg tac cag cag aaa cct ggc cag gct ccc agg     2352
Ser Ser Asn Leu Ala Trp Tyr Gln Gln Lys Pro Gly Gln Ala Pro Arg
    770                 775                 780

ctc ttc atc tat act gca tcc acc agg gcc act gat atc cca gcc agg     2400
Leu Phe Ile Tyr Thr Ala Ser Thr Arg Ala Thr Asp Ile Pro Ala Arg
785                 790                 795                 800

ttc agt ggc agt ggg tct ggg aca gag ttc act ctc acc atc agc agc     2448
Phe Ser Gly Ser Gly Ser Gly Thr Glu Phe Thr Leu Thr Ile Ser Ser
                805                 810                 815

ctg cag tct gaa gat ttt gca gtt tat tac tgt cag cag tat aat aac     2496
Leu Gln Ser Glu Asp Phe Ala Val Tyr Tyr Cys Gln Gln Tyr Asn Asn
            820                 825                 830

tgg cct tcg atc acc ttc ggc caa ggg aca cga ctg gag att aaa cga     2544
Trp Pro Ser Ile Thr Phe Gly Gln Gly Thr Arg Leu Glu Ile Lys Arg
        835                 840                 845

act gtg gct gca cca tct gtc ttc atc ttc ccg cca tct gat gag cag     2592
Thr Val Ala Ala Pro Ser Val Phe Ile Phe Pro Pro Ser Asp Glu Gln
    850                 855                 860

ttg aaa tct gga act gct agc gtt gtg tgc ctg ctg aat aac ttc tat     2640
Leu Lys Ser Gly Thr Ala Ser Val Val Cys Leu Leu Asn Asn Phe Tyr
865                 870                 875                 880

ccc aga gag gcc aaa gta cag tgg aag gtg gat aac gcc ctc caa tcg     2688
Pro Arg Glu Ala Lys Val Gln Trp Lys Val Asp Asn Ala Leu Gln Ser
                885                 890                 895

ggt aac tcc cag gag agt gtc aca gag cag gac agc aag gac agc acc     2736
Gly Asn Ser Gln Glu Ser Val Thr Glu Gln Asp Ser Lys Asp Ser Thr
            900                 905                 910

tac agc ctc agc agc acc ctg acg ctg agc aaa gca gac tac gag aaa     2784
Tyr Ser Leu Ser Ser Thr Leu Thr Leu Ser Lys Ala Asp Tyr Glu Lys
        915                 920                 925

cac aaa gtc tac gcc tgc gaa gtc acc cat cag ggc ctg agc tcg ccc     2832
His Lys Val Tyr Ala Cys Glu Val Thr His Gln Gly Leu Ser Ser Pro
    930                 935                 940

gtc aca aag agc ttc aac agg gga gag tgt tga                         2865
Val Thr Lys Ser Phe Asn Arg Gly Glu Cys
945                 950


<210>  42
<211>  954
<212>  PRT
<213>  Artificial

<220>
<223>  Synthetic Construct

<400>  42

Met Glu Phe Gly Leu Ser Trp Leu Phe Leu Val Ala Ile Leu Lys Gly
1               5                   10                  15

Val Gln Cys Glu Val Gln Leu Val Gln Ser Gly Thr Glu Val Lys Lys
            20                  25                  30

Pro Gly Glu Ser Leu Lys Ile Ser Cys Lys Gly Ser Gly Tyr Thr Val
        35                  40                  45

Thr Ser Tyr Trp Ile Gly Trp Val Arg Gln Met Pro Gly Lys Gly Leu
    50                  55                  60

Glu Trp Met Gly Phe Ile Tyr Pro Gly Asp Ser Glu Thr Arg Tyr Ser
65                  70                  75                  80

Pro Thr Phe Gln Gly Gln Val Thr Ile Ser Ala Asp Lys Ser Phe Asn
                85                  90                  95

Thr Ala Phe Leu Gln Trp Ser Ser Leu Lys Ala Ser Asp Thr Ala Met
            100                 105                 110

Tyr Tyr Cys Ala Arg Val Gly Ser Gly Trp Tyr Pro Tyr Thr Phe Asp
        115                 120                 125

Ile Trp Gly Gln Gly Thr Met Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu
                245                 250                 255

Ala Ala Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Glu Glu Met Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Arg Gly Lys Arg Glu Pro Val Tyr Phe Gln Gly Ser Leu
465                 470                 475                 480

Phe Lys Gly Pro Arg Asp Tyr Asn Pro Ile Ser Ser Ala Ile Cys His
                485                 490                 495

Leu Thr Asn Glu Ser Asp Gly His Thr Thr Ser Leu Tyr Gly Ile Gly
            500                 505                 510

Phe Gly Pro Phe Ile Ile Thr Asn Lys His Leu Phe Arg Arg Asn Asn
        515                 520                 525

Gly Thr Leu Leu Val Gln Ser Leu His Gly Val Phe Lys Val Lys Asn
    530                 535                 540

Thr Thr Thr Leu Gln Gln His Leu Ile Asp Gly Arg Asp Met Met Leu
545                 550                 555                 560

Ile Arg Met Pro Lys Asp Phe Pro Pro Phe Pro Gln Lys Leu Lys Phe
                565                 570                 575

Arg Glu Pro Gln Arg Glu Glu Arg Ile Cys Leu Val Thr Thr Asn Phe
            580                 585                 590

Gln Thr Lys Ser Met Ser Ser Met Val Ser Asp Thr Ser Cys Thr Phe
        595                 600                 605

Pro Ser Ser Asp Gly Ile Phe Trp Lys His Trp Ile Gln Thr Lys Asp
    610                 615                 620

Gly His Cys Gly Ser Pro Leu Val Ser Thr Arg Asp Gly Phe Ile Val
625                 630                 635                 640

Gly Ile His Ser Ala Ser Asn Phe Thr Asn Thr Asn Asn Tyr Phe Thr
                645                 650                 655

Ser Val Pro Lys Asp Phe Met Asp Leu Leu Thr Asn Gln Glu Ala Gln
            660                 665                 670

Gln Trp Val Ser Gly Trp Arg Leu Asn Ala Asp Ser Val Leu Trp Gly
        675                 680                 685

Gly His Lys Val Phe Met Ser Lys Pro Glu Glu Pro Phe Gln Pro Val
    690                 695                 700

Lys Glu Ala Thr Gln Leu Met Ser Glu Leu Val Tyr Ser Gln Gly Met
705                 710                 715                 720

Glu Ala Pro Ala Gln Leu Leu Phe Leu Leu Leu Leu Trp Leu Pro Asp
                725                 730                 735

Thr Thr Gly Glu Ile Val Met Thr Gln Ser Pro Ala Thr Leu Ser Val
            740                 745                 750

Ser Pro Gly Glu Arg Ala Thr Leu Ser Cys Arg Ala Ser Glu Ser Ile
        755                 760                 765

Ser Ser Asn Leu Ala Trp Tyr Gln Gln Lys Pro Gly Gln Ala Pro Arg
    770                 775                 780

Leu Phe Ile Tyr Thr Ala Ser Thr Arg Ala Thr Asp Ile Pro Ala Arg
785                 790                 795                 800

Phe Ser Gly Ser Gly Ser Gly Thr Glu Phe Thr Leu Thr Ile Ser Ser
                805                 810                 815

Leu Gln Ser Glu Asp Phe Ala Val Tyr Tyr Cys Gln Gln Tyr Asn Asn
            820                 825                 830

Trp Pro Ser Ile Thr Phe Gly Gln Gly Thr Arg Leu Glu Ile Lys Arg
        835                 840                 845

Thr Val Ala Ala Pro Ser Val Phe Ile Phe Pro Pro Ser Asp Glu Gln
    850                 855                 860

Leu Lys Ser Gly Thr Ala Ser Val Val Cys Leu Leu Asn Asn Phe Tyr
865                 870                 875                 880

Pro Arg Glu Ala Lys Val Gln Trp Lys Val Asp Asn Ala Leu Gln Ser
                885                 890                 895

Gly Asn Ser Gln Glu Ser Val Thr Glu Gln Asp Ser Lys Asp Ser Thr
            900                 905                 910

Tyr Ser Leu Ser Ser Thr Leu Thr Leu Ser Lys Ala Asp Tyr Glu Lys
        915                 920                 925

His Lys Val Tyr Ala Cys Glu Val Thr His Gln Gly Leu Ser Ser Pro
    930                 935                 940

Val Thr Lys Ser Phe Asn Arg Gly Glu Cys
945                 950

<210>  43
<211>  10242
<212>  DNA
<213>  Artificial

<220>
<223>  Synthetic construct, ABT-325 TEV polyprotein expression vector.

<400>  43
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga     240

cggtaaatgg cccgcctggc attatgccca gtacatgacc ttatgggact ttcctacttg     300

gcagtacatc tacgtattag tcatcgctat taccatggtg atgcggtttt ggcagtacat     360

caatgggcgt ggatagcggt ttgactcacg gggatttcca agtctccacc ccattgacgt     420

caatgggagt ttgttttggc accaaaatca acgggacttt ccaaaatgtc gtaacaactc     480

cgccccattg acgcaaatgg gcagggaatt cgagctcggt actcgagcgg tgttccgcgg     540

tcctcctcgt atagaaactc ggaccactct gagacgaagg ctcgcgtcca ggccagcacg 600
aaggaggcta agtgggaggg gtagcggtcg ttgtccacta gggggtccac tcgctccagg 660
gtgtgaagac acatgtcgcc ctcttcggca tcaaggaagg tgattggttt ataggtgtag 720
gccacgtgac cgggtgttcc tgaagggggg ctataaaagg gggtgggggc gcgttcgtcc 780
tcactctctt ccgcatcgct gtctgcgagg gccagctgtt gggctcgcgg ttgaggacaa 840
actcttcgcg gtctttccag tactcttgga tcggaaaccc gtcggcctcc gaacggtact 900
ccgccaccga gggacctgag cgagtccgca tcgaccggat cggaaaacct ctcgactgtt 960
ggggtgagta ctccctctca aaagcgggca tgacttctgc gctaagattg tcagtttcca 1020
aaaacgagga ggatttgata ttcacctggc ccgcggtgat gcctttgagg gtggccgcgt 1080
ccatctggtc agaaaagaca atctttttgt tgtcaagctt gaggtgtggc aggcttgaga 1140
tctggccata cacttgagtg acaatgacat ccactttgcc tttctctcca caggtgtcca 1200
ctcccaggtc caaccggaat tgtacccgcg gccagagctt gcccgggcgc caccatggag 1260
tttgggctga gctggctttt ccttgtcgcg attttaaaag gtgtccagtg tgaggtgcag 1320
ctggtgcagt ctggaacaga ggtgaaaaaa cccggggagt ctctgaagat ctcctgtaag 1380
ggttctggat acactgttac cagttactgg atcggctggg tgcgccagat gcccgggaaa 1440
ggcctggagt ggatgggatt catctatcct ggtgactctg aaaccagata cagtccgacc 1500
ttccaaggcc aggtcaccat ctcagccgac aagtccttca ataccgcctt cctgcagtgg 1560
agcagtctaa aggcctcgga caccgccatg tattactgtg cgcgagtcgg cagtggctgg 1620
tacccttata cttttgatat ctggggccaa gggacaatgg tcaccgtctc ttcagcgtcg 1680
accaagggcc catcggtctt ccccctggca ccctcctcca agagcacctc tgggggcaca 1740
gcggccctgg gctgcctggt caaggactac ttccccgaac cggtgacggt gtcgtggaac 1800
tcaggcgccc tgaccagcgg cgtgcacacc ttcccggctg tcctacagtc ctcaggactc 1860
tactccctca gcagcgtggt gaccgtgccc tccagcagct tgggcaccca gacctacatc 1920
tgcaacgtga atcacaagcc cagcaacacc aaggtggaca agaaagttga gcccaaatct 1980
tgtgacaaaa ctcacacatg cccaccgtgc ccagcacctg aagccgcggg gggaccgtca 2040
gtcttcctct tccccccaaa acccaaggac accctcatga tctcccggac ccctgaggtc 2100
acatgcgtgg tggtggacgt gagccacgaa gaccctgagg tcaagttcaa ctggtacgtg 2160
gacggcgtgg aggtgcataa tgccaagaca aagccgcggg aggagcagta caacagcacg 2220
taccgtgtgg tcagcgtcct caccgtcctg caccaggact ggctgaatgg caaggagtac 2280
aagtgcaagg tctccaacaa agccctccca gcccccatcg agaaaaccat ctccaaagcc 2340
aaagggcagc cccgagaacc acaggtgtac accctgcccc catcccgcga ggagatgacc 2400
aagaaccagg tcagcctgac ctgcctggtc aaaggcttct atcccagcga catcgccgtg 2460
gagtgggaga gcaatgggca gccggagaac aactacaaga ccacgcctcc cgtgctggac 2520
tccgacggct ccttcttcct ctacagcaag ctcaccgtgg acaagagcag gtggcagcag 2580

gggaacgtct tctcatgctc cgtgatgcat gaggctctgc acaaccacta cacgcagaag 2640
agcctctccc tgtctagggg taaacgcgaa ccagtttatt tccaggggag cttgtttaag 2700
gggccgcgtg attataaccc aatatcgagt gccatttgtc atctaacgaa tgaatctgat 2760
gggcacacaa catcgttgta tggtattggt tttggccctt tcatcatcac aaacaagcat 2820
ttgtttagaa gaaataatgg tacactgtta gttcaatcac tacatggtgt gttcaaggta 2880
aagaatacca caactttgca acaacacctc attgatggga gggacatgat gctcattcgc 2940
atgcctaagg atttcccacc atttcctcaa aagctgaaat tcagagagcc acaaagggaa 3000
gagcgcatat gtcttgtgac aaccaacttc caaactaaga gcatgtctag catggtttca 3060
gatactagtt gcacattccc ttcatctgat ggtatattct ggaaacattg gattcagacc 3120
aaggatgggc actgtggtag cccgttggtg tcaactagag atgggtttat tgttggtata 3180
cactcagcat caaatttcac caacacaaac aattatttta caagtgtgcc gaaagacttc 3240
atggatttat tgacaaatca agaggcgcag caatgggtta gtggttggcg attgaatgct 3300
gactcagtgt tatggggagg ccacaaagtt ttcatgagca aacctgaaga accctttcag 3360
ccagtcaaag aagcaactca actcatgagt gaattagtct actcgcaagg gatggaagcc 3420
ccagcgcagc ttctcttcct cctgctactc tggctcccag ataccactgg agaaatagtg 3480
atgacgcagt ctccagccac cctgtctgtg tctccagggg aaagagccac cctctcctgc 3540
agggccagtg agagtattag cagcaactta gcctggtacc agcagaaacc tggccaggct 3600
cccaggctct tcatctatac tgcatccacc agggccactg atatcccagc caggttcagt 3660
ggcagtgggt ctgggacaga gttcactctc accatcagca gcctgcagtc tgaagatttt 3720
gcagtttatt actgtcagca gtataataac tggccttcga tcaccttcgg ccaagggaca 3780
cgactggaga ttaaacgaac tgtggctgca ccatctgtct tcatcttccc gccatctgat 3840
gagcagttga aatctggaac tgctagcgtt gtgtgcctgc tgaataactt ctatcccaga 3900
gaggccaaag tacagtggaa ggtggataac gccctccaat cgggtaactc ccaggagagt 3960
gtcacagagc aggacagcaa ggacagcacc tacagcctca gcagcaccct gacgctgagc 4020
aaagcagact acgagaaaca caaagtctac gcctgcgaag tcacccatca gggcctgagc 4080
tcgcccgtca caaagagctt caacagggga gagtgttgag cggccgcgtt taaactgaat 4140
gagcgcgtcc atccagacat gataagatac attgatgagt ttggacaaac cacaactaga 4200
atgcagtgaa aaaaatgctt tatttgtgaa atttgtgatg ctattgcttt atttgtaacc 4260
attataagct gcaataaaca agttaacaac aacaattgca ttcattttat gtttcaggtt 4320
cagggggagg tgtgggaggt tttttaaagc aagtaaaacc tctacaaatg tggtatggct 4380
gattatgatc cggctgcctc gcgcgtttcg gtgatgacgg tgaaaacctc tgacacatgc 4440
agctcccgga gacggtcaca gcttgtctgt aagcggatgc cgggagcaga caagcccgtc 4500
agggcgcgtc agcgggtgtt ggcgggtgtc ggggcgcagc catgaccggt cgacggcgcg 4560
cctttttttt taatttttat tttattttat ttttgacgcg ccgaaggcgc gatctgagct 4620

cggtacagct tggctgtgga atgtgtgtca gttagggtgt ggaaagtccc caggctcccc 4680
agcaggcaga agtatgcaaa gcatgcatct caattagtca gcaaccaggt gtggaaagtc 4740
cccaggctcc ccagcaggca gaagtatgca aagcatgcat ctcaattagt cagcaaccat 4800
agtcccgccc ctaactccgc ccatcccgcc cctaactccg cccagttccg cccattctcc 4860
gccccatggc tgactaattt tttttattta tgcagaggcc gaggccgcct cggcctctga 4920
gctattccag aagtagtgag gaggcttttt tggaggccta ggcttttgca aaaagctcct 4980
cgaggaactg aaaaaccaga aagttaactg gtaagtttag tctttttgtc ttttatttca 5040
ggtcccggat ccggtggtgg tgcaaatcaa agaactgctc ctcagtggat gttgccttta 5100
cttctaggcc tgtacggaag tgttacttct gctctaaaag ctgcggaatt gtacccgcgg 5160
cctaatacga ctcactatag ggactagtat ggttcgacca ttgaactgca tcgtcgccgt 5220
gtcccaaaat atggggattg gcaagaacgg agacctaccc tggcctccgc tcaggaacga 5280
gttcaagtac ttccaaagaa tgaccacaac ctcttcagtg gaaggtaaac agaatctggt 5340
gattatgggt aggaaaacct ggttctccat tcctgagaag aatcgacctt taaaggacag 5400
aattaatata gttctcagta gagaactcaa agaaccacca cgaggagctc attttcttgc 5460
caaaagttta gatgatgcct taagacttat tgaacaaccg gaattggcaa gtaaagtaga 5520
catggtttgg atagtcggag gcagttctgt ttaccaggaa gccatgaatc aaccaggcca 5580
cctcagactc tttgtgacaa ggatcatgca ggaatttgaa agtgacacgt ttttcccaga 5640
aattgatttg gggaaatata aacttctccc agaataccca ggcgtcctct ctgaggtcca 5700
ggaggaaaaa ggcatcaagt ataagtttga agtctacgag aagaaagact aagcggccga 5760
gcgcgcggat ctggaaacgg gagatggggg aggctaactg aagcacggaa ggagacaata 5820
ccggaaggaa cccgcgctat gacggcaata aaaagacaga ataaaacgca cgggtgttgg 5880
gtcgtttgtt cataaacgcg gggttcggtc ccagggctgg cactctgtcg ataccccacc 5940
gagaccccat tggggccaat acgcccgcgt ttcttccttt tccccacccc accccccaag 6000
ttcgggtgaa ggcccagggc tcgcagccaa cgtcggggcg gcaggccctg ccatagccac 6060
tggccccgtg ggttagggac ggggtccccc atggggaatg gtttatggtt cgtgggggtt 6120
attattttgg gcgttgcgtg gggtctggag atcccccggg ctgcaggaat tccgttacat 6180
tacttacggt aaatggcccg cctggctgac cgcccaacga cccccgccca ttgacgtcaa 6240
taatgacgta tgttcccata gtaacgccaa tagggacttt ccattgacgt caatgggtgg 6300
agtatttacg gtaaactgcc cacttggcag tacatcaagt gtatcatatg ccaagtacgc 6360
cccctattga cgtcaatgac ggtaaatggc ccgcctggca ttatgcccag tacatgacct 6420
tatgggactt tcctacttgg cagtacatct acgtattagt catcgctatt accatggtga 6480
tgcggttttg gcagtacatc aatgggcgtg gatagcggtt tgactcacgg ggatttccaa 6540
gtctccaccc cattgacgtc aatgggagtt tgttttggca ccaaaatcaa cgggactttc 6600
caaaatgtcg taacaactcc gccccattga cgcaaatggg cgggaattcg agctcggtac 6660

tcgagcggtg ttccgcggtc ctcctcgtat agaaactcgg accactctga gacgaaggct 6720
cgcgtccagg ccagcacgaa ggaggctaag tgggaggggt agcggtcgtt gtccactagg 6780
gggtccactc gctccagggt gtgaagacac atgtcgccct cttcggcatc aaggaaggtg 6840
attggtttat aggtgtaggc cacgtgaccg ggtgttcctg aaggggggct ataaaagggg 6900
gtgggggcgc gttcgtcctc actctcttcc gcatcgctgt ctgcgagggc cagctgttgg 6960
gctcgcggtt gaggacaaac tcttcgcggt ctttccagta ctcttggatc ggaaacccgt 7020
cggcctccga acggtactcc gccaccgagg gacctgagcg agtccgcatc gaccggatcg 7080
gaaaacctct cgactgttgg ggtgagtact ccctctcaaa agcgggcatg acttctgcgc 7140
taagattgtc agtttccaaa aacgaggagg atttgatatt cacctggccc gcggtgatgc 7200
ctttgagggt ggccgcgtcc atctggtcag aaaagacaat ctttttgttg tcaagcttga 7260
ggtgtggcag gcttgagatc tggccataca cttgagtgac aatgacatcc actttgcctt 7320
tctctccaca ggtgtccact cccaggtcca accggaattg tacccgcggc cagagcttgc 7380
gggcgccacc gcggccgcgg ggatccagac atgataagat acattgatga gtttggacaa 7440
accacaacta gaatgcagtg aaaaaaatgc tttatttgtg aaatttgtga tgctattgct 7500
ttatttgtaa ccattataag ctgcaataaa caagttaaca acaacaattg cattcatttt 7560
atgtttcagg ttcaggggga ggtgtgggag gttttttcgg atcctcttgg cgtaatcatg 7620
gtcatagctg tttcctgtgt gaaattgtta tccgctcaca attccacaca acatacgagc 7680
cggaagcata aagtgtaaag cctggggtgc ctaatgagtg agctaactca cattaattgc 7740
gttgcgctca ctgcccgctt tccagtcggg aaacctgtcg tgccagctgc attaatgaat 7800
cggccaacgc gcggggaaag gcggtttgcg tattgggcgc tcttccgctt cctcgctcac 7860
tgactcgctg cgctcggtcg ttcggctgcg gcgagcggta tcagctcact caaaggcggt 7920
aatacggtta tccacagaat caggggataa cgcaggaaag aacatgtgag caaaaggcca 7980
gcaaaaggcc aggaaccgta aaaaggccgc gttgctggcg ttcttccata ggctccgccc 8040
ccctgacgag catcacaaaa atcgacgctc aagtcagagg tggcgaaacc cgacaggact 8100
ataaagatac caggcgtttc cccctggaag ctccctcgtg cgctctcctg ttccgaccct 8160
gccgcttacc ggatacctgt ccgcctttct cccttcggga agcgtggcgc tttctcatag 8220
ctcacgctgt aggtatctca gttcggtgta ggtcgttcgc tccaagctgg gctgtgtgca 8280
cgaacccccc gttcagcccg accgctgcgc cttatccggt aactatcgtc ttgagtccaa 8340
cccggtaaga cacgacttat cgccactggc agcagccact ggtaacagga ttagcagagc 8400
gaggtatgta ggcggtgcta cagagttctt gaagtggtgg cctaactacg gctacactag 8460
aagaacagta tttggtatct gcgctctgct gaagccagtt accttcggaa aaagagttgg 8520
tagctcttga tccggcaaac aaaccaccgc tggtagcggt ggtttttttg tttgcaagca 8580
gcagattacg cgcagaaaaa aaggatctca agaagatcct ttgatctttt ctacggggtc 8640
tgacgctcag tggaacgaaa actcacgtta agggattttg gtcatgagat tatcaaaaag 8700

gatcttcacc tagatccttt ttaattaaaa atgaagtttt aaatcaatct aaagtatata 8760

tgagtaaact tggtctgaca gttaccaatg cttaatcagt gaggcaccta tctcagcgat 8820

ctgtctattt cgttcatcca tagttgcctg actccccgtc gtgtagataa ctacgatacg 8880

ggagggctta ccatctggcc ccagtgctgc aatgataccg cgagacccac gctcaccggc 8940

tccagattta tcagcaataa accagccagc cggaagggcc gagcgcagaa gtggtcctgc 9000

aactttatcc gcctccatcc agtctattaa ttgttgccgg gaagctagag taagtagttc 9060

gccagttaat agtttgcgca acgttgttgc cattgctaca ggcatcgtgg tgtcacgctc 9120

gtcgtttggt atggcttcat tcagctccgg ttcccaacga tcaaggcgag ttacatgatc 9180

ccccatgttg tgcaaaaaag cggttagctc cttcggtcct ccgatcgttg tcagaagtaa 9240

gttggccgca gtgttatcac tcatggttat ggcagcactg cataattctc ttactgtcat 9300

gccatccgta agatgctttt ctgtgactgg tgagtactca accaagtcat tctgagaata 9360

gtgtatgcgg cgaccgagtt gctcttgccc ggcgtcaata cgggataata ccgcgccaca 9420

tagcagaact ttaaaagtgc tcatcattgg aaaacgttct tcggggcgaa aactctcaag 9480

gatcttaccg ctgttgagat ccagttcgat gtaacccact cgtgcaccca actgatcttc 9540

agcatctttt actttcacca gcgtttctgg gtgagcaaaa acaggaaggc aaaatgccgc 9600

aaaaaaggga ataagggcga cacggaaatg ttgaatactc atactcttcc tttttcaata 9660

ttattgaagc atttatcagg gttattgtct catgagcgga tacatatttg aatgtattta 9720

gaaaaataaa caaatagggg ttccgcgcac atttccccga aaagtgccac ctgacgtcta 9780

agaaaccatt attatcatga cattaaccta taaaaatagg cgtatcacga ggccctttcg 9840

tctcgcgcgt ttcggtgatg acggtgaaaa cctctgacac atgcagctcc cggagacggt 9900

cacagcttgt ctgtaagcgg atgccgggag cagacaagcc cgtcagggcg cgtcagcggg 9960

tgttggcggg tgtcggggct ggcttaacta tgcggcatca gagcagattg tactgagagt 10020

gcaccatatg cggtgtgaaa taccgcacag atgcgtaagg agaaaatacc gcatcaggcg 10080

ccattcgcca ttcaggctgc gcaactgttg ggaagggcga tcggtgcggg cctcttcgct 10140

attacgccag ctggcgaaag ggggatgtgc tgcaaggcga ttaagttggg taacgccagg 10200

gttttcccag ttacgacgtt gtaaaacgac ggccagtgaa tt 10242

<210> 44
<211> 10245
<212> DNA
<213> Artificial

<220>
<223> Synthetic construct, D2E7 TEV polyprotein exprsesion vector.

<400> 44
gaagttccta ttccgaagtt cctattctct agacgttaca taacttacgg taaatggccc 60

gcctggctga ccgcccaacg acccccgccc attgacgtca ataatgacgt atgttcccat 120

agtaacgcca atagggactt tccattgacg tcaatgggtg gagtatttac ggtaaactgc 180

ccacttggca gtacatcaag tgtatcatat gccaagtacg ccccctattg acgtcaatga 240
cggtaaatgg cccgcctggc attatgccca gtacatgacc ttatgggact ttcctacttg 300
gcagtacatc tacgtattag tcatcgctat taccatggtg atgcggtttt ggcagtacat 360
caatgggcgt ggatagcggt ttgactcacg gggatttcca agtctccacc ccattgacgt 420
caatgggagt ttgttttggc accaaaatca acgggacttt ccaaaatgtc gtaacaactc 480
cgccccattg acgcaaatgg gcagggaatt cgagctcggt actcgagcgg tgttccgcgg 540
tcctcctcgt atagaaactc ggaccactct gagacgaagg ctcgcgtcca ggccagcacg 600
aaggaggcta agtgggaggg gtagcggtcg ttgtccacta gggggtccac tcgctccagg 660
gtgtgaagac acatgtcgcc ctcttcggca tcaaggaagg tgattggttt ataggtgtag 720
gccacgtgac cgggtgttcc tgaagggggg ctataaaagg gggtgggggc gcgttcgtcc 780
tcactctctt ccgcatcgct gtctgcgagg gccagctgtt gggctcgcgg ttgaggacaa 840
actcttcgcg gtctttccag tactcttgga tcggaaaccc gtcggcctcc gaacggtact 900
ccgccaccga gggacctgag cgagtccgca tcgaccggat cggaaaacct ctcgactgtt 960
ggggtgagta ctccctctca aaagcgggca tgacttctgc gctaagattg tcagtttcca 1020
aaaacgagga ggatttgata ttcacctggc ccgcggtgat gcctttgagg gtggccgcgt 1080
ccatctggtc agaaaagaca atctttttgt tgtcaagctt gaggtgtggc aggcttgaga 1140
tctggccata cacttgagtg acaatgacat ccactttgcc tttctctcca caggtgtcca 1200
ctcccaggtc caaccggaat tgtacccgcg gccagagctt gcccgggcgc caccatggag 1260
tttgggctga gctggctttt tcttgtcgcg attttaaaag gtgtccagtg tgaggtgcag 1320
ctggtggagt ctgggggagg cttggtacag cccggcaggt ccctgagact ctcctgtgcg 1380
gcctctggat tcacctttga tgattatgcc atgcactggg tccggcaagc tccagggaag 1440
ggcctggaat gggtctcagc tatcacttgg aatagtggtc acatagacta tgcggactct 1500
gtggagggcc gattcaccat ctccagagac aacgccaaga actccctgta tctgcaaatg 1560
aacagtctga gagctgagga tacggccgta tattactgtg cgaaagtctc gtaccttagc 1620
accgcgtcct cccttgacta ttggggccaa ggtaccctgg tcaccgtctc gagtgcgtcg 1680
accaagggcc catcggtctt ccccctggca ccctcctcca agagcacctc tgggggcaca 1740
gcggccctgg gctgcctggt caaggactac ttccccgaac cggtgacggt gtcgtggaac 1800
tcaggcgccc tgaccagcgg cgtgcacacc ttcccggctg tcctacagtc ctcaggactc 1860
tactccctca gcagcgtggt gaccgtgccc tccagcagct tgggcaccca gacctacatc 1920
tgcaacgtga atcacaagcc cagcaacacc aaggtggaca agaaagttga gcccaaatct 1980
tgtgacaaaa ctcacacatg cccaccgtgc ccagcacctg aactcctggg gggaccgtca 2040
gtcttcctct tccccccaaa acccaaggac accctcatga tctcccggac ccctgaggtc 2100
acatgcgtgg tggtggacgt gagccacgaa gaccctgagg tcaagttcaa ctggtacgtg 2160
gacggcgtgg aggtgcataa tgccaagaca aagccgcggg aggagcagta caacagcacg 2220

taccgtgtgg tcagcgtcct caccgtcctg caccaggact ggctgaatgg caaggagtac 2280
aagtgcaagg tctccaacaa agccctccca gcccccatcg agaaaaccat ctccaaagcc 2340
aaagggcagc cccgagaacc acaggtgtac accctgcccc catcccggga tgagctgacc 2400
aagaaccagg tcagcctgac ctgcctggtc aaaggcttct atcccagcga catcgccgtg 2460
gagtgggaga gcaatgggca gccggagaac aactacaaga ccacgcctcc cgtgctggac 2520
tccgacggct ccttcttcct ctacagcaag ctcaccgtgg acaagagcag gtggcagcag 2580
gggaacgtct tctcatgctc cgtgatgcat gaggctctgc acaaccacta cacgcagaag 2640
agcctctccc tgtctagggg taaacgcgaa ccagtttatt tccaggggag cttgtttaag 2700
gggccgcgtg attataaccc aatatcgagt gccatttgtc atctaacgaa tgaatctgat 2760
gggcacacaa catcgttgta tggtattggt tttggccctt tcatcatcac aaacaagcat 2820
ttgtttagaa gaaataatgg tacactgtta gttcaatcac tacatggtgt gttcaaggta 2880
aagaatacca caactttgca acaacacctc attgatggga gggacatgat gctcattcgc 2940
atgcctaagg atttcccacc atttcctcaa aagctgaaat tcagagagcc acaaagggaa 3000
gagcgcatat gtcttgtgac aaccaacttc caaactaaga gcatgtctag catggtttca 3060
gatactagtt gcacattccc ttcatctgat ggtatattct ggaaacattg gattcagacc 3120
aaggatgggc actgtggtag cccgttggtg tcaactagag atgggtttat tgttggtata 3180
cactcagcat caaatttcac caacacaaac aattatttta caagtgtgcc gaaagacttc 3240
atggatttat tgacaaatca agaggcgcag caatgggtta gtggttggcg attgaatgct 3300
gactcagtgt tatggggagg ccacaaagtt ttcatgagca aacctgaaga accctttcag 3360
ccagtcaaag aagcaactca actcatgagt gaattagtct actcgcaagg gatggacatg 3420
cgcgtgcccg cccagctgct gggcctgctg ctgctgtggt tccccggctc gcgatgcgac 3480
atccagatga cccagtctcc atcctccctg tctgcatctg taggggacag agtcaccatc 3540
acttgtcggg caagtcaggg catcagaaat tacttagcct ggtatcagca aaaaccaggg 3600
aaagccccta agctcctgat ctatgctgca tccactttgc aatcaggggt cccatctcgg 3660
ttcagtggca gtggatctgg gacagatttc actctcacca tcagcagcct acagcctgaa 3720
gatgttgcaa cttattactg tcaaaggtat aaccgtgcac cgtatacttt tggccagggg 3780
accaaggtgg aaatcaaacg tacggtggct gcaccatctg tcttcatctt cccgccatct 3840
gatgagcagt tgaaatctgg aactgcctct gttgtgtgcc tgctgaataa cttctatccc 3900
agagaggcca aagtacagtg gaaggtggat aacgccctcc aatcgggtaa ctcccaggag 3960
agtgtcacag agcaggacag caaggacagc acctacagcc tcagcagcac cctgacgctg 4020
agcaaagcag actacgagaa acacaaagtc tacgcctgcg aagtcaccca tcagggcctg 4080
agctcgcccg tcacaaagag cttcaacagg ggagagtgtt gagcggccgc gtttaaactg 4140
aatgagcgcg tccatccaga catgataaga tacattgatg agtttggaca aaccacaact 4200
agaatgcagt gaaaaaaatg ctttatttgt gaaatttgtg atgctattgc tttatttgta 4260

accattataa gctgcaataa acaagttaac aacaacaatt gcattcattt tatgtttcag 4320
gttcaggggg aggtgtggga ggttttttaa agcaagtaaa acctctacaa atgtggtatg 4380
gctgattatg atccggctgc ctcgcgcgtt tcggtgatga cggtgaaaac ctctgacaca 4440
tgcagctccc ggagacggtc acagcttgtc tgtaagcgga tgccgggagc agacaagccc 4500
gtcagggcgc gtcagcgggt gttggcgggt gtcggggcgc agccatgacc ggtcgacggc 4560
gcgccttttt ttttaatttt tattttattt tatttttgac gcgccgaagg cgcgatctga 4620
gctcggtaca gcttggctgt ggaatgtgtg tcagttaggg tgtggaaagt ccccaggctc 4680
cccagcaggc agaagtatgc aaagcatgca tctcaattag tcagcaacca ggtgtggaaa 4740
gtccccaggc tccccagcag gcagaagtat gcaaagcatg catctcaatt agtcagcaac 4800
catagtcccg cccctaactc cgcccatccc gcccctaact ccgcccagtt ccgcccattc 4860
tccgccccat ggctgactaa ttttttttat ttatgcagag gccgaggccg cctcggcctc 4920
tgagctattc cagaagtagt gaggaggctt ttttggaggc ctaggctttt gcaaaaagct 4980
cctcgaggaa ctgaaaaacc agaaagttaa ctggtaagtt tagtcttttt gtcttttatt 5040
tcaggtcccg gatccggtgg tggtgcaaat caaagaactg ctcctcagtg gatgttgcct 5100
ttacttctag gcctgtacgg aagtgttact tctgctctaa aagctgcgga attgtacccg 5160
cggcctaata cgactcacta tagggactag tatggttcga ccattgaact gcatcgtcgc 5220
cgtgtcccaa aatatgggga ttggcaagaa cggagaccta ccctggcctc cgctcaggaa 5280
cgagttcaag tacttccaaa gaatgaccac aacctcttca gtggaaggta aacagaatct 5340
ggtgattatg ggtaggaaaa cctggttctc cattcctgag aagaatcgac ctttaaagga 5400
cagaattaat atagttctca gtagagaact caaagaacca ccacgaggag ctcattttct 5460
tgccaaaagt ttagatgatg ccttaagact tattgaacaa ccggaattgg caagtaaagt 5520
agacatggtt tggatagtcg gaggcagttc tgtttaccag gaagccatga atcaaccagg 5580
ccacctcaga ctctttgtga caaggatcat gcaggaattt gaaagtgaca cgtttttccc 5640
agaaattgat ttggggaaat ataaacttct cccagaatac ccaggcgtcc tctctgaggt 5700
ccaggaggaa aaaggcatca agtataagtt tgaagtctac gagaagaaag actaagcggc 5760
cgagcgcgcg gatctggaaa cgggagatgg gggaggctaa ctgaagcacg gaaggagaca 5820
ataccggaag gaacccgcgc tatgacggca ataaaaagac agaataaaac gcacgggtgt 5880
tgggtcgttt gttcataaac gcggggttcg gtcccagggc tggcactctg tcgatacccc 5940
accgagaccc cattggggcc aatacgcccg cgtttcttcc ttttccccac cccacccccc 6000
aagttcgggt gaaggcccag ggctcgcagc caacgtcggg gcggcaggcc ctgccatagc 6060
cactggcccc gtgggttagg gacggggtcc cccatgggga atggtttatg gttcgtgggg 6120
gttattattt tgggcgttgc gtggggtctg gagatccccc gggctgcagg aattccgtta 6180
cattacttac ggtaaatggc ccgcctggct gaccgcccaa cgacccccgc ccattgacgt 6240
caataatgac gtatgttccc atagtaacgc caatagggac tttccattga cgtcaatggg 6300

tggagtattt acggtaaact gcccacttgg cagtacatca agtgtatcat atgccaagta 6360
cgccccctat tgacgtcaat gacggtaaat ggcccgcctg gcattatgcc cagtacatga 6420
ccttatggga ctttcctact tggcagtaca tctacgtatt agtcatcgct attaccatgg 6480
tgatgcggtt ttggcagtac atcaatgggc gtggatagcg gtttgactca cggggatttc 6540
caagtctcca ccccattgac gtcaatggga gtttgttttg gcaccaaaat caacgggact 6600
ttccaaaatg tcgtaacaac tccgccccat tgacgcaaaa gggcgggaat tcgagctcgg 6660
tactcgagcg gtgttccgcg gtcctcctcg tatagaaact cggaccactc tgagacgaag 6720
gctcgcgtcc aggccagcac gaaggaggct aagtgggagg ggtagcggtc gttgtccact 6780
agggggtcca ctcgctccag ggtgtgaaga cacatgtcgc cctcttcggc atcaaggaag 6840
gtgattggtt tataggtgta ggccacgtga ccgggtgttc ctgaaggggg gctataaaag 6900
ggggtggggg cgcgttcgtc ctcactctct tccgcatcgc tgtctgcgag ggccagctgt 6960
tgggctcgcg gttgaggaca aactcttcgc ggtctttcca gtactcttgg atcggaaacc 7020
cgtcggcctc cgaacggtac tccgccaccg agggacctga gcgagtccgc atcgaccgga 7080
tcggaaaacc tctcgactgt tggggtgagt actccctctc aaaagcgggc atgacttctg 7140
cgctaagatt gtcagtttcc aaaaacgagg aggatttgat attcacctgg cccgcggtga 7200
tgcctttgag ggtggccgcg tccatctggt cagaaaagac aatctttttg ttgtcaagct 7260
tgaggtgtgg caggcttgag atctggccat acacttgagt gacaatgaca tccactttgc 7320
ctttctctcc acaggtgtcc actcccaggt ccaaccggaa ttgtacccgc ggccagagct 7380
tgcgggcgcc accgcggccg cggggatcca gacatgataa gatacattga tgagtttgga 7440
caaaccacaa ctagaatgca gtgaaaaaaa tgctttattt gtgaaatttg tgatgctatt 7500
gctttatttg taaccattat aagctgcaat aaacaagtta acaacaacaa ttgcattcat 7560
tttatgtttc aggttcaggg ggaggtgtgg gaggtttttt cggatcctct tggcgtaatc 7620
atggtcatag ctgtttcctg tgtgaaattg ttatccgctc acaattccac acaacatacg 7680
agccggaagc ataaagtgta aagcctgggg tgcctaatga gtgagctaac tcacattaat 7740
tgcgttgcgc tcactgcccg ctttccagtc gggaaacctg tcgtgccagc tgcattaatg 7800
aatcggccaa cgcgcgggga aaggcggttt gcgtattggg cgctcttccg cttcctcgct 7860
cactgactcg ctgcgctcgg tcgttcggct gcggcgagcg gtatcagctc actcaaaggc 7920
ggtaatacgg ttatccacag aatcagggga taacgcagga aagaacatgt gagcaaaagg 7980
ccagcaaaag gccaggaacc gtaaaaaggc cgcgttgctg gcgtttttcc ataggctccg 8040
cccccctgac gagcatcaca aaaatcgacg ctcaagtcag aggtggcgaa acccgacagg 8100
actataaaga taccaggcgt ttccccctgg aagctccctc gtgcgctctc ctgttccgac 8160
cctgccgctt accggatacc tgtccgcctt tctcccttcg ggaagcgtgg cgctttctca 8220
tagctcacgc tgtaggtatc tcagttcggt gtaggtcgtt cgctccaagc tgggctgtgt 8280
gcacgaaccc cccgttcagc ccgaccgctg cgccttatcc ggtaactatc gtcttgagtc 8340

caacccggta agacacgact tatcgccact ggcagcagcc actggtaaca ggattagcag 8400
agcgaggtat gtaggcggtg ctacagagtt cttgaagtgg tggcctaact acggctacac 8460
tagaagaaca gtatttggta tctgcgctct gctgaagcca gttaccttcg gaaaaagagt 8520
tggtagctct tgatccggca aacaaaccac cgctggtagc ggtggttttt ttgtttgcaa 8580
gcagcagatt acgcgcagaa aaaaaggatc tcaagaagat cctttgatct tttctacggg 8640
gtctgacgct cagtggaacg aaaactcacg ttaagggatt ttggtcatga gattatcaaa 8700
aaggatcttc acctagatcc ttttaaatta aaaatgaagt tttaaatcaa tctaaagtat 8760
atatgagtaa acttggtctg acagttacca atgcttaatc agtgaggcac ctatctcagc 8820
gatctgtcta tttcgttcat ccatagttgc ctgactcccc gtcgtgtaga taactacgat 8880
acgggagggc ttaccatctg gccccagtgc tgcaatgata ccgcgagacc cacgctcacc 8940
ggctccagat ttatcagcaa taaaccagcc agccggaagg gccgagcgca gaagtggtcc 9000
tgcaacttta tccgcctcca tccagtctat taattgttgc cgggaagcta gagtaagtag 9060
ttcgccagtt aatagtttgc gcaacgttgt tgccattgct acaggcatcg tggtgtcacg 9120
ctcgtcgttt ggtatggctt cattcagctc cggttcccaa cgatcaaggc gagttacatg 9180
atcccccatg ttgtgcaaaa aagcggttag ctccttcggt cctccgatcg ttgtcagaag 9240
taagttggcc gcagtgttat cactcatggt tatggcagca ctgcataatt ctcttactgt 9300
catgccatcc gtaagatgct tttctgtgac tggtgagtac tcaaccaagt cattctgaga 9360
atagtgtatg cggcgaccga gttgctcttg cccggcgtca atacgggata ataccgcgcc 9420
acatagcaga actttaaaag tgctcatcat tggaaaacgt tcttcggggc gaaaactctc 9480
aaggatctta ccgctgttga gatccagttc gatgtaaccc actcgtgcac ccaactgatc 9540
ttcagcatct tttactttca ccagcgtttc tgggtgagca aaaacaggaa ggcaaaatgc 9600
cgcaaaaaag ggaataaggg cgacacggaa atgttgaata ctcatactct tcctttttca 9660
atattattga agcatttatc agggttattg tctcatgagc ggatacatat ttgaatgtat 9720
ttagaaaaat aaacaaatag gggttccgcg cacatttccc cgaaaagtgc cacctgacgt 9780
ctaagaaacc attattatca tgacattaac ctataaaaat aggcgtatca cgaggccctt 9840
tcgtctcgcg cgtttcggtg atgacggtga aaacctctga cacatgcagc tcccggagac 9900
ggtcacagct tgtctgtaag cggatgccgg gagcagacaa gcccgtcagg gcgcgtcagc 9960
gggtgttggc gggtgtcggg gctggcttaa ctatgcggca tcagagcaga ttgtactgag 10020
agtgcaccat atgcggtgtg aaataccgca cagatgcgta aggagaaaat accgcatcag 10080
gcgccattcg ccattcaggc tgcgcaactg ttgggaaggg cgatcggtgc gggcctcttc 10140
gctattacgc cagctggcga aagggggatg tgctgcaagg cgattaagtt gggtaacgcc 10200
agggttttcc cagtcacgac gttgtaaaac gacggccagt gaatt 10245

<210> 45
<211> 2196

<212>  DNA
<213>  Artificial

<220>
<223>  Synthetic construct, sequence encodign D2E7 internal cleavabe
       signal peptide construct.


<220>
<221>  CDS
<222>  (1)..(2193)

<400>  45
atg gag ttt ggg ctg agc tgg ctt ttt ctt gtc gcg att tta aaa ggt      48
Met Glu Phe Gly Leu Ser Trp Leu Phe Leu Val Ala Ile Leu Lys Gly
1               5                   10                  15

gtc cag tgt gag gtg cag ctg gtg gag tct ggg gga ggc ttg gta cag      96
Val Gln Cys Glu Val Gln Leu Val Glu Ser Gly Gly Gly Leu Val Gln
            20                  25                  30

ccc ggc agg tcc ctg aga ctc tcc tgt gcg gcc tct gga ttc acc ttt      144
Pro Gly Arg Ser Leu Arg Leu Ser Cys Ala Ala Ser Gly Phe Thr Phe
        35                  40                  45

gat gat tat gcc atg cac tgg gtc cgg caa gct cca ggg aag ggc ctg      192
Asp Asp Tyr Ala Met His Trp Val Arg Gln Ala Pro Gly Lys Gly Leu
    50                  55                  60

gaa tgg gtc tca gct atc act tgg aat agt ggt cac ata gac tat gcg      240
Glu Trp Val Ser Ala Ile Thr Trp Asn Ser Gly His Ile Asp Tyr Ala
65                  70                  75                  80

gac tct gtg gag ggc cga ttc acc atc tcc aga gac aac gcc aag aac      288
Asp Ser Val Glu Gly Arg Phe Thr Ile Ser Arg Asp Asn Ala Lys Asn
                85                  90                  95

tcc ctg tat ctg caa atg aac agt ctg aga gct gag gat acg gcc gta      336
Ser Leu Tyr Leu Gln Met Asn Ser Leu Arg Ala Glu Asp Thr Ala Val
            100                 105                 110

tat tac tgt gcg aaa gtc tcg tac ctt agc acc gcg tcc tcc ctt gac      384
Tyr Tyr Cys Ala Lys Val Ser Tyr Leu Ser Thr Ala Ser Ser Leu Asp
        115                 120                 125

tat tgg ggc caa ggt acc ctg gtc acc gtc tcg agt gcg tcg acc aag      432
Tyr Trp Gly Gln Gly Thr Leu Val Thr Val Ser Ser Ala Ser Thr Lys
    130                 135                 140

ggc cca tcg gtc ttc ccc ctg gca ccc tcc tcc aag agc acc tct ggg      480
Gly Pro Ser Val Phe Pro Leu Ala Pro Ser Ser Lys Ser Thr Ser Gly
145                 150                 155                 160

ggc aca gcg gcc ctg ggc tgc ctg gtc aag gac tac ttc ccc gaa ccg      528
Gly Thr Ala Ala Leu Gly Cys Leu Val Lys Asp Tyr Phe Pro Glu Pro
                165                 170                 175

gtg acg gtg tcg tgg aac tca ggc gcc ctg acc agc ggc gtg cac acc      576
Val Thr Val Ser Trp Asn Ser Gly Ala Leu Thr Ser Gly Val His Thr
            180                 185                 190

ttc ccg gct gtc cta cag tcc tca gga ctc tac tcc ctc agc agc gtg      624
Phe Pro Ala Val Leu Gln Ser Ser Gly Leu Tyr Ser Leu Ser Ser Val
        195                 200                 205

gtg acc gtg ccc tcc agc agc ttg ggc acc cag acc tac atc tgc aac      672
Val Thr Val Pro Ser Ser Ser Leu Gly Thr Gln Thr Tyr Ile Cys Asn
    210                 215                 220

gtg aat cac aag ccc agc aac acc aag gtg gac aag aaa gtt gag ccc 720
Val Asn His Lys Pro Ser Asn Thr Lys Val Asp Lys Lys Val Glu Pro
225                 230                 235                 240

aaa tct tgt gac aaa act cac aca tgc cca ccg tgc cca gca cct gaa 768
Lys Ser Cys Asp Lys Thr His Thr Cys Pro Pro Cys Pro Ala Pro Glu
                245                 250                 255

ctc ctg ggg gga ccg tca gtc ttc ctc ttc ccc cca aaa ccc aag gac 816
Leu Leu Gly Gly Pro Ser Val Phe Leu Phe Pro Pro Lys Pro Lys Asp
            260                 265                 270

acc ctc atg atc tcc cgg acc cct gag gtc aca tgc gtg gtg gtg gac 864
Thr Leu Met Ile Ser Arg Thr Pro Glu Val Thr Cys Val Val Val Asp
        275                 280                 285

gtg agc cac gaa gac cct gag gtc aag ttc aac tgg tac gtg gac ggc 912
Val Ser His Glu Asp Pro Glu Val Lys Phe Asn Trp Tyr Val Asp Gly
    290                 295                 300

gtg gag gtg cat aat gcc aag aca aag ccg cgg gag gag cag tac aac 960
Val Glu Val His Asn Ala Lys Thr Lys Pro Arg Glu Glu Gln Tyr Asn
305                 310                 315                 320

agc acg tac cgt gtg gtc agc gtc ctc acc gtc ctg cac cag gac tgg 1008
Ser Thr Tyr Arg Val Val Ser Val Leu Thr Val Leu His Gln Asp Trp
                325                 330                 335

ctg aat ggc aag gag tac aag tgc aag gtc tcc aac aaa gcc ctc cca 1056
Leu Asn Gly Lys Glu Tyr Lys Cys Lys Val Ser Asn Lys Ala Leu Pro
            340                 345                 350

gcc ccc atc gag aaa acc atc tcc aaa gcc aaa ggg cag ccc cga gaa 1104
Ala Pro Ile Glu Lys Thr Ile Ser Lys Ala Lys Gly Gln Pro Arg Glu
        355                 360                 365

cca cag gtg tac acc ctg ccc cca tcc cgg gat gag ctg acc aag aac 1152
Pro Gln Val Tyr Thr Leu Pro Pro Ser Arg Asp Glu Leu Thr Lys Asn
    370                 375                 380

cag gtc agc ctg acc tgc ctg gtc aaa ggc ttc tat ccc agc gac atc 1200
Gln Val Ser Leu Thr Cys Leu Val Lys Gly Phe Tyr Pro Ser Asp Ile
385                 390                 395                 400

gcc gtg gag tgg gag agc aat ggg cag ccg gag aac aac tac aag acc 1248
Ala Val Glu Trp Glu Ser Asn Gly Gln Pro Glu Asn Asn Tyr Lys Thr
                405                 410                 415

acg cct ccc gtg ctg gac tcc gac ggc tcc ttc ttc ctc tac agc aag 1296
Thr Pro Pro Val Leu Asp Ser Asp Gly Ser Phe Phe Leu Tyr Ser Lys
            420                 425                 430

ctc acc gtg gac aag agc agg tgg cag cag ggg aac gtc ttc tca tgc 1344
Leu Thr Val Asp Lys Ser Arg Trp Gln Gln Gly Asn Val Phe Ser Cys
        435                 440                 445

tcc gtg atg cat gag gct ctg cac aac cac tac acg cag aag agc ctc 1392
Ser Val Met His Glu Ala Leu His Asn His Tyr Thr Gln Lys Ser Leu
    450                 455                 460

tcc ctg tct agg ggt aaa cgc atg gga cga atg gca atg aaa tgg tta 1440
Ser Leu Ser Arg Gly Lys Arg Met Gly Arg Met Ala Met Lys Trp Leu
465                 470                 475                 480

gtt gtt ata ata tgt ttc tct ata aca agt caa cct gct tct gct atg 1488
Val Val Ile Ile Cys Phe Ser Ile Thr Ser Gln Pro Ala Ser Ala Met
                485                 490                 495

gac atg cgc gtg ccc gcc cag ctg ctg ggc ctg ctg ctg ctg tgg ttc 1536
Asp Met Arg Val Pro Ala Gln Leu Leu Gly Leu Leu Leu Leu Trp Phe
            500                 505                 510

ccc ggc tcg cga tgc gac atc cag atg acc cag tct cca tcc tcc ctg 1584
Pro Gly Ser Arg Cys Asp Ile Gln Met Thr Gln Ser Pro Ser Ser Leu
        515                 520                 525

tct gca tct gta ggg gac aga gtc acc atc act tgt cgg gca agt cag 1632
Ser Ala Ser Val Gly Asp Arg Val Thr Ile Thr Cys Arg Ala Ser Gln
    530                 535                 540

ggc atc aga aat tac tta gcc tgg tat cag caa aaa cca ggg aaa gcc 1680
Gly Ile Arg Asn Tyr Leu Ala Trp Tyr Gln Gln Lys Pro Gly Lys Ala
545                 550                 555                 560

cct aag ctc ctg atc tat gct gca tcc act ttg caa tca ggg gtc cca 1728
Pro Lys Leu Leu Ile Tyr Ala Ala Ser Thr Leu Gln Ser Gly Val Pro
                565                 570                 575

tct cgg ttc agt ggc agt gga tct ggg aca gat ttc act ctc acc atc 1776
Ser Arg Phe Ser Gly Ser Gly Ser Gly Thr Asp Phe Thr Leu Thr Ile
            580                 585                 590

agc agc cta cag cct gaa gat gtt gca act tat tac tgt caa agg tat 1824
Ser Ser Leu Gln Pro Glu Asp Val Ala Thr Tyr Tyr Cys Gln Arg Tyr
        595                 600                 605

aac cgt gca ccg tat act ttt ggc cag ggg acc aag gtg gaa atc aaa 1872
Asn Arg Ala Pro Tyr Thr Phe Gly Gln Gly Thr Lys Val Glu Ile Lys
    610                 615                 620

cgt acg gtg gct gca cca tct gtc ttc atc ttc ccg cca tct gat gag 1920
Arg Thr Val Ala Ala Pro Ser Val Phe Ile Phe Pro Pro Ser Asp Glu
625                 630                 635                 640

cag ttg aaa tct gga act gcc tct gtt gtg tgc ctg ctg aat aac ttc 1968
Gln Leu Lys Ser Gly Thr Ala Ser Val Val Cys Leu Leu Asn Asn Phe
                645                 650                 655

tat ccc aga gag gcc aaa gta cag tgg aag gtg gat aac gcc ctc caa 2016
Tyr Pro Arg Glu Ala Lys Val Gln Trp Lys Val Asp Asn Ala Leu Gln
            660                 665                 670

tcg ggt aac tcc cag gag agt gtc aca gag cag gac agc aag gac agc 2064
Ser Gly Asn Ser Gln Glu Ser Val Thr Glu Gln Asp Ser Lys Asp Ser
        675                 680                 685

acc tac agc ctc agc agc acc ctg acg ctg agc aaa gca gac tac gag 2112
Thr Tyr Ser Leu Ser Ser Thr Leu Thr Leu Ser Lys Ala Asp Tyr Glu
    690                 695                 700

aaa cac aaa gtc tac gcc tgc gaa gtc acc cat cag ggc ctg agc tcg 2160
Lys His Lys Val Tyr Ala Cys Glu Val Thr His Gln Gly Leu Ser Ser
705                 710                 715                 720

ccc gtc aca aag agc ttc aac agg gga gag tgt tga 2196
Pro Val Thr Lys Ser Phe Asn Arg Gly Glu Cys
                725                 730

<210> 46
<211> 731
<212> PRT
<213> Artificial

<220>

<223>  Synthetic Construct

<400>  46

Met Glu Phe Gly Leu Ser Trp Leu Phe Leu Val Ala Ile Leu Lys Gly
1               5                   10                  15

Val Gln Cys Glu Val Gln Leu Val Glu Ser Gly Gly Gly Leu Val Gln
            20                  25                  30

Pro Gly Arg Ser Leu Arg Leu Ser Cys Ala Ala Ser Gly Phe Thr Phe
        35                  40                  45

Asp Asp Tyr Ala Met His Trp Val Arg Gln Ala Pro Gly Lys Gly Leu
    50                  55                  60

Glu Trp Val Ser Ala Ile Thr Trp Asn Ser Gly His Ile Asp Tyr Ala
65                  70                  75                  80

Asp Ser Val Glu Gly Arg Phe Thr Ile Ser Arg Asp Asn Ala Lys Asn
                85                  90                  95

Ser Leu Tyr Leu Gln Met Asn Ser Leu Arg Ala Glu Asp Thr Ala Val
            100                 105                 110

Tyr Tyr Cys Ala Lys Val Ser Tyr Leu Ser Thr Ala Ser Ser Leu Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Asp Glu Leu Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Arg Gly Lys Arg Met Gly Arg Met Ala Met Lys Trp Leu
465                 470                 475                 480

Val Val Ile Ile Cys Phe Ser Ile Thr Ser Gln Pro Ala Ser Ala Met
                485                 490                 495

Asp Met Arg Val Pro Ala Gln Leu Leu Gly Leu Leu Leu Leu Trp Phe
            500                 505                 510

Pro Gly Ser Arg Cys Asp Ile Gln Met Thr Gln Ser Pro Ser Ser Leu
        515                 520                 525

Ser Ala Ser Val Gly Asp Arg Val Thr Ile Thr Cys Arg Ala Ser Gln
    530                 535                 540

Gly Ile Arg Asn Tyr Leu Ala Trp Tyr Gln Gln Lys Pro Gly Lys Ala
545                 550                 555                 560

Pro Lys Leu Leu Ile Tyr Ala Ala Ser Thr Leu Gln Ser Gly Val Pro
                565                 570                 575

Ser Arg Phe Ser Gly Ser Gly Ser Gly Thr Asp Phe Thr Leu Thr Ile
            580                 585                 590

Ser Ser Leu Gln Pro Glu Asp Val Ala Thr Tyr Tyr Cys Gln Arg Tyr
        595                 600                 605

Asn Arg Ala Pro Tyr Thr Phe Gly Gln Gly Thr Lys Val Glu Ile Lys
    610                 615                 620

Arg Thr Val Ala Ala Pro Ser Val Phe Ile Phe Pro Pro Ser Asp Glu
625                 630                 635                 640

Gln Leu Lys Ser Gly Thr Ala Ser Val Val Cys Leu Leu Asn Asn Phe
                645                 650                 655

Tyr Pro Arg Glu Ala Lys Val Gln Trp Lys Val Asp Asn Ala Leu Gln
            660                 665                 670

Ser Gly Asn Ser Gln Glu Ser Val Thr Glu Gln Asp Ser Lys Asp Ser
        675                 680                 685

Thr Tyr Ser Leu Ser Ser Thr Leu Thr Leu Ser Lys Ala Asp Tyr Glu
    690                 695                 700

Lys His Lys Val Tyr Ala Cys Glu Val Thr His Gln Gly Leu Ser Ser
705                 710                 715                 720

Pro Val Thr Lys Ser Phe Asn Arg Gly Glu Cys
                725                 730

<210>  47
<211>  9573
<212>  DNA
<213>  Artificial

<220>
<223>  Synthetic construct, D2E7 internal cleavable signal peptide
       polyprotein expression vector.

<400>  47
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga 240
cggtaaatgg cccgcctggc attatgccca gtacatgacc ttatgggact ttcctacttg 300
gcagtacatc tacgtattag tcatcgctat taccatggtg atgcggtttt ggcagtacat 360
caatgggcgt ggatagcggt ttgactcacg gggatttcca agtctccacc ccattgacgt 420
caatgggagt ttgttttggc accaaaatca acgggacttt ccaaaatgtc gtaacaactc 480
cgccccattg acgcaaatgg gcagggaatt cgagctcggt actcgagcgg tgttccgcgg 540
tcctcctcgt atagaaactc ggaccactct gagacgaagg ctcgcgtcca ggccagcacg 600
aaggaggcta agtgggaggg gtagcggtcg ttgtccacta gggggtccac tcgctccagg 660
gtgtgaagac acatgtcgcc ctcttcggca tcaaggaagg tgattggttt ataggtgtag 720
gccacgtgac cgggtgttcc tgaagggggg ctataaaagg gggtgggggc gcgttcgtcc 780
tcactctctt ccgcatcgct gtctgcgagg gccagctgtt gggctcgcgg ttgaggacaa 840
actcttcgcg gtctttccag tactcttgga tcggaaaccc gtcggcctcc gaacggtact 900
ccgccaccga gggacctgag cgagtccgca tcgaccggat cggaaaacct ctcgactgtt 960
ggggtgagta ctccctctca aaagcgggca tgacttctgc gctaagattg tcagtttcca 1020
aaaacgagga ggatttgata ttcacctggc ccgcggtgat gcctttgagg gtggccgcgt 1080
ccatctggtc agaaaagaca atctttttgt tgtcaagctt gaggtgtggc aggcttgaga 1140
tctggccata cacttgagtg acaatgacat ccactttgcc tttctctcca caggtgtcca 1200
ctcccaggtc caaccggaat tgtacccgcg gccagagctt gcccgggcgc caccatggag 1260
tttgggctga gctggctttt tcttgtcgcg attttaaaag gtgtccagtg tgaggtgcag 1320
ctggtggagt ctgggggagg cttggtacag cccggcaggt ccctgagact ctcctgtgcg 1380
gcctctggat tcacctttga tgattatgcc atgcactggg tccggcaagc tccagggaag 1440
ggcctggaat gggtctcagc tatcacttgg aatagtggtc acatagacta tgcggactct 1500
gtggagggcc gattcaccat ctccagagac aacgccaaga actccctgta tctgcaaatg 1560
aacagtctga gagctgagga tacggccgta tattactgtg cgaaagtctc gtaccttagc 1620
accgcgtcct cccttgacta ttggggccaa ggtaccctgg tcaccgtctc gagtgcgtcg 1680
accaagggcc catcggtctt ccccctggca ccctcctcca agagcacctc tgggggcaca 1740
gcggccctgg gctgcctggt caaggactac ttccccgaac cggtgacggt gtcgtggaac 1800
tcaggcgccc tgaccagcgg cgtgcacacc ttcccggctg tcctacagtc ctcaggactc 1860
tactccctca gcagcgtggt gaccgtgccc tccagcagct tgggcaccca gacctacatc 1920
tgcaacgtga atcacaagcc cagcaacacc aaggtggaca agaaagttga gcccaaatct 1980
tgtgacaaaa ctcacacatg cccaccgtgc ccagcacctg aactcctggg gggaccgtca 2040
gtcttcctct tccccccaaa acccaaggac accctcatga tctcccggac ccctgaggtc 2100
acatgcgtgg tggtggacgt gagccacgaa gaccctgagg tcaagttcaa ctggtacgtg 2160
gacggcgtgg aggtgcataa tgccaagaca aagccgcggg aggagcagta caacagcacg 2220

taccgtgtgg tcagcgtcct caccgtcctg caccaggact ggctgaatgg caaggagtac 2280
aagtgcaagg tctccaacaa agccctccca gcccccatcg agaaaaccat ctccaaagcc 2340
aaagggcagc cccgagaacc acaggtgtac accctgcccc catcccggga tgagctgacc 2400
aagaaccagg tcagcctgac ctgcctggtc aaaggcttct atcccagcga catcgccgtg 2460
gagtgggaga gcaatgggca gccggagaac aactacaaga ccacgcctcc cgtgctggac 2520
tccgacggct ccttcttcct ctacagcaag ctcaccgtgg acaagagcag gtggcagcag 2580
gggaacgtct tctcatgctc cgtgatgcat gaggctctgc acaaccacta cacgcagaag 2640
agcctctccc tgtctagggg taaacgcatg ggacgaatgg caatgaaatg gttagttgtt 2700
ataatatgtt tctctataac aagtcaacct gcttctgcta tggacatgcg cgtgcccgcc 2760
cagctgctgg gcctgctgct gctgtggttc cccggctcgc gatgcgacat ccagatgacc 2820
cagtctccat cctccctgtc tgcatctgta ggggacagag tcaccatcac ttgtcgggca 2880
agtcagggca tcagaaatta cttagcctgg tatcagcaaa aaccagggaa agcccctaag 2940
ctcctgatct atgctgcatc cactttgcaa tcaggggtcc catctcggtt cagtggcagt 3000
ggatctggga cagatttcac tctcaccatc agcagcctac agcctgaaga tgttgcaact 3060
tattactgtc aaaggtataa ccgtgcaccg tatacttttg gccaggggac caaggtggaa 3120
atcaaacgta cggtggctgc accatctgtc ttcatcttcc cgccatctga tgagcagttg 3180
aaatctggaa ctgcctctgt tgtgtgcctg ctgaataact tctatcccag agaggccaaa 3240
gtacagtgga aggtggataa cgccctccaa tcgggtaact cccaggagag tgtcacagag 3300
caggacagca aggacagcac ctacagcctc agcagcaccc tgacgctgag caaagcagac 3360
tacgagaaac acaaagtcta cgcctgcgaa gtcacccatc agggcctgag ctcgcccgtc 3420
acaaagagct tcaacagggg agagtgttga gcggccgcgt ttaaactgaa tgagcgcgtc 3480
catccagaca tgataagata cattgatgag tttggacaaa ccacaactag aatgcagtga 3540
aaaaaatgct ttatttgtga aatttgtgat gctattgctt tatttgtaac cattataagc 3600
tgcaataaac aagttaacaa caacaattgc attcatttta tgtttcaggt tcagggggag 3660
gtgtgggagg ttttttaaag caagtaaaac ctctacaaat gtggtatggc tgattatgat 3720
ccggctgcct cgcgcgtttc ggtgatgacg gtgaaaacct ctgacacatg cagctcccgg 3780
agacggtcac agcttgtctg taagcggatg ccgggagcag acaagcccgt cagggcgcgt 3840
cagcgggtgt tggcgggtgt cggggcgcag ccatgaccgg tcgacggcgc gccttttttt 3900
ttaattttta ttttatttta tttttgacgc gccgaaggcg cgatctgagc tcggtacagc 3960
ttggctgtgg aatgtgtgtc agttagggtg tggaaagtcc ccaggctccc cagcaggcag 4020
aagtatgcaa agcatgcatc tcaattagtc agcaaccagg tgtggaaagt ccccaggctc 4080
cccagcaggc agaagtatgc aaagcatgca tctcaattag tcagcaacca tagtcccgcc 4140
cctaactccg cccatcccgc ccctaactcc gcccagttcc gcccattctc cgccccatgg 4200
ctgactaatt ttttttattt atgcagaggc cgaggccgcc tcggcctctg agctattcca 4260

gaagtagtga ggaggctttt ttggaggcct aggcttttgc aaaaagctcc tcgaggaact 4320
gaaaaaccag aaagttaact ggtaagttta gtctttttgt cttttatttc aggtcccgga 4380
tccggtggtg gtgcaaatca aagaactgct cctcagtgga tgttgccttt acttctaggc 4440
ctgtacggaa gtgttacttc tgctctaaaa gctgcggaat tgtacccgcg gcctaatacg 4500
actcactata gggactagta tggttcgacc attgaactgc atcgtcgccg tgtcccaaaa 4560
tatggggatt ggcaagaacg gagacctacc ctggcctccg ctcaggaacg agttcaagta 4620
cttccaaaga atgaccacaa cctcttcagt ggaaggtaaa cagaatctgg tgattatggg 4680
taggaaaacc tggttctcca ttcctgagaa gaatcgacct ttaaaggaca gaattaatat 4740
agttctcagt agagaactca aagaaccacc acgaggagct cattttcttg ccaaaagttt 4800
agatgatgcc ttaagactta ttgaacaacc ggaattggca agtaaagtag acatggtttg 4860
gatagtcgga ggcagttctg tttaccagga agccatgaat caaccaggcc acctcagact 4920
ctttgtgaca aggatcatgc aggaatttga aagtgacacg tttttcccag aaattgattt 4980
ggggaaatat aaacttctcc cagaataccc aggcgtcctc tctgaggtcc aggaggaaaa 5040
aggcatcaag tataagtttg aagtctacga gaagaaagac taagcggccg agcgcggcga 5100
tctggaaacg ggagatgggg gaggctaact gaagcacgga aggagacaat accggaagga 5160
acccgcgcta tgacggcaat aaaaagacag aataaaacgc acgggtgttg ggtcgtttgt 5220
tcataaacgc ggggttcggt cccagggctg gcactctgtc gataccccac cgagacccca 5280
ttggggccaa tacgcccgcg tttcttcctt ttccccaccc caccccccaa gttcgggtga 5340
aggcccaggg ctcgcagcca acgtcggggc ggcaggccct gccatagcca ctggccccgt 5400
gggttaggga cggggtcccc catggggaat ggtttatggt tcgtgggggt tattattttg 5460
ggcgttgcgt ggggtctgga gatcccccgg gctgcaggaa ttccgttaca ttacttacgg 5520
taaatggccc gcctggctga ccgcccaacg acccccgccc attgacgtca ataatgacgt 5580
atgttcccat agtaacgcca atagggactt tccattgacg tcaatgggtg gagtatttac 5640
ggtaaactgc ccacttggca gtacatcaag tgtatcatat gccaagtacg ccccctattg 5700
acgtcaatga cggtaaatgg cccgcctggc attatgccca gtacatgacc ttatgggact 5760
ttcctacttg gcagtacatc tacgtattag tcatcgctat taccatggtg atgcggtttt 5820
ggcagtacat caatgggcgt ggatagcggt ttgactcacg gggatttcca agtctccacc 5880
ccattgacgt caatgggagt ttgttttggc accaaaatca acgggacttt ccaaaatgtc 5940
gtaacaactc cgccccattg acgcaaaagg gcgggaattc gagctcggta ctcgagcggt 6000
gttccgcggt cctcctcgta tagaaactcg gaccactctg agacgaaggc tcgcgtccag 6060
gccagcacga aggaggctaa gtgggagggg tagcggtcgt tgtccactag ggggtccact 6120
cgctccaggg tgtgaagaca catgtcgccc tcttcggcat caaggaaggt gattggttta 6180
taggtgtagg ccacgtgacc gggtgttcct gaaggggggc tataaaaggg ggtgggggcg 6240
cgttcgtcct cactctcttc cgcatcgctg tctgcgaggg ccagctgttg ggctcgcggt 6300

tgaggacaaa ctcttcgcgg tctttccagt actcttggat cggaaacccg tcggcctccg 6360
aacggtactc cgccaccgag ggacctgagc gagtccgcat cgaccggatc ggaaaacctc 6420
tcgactgttg gggtgagtac tccctctcaa aagcgggcat gacttctgcg ctaagattgt 6480
cagtttccaa aaacgaggag gatttgatat tcacctggcc cgcggtgatg cctttgaggg 6540
tggccgcgtc catctggtca gaaaagacaa tctttttgtt gtcaagcttg aggtgtggca 6600
ggcttgagat ctggccatac acttgagtga caatgacatc cactttgcct ttctctccac 6660
aggtgtccac tcccaggtcc aaccggaatt gtacccgcgg ccagagcttg cgggcgccac 6720
cgcggccgcg gggatccaga catgataaga tacattgatg agtttggaca aaccacaact 6780
agaatgcagt gaaaaaaatg ctttatttgt gaaatttgtg atgctattgc tttatttgta 6840
accattataa gctgcaataa acaagttaac aacaacaatt gcattcattt tatgtttcag 6900
gttcaggggg aggtgtggga ggttttttcg gatcctcttg gcgtaatcat ggtcatagct 6960
gtttcctgtg tgaaattgtt atccgctcac aattccacac aacatacgag ccggaagcat 7020
aaagtgtaaa gcctggggtg cctaatgagt gagctaactc acattaattg cgttgcgctc 7080
actgcccgct ttccagtcgg gaaacctgtc gtgccagctg cattaatgaa tcggccaacg 7140
cgcggggaaa ggcggtttgc gtattgggcg ctcttccgct tcctcgctca ctgactcgct 7200
gcgctcggtc gttcggctgc ggcgagcggt atcagctcac tcaaaggcgg taatacggtt 7260
atccacagaa tcaggggata acgcaggaaa gaacatgtga gcaaaaggcc agcaaaaggc 7320
caggaaccgt aaaaaggccg cgttgctggc gtttttccat aggctccgcc cccctgacga 7380
gcatcacaaa aatcgacgct caagtcagag gtggcgaaac ccgacaggac tataaagata 7440
ccaggcgttt ccccctggaa gctccctcgt gcgctctcct gttccgaccc tgccgcttac 7500
cggatacctg tccgcctttc tcccttcggg aagcgtggcg ctttctcata gctcacgctg 7560
taggtatctc agttcggtgt aggtcgttcg ctccaagctg ggctgtgtgc acgaaccccc 7620
cgttcagccc gaccgctgcg ccttatccgg taactatcgt cttgagtcca acccggtaag 7680
acacgactta tcgccactgg cagcagccac tggtaacagg attagcagag cgaggtatgt 7740
aggcggtgct acagagttct tgaagtggtg gcctaactac ggctacacta gaagaacagt 7800
atttggtatc tgcgctctgc tgaagccagt taccttcgga aaaagagttg gtagctcttg 7860
atccggcaaa caaaccaccg ctggtagcgg tggttttttt gtttgcaagc agcagattac 7920
gcgcagaaaa aaaggatctc aagaagatcc tttgatcttt tctacggggt ctgacgctca 7980
gtggaacgaa aactcacgtt aagggatttt ggtcatgaga ttatcaaaaa ggatcttcac 8040
ctagatccct tttaattaaa aatgaagttt taaatcaatc taaagtatat atgagtaaac 8100
ttggtctgac agttaccaat gcttaatcag tgaggcacct atctcagcga tctgtctatt 8160
tcgttcatcc atagttgcct gactccccgt cgtgtagata actacgatac gggagggctt 8220
accatctggc cccagtgctg caatgatacc gcgagaccca cgctcaccgg ctccagattt 8280
atcagcaata aaccagccag ccggaagggc cgagcgcaga agtggtcctg caactttatc 8340

cgcctccatc cagtctatta attgttgccg ggaagctaga gtaagtagtt cgccagttaa 8400

tagtttgcgc aacgttgttg ccattgctac aggcatcgtg gtgtcacgct cgtcgtttgg 8460

tatggcttca ttcagctccg gttcccaacg atcaaggcga gttacatgat cccccatgtt 8520

gtgcaaaaaa gcggttagct ccttcggtcc tccgatcgtt gtcagaagta agttggccgc 8580

agtgttatca ctcatggtta tggcagcact gcataattct cttactgtca tgccatccgt 8640

aagatgcttt tctgtgactg gtgagtactc aaccaagtca ttctgagaat agtgtatgcg 8700

gcgaccgagt tgctcttgcc cggcgtcaat acgggataat accgcgccac atagcagaac 8760

tttaaaagtg ctcatcattg gaaaacgttc ttcggggcga aaactctcaa ggatcttacc 8820

gctgttgaga tccagttcga tgtaacccac tcgtgcaccc aactgatctt cagcatcttt 8880

tactttcacc agcgtttctg ggtgagcaaa aacaggaagg caaaatgccg caaaaaaggg 8940

aataagggcg acacggaaat gttgaatact catactcttc ctttttcaat attattgaag 9000

catttatcag ggttattgtc tcatgagcgg atacatattt gaatgtattt agaaaaataa 9060

acaaataggg gttccgcgca catttccccg aaaagtgcca cctgacgtct aagaaaccat 9120

tattatcatg acattaacct ataaaaatag gcgtatcacg aggccctttc gtctcgcgcg 9180

tttcggtgat gacggtgaaa acctctgaca catgcagctc ccggagacgg tcacagcttg 9240

tctgtaagcg gatgccggga gcagacaagc ccgtcagggc gcgtcagcgg gtgttggcgg 9300

gtgtcggggc tggcttaact atgcggcatc agagcagatt gtactgagag tgcaccatat 9360

gcggtgtgaa ataccgcaca gatgcgtaag gagaaaatac cgcatcaggc gccattcgcc 9420

attcaggctg cgcaactgtt gggaagggcg atcggtgcgg gcctcttcgc tattacgcca 9480

gctggcgaaa gggggatgtg ctgcaaggcg attaagttgg gtaacgccag ggttttccca 9540

gttacgacgt tgtaaaacga cggccagtga att 9573

<210> 48
<211> 3252
<212> DNA
<213> Artificial

<220>
<223> Synthetic construct, D2E7 intein fusion polyprotein coding
sequence.

<220>
<221> CDS
<222> (1)..(3249)

<400> 48
atg gag ttt ggg ctg agc tgg ctt ttt ctt gtc gcg att tta aaa ggt 48
Met Glu Phe Gly Leu Ser Trp Leu Phe Leu Val Ala Ile Leu Lys Gly
1               5                   10                  15

gtc cag tgt gag gtg cag ctg gtg gag tct ggg gga ggc ttg gta cag 96
Val Gln Cys Glu Val Gln Leu Val Glu Ser Gly Gly Gly Leu Val Gln
            20                  25                  30

ccc ggc agg tcc ctg aga ctc tcc tgt gcg gcc tct gga ttc acc ttt 144
Pro Gly Arg Ser Leu Arg Leu Ser Cys Ala Ala Ser Gly Phe Thr Phe

35                  40                  45

gat gat tat gcc atg cac tgg gtc cgg caa gct cca ggg aag ggc ctg     192
Asp Asp Tyr Ala Met His Trp Val Arg Gln Ala Pro Gly Lys Gly Leu
    50                  55                  60

gaa tgg gtc tca gct atc act tgg aat agt ggt cac ata gac tat gcg     240
Glu Trp Val Ser Ala Ile Thr Trp Asn Ser Gly His Ile Asp Tyr Ala
65                  70                  75                  80

gac tct gtg gag ggc cga ttc acc atc tcc aga gac aac gcc aag aac     288
Asp Ser Val Glu Gly Arg Phe Thr Ile Ser Arg Asp Asn Ala Lys Asn
                85                  90                  95

tcc ctg tat ctg caa atg aac agt ctg aga gct gag gat acg gcc gta     336
Ser Leu Tyr Leu Gln Met Asn Ser Leu Arg Ala Glu Asp Thr Ala Val
            100                 105                 110

tat tac tgt gcg aaa gtc tcg tac ctt agc acc gcg tcc tcc ctt gac     384
Tyr Tyr Cys Ala Lys Val Ser Tyr Leu Ser Thr Ala Ser Ser Leu Asp
        115                 120                 125

tat tgg ggc caa ggt acc ctg gtc acc gtc tcg agt gcg tcg acc aag     432
Tyr Trp Gly Gln Gly Thr Leu Val Thr Val Ser Ser Ala Ser Thr Lys
    130                 135                 140

ggc cca tcg gtc ttc ccc ctg gca ccc tcc tcc aag agc acc tct ggg     480
Gly Pro Ser Val Phe Pro Leu Ala Pro Ser Ser Lys Ser Thr Ser Gly
145                 150                 155                 160

ggc aca gcg gcc ctg ggc tgc ctg gtc aag gac tac ttc ccc gaa ccg     528
Gly Thr Ala Ala Leu Gly Cys Leu Val Lys Asp Tyr Phe Pro Glu Pro
                165                 170                 175

gtg acg gtg tcg tgg aac tca ggc gcc ctg acc agc ggc gtg cac acc     576
Val Thr Val Ser Trp Asn Ser Gly Ala Leu Thr Ser Gly Val His Thr
            180                 185                 190

ttc ccg gct gtc cta cag tcc tca gga ctc tac tcc ctc agc agc gtg     624
Phe Pro Ala Val Leu Gln Ser Ser Gly Leu Tyr Ser Leu Ser Ser Val
        195                 200                 205

gtg acc gtg ccc tcc agc agc ttg ggc acc cag acc tac atc tgc aac     672
Val Thr Val Pro Ser Ser Ser Leu Gly Thr Gln Thr Tyr Ile Cys Asn
    210                 215                 220

gtg aat cac aag ccc agc aac acc aag gtg gac aag aaa gtt gag ccc     720
Val Asn His Lys Pro Ser Asn Thr Lys Val Asp Lys Lys Val Glu Pro
225                 230                 235                 240

aaa tct tgt gac aaa act cac aca tgc cca ccg tgc cca gca cct gaa     768
Lys Ser Cys Asp Lys Thr His Thr Cys Pro Pro Cys Pro Ala Pro Glu
                245                 250                 255

ctc ctg ggg gga ccg tca gtc ttc ctc ttc ccc cca aaa ccc aag gac     816
Leu Leu Gly Gly Pro Ser Val Phe Leu Phe Pro Pro Lys Pro Lys Asp
            260                 265                 270

acc ctc atg atc tcc cgg acc cct gag gtc aca tgc gtg gtg gtg gac     864
Thr Leu Met Ile Ser Arg Thr Pro Glu Val Thr Cys Val Val Val Asp
        275                 280                 285

gtg agc cac gaa gac cct gag gtc aag ttc aac tgg tac gtg gac ggc     912
Val Ser His Glu Asp Pro Glu Val Lys Phe Asn Trp Tyr Val Asp Gly
    290                 295                 300

gtg gag gtg cat aat gcc aag aca aag ccg cgg gag gag cag tac aac     960
Val Glu Val His Asn Ala Lys Thr Lys Pro Arg Glu Glu Gln Tyr Asn

305                    310                    315                    320

agc acg tac cgt gtg gtc agc gtc ctc acc gtc ctg cac cag gac tgg      1008
Ser Thr Tyr Arg Val Val Ser Val Leu Thr Val Leu His Gln Asp Trp
                325                 330                 335

ctg aat ggc aag gag tac aag tgc aag gtc tcc aac aaa gcc ctc cca      1056
Leu Asn Gly Lys Glu Tyr Lys Cys Lys Val Ser Asn Lys Ala Leu Pro
            340                 345                 350

gcc ccc atc gag aaa acc atc tcc aaa gcc aaa ggg cag ccc cga gaa      1104
Ala Pro Ile Glu Lys Thr Ile Ser Lys Ala Lys Gly Gln Pro Arg Glu
        355                 360                 365

cca cag gtg tac acc ctg ccc cca tcc cgg gat gag ctg acc aag aac      1152
Pro Gln Val Tyr Thr Leu Pro Pro Ser Arg Asp Glu Leu Thr Lys Asn
    370                 375                 380

cag gtc agc ctg acc tgc ctg gtc aaa ggc ttc tat ccc agc gac atc      1200
Gln Val Ser Leu Thr Cys Leu Val Lys Gly Phe Tyr Pro Ser Asp Ile
385                 390                 395                 400

gcc gtg gag tgg gag agc aat ggg cag ccg gag aac aac tac aag acc      1248
Ala Val Glu Trp Glu Ser Asn Gly Gln Pro Glu Asn Asn Tyr Lys Thr
                405                 410                 415

acg cct ccc gtg ctg gac tcc gac ggc tcc ttc ttc ctc tac agc aag      1296
Thr Pro Pro Val Leu Asp Ser Asp Gly Ser Phe Phe Leu Tyr Ser Lys
            420                 425                 430

ctc acc gtg gac aag agc agg tgg cag cag ggg aac gtc ttc tca tgc      1344
Leu Thr Val Asp Lys Ser Arg Trp Gln Gln Gly Asn Val Phe Ser Cys
        435                 440                 445

tcc gtg atg cat gag gct ctg cac aac cac tac acg cag aag agc ctc      1392
Ser Val Met His Glu Ala Leu His Asn His Tyr Thr Gln Lys Ser Leu
    450                 455                 460

tcc ctg tct ccg ggt aaa acc att tta ccg gaa gaa tgg gtt cca cta      1440
Ser Leu Ser Pro Gly Lys Thr Ile Leu Pro Glu Glu Trp Val Pro Leu
465                 470                 475                 480

att aaa aac ggt aaa gtt aag ata ttc cgc att ggg gac ttc gtt gat      1488
Ile Lys Asn Gly Lys Val Lys Ile Phe Arg Ile Gly Asp Phe Val Asp
                485                 490                 495

gga ctt atg aag gcg aac caa gga aaa gtg aag aaa acg ggg gat aca      1536
Gly Leu Met Lys Ala Asn Gln Gly Lys Val Lys Lys Thr Gly Asp Thr
            500                 505                 510

gaa gtt tta gaa gtt gca gga att cat gcg ttt tcc ttt gac agg aag      1584
Glu Val Leu Glu Val Ala Gly Ile His Ala Phe Ser Phe Asp Arg Lys
        515                 520                 525

tcc aag aag gcc cgt gta atg gca gtg aaa gcc gtg ata aga cac cgt      1632
Ser Lys Lys Ala Arg Val Met Ala Val Lys Ala Val Ile Arg His Arg
    530                 535                 540

tat tcc gga aat gtt tat aga ata gtc tta aac tct ggt aga aaa ata      1680
Tyr Ser Gly Asn Val Tyr Arg Ile Val Leu Asn Ser Gly Arg Lys Ile
545                 550                 555                 560

aca ata aca gaa ggg cat agc cta ttt gtc tat agg aac ggg gat ctc      1728
Thr Ile Thr Glu Gly His Ser Leu Phe Val Tyr Arg Asn Gly Asp Leu
                565                 570                 575

gtt gag gca act ggg gag gat gtc aaa att ggg gat ctt ctt gca gtt      1776
Val Glu Ala Thr Gly Glu Asp Val Lys Ile Gly Asp Leu Leu Ala Val

            580                 585                 590

cca aga tca gta aac cta cca gag aaa agg gaa cgc ttg aat att gtt 1824
Pro Arg Ser Val Asn Leu Pro Glu Lys Arg Glu Arg Leu Asn Ile Val
        595                 600                 605

gaa ctt ctt ctg aat ctc tca ccg gaa gag aca gaa gat ata ata ctt 1872
Glu Leu Leu Leu Asn Leu Ser Pro Glu Glu Thr Glu Asp Ile Ile Leu
    610                 615                 620

acg att cca gtt aaa ggc aga aag aac ttc ttc aag gga atg ttg aga 1920
Thr Ile Pro Val Lys Gly Arg Lys Asn Phe Phe Lys Gly Met Leu Arg
625                 630                 635                 640

aca tta cgt tgg att ttt ggt gag gaa aag aga gta agg aca gcg agc 1968
Thr Leu Arg Trp Ile Phe Gly Glu Glu Lys Arg Val Arg Thr Ala Ser
                645                 650                 655

cgc tat cta aga cac ctt gaa aat ctc gga tac ata agg ttg agg aaa 2016
Arg Tyr Leu Arg His Leu Glu Asn Leu Gly Tyr Ile Arg Leu Arg Lys
            660                 665                 670

att gga tac gac atc att gat aag gag ggg ctt gag aaa tat aga acg 2064
Ile Gly Tyr Asp Ile Ile Asp Lys Glu Gly Leu Glu Lys Tyr Arg Thr
        675                 680                 685

ttg tac gag aaa ctt gtt gat gtt gtc cgc tat aat ggc aac aag aga 2112
Leu Tyr Glu Lys Leu Val Asp Val Val Arg Tyr Asn Gly Asn Lys Arg
    690                 695                 700

gag tat tta gtt gaa ttt aat gct gtc cgg gac gtt atc tca cta atg 2160
Glu Tyr Leu Val Glu Phe Asn Ala Val Arg Asp Val Ile Ser Leu Met
705                 710                 715                 720

cca gag gaa gaa ctg aag gaa tgg cgt att gga act aga aat gga ttc 2208
Pro Glu Glu Glu Leu Lys Glu Trp Arg Ile Gly Thr Arg Asn Gly Phe
                725                 730                 735

aga atg ggt acg ttc gta gat att gat gaa gat ttt gcc aag ctt gga 2256
Arg Met Gly Thr Phe Val Asp Ile Asp Glu Asp Phe Ala Lys Leu Gly
            740                 745                 750

tac gat agc gga gtc tac agg gtt tat gta aac gag gaa ctt aag ttt 2304
Tyr Asp Ser Gly Val Tyr Arg Val Tyr Val Asn Glu Glu Leu Lys Phe
        755                 760                 765

acg gaa tac aga aag aaa aag aat gta tat cac tct cac att gtt cca 2352
Thr Glu Tyr Arg Lys Lys Lys Asn Val Tyr His Ser His Ile Val Pro
    770                 775                 780

aag gat att ctc aaa gaa act ttt ggt aag gtc ttc cag aaa aat ata 2400
Lys Asp Ile Leu Lys Glu Thr Phe Gly Lys Val Phe Gln Lys Asn Ile
785                 790                 795                 800

agt tac aag aaa ttt aga gag ctt gta gaa aat gga aaa ctt gac agg 2448
Ser Tyr Lys Lys Phe Arg Glu Leu Val Glu Asn Gly Lys Leu Asp Arg
                805                 810                 815

gag aaa gcc aaa cgc att gag tgg tta ctt aac gga gat ata gtc cta 2496
Glu Lys Ala Lys Arg Ile Glu Trp Leu Leu Asn Gly Asp Ile Val Leu
            820                 825                 830

gat aga gtc gta gag att aag aga gag tac tat gat ggt tac gtt tac 2544
Asp Arg Val Val Glu Ile Lys Arg Glu Tyr Tyr Asp Gly Tyr Val Tyr
        835                 840                 845

gat cta agt gtc gat gaa gat gag aat ttc ctt gct ggc ttt gga ttc 2592
Asp Leu Ser Val Asp Glu Asp Glu Asn Phe Leu Ala Gly Phe Gly Phe

850                 855                 860

ctc tat gca cat aat gac atc cag atg acc cag tct cca tcc tcc ctg      2640
Leu Tyr Ala His Asn Asp Ile Gln Met Thr Gln Ser Pro Ser Ser Leu
865                 870                 875                 880

tct gca tct gta ggg gac aga gtc acc atc act tgt cgg gca agt cag      2688
Ser Ala Ser Val Gly Asp Arg Val Thr Ile Thr Cys Arg Ala Ser Gln
                885                 890                 895

ggc atc aga aat tac tta gcc tgg tat cag caa aaa cca ggg aaa gcc      2736
Gly Ile Arg Asn Tyr Leu Ala Trp Tyr Gln Gln Lys Pro Gly Lys Ala
            900                 905                 910

cct aag ctc ctg atc tat gct gca tcc act ttg caa tca ggg gtc cca      2784
Pro Lys Leu Leu Ile Tyr Ala Ala Ser Thr Leu Gln Ser Gly Val Pro
        915                 920                 925

tct cgg ttc agt ggc agt gga tct ggg aca gat ttc act ctc acc atc      2832
Ser Arg Phe Ser Gly Ser Gly Ser Gly Thr Asp Phe Thr Leu Thr Ile
    930                 935                 940

agc agc cta cag cct gaa gat gtt gca act tat tac tgt caa agg tat      2880
Ser Ser Leu Gln Pro Glu Asp Val Ala Thr Tyr Tyr Cys Gln Arg Tyr
945                 950                 955                 960

aac cgt gca ccg tat act ttt ggc cag ggg acc aag gtg gaa atc aaa      2928
Asn Arg Ala Pro Tyr Thr Phe Gly Gln Gly Thr Lys Val Glu Ile Lys
                965                 970                 975

cgt acg gtg gct gca cca tct gtc ttc atc ttc ccg cca tct gat gag      2976
Arg Thr Val Ala Ala Pro Ser Val Phe Ile Phe Pro Pro Ser Asp Glu
            980                 985                 990

cag ttg aaa tct gga act gcc tct  gtt gtg tgc ctg ctg  aat aac ttc      3024
Gln Leu Lys Ser Gly Thr Ala Ser  Val Val Cys Leu Leu  Asn Asn Phe
        995                 1000                1005

tat ccc  aga gag gcc aaa gta  cag tgg aag gtg gat  aac gcc ctc      3069
Tyr Pro  Arg Glu Ala Lys Val  Gln Trp Lys Val Asp  Asn Ala Leu
    1010                1015                1020

caa tcg  ggt aac tcc cag gag  agt gtc aca gag cag  gac agc aag      3114
Gln Ser  Gly Asn Ser Gln Glu  Ser Val Thr Glu Gln  Asp Ser Lys
    1025                1030                1035

gac agc  acc tac agc ctc agc  agc acc ctg acg ctg  agc aaa gca      3159
Asp Ser  Thr Tyr Ser Leu Ser  Ser Thr Leu Thr Leu  Ser Lys Ala
    1040                1045                1050

gac tac  gag aaa cac aaa gtc  tac gcc tgc gaa gtc  acc cat cag      3204
Asp Tyr  Glu Lys His Lys Val  Tyr Ala Cys Glu Val  Thr His Gln
    1055                1060                1065

ggc ctg  agc tcg ccc gtc aca  aag agc ttc aac agg  gga gag tgt      3249
Gly Leu  Ser Ser Pro Val Thr  Lys Ser Phe Asn Arg  Gly Glu Cys
    1070                1075                1080

tga                                                                  3252

<210>  49
<211>  1083
<212>  PRT
<213>  Artificial

<220>
<223>  Synthetic Construct

<400>  49

Met Glu Phe Gly Leu Ser Trp Leu Phe Leu Val Ala Ile Leu Lys Gly
1               5                   10                  15

Val Gln Cys Glu Val Gln Leu Val Glu Ser Gly Gly Gly Leu Val Gln
            20                  25                  30

Pro Gly Arg Ser Leu Arg Leu Ser Cys Ala Ala Ser Gly Phe Thr Phe
        35                  40                  45

Asp Asp Tyr Ala Met His Trp Val Arg Gln Ala Pro Gly Lys Gly Leu
    50                  55                  60

Glu Trp Val Ser Ala Ile Thr Trp Asn Ser Gly His Ile Asp Tyr Ala
65                  70                  75                  80

Asp Ser Val Glu Gly Arg Phe Thr Ile Ser Arg Asp Asn Ala Lys Asn
                85                  90                  95

Ser Leu Tyr Leu Gln Met Asn Ser Leu Arg Ala Glu Asp Thr Ala Val
            100                 105                 110

Tyr Tyr Cys Ala Lys Val Ser Tyr Leu Ser Thr Ala Ser Ser Leu Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp

260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Asp Glu Leu Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly Lys Thr Ile Leu Pro Glu Glu Trp Val Pro Leu
465                 470                 475                 480

Ile Lys Asn Gly Lys Val Lys Ile Phe Arg Ile Gly Asp Phe Val Asp
                485                 490                 495

Gly Leu Met Lys Ala Asn Gln Gly Lys Val Lys Lys Thr Gly Asp Thr
            500                 505                 510

Glu Val Leu Glu Val Ala Gly Ile His Ala Phe Ser Phe Asp Arg Lys
        515                 520                 525

Ser Lys Lys Ala Arg Val Met Ala Val Lys Ala Val Ile Arg His Arg

530                 535                 540

Tyr Ser Gly Asn Val Tyr Arg Ile Val Leu Asn Ser Gly Arg Lys Ile
545                 550                 555                 560

Thr Ile Thr Glu Gly His Ser Leu Phe Val Tyr Arg Asn Gly Asp Leu
                565                 570                 575

Val Glu Ala Thr Gly Glu Asp Val Lys Ile Gly Asp Leu Leu Ala Val
            580                 585                 590

Pro Arg Ser Val Asn Leu Pro Glu Lys Arg Glu Arg Leu Asn Ile Val
        595                 600                 605

Glu Leu Leu Leu Asn Leu Ser Pro Glu Glu Thr Glu Asp Ile Ile Leu
    610                 615                 620

Thr Ile Pro Val Lys Gly Arg Lys Asn Phe Phe Lys Gly Met Leu Arg
625                 630                 635                 640

Thr Leu Arg Trp Ile Phe Gly Glu Glu Lys Arg Val Arg Thr Ala Ser
                645                 650                 655

Arg Tyr Leu Arg His Leu Glu Asn Leu Gly Tyr Ile Arg Leu Arg Lys
            660                 665                 670

Ile Gly Tyr Asp Ile Ile Asp Lys Glu Gly Leu Glu Lys Tyr Arg Thr
        675                 680                 685

Leu Tyr Glu Lys Leu Val Asp Val Val Arg Tyr Asn Gly Asn Lys Arg
    690                 695                 700

Glu Tyr Leu Val Glu Phe Asn Ala Val Arg Asp Val Ile Ser Leu Met
705                 710                 715                 720

Pro Glu Glu Glu Leu Lys Glu Trp Arg Ile Gly Thr Arg Asn Gly Phe
                725                 730                 735

Arg Met Gly Thr Phe Val Asp Ile Asp Glu Asp Phe Ala Lys Leu Gly
            740                 745                 750

Tyr Asp Ser Gly Val Tyr Arg Val Tyr Val Asn Glu Glu Leu Lys Phe
        755                 760                 765

Thr Glu Tyr Arg Lys Lys Lys Asn Val Tyr His Ser His Ile Val Pro
    770                 775                 780

Lys Asp Ile Leu Lys Glu Thr Phe Gly Lys Val Phe Gln Lys Asn Ile
785                 790                 795                 800

Ser Tyr Lys Lys Phe Arg Glu Leu Val Glu Asn Gly Lys Leu Asp Arg

                805                 810                 815

Glu Lys Ala Lys Arg Ile Glu Trp Leu Leu Asn Gly Asp Ile Val Leu
            820                 825                 830

Asp Arg Val Val Glu Ile Lys Arg Glu Tyr Tyr Asp Gly Tyr Val Tyr
        835                 840                 845

Asp Leu Ser Val Asp Glu Asp Glu Asn Phe Leu Ala Gly Phe Gly Phe
    850                 855                 860

Leu Tyr Ala His Asn Asp Ile Gln Met Thr Gln Ser Pro Ser Ser Leu
865                 870                 875                 880

Ser Ala Ser Val Gly Asp Arg Val Thr Ile Thr Cys Arg Ala Ser Gln
                885                 890                 895

Gly Ile Arg Asn Tyr Leu Ala Trp Tyr Gln Gln Lys Pro Gly Lys Ala
            900                 905                 910

Pro Lys Leu Leu Ile Tyr Ala Ala Ser Thr Leu Gln Ser Gly Val Pro
        915                 920                 925

Ser Arg Phe Ser Gly Ser Gly Ser Gly Thr Asp Phe Thr Leu Thr Ile
    930                 935                 940

Ser Ser Leu Gln Pro Glu Asp Val Ala Thr Tyr Tyr Cys Gln Arg Tyr
945                 950                 955                 960

Asn Arg Ala Pro Tyr Thr Phe Gly Gln Gly Thr Lys Val Glu Ile Lys
                965                 970                 975

Arg Thr Val Ala Ala Pro Ser Val Phe Ile Phe Pro Pro Ser Asp Glu
            980                 985                 990

Gln Leu Lys Ser Gly Thr Ala Ser Val Val Cys Leu Leu Asn Asn Phe
        995                 1000                1005

Tyr Pro Arg Glu Ala Lys Val Gln Trp Lys Val Asp Asn Ala Leu
    1010                1015                1020

Gln Ser Gly Asn Ser Gln Glu Ser Val Thr Glu Gln Asp Ser Lys
    1025                1030                1035

Asp Ser Thr Tyr Ser Leu Ser Ser Thr Leu Thr Leu Ser Lys Ala
    1040                1045                1050

Asp Tyr Glu Lys His Lys Val Tyr Ala Cys Glu Val Thr His Gln
    1055                1060                1065

Gly Leu Ser Ser Pro Val Thr Lys Ser Phe Asn Arg Gly Glu Cys

1070                1075                1080

<210>  50
<211>  10629
<212>  DNA
<213>  Artificial

<220>
<223>  Synthetic construct, D2E7 intein fusion protein expression
       vector.

<400>  50
gaagttccta ttccgaagtt cctattctct agacgttaca taacttacgg taaatggccc      60

gcctggctga ccgcccaacg acccccgccc attgacgtca ataatgacgt atgttcccat     120

agtaacgcca atagggactt tccattgacg tcaatgggtg gagtatttac ggtaaactgc     180

ccacttggca gtacatcaag tgtatcatat gccaagtacg ccccctattg acgtcaatga     240

cggtaaatgg cccgcctggc attatgccca gtacatgacc ttatgggact ttcctacttg     300

gcagtacatc tacgtattag tcatcgctat taccatggtg atgcggtttt ggcagtacat     360

caatgggcgt ggatagcggt ttgactcacg gggatttcca agtctccacc ccattgacgt     420

caatgggagt ttgttttggc accaaaatca acgggacttt ccaaaatgtc gtaacaactc     480

cgccccattg acgcaaatgg gcagggaatt cgagctcggt actcgagcgg tgttccgcgg     540

tcctcctcgt atagaaactc ggaccactct gagacgaagg ctcgcgtcca ggccagcacg     600

aaggaggcta agtgggaggg gtagcggtcg ttgtccacta gggggtccac tcgctccagg     660

gtgtgaagac acatgtcgcc ctcttcggca tcaaggaagg tgattggttt ataggtgtag     720

gccacgtgac cgggtgttcc tgaagggggg ctataaaagg gggtgggggc gcgttcgtcc     780

tcactctctt ccgcatcgct gtctgcgagg gccagctgtt gggctcgcgg ttgaggacaa     840

actcttcgcg gtctttccag tactcttgga tcggaaaccc gtcggcctcc gaacggtact     900

ccgccaccga gggacctgag cgagtccgca tcgaccggat cggaaaacct ctcgactgtt     960

ggggtgagta ctccctctca aaagcgggca tgacttctgc gctaagattg tcagtttcca    1020

aaaacgagga ggatttgata ttcacctggc ccgcggtgat gcctttgagg gtggccgcgt    1080

ccatctggtc agaaaagaca atctttttgt tgtcaagctt gaggtgtggc aggcttgaga    1140

tctggccata cacttgagtg acaatgacat ccactttgcc tttctctcca caggtgtcca    1200

ctcccaggtc caaccggaat tgtacccgcg gccagagctt gcccgggcgc caccatggag    1260

tttgggctga gctggctttt tcttgtcgcg attttaaaag gtgtccagtg tgaggtgcag    1320

ctggtggagt ctgggggagg cttggtacag cccggcaggt ccctgagact ctcctgtgcg    1380

gcctctggat tcacctttga tgattatgcc atgcactggg tccggcaagc tccagggaag    1440

ggcctggaat gggtctcagc tatcacttgg aatagtggtc acatagacta tgcggactct    1500

gtggagggcc gattcaccat ctccagagac aacgccaaga actccctgta tctgcaaatg    1560

aacagtctga gagctgagga tacggccgta tattactgtg cgaaagtctc gtaccttagc    1620

accgcgtcct cccttgacta ttggggccaa ggtaccctgg tcaccgtctc gagtgcgtcg    1680

accaagggcc catcggtctt ccccctggca ccctcctcca agagcacctc tgggggcaca 1740
gcggccctgg gctgcctggt caaggactac ttccccgaac cggtgacggt gtcgtggaac 1800
tcaggcgccc tgaccagcgg cgtgcacacc ttcccggctg tcctacagtc ctcaggactc 1860
tactccctca gcagcgtggt gaccgtgccc tccagcagct tgggcaccca gacctacatc 1920
tgcaacgtga atcacaagcc cagcaacacc aaggtggaca agaaagttga gcccaaatct 1980
tgtgacaaaa ctcacacatg cccaccgtgc ccagcacctg aactcctggg gggaccgtca 2040
gtcttcctct tccccccaaa acccaaggac accctcatga tctcccggac ccctgaggtc 2100
acatgcgtgg tggtggacgt gagccacgaa gaccctgagg tcaagttcaa ctggtacgtg 2160
gacggcgtgg aggtgcataa tgccaagaca aagccgcggg aggagcagta caacagcacg 2220
taccgtgtgg tcagcgtcct caccgtcctg caccaggact ggctgaatgg caaggagtac 2280
aagtgcaagg tctccaacaa agccctccca gcccccatcg agaaaaccat ctccaaagcc 2340
aaagggcagc cccgagaacc acaggtgtac accctgcccc catcccggga tgagctgacc 2400
aagaaccagg tcagcctgac ctgcctggtc aaaggcttct atcccagcga catcgccgtg 2460
gagtgggaga gcaatgggca gccggagaac aactacaaga ccacgcctcc cgtgctggac 2520
tccgacggct ccttcttcct ctacagcaag ctcaccgtgg acaagagcag gtggcagcag 2580
gggaacgtct tctcatgctc cgtgatgcat gaggctctgc acaaccacta cacgcagaag 2640
agcctctccc tgtctccggg taaaaccatt ttaccggaag aatgggttcc actaattaaa 2700
aacggtaaag ttaagatatt ccgcattggg gacttcgttg atggacttat gaaggcgaac 2760
caaggaaaag tgaagaaaac gggggataca gaagttttag aagttgcagg aattcatgcg 2820
ttttcctttg acaggaagtc caagaaggcc cgtgtaatgg cagtgaaagc cgtgataaga 2880
caccgttatt ccggaaatgt ttatagaata gtcttaaact ctggtagaaa aataacaata 2940
acagaagggc atagcctatt tgtctatagg aacggggatc tcgttgaggc aactggggag 3000
gatgtcaaaa ttggggatct tcttgcagtt ccaagatcag taaacctacc agagaaaagg 3060
gaacgcttga atattgttga acttcttctg aatctctcac cggaagagac agaagatata 3120
atacttacga ttccagttaa aggcagaaag aacttcttca agggaatgtt gagaacatta 3180
cgttggattt ttggtgagga aaagagagta aggacagcga gccgctatct aagacacctt 3240
gaaaatctcg gatacataag gttgaggaaa attggatacg acatcattga taaggagggg 3300
cttgagaaat atagaacgtt gtacgagaaa cttgttgatg ttgtccgcta taatggcaac 3360
aagagagagt atttagttga atttaatgct gtccgggacg ttatctcact aatgccagag 3420
gaagaactga aggaatggcg tattggaact agaaatggat tcagaatggg tacgttcgta 3480
gatattgatg aagattttgc caagcttgga tacgatagcg gagtctacag ggtttatgta 3540
aacgaggaac ttaagtttac ggaatacaga aagaaaaaga atgtatatca ctctcacatt 3600
gttccaaagg atattctcaa agaaactttt ggtaaggtct tccagaaaaa tataagttac 3660
aagaaattta gagagcttgt agaaaatgga aaacttgaca gggagaaagc caaacgcatt 3720

gagtggttac ttaacggaga tatagtccta gatagagtcg tagagattaa gagagagtac 3780
tatgatggtt acgtttacga tctaagtgtc gatgaagatg agaatttcct tgctggcttt 3840
ggattcctct atgcacataa tgacatccag atgacccagt ctccatcctc cctgtctgca 3900
tctgtagggg acagagtcac catcacttgt cgggcaagtc agggcatcag aaattactta 3960
gcctggtatc agcaaaaacc agggaaagcc cctaagctcc tgatctatgc tgcatccact 4020
ttgcaatcag gggtcccatc tcggttcagt ggcagtggat ctgggacaga tttcactctc 4080
accatcagca gcctacagcc tgaagatgtt gcaacttatt actgtcaaag gtataaccgt 4140
gcaccgtata cttttggcca ggggaccaag gtggaaatca aacgtacggt ggctgcacca 4200
tctgtcttca tcttcccgcc atctgatgag cagttgaaat ctggaactgc ctctgttgtg 4260
tgcctgctga ataacttcta tcccagagag gccaaagtac agtggaaggt ggataacgcc 4320
ctccaatcgg gtaactccca ggagagtgtc acagagcagg acagcaagga cagcacctac 4380
agcctcagca gcaccctgac gctgagcaaa gcagactacg agaaacacaa agtctacgcc 4440
tgcgaagtca cccatcaggg cctgagctcg cccgtcacaa agagcttcaa caggggagag 4500
tgttgagcgg ccgcgtttaa actgaatgag cgcgtccatc cagacatgat aagatacatt 4560
gatgagtttg gacaaaccac aactagaatg cagtgaaaaa aatgctttat ttgtgaaatt 4620
tgtgatgcta ttgctttatt tgtaaccatt ataagctgca ataaacaagt taacaacaac 4680
aattgcattc attttatgtt tcaggttcag ggggaggtgt gggaggtttt ttaaagcaag 4740
taaaacctct acaaatgtgg tatggctgat tatgatccgg ctgcctcgcg cgtttcggtg 4800
atgacggtga aaacctctga cacatgcagc tcccggagac ggtcacagct tgtctgtaag 4860
cggatgccgg gagcagacaa gcccgtcagg gcgcgtcagc gggtgttggc gggtgtcggg 4920
gcgcagccat gaccggtcga cggcgcgcct ttttttttaa tttttatttt attttatttt 4980
tgacgcgccg aaggcgcgat ctgagctcgg tacagcttgg ctgtggaatg tgtgtcagtt 5040
agggtgtgga aagtccccag gctccccagc aggcagaagt atgcaaagca tgcatctcaa 5100
ttagtcagca accaggtgtg gaaagtcccc aggctcccca gcaggcagaa gtatgcaaag 5160
catgcatctc aattagtcag caaccatagt cccgccccta actccgccca tcccgcccct 5220
aactccgccc agttccgccc attctccgcc ccatggctga ctaatttttt ttatttatgc 5280
agaggccgag gccgcctcgg cctctgagct attccagaag tagtgaggag gcttttttgg 5340
aggcctaggc ttttgcaaaa agctcctcga ggaactgaaa aaccagaaag ttaactggta 5400
agtttagtct ttttgtcttt tatttcaggt cccggatccg gtggtggtgc aaatcaaaga 5460
actgctcctc agtggatgtt gcctttactt ctaggcctgt acggaagtgt tacttctgct 5520
ctaaaagctg cggaattgta cccgcggcct aatacgactc actataggga ctagtatggt 5580
tcgaccattg aactgcatcg tcgccgtgtc ccaaaatatg gggattggca agaacggaga 5640
cctaccctgg cctccgctca ggaacgagtt caagtacttc caaagaatga ccacaacctc 5700
ttcagtggaa ggtaaacaga atctggtgat tatgggtagg aaaacctggt tctccattcc 5760

tgagaagaat cgacctttaa aggacagaat taatatagtt ctcagtagag aactcaaaga 5820
accaccacga ggagctcatt ttcttgccaa aagtttagat gatgccttaa gacttattga 5880
acaaccggaa ttggcaagta aagtagacat ggtttggata gtcggaggca gttctgttta 5940
ccaggaagcc atgaatcaac caggccacct cagactcttt gtgacaagga tcatgcagga 6000
atttgaaagt gacacgtttt tcccagaaat tgatttgggg aaatataaac ttctcccaga 6060
atacccaggc gtcctctctg aggtccagga ggaaaaaggc atcaagtata agtttgaagt 6120
ctacgagaag aaagactaag cggccgagcg cgcggatctg gaaacgggag atgggggagg 6180
ctaactgaag cacggaagga gacaataccg gaaggaaccc gcgctatgac ggcaataaaa 6240
agacagaata aaacgcacgg gtgttgggtc gtttgttcat aaacgcgggg ttcggtccca 6300
gggctggcac tctgtcgata ccccaccgag accccattgg ggccaatacg cccgcgtttc 6360
ttccttttcc ccaccccacc ccccaagttc gggtgaaggc ccagggctcg cagccaacgt 6420
cggggcggca ggccctgcca tagccactgg ccccgtgggt tagggacggg gtcccccatg 6480
gggaatggtt tatggttcgt gggggttatt attttgggcg ttgcgtgggg tctggagatc 6540
ccccgggctg caggaattcc gttacattac ttacggtaaa tggcccgcct ggctgaccgc 6600
ccaacgaccc ccgcccattg acgtcaataa tgacgtatgt tcccatagta acgccaatag 6660
ggactttcca ttgacgtcaa tgggtggagt atttacggta aactgcccac ttggcagtac 6720
atcaagtgta tcatatgcca agtacgcccc ctattgacgt caatgacggt aaatggcccg 6780
cctggcatta tgcccagtac atgaccttat gggactttcc tacttggcag tacatctacg 6840
tattagtcat cgctattacc atggtgatgc ggttttggca gtacatcaat gggcgtggat 6900
agcggtttga ctcacgggga tttccaagtc tccaccccat tgacgtcaat gggagtttgt 6960
tttggcacca aaatcaacgg gactttccaa aatgtcgtaa caactccgcc ccattgacgc 7020
aaaagggcgg gaattcgagc tcggtactcg agcggtgttc cgcggtcctc ctcgtataga 7080
aactcggacc actctgagac gaaggctcgc gtccaggcca gcacgaagga ggctaagtgg 7140
gaggggtagc ggtcgttgtc cactaggggg tccactcgct ccagggtgtg aagacacatg 7200
tcgccctctt cggcatcaag gaaggtgatt ggtttatagg tgtaggccac gtgaccgggt 7260
gttcctgaag gggggctata aaagggggtg ggggcgcgtt cgtcctcact ctcttccgca 7320
tcgctgtctg cgagggccag ctgttgggct cgcggttgag gacaaactct tcgcggtctt 7380
tccagtactc ttggatcgga aacccgtcgg cctccgaacg gtactccgcc accgagggac 7440
ctgagcgagt ccgcatcgac cggatcggaa aacctctcga ctgttggggt gagtactccc 7500
tctcaaaagc gggcatgact tctgcgctaa gattgtcagt ttccaaaaac gaggaggatt 7560
tgatattcac ctggcccgcg gtgatgcctt tgagggtggc cgcgtccatc tggtcagaaa 7620
agacaatctt tttgttgtca agcttgaggt gtggcaggct tgagatctgg ccatacactt 7680
gagtgacaat gacatccact ttgcctttct ctccacaggt gtccactccc aggtccaacc 7740
ggaattgtac ccgcggccag agcttgcggg cgccaccgcg gccgcgggga tccagacatg 7800

ataagataca ttgatgagtt tggacaaacc acaactagaa tgcagtgaaa aaaatgcttt 7860
atttgtgaaa tttgtgatgc tattgcttta tttgtaacca ttataagctg caataaacaa 7920
gttaacaaca acaattgcat tcattttatg tttcaggttc agggggaggt gtgggaggtt 7980
ttttcggatc ctcttggcgt aatcatggtc atagctgttt cctgtgtgaa attgttatcc 8040
gctcacaatt ccacacaaca tacgagccgg aagcataaag tgtaaagcct ggggtgccta 8100
atgagtgagc taactcacat taattgcgtt gcgctcactg cccgctttcc agtcgggaaa 8160
cctgtcgtgc cagctgcatt aatgaatcgg ccaacgcgcg gggaaaggcg gtttgcgtat 8220
tgggcgctct tccgcttcct cgctcactga ctcgctgcgc tcggtcgttc ggctgcggcg 8280
agcggtatca gctcactcaa aggcggtaat acggttatcc acagaatcag gggataacgc 8340
aggaaagaac atgtgagcaa aaggccagca aaaggccagg aaccgtaaaa aggccgcgtt 8400
gctggcgttc ttccataggc tccgcccccc tgacgagcat cacaaaaatc gacgctcaag 8460
tcagaggtgg cgaaacccga caggactata aagataccag gcgtttcccc ctggaagctc 8520
cctcgtgcgc tctcctgttc cgaccctgcc gcttaccgga tacctgtccg cctttctccc 8580
ttcgggaagc gtggcgcttt ctcatagctc acgctgtagg tatctcagtt cggtgtaggt 8640
cgttcgctcc aagctgggct gtgtgcacga accccccgtt cagcccgacc gctgcgcctt 8700
atccggtaac tatcgtcttg agtccaaccc ggtaagacac gacttatcgc cactggcagc 8760
agccactggt aacaggatta gcagagcgag gtatgtaggc ggtgctacag agttcttgaa 8820
gtggtggcct aactacggct acactagaag aacagtattt ggtatctgcg ctctgctgaa 8880
gccagttacc ttcggaaaaa gagttggtag ctcttgatcc ggcaaacaaa ccaccgctgg 8940
tagcggtggt ttttttgttt gcaagcagca gattacgcgc agaaaaaaag gatctcaaga 9000
agatcctttg atcttttcta cggggtctga cgctcagtgg aacgaaaact cacgttaagg 9060
gattttggtc atgagattat caaaaaggat cttcacctag atccttttaa attaaaaatg 9120
aagttttaaa tcaatctaaa gtatatatga gtaaacttgg tctgacagtt accaatgctt 9180
aatcagtgag gcacctatct cagcgatctg tctatttcgt tcatccatag ttgcctgact 9240
ccccgtcgtg tagataacta cgatacggga gggcttacca tctggcccca gtgctgcaat 9300
gataccgcga gacccacgct caccggctcc agatttatca gcaataaacc agccagccgg 9360
aagggccgag cgcagaagtg gtcctgcaac tttatccgcc tccatccagt ctattaattg 9420
ttgccgggaa gctagagtaa gtagttcgcc agttaatagt ttgcgcaacg ttgttgccat 9480
tgctacaggc atcgtggtgt cacgctcgtc gtttggtatg gcttcattca gctccggttc 9540
ccaacgatca aggcgagtta catgatcccc catgttgtgc aaaaaagcgg ttagctcctt 9600
cggtcctccg atcgttgtca gaagtaagtt ggccgcagtg ttatcactca tggttatggc 9660
agcactgcat aattctctta ctgtcatgcc atccgtaaga tgcttttctg tgactggtga 9720
gtactcaacc aagtcattct gagaatagtg tatgcggcga ccgagttgct cttgcccggc 9780
gtcaatacgg gataataccg cgccacatag cagaacttta aaagtgctca tcattggaaa 9840

acgttcttcg gggcgaaaac tctcaaggat cttaccgctg ttgagatcca gttcgatgta 9900

acccactcgt gcacccaact gatcttcagc atcttttact ttcaccagcg tttctgggtg 9960

agcaaaaaca ggaaggcaaa atgccgcaaa aaagggaata agggcgacac ggaaatgttg 10020

aatactcata ctcttccttt ttcaatatta ttgaagcatt tatcagggtt attgtctcat 10080

gagcggatac atatttgaat gtatttagaa aaataaacaa ataggggttc cgcgcacatt 10140

tccccgaaaa gtgccacctg acgtctaaga aaccattatt atcatgacat taacctataa 10200

aaataggcgt atcacgaggc cctttcgtct cgcgcgtttc ggtgatgacg gtgaaaacct 10260

ctgacacatg cagctcccgg agacggtcac agcttgtctg taagcggatg ccgggagcag 10320

acaagcccgt cagggcgcgt cagcgggtgt tggcgggtgt cggggctggc ttaactatgc 10380

ggcatcagag cagattgtac tgagagtgca ccatatgcgg tgtgaaatac cgcacagatg 10440

cgtaaggaga aaataccgca tcaggcgcca ttcgccattc aggctgcgca actgttggga 10500

agggcgatcg gtgcgggcct cttcgctatt acgccagctg gcgaaagggg gatgtgctgc 10560

aaggcgatta agttgggtaa cgccagggtt ttcccagtta cgacgttgta aaacgacggc 10620

cagtgaatt 10629

<210> 51
<211> 547
<212> PRT
<213> Pyrococcus sp.

<400> 51

Asn Ser Ile Leu Pro Glu Glu Trp Val Pro Leu Ile Lys Asn Gly Lys
1               5                   10                  15

Val Lys Ile Phe Arg Ile Gly Asp Phe Val Asp Gly Leu Met Lys Ala
            20                  25                  30

Asn Gln Gly Lys Val Lys Lys Thr Gly Asp Thr Glu Val Leu Glu Val
        35                  40                  45

Ala Gly Ile His Ala Phe Ser Phe Asp Arg Lys Ser Lys Lys Ala Arg
    50                  55                  60

Val Met Ala Val Lys Ala Val Ile Arg His Arg Tyr Ser Gly Asn Val
65                  70                  75                  80

Tyr Arg Ile Val Leu Asn Ser Gly Arg Lys Ile Thr Ile Thr Glu Gly
                85                  90                  95

His Ser Leu Phe Val Tyr Arg Asn Gly Asp Leu Val Glu Ala Thr Gly
            100                 105                 110

Glu Asp Val Lys Ile Gly Asp Leu Leu Ala Val Pro Arg Ser Val Asn
        115                 120                 125

Leu Pro Glu Lys Arg Glu Arg Leu Asn Ile Val Glu Leu Leu Leu Asn
    130                 135                 140

Leu Ser Pro Glu Glu Thr Glu Asp Ile Ile Leu Thr Ile Pro Val Lys
145                 150                 155                 160

Gly Arg Lys Asn Phe Phe Lys Gly Met Leu Arg Thr Leu Arg Trp Ile
                165                 170                 175

Phe Gly Glu Glu Lys Arg Val Arg Thr Ala Ser Arg Tyr Leu Arg His
            180                 185                 190

Leu Glu Asn Leu Gly Tyr Ile Arg Leu Arg Lys Ile Gly Tyr Asp Ile
        195                 200                 205

Ile Asp Lys Glu Gly Leu Glu Lys Tyr Arg Thr Leu Tyr Glu Lys Leu
    210                 215                 220

Val Asp Val Val Arg Tyr Asn Gly Asn Lys Arg Glu Tyr Leu Val Glu
225                 230                 235                 240

Phe Asn Ala Val Arg Asp Val Ile Ser Leu Met Pro Glu Glu Glu Leu
                245                 250                 255

Lys Glu Trp Arg Ile Gly Thr Arg Asn Gly Phe Arg Met Gly Thr Phe
            260                 265                 270

Val Asp Ile Asp Glu Asp Phe Ala Lys Leu Leu Gly Tyr Tyr Val Ser
        275                 280                 285

Glu Gly Ser Ala Arg Lys Trp Lys Asn Gln Thr Gly Gly Trp Ser Tyr
    290                 295                 300

Thr Val Arg Leu Tyr Asn Glu Asn Asp Glu Val Leu Asp Asp Met Glu
305                 310                 315                 320

His Leu Ala Lys Lys Phe Phe Gly Lys Val Lys Arg Gly Lys Asn Tyr
                325                 330                 335

Val Glu Ile Pro Lys Lys Met Ala Tyr Ile Ile Phe Glu Ser Leu Cys
            340                 345                 350

Gly Thr Leu Ala Glu Asn Lys Arg Val Pro Glu Val Ile Phe Thr Ser
        355                 360                 365

Ser Lys Gly Val Arg Trp Ala Phe Leu Glu Gly Tyr Phe Ile Gly Asp
    370                 375                 380

Gly Asp Val His Pro Ser Lys Arg Val Arg Leu Ser Thr Lys Ser Glu
385                 390                 395                 400

Leu Leu Val Asn Gly Leu Val Leu Leu Leu Asn Ser Leu Gly Val Ser
                405                 410                 415

Ala Ile Lys Leu Gly Tyr Asp Ser Gly Val Tyr Arg Val Tyr Val Asn
            420                 425                 430

Glu Glu Leu Lys Phe Thr Glu Tyr Arg Lys Lys Lys Asn Val Tyr His
        435                 440                 445

Ser His Ile Val Pro Lys Asp Ile Leu Lys Glu Thr Phe Gly Lys Val
    450                 455                 460

Phe Gln Lys Asn Ile Ser Tyr Lys Lys Phe Arg Glu Leu Val Glu Asn
465                 470                 475                 480

Gly Lys Leu Asp Arg Glu Lys Ala Lys Arg Ile Glu Trp Leu Leu Asn
                485                 490                 495

Gly Asp Ile Val Leu Asp Arg Val Val Glu Ile Lys Arg Glu Tyr Tyr
            500                 505                 510

Asp Gly Tyr Val Tyr Asp Leu Ser Val Asp Glu Asp Glu Asn Phe Leu
        515                 520                 525

Ala Gly Phe Gly Phe Leu Tyr Ala His Asn Ser Tyr Tyr Gly Tyr Tyr
    530                 535                 540

Gly Tyr Ala
545

<210>  52
<211>  26
<212>  DNA
<213>  Artificial

<220>
<223>  Synthetic construct, oligonucleotide useful as primer.

<400>  52
agcattttac cagatgaatg gctccc                                          26

<210>  53
<211>  27
<212>  DNA
<213>  Artificial

<220>
<223>  Synthetic construct, oligonucleotide useful as primer.

<400>  53
aacgaggaag ttctcattat cctcaac                                         27

<210>  54
<211>  44
<212>  DNA

<213>  Artificial

<220>
<223>  Synthetic construct; oligonucleotide useful as a primer.

<400>  54
agcctctccc tgtctccggg taaaagcatt ttaccagatg aatg                    44


<210>  55
<211>  42
<212>  DNA
<213>  Artificial

<220>
<223>  Synthetic construct:  oligonucleotide useful as a primer.

<400>  55
gggcgggcac gcgcatgtcc atgttgtgtg cgtaaagtag tc                      42


<210>  56
<211>  47
<212>  DNA
<213>  Artificial

<220>
<223>  Synthetic construct:  oligonucleotide useful as a primer.

<400>  56
agcctctccc tgtctccggg taaaaacagc attttaccag atgaatg                 47


<210>  57
<211>  45
<212>  DNA
<213>  Artificial

<220>
<223>  Synthetic construct:  oligonucleotide useful as a primer.

<400>  57
gggcgggcac gcgcatgtcc atactgttgt gtgcgtaaag tagtc                   45


<210>  58
<211>  53
<212>  DNA
<213>  Artificial

<220>
<223>  Synthetic construct:  oligonucleotide useful as a primer.

<400>  58
agcctctccc tgtctccggg taaattagca aacagcattt taccagatga atg          53


<210>  59
<211>  51
<212>  DNA
<213>  Artificial

<220>
<223>  Synthetic construct:  oligonucleotide useful as a primer.

<400>  59
gggcgggcac gcgcatgtcc atgtaataac tgttgtgtgc gtaaagtagt c            51

<210> 60
<211> 36
<212> DNA
<213> Artificial

<220>
<223> Synthetic construct: oligonucleotide useful as a primer.

<400> 60
tgcccgggcg ccaccatgga gtttgggctg agctgg 36


<210> 61
<211> 36
<212> DNA
<213> Artificial

<220>
<223> Synthetic construct: oligonucleotide useful as a primer.

<400> 61
tgcccgggcg ccaccatgga gtttgggctg agctgg 36


<210> 62
<211> 9460
<212> DNA
<213> Artificial

<220>
<223> Synthetic construct: sequence of plasmid pTT3-HcintLC-p.hori

<400> 62
gcggccgctc gaggccggca aggccggatc ccccgacctc gacctctggc taataaagga 60

aatttatttt cattgcaata gtgtgttgga attttttgtg tctctcactc ggaaggacat 120

atgggagggc aaatcatttg gtcgagatcc ctcggagatc tctagctaga ggatcgatcc 180

ccgccccgga cgaactaaac ctgactacga catctctgcc ccttcttcgc ggggcagtgc 240

atgtaatccc ttcagttggt tggtacaact tgccaactgg gccctgttcc acatgtgaca 300

cgggggggga ccaaacacaa aggggttctc tgactgtagt tgacatcctt ataaatggat 360

gtgcacattt gccaacactg agtggctttc atcctggagc agactttgca gtctgtggac 420

tgcaacacaa cattgccttt atgtgtaact cttggctgaa gctcttacac caatgctggg 480

ggacatgtac ctcccagggg cccaggaaga ctacgggagg ctacaccaac gtcaatcaga 540

ggggcctgtg tagctaccga taagcggacc ctcaagaggg cattagcaat agtgtttata 600

aggccccctt gttaacccta aacgggtagc atatgcttcc cgggtagtag tatatactat 660

ccagactaac cctaattcaa tagcatatgt tacccaacgg gaagcatatg ctatcgaatt 720

agggttagta aaagggtcct aaggaacagc gatatctccc accccatgag ctgtcacggt 780

tttatttaca tggggtcagg attccacgag ggtagtgaac cattttagtc acaagggcag 840

tggctgaaga tcaaggagcg ggcagtgaac tctcctgaat cttcgcctgc ttcttcattc 900

tccttcgttt agctaataga ataactgctg agttgtgaac agtaaggtgt atgtgaggtg 960

ctcgaaaaca aggtttcagg tgacgccccc agaataaaat ttggacgggg ggttcagtgg 1020

tggcattgtg ctatgacacc aatataaccc tcacaaaccc cttgggcaat aaatactagt 1080
gtaggaatga aacattctga atatctttaa caatagaaat ccatggggtg gggacaagcc 1140
gtaaagactg gatgtccatc tcacacgaat ttatggctat gggcaacaca taatcctagt 1200
gcaatatgat actggggtta ttaagatgtg tcccaggcag ggaccaagac aggtgaacca 1260
tgttgttaca ctctatttgt aacaagggga aagagagtgg acgccgacag cagcggactc 1320
cactggttgt ctctaacacc cccgaaaatt aaacggggct ccacgccaat ggggcccata 1380
aacaaagaca agtggccact cttttttttg aaattgtgga gtgggggcac gcgtcagccc 1440
ccacacgccg ccctgcggtt ttggactgta aaataagggt gtaataactt ggctgattgt 1500
aaccccgcta accactgcgg tcaaaccact tgcccacaaa accactaatg gcaccccggg 1560
gaatacctgc ataagtaggt gggcgggcca agataggggc gcgattgctg cgatctggag 1620
gacaaattac acacacttgc gcctgagcgc caagcacagg gttgttggtc ctcatattca 1680
cgaggtcgct gagagcacgg tgggctaatg ttgccatggg tagcatatac tacccaaata 1740
tctggatagc atatgctatc ctaatctata tctgggtagc ataggctatc ctaatctata 1800
tctgggtagc atatgctatc ctaatctata tctgggtagt atatgctatc ctaatttata 1860
tctgggtagc ataggctatc ctaatctata tctgggtagc atatgctatc ctaatctata 1920
tctgggtagt atatgctatc ctaatctgta tccgggtagc atatgctatc ctaatagaga 1980
ttagggtagt atatgctatc ctaatttata tctgggtagc atatactacc caaatatctg 2040
gatagcatat gctatcctaa tctatatctg ggtagcatat gctatcctaa tctatatctg 2100
ggtagcatag gctatcctaa tctatatctg ggtagcatat gctatcctaa tctatatctg 2160
ggtagtatat gctatcctaa tttatatctg ggtagcatag gctatcctaa tctatatctg 2220
ggtagcatat gctatcctaa tctatatctg ggtagtatat gctatcctaa tctgtatccg 2280
ggtagcatat gctatcctca tgataagctg tcaaacatga gaattttctt gaagacgaaa 2340
gggcctcgtg atacgcctat ttttataggt taatgtcatg ataataatgg tttcttagac 2400
gtcaggtggc acttttcggg gaaatgtgcg cggaacccct atttgtttat ttttctaaat 2460
acattcaaat atgtatccgc tcatgagaca ataaccctga taaatgcttc aataatattg 2520
aaaaaggaag agtatgagta ttcaacattt ccgtgtcgcc cttattccct tttttgcggc 2580
attttgcctt cctgtttttg ctcacccaga aacgctggtg aaagtaaaag atgctgaaga 2640
tcagttgggt gcacgagtgg gttacatcga actggatctc aacagcggta agatccttga 2700
gagttttcgc cccgaagaac gttttccaat gatgagcact tttaaagttc tgctatgtgg 2760
cgcggtatta tcccgtgttg acgccgggca agagcaactc ggtcgccgca tacactattc 2820
tcagaatgac ttggttgagt actcaccagt cacagaaaag catcttacgg atggcatgac 2880
agtaagagaa ttatgcagtg ctgccataac catgagtgat aacactgcgg ccaacttact 2940
tctgacaacg atcggaggac cgaaggagct aaccgctttt ttgcacaaca tgggggatca 3000
tgtaactcgc cttgatcgtt gggaaccgga gctgaatgaa gccataccaa acgacgagcg 3060

tgacaccacg atgcctgcag caatggcaac aacgttgcgc aaactattaa ctggcgaact 3120
acttactcta gcttcccggc aacaattaat agactggatg gaggcggata aagttgcagg 3180
accacttctg cgctcggccc ttccggctgg ctggtttatt gctgataaat ctggagccgg 3240
tgagcgtggg tctcgcggta tcattgcagc actggggcca gatggtaagc cctcccgtat 3300
cgtagttatc tacacgacgg ggagtcaggc aactatggat gaacgaaata gacagatcgc 3360
tgagataggt gcctcactga ttaagcattg gtaactgtca gaccaagttt actcatatat 3420
actttagatt gatttaaaac ttcattttta atttaaaagg atctaggtga agatcctttt 3480
tgataatctc atgaccaaaa tcccttaacg tgagttttcg ttccactgag cgtcagaccc 3540
cgtagaaaag atcaaaggat cttcttgaga tccttttttt ctgcgcgtaa tctgctgctt 3600
gcaaacaaaa aaaccaccgc taccagcggt ggtttgtttg ccggatcaag agctaccaac 3660
tctttttccg aaggtaactg gcttcagcag agcgcagata ccaaatactg ttcttctagt 3720
gtagccgtag ttaggccacc acttcaagaa ctctgtagca ccgcctacat acctcgctct 3780
gctaatcctg ttaccagtgg ctgctgccag tggcgataag tcgtgtctta ccgggttgga 3840
ctcaagacga tagttaccgg ataaggcgca gcggtcgggc tgaacggggg gttcgtgcac 3900
acagcccagc ttggagcgaa cgacctacac cgaactgaga tacctacagc gtgagctatg 3960
agaaagcgcc acgcttcccg aagggagaaa ggcggacagg tatccggtaa gcggcagggt 4020
cggaacagga gagcgcacga gggagcttcc agggggaaac gcctggtatc tttatagtcc 4080
tgtcgggttt cgccacctct gacttgagcg tcgatttttg tgatgctcgt caggggggcg 4140
gagcctatgg aaaaacgcca gcaacgcggc ctttttacgg ttcctggcct tttgctggcc 4200
ttttgctcac atgttctttc ctgcgttatc ccctgattct gtggataacc gtattaccgc 4260
ctttgagtga gctgataccg ctcgccgcag ccgaacgacc gagcgcagcg agtcagtgag 4320
cgaggaagcg gaagagcgcc caatacgcaa accgcctctc cccgcgcgtt ggccgattca 4380
ttaatgcagc tggcacgaca ggtttcccga ctggaaagcg ggcagtgagc gcaacgcaat 4440
taatgtgagt tagctcactc attaggcacc ccaggcttta cactttatgc ttccggctcg 4500
tatgttgtgt ggaattgtga gcggataaca atttcacaca ggaaacagct atgaccatga 4560
ttacgccaag ctctagctag aggtcgacca attctcatgt ttgacagctt atcatcgcag 4620
atccgggcaa cgttgttgcc attgctgcag gcgcagaact ggtaggtatg gaagatctat 4680
acattgaatc aatattggca attagccata ttagtcattg gttatatagc ataaatcaat 4740
attggctatt ggccattgca tacgttgtat ctatatcata atatgtacat ttatattggc 4800
tcatgtccaa tatgaccgcc atgttgacat tgattattga ctagttatta atagtaatca 4860
attacggggt cattagttca tagcccatat atggagttcc gcgttacata acttacggta 4920
aatggcccgc ctggctgacc gcccaacgac ccccgcccat tgacgtcaat aatgacgtat 4980
gttcccatag taacgccaat agggactttc cattgacgtc aatgggtgga gtatttacgg 5040
taaactgccc acttggcagt acatcaagtg tatcatatgc caagtccgcc ccctattgac 5100

gtcaatgacg gtaaatggcc cgcctggcat tatgcccagt acatgacctt acgggacttt 5160
cctacttggc agtacatcta cgtattagtc atcgctatta ccatggtgat gcggttttgg 5220
cagtacacca atgggcgtgg atagcggttt gactcacggg gatttccaag tctccacccc 5280
attgacgtca atgggagttt gttttggcac caaaatcaac gggactttcc aaaatgtcgt 5340
aataaccccg ccccgttgac gcaaatgggc ggtaggcgtg tacggtggga ggtctatata 5400
agcagagctc gtttagtgaa ccgtcagatc ctcactctct tccgcatcgc tgtctgcgag 5460
ggccagctgt tgggctcgcg gttgaggaca aactcttcgc ggtctttcca gtactcttgg 5520
atcggaaacc cgtcggcctc cgaacggtac tccgccaccg agggacctga gcgagtccgc 5580
atcgaccgga tcggaaaacc tctcgagaaa ggcgtctaac cagtcacagt cgcaaggtag 5640
gctgagcacc gtggcgggcg gcagcgggtg gcggtcgggg ttgtttctgg cggaggtgct 5700
gctgatgatg taattaaagt aggcggtctt gagacggcgg atggtcgagg tgaggtgtgg 5760
caggcttgag atccagctgt tggggtgagt actccctctc aaaagcgggc attacttctg 5820
cgctaagatt gtcagtttcc aaaaacgagg aggatttgat attcacctgg cccgatctgg 5880
ccatacactt gagtgacaat gacatccact ttgcctttct ctccacaggt gtccactccc 5940
aggtccaagt ttgggcgcca ccatggagtt tgggctgagc tggctttttc ttgtcgcgat 6000
tttaaaaggt gtccagtgtg aggtgcagct ggtggagtct gggggaggct tggtacagcc 6060
cggcaggtcc ctgagactct cctgtgcggc ctctggattc acctttgatg attatgccat 6120
gcactgggtc cggcaagctc cagggaaggg cctggaatgg gtctcagcta tcacttggaa 6180
tagtggtcac atagactatg cggactctgt ggagggccga ttcaccatct ccagagacaa 6240
cgccaagaac tccctgtatc tgcaaatgaa cagtctgaga gctgaggata cggccgtata 6300
ttactgtgcg aaagtctcgt accttagcac cgcgtcctcc cttgactatt ggggccaagg 6360
taccctggtc accgtctcga gtgcgtcgac caagggccca tcggtcttcc ccctggcacc 6420
ctcctccaag agcacctctg ggggcacagc ggccctgggc tgcctggtca aggactactt 6480
ccccgaaccg gtgacggtgt cgtggaactc aggcgccctg accagcggcg tgcacacctt 6540
cccggctgtc ctacagtcct caggactcta ctccctcagc agcgtggtga ccgtgccctc 6600
cagcagcttg ggcacccaga cctacatctg caacgtgaat cacaagccca gcaacaccaa 6660
ggtggacaag aaagttgagc ccaaatcttg tgacaaaact cacacatgcc caccgtgccc 6720
agcacctgaa ctcctggggg gaccgtcagt cttcctcttc cccccaaaac ccaaggacac 6780
cctcatgatc tcccggaccc ctgaggtcac atgcgtggtg gtggacgtga gccacgaaga 6840
ccctgaggtc aagttcaact ggtacgtgga cggcgtggag gtgcataatg ccaagacaaa 6900
gccgcgggag gagcagtaca acagcacgta ccgtgtggtc agcgtcctca ccgtcctgca 6960
ccaggactgg ctgaatggca aggagtacaa gtgcaaggtc tccaacaaag ccctcccagc 7020
ccccatcgag aaaaccatct ccaaagccaa agggcagccc cgagaaccac aggtgtacac 7080
cctgccccca tcccgggatg agctgaccaa gaaccaggtc agcctgacct gcctggtcaa 7140

aggcttctat cccagcgaca tcgccgtgga gtgggagagc aatgggcagc cggagaacaa 7200
ctacaagacc acgcctcccg tgctggactc cgacggctcc ttcttcctct acagcaagct 7260
caccgtggac aagagcaggt ggcagcaggg gaacgtcttc tcatgctccg tgatgcatga 7320
ggctctgcac aaccactaca cgcagaagag cctctccctg tctccgggta aaagcatttt 7380
accagatgaa tggctcccaa ttgttgaaaa tgaaaaagtt cgattcgtaa aaattggaga 7440
cttcatagat agggagattg aggaaaacgc tgagagagtg aagagggatg gtgaaactga 7500
aattctagag gttaaagatc ttaaagccct ttccttcaat agagaaacaa aaaagagcga 7560
gctcaagaag gtaaaggccc taattagaca ccgctattca gggaaggttt acagcattaa 7620
actaaagtca gggagaagga tcaaaataac ctcaggtcat agtctgttct cagtaaaaaa 7680
tggaaagcta gttaaggtca ggggagatga actcaagcct ggtgatctcg ttgtcgttcc 7740
aggaaggtta aaacttccag aaagcaagca agtgctaaat ctcgttgaac tactcctgaa 7800
attacccgaa gaggagacat cgaacatcgt aatgatgatc ccagttaaag gtagaaagaa 7860
tttcttcaaa gggatgctca aaacattata ctggatcttc ggggagggag aaaggccaag 7920
aaccgcaggg cgctatctca agcatcttga aagattagga tacgttaagc tcaagagaag 7980
aggctgtgaa gttctcgact gggagtcact taagaggtac aggaagcttt acgagaccct 8040
cattaagaac ctgaaatata acggtaatag cagggcatac atggttgaat ttaactctct 8100
cagggatgta gtgagcttaa tgccaataga agaacttaag gagtggataa ttggagaacc 8160
taggggtcct aagataggta ccttcattga tgtagatgat tcatttgcaa agctcctagg 8220
ttactacata agtagcggag atgtagagaa agatagggtg aagttccaca gtaaagatca 8280
aaacgttctc gaggatatag cgaaacttgc cgagaagtta tttggaaagg tgaggagagg 8340
aagaggatat attgaggtat cagggaaaat tagccatgcc atatttagag ttttagcgga 8400
aggtaagaga attccagagt tcatcttcac atccccaatg gatattaagg tagccttcct 8460
taagggactc aacggtaatg ctgaagaatt aacgttctcc actaagagtg agctattagt 8520
taaccagctt atccttctcc tgaactccat tggagtttcg gatataaaga ttgaacatga 8580
gaaaggggtt tacagagttt acataaataa gaaggaatcc tccaatgggg atatagtact 8640
tgatagcgtc gaatctatcg aagttgaaaa atacgagggc tacgtttatg atctaagtgt 8700
tgaggataat gagaacttcc tcgttggctt cggactactt tacgcacaca acatggacat 8760
gcgcgtgccc gcccagctgc tgggcctgct gctgctgtgg ttccccggct cgcgatgcga 8820
catccagatg acccagtctc catcctccct gtctgcatct gtaggggaca gagtcaccat 8880
cacttgtcgg gcaagtcagg gcatcagaaa ttacttagcc tggtatcagc aaaaaccagg 8940
gaaagcccct aagctcctga tctatgctgc atccactttg caatcagggg tcccatctcg 9000
gttcagtggc agtggatctg ggacagattt cactctcacc atcagcagcc tacagcctga 9060
agatgttgca acttattact gtcaaaggta taaccgtgca ccgtatactt ttggccaggg 9120
gaccaaggtg gaaatcaaac gtacggtggc tgcaccatct gtcttcatct tcccgccatc 9180

tgatgagcag ttgaaatctg gaactgcctc tgttgtgtgc ctgctgaata acttctatcc 9240

cagagaggcc aaagtacagt ggaaggtgga taacgccctc caatcgggta actcccagga 9300

gagtgtcaca gagcaggaca gcaaggacag cacctacagc ctcagcagca ccctgacgct 9360

gagcaaagca gactacgaga aacacaaagt ctacgcctgc gaagtcaccc atcagggcct 9420

gagctcgccc gtcacaaaga gcttcaacag gggagagtgt 9460

<210> 63
<211> 1166
<212> PRT
<213> Artificial

<220>
<223> Synthetic: amino Acid Sequence of the open reading frame in
pTT3-HcintLC-p.hori

<400> 63

<210> 64
<211> 1404
<212> DNA
<213> Artificial

<220>
<223> Synthetic construct: partial coding sequence from
pTT3-HcintLC1aa-p.hori

<400> 64
ccgggtaaaa acagcatttt accagatgaa tggctcccaa ttgttgaaaa tgaaaaagtt 60

cgattcgtaa aaattggaga cttcatagat agggagattg aggaaaacgc tgagagagtg 120

aagagggatg gtgaaactga aattctagag gttaaagatc ttaaagccct ttccttcaat 180

agagaaacaa aaaagagcga gctcaagaag gtaaaggccc taattagaca ccgctattca 240

gggaaggttt acagcattaa actaaagtca gggagaagga tcaaaataac ctcaggtcat 300

agtctgttct cagtaaaaaa tggaaagcta gttaaggtca ggggagatga actcaagcct 360

ggtgatctcg ttgtcgttcc aggaaggtta aaacttccag aaagcaagca agtgctaaat 420

ctcgttgaac tactcctgaa attacccgaa gaggagacat cgaacatcgt aatgatgatc 480

ccagttaaag gtagaaagaa tttcttcaaa gggatgctca aaacattata ctggatcttc 540

ggggagggag aaaggccaag aaccgcaggg cgctatctca agcatcttga aagattagga 600

tacgttaagc tcaagagaag aggctgtgaa gttctcgact gggagtcact taagaggtac 660

aggaagcttt acgagaccct cattaagaac ctgaaatata acggtaatag cagggcatac 720

atggttgaat ttaactctct cagggatgta gtgagcttaa tgccaataga agaacttaag 780

gagtggataa ttggagaacc taggggtcct aagataggta ccttcattga tgtagatgat 840

tcatttgcaa agctcctagg ttactacata agtagcggag atgtagagaa agatagggtg 900

aagttccaca gtaaagatca aaacgttctc gaggatatag cgaaacttgc cgagaagtta 960

tttggaaagg tgaggagagg aagaggatat attgaggtat cagggaaaat tagccatgcc 1020

atatttagag ttttagcgga aggtaagaga attccagagt tcatcttcac atccccaatg 1080

gatattaagg tagccttcct taagggactc aacggtaatg ctgaagaatt aacgttctcc 1140

actaagagtg agctattagt taaccagctt atccttctcc tgaactccat tggagtttcg 1200

gatataaaga ttgaacatga gaaaggggtt tacagagttt acataaataa gaaggaatcc 1260

tccaatgggg atatagtact tgatagcgtc gaatctatcg aagttgaaaa atacgagggc 1320

tacgtttatg atctaagtgt tgaggataat gagaacttcc tcgttggctt cggactactt 1380

tacgcacaca acagtatgga catg 1404

<210> 65
<211> 468
<212> PRT
<213> Artificial

<220>
<223> Synthetic: partial amino acid sequence from
pTT3-HcintLC1aa-p.hori, showing 4 amino acids upstream of the
heavy chain and r amino acids downstream of the intein.

<400> 65

Pro Gly Lys Asn Ser Ile Leu Pro Asp Glu Trp Leu Pro Ile Val Glu
1               5                   10                  15

Asn Glu Lys Val Arg Phe Val Lys Ile Gly Asp Phe Ile Asp Arg Glu
            20                  25                  30

Ile Glu Glu Asn Ala Glu Arg Val Lys Arg Asp Gly Glu Thr Glu Ile
        35                  40                  45

Leu Glu Val Lys Asp Leu Lys Ala Leu Ser Phe Asn Arg Glu Thr Lys
    50                  55                  60

Lys Ser Glu Leu Lys Lys Val Lys Ala Leu Ile Arg His Arg Tyr Ser
65                  70                  75                  80

Gly Lys Val Tyr Ser Ile Lys Leu Lys Ser Gly Arg Arg Ile Lys Ile
                85                  90                  95

Thr Ser Gly His Ser Leu Phe Ser Val Lys Asn Gly Lys Leu Val Lys
            100                 105                 110

Val Arg Gly Asp Glu Leu Lys Pro Gly Asp Leu Val Val Val Pro Gly
        115                 120                 125

Arg Leu Lys Leu Pro Glu Ser Lys Gln Val Leu Asn Leu Val Glu Leu
    130                 135                 140

Leu Leu Lys Leu Pro Glu Glu Glu Thr Ser Asn Ile Val Met Met Ile
145                 150                 155                 160

Pro Val Lys Gly Arg Lys Asn Phe Phe Lys Gly Met Leu Lys Thr Leu
                165                 170                 175

Tyr Trp Ile Phe Gly Glu Gly Glu Arg Pro Arg Thr Ala Gly Arg Tyr
            180                 185                 190

Leu Lys His Leu Glu Arg Leu Gly Tyr Val Lys Leu Lys Arg Arg Gly
        195                 200                 205

Cys Glu Val Leu Asp Trp Glu Ser Leu Lys Arg Tyr Arg Lys Leu Tyr
    210                 215                 220

Glu Thr Leu Ile Lys Asn Leu Lys Tyr Asn Gly Asn Ser Arg Ala Tyr
225                 230                 235                 240

Met Val Glu Phe Asn Ser Leu Arg Asp Val Val Ser Leu Met Pro Ile
                245                 250                 255

Glu Glu Leu Lys Glu Trp Ile Ile Gly Glu Pro Arg Gly Pro Lys Ile
            260                 265                 270

Gly Thr Phe Ile Asp Val Asp Asp Ser Phe Ala Lys Leu Leu Gly Tyr
        275                 280                 285

Tyr Ile Ser Ser Gly Asp Val Glu Lys Asp Arg Val Lys Phe His Ser
    290                 295                 300

Lys Asp Gln Asn Val Leu Glu Asp Ile Ala Lys Leu Ala Glu Lys Leu
305                 310                 315                 320

Phe Gly Lys Val Arg Arg Gly Arg Gly Tyr Ile Glu Val Ser Gly Lys
                325                 330                 335

Ile Ser His Ala Ile Phe Arg Val Leu Ala Glu Gly Lys Arg Ile Pro
            340                 345                 350

Glu Phe Ile Phe Thr Ser Pro Met Asp Ile Lys Val Ala Phe Leu Lys
        355                 360                 365

Gly Leu Asn Gly Asn Ala Glu Glu Leu Thr Phe Ser Thr Lys Ser Glu
    370                 375                 380

Leu Leu Val Asn Gln Leu Ile Leu Leu Leu Asn Ser Ile Gly Val Ser
385                 390                 395                 400

Asp Ile Lys Ile Glu His Glu Lys Gly Val Tyr Arg Val Tyr Ile Asn
                405                 410                 415

Lys Lys Glu Ser Ser Asn Gly Asp Ile Val Leu Asp Ser Val Glu Ser
            420                 425                 430

Ile Glu Val Glu Lys Tyr Glu Gly Tyr Val Tyr Asp Leu Ser Val Glu
        435                 440                 445

Asp Asn Glu Asn Phe Leu Val Gly Phe Gly Leu Leu Tyr Ala His Asn
    450                 455                 460

Ser Met Asp Met
465

<210>  66
<211>  1416
<212>  DNA
<213>  Artificial

<220>
<223>  Synthetic construct:  pTT3-HcintLC3aa-p.hori partial coding
       sequence.

<400>  66
ccgggtaaat tagcaaacag cattttacca gatgaatggc tcccaattgt tgaaaatgaa      60

aaagttcgat tcgtaaaaat tggagacttc atagataggg agattgagga aaacgctgag     120

agagtgaaga gggatggtga aactgaaatt ctagaggtta aagatcttaa agccctttcc     180

ttcaatagag aaacaaaaaa gagcgagctc aagaaggtaa aggccctaat tagacaccgc     240

tattcaggga aggtttacag cattaaacta aagtcaggga gaaggatcaa aataacctca     300

ggtcatagtc tgttctcagt aaaaaatgga aagctagtta aggtcagggg agatgaactc     360

aagcctggtg atctcgttgt cgttccagga aggttaaaac ttccagaaag caagcaagtg     420

ctaaatctcg ttgaactact cctgaaatta cccgaagagg agacatcgaa catcgtaatg     480

atgatcccag ttaaaggtag aaagaatttc ttcaaaggga tgctcaaaac attatactgg     540

atcttcgggg agggagaaag gccaagaacc gcagggcgct atctcaagca tcttgaaaga     600

ttaggatacg ttaagctcaa gagaagaggc tgtgaagttc tcgactggga gtcacttaag     660

aggtacagga agctttacga gaccctcatt aagaacctga aatataacgg taatagcagg     720

gcatacatgg ttgaatttaa ctctctcagg gatgtagtga gcttaatgcc aatagaagaa     780

cttaaggagt ggataattgg agaacctagg ggtcctaaga taggtacctt cattgatgta 840

gatgattcat ttgcaaagct cctaggttac tacataagta gcggagatgt agagaaagat 900

agggtgaagt tccacagtaa agatcaaaac gttctcgagg atatagcgaa acttgccgag 960

aagttatttg gaaaggtgag gagaggaaga ggatatattg aggtatcagg gaaaattagc 1020

catgccatat ttagagtttt agcggaaggt aagagaattc cagagttcat cttcacatcc 1080

ccaatggata ttaaggtagc cttccttaag ggactcaacg gtaatgctga agaattaacg 1140

ttctccacta agagtgagct attagttaac cagcttatcc ttctcctgaa ctccattgga 1200

gtttcggata taaagattga acatgagaaa ggggtttaca gagtttacat aaataagaag 1260

gaatcctcca atggggatat agtacttgat agcgtcgaat ctatcgaagt tgaaaaatac 1320

gagggctacg tttatgatct aagtgttgag gataatgaga acttcctcgt tggcttcgga 1380

ctactttacg cacacaacag ttattacatg gacatg 1416

<210> 67
<211> 472
<212> PRT
<213> Artificial

<220>
<223> Synthetic: pTT3-HcintLC3aa-p.hori partial amino acid sequence
showing intein and flanking sequences.

<400> 67

Pro Gly Lys Leu Ala Asn Ser Ile Leu Pro Asp Glu Trp Leu Pro Ile
1               5                   10                  15

Val Glu Asn Glu Lys Val Arg Phe Val Lys Ile Gly Asp Phe Ile Asp
            20                  25                  30

Arg Glu Ile Glu Glu Asn Ala Glu Arg Val Lys Arg Asp Gly Glu Thr
        35                  40                  45

Glu Ile Leu Glu Val Lys Asp Leu Lys Ala Leu Ser Phe Asn Arg Glu
    50                  55                  60

Thr Lys Lys Ser Glu Leu Lys Lys Val Lys Ala Leu Ile Arg His Arg
65                  70                  75                  80

Tyr Ser Gly Lys Val Tyr Ser Ile Lys Leu Lys Ser Gly Arg Arg Ile
                85                  90                  95

Lys Ile Thr Ser Gly His Ser Leu Phe Ser Val Lys Asn Gly Lys Leu
            100                 105                 110

Val Lys Val Arg Gly Asp Glu Leu Lys Pro Gly Asp Leu Val Val Val
        115                 120                 125

Pro Gly Arg Leu Lys Leu Pro Glu Ser Lys Gln Val Leu Asn Leu Val

130                 135                 140

Glu Leu Leu Leu Lys Leu Pro Glu Glu Glu Thr Ser Asn Ile Val Met
145                 150                 155                 160

Met Ile Pro Val Lys Gly Arg Lys Asn Phe Phe Lys Gly Met Leu Lys
                165                 170                 175

Thr Leu Tyr Trp Ile Phe Gly Glu Gly Glu Arg Pro Arg Thr Ala Gly
            180                 185                 190

Arg Tyr Leu Lys His Leu Glu Arg Leu Gly Tyr Val Lys Leu Lys Arg
        195                 200                 205

Arg Gly Cys Glu Val Leu Asp Trp Glu Ser Leu Lys Arg Tyr Arg Lys
    210                 215                 220

Leu Tyr Glu Thr Leu Ile Lys Asn Leu Lys Tyr Asn Gly Asn Ser Arg
225                 230                 235                 240

Ala Tyr Met Val Glu Phe Asn Ser Leu Arg Asp Val Val Ser Leu Met
                245                 250                 255

Pro Ile Glu Glu Leu Lys Glu Trp Ile Ile Gly Glu Pro Arg Gly Pro
            260                 265                 270

Lys Ile Gly Thr Phe Ile Asp Val Asp Asp Ser Phe Ala Lys Leu Leu
        275                 280                 285

Gly Tyr Tyr Ile Ser Ser Gly Asp Val Glu Lys Asp Arg Val Lys Phe
    290                 295                 300

His Ser Lys Asp Gln Asn Val Leu Glu Asp Ile Ala Lys Leu Ala Glu
305                 310                 315                 320

Lys Leu Phe Gly Lys Val Arg Arg Gly Arg Gly Tyr Ile Glu Val Ser
                325                 330                 335

Gly Lys Ile Ser His Ala Ile Phe Arg Val Leu Ala Glu Gly Lys Arg
            340                 345                 350

Ile Pro Glu Phe Ile Phe Thr Ser Pro Met Asp Ile Lys Val Ala Phe
        355                 360                 365

Leu Lys Gly Leu Asn Gly Asn Ala Glu Glu Leu Thr Phe Ser Thr Lys
    370                 375                 380

Ser Glu Leu Leu Val Asn Gln Leu Ile Leu Leu Leu Asn Ser Ile Gly
385                 390                 395                 400

Val Ser Asp Ile Lys Ile Glu His Glu Lys Gly Val Tyr Arg Val Tyr

405                 410                 415

Ile Asn Lys Lys Glu Ser Ser Asn Gly Asp Ile Val Leu Asp Ser Val
            420                 425                 430

Glu Ser Ile Glu Val Glu Lys Tyr Glu Gly Tyr Val Tyr Asp Leu Ser
        435                 440                 445

Val Glu Asp Asn Glu Asn Phe Leu Val Gly Phe Gly Leu Leu Tyr Ala
    450                 455                 460

His Asn Ser Tyr Tyr Met Asp Met
465                 470

<210>  68
<211>  31
<212>  DNA
<213>  Artificial

<220>
<223>  Synthetic construct:  oligonucleotide useful as a primer.

<400>  68
ggactacttt acgcagccaa catggacatg c                                    31

<210>  69
<211>  31
<212>  DNA
<213>  Artificial

<220>
<223>  Synthetic construct:  oligonucleotide useful as a primer.

<400>  69
gcatgtccat gttggctgcg taaagtagtc c                                    31

<210>  70
<211>  34
<212>  DNA
<213>  Artificial

<220>
<223>  Synthetic construct:  oligonucleotide useful as a primer.

<400>  70
ggactacttt acgcagccaa cagtatggac atgc                                 34

<210>  71
<211>  34
<212>  DNA
<213>  Artificial

<220>
<223>  Synthetic construct:  oligonucleotide useful as a primer.

<400>  71
gcatgtccat actgttggct gcgtaaagta gtcc                                 34

<210>  72

<211>  18
<212>  DNA
<213>  Artificial

<220>
<223>  Synthetic  construct:  oligonucleotide useful as a primer.

<400>  72
ggtgaggaga ggaagagg                                                    18


<210>  73
<211>  16
<212>  DNA
<213>  Artificial

<220>
<223>  Synthetic construct:  oligonucleotide useful as a primer.

<400>  73
ccagaggtcg aggtcg                                                      16


<210>  74
<211>  14
<212>  DNA
<213>  Artificial

<220>
<223>  Synthetic construct:  oligonucleotide useful as a primer.

<400>  74
cggcgtggag gtgc                                                        14


<210>  75
<211>  45
<212>  DNA
<213>  Artificial

<220>
<223>  Synthetic construct:  oligonucleotide useful as a primer.

<400>  75
caacaattgg gagccattca tctggtaaaa tggttttacc cggag                      45


<210>  76
<211>  40
<212>  DNA
<213>  Artificial

<220>
<223>  Synthetic construct:  oligonucleotide useful as a primer.

<400>  76
ccgcccagct gctgggcgac gagtggttcc ccggctcgcg                            40


<210>  77
<211>  40
<212>  DNA
<213>  Artificial

<220>
<223>  Synthetic construct:  oligonucleotide useful as a primer.

<400>  77

cgcgagccgg ggaaccactc gtcgcccagc agctgggcgg                                40


<210>  78
<211>  15
<212>  DNA
<213>  Artificial

<220>
<223>  Synthetic construct:  oligonucleotide useful as a primer.

<400>  78
tgagcggccg ctcga                                                          15


<210>  79
<211>  15
<212>  DNA
<213>  Artificial

<220>
<223>  Synthetic construct:  oligonucleotide useful as a primer.

<400>  79
gttgtgtgcg taaag                                                          15


<210>  80
<211>  15
<212>  DNA
<213>  Artificial

<220>
<223>  Synthetic construct:  oligonucleotide useful as a primer.

<400>  80
agcattttac cagat                                                          15


<210>  81
<211>  15
<212>  DNA
<213>  Artificial

<220>
<223>  Synthetic construct:  oligonucleotide useful as a primer.

<400>  81
ggtggcgccc aaact                                                          15


<210>  82
<211>  30
<212>  DNA
<213>  Artificial

<220>
<223>  Synthetic construct:  oligonucleotide useful as a primer.

<400>  82
ctttacgcac acaacatgga catgcgcgtg                                          30


<210>  83
<211>  27
<212>  DNA
<213>  Artificial

<220>
<223>  Synthetic construct:  oligonucleotide useful as a primer.

<400>  83
tcgagcggcc gctcaacact ctcccct                                          27


<210>  84
<211>  30
<212>  DNA
<213>  Artificial

<220>
<223>  Synthetic construct:  oligonucleotide useful as a primer.

<400>  84
agtttgggcg ccaccatgga gtttgggctg                                       30


<210>  85
<211>  30
<212>  DNA
<213>  Artificial

<220>
<223>  Synthetic construct:  oligonucleotide useful as a primer.

<400>  85
atctggtaaa atgcttttac ccggagacag                                       30


<210>  86
<211>  30
<212>  DNA
<213>  Artificial

<220>
<223>  Synthetic construct:  oligonucleotide useful as a primer.

<400>  86
agtttgggcg ccaccatgga catgcgcgtg                                       30


<210>  87
<211>  31
<212>  DNA
<213>  Artificial

<220>
<223>  Synthetic construct:  oligonucleotide useful as a primer.

<400>  87
atctggtaaa atgctacact ctcccctgtt g                                     31


<210>  88
<211>  30
<212>  DNA
<213>  Artificial

<220>
<223>  Synthetic construct:  oligonucleotide useful as a primer.

<400>  88
ctttacgcac acaacatgga gtttgggctg                                       30


<210>  89

<211>  30
<212>  DNA
<213>  Artificial

<220>
<223>  Synthetic construct:  oligonucleotide useful as a primer.

<400>  89
tcgagcggcc gctcatttac ccggagacag                                        30


<210>  90
<211>  14
<212>  DNA
<213>  Artificial

<220>
<223>  Synthetic construct:  oligonucleotide useful as a primer.

<400>  90
cgccaagctc tagc                                                         14


<210>  91
<211>  14
<212>  DNA
<213>  Artificial

<220>
<223>  Synthetic construct:  oligonucleotide useful as a primer.

<400>  91
ggtcgaggtc gggg                                                         14


<210>  92
<211>  40
<212>  DNA
<213>  Artificial

<220>
<223>  Synthetic construct:  oligonucleotide useful as a primer.

<400>  92
acatgcgcgt gcccgcccag tggttccccg gctcgcgatg                             40


<210>  93
<211>  40
<212>  DNA
<213>  Artificial

<220>
<223>  Synthetic construct:  oligonucleotide useful as a primer.

<400>  93
catcgcgagc cggggaacca ctgggcgggc acgcgcatgt                             40


<210>  94
<211>  30
<212>  DNA
<213>  Artificial

<220>
<223>  Synthetic construct:  oligonucleotide useful as a primer.

<400>  94

ctttacgcac acaacgacat ccagatgacc 30

<210> 95
<211> 30
<212> DNA
<213> Artificial

<220>
<223> Synthetic construct: oligonucleotide useful as a primer.

<400> 95
ggtcatctgg atgtcgttgt gtgcgtaaag 30

<210> 96
<211> 36
<212> DNA
<213> Artificial

<220>
<223> Synthetic construct: oligonucleotide useful as a primer.

<400> 96
tggttccccg gctcgggagg cgacatccag atgacc 36

<210> 97
<211> 36
<212> DNA
<213> Artificial

<220>
<223> Synthetic construct: oligonucleotide useful as a primer.

<400> 97
ggtcatctgg atgtcgcctc ccgagccggg gaacca 36

<210> 98
<211> 1464
<212> DNA
<213> Artificial

<220>
<223> Synthetic construct: partial coding sequence of Construct A.

<400> 98
ccgggtaaaa gcattttacc agatgaatgg ctcccaattg ttgaaaatga aaaagttcga 60

ttcgtaaaaa ttggagactt catagatagg gagattgagg aaaacgctga gagagtgaag 120

agggatggtg aaactgaaat tctagaggtt aaagatctta aagccctttc cttcaataga 180

gaaacaaaaa agagcgagct caagaaggta aaggccctaa ttagacaccg ctattcaggg 240

aaggtttaca gcattaaact aaagtcaggg agaaggatca aaataacctc aggtcatagt 300

ctgttctcag taaaaaatgg aaagctagtt aaggtcaggg gagatgaact caagcctggt 360

gatctcgttg tcgttccagg aaggttaaaa cttccagaaa gcaagcaagt gctaaatctc 420

gttgaactac tcctgaaatt acccgaagag gagacatcga acatcgtaat gatgatccca 480

gttaaaggta gaaagaattt cttcaaaggg atgctcaaaa cattatactg gatcttcggg 540

gagggagaaa ggccaagaac cgcagggcgc tatctcaagc atcttgaaag attaggatac 600

gttaagctca agagaagagg ctgtgaagtt ctcgactggg agtcacttaa gaggtacagg 660
aagctttacg agaccctcat taagaacctg aaatataacg gtaatagcag ggcatacatg 720
gttgaattta actctctcag ggatgtagtg agcttaatgc caatagaaga acttaaggag 780
tggataattg gagaacctag gggtcctaag ataggtacct tcattgatgt agatgattca 840
tttgcaaagc tcctaggtta ctacataagt agcggagatg tagagaaaga tagggtgaag 900
ttccacagta aagatcaaaa cgttctcgag gatatagcga aacttgccga gaagttattt 960
ggaaaggtga ggagaggaag aggatatatt gaggtatcag ggaaaattag ccatgccata 1020
tttagagttt tagcggaagg taagagaatt ccagagttca tcttcacatc cccaatggat 1080
attaaggtag ccttccttaa gggactcaac ggtaatgctg aagaattaac gttctccact 1140
aagagtgagc tattagttaa ccagcttatc cttctcctga actccattgg agtttcggat 1200
ataaagattg aacatgagaa aggggtttac agagtttaca taaataagaa ggaatcctcc 1260
aatggggata tagtacttga tagcgtcgaa tctatcgaag ttgaaaaata cgagggctac 1320
gtttatgatc taagtgttga ggataatgag aacttcctcg ttggcttcgg actactttac 1380
gcagccaaca tggacatgcg cgtgcccgcc cagctgctgg gcctgctgct gctgtggttc 1440
cccggctcgc gatgcgacat ccag 1464

<210> 99
<211> 488
<212> PRT
<213> Artificial

<220>
<223> Synthetic: partial amino acid sequence of Construct A.

<400> 99

Pro Gly Lys Ser Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr
                85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro
145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly
        355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile
            420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala Ala Asn Met
    450                 455                 460

Asp Met Arg Val Pro Ala Gln Leu Leu Gly Leu Leu Leu Leu Trp Phe
465                 470                 475                 480

Pro Gly Ser Arg Cys Asp Ile Gln
                485

<210>  100
<211>  1467
<212>  DNA
<213>  Artificial

<220>
<223>  Synthetic construct:  partial coding sequence of construct B.

<400>  100
ccgggtaaaa gcattttacc agatgaatgg ctcccaattg ttgaaaatga aaaagttcga      60

ttcgtaaaaa ttggagactt catagatagg gagattgagg aaaacgctga gagagtgaag     120

agggatggtg aaactgaaat tctagaggtt aaagatctta aagccctttc cttcaataga     180

gaaacaaaaa agagcgagct caagaaggta aaggccctaa ttagacaccg ctattcaggg     240

aaggtttaca gcattaaact aaagtcaggg agaaggatca aaataacctc aggtcatagt     300

ctgttctcag taaaaaatgg aaagctagtt aaggtcaggg gagatgaact caagcctggt     360

gatctcgttg tcgttccagg aaggttaaaa cttccagaaa gcaagcaagt gctaaatctc     420

gttgaactac tcctgaaatt acccgaagag gagacatcga acatcgtaat gatgatccca     480

gttaaaggta gaaagaattt cttcaaaggg atgctcaaaa cattatactg gatcttcggg     540

gagggagaaa ggccaagaac cgcagggcgc tatctcaagc atcttgaaag attaggatac     600

gttaagctca agagaagagg ctgtgaagtt ctcgactggg agtcacttaa gaggtacagg     660

aagctttacg agaccctcat taagaacctg aaatataacg gtaatagcag ggcatacatg     720

gttgaattta actctctcag ggatgtagtg agcttaatgc caatagaaga acttaaggag     780

tggataattg gagaacctag gggtcctaag ataggtacct tcattgatgt agatgattca     840

tttgcaaagc tcctaggtta ctacataagt agcggagatg tagagaaaga tagggtgaag     900

ttccacagta aagatcaaaa cgttctcgag gatatagcga aacttgccga gaagttattt 960

ggaaaggtga ggagaggaag aggatatatt gaggtatcag ggaaaattag ccatgccata 1020

tttagagttt tagcggaagg taagagaatt ccagagttca tcttcacatc cccaatggat 1080

attaaggtag ccttccttaa gggactcaac ggtaatgctg aagaattaac gttctccact 1140

aagagtgagc tattagttaa ccagcttatc cttctcctga actccattgg agtttcggat 1200

ataaagattg aacatgagaa aggggtttac agagtttaca taaataagaa ggaatcctcc 1260

aatggggata tagtacttga tagcgtcgaa tctatcgaag ttgaaaaata cgagggctac 1320

gtttatgatc taagtgttga ggataatgag aacttcctcg ttggcttcgg actactttac 1380

gcagccaaca gtatggacat gcgcgtgccc gcccagctgc tgggcctgct gctgctgtgg 1440

ttccccggct cgcgatgcga catccag 1467

<210> 101
<211> 489
<212> PRT
<213> Artificial

<220>
<223> Synthetic construct: partial amino acid sequence of construct A.

<400> 101

Pro Gly Lys Ser Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr
                85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro

145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly
        355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile

420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala Ala Asn Ser
    450                 455                 460

Met Asp Met Arg Val Pro Ala Gln Leu Leu Gly Leu Leu Leu Leu Trp
465                 470                 475                 480

Phe Pro Gly Ser Arg Cys Asp Ile Gln
                485

<210>  102
<211>  1467
<212>  DNA
<213>  Artificial

<220>
<223>  Synthetic construct:  partial coding sequence in construct E.

<400>  102
ccgggtaaaa ccattttacc agatgaatgg ctcccaattg ttgaaaatga aaaagttcga      60

ttcgtaaaaa ttggagactt catagatagg gagattgagg aaaacgctga gagagtgaag     120

agggatggtg aaactgaaat tctagaggtt aaagatctta aagccctttc cttcaataga     180

gaaacaaaaa agagcgagct caagaaggta aaggccctaa ttagacaccg ctattcaggg     240

aaggtttaca gcattaaact aaagtcaggg agaaggatca aaataacctc aggtcatagt     300

ctgttctcag taaaaaatgg aaagctagtt aaggtcaggg gagatgaact caagcctggt     360

gatctcgttg tcgttccagg aaggttaaaa cttccagaaa gcaagcaagt gctaaatctc     420

gttgaactac tcctgaaatt acccgaagag gagacatcga acatcgtaat gatgatccca     480

gttaaaggta gaaagaattt cttcaaaggg atgctcaaaa cattatactg gatcttcggg     540

gagggagaaa ggccaagaac cgcagggcgc tatctcaagc atcttgaaag attaggatac     600

gttaagctca agagaagagg ctgtgaagtt ctcgactggg agtcacttaa gaggtacagg     660

aagctttacg agaccctcat taagaacctg aaatataacg gtaatagcag ggcatacatg     720

gttgaattta actctctcag ggatgtagtg agcttaatgc caatagaaga acttaaggag     780

tggataattg gagaacctag gggtcctaag ataggtacct tcattgatgt agatgattca     840

tttgcaaagc tcctaggtta ctacataagt agcggagatg tagagaaaga tagggtgaag     900

ttccacagta aagatcaaaa cgttctcgag gatatagcga aacttgccga gaagttattt     960

ggaaaggtga ggagaggaag aggatatatt gaggtatcag ggaaaattag ccatgccata    1020

tttagagttt tagcggaagg taagagaatt ccagagttca tcttcacatc cccaatggat    1080

attaaggtag ccttccttaa gggactcaac ggtaatgctg aagaattaac gttctccact    1140

aagagtgagc tattagttaa ccagcttatc cttctcctga actccattgg agtttcggat    1200

ataaagattg aacatgagaa aggggtttac agagtttaca taaataagaa ggaatcctcc 1260

aatggggata tagtacttga tagcgtcgaa tctatcgaag ttgaaaaata cgagggctac 1320

gtttatgatc taagtgttga ggataatgag aacttcctcg ttggcttcgg actactttac 1380

gcacacaaca gtatggacat gcgcgtgccc gcccagctgc tgggcctgct gctgctgtgg 1440

ttccccggct cgcgatgcga catccag 1467

<210> 103
<211> 489
<212> PRT
<213> Artificial

<220>
<223> Synthetic construct: partial amino acid sequence from construct
E.

<400> 103

Pro Gly Lys Thr Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr
                85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro
145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly
        355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile
            420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala His Asn Ser
    450                 455                 460

Met Asp Met Arg Val Pro Ala Gln Leu Leu Gly Leu Leu Leu Leu Trp
465                 470                 475                 480

Phe Pro Gly Ser Arg Cys Asp Ile Gln
                485

<210>  104
<211>  1458
<212>  DNA
<213>  Artificial

<220>
<223>  Synthetic construct:  partial coding sequence from construct H.

<400>  104
ccgggtaaaa gcattttacc agatgaatgg ctcccaattg ttgaaaatga aaaagttcga      60

ttcgtaaaaa ttggagactt catagatagg gagattgagg aaaacgctga gagagtgaag     120

agggatggtg aaactgaaat tctagaggtt aaagatctta aagccctttc cttcaataga     180

gaaacaaaaa agagcgagct caagaaggta aaggccctaa ttagacaccg ctattcaggg     240

aaggtttaca gcattaaact aaagtcaggg agaaggatca aaataacctc aggtcatagt     300

ctgttctcag taaaaaatgg aaagctagtt aaggtcaggg gagatgaact caagcctggt     360

gatctcgttg tcgttccagg aaggttaaaa cttccagaaa gcaagcaagt gctaaatctc     420

gttgaactac tcctgaaatt acccgaagag gagacatcga acatcgtaat gatgatccca     480

gttaaaggta gaaagaattt cttcaaaggg atgctcaaaa cattatactg gatcttcggg     540

gagggagaaa ggccaagaac cgcagggcgc tatctcaagc atcttgaaag attaggatac     600

gttaagctca agagaagagg ctgtgaagtt ctcgactggg agtcacttaa gaggtacagg     660

aagctttacg agaccctcat taagaacctg aaatataacg gtaatagcag ggcatacatg     720

gttgaattta actctctcag ggatgtagtg agcttaatgc caatagaaga acttaaggag     780

tggataattg gagaacctag gggtcctaag ataggtacct tcattgatgt agatgattca     840

tttgcaaagc tcctaggtta ctacataagt agcggagatg tagagaaaga tagggtgaag     900

ttccacagta aagatcaaaa cgttctcgag gatatagcga aacttgccga gaagttattt     960

ggaaaggtga ggagaggaag aggatatatt gaggtatcag ggaaaattag ccatgccata    1020

tttagagttt tagcggaagg taagagaatt ccagagttca tcttcacatc cccaatggat    1080

attaaggtag ccttccttaa gggactcaac ggtaatgctg aagaattaac gttctccact    1140

aagagtgagc tattagttaa ccagcttatc cttctcctga actccattgg agtttcggat    1200

ataaagattg aacatgagaa aggggtttac agagtttaca taaataagaa ggaatcctcc    1260

aatggggata tagtacttga tagcgtcgaa tctatcgaag ttgaaaaata cgagggctac    1320

gtttatgatc taagtgttga ggataatgag aacttcctcg ttggcttcgg actactttac    1380

gcacacaaca tggacatgcg cgtgcccgcc cagctgctgg gcgacgagtg gttccccggc    1440

tcgcgatgcg acatccag                                                  1458

<210>  105
<211>  486
<212>  PRT
<213>  Artificial

<220>
<223>  Synthetic construct:  partial amino acid sequence from construct
       H.

<400>  105

Pro Gly Lys Ser Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr
                85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro
145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly
        355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile
            420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala His Asn Met
    450                 455                 460

Asp Met Arg Val Pro Ala Gln Leu Leu Gly Asp Glu Trp Phe Pro Gly
465                 470                 475                 480

Ser Arg Cys Asp Ile Gln
                485

<210>  106
<211>  1443
<212>  DNA
<213>  Artificial

<220>
<223>  Synthetic construct:  partial coding sequence for construct J.

<400>  106
ccgggtaaaa gcattttacc agatgaatgg ctcccaattg ttgaaaatga aaaagttcga      60

ttcgtaaaaa ttggagactt catagatagg gagattgagg aaaacgctga gagagtgaag     120

agggatggtg aaactgaaat tctagaggtt aaagatctta aagccctttc cttcaataga     180

gaaacaaaaa agagcgagct caagaaggta aaggccctaa ttagacaccg ctattcaggg     240

aaggtttaca gcattaaact aaagtcaggg agaaggatca aaataacctc aggtcatagt     300

ctgttctcag taaaaaatgg aaagctagtt aaggtcaggg gagatgaact caagcctggt     360

gatctcgttg tcgttccagg aaggttaaaa cttccagaaa gcaagcaagt gctaaatctc     420

gttgaactac tcctgaaatt acccgaagag gagacatcga acatcgtaat gatgatccca     480

gttaaaggta gaaagaattt cttcaaaggg atgctcaaaa cattatactg gatcttcggg     540

gagggagaaa ggccaagaac cgcagggcgc tatctcaagc atcttgaaag attaggatac     600

gttaagctca agagaagagg ctgtgaagtt ctcgactggg agtcacttaa gaggtacagg     660

aagctttacg agaccctcat taagaacctg aaatataacg gtaatagcag ggcatacatg     720

gttgaattta actctctcag ggatgtagtg agcttaatgc caatagaaga acttaaggag     780

tggataattg gagaacctag gggtcctaag ataggtacct tcattgatgt agatgattca     840

tttgcaaagc tcctaggtta ctacataagt agcggagatg tagagaaaga tagggtgaag     900

ttccacagta aagatcaaaa cgttctcgag gatatagcga aacttgccga gaagttattt     960

ggaaaggtga ggagaggaag aggatatatt gaggtatcag ggaaaattag ccatgccata    1020

tttagagttt tagcggaagg taagagaatt ccagagttca tcttcacatc cccaatggat    1080

attaaggtag ccttccttaa gggactcaac ggtaatgctg aagaattaac gttctccact    1140

aagagtgagc tattagttaa ccagcttatc cttctcctga actccattgg agtttcggat    1200

ataaagattg aacatgagaa aggggtttac agagtttaca taaataagaa ggaatcctcc    1260

aatggggata tagtacttga tagcgtcgaa tctatcgaag ttgaaaaata cgagggctac    1320

gtttatgatc taagtgttga ggataatgag aacttcctcg ttggcttcgg actactttac    1380

gcacacaaca tggacatgcg cgtgcccgcc cagtggttcc ccggctcgcg atgcgacatc    1440

cag                                                                  1443

<210>  107
<211>  481
<212>  PRT
<213>  Artificial

<220>
<223>  Synthetic construct:  partial amino acid sequence from construct

J.

<400> 107

Pro Gly Lys Ser Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr
                85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro
145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly
        355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile
            420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala His Asn Met
    450                 455                 460

Asp Met Arg Val Pro Ala Gln Trp Phe Pro Gly Ser Arg Cys Asp Ile
465                 470                 475                 480

Gln

<210>  108
<211>  1398
<212>  DNA
<213>  Artificial

<220>
<223>  Synthetic construct:  partial coding sequence for construct K.

<400> 108
ccgggtaaaa gcattttacc agatgaatgg ctcccaattg ttgaaaatga aaaagttcga 60

ttcgtaaaaa ttggagactt catagatagg gagattgagg aaaacgctga gagagtgaag 120

agggatggtg aaactgaaat tctagaggtt aaagatctta aagccctttc cttcaataga 180

gaaacaaaaa agagcgagct caagaaggta aaggccctaa ttagacaccg ctattcaggg 240

aaggtttaca gcattaaact aaagtcaggg agaaggatca aaataacctc aggtcatagt 300

ctgttctcag taaaaaatgg aaagctagtt aaggtcaggg gagatgaact caagcctggt 360

gatctcgttg tcgttccagg aaggttaaaa cttccagaaa gcaagcaagt gctaaatctc 420

gttgaactac tcctgaaatt acccgaagag gagacatcga acatcgtaat gatgatccca 480

gttaaaggta gaaagaattt cttcaaaggg atgctcaaaa cattatactg gatcttcggg 540

gagggagaaa ggccaagaac cgcagggcgc tatctcaagc atcttgaaag attaggatac 600

gttaagctca agagaagagg ctgtgaagtt ctcgactggg agtcacttaa gaggtacagg 660

aagctttacg agaccctcat taagaacctg aaatataacg gtaatagcag ggcatacatg 720

gttgaattta actctctcag ggatgtagtg agcttaatgc caatagaaga acttaaggag 780

tggataattg gagaacctag gggtcctaag ataggtacct tcattgatgt agatgattca 840

tttgcaaagc tcctaggtta ctacataagt agcggagatg tagagaaaga tagggtgaag 900

ttccacagta aagatcaaaa cgttctcgag gatatagcga aacttgccga gaagttattt 960

ggaaaggtga ggagaggaag aggatatatt gaggtatcag ggaaaattag ccatgccata 1020

tttagagttt tagcggaagg taagagaatt ccagagttca tcttcacatc ccccatggat 1080

attaaggtag ccttccttaa gggactcaac ggtaatgctg aagaattaac gttctccact 1140

aagagtgagc tattagttaa ccagcttatc cttctcctga actccattgg agtttcggat 1200

ataaagattg aacatgagaa aggggtttac agagtttaca taaataagaa ggaatcctcc 1260

aatggggata tagtacttga tagcgtcgaa tctatcgaag ttgaaaaata cgagggctac 1320

gtttatgatc taagtgttga ggataatgag aacttcctcg ttggcttcgg actactttac 1380

gcacacaacg acatccag 1398

<210> 109
<211> 466
<212> PRT
<213> Artificial

<220>
<223> Synthetic construct: partial amino acid sequence for construct
K.

<400> 109

Pro Gly Lys Ser Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr
                85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro
145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly
        355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile
            420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala His Asn Asp
    450                 455                 460

Ile Gln
465

<210>  110
<211>  1464
<212>  DNA
<213>  Artificial

<220>
<223>  Synthetic construct:  partial coding sequence for construct L.

<400>  110
ccgggtaaaa gcattttacc agatgaatgg ctcccaattg ttgaaaatga aaaagttcga      60

ttcgtaaaaa ttggagactt catagatagg gagattgagg aaaacgctga gagagtgaag     120

agggatggtg aaactgaaat tctagaggtt aaagatctta aagccctttc cttcaataga     180

gaaacaaaaa agagcgagct caagaaggta aaggccctaa ttagacaccg ctattcaggg     240

aaggtttaca gcattaaact aaagtcaggg agaaggatca aaataacctc aggtcatagt     300

ctgttctcag taaaaaatgg aaagctagtt aaggtcaggg gagatgaact caagcctggt     360

gatctcgttg tcgttccagg aaggttaaaa cttccagaaa gcaagcaagt gctaaatctc     420

gttgaactac tcctgaaatt acccgaagag gagacatcga acatcgtaat gatgatccca 480
gttaaaggta gaaagaattt cttcaaaggg atgctcaaaa cattatactg gatcttcggg 540
gagggagaaa ggccaagaac cgcagggcgc tatctcaagc atcttgaaag attaggatac 600
gttaagctca agagaagagg ctgtgaagtt ctcgactggg agtcacttaa gaggtacagg 660
aagctttacg agaccctcat taagaacctg aaatataacg gtaatagcag ggcatacatg 720
gttgaattta actctctcag ggatgtagtg agcttaatgc caatagaaga acttaaggag 780
tggataattg gagaacctag gggtcctaag ataggtacct tcattgatgt agatgattca 840
tttgcaaagc tcctaggtta ctacataagt agcggagatg tagagaaaga tagggtgaag 900
ttccacagta aagatcaaaa cgttctcgag gatatagcga aacttgccga gaagttattt 960
ggaaaggtga ggagaggaag aggatatatt gaggtatcag ggaaaattag ccatgccata 1020
tttagagttt tagcggaagg taagagaatt ccagagttca tcttcacatc cccaatggat 1080
attaaggtag ccttccttaa gggactcaac ggtaatgctg aagaattaac gttctccact 1140
aagagtgagc tattagttaa ccagcttatc cttctcctga actccattgg agtttcggat 1200
ataaagattg aacatgagaa aggggtttac agagtttaca taaataagaa ggaatcctcc 1260
aatggggata tagtacttga tagcgtcgaa tctatcgaag ttgaaaaata cgagggctac 1320
gtttatgatc taagtgttga ggataatgag aacttcctcg ttggcttcgg actactttac 1380
gcacacaaca tggacatgcg cgtgcccgcc cagctgctgg gcctgctgct gctgtggttc 1440
cccggctcgg gaggcgacat ccag 1464

<210> 111
<211> 488
<212> PRT
<213> Artificial

<220>
<223> Synthetic construct: partial amino acid sequence of construct L.

<400> 111

Pro Gly Lys Ser Ile Leu Pro Asp Glu Trp Leu Pro Ile Val Glu Asn
1               5                   10                  15

Glu Lys Val Arg Phe Val Lys Ile Gly Asp Phe Ile Asp Arg Glu Ile
            20                  25                  30

Glu Glu Asn Ala Glu Arg Val Lys Arg Asp Gly Glu Thr Glu Ile Leu
        35                  40                  45

Glu Val Lys Asp Leu Lys Ala Leu Ser Phe Asn Arg Glu Thr Lys Lys
    50                  55                  60

Ser Glu Leu Lys Lys Val Lys Ala Leu Ile Arg His Arg Tyr Ser Gly
65                  70                  75                  80

Lys Val Tyr Ser Ile Lys Leu Lys Ser Gly Arg Arg Ile Lys Ile Thr

85                  90                  95

Ser Gly His Ser Leu Phe Ser Val Lys Asn Gly Lys Leu Val Lys Val
            100                 105                 110

Arg Gly Asp Glu Leu Lys Pro Gly Asp Leu Val Val Val Pro Gly Arg
        115                 120                 125

Leu Lys Leu Pro Glu Ser Lys Gln Val Leu Asn Leu Val Glu Leu Leu
    130                 135                 140

Leu Lys Leu Pro Glu Glu Glu Thr Ser Asn Ile Val Met Met Ile Pro
145                 150                 155                 160

Val Lys Gly Arg Lys Asn Phe Phe Lys Gly Met Leu Lys Thr Leu Tyr
                165                 170                 175

Trp Ile Phe Gly Glu Gly Glu Arg Pro Arg Thr Ala Gly Arg Tyr Leu
            180                 185                 190

Lys His Leu Glu Arg Leu Gly Tyr Val Lys Leu Lys Arg Arg Gly Cys
        195                 200                 205

Glu Val Leu Asp Trp Glu Ser Leu Lys Arg Tyr Arg Lys Leu Tyr Glu
    210                 215                 220

Thr Leu Ile Lys Asn Leu Lys Tyr Asn Gly Asn Ser Arg Ala Tyr Met
225                 230                 235                 240

Val Glu Phe Asn Ser Leu Arg Asp Val Val Ser Leu Met Pro Ile Glu
                245                 250                 255

Glu Leu Lys Glu Trp Ile Ile Gly Glu Pro Arg Gly Pro Lys Ile Gly
            260                 265                 270

Thr Phe Ile Asp Val Asp Asp Ser Phe Ala Lys Leu Leu Gly Tyr Tyr
        275                 280                 285

Ile Ser Ser Gly Asp Val Glu Lys Asp Arg Val Lys Phe His Ser Lys
    290                 295                 300

Asp Gln Asn Val Leu Glu Asp Ile Ala Lys Leu Ala Glu Lys Leu Phe
305                 310                 315                 320

Gly Lys Val Arg Arg Gly Arg Gly Tyr Ile Glu Val Ser Gly Lys Ile
                325                 330                 335

Ser His Ala Ile Phe Arg Val Leu Ala Glu Gly Lys Arg Ile Pro Glu
            340                 345                 350

Phe Ile Phe Thr Ser Pro Met Asp Ile Lys Val Ala Phe Leu Lys Gly

355                 360                 365

Leu Asn Gly Asn Ala Glu Glu Leu Thr Phe Ser Thr Lys Ser Glu Leu
    370                 375                 380

Leu Val Asn Gln Leu Ile Leu Leu Leu Asn Ser Ile Gly Val Ser Asp
385                 390                 395                 400

Ile Lys Ile Glu His Glu Lys Gly Val Tyr Arg Val Tyr Ile Asn Lys
                405                 410                 415

Lys Glu Ser Ser Asn Gly Asp Ile Val Leu Asp Ser Val Glu Ser Ile
            420                 425                 430

Glu Val Glu Lys Tyr Glu Gly Tyr Val Tyr Asp Leu Ser Val Glu Asp
        435                 440                 445

Asn Glu Asn Phe Leu Val Gly Phe Gly Leu Leu Tyr Ala His Asn Met
    450                 455                 460

Asp Met Arg Val Pro Ala Gln Leu Leu Gly Leu Leu Leu Leu Trp Phe
465                 470                 475                 480

Pro Gly Ser Gly Gly Asp Ile Gln
                485

<210>  112
<211>  26
<212>  DNA
<213>  Artificial

<220>
<223>  Synthetic construct:  oligonucleotide useful as a primer.

<400>  112
tgctttgcca agggtaccaa tgtttt                                          26

<210>  113
<211>  26
<212>  DNA
<213>  Artificial

<220>
<223>  Synthetic construct:  oligonucleotide useful as a primer.

<400>  113
attatggacg acaacctggt tggcaa                                          26

<210>  114
<211>  59
<212>  DNA
<213>  Artificial

<220>
<223>  Synthetic construct:  oligonucleotide useful as a primer.

<400>  114

ccgcagaaga gcctctccct gtctccgggt aaatgctttg ccaagggtac caatgtttt     59

<210>  115
<211>  62
<212>  DNA
<213>  Artificial

<220>
<223>  Synthetic construct:  oligonucleotide useful as a primer.

<400>  115
ccgcagaaga gcctctccct gtctccgggt aaagggtgct ttgccaaggg taccaatgtt     60

tt                                                                    62

<210>  116
<211>  68
<212>  DNA
<213>  Artificial

<220>
<223>  Synthetic construct:  oligonucleotide useful as a primer.

<400>  116
ccgcagaaga gcctctccct gtctccgggt aaatatgtcg ggtgctttgc caagggtacc     60

aatgtttt                                                              68

<210>  117
<211>  65
<212>  DNA
<213>  Artificial

<220>
<223>  Synthetic construct:  oligonucleotide useful as a primer.

<400>  117
cagcaggccc agcagctggg cgggcacgcg catgtccata ttatggacga caacctggtt     60

ggcaa                                                                 65

<210>  118
<211>  68
<212>  DNA
<213>  Artificial

<220>
<223>  Synthetic construct:  oligonucleotide useful as a primer.

<400>  118
cagcaggccc agcagctggg cgggcacgcg catgtccatg caattatgga cgacaacctg     60

gttggcaa                                                              68

<210>  119
<211>  74
<212>  DNA
<213>  Artificial

<220>
<223>  Synthetic construct:  oligonucleotide useful as a primer.

<400>  119

cagcaggccc agcagctggg cgggcacgcg catgtccatt tctccgcaat tatggacgac 60

aacctggttg gcaa 74

<210> 120
<211> 40
<212> DNA
<213> Artificial

<220>
<223> Synthetic construct: oligonucleotide useful as a primer.

<400> 120
ccactacacg cagaagagcc tctccctgtc tccgggtaaa 40

<210> 121
<211> 40
<212> DNA
<213> Artificial

<220>
<223> Synthetic construct: oligonucleotide useful as a primer.

<400> 121
gcagcaggcc cagcagctgg gcgggcacgc gcatgtccat 40

<210> 122
<211> 40
<212> DNA
<213> Artificial

<220>
<223> Synthetic construct: oligonucleotide useful as a primer.

<400> 122
atggacatgc gcgtgcccgc ccagctgctg ggcctgctgc 40

<210> 123
<211> 41
<212> DNA
<213> Artificial

<220>
<223> Synthetic construct: oligonucleotide useful as a primer.

<400> 123
tttacccgga gacagggaga ggctcttctg cgtgtagtgg t 41

<210> 124
<211> 9442
<212> DNA
<213> Artificial

<220>
<223> Synthetic construct: nucleotide sequence of plasmid pTT3-D2E7
Heavy Chain - intein - D2E7 Light Chain.

<400> 124
gcggccgctc gaggccggca aggccggatc ccccgacctc gacctctggc taataaagga 60

aatttatttt cattgcaata gtgtgttgga attttttgtg tctctcactc ggaaggacat 120

atgggagggc aaatcatttg gtcgagatcc ctcggagatc tctagctaga ggatcgatcc 180

ccgccccgga cgaactaaac ctgactacga catctctgcc ccttcttcgc ggggcagtgc 240
atgtaatccc ttcagttggt tggtacaact tgccaactgg gccctgttcc acatgtgaca 300
cgggggggga ccaaacacaa aggggttctc tgactgtagt tgacatcctt ataaatggat 360
gtgcacattt gccaacactg agtggctttc atcctggagc agactttgca gtctgtggac 420
tgcaacacaa cattgccttt atgtgtaact cttggctgaa gctcttacac caatgctggg 480
ggacatgtac ctcccagggg cccaggaaga ctacggaggc ctacaccaac gtcaatcaga 540
ggggcctgtg tagctaccga taagcggacc ctcaagaggg cattagcaat agtgtttata 600
aggccccctt gttaacccta aacgggtagc atatgcttcc cgggtagtag tatatactat 660
ccagactaac cctaattcaa tagcatatgt tacccaacgg gaagcatatg ctatcgaatt 720
agggttagta aaagggtcct aaggaacagc gatatctccc accccatgag ctgtcacggt 780
tttatttaca tggggtcagg attccacgag ggtagtgaac cattttagtc acaagggcag 840
tggctgaaga tcaaggagcg ggcagtgaac tctcctgaat cttcgcctgc ttcttcattc 900
tccttcgttt agctaataga ataactgctg agttgtgaac agtaaggtgt atgtgaggtg 960
ctcgaaaaca aggtttcagg tgacgccccc agaataaaat ttggacgggg ggttcagtgg 1020
tggcattgtg ctatgacacc aatataaccc tcacaaaccc cttgggcaat aaatactagt 1080
gtaggaatga aacattctga atatctttaa caatagaaat ccatggggtg gggacaagcc 1140
gtaaagactg gatgtccatc tcacacgaat ttatggctat gggcaacaca taatcctagt 1200
gcaatatgat actggggtta ttaagatgtg tcccaggcag ggaccaagac aggtgaacca 1260
tgttgttaca ctctatttgt aacaagggga aagagagtgg acgccgacag cagcggactc 1320
cactggttgt ctctaacacc cccgaaaatt aaacggggct ccacgccaat ggggcccata 1380
aacaaagaca agtggccact cttttttttg aaattgtgga gtgggggcac gcgtcagccc 1440
ccacacgccg ccctgcggtt ttggactgta aaataagggt gtaataactt ggctgattgt 1500
aaccccgcta accactgcgg tcaaaccact tgcccacaaa accactaatg gcaccccggg 1560
gaatacctgc ataagtaggt gggcgggcca agataggggc gcgattgctg cgatctggag 1620
gacaaattac acacacttgc gcctgagcgc caagcacagg gttgttggtc ctcatattca 1680
cgaggtcgct gagagcacgg tgggctaatg ttgccatggg tagcatatac tacccaaata 1740
tctggatagc atatgctatc ctaatctata tctgggtagc ataggctatc ctaatctata 1800
tctgggtagc atatgctatc ctaatctata tctgggtagt atatgctatc ctaatttata 1860
tctgggtagc ataggctatc ctaatctata tctgggtagc atatgctatc ctaatctata 1920
tctgggtagt atatgctatc ctaatctgta tccgggtagc atatgctatc ctaatagaga 1980
ttagggtagt atatgctatc ctaatttata tctgggtagc atatactacc caaatatctg 2040
gatagcatat gctatcctaa tctatatctg ggtagcatat gctatcctaa tctatatctg 2100
ggtagcatag gctatcctaa tctatatctg ggtagcatat gctatcctaa tctatatctg 2160
ggtagtatat gctatcctaa tttatatctg ggtagcatag gctatcctaa tctatatctg 2220

ggtagcatat gctatcctaa tctatatctg ggtagtatat gctatcctaa tctgtatccg 2280
ggtagcatat gctatcctca tgataagctg tcaaacatga gaattttctt gaagacgaaa 2340
gggcctcgtg atacgcctat ttttataggt taatgtcatg ataataatgg tttcttagac 2400
gtcaggtggc acttttcggg gaaatgtgcg cggaacccct atttgtttat ttttctaaat 2460
acattcaaat atgtatccgc tcatgagaca ataaccctga taaatgcttc aataatattg 2520
aaaaaggaag agtatgagta ttcaacattt ccgtgtcgcc cttattccct tttttgcggc 2580
attttgcctt cctgtttttg ctcacccaga aacgctggtg aaagtaaaag atgctgaaga 2640
tcagttgggt gcacgagtgg gttacatcga actggatctc aacagcggta agatccttga 2700
gagttttcgc cccgaagaac gttttccaat gatgagcact tttaaagttc tgctatgtgg 2760
cgcggtatta tcccgtgttg acgccgggca agagcaactc ggtcgccgca tacactattc 2820
tcagaatgac ttggttgagt actcaccagt cacagaaaag catcttacgg atggcatgac 2880
agtaagagaa ttatgcagtg ctgccataac catgagtgat aacactgcgg ccaacttact 2940
tctgacaacg atcggaggac cgaaggagct aaccgctttt ttgcacaaca tgggggatca 3000
tgtaactcgc cttgatcgtt gggaaccgga gctgaatgaa gccataccaa acgacgagcg 3060
tgacaccacg atgcctgcag caatggcaac aacgttgcgc aaactattaa ctggcgaact 3120
acttactcta gcttcccggc aacaattaat agactggatg gaggcggata aagttgcagg 3180
accacttctg cgctcggccc ttccggctgg ctggtttatt gctgataaat ctggagccgg 3240
tgagcgtggg tctcgcggta tcattgcagc actggggcca gatggtaagc cctcccgtat 3300
cgtagttatc tacacgacgg ggagtcaggc aactatggat gaacgaaata gacagatcgc 3360
tgagataggt gcctcactga ttaagcattg gtaactgtca gaccaagttt actcatatat 3420
actttagatt gatttaaaac ttcattttta atttaaaagg atctaggtga agatcctttt 3480
tgataatctc atgaccaaaa tcccttaacg tgagttttcg ttccactgag cgtcagaccc 3540
cgtagaaaag atcaaaggat cttcttgaga tccttttttt ctgcgcgtaa tctgctgctt 3600
gcaaacaaaa aaaccaccgc taccagcggt ggtttgtttg ccggatcaag agctaccaac 3660
tctttttccg aaggtaactg gcttcagcag agcgcagata ccaaatactg ttcttctagt 3720
gtagccgtag ttaggccacc acttcaagaa ctctgtagca ccgcctacat acctcgctct 3780
gctaatcctg ttaccagtgg ctgctgccag tggcgataag tcgtgtctta ccgggttgga 3840
ctcaagacga tagttaccgg ataaggcgca gcggtcgggc tgaacggggg gttcgtgcac 3900
acagcccagc ttggagcgaa cgacctacac cgaactgaga tacctacagc gtgagctatg 3960
agaaagcgcc acgcttcccg aagggagaaa ggcggacagg tatccggtaa gcggcagggt 4020
cggaacagga gagcgcacga gggagcttcc agggggaaac gcctggtatc tttatagtcc 4080
tgtcgggttt cgccacctct gacttgagcg tcgatttttg tgatgctcgt caggggggcg 4140
gagcctatgg aaaaacgcca gcaacgcggc ctttttacgg ttcctggcct tttgctggcc 4200
ttttgctcac atgttctttc ctgcgttatc ccctgattct gtggataacc gtattaccgc 4260

ctttgagtga gctgataccg ctcgccgcag ccgaacgacc gagcgcagcg agtcagtgag 4320
cgaggaagcg gaagagcgcc caatacgcaa accgcctctc cccgcgcgtt ggccgattca 4380
ttaatgcagc tggcacgaca ggtttcccga ctggaaagcg ggcagtgagc gcaacgcaat 4440
taatgtgagt tagctcactc attaggcacc ccaggcttta cactttatgc ttccggctcg 4500
tatgttgtgt ggaattgtga gcggataaca atttcacaca ggaaacagct atgaccatga 4560
ttacgccaag ctctagctag aggtcgacca attctcatgt ttgacagctt atcatcgcag 4620
atccgggcaa cgttgttgcc attgctgcag gcgcagaact ggtaggtatg gaagatctat 4680
acattgaatc aatattggca attagccata ttagtcattg gttatatagc ataaatcaat 4740
attggctatt ggccattgca tacgttgtat ctatatcata atatgtacat ttatattggc 4800
tcatgtccaa tatgaccgcc atgttgacat tgattattga ctagttatta atagtaatca 4860
attacggggt cattagttca tagcccatat atggagttcc gcgttacata acttacggta 4920
aatggcccgc ctggctgacc gcccaacgac ccccgcccat tgacgtcaat aatgacgtat 4980
gttcccatag taacgccaat agggactttc cattgacgtc aatgggtgga gtatttacgg 5040
taaactgccc acttggcagt acatcaagtg tatcatatgc caagtccgcc ccctattgac 5100
gtcaatgacg gtaaatggcc cgcctggcat tatgcccagt acatgacctt acgggacttt 5160
cctacttggc agtacatcta cgtattagtc atcgctatta ccatggtgat gcggttttgg 5220
cagtacacca atgggcgtgg atagcggttt gactcacggg gatttccaag tctccacccc 5280
attgacgtca atgggagttt gttttggcac caaaatcaac gggactttcc aaaatgtcgt 5340
aataaccccg ccccgttgac gcaaatgggc ggtaggcgtg tacggtggga ggtctatata 5400
agcagagctc gtttagtgaa ccgtcagatc ctcactctct tccgcatcgc tgtctgcgag 5460
ggccagctgt tgggctcgcg gttgaggaca aactcttcgc ggtctttcca gtactcttgg 5520
atcggaaacc cgtcggcctc cgaacggtac tccgccaccg agggacctga gcgagtccgc 5580
atcgaccgga tcggaaaacc tctcgagaaa ggcgtctaac cagtcacagt cgcaaggtag 5640
gctgagcacc gtggcgggcg gcagcgggtg gcggtcgggg ttgtttctgg cggaggtgct 5700
gctgatgatg taattaaagt aggcggtctt gagacggcgg atggtcgagg tgaggtgtgg 5760
caggcttgag atccagctgt tggggtgagt actccctctc aaaagcgggc attacttctg 5820
cgctaagatt gtcagtttcc aaaaacgagg aggatttgat attcacctgg cccgatctgg 5880
ccatacactt gagtgacaat gacatccact ttgcctttct ctccacaggt gtccactccc 5940
aggtccaagt ttgggcgcca ccatggagtt tgggctgagc tggctttttc ttgtcgcgat 6000
tttaaaaggt gtccagtgtg aggtgcagct ggtggagtct gggggaggct tggtacagcc 6060
cggcaggtcc ctgagactct cctgtgcggc ctctggattc acctttgatg attatgccat 6120
gcactgggtc cggcaagctc cagggaaggg cctggaatgg gtctcagcta tcacttggaa 6180
tagtggtcac atagactatg cggactctgt ggagggccga ttcaccatct ccagagacaa 6240
cgccaagaac tccctgtatc tgcaaatgaa cagtctgaga gctgaggata cggccgtata 6300

ttactgtgcg aaagtctcgt accttagcac cgcgtcctcc cttgactatt ggggccaagg 6360
taccctggtc accgtctcga gtgcgtcgac caagggccca tcggtcttcc ccctggcacc 6420
ctcctccaag agcacctctg ggggcacagc ggccctgggc tgcctggtca aggactactt 6480
ccccgaaccg gtgacggtgt cgtggaactc aggcgccctg accagcggcg tgcacacctt 6540
cccggctgtc ctacagtcct caggactcta ctccctcagc agcgtggtga ccgtgccctc 6600
cagcagcttg ggcacccaga cctacatctg caacgtgaat cacaagccca gcaacaccaa 6660
ggtggacaag aaagttgagc ccaaatcttg tgacaaaact cacacatgcc caccgtgccc 6720
agcacctgaa ctcctggggg gaccgtcagt cttcctcttc cccccaaaac ccaaggacac 6780
cctcatgatc tcccggaccc ctgaggtcac atgcgtggtg gtggacgtga gccacgaaga 6840
ccctgaggtc aagttcaact ggtacgtgga cggcgtggag gtgcataatg ccaagacaaa 6900
gccgcgggag gagcagtaca acagcacgta ccgtgtggtc agcgtcctca ccgtcctgca 6960
ccaggactgg ctgaatggca aggagtacaa gtgcaaggtc tccaacaaag ccctcccagc 7020
ccccatcgag aaaaccatct ccaaagccaa agggcagccc cgagaaccac aggtgtacac 7080
cctgccccca tcccgggatg agctgaccaa gaaccaggtc agcctgacct gcctggtcaa 7140
aggcttctat cccagcgaca tcgccgtgga gtgggagagc aatgggcagc cggagaacaa 7200
ctacaagacc acgcctcccg tgctggactc cgacggctcc ttcttcctct acagcaagct 7260
caccgtggac aagagcaggt ggcagcaggg gaacgtcttc tcatgctccg tgatgcatga 7320
ggctctgcac aaccactaca cgcagaagag cctctccctg tctccgggta aatgctttgc 7380
caagggtacc aatgttttaa tggcggatgg gtctattgaa tgtattgaaa acattgaggt 7440
tggtaataag gtcatgggta aagatggcag acctcgtgag gtaattaaat tgcccagagg 7500
aagagaaact atgtacagcg tcgtgcagaa aagtcagcac agagcccaca aaagtgactc 7560
aagtcgtgaa gtgccagaat tactcaagtt tacgtgtaat gcgacccatg agttggttgt 7620
tagaacacct cgtagtgtcc gccgtttgtc tcgtaccatt aagggtgtcg aatattttga 7680
agttattact tttgagatgg gccaaaagaa agcccccgac ggtagaattg ttgagcttgt 7740
caaggaagtt tcaaagagct acccaatatc tgagggccct gagagagcca acgaattagt 7800
agaatcctat agaaaggctt caaataaagc ttattttgag tggactattg aggccagaga 7860
tctttctctg ttgggttccc atgttcgtaa agctacctac cagacttacg ctccaattct 7920
ttatgagaat gaccactttt tcgactacat gcaaaaaagt aagtttcatc tcaccattga 7980
aggtccaaaa gtacttgctt atttacttgg tttatggatt ggtgatggat tgtctgacag 8040
ggcaactttt tcggttgatt ccagagatac ttctttgatg gaacgtgtta ctgaatatgc 8100
tgaaaagttg aatttgtgcg ccgagtataa ggacagaaaa gaaccacaag ttgccaaaac 8160
tgttaatttg tactctaaag ttgtcagagg taatggtatt cgcaataatc ttaatactga 8220
gaatccatta tgggacgcta ttgttggctt aggattcttg aaggacggtg tcaaaaatat 8280
tccttctttc ttgtctacgg acaatatcgg tactcgtgaa acatttcttg ctggtctaat 8340

tgattctgat ggctatgtta ctgatgagca tggtattaaa gcaacaataa agacaattca 8400

tacttctgtc agagatggtt tggtttccct tgctcgttct ttaggcttag tagtctcggt 8460

taacgcagaa cctgctaagg ttgacatgaa tggcaccaaa cataaaatta gttatgctat 8520

ttatatgtct ggtggagatg ttttgcttaa cgttctttcg aagtgtgccg gctctaaaaa 8580

attcaggcct gctcccgccg ctgcttttgc acgtgagtgc cgcggatttt atttcgagtt 8640

acaagaattg aaggaagacg attattatgg gattacttta tctgatgatt ctgatcatca 8700

gtttttgctt gccaaccagg ttgtcgtcca taatatggac atgcgcgtgc ccgcccagct 8760

gctgggcctg ctgctgctgt ggttccccgg ctcgcgatgc gacatccaga tgacccagtc 8820

tccatcctcc ctgtctgcat ctgtagggga cagagtcacc atcacttgtc gggcaagtca 8880

gggcatcaga aattacttag cctggtatca gcaaaaacca gggaaagccc ctaagctcct 8940

gatctatgct gcatccactt tgcaatcagg ggtcccatct cggttcagtg gcagtggatc 9000

tgggacagat ttcactctca ccatcagcag cctacagcct gaagatgttg caacttatta 9060

ctgtcaaagg tataaccgtg caccgtatac ttttggccag gggaccaagg tggaaatcaa 9120

acgtacggtg gctgcaccat ctgtcttcat cttcccgcca tctgatgagc agttgaaatc 9180

tggaactgcc tctgttgtgt gcctgctgaa taacttctat cccagagagg ccaaagtaca 9240

gtggaaggtg gataacgccc tccaatcggg taactcccag gagagtgtca cagagcagga 9300

cagcaaggac agcacctaca gcctcagcag caccctgacg ctgagcaaag cagactacga 9360

gaaacacaaa gtctacgcct gcgaagtcac ccatcagggc ctgagctcgc ccgtcacaaa 9420

gagcttcaac aggggagagt gt 9442

<210> 125
<211> 1386
<212> DNA
<213> Artificial

<220>
<223> Synthetic construct: partial coding sequence in
pTT3-HC-VMAint-LC-1aa.

<400> 125
ccgggtaaag ggtgctttgc caagggtacc aatgttttaa tggcggatgg gtctattgaa 60

tgtattgaaa acattgaggt tggtaataag gtcatgggta aagatggcag acctcgtgag 120

gtaattaaat tgcccagagg aagagaaact atgtacagcg tcgtgcagaa aagtcagcac 180

agagcccaca aaagtgactc aagtcgtgaa gtgccagaat tactcaagtt tacgtgtaat 240

gcgacccatg agttggttgt tagaacacct cgtagtgtcc gccgtttgtc tcgtaccatt 300

aagggtgtcg aatattttga agttattact tttgagatgg gccaaaagaa agcccccgac 360

ggtagaattg ttgagcttgt caaggaagtt tcaaagagct acccaatatc tgaggggcct 420

gagagagcca acgaattagt agaatcctat agaaaggctt caaataaagc ttattttgag 480

tggactattg aggccagaga tctttctctg ttgggttccc atgttcgtaa agctacctac 540

cagacttacg ctccaattct ttatgagaat gaccactttt tcgactacat gcaaaaaagt 600

aagtttcatc tcaccattga aggtccaaaa gtacttgctt atttacttgg tttatggatt 660

ggtgatggat tgtctgacag ggcaactttt tcggttgatt ccagagatac ttctttgatg 720

gaacgtgtta ctgaatatgc tgaaaagttg aatttgtgcg ccgagtataa ggacagaaaa 780

gaaccacaag ttgccaaaac tgttaatttg tactctaaag ttgtcagagg taatggtatt 840

cgcaataatc ttaatactga gaatccatta tgggacgcta ttgttggctt aggattcttg 900

aaggacggtg tcaaaaatat tccttctttc ttgtctacgg acaatatcgg tactcgtgaa 960

acatttcttg ctggtctaat tgattctgat ggctatgtta ctgatgagca tggtattaaa 1020

gcaacaataa agacaattca tacttctgtc agagatggtt tggtttccct tgctcgttct 1080

ttaggcttag tagtctcggt taacgcagaa cctgctaagg ttgacatgaa tggcaccaaa 1140

cataaaatta gttatgctat ttatatgtct ggtggagatg ttttgcttaa cgttctttcg 1200

aagtgtgccg gctctaaaaa attcaggcct gctcccgccg ctgcttttgc acgtgagtgc 1260

cgcggatttt atttcgagtt acaagaattg aaggaagacg attattatgg gattacttta 1320

tctgatgatt ctgatcatca gtttttgctt gccaaccagg ttgtcgtcca taattgcatg 1380

gacatg 1386

<210> 126
<211> 1398
<212> DNA
<213> Artificial

<220>
<223> Synthetic construct: partial coding sequence from
pTT3-HC-VMAint-LC-3aa.

<400> 126
ccgggtaaat atgtcgggtg ctttgccaag ggtaccaatg ttttaatggc ggatgggtct 60

attgaatgta ttgaaaacat tgaggttggt aataaggtca tgggtaaaga tggcagacct 120

cgtgaggtaa ttaaattgcc cagaggaaga gaaactatgt acagcgtcgt gcagaaaagt 180

cagcacagag cccacaaaag tgactcaagt cgtgaagtgc cagaattact caagtttacg 240

tgtaatgcga cccatgagtt ggttgttaga acacctcgta gtgtccgccg tttgtctcgt 300

accattaagg gtgtcgaata ttttgaagtt attacttttg agatgggcca aaagaaagcc 360

cccgacggta gaattgttga gcttgtcaag gaagtttcaa agagctaccc aatatctgag 420

gggcctgaga gagccaacga attagtagaa tcctatagaa aggcttcaaa taaagcttat 480

tttgagtgga ctattgaggc cagagatctt tctctgttgg gttcccatgt tcgtaaagct 540

acctaccaga cttacgctcc aattctttat gagaatgacc actttttcga ctacatgcaa 600

aaaagtaagt ttcatctcac cattgaaggt ccaaaagtac ttgcttattt acttggttta 660

tggattggtg atggattgtc tgacagggca actttttcgg ttgattccag agatacttct 720

ttgatggaac gtgttactga atatgctgaa aagttgaatt tgtgcgccga gtataaggac 780

agaaaagaac cacaagttgc caaaactgtt aatttgtact ctaaagttgt cagaggtaat 840

ggtattcgca ataatcttaa tactgagaat ccattatggg acgctattgt tggcttagga 900

ttcttgaagg acggtgtcaa aaatattcct tctttcttgt ctacggacaa tatcggtact 960

cgtgaaacat ttcttgctgg tctaattgat tctgatggct atgttactga tgagcatggt 1020

attaaagcaa caataaagac aattcatact tctgtcagag atggtttggt ttcccttgct 1080

cgttctttag gcttagtagt ctcggttaac gcagaacctg ctaaggttga catgaatggc 1140

accaaacata aaattagtta tgctatttat atgtctggtg gagatgtttt gcttaacgtt 1200

ctttcgaagt gtgccggctc taaaaaattc aggcctgctc ccgccgctgc ttttgcacgt 1260

gagtgccgcg gatttatttt cgagttacaa gaattgaagg aagacgatta ttatgggatt 1320

actttatctg atgattctga tcatcagttt ttgcttgcca accaggttgt cgtccataat 1380

tgcggagaaa tggacatg 1398

<210> 127
<211> 1050
<212> DNA
<213> Artificial

<220>
<223> Synthetic construct: engineered Synechococcus intein coding
      sequence.

<400> 127
gggcgaattg ggtaccgaat tctgcctgtc cttcggcacc gagatcctga ccgtggagta 60

cccgcttaac ccatggctta agacggacag gaagccgtgg ctctaggact ggcacctcat 120

cggccctctg cctatcggca agatcgtgtc cgaagagatc aactgctccg tgtactccgt 180

gccgggagac ggatagccgt tctagcacag gcttctctag ttgacgaggc acatgaggca 240

ggaccctgag ggccgggtgt atactcaggc catcgcccag tggcacgacc ggggcgagca 300

cctgggactc ccggcccaca tatgagtccg gtagcgggtc accgtgctgg ccccgctcgt 360

ggaggtgctg gagtacgagc tggaggacgg ctccgtgatc cgggccacct ccgaccaccg 420

cctccacgac ctcatgctcg acctcctgcc gaggcactag gcccggtgga ggctggtggc 480

gtttctgacc accgactatc agctgctggc catcgaggag atcttcgccc ggcagctgga 540

caaagactgg tggctgatag tcgacgaccg gtagctcctc tagaagcggg ccgtcgacct 600

cctgctgacc ctggagaaca tcaagcagac cgaggaggcc ctggacaacc accggctgcc 660

ggacgactgg gacctcttgt agttcgtctg gctcctccgg gacctgttgg tggccgacgg 720

tttccctctg ctggacgccg gcaccatcaa gatggtgaag gtgatcggca ggcggtccct 780

aaagggagac gacctgcggc cgtggtagtt ctaccacttc cactagccgt ccgccaggga 840

gggcgtgcag cggatcttcg acatcggcct gcctcaggac cacaactttc tgctggccaa 900

cccgcacgtc gcctagaagc tgtagccgga cggagtcctg gtgttgaaag acgaccggtt 960

cggcgccatc gccgccaaca agcttgagct ccagcttttg ttcccgccgc ggtagcggcg 1020

gttgttcgaa ctcgaggtcg aaaacaaggg 1050

<210>  128
<211>  159
<212>  PRT
<213>  Artificial

<220>
<223>  Synthetic:  intein encoded by engineered Synechococcus sequence.

<400>  128

Cys Leu Ser Phe Gly Thr Glu Ile Leu Thr Val Glu Tyr Gly Pro Leu
1               5                   10                  15

Pro Ile Gly Lys Ile Val Ser Glu Glu Ile Asn Cys Ser Val Tyr Ser
            20                  25                  30

Val Asp Pro Glu Gly Arg Val Tyr Thr Gln Ala Ile Ala Gln Trp His
        35                  40                  45

Asp Arg Gly Glu Gln Glu Val Leu Glu Tyr Glu Leu Glu Asp Gly Ser
    50                  55                  60

Val Ile Arg Ala Thr Ser Asp His Arg Phe Leu Thr Thr Asp Tyr Gln
65                  70                  75                  80

Leu Leu Ala Ile Glu Glu Ile Phe Ala Arg Gln Leu Asp Leu Leu Thr
                85                  90                  95

Leu Glu Asn Ile Lys Gln Thr Glu Glu Ala Leu Asp Asn His Arg Leu
            100                 105                 110

Pro Phe Pro Leu Leu Asp Ala Gly Thr Ile Lys Met Val Lys Val Ile
        115                 120                 125

Gly Arg Arg Ser Leu Gly Val Gln Arg Ile Phe Asp Ile Gly Leu Pro
    130                 135                 140

Gln Asp His Asn Phe Leu Leu Ala Asn Gly Ala Ile Ala Ala Asn
145                 150                 155


<210>  129
<211>  61
<212>  DNA
<213>  Artificial

<220>
<223>  Synthetic construct:  oligonucleotide useful as a primer.

<400>  129
ccactacacg cagaagagcc tctccctgtc tccgggtaaa tgcctgtcct tcggcaccga      60

g                                                                      61


<210>  130
<211>  65
<212>  DNA

<213>  Artificial

<220>
<223>  Synthetic construct:  oligonucleotide useful as a primer.

<400>  130
gcagcaggcc cagcagctgg gcgggcacgc gcatgtccat gttggcggcg atggcgccgt      60

tggcc                                                                  65


<210>  131
<211>  64
<212>  DNA
<213>  Artificial

<220>
<223>  Synthetic construct:  oligonucleotide useful as a primer.

<400>  131
ccactacacg cagaagagcc tctccctgtc tccgggtaaa tattgcctgt ccttcggcac      60

cgag                                                                   64


<210>  132
<211>  63
<212>  DNA
<213>  Artificial

<220>
<223>  Synthetic construct:  oligonucleotide useful as a primer.

<400>  132
gcagcaggcc cagcagctgg gcgggcacgc gcatgtccat acagttggcg gcgatggcgc      60

cgt                                                                    63


<210>  133
<211>  70
<212>  DNA
<213>  Artificial

<220>
<223>  Synthetic construct:  oligonucleotide useful as a primer.

<400>  133
ccactacacg cagaagagcc tctccctgtc tccgggtaaa gccgagtatt gcctgtcctt      60

cggcaccgag                                                             70


<210>  134
<211>  70
<212>  DNA
<213>  Artificial

<220>
<223>  Synthetic construct:  oligonucleotide useful as a primer.

<400>  134
ccactacacg cagaagagcc tctccctgtc tccgggtaaa gccgagtatt gcctgtcctt      60

cggcaccgag                                                             70


<210>  135

<211>  8557
<212>  DNA
<213>  Artificial

<220>
<223>  Synthetic construct:  nucleotide equence of plasmid  pTT3-D2E7
       Heavy Chain - Ssp-GA-intein - D2E7 Light Chain.

<400>  135
gcggccgctc gaggccggca aggccggatc ccccgacctc gacctctggc taataaagga      60

aatttatttt cattgcaata gtgtgttgga attttttgtg tctctcactc ggaaggacat     120

atgggagggc aaatcatttg gtcgagatcc ctcggagatc tctagctaga ggatcgatcc     180

ccgccccgga cgaactaaac ctgactacga catctctgcc ccttcttcgc ggggcagtgc     240

atgtaatccc ttcagttggt tggtacaact tgccaactgg gccctgttcc acatgtgaca     300

cgggggggga ccaaacacaa aggggttctc tgactgtagt tgacatcctt ataaatggat     360

gtgcacattt gccaacactg agtggctttc atcctggagc agactttgca gtctgtggac     420

tgcaacacaa cattgccttt atgtgtaact cttggctgaa gctcttacac caatgctggg     480

ggacatgtac ctcccagggg cccaggaaga ctacgggagg ctacaccaac gtcaatcaga     540

ggggcctgtg tagctaccga taagcggacc ctcaagaggg cattagcaat agtgtttata     600

aggccccctt gttaacccta aacgggtagc atatgcttcc cgggtagtag tatatactat     660

ccagactaac cctaattcaa tagcatatgt tacccaacgg gaagcatatg ctatcgaatt     720

agggttagta aaagggtcct aaggaacagc gatatctccc accccatgag ctgtcacggt     780

tttatttaca tggggtcagg attccacgag ggtagtgaac cattttagtc acaagggcag     840

tggctgaaga tcaaggagcg ggcagtgaac tctcctgaat cttcgcctgc ttcttcattc     900

tccttcgttt agctaataga ataactgctg agttgtgaac agtaaggtgt atgtgaggtg     960

ctcgaaaaca aggtttcagg tgacgccccc agaataaaat ttggacgggg ggttcagtgg    1020

tggcattgtg ctatgacacc aatataaccc tcacaaaccc cttgggcaat aaatactagt    1080

gtaggaatga aacattctga atatctttaa caatagaaat ccatggggtg gggacaagcc    1140

gtaaagactg gatgtccatc tcacacgaat ttatggctat gggcaacaca taatcctagt    1200

gcaatatgat actggggtta ttaagatgtg tcccaggcag ggaccaagac aggtgaacca    1260

tgttgttaca ctctatttgt aacaagggga aagagagtgg acgccgacag cagcggactc    1320

cactggttgt ctctaacacc cccgaaaatt aaacggggct ccacgccaat ggggcccata    1380

aacaaagaca agtggccact cttttttttg aaattgtgga gtgggggcac gcgtcagccc    1440

ccacacgccg ccctgcggtt ttggactgta aaataagggt gtaataactt ggctgattgt    1500

aaccccgcta accactgcgg tcaaaccact tgcccacaaa accactaatg gcaccccggg    1560

gaatacctgc ataagtaggt gggcgggcca agataggggc gcgattgctg cgatctggag    1620

gacaaattac acacacttgc gcctgagcgc caagcacagg gttgttggtc ctcatattca    1680

cgaggtcgct gagagcacgg tgggctaatg ttgccatggg tagcatatac tacccaaata    1740

tctggatagc atatgctatc ctaatctata tctgggtagc ataggctatc ctaatctata    1800

tctgggtagc atatgctatc ctaatctata tctgggtagt atatgctatc ctaatttata 1860
tctgggtagc ataggctatc ctaatctata tctgggtagc atatgctatc ctaatctata 1920
tctgggtagt atatgctatc ctaatctgta tccgggtagc atatgctatc ctaatagaga 1980
ttagggtagt atatgctatc ctaatttata tctgggtagc atatactacc caaatatctg 2040
gatagcatat gctatcctaa tctatatctg ggtagcatat gctatcctaa tctatatctg 2100
ggtagcatag gctatcctaa tctatatctg ggtagcatat gctatcctaa tctatatctg 2160
ggtagtatat gctatcctaa tttatatctg ggtagcatag gctatcctaa tctatatctg 2220
ggtagcatat gctatcctaa tctatatctg ggtagtatat gctatcctaa tctgtatccg 2280
ggtagcatat gctatcctca tgataagctg tcaaacatga gaattttctt gaagacgaaa 2340
gggcctcgtg atacgcctat ttttataggt taatgtcatg ataataatgg tttcttagac 2400
gtcaggtggc acttttcggg gaaatgtgcg cggaacccct atttgtttat ttttctaaat 2460
acattcaaat atgtatccgc tcatgagaca ataaccctga taaatgcttc aataatattg 2520
aaaaaggaag agtatgagta ttcaacattt ccgtgtcgcc cttattccct tttttgcggc 2580
attttgcctt cctgtttttg ctcacccaga aacgctggtg aaagtaaaag atgctgaaga 2640
tcagttgggt gcacgagtgg gttacatcga actggatctc aacagcggta agatccttga 2700
gagttttcgc cccgaagaac gttttccaat gatgagcact tttaaagttc tgctatgtgg 2760
cgcggtatta tcccgtgttg acgccgggca agagcaactc ggtcgccgca tacactattc 2820
tcagaatgac ttggttgagt actcaccagt cacagaaaag catcttacgg atggcatgac 2880
agtaagagaa ttatgcagtg ctgccataac catgagtgat aacactgcgg ccaacttact 2940
tctgacaacg atcggaggac cgaaggagct aaccgctttt ttgcacaaca tgggggatca 3000
tgtaactcgc cttgatcgtt gggaaccgga gctgaatgaa gccataccaa acgacgagcg 3060
tgacaccacg atgcctgcag caatggcaac aacgttgcgc aaactattaa ctggcgaact 3120
acttactcta gcttcccggc aacaattaat agactggatg gaggcggata aagttgcagg 3180
accacttctg cgctcggccc ttccggctgg ctggtttatt gctgataaat ctggagccgg 3240
tgagcgtggg tctcgcggta tcattgcagc actggggcca gatggtaagc cctcccgtat 3300
cgtagttatc tacacgacgg ggagtcaggc aactatggat gaacgaaata gacagatcgc 3360
tgagataggt gcctcactga ttaagcattg gtaactgtca gaccaagttt actcatatat 3420
actttagatt gatttaaaac ttcattttta atttaaaagg atctaggtga agatcctttt 3480
tgataatctc atgaccaaaa tcccttaacg tgagttttcg ttccactgag cgtcagaccc 3540
cgtagaaaag atcaaaggat cttcttgaga tccttttttt ctgcgcgtaa tctgctgctt 3600
gcaaacaaaa aaaccaccgc taccagcggt ggtttgtttg ccggatcaag agctaccaac 3660
tctttttccg aaggtaactg gcttcagcag agcgcagata ccaaatactg ttcttctagt 3720
gtagccgtag ttaggccacc acttcaagaa ctctgtagca ccgcctacat acctcgctct 3780
gctaatcctg ttaccagtgg ctgctgccag tggcgataag tcgtgtctta ccgggttgga 3840

ctcaagacga tagttaccgg ataaggcgca gcggtcgggc tgaacggggg gttcgtgcac 3900
acagcccagc ttggagcgaa cgacctacac cgaactgaga tacctacagc gtgagctatg 3960
agaaagcgcc acgcttcccg aagggagaaa ggcggacagg tatccggtaa gcggcagggt 4020
cggaacagga gagcgcacga gggagcttcc agggggaaac gcctggtatc tttatagtcc 4080
tgtcgggttt cgccacctct gacttgagcg tcgatttttg tgatgctcgt caggggggcg 4140
gagcctatgg aaaaacgcca gcaacgcggc ctttttacgg ttcctggcct tttgctggcc 4200
ttttgctcac atgttctttc ctgcgttatc ccctgattct gtggataacc gtattaccgc 4260
ctttgagtga gctgataccg ctcgccgcag ccgaacgacc gagcgcagcg agtcagtgag 4320
cgaggaagcg gaagagcgcc caatacgcaa accgcctctc cccgcgcgtt ggccgattca 4380
ttaatgcagc tggcacgaca ggtttcccga ctggaaagcg ggcagtgagc gcaacgcaat 4440
taatgtgagt tagctcactc attaggcacc ccaggcttta cactttatgc ttccggctcg 4500
tatgttgtgt ggaattgtga gcggataaca atttcacaca ggaaacagct atgaccatga 4560
ttacgccaag ctctagctag aggtcgacca attctcatgt ttgacagctt atcatcgcag 4620
atccgggcaa cgttgttgcc attgctgcag gcgcagaact ggtaggtatg gaagatctat 4680
acattgaatc aatattggca attagccata ttagtcattg gttatatagc ataaatcaat 4740
attggctatt ggccattgca tacgttgtat ctatatcata atatgtacat ttatattggc 4800
tcatgtccaa tatgaccgcc atgttgacat tgattattga ctagttatta atagtaatca 4860
attacggggt cattagttca tagcccatat atggagttcc gcgttacata acttacggta 4920
aatggcccgc ctggctgacc gcccaacgac ccccgcccat tgacgtcaat aatgacgtat 4980
gttcccatag taacgccaat agggactttc cattgacgtc aatgggtgga gtatttacgg 5040
taaactgccc acttggcagt acatcaagtg tatcatatgc caagtccgcc ccctattgac 5100
gtcaatgacg gtaaatggcc cgcctggcat tatgcccagt acatgacctt acgggacttt 5160
cctacttggc agtacatcta cgtattagtc atcgctatta ccatggtgat gcggttttgg 5220
cagtacacca atgggcgtgg atagcggttt gactcacggg gatttccaag tctccacccc 5280
attgacgtca atgggagttt gttttggcac caaaatcaac gggactttcc aaaatgtcgt 5340
aataaccccg ccccgttgac gcaaatgggc ggtaggcgtg tacggtggga ggtctatata 5400
agcagagctc gtttagtgaa ccgtcagatc ctcactctct tccgcatcgc tgtctgcgag 5460
ggccagctgt tgggctcgcg gttgaggaca aactcttcgc ggtctttcca gtactcttgg 5520
atcggaaacc cgtcggcctc cgaacggtac tccgccaccg agggacctga gcgagtccgc 5580
atcgaccgga tcggaaaacc tctcgagaaa ggcgtctaac cagtcacagt cgcaaggtag 5640
gctgagcacc gtggcgggcg gcagcgggtg gcggtcgggg ttgtttctgg cggaggtgct 5700
gctgatgatg taattaaagt aggcggtctt gagacggcgg atggtcgagg tgaggtgtgg 5760
caggcttgag atccagctgt tggggtgagt actccctctc aaaagcgggc attacttctg 5820
cgctaagatt gtcagtttcc aaaaacgagg aggatttgat attcacctgg cccgatctgg 5880

ccatacactt gagtgacaat gacatccact ttgcctttct ctccacaggt gtccactccc 5940
aggtccaagt ttgggcgcca ccatggagtt tgggctgagc tggctttttc ttgtcgcgat 6000
tttaaaaggt gtccagtgtg aggtgcagct ggtggagtct gggggaggct tggtacagcc 6060
cggcaggtcc ctgagactct cctgtgcggc ctctggattc acctttgatg attatgccat 6120
gcactgggtc cggcaagctc cagggaaggg cctggaatgg gtctcagcta tcacttggaa 6180
tagtggtcac atagactatg cggactctgt ggagggccga ttcaccatct ccagagacaa 6240
cgccaagaac tccctgtatc tgcaaatgaa cagtctgaga gctgaggata cggccgtata 6300
ttactgtgcg aaagtctcgt accttagcac cgcgtcctcc cttgactatt ggggccaagg 6360
taccctggtc accgtctcga gtgcgtcgac caagggccca tcggtcttcc ccctggcacc 6420
ctcctccaag agcacctctg ggggcacagc ggccctgggc tgcctggtca aggactactt 6480
ccccgaaccg gtgacggtgt cgtggaactc aggcgccctg accagcggcg tgcacacctt 6540
cccggctgtc ctacagtcct caggactcta ctccctcagc agcgtggtga ccgtgccctc 6600
cagcagcttg ggcacccaga cctacatctg caacgtgaat cacaagccca gcaacaccaa 6660
ggtggacaag aaagttgagc ccaaatcttg tgacaaaact cacacatgcc caccgtgccc 6720
agcacctgaa ctcctggggg gaccgtcagt cttcctcttc cccccaaaac ccaaggacac 6780
cctcatgatc tcccggaccc ctgaggtcac atgcgtggtg gtggacgtga gccacgaaga 6840
ccctgaggtc aagttcaact ggtacgtgga cggcgtggag gtgcataatg ccaagacaaa 6900
gccgcgggag gagcagtaca acagcacgta ccgtgtggtc agcgtcctca ccgtcctgca 6960
ccaggactgg ctgaatggca aggagtacaa gtgcaaggtc tccaacaaag ccctcccagc 7020
ccccatcgag aaaaccatct ccaaagccaa agggcagccc cgagaaccac aggtgtacac 7080
cctgccccca tcccgggatg agctgaccaa gaaccaggtc agcctgacct gcctggtcaa 7140
aggcttctat cccagcgaca tcgccgtgga gtgggagagc aatgggcagc cggagaacaa 7200
ctacaagacc acgcctcccg tgctggactc cgacggctcc ttcttcctct acagcaagct 7260
caccgtggac aagagcaggt ggcagcaggg gaacgtcttc tcatgctccg tgatgcatga 7320
ggctctgcac aaccactaca cgcagaagag cctctccctg tctccgggta aatgcctgtc 7380
cttcggcacc gagatcctga ccgtggagta cggccctctg cctatcggca agatcgtgtc 7440
cgaagagatc aactgctccg tgtactccgt ggaccctgag ggccgggtgt atactcaggc 7500
catcgcccag tggcacgacc ggggcgagca ggaggtgctg gagtacgagc tggaggacgg 7560
ctccgtgatc cgggccacct ccgaccaccg gtttctgacc accgactatc agctgctggc 7620
catcgaggag atcttcgccc ggcagctgga cctgctgacc ctggagaaca tcaagcagac 7680
cgaggaggcc ctggacaacc accggctgcc tttccctctg ctggacgccg gcaccatcaa 7740
gatggtgaag gtgatcggca ggcggtccct gggcgtgcag cggatcttcg acatcggcct 7800
gcctcaggac cacaactttc tgctggccaa cggcgccatc gccgccaaca tggacatgcg 7860
cgtgcccgcc cagctgctgg gcctgctgct gctgtggttc cccggctcgc gatgcgacat 7920

ccagatgacc cagtctccat cctccctgtc tgcatctgta ggggacagag tcaccatcac 7980

ttgtcgggca agtcagggca tcagaaatta cttagcctgg tatcagcaaa aaccagggaa 8040

agcccctaag ctcctgatct atgctgcatc cactttgcaa tcaggggtcc catctcggtt 8100

cagtggcagt ggatctggga cagatttcac tctcaccatc agcagcctac agcctgaaga 8160

tgttgcaact tattactgtc aaaggtataa ccgtgcaccg tatacttttg gccaggggac 8220

caaggtggaa atcaaacgta cggtggctgc accatctgtc ttcatcttcc cgccatctga 8280

tgagcagttg aaatctggaa ctgcctctgt tgtgtgcctg ctgaataact tctatcccag 8340

agaggccaaa gtacagtgga aggtggataa cgccctccaa tcgggtaact cccaggagag 8400

tgtcacagag caggacagca aggacagcac ctacagcctc agcagcaccc tgacgctgag 8460

caaagcagac tacgagaaac acaaagtcta cgcctgcgaa gtcacccatc agggcctgag 8520

ctcgcccgtc acaaagagct tcaacagggg agagtgt 8557

<210> 136
<211> 501
<212> DNA
<213> Artificial

<220>
<223> Synthetic construct: partial coding sequence from
pTT3-HC-Ssp-GA-int-LC-1aa.

<400> 136
ccgggtaaat attgcctgtc cttcggcacc gagatcctga ccgtggagta cggccctctg 60

cctatcggca agatcgtgtc cgaagagatc aactgctccg tgtactccgt ggaccctgag 120

ggccgggtgt atactcaggc catcgcccag tggcacgacc ggggcgagca ggaggtgctg 180

gagtacgagc tggaggacgg ctccgtgatc cgggccacct ccgaccaccg gtttctgacc 240

accgactatc agctgctggc catcgaggag atcttcgccc ggcagctgga cctgctgacc 300

ctggagaaca tcaagcagac cgaggaggcc ctggacaacc accggctgcc tttccctctg 360

ctggacgccg gcaccatcaa gatggtgaag gtgatcggca ggcggtccct gggcgtgcag 420

cggatcttcg acatcggcct gcctcaggac cacaactttc tgctggccaa cggcgccatc 480

gccgccaact gtatggacat g 501

<210> 137
<211> 513
<212> DNA
<213> Artificial

<220>
<223> Synthetic construct:: relevant portion of coding sequence from
plasmid pTT3-HC-Ssp-GA-int-LC-3aa.

<400> 137
ccgggtaaag ccgagtattg cctgtccttc ggcaccgaga tcctgaccgt ggagtacggc 60

cctctgccta tcggcaagat cgtgtccgaa gagatcaact gctccgtgta ctccgtggac 120

cctgagggcc gggtgtatac tcaggccatc gcccagtggc acgaccgggg cgagcaggag 180

gtgctggagt acgagctgga ggacggctcc gtgatccggg ccacctccga ccaccggttt 240

ctgaccaccg actatcagct gctggccatc gaggagatct tcgcccggca gctggacctg 300

ctgaccctgg agaacatcaa gcagaccgag gaggccctgg acaaccaccg gctgcctttc 360

cctctgctgg acgccggcac catcaagatg gtgaaggtga tcggcaggcg gtccctgggc 420

gtgcagcgga tcttcgacat cggcctgcct caggaccaca actttctgct ggccaacggc 480

gccatcgccg ccaactgttt caacatggac atg 513

<210> 138
<211> 11
<212> PRT
<213> Pyrococcus sp.

<400> 138

Arg Gln Arg Ala Ile Lys Ile Leu Ala Asn Ser
1               5                   10

<210> 139
<211> 12
<212> PRT
<213> Pyrococcus sp.

<400> 139

His Asn Ser Tyr Tyr Gly Tyr Tyr Gly Tyr Ala Lys
1               5                   10

<210> 140
<211> 214
<212> PRT
<213> Artificial

<220>
<223> Synthetic construct: partial amino acid sequence encompassing
cleavage sites in Hedgehog-antibody constructs.

<400> 140

Cys Phe Thr Pro Glu Ser Thr Ala Leu Leu Glu Ser Gly Val Arg Lys
1               5                   10                  15

Pro Leu Gly Glu Leu Ser Ile Gly Asp Arg Val Leu Ser Met Thr Ala
            20                  25                  30

Asn Gly Gln Ala Val Tyr Ser Glu Val Ile Leu Phe Met Asp Arg Asn
        35                  40                  45

Leu Glu Gln Met Gln Asn Phe Val Gln Leu His Thr Asp Gly Gly Ala
    50                  55                  60

Val Leu Thr Val Thr Pro Ala His Leu Val Ser Val Trp Gln Pro Glu
65                  70                  75                  80

Ser Gln Lys Leu Thr Phe Val Phe Ala Asp Arg Ile Glu Glu Lys Asn

85                  90                  95

Gln Val Leu Val Arg Asp Val Glu Thr Gly Glu Leu Arg Pro Gln Arg
            100                 105                 110

Val Val Lys Val Gly Ser Val Arg Ser Lys Gly Val Val Ala Pro Leu
        115                 120                 125

Thr Arg Glu Gly Thr Ile Val Val Asn Ser Val Ala Ala Ser Cys Tyr
    130                 135                 140

Ala Val Ile Asn Ser Gln Ser Leu Ala His Trp Gly Leu Ala Pro Met
145                 150                 155                 160

Arg Leu Leu Ser Thr Leu Glu Ala Trp Leu Pro Ala Lys Glu Gln Leu
                165                 170                 175

His Ser Ser Pro Lys Val Val Ser Ser Ala Gln Gln Gln Asn Gly Ile
            180                 185                 190

His Trp Tyr Ala Asn Ala Leu Tyr Lys Val Lys Asp Tyr Val Leu Pro
        195                 200                 205

Gln Ser Trp Arg His Asp
    210

<210>  141
<211>  40
<212>  PRT
<213>  Artificial

<220>
<223>  Variant of 2A sequence.

<400>  141

Leu Leu Ala Ile His Pro Thr Glu Ala Arg His Lys Gln Lys Ile Val
1               5                   10                  15

Ala Pro Val Lys Gln Thr Leu Asn Phe Asp Leu Leu Lys Leu Ala Gly
            20                  25                  30

Asp Val Glu Ser Asn Pro Gly Pro
        35                  40

<210>  142
<211>  33
<212>  PRT
<213>  Artificial

<220>
<223>  Variant of 2A sequence.

<400>  142

Glu Ala Arg His Lys Gln Lys Ile Val Ala Pro Val Lys Gln Thr Leu

1               5                   10                  15

Asn Phe Asp Leu Leu Lys Leu Ala Gly Asp Val Glu Ser Asn Pro Gly
            20                  25                  30

Pro

<210>  143
<211>  20
<212>  DNA
<213>  Artificial

<220>
<223>  Synthetic construct:  oligonucleotide useful as a primer.

<400>  143
atcgtggcgc cagctctgcg                                                   20

<210>  144
<211>  20
<212>  DNA
<213>  Artificial

<220>
<223>  Synthetic construct:  oligonucleotide useful as a primer.

<400>  144
gcaactggcg gccaccgagt                                                   20

<210>  145
<211>  20
<212>  DNA
<213>  Artificial

<220>
<223>  Synthetic construct:  oligonucleotide useful as a primer.

<400>  145
cgcatagcaa ctggcggcca                                                   20

<210>  146
<211>  20
<212>  DNA
<213>  Artificial

<220>
<223>  Synthetic construct:  oligonucleotide useful as a primer.

<400>  146
gttgtgggcg gccaccgagt                                                   20

<210>  147
<211>  60
<212>  DNA
<213>  Artificial

<220>
<223>  Synthetic construct:  oligonucleotide useful as a primer.

<400>  147

ccactacacg cagaagagcc tctccctgtc tccgggtaaa tgcttcacgc cggagagcac 60

<210> 148
<211> 60
<212> DNA
<213> Artificial

<220>
<223> Synthetic construct: oligonucleotide useful as a primer.

<400> 148
gcagcaggcc cagcagctgg gcgggcacgc gcatgtccat gcactggctg ttgatcaccg 60

<210> 149
<211> 60
<212> DNA
<213> Artificial

<220>
<223> Synthetic construct: oligonucleotide useful as a primer.

<400> 149
gcagcaggcc cagcagctgg gcgggcacgc gcatgtccat atcgtggcgc cagctctgcg 60

<210> 150
<211> 60
<212> DNA
<213> Artificial

<220>
<223> Synthetic construct: oligonucleotide useful as a primer.

<400> 150
gcagcaggcc cagcagctgg gcgggcacgc gcatgtccat gcaactggcg gccaccgagt 60

<210> 151
<211> 60
<212> DNA
<213> Artificial

<220>
<223> Synthetic construct: oligonucleotide useful as a primer.

<400> 151
gcagcaggcc cagcagctgg gcgggcacgc gcatgtccat cgcatagcaa ctggcggcca 60

<210> 152
<211> 60
<212> DNA
<213> Artificial

<220>
<223> Synthetic construct: oligonucleotide useful as a primer.

<400> 152
gcagcaggcc cagcagctgg gcgggcacgc gcatgtccat gttgtgggcg gccaccgagt 60

<210> 153
<211> 40
<212> DNA
<213> Artificial

<220>
<223>  Synthetic construct:  oligonucleotide useful as a primer.

<400>  153
atggacatgc gcgtgcccgc ccagctgctg ggcctgctgc                          40


<210>  154
<211>  41
<212>  DNA
<213>  Artificial

<220>
<223>  Synthetic construct:  oligonucleotide useful as a primer.

<400>  154
tttacccgga gacagggaga ggctcttctg cgtgtagtgg t                        41


<210>  155
<211>  8533
<212>  DNA
<213>  Artificial

<220>
<223>  Synthetic construct:  nucleotide sequence of plasmid  pTT3-D2E7
       Heavy Chain - Hh-C17- D2E7 Light Chain.

<400>  155
gcggccgctc gaggccggca aggccggatc ccccgacctc gacctctggc taataaagga      60

aatttatttt cattgcaata gtgtgttgga attttttgtg tctctcactc ggaaggacat     120

atgggagggc aaatcatttg gtcgagatcc ctcggagatc tctagctaga ggatcgatcc     180

ccgccccgga cgaactaaac ctgactacga catctctgcc ccttcttcgc ggggcagtgc     240

atgtaatccc ttcagttggt tggtacaact tgccaactgg gccctgttcc acatgtgaca     300

cgggggggga ccaaacacaa aggggttctc tgactgtagt tgacatcctt ataaatggat     360

gtgcacattt gccaacactg agtggctttc atcctggagc agactttgca gtctgtggac     420

tgcaacacaa cattgccttt atgtgtaact cttggctgaa gctcttacac caatgctggg     480

ggacatgtac ctcccagggg cccaggaaga ctacgggagg ctacaccaac gtcaatcaga     540

ggggcctgtg tagctaccga taagcggacc ctcaagaggg cattagcaat agtgtttata     600

aggccccctt gttaacccta aacgggtagc atatgcttcc cgggtagtag tatatactat     660

ccagactaac cctaattcaa tagcatatgt tacccaacgg gaagcatatg ctatcgaatt     720

agggttagta aaagggtcct aaggaacagc gatatctccc accccatgag ctgtcacggt     780

tttatttaca tggggtcagg attccacgag ggtagtgaac cattttagtc acaagggcag     840

tggctgaaga tcaaggagcg ggcagtgaac tctcctgaat cttcgcctgc ttcttcattc     900

tccttcgttt agctaataga ataactgctg agttgtgaac agtaaggtgt atgtgaggtg     960

ctcgaaaaca aggtttcagg tgacgccccc agaataaaat ttggacgggg ggttcagtgg    1020

tggcattgtg ctatgacacc aatataaccc tcacaaaccc cttgggcaat aaatactagt    1080

gtaggaatga aacattctga atatctttaa caatagaaat ccatggggtg gggacaagcc    1140

gtaaagactg gatgtccatc tcacacgaat ttatggctat gggcaacaca taatcctagt    1200

gcaatatgat actggggtta ttaagatgtg tcccaggcag ggaccaagac aggtgaacca 1260
tgttgttaca ctctatttgt aacaagggga aagagagtgg acgccgacag cagcggactc 1320
cactggttgt ctctaacacc cccgaaaatt aaacggggct ccacgccaat ggggcccata 1380
aacaaagaca agtggccact cttttttttg aaattgtgga gtgggggcac gcgtcagccc 1440
ccacacgccg ccctgcggtt ttggactgta aaataagggt gtaataactt ggctgattgt 1500
aaccccgcta accactgcgg tcaaaccact tgcccacaaa accactaatg gcaccccggg 1560
gaatacctgc ataagtaggt gggcgggcca agataggggc gcgattgctg cgatctggag 1620
gacaaattac acacacttgc gcctgagcgc caagcacagg gttgttggtc ctcatattca 1680
cgaggtcgct gagagcacgg tgggctaatg ttgccatggg tagcatatac tacccaaata 1740
tctggatagc atatgctatc ctaatctata tctgggtagc ataggctatc ctaatctata 1800
tctgggtagc atatgctatc ctaatctata tctgggtagt atatgctatc ctaatttata 1860
tctgggtagc ataggctatc ctaatctata tctgggtagc atatgctatc ctaatctata 1920
tctgggtagt atatgctatc ctaatctgta tccgggtagc atatgctatc ctaatagaga 1980
ttagggtagt atatgctatc ctaatttata tctgggtagc atatactacc caaatatctg 2040
gatagcatat gctatcctaa tctatatctg ggtagcatat gctatcctaa tctatatctg 2100
ggtagcatag gctatcctaa tctatatctg ggtagcatat gctatcctaa tctatatctg 2160
ggtagtatat gctatcctaa tttatatctg ggtagcatag gctatcctaa tctatatctg 2220
ggtagcatat gctatcctaa tctatatctg ggtagtatat gctatcctaa tctgtatccg 2280
ggtagcatat gctatcctca tgataagctg tcaaacatga gaattttctt gaagacgaaa 2340
gggcctcgtg atacgcctat ttttataggt taatgtcatg ataataatgg tttcttagac 2400
gtcaggtggc acttttcggg gaaatgtgcg cggaacccct atttgtttat ttttctaaat 2460
acattcaaat atgtatccgc tcatgagaca ataaccctga taaatgcttc aataatattg 2520
aaaaaggaag agtatgagta ttcaacattt ccgtgtcgcc cttattccct tttttgcggc 2580
attttgcctt cctgtttttg ctcacccaga aacgctggtg aaagtaaaag atgctgaaga 2640
tcagttgggt gcacgagtgg gttacatcga actggatctc aacagcggta agatccttga 2700
gagttttcgc cccgaagaac gttttccaat gatgagcact tttaaagttc tgctatgtgg 2760
cgcggtatta tcccgtgttg acgccgggca agagcaactc ggtcgccgca tacactattc 2820
tcagaatgac ttggttgagt actcaccagt cacagaaaag catcttacgg atggcatgac 2880
agtaagagaa ttatgcagtg ctgccataac catgagtgat aacactgcgg ccaacttact 2940
tctgacaacg atcggaggac cgaaggagct aaccgctttt ttgcacaaca tgggggatca 3000
tgtaactcgc cttgatcgtt gggaaccgga gctgaatgaa gccataccaa acgacgagcg 3060
tgacaccacg atgcctgcag caatggcaac aacgttgcgc aaactattaa ctggcgaact 3120
acttactcta gcttcccggc aacaattaat agactggatg gaggcggata aagttgcagg 3180
accacttctg cgctcggccc ttccggctgg ctggtttatt gctgataaat ctggagccgg 3240

tgagcgtggg tctcgcggta tcattgcagc actggggcca gatggtaagc cctcccgtat 3300
cgtagttatc tacacgacgg ggagtcaggc aactatggat gaacgaaata gacagatcgc 3360
tgagataggt gcctcactga ttaagcattg gtaactgtca gaccaagttt actcatatat 3420
actttagatt gatttaaaac ttcattttta atttaaaagg atctaggtga agatcctttt 3480
tgataatctc atgaccaaaa tcccttaacg tgagttttcg ttccactgag cgtcagaccc 3540
cgtagaaaag atcaaaggat cttcttgaga tccttttttt ctgcgcgtaa tctgctgctt 3600
gcaaacaaaa aaaccaccgc taccagcggt ggtttgtttg ccggatcaag agctaccaac 3660
tctttttccg aaggtaactg gcttcagcag agcgcagata ccaaatactg ttcttctagt 3720
gtagccgtag ttaggccacc acttcaagaa ctctgtagca ccgcctacat acctcgctct 3780
gctaatcctg ttaccagtgg ctgctgccag tggcgataag tcgtgtctta ccgggttgga 3840
ctcaagacga tagttaccgg ataaggcgca gcggtcgggc tgaacggggg gttcgtgcac 3900
acagcccagc ttggagcgaa cgacctacac cgaactgaga tacctacagc gtgagctatg 3960
agaaagcgcc acgcttcccg aagggagaaa ggcggacagg tatccggtaa gcggcagggt 4020
cggaacagga gagcgcacga gggagcttcc agggggaaac gcctggtatc tttatagtcc 4080
tgtcgggttt cgccacctct gacttgagcg tcgatttttg tgatgctcgt caggggggcg 4140
gagcctatgg aaaaacgcca gcaacgcggc ctttttacgg ttcctggcct tttgctggcc 4200
ttttgctcac atgttctttc ctgcgttatc ccctgattct gtggataacc gtattaccgc 4260
ctttgagtga gctgataccg ctcgccgcag ccgaacgacc gagcgcagcg agtcagtgag 4320
cgaggaagcg gaagagcgcc caatacgcaa accgcctctc cccgcgcgtt ggccgattca 4380
ttaatgcagc tggcacgaca ggtttcccga ctggaaagcg ggcagtgagc gcaacgcaat 4440
taatgtgagt tagctcactc attaggcacc ccaggcttta cactttatgc ttccggctcg 4500
tatgttgtgt ggaattgtga gcggataaca atttcacaca ggaaacagct atgaccatga 4560
ttacgccaag ctctagctag aggtcgacca attctcatgt ttgacagctt atcatcgcag 4620
atccgggcaa cgttgttgcc attgctgcag gcgcagaact ggtaggtatg gaagatctat 4680
acattgaatc aatattggca attagccata ttagtcattg gttatatagc ataaatcaat 4740
attggctatt ggccattgca tacgttgtat ctatatcata atatgtacat ttatattggc 4800
tcatgtccaa tatgaccgcc atgttgacat tgattattga ctagttatta atagtaatca 4860
attacggggt cattagttca tagcccatat atggagttcc gcgttacata acttacggta 4920
aatggcccgc ctggctgacc gcccaacgac ccccgcccat tgacgtcaat aatgacgtat 4980
gttcccatag taacgccaat agggactttc cattgacgtc aatgggtgga gtatttacgg 5040
taaactgccc acttggcagt acatcaagtg tatcatatgc caagtccgcc ccctattgac 5100
gtcaatgacg gtaaatggcc cgcctggcat tatgcccagt acatgacctt acgggacttt 5160
cctacttggc agtacatcta cgtattagtc atcgctatta ccatggtgat gcggttttgg 5220
cagtacacca atgggcgtgg atagcggttt gactcacggg gatttccaag tctccacccc 5280

attgacgtca atgggagttt gttttggcac caaaatcaac gggactttcc aaaatgtcgt 5340
aataaccccg ccccgttgac gcaaatgggc ggtaggcgtg tacggtggga ggtctatata 5400
agcagagctc gtttagtgaa ccgtcagatc ctcactctct tccgcatcgc tgtctgcgag 5460
ggccagctgt tgggctcgcg gttgaggaca aactcttcgc ggtctttcca gtactcttgg 5520
atcggaaacc cgtcggcctc cgaacggtac tccgccaccg agggacctga gcgagtccgc 5580
atcgaccgga tcggaaaacc tctcgagaaa ggcgtctaac cagtcacagt cgcaaggtag 5640
gctgagcacc gtggcgggcg gcagcgggtg gcggtcgggg ttgtttctgg cggaggtgct 5700
gctgatgatg taattaaagt aggcggtctt gagacggcgg atggtcgagg tgaggtgtgg 5760
caggcttgag atccagctgt tggggtgagt actccctctc aaaagcgggc attacttctg 5820
cgctaagatt gtcagtttcc aaaaacgagg aggatttgat attcacctgg cccgatctgg 5880
ccatacactt gagtgacaat gacatccact ttgcctttct ctccacaggt gtccactccc 5940
aggtccaagt ttgggcgcca ccatggagtt tgggctgagc tggctttttc ttgtcgcgat 6000
tttaaaaggt gtccagtgtg aggtgcagct ggtggagtct gggggaggct tggtacagcc 6060
cggcaggtcc ctgagactct cctgtgcggc ctctggattc acctttgatg attatgccat 6120
gcactgggtc cggcaagctc cagggaaggg cctggaatgg gtctcagcta tcacttggaa 6180
tagtggtcac atagactatg cggactctgt ggagggccga ttcaccatct ccagagacaa 6240
cgccaagaac tccctgtatc tgcaaatgaa cagtctgaga gctgaggata cggccgtata 6300
ttactgtgcg aaagtctcgt accttagcac cgcgtcctcc cttgactatt ggggccaagg 6360
taccctggtc accgtctcga gtgcgtcgac caagggccca tcggtcttcc ccctggcacc 6420
ctcctccaag agcacctctg ggggcacagc ggccctgggc tgcctggtca aggactactt 6480
ccccgaaccg gtgacggtgt cgtggaactc aggcgccctg accagcggcg tgcacacctt 6540
cccggctgtc ctacagtcct caggactcta ctccctcagc agcgtggtga ccgtgccctc 6600
cagcagcttg ggcacccaga cctacatctg caacgtgaat cacaagccca gcaacaccaa 6660
ggtggacaag aaagttgagc ccaaatcttg tgacaaaact cacacatgcc caccgtgccc 6720
agcacctgaa ctcctggggg gaccgtcagt cttcctcttc cccccaaaac ccaaggacac 6780
cctcatgatc tcccggaccc ctgaggtcac atgcgtggtg gtggacgtga gccacgaaga 6840
ccctgaggtc aagttcaact ggtacgtgga cggcgtggag gtgcataatg ccaagacaaa 6900
gccgcgggag gagcagtaca acagcacgta ccgtgtggtc agcgtcctca ccgtcctgca 6960
ccaggactgg ctgaatggca aggagtacaa gtgcaaggtc tccaacaaag ccctcccagc 7020
ccccatcgag aaaaccatct ccaaagccaa agggcagccc cgagaaccac aggtgtacac 7080
cctgccccca tcccgggatg agctgaccaa gaaccaggtc agcctgacct gcctggtcaa 7140
aggcttctat cccagcgaca tcgccgtgga gtgggagagc aatgggcagc cggagaacaa 7200
ctacaagacc acgcctcccg tgctggactc cgacggctcc ttcttcctct acagcaagct 7260
caccgtggac aagagcaggt ggcagcaggg gaacgtcttc tcatgctccg tgatgcatga 7320

ggctctgcac aaccactaca cgcagaagag cctctccctg tctccgggta aatgcttcac 7380

gccggagagc acagcgctgc tggagagtgg agtccggaag ccgctcggcg agctctctat 7440

cggagatcgt gttttgagca tgaccgccaa cggacaggcc gtctacagcg aagtgatcct 7500

cttcatggac cgcaacctcg agcagatgca aaactttgtg cagctgcaca cggacggtgg 7560

agcagtgctc acggtgacgc cggctcacct ggttagcgtt tggcagccgg agagccagaa 7620

gctcacgttt gtgtttgcgg atcgcatcga ggagaagaac caggtgctcg tacgggatgt 7680

ggagacgggc gagctgaggc cccagcgagt cgtcaaggtg ggcagtgtgc gcagtaaggg 7740

cgtggtcgcg ccgctgaccc gcgagggcac cattgtggtc aactcggtgg ccgccagttg 7800

ctatgcggtg atcaacagcc agtcgatgga catgcgcgtg cccgcccagc tgctgggcct 7860

gctgctgctg tggttccccg gctcgcgatg cgacatccag atgacccagt ctccatcctc 7920

cctgtctgca tctgtagggg acagagtcac catcacttgt cgggcaagtc agggcatcag 7980

aaattactta gcctggtatc agcaaaaacc agggaaagcc cctaagctcc tgatctatgc 8040

tgcatccact ttgcaatcag gggtcccatc tcggttcagt ggcagtggat ctgggacaga 8100

tttcactctc accatcagca gcctacagcc tgaagatgtt gcaacttatt actgtcaaag 8160

gtataaccgt gcaccgtata cttttggcca ggggaccaag gtggaaatca aacgtacggt 8220

ggctgcacca tctgtcttca tcttcccgcc atctgatgag cagttgaaat ctggaactgc 8280

ctctgttgtg tgcctgctga ataacttcta tcccagagag gccaaagtac agtggaaggt 8340

ggataacgcc ctccaatcgg gtaactccca ggagagtgtc acagagcagg acagcaagga 8400

cagcacctac agcctcagca gcaccctgac gctgagcaaa gcagactacg agaaacacaa 8460

agtctacgcc tgcgaagtca cccatcaggg cctgagctcg cccgtcacaa agagcttcaa 8520

caggggagag tgt 8533

<210> 156
<211> 447
<212> DNA
<213> Artificial

<220>
<223> Synthetic construct: partial coding sequence of plasmid
pTT3-HC-C17-sc-LC.

<400> 156
ccgggtaaat gcttcacgcc ggagagcaca gcgctgctgg agagtggagt ccggaagccg 60

ctcggcgagc tctctatcgg agatcgtgtt ttgagcatga ccgccaacgg acaggccgtc 120

tacagcgaag tgatcctctt catggaccgc aacctcgagc agatgcaaaa ctttgtgcag 180

ctgcacacgg acggtggagc agtgctcacg gtgacgccgg ctcacctggt tagcgtttgg 240

cagccggaga gccagaagct cacgtttgtg tttgcggatc gcatcgagga gaagaaccag 300

gtgctcgtac gggatgtgga gacgggcgag ctgaggcccc agcgagtcgt caaggtgggc 360

agtgtgcgca gtaagggcgt ggtcgcgccg ctgacccgcg agggcaccat tgtggtcaac 420

tcggtggccg ccagttgcat ggacatg                                          447

<210>  157
<211>  447
<212>  DNA
<213>  Artificial

<220>
<223>  Synthetic construct:  partial coding sequence from plasmid
       pTT3-HC-C17-hn-LC.

<400>  157
ccgggtaaat gcttcacgcc ggagagcaca gcgctgctgg agagtggagt ccggaagccg      60

ctcggcgagc tctctatcgg agatcgtgtt ttgagcatga ccgccaacgg acaggccgtc     120

tacagcgaag tgatcctctt catggaccgc aacctcgagc agatgcaaaa ctttgtgcag     180

ctgcacacgg acggtggagc agtgctcacg gtgacgccgg ctcacctggt tagcgtttgg     240

cagccggaga gccagaagct cacgtttgtg tttgcggatc gcatcgagga gaagaaccag     300

gtgctcgtac gggatgtgga gacgggcgag ctgaggcccc agcgagtcgt caaggtgggc     360

agtgtgcgca gtaagggcgt ggtcgcgccg ctgacccgcg agggcaccat tgtggtcaac     420

tcggtggccg cccacaacat ggacatg                                          447

<210>  158
<211>  660
<212>  DNA
<213>  Artificial

<220>
<223>  Synthetic construct:  partial coding sequence from
       pTT3-HC-C25-Hint-LC.

<400>  158
ccgggtaaat gcttcacgcc ggagagcaca gcgctgctgg agagtggagt ccggaagccg      60

ctcggcgagc tctctatcgg agatcgtgtt ttgagcatga ccgccaacgg acaggccgtc     120

tacagcgaag tgatcctctt catggaccgc aacctcgagc agatgcaaaa ctttgtgcag     180

ctgcacacgg acggtggagc agtgctcacg gtgacgccgg ctcacctggt tagcgtttgg     240

cagccggaga gccagaagct cacgtttgtg tttgcggatc gcatcgagga gaagaaccag     300

gtgctcgtac gggatgtgga gacgggcgag ctgaggcccc agcgagtcgt caaggtgggc     360

agtgtgcgca gtaagggcgt ggtcgcgccg ctgacccgcg agggcaccat tgtggtcaac     420

tcggtggccg ccagttgcta tgcggtgatc aacagccagt cgctggccca ctggggactg     480

gctcccatgc gcctgctgtc cacgctggag gcgtggctgc ccgccaagga gcagttgcac     540

agttcgccga aggtggtgag ctcggcgcag cagcagaatg gcatccattg gtatgccaat     600

gcgctctaca aggtcaagga ctacgttctg ccgcagagct ggcgccacga tatggacatg     660

## Description

### CROSS-REFERENCES TO RELATED APPLICATIONS

This application claims the benefit of U.S. Provisional Application No. 60/701855, filed July 21, 2005, which is incorporated herein by reference in entirety.

### STATEMENT ON FEDERALLY SPONSORED RESEARCH OR DEVELOPMENT

Not applicable

### REFERENCE TO SEQUENCE LISTING, A TABLE, OR A COMPUTER PROGRAM LISTING COMPACT DISK APPENDIX

Not Applicable (sequence listing provided but not as compact disk appendix).

### BACKGROUND OF THE INVENTION

The field of the present invention is molecular biology, especially as generally related to the area of recombinant protein expression, and the expression and processing, including post-translational processing, of recombinant polyproteins or pre-proteins in particular.

The use of antibodies as diagnostic tools and therapeutic modalities has found increasing use in recent years. The first FDA-approved monoclonal antibody, OKT3 (Johnson and Johnson) was approved for the treatment of patients with kidney transplant rejection. Herceptin (trademark of Genentech Inc., South San Francisco, CA), a humanized monoclonal antibody for treatment of patients with metastatic breast cancer, was approved in 1998. Numerous antibody-based therapies are showing promise in various stages of clinical development. One limitation in widespread clinical application of antibody technology is that typically large amounts of antibody are required for therapeutic efficacy and the costs associated with sufficient production are significant. Chinese Hamster Ovary (CHO) cells and NS0 myeloma cells are the most commonly used mammalian cell lines for commercial scale production of glycosylated human proteins such as antibodies and other biotherapeutics (Humphreys and Glover 2001. Curr. Opin. Drug Discov. Devel. 4:172-85). Mammalian cell line production yields typically range from 50-250 mg/L for 5-7 day culture in a batch fermentor or 300-600 mg/L in 7-12 days in fed batch fermentors. Non-glycosylated immunoglobulin proteins can be successfully produced in yeast or E. coli (see, e.g., Humphreys DP, et al., 2000, Protein Expr Purif. 20(2):252-64), however most successes in bacterial expression systems have been with antibody fragments (Humphreys, D.P. 2003. Curr. Opin. Drug Discov. Devel. 2003 6:188-96).

An important development in the field of expressing multiple gene segments or genes has been the discovery of inteins (see, e.g., Hirata, R et al., 1990, J. Biol. Chem. 265:6726-6733; Kane, PM et al., 1990, Science 250:, 651-657; Xu, M-Q and Perler, FB, 1996, EMBO Journal 15(19):5146-5153). Inteins are considered the protein equivalent of gene introns and facilitate protein splicing. As noted in US 7026526 by Snell K., protein splicing is a process in which an interior region of a precursor protein (an intein) is excised and the flanking regions of the protein (exteins) are ligated to form the mature protein. This process has been observed in numerous proteins from both prokaryotes and eukaryotes (Perler, F. B., Xu, M. Q., Paulus, H. Current Opinion in Chemical Biology 1997, 1, 292-299; Perler, F. B. Nucleic Acids Research 1999, 27, 346-347). The intein unit contains the necessary components needed to catalyze protein splicing and often contains an endonuclease domain that participates in intein mobility (Perler, F. B., et al., Nucleic Acids Research 1994, 22, 1127-1127).

While the main focus of intein-based systems has been on the generation of purification technologies and new fusion proteins from expressing gene segments, US 7026526 reports DNA constructs with modified inteins for expression of multiple gene products as separate proteins to achieve stacked traits in plants. Still lacking, however, is an indication that those systems can be successfully used for expression of separate proteins that assemble into functional multimeric proteins, extracellularly secreted proteins, mammalian proteins, or proteins produced in eukaryotic host cells. It is noteworthy that immunoglobulins fall into all of these categories.

Compounding the difficulty of extending the modified intein approach of US 7026526 to other genes or purposes is the recognition of the potential importance of the contributions of the desired extein gene segments relative to the intein system that is involved. Paulus reports, "Indeed, protein splicing, even though catalyzed entirely by the intein, can be strikingly influenced by extein sequences. This influence is shown by the fact that the expression of chimeric protein splicing systems, in which intein sequences are inserted in-frame between foreign coding sequences, often leads to substantial side reactions, such as cleavage at the upstream or downstream splice junctions (Xu M-Q, et al., 1993, Cell 75:1371-77; and Shingledecker K, et al., 1998, Gene 207:187-95). This suggests that the ability of inteins to assume a structure optimal for protein splicing without side reactions has evolved in the context of specific exteins." See Paulus H, 2000, Protein splicing and related forms of protein autoprocessing, Annu. Rev. Biochem. 69:447-96. Another commentator states: "Although it is possible to introduce desirable properties and activities into proteins using rational design, subtle changes necessary to make an engineered product efficient and practical are often still beyond our predictive capacity (Shao, Z. and Arnold, F.H. 1996. Curr. Opin. Struct. Biol. 6, 513-518). ...Nevertheless, the regions immediately flanking inteins have been found to affect the efficiency of splicing (Chong, S. et al., 1998, Nucleic Acids Res. 26, 5109-5115; Southworth, M.W. et al., 199, Biotechniques 27, 110-114) and some protein hosts might be incompatible with intein activity. Although high expression and product purity are important considerations, they are moot if the final product is inactive." See Amitai G and Pietrokovski, 1999, Nature Biotechnology 17:854-855:

Therefore, in a modified intein system where a preferred outcome is cleavage without re-ligation, the presence of a foreign extein relative to a given intein sequence may affect a practically efficient combination of precise cleavages, absence of re-ligation, and absence of side reactions. Clearly the adaptation of a modified intein approach for recombinant production of certain proteins that retain functional activity as final product, e.g., immunoglobulins and other biotherapeutics, represents a substantial challenge for innovation.

In the present invention this challenge has been taken up not only for intein-based systems but also has been explored in a pioneering sense for useful applications regarding hedgehog domains. Proteins in the *hedgehog* family are intercellular signaling molecules essential for patterning in vertebrate embryos. See, e.g., Mann, R.K. and Beachy, P.A. (2000) Biochim. Biophys. Acta. 1529, 188-202; Beachy, PA, (1997) Cold Spring Harb Symp Quant Biol 62: 191-204. Native hedgehog precursor proteins are cleaved into C-terminal (Hh-C) and N-terminal fragments (Hh-N) by an autoprocessing reaction that has similarity to protein splicing. The hedgehog system presents an untested opportunity for the creative development of systems including modified versions suitable for expression of multiple separate protein segments.

Previous attempts to express a full length antibody/immunoglobulin molecule via recombinant DNA technology using a single vector have met with limited success, typically resulting in significantly dissimilar levels of expression of the heavy and light chains of the antibody/immunoglobulin molecule, and more particularly, a lower level of expression for the second gene. Other factors may require relatively higher expression levels of one chain compared to the other for optimal production of a properly assembled, multimeric antibody or functional fragment thereof. Thus one problem is a suboptimal stoichiometry of expression of heavy and light chains within the cell which results in an overall low yield of assembled, multimeric antibody. Fang et al. indicate that in order to express high levels of a fully biological functional antibody from a single vector, equimolar expression of the heavy and light chains is required (see Fang et al., 2005, Nature Biotechnology 23:584-590; US Patent Publication 2004/0265955A1). Additionally, conventional expression systems relying on vector systems that independently express multiple polypeptides are significantly affected by such factors as promoter interactions (e.g., promoter interference). These interactions may compromise efficient expression of the genes and/or assembly of the expressed chains, or require the use of more than one vector (see, e.g., US Patent 6331415, Cabilly et al.). The requirement of multiple vectors is disadvantageous due to potential complications such as loss of one or more of the individual vectors in addition to generally needing additional manipulations.

Other factors that limit the ability to express two or more coding sequences from a single vector include the packaging capacity of the vector itself. For example, in considering the appropriate vector/coding sequence, factors to be considered include the packaging capacity of the vector (e.g., approx. 4,500 bp for adeno-associated virus, AAV); the duration of in vitro/in vivo expression of the recombinant protein by a vector-transfected cell or organ (e.g., short term expression for adenoviral vectors); the cell types supporting efficient infection by the vector if a viral vector is used; and the desired expression level of the gene product(s). The requirement for controlled expression of two or more gene products together with the packaging limitations of viral vectors such as adenovirus and AAV limits the choices with respect to vector construction and systems for expression of certain genes such as immunoglobulins or fragments thereof.

In further approaches to express two or more protein or polypeptide sequences from a single vector, two or more promoters or a single promoter and an internal ribosome entry site (IRES) sequence between the coding sequences of interest are used to drive expression of individual coding sequences. The use of two promoters within a single vector can result in low protein expression due to promoter interference. When two coding sequences are separated by an IRES sequence, the translational expression of the second coding sequence is often significantly weaker than that of the first (Furler et al. 2001. Gene Therapy 8:864-873). US Patent Publication 2004/0241821 describes flavivirus vectors in which a heterologous coding sequence is incorporated downstream of the virus polyprotein coding sequence, and separated therefrom by an IRES. A nuclear-anchored vector strategy for recombinant gene expression, including fusion proteins in which segments are separated by protease recognition sites, is described in US Patent Publication 2005/0026137.

The linking of proteins in the form of polyproteins in a single open reading frame (sORF) is a strategy observed in the replication of many natural viruses including the picornaviridae. Upon translation, virus-encoded proteinases mediate rapid intramolecular (cis) cleavage of a polyprotein to yield discrete mature protein products. Foot and Mouth Disease viruses (FMDV) are a group within the picornaviridae which express a single, long open reading frame encoding a polyprotein of approximately 225 kD. The full length translation product undergoes rapid intramolecular (cis) cleavage at the C-terminus of a 2A region occurring between the capsid protein precursor (P1-2A) and replicative domains of the polyprotein 2BC and P3, and this cleavage is mediated by the 2A region itself via a ribosomal stutter mechanism (Ryan et al. 1991. J. Gen. Virol. 72:2727-2732); Vakharia et al. 1987. J. Virol. 61:3199-3207). The essential amino acid residues for expression of the cleavage activity by the FMDV 2A region have been identified. The 2A and similar domains have also been characterized from aphthoviridae and cardioviridae of the picornavirus family (Donnelly et al. 1997. J. Gen. Virol. 78:13-21).

In still other attempts to use proteolytic processing techniques, early descriptions of recombinant insulin production include, e.g., EP055945 (Genentech); and EP037723 (The Regents of the University of California). It is a tremendous leap, however, to be able to apply such efforts in the context of exploiting recombinant expression of much larger and more complex functional proteins such as immunoglobulins. Examples of functional antibody molecules can involve heteromultimers requiring assembly of four or more chains (e.g., two immunoglobulin heavy chains and two light chains).

There remains a need for alternative and/or improved expression systems for generating recombinant proteins. A particular need is reflected in the area of efficient and/or correct expression of full length immunoglobulins and antigen-binding fragments thereof which provide advantages relative to currently available technology. The present invention addresses these needs by providing single vector constructs using a variety of strategies such as inteins, hedgehog autoprocessing segments, autocatalytic viral proteases, and variations thereof respectively. Independently, the need of efficient multimeric (e.g., immunoglobulin) assembly is addressed by adjusting the stoichiometric relationship of the subunits (e.g., heavy and light chains or fragments thereof). In embodiments, the constructs in a sORF encode a self-processing peptide component for expression of an industrially or biologically functional polypeptide, such as an enzyme, immunoglobulin, cytokine, chemokine, receptor, hormone, components of a two hybrid system, or other multi-subunit proteins of interest.

### BRIEF SUMMARY OF THE INVENTION

The present invention provides expression cassettes, vectors, recombinant host cells and methods for the recombinant expression and processing, including post-translational processing, of recombinant polyproteins and pre-proteins.
In an embodiment, the invention provides an expression vector for generating one or more recombinant protein products comprising a sORF insert; said sORF insert comprising a first nucleic acid sequence encoding a first polypeptide, an intervening nucleic acid sequence encoding a first protein cleavage site, and a second nucleic acid sequence encoding a second polypeptide; wherein said intervening nucleic acid sequence encoding said first protein cleavage site is operably positioned between said first nucleic acid sequence and said second nucleic acid sequence; and wherein said expression vector is capable of expressing a sORF polypeptide cleavable at said first protein cleavage site. In an embodiment, the first protein cleavage site comprises a self-processing cleavage site. In an embodiment, the self-processing cleavage site comprises an intein segment or modified intein segment, wherein the modified (or unmodified) intein segment permits cleavage but not complete ligation of expressed first polypeptides to expressed second polypeptides. In an embodiment, the self-processing cleavage site comprises a hedgehog segment or modified hedgehog segment, wherein the modified (or unmodified) hedgehog segment permits cleavage of expressed first polypeptides and expressed second polypeptides. In an embodiment, multiple separate proteins (e.g., first polypeptides, second polypeptides, third polypeptides, etc.) are expressed. In an embodiment, the first polypeptide and second polypeptide are capable of multimeric assembly. In an embodiment, at least one of said first polypeptide and second polypeptide are capable of extracellular secretion. In an embodiment, at least one of said first polypeptide and second polypeptide are of mammalian origin. In an embodiment, vectors and methods generating assembled antibodies are provided.

In embodiments, the invention provides constructs and methods for recombinant expression of multiple separate proteins. In particular embodiments, the proteins are capable of extracellular secretion. In particular embodiments, the proteins are of mammalian origin. In particular embodiments, the proteins are capable of multimeric assembly. In particular embodiments, the proteins are immunoglobulins.

In an embodiment, the incorporation of a protease recognition site, cleavable signal peptide or an autoprocessing polypeptide sequence (including an intein, a C-terminal auto-processing domain of hedgehog from drosophila, mouse, human, and other species (Dassa et al, Trends in Genetics, Vol. 20 No. 11 Nov, 2004, 533-542; Ibrahim et al, Biochimica et Biophysics Acta 1760 (2006) 347-355). We note that in some cases an autoprocessing polypeptide sequence can be referred to as a proteolytic site in connection with proteolytic processing. The C-terminal auto-processing domains of warthog, groundhog, and other hog-containing gene from nematodes such as Caenorhabditis elegans (Snell EA et al, Proc. R. Soc. B (2006) 273, 401-407; Aspock et al, Genome Research, 1999, 9:909-923); and Hoglet-C autoprocessing domain from choanoflagellate (Aspock et al, Genome Research, 1999, 9:909-923) are used. A-type bacterial intein-like (BIL) domains such as those from bacteria such as Clostridium thermocellum, and B-type BIL domains from bacteria such as Rhodobacter sphaeroides (Dassa et al, Journal of Biological Chemistry, Vol. 279, No. 31, July 30, 32001-32007), in wild type, truncated, or otherwise modified forms) into a recombinant pre-protein sequence allows efficient expression and cleavage of a pro-protein such that the bioactive portion is released or so that desired proteins expressed within a polyprotein are released. This embodiment eliminates the need for co-expression of the pro-protein's natural proteolytic processing enzymes. Alternatively, a protease cognate to the particular recognition site can be expressed coextensively with the pre-protein sequence, with a protease recognition site there between such that the protease can be released via proteolytic action and the precursor portion of the pre-protein is then released by subsequent proteolytic cleavage, such that the active portion of the pre-protein is released. In a still further embodiment, the 2A autoproteolytic processing peptide sequence can be engineered into the pre-protein between the mature (bioactive) portion and the precursor protein so that there is a self-processing of the engineered recombinant protein after expression.

In another embodiment of the invention, the present invention provides a method for efficient expression of recombinant immunoglobulin molecules, by recombinantly expressing a polyprotein comprising at least one heavy chain region and at least one light chain regions, wherein said regions are separated by one or more protease recognition sites, signal peptides, intein sequences which mediate cleavage but not joining of polypeptides, hedgehog sequence, other intein-like or hedgehog-like autoprocessing sequence or variation thereof, or by sequences such as as the 2A peptide that separate the flanking peptides during translation. In a further embodiment, a protease can be expressed as part of the polyprotein, separated from the remainder of the polyprotein by protease recognition sites, and wherein each protease recognition site is cognate to the concomitantly expressed protease. Then proteolytic or signal peptidase action releases the protease and the other individual proteins from the primary translation product. The above described methods for separating protein subunits in a poly protein can also be used in combination to achieve desired cleavage and protein expression outcomes.

In the case of an embodiment of immunoglobulin expression, the duplication of the light chain coding region allows for improved assembly and/or expression of the complete immunoglobulin molecule over the situation where the light chain coding regions are present in the expression cassette and/or expression vector at a 1:1 ratio with the heavy chain coding region. In the context of the present invention, heavy and light chain proteins can be functional fragments of the naturally occurring heavy and light chains (a functional fragment retains the ability to bind to its counterpart antibody chain and the ability to bind the cognate antigen is also retained, as well known in the art. Thus the invention provides constructs and methods wherein the coding region ratio of light chain component to heavy chain component is either 1:1 or greater than 1:1. For example, in an embodiment the L:H ratio is 2:1 or greater than 2:1; in other embodiments the ratio is 3:1, 3:2, 4:1, or greater than 4:1.

In a preferred aspect of the invention, the light chain immunoglobulin coding sequence, or component fragment thereof, is duplicated within the polyprotein coding sequence, and heavy and light chain immunoglobulin coding sequences are present at a molar ratio of about 2 light chains to about one heavy chains, and expressed at a ratio of greater than 1:1 light chain:heavy chain. The light and heavy chain sequences are linked in the polyprotein by protease cleavage sites, signal (or leader) peptides, inteins or self-processing sites.

Proteases (endoproteases) and signal peptidases and the amino acid sequences of their recognition sites useful for separating components of the biologically active protein within the polyprotein translation product and their recognition sequences include, without limitation, furin, RXR/K-R (SEQ ID NO:1); VP4 of IPNV, S/TXA-S/AG (SEQ ID NO:2); Tobacco etch virus (TEV) protease, EXXYXQ-G(SEQ ID NO:3); 3C protease of rhinovirus, LEVLFQ-GP (SEQ ID NO:4); PC5/6 protease; PACE protease, LPC/PC7 protease; enterokinase, DDDDK-X (SEQ ID NO:5); Factor Xa protease, IE/DGR-X (SEQ ID NO:6); thrombin, LVPR-GS (SEQ ID NO:7); genenase I, PGAAH-Y(SEQ ID NO:8); and MMP protease; Nuclear inclusion protein a(N1a) of turnip mosaic potyvirus; NS2B/NS3 of Dengue type 4 (DEN4) flaviviruses, NS3 protease of yellow fever virus (YFV); ORF V of cauliflower mosaic virus; and KEX2 protease, MYKR-EAD (SEQ ID). Another internal cleavage site option is CB2. The position within the recognition sequence at which cleavage occurs is shown with a hyphen.

In an embodiment, signal sequences employed are wild-type, mutated, or randomly mutated and selected via screening using techniques understood in the art.

Also within the scope of the invention as set forth above is an expression cassette, wherein the particular polyprotein or pre-protein (proprotein, polyprotein) coding sequence is operably linked to transcription regulatory sequences, expression vectors and recombinant host cells containing the expression vector or expression cassette.

The present invention provides a system for expression of a full length immunoglobulin or fragment thereof based on expression of heavy and light chain coding sequences under the transcriptional control of a single promoter, wherein separation of the heavy and light chains is mediated by inteins or modified inteins (which cleave but not do ligate the released protein molecules, or the antibody or other flanking protein sequences can be modified so as to prevent ligation of the proteins), or by C-terminal auto-processing domain of hedgehog from drosophila, mouse, human, and other species, or by C-terminal auto-processing domains of warthog, groundhog, and other hog-containing gene from nematodes such as Caenorhabditis elegans. Hoglet-C autoprocessing domain from choanoflagellate, or by an A-type bacterial intein-like (BIL) domains such as those from bacteria such as Clostridium thermocellum, or by a B-type BIL domains from bacteria such as Rhodobacter sphaeroides. Inteins useful in the present invention include, without limitation the Saccharomyces cerevisiae VMA, Pyrococcus, Synechocystis, and other inteins known to the art. The separation of heavy and light chains can also be mediated by self-processing cleavage site, e.g., a 2A or 2A-like sequence.

In one aspect, the invention provides a vector for expression of a recombinant immunoglobulin, which includes a promoter operably linked to the coding sequence for a first chain of an immunoglobulin molecule or a fragment thereof, a sequence encoding a self-processing cleavage site and the coding sequence for a second chain of an immunoglobulin molecule or fragment thereof, wherein the sequence encoding the self-processing cleavage site is inserted between the coding sequence for the first chain of the immunoglobulin molecule and the coding sequence for the second chain of the immunoglobulin molecule. Either the first or second chain of the immunoglobulin molecule may be a heavy chain or a light chain, and the sequence encoding the recombinant immunoglobulin may be a full length coding sequence or a fragment thereof. A second region corresponding to light chain is separated from an adjacent region by a protease recognition site, signal peptide or a self-processing site, such as a 2A site. There may be two copies of the L chain sequence and one of the H chain sequence (or multiple copies of each), with the proviso that each antibody chain component has the appropriate processing site or sequence associated with it so that correctly processed antibody chains are produced.

The vector may be any recombinant vector capable of expression of a full length polypeptide, e.g. an immunoglobulin molecule or fragment thereof, for example, a plasmid vector, especially one suitable for gene expression in mammalian cells, a baculovirus vector for expression in insect cells, an adeno-associated virus (AAV) vector, a lentivirus vector, a retrovirus vector, a replication competent adenovirus vector, a replication deficient adenovirus vector and a gutless adenovirus vector, a herpes virus vector or a nonviral vector (plasmid), among others.

Self-processing cleavage sites include a 2A peptide sequence, e.g., a 2A sequence derived from Foot and Mouth Disease Virus (FMDV). In a further preferred aspect, the vector comprises a sequence which encodes an additional proteolytic cleavage site located between the coding sequence for the first chain of the immunoglobulin molecule or fragment thereof and the coding sequence for the second chain of the immunoglobulin molecule or fragment thereof (i.e., adjacent the sequence for a self-processing cleavage site, such as a 2A cleavage site) and also adjacent to the second light chain sequence. In one exemplary approach, the additional proteolytic cleavage site is a furin cleavage site with the consensus sequence RXK/R-R (SEQ ID NO:1). A vector for recombinant immunoglobulin expression using a self-processing peptide may include any of a number of promoters, wherein the promoter is constitutive, regulatable or inducible, cell type specific, tissue-specific, or species specific. The vector may further comprise a sequence encoding a signal sequence for one or more of the coding sequences of immunoglobulin chains, pre-proteins or the like.

The invention further provides host cells or stable clones of host cells infected with a vector that comprises a sequence encoding heavy and light chains of an immunoglobulin (i.e., an antibody); a sequence encoding a self-processing cleavage site; and may further comprise a sequence encoding an additional proteolytic cleavage site, and optionally a protease coding region similarly separated from the remainder of the coding sequence(s) by a self-processing site or a protease recognition sequence. Use of such cells or clones in generating full length recombinant immunoglobulins or fragments thereof is also included within the scope of the invention. Suitable host cells include, without limitation, insect cultured cells such as Spodoptera frugiperda cells, microbes including bacteria, yeast cells such as Saccharomyces cerevisiae or Pichia pastoris, fungi such as Trichoderma reesei, Aspergillus, Aureobasidum and Penicillium species, as well as mammalian cells such as Chinese hamster ovary (e.g., CHO-K1, ATCC CCL 61; CHO DG44, Chasin et al. 1986, Som. Cell. Molec. Genet. 12:555), baby hamster kidney (BHK-21, BHK-570, ATCC CRL 8544, ATCC CRL 10314), COS, mouse embryonic (NIH-3T3, ATCC CRL 1658), Vero cells (African green monkey kidney, available as ATCC CRL 1587), canine kidney cells (e.g., MDCK, ATCC CCL 34), rat pituitary cells (GH1, ATCC CCL 34), certain human cell lines including human embryonic kidney cells (e.g. HEK293, ATCC CRL 1573), and various transgenic animal systems, including without limitation, pigs, mice, rats, sheep, goat, cows, can be used as well. Chicken systems for expression in egg white and transgenic sheep, goat and cow systems are known for expression in milk, among others. Plant cells are also suitable as host cells.

In a related aspect, the invention provides a recombinant immunoglobulin molecule or fragment thereof produced by such a cell or clones, wherein the immunoglobulin comprises amino acids derived from a self processing cleavage site, signal peptide, intein, C-terminal auto-processing hog-containing genes, bacterial intein-like (BIL) domains, or protease recognition sequence, and methods for producing the same. Where an intein is use, it is preferably a modified intein so that the two antibody chains are not spliced together to form a single polypeptide chain or the termini of the antibody polypeptides are such that they cannot be spliced together by the intein. The intein is placed as an in frame fusion between an N-extein and a C-extein, for example, between an immunoglobulin heavy chain and an immunoglobulin light chain, with the proviso that the intein and/or junction proximal amino acid sequence of the polyprotein primary translation product results in cleavage to release the exteins, but no ligation of those extein proteins occurs.

The present invention further provides a post-translational protein processing strategy using a hedgehog protein processing domain positioned between a first expressed protein portion and a second protein portion. Optionally the hedgehog protein processing domain (Hh-C) can be truncated to delete the cholesterol transfer portion so that only protein cleavage occurs. In case complete excision of the Hh-C does not occur, inclusion of a signal peptide domain at the N-terminus of the second protein portion may allow for proteolytic separation of a mature second protein from the Hh-C/first protein portion. Also within the scope of this aspect of the present invention are non-naturally occurring recombinant DNA molecules comprising a sequence encoding a polyprotein which includes a hedgehog protein processing domain positioned between a first expressed protein portion coding sequence and a second protein portion coding sequence so that a polyprotein is produced by translation from a single message.

In an additional aspect of the present invention is a modified furin, characterized by the addition of a peptide region which targets the newly synthesized furin protein to the lumen of the endoplasmic reticulum. Also encompassed is the intein or modified intein strategy, as set forth herein.

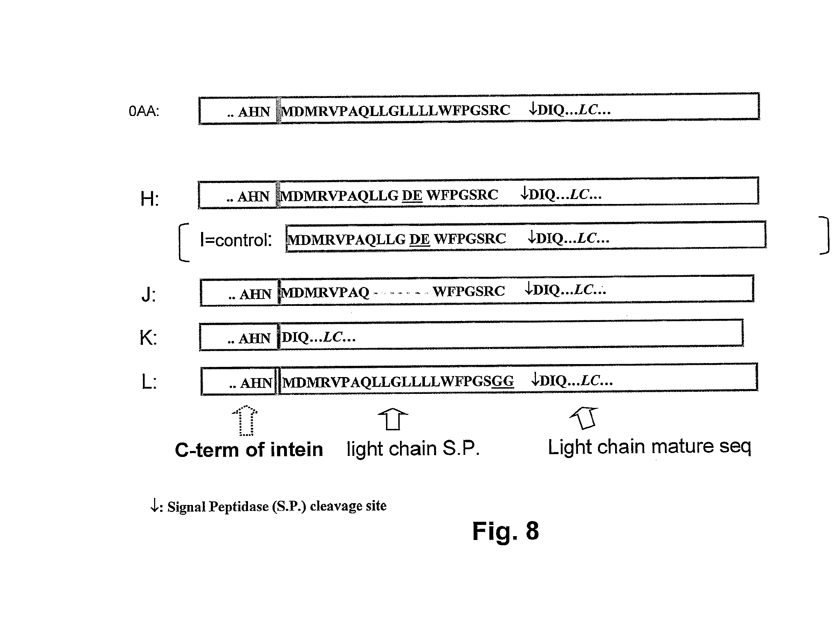

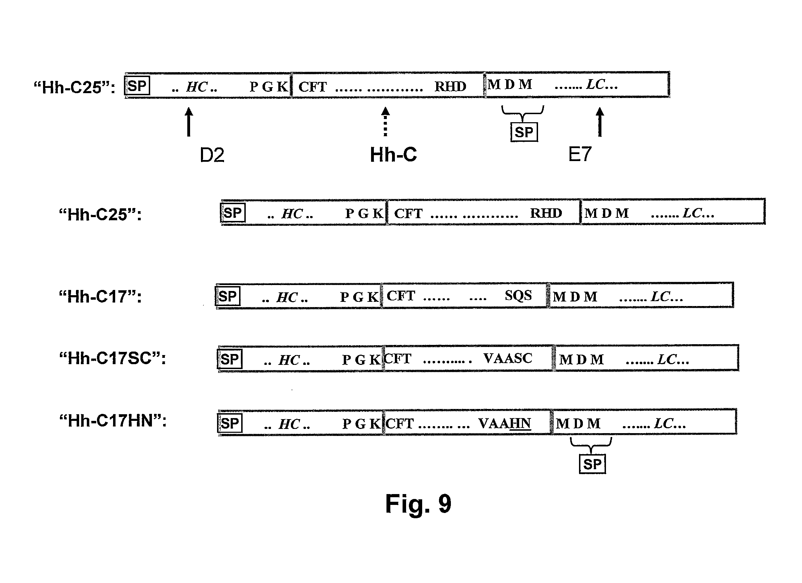

Another aspect of the present invention is the application to the polyprotein/self processing, intein processing, signal peptide cleavage or proteolytic cleavage approach to the two-hybrid and three-hybrid (and variants) technology. The first and second or first, second and third proteins are expressed as a polyprotein from a single transcript in a suitable host cell, and the coding sequences for these proteins are separated by a self processing site (e.g., 2A), intein, signal peptide or by protease recognition sites. This strategy eliminates the need for co-transfecting with more than one vector or by expressing each protein off a single transcript, as is done conventionally, with the result using the present invention that there is improved economy, efficiency and protein expression, and the potential binding pairs are within close proximity of one another which is believed to improve the likelihood of binding partners associating with one another. In a particular embodiment, the polyprotein comprises a bait protein, and self processing, intein, signal peptide or protease recognition sequence and inserted cDNA sequences, which represent one or more potential prey proteins that interact with the bait protein of interest. This cloning and expression strategy is shown schematically in Figs. 8 and 9.

In an embodiment, the invention provides DNA constructs for expression of multiple gene products in a cell comprising a single promoter at the 5' end of the construct, an intein-containing unit comprising two or more extein sequences encoding separate proteins, and one or more intein sequences fused to the carboxy-terminus encoding portion of each extein sequence, except the last extein sequence to be expressed; and a 3' termination sequence comprising a polyadenylation signal following the last extein protein coding sequence; wherein the intein-containing unit is expressed as a precursor protein containing at least one intein flanked by extein encoded proteins; wherein at least one of the inteins can catalyze excision of the exteins; and, preferably, wherein at least one amino acid residue is substituted in, or added to, the intein-containing unit so that the excised exteins are not ligated by the intein. In a particular embodiment, the constructs are configured wherein at least two of the extein sequences, upon expression as proteins, are capable of associating in multimeric assembly. In an embodiment, at least two extein sequences are cabable of encoding an immunoglobulin or other antigen recognition molecule. In an embodiment, at least one extein sequence, upon expression as a protein, is capable of extracellular secretion. In an embodiment, at least one extein sequence is a mammalian gene.

In embodiments, the invention provides constructs and methods for immunoglobulin expression using a modified or non-modified intein where expressed immunoglobulin segments are not re-ligated/fused, thereby allowing production of a assembled antibody from multiple subunits. In a particular embodiment, the modified intein includes a change in an amino acid residue located in the first position of the C-extein. In a particular embodiment, there is a change at the second to last amino acid within the intein segment.

In embodiments, the invention provides constructs and methods for expression of any gene or combination of genes. In a particular embodiment, the C-extein is modified. In a further particular embodiment, the C-extein is modified using a signal sequence. In another particular embodiment, there is an absence of a terminal C-extein component.

In embodiments, the invention provides constructs and methods for expression of antibody genes using a modified signal peptide for the second chain of immunoglobulin (either heavy chain or light chain), and third if used, which are placed after an intein or a hedgehog auto-processing domain. In an embodiment, an order of segments is as follows: first chain - first intein or hedgehog - first modified signal peptide - second chain - second modified signal peptide - third chain (in a two-chain situation, e.g., the third chain or the 'second modified signal peptide - third chain' segment is omitted). In another embodiment, a second intein or hedgehog segment is included after the second chain. In a particular embodiment, the use of such a modified signal peptide gives rise to increased antibody secretion. In an embodiment, the signal peptide used is modified to reduce hydrophobicity. In an embodiment, a signal peptide is unmodified.

In embodiments, sORF vectors are provided for transient expression. In other embodiment, sORF vectors are provided in stable expression systems. In an embodiment, stable host cells are generated as understood in the art, e.g., by transfection and other techniques.

While many exemplary constructs are specifically disclosed herein for the expression of antibody specific for tumor necrosis factor α (alpha), it is understood that constructs can be readily prepared using the same strategies with the substitution of sequences encoding other proteins. Particular examples include other immunoglobulins and biotherapeutic molecules. Further particular examples include antibodies specific for E/L selectin, interleukin-12, interleukin-18 or erythropoietin receptor, or any other antibody of desired specificity for which the amino acid sequence and/or the coding sequence is available to the art.

In an embodiment, the invention provides an expression vector for generating one or more recombinant protein products comprising a sORF insert; said sORF insert comprising a first nucleic acid sequence encoding a first polypeptide, a first intervening nucleic acid sequence encoding a first protein cleavage site, and a second nucleic acid sequence encoding a second polypeptide; wherein said intervening nucleic acid sequence encoding said first protein cleavage site is operably positioned between said first nucleic acid sequence and said second nucleic acid sequence; and wherein said expression vector is capable of expressing a sORF polypeptide cleavable at said first protein cleavage site. In an embodiment, said first protein cleavage site comprises a self-processing cleavage site.

In an embodiment, the self-processing cleavage site comprises an intein segment or modified intein segment, wherein the modified intein segment permits cleavage but not complete ligation of said first polypeptide to said second polypeptide. In an embodiment, the self-processing cleavage site comprises a hedgehog segment or modified hedgehog segment, wherein the modified hedgehog segment permits cleavage of said first polypeptide from said second polypeptide. In an embodiment, the first polypeptide and second polypeptide are capable of multimeric assembly. In an embodiment, at least one of said first polypeptide and second polypeptide are capable of extracellular secretion. In an embodiment, at least one of said first polypeptide and second polypeptide are of mammalian origin.

In an embodiment, at least one of said first polypeptide and second polypeptide comprises an immunoglobulin heavy chain or functional fragment thereof. In an embodiment, at least one of said first polypeptide and second polypeptide comprises an immunoglobulin light chain or functional fragment thereof. In an embodiment, said first polypeptide comprises an immunoglobulin heavy chain or functional fragment thereof and said second polypeptide comprises an immunoglobulin light chain or functional fragment thereof; and wherein said first and second polypeptides are in any order. In an embodiment, said first polypeptide and second polypeptide taken together are capable of associating in multimeric assembly to form a functional antibody or other antigen recognition molecule.

In an embodiment, said first polypeptide is upstream of said second polypeptide. In an embodiment, said second polypeptide is upstream of said first polypeptide.

In an embodiment, an expression vector further comprises a third nucleic acid sequence encoding a third polypeptide, wherein said third nucleic acid sequence is operably positioned after said second nucleic acid sequence; and wherein said third sequence may independently be the same or different from either of said first or second nucleic acid sequence. In an embodiment, at least two of said first, second, and third polypeptides taken together are capable of associating in multimeric assembly.

In an embodiment, the expression vector further comprises a second intervening nucleic acid sequence encoding a second protein cleavage site, wherein said second intervening nucleic acid sequence is operably positioned after said first and said second nucleic acid sequence; and wherein said second intervening sequence may be the same or different from said first intervening nucleic acid sequence. In an embodiment, an expression vector further comprises a third nucleic acid sequence encoding a third polypeptide, and a second intervening nucleic acid sequence encoding a second protein cleavage site; wherein the second intervening nucleic acid sequence and third nucleic acid sequence, in that order, are operably positioned after said second nucleic acid sequence. In an embodiment, said third nucleic acid sequence encodes an immunoglobulin heavy chain, light chain, or respectively a functional fragment thereof. In an embodiment, said third nucleic acid sequence encodes an immunoglobulin light chain or functional fragment thereof. In an embodiment, said third nucleic acid sequence encodes an immunoglobulin heavy chain or functional fragment thereof.

In an embodiment of an expression vector, said first intervening nucleic acid sequence encoding a first protein cleavage site comprises a signal peptide nucleic acid encoding a signal peptide cleavage site or modified signal peptide cleavage site sequence. In an embodiment, the expression vector further comprises a signal peptide nucleic acid sequence encoding a signal peptide cleavage site, operably positioned before said first nucleic acid sequence or said second nucleic acid sequence.

In an embodiment, an expression vector further comprises two signal peptide nucleic acid sequences, each independently encoding a signal peptide cleavage site, wherein one signal peptide nucleic acid sequence is operably positioned before said first nucleic acid encoding said first polypeptide and the other signal peptide nucleic acid sequence is operably positioned before said second nucleic acid encoding said second polypeptide. In embodiments, the two signal peptide sequences are the same or different.

In an embodiment, a signal peptide nucleic acid sequence encodes an immunoglobulin light chain signal peptide cleavage site or modified immunoglobulin light chain signal peptide cleavage site. In an embodiment, a signal peptide nucleic acid sequence encodes a modified or unmodified immunoglobulin light chain signal peptide cleavage site, and wherein said modified site is capable of effecting cleavage and increasing secretion of at least one of said first polypeptide, said second polypeptide, and an assembled molecule of said first and second polypeptides; and wherein a secretion level in the presence of said signal peptide site is about 10% greater to about 100-fold greater than a secretion level in the absence of said signal peptide site.

In an embodiment, an intervening nucleic acid sequence encoding a first protein cleavage site comprises an intein or modified intein sequence selected from the group consisting of: a Pyrococcus horikoshii Pho Pol I sequence, a Saccharomyces cerevisiae VMA sequence, Synechocystis spp. Strain PCC6803 DnaE sequence, Mycobacterium xenopi GyrA sequence, Pyrococcus species GB-D DNA polymerase, A-type bacterial intein-like (BIL) domain, and B-type BIL.

In an embodiment, an intervening nucleic acid sequence encoding a first protein cleavage site comprises a C-terminal auto-processing domain of a hedgehog family member, wherein the hedgehog family member is from Drosophila, mouse, human, or other insect or animal species. In an embodiment, an intervening nucleic acid sequence encoding a first protein cleavage site comprises a C-terminal auto-processing domain from a warthog, groundhog, or other hog-containing gene from a nematode, or Hoglet domain from a choanoflagellate.

In an embodiment, the first and said second polypeptide comprise a functional antibody or other antigen recognition molecule; with an antigen specificity directed to binding an antigen selected from the group consisting of: tumor necrosis factor-α, erythropoietin receptor, RSV, EUselectin, interleukin-1, interleukin-12, interleukin-13, interleukin-18, interleukin-23, CXCL-13, GLP-1 R, and amyloid beta. In an embodiment, the first and second polypeptides comprise a pair of immunoglobulin chains from an antibody of D2E7, ABT-007, ABT-325, EL246, or ABT-874. In an embodiment, the first and second polypeptide are each independently selected from an immunoglobulin heavy chain or an immunoglobulin light chain segment from an analogous segment of D2E7, ABT-007, ABT-325, EL246, ABT-874, or other antibody.

In an embodiment, a vector further comprises a promoter regulatory element for said sORF insert. In an embodiment, said promoter regulatory element is inducible or constitutive. In an embodiment, said promoter regulatory element is tissue specific. In an embodiment, said promoter comprises an adenovirus major late promoter.

In an embodiment, a vector further comprises a nucleic acid encoding a protease capable of cleaving said first protein cleavage site. In anembodiment, said nucleic acid encoding a protease is operably positioned within said sORF insert; said expression vector further comprising an additional nucleic acid encoding a second cleavage site located between said nucleic acid encoding a protease and at least one of said first nucleic acid and said second nucleic acid.

In an embodiment, the invention provides a host cell comprising a vector described herein. In an embodiment, the host cell is a prokaryotic cell. In an embodiment, said host cell is Escherichia coli. In an embodiment, said host cell is a eukaryotic cell. In an embodiment, said eukaryotic cell is selected from the group consisting of a protist cell, animal cell, plant cell and fungal cell. In an embodiment, said eukaryotic cell is an animal cell selected from the group consisting of a mammalian cell, an avian cell, and an insect cell. In a preferred embodiment, said host cell is a CHO cell or a dihydrofolate reductase-deficient CHO cell. In an embodiment, said host cell is a COS cell. In an embodiment, said host cell is a yeast cell. In an embodiment, said yeast cell is Saccharomyces cerevisiae. In an embodiment, said host cell is an insect Spodoptera frugiperda Sf9 cell. In an embodiment, said host cell is a human embryonic kidney cell.

In an embodiment, the invention provides a method for producing a recombinant polyprotein or a plurality of proteins, comprising culturing a host cell in a culture medium under conditions sufficient to allow expression of a vector protein. In an embodiment, the method further comprises recovering and/or purifying said vector protein. In an embodiment, said plurality of proteins are capable of multimeric assembly. In an embodiment, the recombinant polyprotein or plurality of proteins are biologically functional and/or therapeutic.

In an embodiment, the invention provides a method for producing an immunoglobulin protein or functional fragment thereof, assembled antibody, or other antigen recognition molecule, comprising culturing a host cell according to claim 38 in a culture medium under conditions sufficient to produce an immunoglobulin protein or functional fragment thereof, assembled antibody, or other antigen recognition molecule.

In an embodiment, the invention provides a protein or polyprotein produced according to a method herein. In an embodiment, the invention provides an assembled immunoglobulin; assembled other antigen recognition molecule; or individual immunoglobulin chain or functional fragment thereof produced according to the methods herein. In an embodiment, the immunoglobulin; other antigen recognition molecule; or individual immunoglobulin chain or functional fragment thereof has a capability to effect or contribute to specific antigen binding to tumor necrosis factor-α, erythropoietin receptor, interleukin-18, EUselectin or interleukin-12. In an embodiment, the immunoglobulin is D2E7 or wherein the functional fragment is a fragment of D2E7.

In an embodiment, the invention provides a pharmaceutical composition or medicament comprising a protein and a pharmaceutically acceptable carrier. Excipients and carriers for pharmaceutical formulations are selected as would be understood in the art.

In an embodiment, the invention provides an expression vector wherein the first protein cleavage site comprises a cellular protease cleavage site or a viral protease cleavage site. In an embodiment, said first protein cleavage site comprises a site recognized by furin; VP4 of IPNV; tobacco etch virus (TEV) protease; 3C protease of rhinovirus; PC5/6 protease; PACE protease, LPC/PC7 protease; enterokinase; Factor Xa protease; thrombin; genenase I; MMP protease; Nuclear inclusion protein a(N1 a) of turnip mosaic potyvirus; NS2B/NS3 of Dengue type 4 flaviviruses, NS3 protease of yellow fever virus; ORF V of cauliflower mosaic virus; KEX2 protease; CB2; or 2A. In an embodiment, said first protein cleavage site is a viral internally cleavable signal peptide cleavage site. In an embodiment, said viral internally cleavable signal peptide cleavage site comprises a site from influenza C virus, hepatitis C virus, hantavirus, flavivirus, or rubella virus.

In an embodiment, the invention provides a method for expression of proteins of a two hybrid system, wherein said two hybrid system comprises a bait protein and a candidate prey protein, said method comprising the steps of: providing a host cell into which has been introduced an expression vector encoding a polyprotein comprising a bait protein portion and a candidate prey protein portion, said portions separated by a self-processing cleavage sequence, a signal peptide sequence or a protease cleavage site; and culturing the host cell under conditions which allow expression of the polyprotein and self processing or protease cleavage of the polyprotein. In an embodiment, the polyprotein further comprises a cleavable component of a three hybrid system.

In an embodiment, an expression vector does not contain a 2A sequence. In an embodiment, an expression vector is provided wherein said first protein cleavage site comprises a FMDV 2A sequence; a 2A-like domain from other Picornaviridae, an insect virus, Type C rotavirus, trypanosome, or Thermatoga maritima.

In an embodiment, the invention provides an expression vector for expressing a recombinant protein, comprising a coding sequence for a polyprotein, wherein the polyprotein comprises at least a first and a second protein segment, wherein said protein segments are separated by a protein cleavage site therebetween, wherein the protein cleavage site comprises a self processing peptide cleavage sequence, a signal peptide cleavage sequence or a protease cleavage sequence; and wherein said coding sequence is expressible in a host cell and is cleaved within the host cell.

In an embodiment, the invention provides an expression vector where an intervening nucleic acid sequence additionally encodes a tag.

Other aspects, features and advantages of the invention are apparent from the following description of the invention, provided for the purpose of disclosure when taken in conjunction with the accompanying drawings.

In general the terms and phrases used herein have their art-recognized meaning, which can be found by reference to standard texts, journal references and contexts known to those skilled in the art. Definitions provided herein are intended to clarify their specific use in the context of the invention.

Without wishing to be bound by any particular theory, there can be discussion herein of beliefs or understandings of underlying principles or mechanisms relating to the invention. It is recognized that regardless of the ultimate correctness of any explanation or hypothesis, an embodiment of the invention can nonetheless be operative and useful.

### BRIEF DESCRIPTION OF THE DRAWINGS

Fig. 1 illustrates a preferred stable sORF expression vector construct.

Fig. 2 illustrates a preferred stable sORF expression vector construct, further comprising additional (second) intervening nucleic acid encoding a second protein cleavage site (which can be an autoprocessing site) and third nucleic acid sequence encoding a third polypeptide.. Such a vector is capable of expression of more than two polypeptides.

Fig. 3 illustrates a preferred transient sORF expression vector construct, (e.g., pTT3-HC-Ssp-GA-int-LC-0aa)..

Fig. 4 illustrates an expression vector with a 2A segment for a two-hybrid system. The vector expression cassette is structured to translate the bait protein first as a GAL4::bait::2A peptide fusion, which is self processed after the translation of the 2A peptide. The second open reading frame (ORF) is an NFkappaB::library fusion protein.

Fig. 5 is an expanded linear view of the expression region of the plasmid of Fig. 4 (2-hybrid system with 2A cleavage).

Fig. 6 illustrates intein-based sORF vectors for immunoglobulin expression.

Fig. 7 illustrates several sORF constructs with selected point mutations for expression of assembling multimeric molecules such as antibodies.

Fig. 8 illustrates sORF constructs with altered signal peptides, e.g., modified immunoglobulin light chain signal peptides.

Fig. 9 illustrates sORF constructs using hedgehog auto-processing domains.

### DETAILED DESCRIPTION OF THE INVENTION

The invention may be further understood by the following description and non-limiting examples.

The present invention provides systems, e.g., constructs and methods, for expression of a structural or a biologically active protein such as an enzyme, hormone (e.g., insulin), cytokine, chemokine, receptor, antibody, or other molecule. Preferably, the protein is an immunomodulatory protein such as an interleukin, a full length immunoglobulin, fragment thereof, other antigen recognition molecule as understood in the art, or other biotherapeutic molecule. An overview of such systems is in the specific context of an immunoglobulin molecule where recombinant production is based on expression of heavy and light chain coding sequences under the transcriptional control of a single promoter, wherein conversion of a single translation product (polyprotein) to the separate heavy and light chains is mediated by inteins, hog-containing auto-processing domains, 2A or 2A-like sequence that separate the flanking peptides at ribosome during translation or is the result of proteolytic processing at one or more protease recognition sequences located between the two chains of the mature biologically active protein.

The intervening site (whether related to an intein segment, hog domain, 2A or 2A-like, or protease recognition site; and variations thereof for each) may be referred to as a cleavage site. In the case where a plurality of three or more protein segments is expressed, such a cleavage site can be located between at least any two of the multiple segments, or a cleavage site can be located after each segment, optionally and preferably not after the last segment. If multiple cleavage sites are used, each may be the same as or independent from another.

In one aspect, the invention provides a vector for expression of a recombinant immunoglobulin, which includes a promoter operably linked to the coding sequence for a first chain of an immunoglobulin molecule or a fragment thereof, a sequence encoding a self-processing or other proteolytic cleavage site and the coding sequence for a second chain of an immunoglobulin molecule or fragment thereof, wherein the sequence encoding the self-processing or other proteolytic cleavage site is inserted between the coding sequence for the first chain of the immunoglobulin molecule and the coding sequence for the second chain of the immunoglobulin molecule, and a third region, encoding an immunoglobulin light chain, also separated from the remainder of the polyprotein by a self-processing or other proteolytic cleavage site.

In an embodiment, either the first or second chain of the immunoglobulin polyprotein molecule may be a heavy chain or a light chain. A sequence encoding a recombinant immunoglobulin segment may be a full length coding sequence or a fragment thereof. In a specific embodiment, a second light chain coding sequence must be part of the sequence encoding the polyprotein to be processed in the practice of the present invention; i.e., taken together there are three segments comprising two light chains and one heavy chain, in any order. In particular embodiments, constructs are configured with these components and in this order: a) IgH-IgL; b) IgL-IgH; c) IgH-IgL-IgL; d) IgL-IgH-IgL; e) IgL-IgL-IgH; f) IgH-IgH-IgL; g) IgH-IgL-IgH; and/or h) IgL-IgH-IgH. In an embodiment, the hyphen can indicate the location where a cleavage site sequence is located.

Alternatively, the immunoglobulin heavy and light chain coding sequences are fused in frame to an intein coding sequence there between, with the intein either modified so as to lack splicing activity or the termini of the heavy and light chains designed so that splicing preferably does not occur or such that splicing occurs with poor efficiency such that unspliced antibody molecules predominate. In addition, a modified intein can further be modified still further so that there is no endonuclease region (where an endonuclease region had previously existed), with the proviso that site specific proteolytic cleavage activity remains so that the light and heavy antibody polypeptides are freed from the intervening intein portion of the primary translation product. Either the light or the heavy antibody polypeptide can be the N-extein, and either can be the C-extein.

The vector may be any recombinant vector capable of expression of a full length polyprotein, for example, an adeno-associated virus (AAV) vector, a lentivirus vector, a retrovirus vector, a replication competent adenovirus vector, a replication deficient adenovirus vector and a gutless adenovirus vector, a herpes virus vector or a nonviral vector (plasmid) or any other vector known to the art, with the choice of vector appropriate for the host cell in which the immunoglobulin or other protein(s) are expressed. Baculovirus vectors are available for expression of genes in insect cells. Numerous vectors are known to the art, and many are commercially available or otherwise readily accessible to the art.

**Cleavage Sites**

Preferred self-processing cleavage sites include an intein sequence; modified intein; hedgehog sequence; other hog-family sequence; a 2A sequence, e.g., a 2A sequence derived from Foot and Mouth Disease Virus (FMDV); and variations thereof for each.

Proteases whose recognition sequences can substitute for the 2A sequence include, without limitation, furin, a modified furin targeted to the endoplasmic reticulum rather than the trans Golgi network, VP4 of IPNV, TEV protease, a nuclear localization signal-deficient TEV protease (TEV Nls-), 3C protease of rhinovirus, PC5/6 protease, PACE protease, LPC/PC7 protease, enterokinase, Xa protease, thrombin, genenase I and MMP protease, as discussed above. Other endoproteases useful in the practice of the present invention are proteases including, but not limited to, nuclear inclusion protein a(N1a) of turnip mosaic potyvirus (Kim et al. 1996. Virology 221:245-249); NS2B/NS3 of Dengue type 4 (DEN4) flaviviruses (Falgout et al. 1993. J. Virol. 67:2034-2042; Lai et al. 1994. Arch. Virol. Suppl. 9:359-368), NS3 protease of yellow fever virus (YFV) (Chambers et al. 1991. J. Virol. 65:6042-6050); ORF V of cauliflower mosaic virus (Torruella et al. 1989. EMBO Journal 8:2819-2825); inteins, an example of which is the Psp-GBD Pol intein (Xu, M.Q. 1996. EMBO 15: 5146-5153); an internally cleavable signal peptide, an example of which is the internally cleavable signal peptide of influenza C virus (Pekosz A. 1992. Proc. Natl. Acad. Sci. USA 95: 3233-13238); and KEX2 protease, MYKR-EAD (SEQ ID NO:9); KEX2 and a modified KEX2 which is targeted to the ER (see Chaudhuri et al. 1992. Eur. J. Biochem. 210:811-822). The modified KEX2 which is uniquely directed to the ER has coding and amino acid sequences as given in Table 7A and 7B, respectively; it is called KEX2-sol-KDEL. The primary amino acid sequence of KEX2 from *Saccharomyces cerevisiae* has been modified to remove the membrane association domain and to add the ER targeting sequence KDEL at the C terminus of the protein. Other human proteases useful for cleaving polyproteins containing the appropriate cleavage recognition sites include those set forth in US Patent Publication 2005/0112565. The sonic hedgehog protein from *Drosophila melanogaster,* especially the processing domain therefrom, can also serve to free proteins from a polyprotein primary translation product.

Within the scope of the present invention is a modified furin protease, which is targeted to the endoplasmic reticulum (ER) rather than to the trans Golgi network (TGN), as is the naturally occurring furin protease. Vorhees et al. 1995. EMBO Journal 14:4961-4975 described the EEDE (SEQ ID NO:10) portion of furin (amino acids 775-778) as involved in the targeting of the protease to the TGN (Nakayama et al. 1997. Biochem. Journal 327:625-635). Zerangue et al. 2001. Proc. Natl. Acad. Sci. USA 98:2431-2436 reported ER trafficking signals, including KKXX at the C terminus of a protein. Thus a modified furin is developed and used to target furin cleavage activity to the ER compartment instead of or in addition to the TGN and later compartments.

In a further aspect, the vector comprises a sequence which encodes an additional cleavage site located between the coding sequence for the first chain of the immunoglobulin molecule or fragment thereof and the coding sequence for the second and/or third chain (e.g., a duplicate of the first or second chain) of the immunoglobulin molecule or fragment thereof (i.e., adjacent the sequence for a cleavage site, which could be a 2A cleavage site). In one exemplary approach, the additional proteolytic cleavage site is a furin cleavage site with the consensus sequence RXK(R)R (SEQ ID NO:1).

**Regulatory Sequences Including Promoters; Host Cells**

A vector for recombinant immunoglobulin or other protein expression may include any of a number of promoters known to the art, wherein the promoter is constitutive, regulatable or inducible, cell type specific, tissue-specific, or species specific. Further specific examples include, e.g., tetracycline-responsive promoters (Gossen M, Bujard H, Proc Natl Acad Sci USA. 1992,15;89(12):5547-51). The vector is a replicon adapted to the host cell in which the chimeric gene is to be expressed, and it desirably also comprises a replicon functional in a bacterial cell as well, advantageously, Escherichia coli, a convenient cell for molecular biological manipulations.

The host cell for gene expression can be, without limitation, an animal cell, especially a mammalian cell, or it can be a microbial cell (bacteria, yeast, fungus, but preferably eukaryotic) or a plant cell. Particularly suitable host cells include insect cultured cells such as *Spodoptera frugiperda* cells, yeast cells such as *Saccharomyces cerevisiae* or *Pichia pastoris,* fungi such as *Trichoderma reesei,* Aspergillus, Aureobasidum and Penicillium species as well as mammalian cells such as CHO (Chinese hamster ovary), BHK (baby hamster kidney), COS, 293, 3T3 (mouse), Vero (African green monkey) cells and various transgenic animal systems, including without limitation, pigs, mice, rats, sheep, goat, cows, can be used as well. Chicken systems for expression in egg white and transgenic sheep, goat and cow systems are known for expression in milk, among others. Baculovirus, especially AcNPV, vectors can be used for the single ORF antibody expression and cleavage of the present invention, for example with expression of the sORF under the regulatory control of a polyhedrin promoter or other strong promote in an insect cell line; such vectors and cell lines are well known to the art and commercially available. Promoters used in mammalian cells can be constitutive (Herpes virus TK promoter, McKnight, Cell 31:355, 1982; SV40 early promoter, Benoist et al. Nature 290:304, 1981 Rous sarcoma virus promoter, Gorman et al. Proc. Natl. Acad. Sci. USA 79:6777, 1982; cytomegalovirus promoter, Foecking et al. Gene 45:101, 1980; mouse mammary tumor virus promoter, generally see Etcheverry in Protein Engineering: Principles and Practice, Cleland et al., eds, pp.162-181, Wiley & Sons, 1996) or regulated (metallothionein promoter, Hamer et al. J. Molec. Appl. Genet. 1:273, 1982, for example). Vectors can be based on viruses that infect particular mammalian cells, especially retroviruses, vaccinia and adenoviruses and their derivatives are known to the art and commercially available. Promoters include, without limitation, cytomegalovirus, adenovirus late, and the vaccinia 7.5K promoters. Yeast and fungal vectors (see, e.g., Van den Handel, C. et al. (1991) In: Bennett, J.W. and Lasure, L.L. (eds.), More Gene Manipulations in Fungi, Academy Press, Inc., New York, 397-428) and promoters are also well known and widely available. Enolase is a well known constitutive yeast promoter, and alcohol dehydrogenase is a well known regulated promoter.

The selection of the specific promoters, transcription termination sequences and other optional sequences, such as sequences encoding tissue specific sequences, will be determined in large part by the type of cell in which expression is desired. The may be bacterial, yeast, fungal, mammalian, insect, chicken or other animal cells.

**Signal Sequences**

The coding sequence of the protein to be cleaved, proteolytically processed or self processed, which is incorporated in the vector, may further comprise one or more sequences encoding one or more signal sequences. These encoded signal sequences can be associated with one or more of the mature segments within the polyprotein. For example, the sequence encoding the immunoglobulin heavy chain leader sequence can precede the coding sequence for the heavy chain, operably linked and in frame with the remainder of the polyprotein coding sequence. Similarly, a light chain leader peptide coding sequence or other leader peptide coding sequence can be associated in frame with one or both of the immunoglobulin light chain coding sequences, with the leader sequence-chain being separated by the adjacent chain from either a self-processing site (such as 2A) or by a sequence encoding a protease recognition sequence, with the appropriate reading frame being maintained.

Stoichiometry of Immunoglobulin Heavy and Light Chains

In many embodiments herein, immunoglobulin/antibody light chains chains (IgL) and heavy chains (IgH) are present at a vector level or at an expressed intracellular level within a host cell at about a 1:1 ratio (IgL:IgH). Whereas recombinant approaches herein and elsewhere have relied on equimolar expression of heavy and light chains (see, e.g., US Patent Publication 2005/0003482A1 or International Publication WO2004/113493), in other embodiments the present invention provides methods and expression cassettes and vectors with light and heavy chain coding sequences in a ratio of 2:1 and co-expressed with self-processing or proteolytic processing of the chains when the primary translation product is a polyprotein. In embodiments, the ratio is greater than 1:1, such as about 2:1 or greater than 2:1. In a particular embodiment, a light chain coding sequence is used at a ratio of greater than 1:1 (IgL:IgH). In a specific embodiment, the ratio of IgL:IgH is 2:1.

The invention further provides host cells or stable clones of host cells transformed or infected with a vector that comprises a sequence encoding a heavy and either one or at least two light chains of an immunoglobulin (i.e., an antibody); sequences encoding cleavage sites, such as self-processing, protease recognition sites or signal peptides there between; and may further comprise a sequence or sequences encoding an additional proteolytic cleavage site. Also included in the scope of the invention is the use of such cells or clones in generating full length recombinant immunoglobulins or fragments thereof or other biologically active proteins which are comprised of multiple subunits (e.g., two-chain or multi-chain molecules or those which are in nature produced as a pro-protein and cleaved or processed to release a precursor-derived protein and the active portion). Non-limiting examples include insulin, interleukin-18, interleukin-1, bone morphogenic protein 4, bone morphogenic protein 2, any other two chain bone morphogenic proteins, nerve growth factor, renin, chymotrypsin, transforming growth factor β, and interleukin 1β.

In a related aspect, the invention provides a recombinant immunoglobulin molecule or fragment thereof or other protein produced by such a cell or clones, wherein the immunoglobulin comprises amino acids derived from a self processing cleavage site (such as an intein or hedgehog domain), cleavage site or signal peptide cleavage and methods, vectors and host cells for producing the same. In embodiments, the invention provides host cells containing one or more constructs as described herein.

The present invention provides single vector constructs for expression of an immunoglobulin molecule or fragment thereof and methods for in vitro or in vivo use of the same. The vectors have self-processing or other protease recognition sequences between a first and second and between a second and third immunoglobulin coding sequence, allowing for expression of a functional antibody molecule using a single promoter and transcript. Exemplary vector constructs comprise a sequence encoding a self-processing cleavage site between open reading frames and may further comprise an additional proteolytic cleavage site adjacent to the self-processing cleavage site for removal of amino acids that comprise the self-processing cleavage site following cleavage. The vector constructs find utility in methods relating to enhanced production of full length biologically active immunoglobulins or fragments thereof in vitro and in vivo. Other biologically active proteins with at least two different chains can be made using the same strategy, although it is understood that it may not be required that either chain's coding sequence be present in a ratio greater than 1 relative to the other chain's coding sequence.

Although particular compositions and methods are exemplified herein, it is understood that any of a number of alternative compositions and methods are applicable and suitable for use in practicing the invention. It will also be understood that an evaluation of the polyprotein expression cassette and vectors, host cells and methods of the invention may be carried out using procedures standard in the art. The practice of the present invention will employ, unless otherwise indicated, conventional techniques of cell biology, molecular biology (including recombinant techniques), microbiology, biochemistry and immunology, which are within the scope of those of skill in the art. Such techniques are explained fully in the literature, such as, Molecular Cloning: A Laboratory Manual, second edition (Sambrook et al., 1989); Oligonucleotide Synthesis (M. J. Gait, ed., 1984); Animal Cell Culture (R. I. Freshney, ed., 1987); Methods in Enzymology (Academic Press, Inc.); Handbook of Experimental Immunology (D. M. Weir & C. C. Blackwell, eds.); Gene Transfer Vectors for Mammalian Cells (J. M. Miller & M. P. Calos, eds., 1987); Current Protocols in Molecular Biology (F. M. Ausubel et al., eds., 1993); PCR: The Polymerase Chain Reaction, (Mullis et al., eds., 1994); and Current Protocols in Immunology (J. E. Coligan et al., eds., 1991), each of which is expressly incorporated by reference herein.

Unless otherwise indicated, all terms used herein have the same meaning as they would to one skilled in the art and the practice of the present invention will employ, conventional techniques of microbiology and recombinant DNA technology, which are within the knowledge of those of skill of the art.

The term "modified" as generally used herein in the context of a protein refers to a segment wherein at least one amino acid residue is substituted in, deleted from, or added to, the referenced molecule. Similarly, in the context of a nucleic acid the term refers to a segment wherein at least one nucleic acid subunit is substituted in, deleted from, or added to, the referenced molecule.

The term "intein" as used herein typically refers to an internal segment of a protein that facilitates its own removal and effects the joining of flanking segments known as exteins. Many examples of inteins are recognized in a variety of types of organisms, in some cases with shared structural and/or functional features. The invention is broadly able to employ inteins, and variants thereof, as appreciated to exist and further be recognized or discovered. See, e.g., Gogarten JP et al., 2002, Annu Rev Microbiol. 2002;56:263-87; Perler, F. B. (2002), InBase, the Intein Database. Nucleic Acids Res. 30, 383-384 (also via internet at website of New England Biolabs, Inc., Ipswich, MA; http://www.neb.com/neb/inteins.html; Amitai G, et al., Mol Microbiol. 2003, 47(1):61-73; Gorbalenya AE, Nucleic Acids Res. 1998; 26(7): 1741-1748. Non-canonical inteins). In a protein an intein-containing unit or intein splicing unit can be understood as encompassing portions of the flanking exteins where structural aspects can contribute to reactions of cleavage, ligation, etc. The term can also be understood as a category in referring to an intein-based system with a "modified intein" component.

The term "modified intein" as used herein can refer to a synthetic intein or a natural intein wherein at least one at least one amino acid residue is substituted in, deleted from, or added to, the intein splicing unit so that the cleaved or excised exteins are not completely ligated by the intein.

The term "hedgehog" as used herein refers to a gene family (and corresponding protein segments) with members that have structure effecting autoproteolytic function. Family members include, for example, analogs from Drosophila, mouse, human, and other species. Furthermore, the term "hedgehog segment" is intended to encompass not only such family members but also broadly relates to auto-processing domains of warthog, groundhog, and other hog-containing gene from nematodes such as Caenorhabditis elegans, and Hoglet-C autoprocessing domain from choanoflagellates. See, e.g., Perler FB. Protein splicing of inteins and hedgehog autoproteolysis: structure, function, and evolution, Cell. 1998, 92(1):1-4; Koonin, EV et al., (1995) A protein splice-junction motif in hedgehog family proteins. Trends Biochem Sci. 20(4): 141-2; Hall TM et al., (1997) Crystal structure of a Hedgehog autoprocessing domain: homology between Hedgehog and self-splicing proteins. Cell 91(1): 85-97; Snell EA et al, Proc. R. Soc. B (2006) 273, 401-407; Aspock et al, Genome Research, 1999, 9:909-923. A particular example of a hedgehog segment is the sonic hedgehog protein from Drosophila melanogaster. The term can also be understood as a category in referring to a hedgehog-based system with a "modified hedgehog" component.

The term "modified hedgehog" segment can refer to a synthetic hedgehog segment or a natural hedgehog segment wherein at least one at least one amino acid residue is substituted in, deleted from, or added to, the hedgehog splicing unit so that cleaved segments are not completely ligated.

The term "vector", as used herein, refers to a DNA or RNA molecule such as a plasmid, virus or other vehicle, which contains one or more heterologous or recombinant DNA sequences and is designed for transfer between different host cells. The terms "expression vector" and "gene therapy vector" refer to any vector that is effective to incorporate and express heterologous DNA fragments in a cell. A cloning or expression vector may comprise additional elements, for example, the expression vector may have two replication systems, thus allowing it to be maintained in two organisms, for example in human cells for expression and in a prokaryotic host for cloning and amplification. Any suitable vector can be employed that is effective for introduction of nucleic acids into cells such that protein or polypeptide expression results, e.g. a viral vector or non-viral plasmid vector. Any cells effective for expression, e.g., insect cells and eukaryotic cells such as yeast or mammalian cells are useful in practicing the invention.

The terms "heterologous DNA" and "heterologous RNA" refer to nucleotides that are not endogenous (native) to the cell or part of the genome or vector in which they are present. Generally heterologous DNA or RNA is added to a cell by transduction, infection, transfection, transformation, electroporation, biolistic transformation or the like. Such nucleotides generally include at least one coding sequence, but the coding sequence need not be expressed. The term "heterologous DNA" may refer to a "heterologous coding sequence" or a "transgene".

As used herein, the terms "protein" and "polypeptide" may be used interchangeably and typically refer to "proteins" and "polypeptides" of interest that are expresses using the self processing cleavage site-containing vectors of the present invention. Such "proteins" and "polypeptides" may be any protein or polypeptide useful for research, diagnostic or therapeutic purposes, as further described below. As used herein, a polyprotein is a protein which is destined for processing to produce two or more polypeptide products.

As used herein, the term "multimer" refers to a protein comprised of two or more polypeptide chains (sometimes refered to as "subunits"), which assemble to form a function protein. Multimers may be composed of two (dimers), three, (trimers), four (tetramers), or more (e.g., pentamers, and so on) peptide chains. Multimers may result from self-assembly, or may require a component such as a catalyst to assist in assembly. Multimers may be composed solely of identical peptide chains (homo-multimer), or two or more different peptide chains (hetero-multimers). Such multimers may structurally or chemically functional. Many multimers are known and used in the art, including but not limited to enzymes, hormones, antibodies, cytokines, chemokines, and receptors. As such, multimers can have both biological (e.g., pharmaceutical) and industrial (e.g., bioprocessing/bioproduction) utility.

As used herein, the term "tag" refers to a peptide, which may incorporated into an expression vector that that may function to allow detection and/or purification of one or more expression products of the vector inserts. Such tags are well-known in the art and may include a radiolabeled amino acid or attachment to a polypeptide of biotinyl moieties that can be detected by marked avidin (e.g., streptavidin containing a fluorescent marker or enzymatic activity that can be detected by optical or colorimetric methods). Affinity tags such as FLAG, glutathione-S-transferase, maltose binding protein, cellulose-binding domain, thioredoxin, NusA, mistin, chitin-binding domain, cutinase, AGT, GFP and others are widely used such as in protein expression and purification systems. Further nonlimiting examples of tags for polypeptides include, but are not limited to, the following: Histidine tag, radioisotopes or radionuclides (e.g., ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm); fluorescent tags (e.g., FITC, rhodamine, lanthanide phosphors), enzymatic tags (e.g., horseradish peroxidase, luciferase, alkaline phosphatase); chemiluminescent tags; biotinyl groups; predetermined polypeptide epitopes recognized by a secondary reporter (e.g., leucine zipper pair sequences, binding sites for secondary antibodies, metal binding domains, epitope tags); and magnetic agents, such as gadolinium chelates.

The term "replication defective" as used herein relative to a viral gene therapy vector of the invention means the viral vector cannot independently further replicate and package its genome. For example, when a cell of a subject is infected with rAAV virions, the heterologous gene is expressed in the infected cells, however, due to the fact that the infected cells lack AAV rep and cap genes and accessory function genes, the rAAV is not able to replicate.

As used herein, a "retroviral transfer vector" refers to an expression vector that comprises a nucleotide sequence that encodes a transgene and further comprises nucleotide sequences necessary for packaging of the vector. Preferably, the retroviral transfer vector also comprises the necessary sequences for expressing the transgene in cells.

As used herein, "packaging system" refers to a set of viral constructs comprising genes that encode viral proteins involved in packaging a recombinant virus. Typically, the constructs of the packaging system are ultimately incorporated into a packaging cell.

As used herein, a "second generation" lentiviral vector system refers to a lentiviral packaging system that lacks functional accessory genes, such as one from which the accessory genes, vif, vpr, vpu and nef, have been deleted or inactivated. See, e.g., Zufferey et al. 1997. Nat. Biotechnol. 15:871-875.

As used herein, a "third generation" lentiviral vector system refers to a lentiviral packaging system that has the characteristics of a second generation vector system, and further lacks a functional tat gene, such as one from which the tat gene has been deleted or inactivated. Typically, the gene encoding rev is provided on a separate expression construct. See, e.g., Dull et al. 1998. J. Virol. 72:8463-8471.

As used herein with respect to a virus or viral vector, "pseudotyped" refers to the replacement of a native virus envelope protein with a heterologous or functionally modified virus envelope protein.

The term "operably linked" as used herein relative to a recombinant DNA construct or vector means nucleotide components of the recombinant DNA construct or vector are usually covalently joined to one another. Generally, "operably linked" DNA sequences are contiguous, and, in the case of a secretory leader, contiguous and in the same reading frame. However, enhancers do not have to be contiguous with the sequences whose expression is upregulated. The term is consistent with operably positioned.

Enhancer sequences influence promoter-dependent gene expression and may be located in the 5' or 3' regions of the native gene. "Enhancers" are cis-acting elements that stimulate or inhibit transcription of adjacent genes. An enhancer that inhibits transcription also is termed a "silencer". Enhancers can function (i.e., can be associated with a coding sequence) in either orientation, over distances of up to several kilobase pairs (kb) from the coding sequence and from a position downstream of a transcribed region. In addition, insulator or chromatin opening sequences, such as matrix attachment regions (Chung, Cell, 1993, Aug 13;74(3):505-14, Frisch et al, Genome Research, 2001, 12:349-354, Kim et al, J. Biotech 107, 2004, 95-105) may be used to enhance transcription of stably integrated gene cassettes.

As used herein, the term "gene" or "coding sequence" means the nucleic acid sequence which is transcribed (DNA) and translated (mRNA) into a polypeptide in vitro or in vivo when operably linked to appropriate regulatory sequences. The gene may or may not include regions preceding and following the coding region, e.g. 5' untranslated (5' UTR) or "leader" sequences and 3' UTR or "trailer" sequences, as well as intervening sequences (introns) between individual coding segments (exons).

A "promoter" is a DNA sequence that directs the binding of RNA polymerase and thereby promotes RNA synthesis, i.e., a minimal sequence sufficient to direct transcription. Promoters and corresponding protein or polypeptide expression may be cell-type specific, tissue-specific, or species specific. Also included in the nucleic acid constructs or vectors of the invention are enhancer sequences which may or may not be contiguous with the promoter sequence.

"Transcription regulatory sequences", or expression control sequences, as broadly used herein, include a promoter sequence and physically associated sequences which modulate or regulate transcription of an associated coding sequence, often in response to nutritional or environmental signals. Those associated sequences can determine tissue or cell specific expression, response to an environmental signal, binding of a protein which increases or decreases transcription, and the like. A "regulatable promoter" is any promoter whose activity is affected by a cis or trans acting factor (e.g., an inducible promoter, which is activated by an external signal or agent).

A "constitutive promoter" is any promoter that directs RNA production in many or all tissue/cell types at most times, e.g., the human CMV immediate early enhancer/promoter region which promotes constitutive expression of cloned DNA inserts in mammalian cells.

The terms "transcriptional regulatory protein", "transcriptional regulatory factor" and "transcription factor" are used interchangeably herein, and refer to a nuclear protein that binds a DNA response element and thereby transcriptionally regulates the expression of an associated gene or genes. Transcriptional regulatory proteins generally bind directly to a DNA response element, however in some cases binding to DNA may be indirect by way of binding to another protein that in turn binds to, or is bound to a DNA response element.

As used herein, an "internal ribosome entry site" or "IRES" refers to an element that promotes direct internal ribosome entry to the initiation codon, such as ATG, of a cistron (a protein encoding region), thereby leading to the cap-independent translation of the gene. See, e.g., Jackson R .J. et al. 1990. Trends Biochem Sci 15:477-83) and Jackson R. J.and Kaminski, A. 1995. RNA 1:985-1000. The examples described herein are relevant to the use of any IRES element, which is able to promote direct internal ribosome entry to the initiation codon of a cistron. "Under translational control of an IRES" as used herein means that translation is associated with the IRES and proceeds in a cap-independent manner. For example, the heavy and two light chain coding sequences can be translated via IRES separating the individual coding sequences, without the need for proteolytic or self-processing to separate the two chains from one another.

A "self-processing cleavage site" or "self-processing cleavage sequence" is defined herein as a post-translational or co-translational processing cleavage site sequence. Such a "self-processing cleavage" site or sequence refers to a DNA or amino acid sequence, exemplified herein by a 2A site, sequence or domain or a 2A-like site, sequence or domain. As used herein, a "self-processing peptide" is defined herein as the peptide expression product of the DNA sequence that encodes a self-processing cleavage site or sequence, which upon translation, mediates rapid intramolecular (cis) cleavage of a protein or polypeptide comprising the self-processing cleavage site to yield discrete mature protein or polypeptide products.

As used herein, the term "additional proteolytic cleavage site", refers to a sequence which is incorporated into an expression construct of the invention adjacent a self-processing cleavage site, such as a 2A or 2A like sequence, and provides a means to remove additional amino acids that remain following cleavage by the self processing cleavage sequence. Exemplary "additional proteolytic cleavage sites" are described herein and include, but are not limited to, furin cleavage sites with the consensus sequence RXK/R-R. Such furin cleavage sites can be cleaved by endogenous subtilisin-like proteases, such as furin and other serine proteases within the protein secretion pathway.

As used herein, the terms "immunoglobulin" and "antibody" refer to intact molecules as well as fragments thereof, such as Fa, F(ab')2, and Fv, which are capable of binding an antigenic determinant of interest. Such an "immunoglobulin" and "antibody" is composed of two identical light polypeptide chains of molecular weight approximately 23,000 daltons, and two identical heavy chains of molecular weight 53,000-70,000. The four chains are joined by disulfide bonds in a "Y" configuration. Heavy chains are classified as gamma (IgG), mu (IgM), alpha (IgA), delta (IgD) or epsilon (IgE) and are the basis for the class designations of immunoglobulins, which determines the effector function of a given antibody. Light chains are classified-as either kappa or lambda. When reference is made herein to an "immunoglobulin or fragment thereof", it will be understood that such a "fragment thereof" is an immunologically functional immunoglobulin fragment, especially one which binds its cognate ligand with binding affinity of at least 10% that of the intact immunoglobulin.

An Fab fragment of an antibody is a monovalent antigen-binding fragment of an antibody molecule. An Fv fragment is a genetically engineered fragment containing the variable region of a light chain and the variable regions of a heavy chain expressed as two chains.

The term "humanized antibody" refers to an antibody molecule in which one or more amino acids have been replaced in the non-antigen binding regions in order to more closely resemble a human antibody, while still retaining the original binding activity of the antibody. See, e.g., U.S. Pat. No. 6,602,503.

The term "antigenic determinant", as used herein, refers to that fragment of a molecule (i.e., an epitope) that makes contact with a particular antibody. Numerous regions of a protein or peptide or glycopeptide of a protein or glycoprotein may induce the production of antibodies which bind specifically to a given region or three-dimensional structure on the protein. These regions or structures are referred to as antigenic determinants or epitopes. An antigenic determinant may compete with the intact antigen (i.e., the immunogen used to elicit the immune response) for binding to an antibody.

The term "fragment," when referring to a recombinant protein or polypeptide of the invention means a peptide or polypeptide which has an amino acid sequence which is the same as part of, but not all of, the amino acid sequence of the corresponding full length protein or polypeptide, which retains at least one of the functions or activities of the corresponding full length protein or polypeptide. The fragment preferably includes at least 20-100 contiguous amino acid residues of the full length protein or polypeptide.

The terms "administering" or "introducing", as used herein, mean delivering the protein (include immunoglobulin) to a human or animal in need thereof by any route known to the art. Pharmaceutical carriers and formulations or compositions are also well known to the art. Routes of administration can include intravenous, intramuscular, intradermal, subcutaneous, transdermal, mucosal, intratumoral or mucosal. Alternatively, these terms can refer to delivery of a vector for recombinant protein expression to a cell or to cells in culture and or to cells or organs of a subject. Such administering or introducing may take place in vivo, in vitro or ex vivo. A vector for recombinant protein or polypeptide expression may be introduced into a cell by transfection, which typically means insertion of heterologous DNA into a cell by physical means (e.g., calcium phosphate transfection, electroporation, microinjection or lipofection); infection, which typically refers to introduction by way of an infectious agent, i.e. a virus; or transduction, which typically means stable infection of a cell with a virus or the transfer of genetic material from one microorganism to another by way of a viral agent (e.g., a bacteriophage).

"Transformation" is typically used to refer to bacteria comprising heterologous DNA or cells which express an oncogene and have therefore been converted into a continuous growth mode, for example, tumor cells. A vector used to "transform" a cell may be a plasmid, virus or other vehicle.

Typically, a cell is referred to as "transduced", "infected", "transfected" or "transformed" dependent on the means used for administration, introduction or insertion of heterologous DNA (i.e., the vector) into the cell. The terms "transduced", "transfected" and "transformed" may be used interchangeably herein regardless of the method of introduction of heterologous DNA.

As used herein, the terms "stably transformed", "stably transfected" and "transgenic" refer to cells that have a non-native (heterologous) nucleic acid sequence integrated into the genome. Stable transfection is demonstrated by the establishment of cell lines or clones comprised of a population of daughter cells containing the transfected DNA stably replicating by means of integration into their genomes or as an episomal element. In some cases, "transfection" is not stable, i.e., it is transient. In the case of transient transfection, the exogenous or heterologous DNA is expressed, however, the introduced sequence is not integrated into the genome or the host cell is not able to replicate.

As used herein, "ex vivo administration" refers to a process where primary cells are taken from a subject, a vector is administered to the cells to produce transduced, infected or transfected recombinant cells and the recombinant cells are readministered to the same or a different subject.

A "multicistronic transcript" refers to an mRNA molecule that contains more than one protein coding region, or cistron. A mRNA comprising two coding regions is denoted a "bicistronic transcript." The "5'-proximal" coding region or cistron is the coding region whose translation initiation codon (usually AUG) is closest to the 5' end of a multicistronic mRNA molecule. A "5'-distal" coding region or cistron is one whose translation initiation codon (usually AUG) is not the closest initiation codon to the 5' end of the mRNA.

The terms "5'-distal" and "downstream" are used synonymously to refer to coding regions that are not adjacent to the 5' end of a mRNA molecule.

As used herein, "co-transcribed" means that two (or more) open reading frames or coding regions or polynucleotides are under transcriptional control of a single transcriptional control or regulatory element comprising a promoter.

The term "host cell", as used herein refers to a cell which has been transduced, infected, transfected or transformed with a vector. The vector may be a plasmid, a viral particle, a phage, etc. The culture conditions, such as temperature, pH and the like, are those previously used with the host cell selected for expression, and will be apparent to those skilled in the art. It will be appreciated that the term "host cell" refers to the original transduced, infected, transfected or transformed cell and progeny thereof.

As used herein, the terms "biological activity" and "biologically active", refer to the activity attributed to a particular protein in a cell line in culture or in a cell-free system, such as a ligand-receptor assay in ELISA plates. The "biological activity" of an "immunoglobulin", "antibody" or fragment thereof refers to the ability to bind an antigenic determinant and thereby facilitate immunological function. The "biological activity" of a hormone or interleukin is as known in the art.

As used herein, the terms "tumor" and "cancer" refer to a cell that exhibits at least a partial loss of control over normal growth and/or development. For example, often tumor or cancer cells generally have lost contact inhibition and may be invasive and/or have the ability to metastasize.

Antibodies are immunoglobulin proteins that are heterodimers of a heavy and light chain. An typical antibody is multimeric with two heavy chains and two light chains (or functional fragments thereof) which associate together. Antibodies can have a further polymeric order of structure in being dimeric, trimeric, tetrameric, pentameric, etc., often dependent on isotype. They have proven extremely difficult to express in a full length form from a single vector or from two vectors in mammalian culture expression systems. Several methods are currently used for production of antibodies: in vivo immunization of animals to produce "polyclonal" antibodies, in vitro cell culture of B-cell hybridomas to produce monoclonal antibodies (Kohler, et al. 1988. Eur. J. Immunol. 6:511; Antibodies: A Laboratory Manual, Cold Spring Harbor Laboratory, 1988; incorporated by reference herein) and recombinant DNA technology (described for example in Cabilly et al., US Patent No. 6331415, incorporated by reference herein).

The basic molecular structure of immunoglobulin polypeptides is well known to include two identical light chains with a molecular weight of approximately 23,000 daltons, and two identical heavy chains with a molecular weight 53,000-70,000, where the four chains are joined by disulfide bonds in a "Y" configuration. The amino acid sequence runs from the N-terminal end at the top of the Y to the C-terminal end at the bottom of each chain. At the N-terminal end is a variable region (of approximately 100 amino acids in length) which provides for the specificity of antigen binding.

The present invention is directed to improved methods for production of immunoglobulins of all types, including, but not limited to, full length antibodies and antibody fragments having a native sequence (i.e. that sequence produced in response to stimulation by an antigen), single chain antibodies which combine the antigen binding variable region of both the heavy and light chains in a single stably-folded polypeptide chain; univalent antibodies (which comprise a heavy chain/light chain dimer bound to the Fc region of a second heavy chain); "Fab fragments" which include the full "Y" region of the immunoglobulin molecule, i.e., the branches of the "Y", either the light chain or heavy chain alone, or portions, thereof (i.e., aggregates of one heavy and one light chain, commonly known as Fab'); "hybrid immunoglobulins" which have specificity for two or more different antigens (e.g., quadromas or bispecific antibodies as described for example in US Patent No. 6,623,940); "composite immunoglobulins" wherein the heavy and light chains mimic those from different species or specificities; and "chimeric antibodies" wherein portions of each of the amino acid sequences of the heavy and light chain are derived from more than one species (i.e., the variable region is derived from one source such as a murine antibody, while the constant region is derived from another, such as a human antibody).

The compositions and methods of the invention find utility in production of immunoglobulins or fragments thereof wherein the heavy or light chain is "mammalian", "chimeric" or modified in a manner to enhance its efficacy. Modified antibodies include both amino acid and nucleic acid sequence variants which retain the same biological activity of the unmodified form and those which are modified such that the activity is altered, i.e., changes in the constant region that improve complement fixation, interaction with membranes, and other effector functions, or changes in the variable region that improve antigen binding characteristics. The compositions and methods of the invention can further include catalytic immunoglobulins or fragments thereof.

A "variant" immunoglobulin-encoding polynucleotide sequence may encode a "variant" immunoglobulin amino acid sequence which is altered by one or more amino acids from the reference polypeptide sequence. This same discussion which follows is applicable to other biologically active protein sequences (and their coding sequences) of interest. The variant polynucleotide sequence may encode a variant amino acid sequence which contains "conservative" substitutions, wherein the substituted amino acid has structural or chemical properties similar to the amino acid which it replaces. It is understood that a variant of a the protein of interest can be made with an amino acid sequence which is substantially identical (at least about 80 to 99% identical, and all integers there between) to the amino acid sequence of the naturally occurring sequence, and it forms a functionally equivalent, three dimensional structure and retains the biological activity of the naturally occurring protein. It is well known in the biological arts that certain amino acid substitutions can be made in protein sequences without affecting the function of the protein. Generally, conservative amino acid substitutions or substitutions of similar amino acids are tolerated without affecting protein function. Similar amino acids can be those that are similar in size and/or charge properties, for example, aspartate and glutamate and isoleucine and valine are both pairs of similar amino acids. Substitutions of one for another are permitted when native secondary and tertiary structure formation are not disrupted except as intended. Similarity between amino acid pairs has been assessed in the art in a number of ways. For example, Dayhoff et al. , in Atlas of Protein Sequence and Structure, 1978. Volume 5, Supplement 3, Chapter 22, pages 345-352, which is incorporated by reference herein, provides frequency tables for amino acid substitutions which can be employed as a measure of amino acid similarity. Dayhoff et al.'s frequency tables are based on comparisons of amino acid sequences for proteins having the same function from a variety of evolutionarily different sources.

Substitution mutation, insertional, and deletional variants of the disclosed nucleotide (and amino acid) sequences can be readily prepared by methods which are well known to the art. These variants can be used in the same manner as the specifically exemplified sequences so long as the variants have substantial sequence identity with a specifically exemplified sequence of the present invention and the desired functionality is preserved.

As used herein, substantial sequence identity refers to homology (or identity) which is sufficient to enable the variant polynucleotide or protein to function in the same capacity as the polynucleotide or protein from which the variant is derived. Preferably, this sequence identity is greater than 70% or 80%, more preferably, this identity is greater than 85%, or this identity is greater than 90%, and or alternatively, this is greater than 95%, and all integers between 70 and 100%. It is well within the skill of a person trained in this art to make substitution mutation, insertional, and deletional mutations which are equivalent in function or are designed to improve the function of the sequence or otherwise provide a methodological advantage. No embodiments/variants which may read on any naturally occurring proteins or which read on a qualifying prior art item are intended to be within the scope of the present invention as claimed. It is well known in the art that the polynucleotide sequences of the present invention can be truncated and/or otherwise mutated such that certain of the resulting fragments and/or mutants of the original full-length sequence can retain the desired characteristics of the full-length sequence. A wide variety of restriction enzymes which are suitable for generating fragments from larger nucleic acid molecules are well known. In addition, it is well known that Ba/31 exonuclease can be conveniently used for time-controlled limited digestion of DNA. See, for example, Maniatis et al. 1982. Molecular Cloning: A Laboratory Manual, Cold Spring Harbor Laboratory, New York, pages 135-139, incorporated herein by reference. See also Wei et al. 1983. J. Biol. Chem. 258:13006-13512. By use of *Bal*31 exonuclease (commonly referred to as "erase-abase" procedures), the ordinarily skilled artisan can remove nucleotides from either or both ends of the subject nucleic acids to generate a wide spectrum of fragments which are functionally equivalent to the subject nucleotide sequences. One of ordinary skill in the art can, in this manner, generate hundreds of fragments of controlled, varying lengths from locations all along the original coding sequence. The ordinarily skilled artisan can routinely test or screen the generated fragments for their characteristics and determine the utility of the fragments as taught herein. It is also well known that the mutant sequences of the full length sequence, or fragments thereof, can be easily produced with site directed mutagenesis. See, for example, Larionov, O.A. and Nikiforov, V.G. 1982. Genetika 18:349-59; Shortle et al. (1981) Annu. Rev. Genet. 15:265-94; both incorporated herein by reference. The skilled artisan can routinely produce deletion-, insertion-, or substitution-type mutations and identify those resulting mutants which contain the desired characteristics of the full length wild-type sequence, or fragments thereof, e.g., those which retain hormone, cytokine, antigen-binding or other biological activity.

In addition, or alternatively, the variant polynucleotide sequence may encode a variant amino acid sequence which contains "non-conservative" substitutions, wherein the substituted amino acid has dissimilar structural or chemical properties to the amino acid which it replaces. Variant immunoglobulin-encoding polynucleotides may also encode variant amino acid sequences which contain amino acid insertions or deletions, or both. Furthermore, a variant " immunoglobulin-encoding polynucleotide may encode the same polypeptide as the reference polynucleotide sequence but, due to the degeneracy of the genetic code, has a polynucleotide sequence which is altered by one or more bases from the reference polynucleotide sequence.

The term "fragment," when referring to a recombinant immunoglobulin of the invention means a polypeptide which has an amino acid sequence which is the same as part of but not all of the amino acid sequence of the corresponding full length immunoglobulin protein, which either retains essentially the same biological function or activity as the corresponding full length protein, or retains at least one of the functions or activities of the corresponding full length protein. The fragment preferably includes at least 20-100 contiguous amino acid residues of the full length immunoglobulin, and preferably, retains the ability to bind the same antigen as the full length antibody.

As used herein, the term "sequence identity" means nucleic acid or amino acid sequence identity in two or more aligned sequences, when aligned using a sequence alignment program. The term "% homology" is used interchangeably herein with the term "% identity" herein and refers to the level of nucleic acid or amino acid sequence identity between two or more aligned sequences, when aligned using a sequence alignment program. For example, as used herein, 80% homology means the same thing as 80% sequence identity determined by a defined algorithm, and accordingly a homologue of a given sequence has greater than 80% sequence identity over a length of the given sequence.

Optimal alignment of sequences for comparison can be conducted, e.g., by the local homology algorithm of Smith and Waterman. 1981. Adv. Appl. Math. 2:482, by the homology alignment algorithm of Needleman and Wunsch. 1970. J Mol. Biol. 48:443, by the search for similarity method of Pearson and Lipman. 1988. Proc. Natl. Acad. Sci. USA 85:2444, by computerized implementations of these algorithms (GAP, BESTFIT, FASTA, and TFASTA in the Wisconsin Genetics software Package, Genetics Computer Group, Madison, Wis.), by the BLAST algorithm, Altschul et al. 1990. J Mol. Biol. 215:403-410, with software that is publicly available through the National Center for Biotechnology Information website (see nlm.nih.gov/), or by visual inspection (see generally, Ausubel et al., infra). For purposes of the present invention, optimal alignment of sequences for comparison is most preferably conducted by the local homology algorithm of Smith and Waterman. 1981. Adv. Appl. Math. 2:482. See, also, Altschul et al. 1990 and Altschul et al. 1997.

The terms "identical" or percent "identity" in the context of two or more nucleic acid or protein sequences, refer to two or more sequences or subsequences that are the same or have a specified percentage of amino acid residues or nucleotides that are the same, when compared and aligned for maximum correspondence, as measured using one of the sequence comparison algorithms described herein, e.g. the Smith-Waterman algorithm, others known in the art, e.g., BLAST, or by visual inspection.

In accordance with the present invention, also encompassed are sequence variants which encode self-processing cleavage polypeptides and polypeptides themselves that have 80, 85, 88, 89, 90, 91, 92, 93, 94, 95, 96, 97, 98, 99% (and all integers between 80 and 100) or more sequence identity to the native sequence. Also encompassed are amino acid fragments of the polypeptides that represent a continuous stretch of at least 5, at least 10, or at least 15 units; and fragments homologous thereto according to the described identity conditions; and fragments of nucleic acid sequences that represent a continuous stretch of at least 15, at least 30, or at least 45 units.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5º C (5º below the Tm of the probe); "high stringency" at about 5-10º below the Tm; "intermediate stringency" at about 10-20º below the Tm of the probe; and "low stringency" at about 20-25º below the Tm. Functionally, maximum stringency conditions may be used to identify sequences having strict identity or near-strict identity with the hybridization probe; while high stringency conditions are used to identify sequences having about 80% or more sequence identity with the probe.

Moderate and high stringency hybridization conditions are well known in the art (see, for example, Sambrook, et al, 1989, Chapters 9 and 11, and in Ausubel, F. M., et al., 1993. An example of high stringency conditions includes hybridization at about 42º C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42º C. 2A sequence variants that encode a polypeptide with the same biological activity as the naturally occurring protein of interest and hybridize under moderate to high stringency hybridization conditions are considered to be within the scope of the present invention.

As a result of the degeneracy of the genetic code, a number of coding sequences can be produced which encode the same 2A or 2A-like polypeptide sequence or other protease or signal peptidase cleavage sequence. For example, the triplet CGT encodes the amino acid arginine. Arginine is alternatively encoded by CGA, CGC, CGG, AGA, and AGG. Therefore it is appreciated that such substitutions of synonymous codons in the coding region fall within the sequence variants that are covered by the present invention.

It is further appreciated that such sequence variants may or may not hybridize to the parent sequence under conditions of high stringency. This would be possible, for example, when the sequence variant includes a different codon for each of the amino acids encoded by the parent nucleotide. Such variants are, nonetheless, specifically contemplated and encompassed by the present invention.

The potential of antibodies as therapeutic modalities is currently limited by the production capacity and expense of the current technology. An improved viral or non-viral single expression vector for immunoglobulin (or other protein) production facilitates expression and delivery of two or more coding sequences, i.e., immunoglobulins or other proteins with bi- or multiple-specificities from a single vector. The present invention addresses these limitations and is applicable to any immunoglobulin (i.e. an antibody) or fragment thereof or other multipart protein or binding protein pair as further detailed herein, including engineered antibodies such as single chain antibodies, full-length antibodies or antibody fragments, two chain hormones, two chain cytokines, two chain chemokines, two chain receptors, and the like.

**IRES**

Internal ribosome entry site (IRES) elements were first discovered in picornavirus mRNAs (Jackson et al. 1990. Trends Biochem. Sci. 15:477-83) and Jackson and Kaminski. 1995. RNA 1:985-1000). Examples of IRES generally employed by those of skill in the art include those referenced in Table I, as well as those described in US Patent No. 6,692,736. Examples of "IRES" known in the art include, but are not limited to IRES obtainable from picornavirus (Jackson et al., 1990) and IRES obtainable from viral or cellular mRNA sources, such as for example, immunoglobulin heavy-chain binding protein (BiP), the vascular endothelial growth factor (VEGF) (Huez et al. 1998. Mol. Cell. Biol. 18:6178-6190), the fibroblast growth factor 2 (FGF-2), and insulin-like growth factor (IGFII), the translational initiation factor eIF4G and yeast transcription factors TFIID and HAP4, the encephelomyocarditis virus (EMCV) which is commercially available from Novagen (Duke et al. 1992. J. Virol 66:1602-9) and the VEGF IRES (Huez et al. 1998. Mol. Cell. Biol. 18:6178-90). IRES have also been reported in different viruses such as cardiovirus, rhinovirus, aphthovirus, HCV, Friend murine leukemia virus (FrMLV) and Moloney murine leukemia virus (MoMLV). As used herein, "IRES" encompasses functional variations of IRES sequences as long as the variation is able to promote direct internal ribosome entry to the initiation codon of a cistron. An IRES may be mammalian, viral or protozoan.

The IRES promotes direct internal ribosome entry to the initiation codon of a downstream cistron, leading to cap-independent translation. Thus, the product of a downstream cistron can be expressed from a bicistronic (or multicistronic) mRNA, without requiring either cleavage of a polyprotein or generation of a monocistronic mRNA. Internal ribosome entry sites are approximately 450 nucleotides in length and are characterized by moderate conservation of primary sequence and strong conservation of secondary structure. The most significant primary sequence feature of the I RES is a pyrimidine-rich site whose start is located approximately 25 nucleotides upstream of the 3' end of the IRES. See Jackson et al.(1990).

Three major classes of picornavirus IRES have been identified and characterized: the cardio- and aphthovirus class (for example, the encephelomyocarditis virus, Jang et al. 1990. Gene Dev 4:1560-1572); the entero- and rhinovirus class (for example, polioviruses, Borman et al. 1994. EMBO J. 13:3149-3157); and the hepatitis A virus (HAV) class, Glass et al. 1993. Virol 193:842-852). For the first two classes, two general principles apply. First, most of the 450-nucleotide sequence of the IRES functions to maintain particular secondary and tertiary structures conducive to ribosome binding and translational initiation. Second, the ribosome entry site is an AUG triplet located at the 3' end of the IRES, approximately 25 nucleotides downstream of a conserved oligopyrimidine tract. Translation initiation can occur either at the ribosome entry site (cardioviruses) or at the next downstream AUG (entero/rhinovirus class). Initiation occurs at both sites in aphthoviruses. HCV and pestiviruses such as bovine viral diarrhea virus (BVDV) or classical swine fever virus (CSFV) have 341 nt and 370 nt long 5'-UTR respectively. These 5'-UTR fragments form similar RNA secondary structures and can have moderately efficient I RES function (Tsukiyama-Kohara et al. 1992. J. Virol. 66:1476-1483; Frolov et al. 1998. RNA 4:1418-1435). Recent studies showed that both Friend-murine leukemia virus (MLV) 5'-UTR and rat retrotransposon virus-like 30S (VL30) sequences contain IRES structure of retroviral origin (Torrent et al. 1996. Hum. Gene Ther 7:603-612).

In eukaryotic cells, translation is normally initiated by the ribosome scanning from the capped mRNA 5' end, under the control of initiation factors. However, several cellular mRNAs have been found to have IRES structure to mediate the cap-independent translation (van der Velde, et al. 1999. Int J Biochem Cell Biol. 31:87-106). Examples of IRES elements include, without limitation, immunoglobulin heavy-chain binding protein (BiP) (Macejak et al. 1991. Nature 353:90-94), antennapedia mRNA of Drosophila (Oh et al. 1992. Gene and Dev 6:1643-1653), fibroblast growth factor-2 (FGF-2) (Vagner et al. 1995. Mol. Cell. Biol. 15:35-44), platelet-derived growth factor B (PDGF-B) (Bernstein et al. 1997. J. Biol. Chem. 272:9356-9362), insulin-like growth factor II (Teerink et al. (1995) Biochim. Biophys. Acta 1264:403-408), and the translation initiation factor eIF4G (Gan et al. 1996. J. Biol. Chem. 271:623-626). Recently, vascular endothelial growth factor (VEGF) was also found to have IRES element (Stein et al. 1998. Mol. Cell. Biol. 18:3112-3119; Huez et al. 1998. Mol. Cell. Biol.18:6178-6190). Further examples of IRES sequences include Picornavirus HAV (Glass et al. 1993. Virology 193:842-852); EMCV (Jang and Wimmer. 1990. Gene Dev. 4:1560-1572); Poliovirus (Borman et al. 1994. EMBO J. 13:3149-3157); HCV (Tsukiyama-Kohara et al. 1992. J. Virol. 66:1476-1483); pestivirus BVDV (Frolov et al. 1998. RNA. 4:1418-1435); Leishmania LRV-1 (Maga et al. 1995. Mol. Cell. Biol. 15:4884-4889); Retroviruses: MoMLV (Torrent et al. 1996. Hum. Gene Ther. 7:603-612). VL30, Harvey murine sarcoma virus, REV (Lopez-Lastra et al. 1997. Hum. Gene Ther. 8:1855-1865). IRES may be prepared using standard recombinant and synthetic methods known in the art. For cloning convenience, restriction sites may be engineered into the ends of the IRES fragments to be used.

To express two or more proteins from a single transcript determined by a viral or non-viral vector, an internal ribosome entry site (IRES) sequence is commonly used to drive expression of the second, third, fourth coding sequence, etc. When two coding sequences are linked via an IRES, the translational expression level of the second coding sequence is often significantly reduced (Furler et al. 2001. Gene Therapy 8:864-873). In fact, the use of an IRES to control transcription of two or more coding sequences operably linked to the same promoter can result in lower level expression of the second, third, etc. coding sequence relative to the coding sequence adjacent the promoter. In addition, an IRES sequence may be sufficiently long to impact complete packaging of the vector, e.g., the eCMV IRES has a length of 507 base pairs.

The expression of proteins in the form of polyproteins (as a primary translation product) is a strategy adopted in the replication of many viruses, including but not limited to the picornaviridae. Upon translation, virus-encoded self-processing peptides mediate rapid intramolecular (cis) cleavage of the polyprotein to yield discrete (mature) protein products. The present invention provides advantages over the use of an I RES in that a vector for recombinant protein or polypeptide expression comprising a self-processing peptide sequence (exemplified herein by 2A peptide sequence) or other protease cleavage sites is provided which facilitates expression of two or more protein or polypeptide coding sequences using a single promoter, wherein the two or more proteins or polypeptides are expressed in an advantageous molar ratio. For immunoglobulins the polyprotein is encoded by a coding sequence for one heavy chain and coding sequences for one or two light chains, with a self-processing site or protease recognition site encoded between each.

In an intein-containing construct, there can be just one of each of the heavy and light chain segments, expressed in an in frame fusion polyprotein with an intein between the two immunoglobulin chains, with the appropriate features to enable cleavage at the intein-immunoglobulin chain junctions but not re-ligation of the two immunoglobulin proteins. In another intein-containing construct, one or more additional immunoglobulin segments are present, optionally separated from the first and/or second segment by a cleavage site. For example, the intein approach is used to express one heavy chain segment and one light chain segment or to express one heavy chain and two light chains, and so forth.

A "self-processing cleavage site" or "self-processing cleavage sequence" as defined above refers to a DNA coding or amino acid sequence, wherein upon translation, rapid intramolecular (cis) cleavage of a polypeptide comprising the self-processing cleavage site occurs to yield discrete mature protein products. Such a "self-processing cleavage site", may also be referred to as a co-translational or post-translational processing cleavage site, exemplified herein by a 2A site, sequence or domain or an intein. A 2A site, sequence or domain demonstrates a translational effect by modifying the activity of the ribosome to promote hydrolysis of an ester linkage, thereby releasing the polypeptide from the translational complex in a manner that allows the synthesis of a discrete downstream translation product to proceed (Donnelly, 2001). Alternatively, a 2A site or domain demonstrates "autoproteolysis" or "cleavage" by cleaving its own C-terminus in cis to produce primary cleavage products (Furler and Palmenberg. 1990. Ann. Rev. Microbiol. 44:603-623). Other protease recognition sequences, including signal peptidase cleavage sites can be substituted for the self-processing site. Inteins are also useful in polyproteins.

**INTEINS**

As used herein, an intein is a segment within an expressed protein, bounded toward the N-terminus of the primary expression product by an N-extein and bounded toward the C-terminus of the primary expression product by a C-extein. Naturally occurring inteins mediate excision of the inteins and rejoining (protein ligation) of the N- and C-exteins. However, in the context of the present expression products, the primary sequence of the intein or the flanking extein amino acid sequence is such that the cleavage of the protein backbone occurs in the absence of or with reduced or a minimal amount of ligation of the exteins, so that the extein proteins are released from the primary translation product (polyprotein) without their being joined to form a fusion protein. The intein portion of the primary expression product (the protein synthesized by mRNA, prior to any proteolytic cleavage) mediates the proteolytic cleavage at the N-extein/intein and the intein/C-extein junctions. In general, naturally occurring inteins also mediate the splicing together(joining by formation of a peptide bond) of the N-extein and the C-extein. However, in the present invention as applied to the goal of expressing two polypeptides (as specifically exemplified by the heavy and light chains of an antibody molecule), it is preferred that protein ligation does not occur. This can be achieved by incorporating an intein which either naturally or through mutation does not have ligation activity. Alternatively, splicing can be prevented by mutation to change the amino acid(s) at or next to the splice site to prevent ligation of the released proteins. See Xu and Perler, 1996, EMBO J. 15:5146-5153; Ser, Thr or Cys normally occurs at the start of the C-extein.

Inteins are a class of proteins whose genes are found only within the genes of other proteins. Together with the flanking host genes termed exteins, inteins are transcribed as a single mRNA, and translated as a single polypeptide. Post-translationally, inteins initiate an autocatalytic event to remove themselves and joint the flanking host protein segments with a new polypeptide bond. This reaction is catalyzed solely by the intein, require no other cellular proteins, co-factors, or ATP. Inteins are found in a variety of unicellular organisms and they have different sizes. Many inteins contain an endonuclease domain, which accounts for their mobility within genomes.

Intein mediated reactions have been used in biotechnology, especially for *in vitro* settings such as for purifications and for protein chip construction, and in plant strain improvement (Perler, F.B. 2005. IUBMB Life 57(7):469-76). Mutations have been introduced into native intein nucleotide sequences, and some of these mutants are reported to have altered properties (Xu and Perler, 1996. EMBO J. 15(9), 5146-5153). Besides inteins, bacterial intein-like (BIL) domains and hedgehog (Hog) auto-processing domains, the other 2 members of the Hog/intein (HINT) superfamily, are also know to catalyze post-translational self-processing through similar mechanisms (Dassa et.al. 2004. J. Biol. Chem. 279(31):32001-32007).

Inteins occur as in-frame insertions in specific host proteins. In a self-splicing reaction, inteins excise themselves from a precursor protein, while the flanking regions, the exteins, become joined to restore host gene function. These elements also contain an endonuclease function that accounts for their mobility within genomes. Inteins occur in a range of sizes (134 to 1650 amino acids), and they have been identified in the genomes of eubacteria, eukaryota and archaea. Experiments using model splicing/reporter systems have shown that the endonuclease, protein cleavage, and protein splicing functions can be separated (Xu and Perler. 1996. EMBO J. 15:5146-5153). The example described below uses an intein from *Pyrococcus horikoshii* Pho Pol I, *Saccharomyces cerevisiae* VMA, and *Synechocystis* spp. to create a fusion protein with sequences from an antibody heavy and light chain. Mutation of the intein designed to delete the intein's splicing capability results in a single polypeptide that undergoes a self-cleavage to produce correctly encoded antibody heavy and light chains. This strategy can be similarly employed in the expression of other multichain proteins, hormone or cytokines, and it can also be adapted for processing of precursor proteins (proproteins) to their mature, biologically active forms. While the use of the *Pyrococcus horikoshii* Pho Pol I, S. *cerevisiae* VMA, and *Synechocystis* spp. inteins are specifically exemplified herein, other inteins known to the art can be used in the polyprotein expression vectors and methods of the present invention.

Many other inteins besides the *Pyrococcus horikoshii* Pho Pol I, S. *cerevisiae* VMA, and *Synechocystis* spp. inteins are known to the art (See, e.g., Perler, F.B. 2002, InBase, the Intein Database, Nucl. Acids Res. 30(1):383-384 and the Intein Database and Registry, available via the New England Biolabs website, e.g., at http://tools.neb.com/inbasel). Inteins have been identified in a wide range of organisms such as yeast, mycobacteria and extreme thermophilic archaebacteria. Certain inteins have endonuclease activity as well as the site-specific protein cutting and splicing activities. Endonuclease activity is not necessary for the practice of the present invention; an endonuclease coding region can be deleted, provided that the protein cleavage activity is maintained.

The mechanism of the protein splicing process has been studied in great detail (Chong et al. 1996. J. Biol. Chem. 271: 22159-22168; Xu and Perler. 1996. EMBO J 15: 5146-5153) and conserved amino acids have been found at the intein and extein splicing points (Xu et al. 1994. EMBO J 13:5517-5522). The constructs described herein contain an intein sequence fused to the 5'-terminus of the first coding sequence, with a second coding sequence fused in frame a the C-terminus of the intein. Suitable intein sequences can be selected from any of the proteins known to contain protein splicing elements. A database containing all known inteins can be found on the World Wide Web (Perler, F. B. 1999. Nucl. Acids Res. 27: 346-347). The intein coding sequence is fused (in frame) at the 3' end to the 5' end of a second coding sequence. For targeting of this protein to a certain organelle, an appropriate peptide signal can be fused to the coding sequence of the protein.

After the second extein coding sequence, the intein coding sequence-extein coding sequence can be repeated as often as desired for expression of multiple proteins in the same cell. For multi-intein containing constructs, it may be useful to use intein elements from different sources. After the sequence of the last gene to be expressed, a transcription termination sequence, and advantageously including a polyadenylation sequence, is desirably inserted. The order of a polyadenylation sequence and a termination sequence can be as understood in the art. In an embodiment, a polyadenylation sequence can precede a termination sequence.

Modified intein splicing units have been designed so that such a modified intein of interest can catalyze excision of the exteins from the inteins but cannot catalyze ligation of the exteins (see, e.g., US Patent 7026526 and US Patent Publication 20020129400). Mutagenesis of the C-terminal extein junction in the Pyrococcus species GB-D DNA polymerase produced an altered splicing element that induces cleavage of exteins and inteins but prevents subsequent ligation of the exteins (Xu and Perler. 1996. EMBO J 15: 5146-5153). Mutation of serine 538 to either an alanine or glycine (Ser to Ala or Gly) induced cleavage but prevented ligation. At such position, Ser to Met or Ser to Thr are also used to achieve expression of a polyprotein that is cleaved into separate segments and at least partially not re-ligated. Mutation of equivalent residues in other intein splicing units can also prevent ligation of extein segments due to the relative conservation of amino acids at the C-terminal extein junction to the intein. In instances of low conservation/homology, for example, the first several, e.g., about five, residues of the C-extein and/or the last several residues of the intein segment are systematically varied and screened for the ability to support cleavage but not splicing of given extein segments, in particular extein segments disclosed herein and as understood in the art. There are inteins that do not contain an endonuclease domain; these include the Synechocystis spp dnaE intein and the *Mycobacterium xenopi* GyrA protein (Magnasco et al, Biochemistry, 2004, 43, 10265-10276; Telenti et al. 1997. J. Bacteriol. 179: 6378-6382). Others have been found in nature or have been created artificially by removing the endonuclease encoding domains from the sequences encoding endonuclease-containing inteins (Chong et al. 1997. J. Biol. Chem. 272: 15587-15590). Where desired, the intein is selected originally so that it consists of the minimal number of amino acids needed to perform the splicing function, such as the intein from the *Mycobacterium xenopi* GyrA protein (Telenti et al. 1997.supra). In an alternative embodiment, an intein without endonuclease activity is selected, such as the intein from the *Mycobacterium xenopi* GyrA protein or the *Saccharomyces cerevisiae* VMA intein that has been modified to remove endonuclease domains (Chong et al. 1997. supra).

Further modification of the intein splicing unit may allow the reaction rate of the cleavage reaction to be altered, allowing protein dosage to be controlled by simply modifying the gene sequence of the splicing unit.

In an embodiment, the first residue of the C-terminal extein is engineered to contain a glycine or alanine, a modification that was shown to prevent extein ligation with the Pyrococcus species GB-D DNA polymerase (Xu and Perler. 1996. EMBO J 15: 5146-5153). In this embodiment, preferred C-terminal extein proteins naturally contain a glycine or an alanine residue following the N-terminal methionine in the native amino acid sequence. Fusion of the glycine or alanine of the extein to the C-terminus of the intein provides the native amino acid sequence after processing of the polyprotein. In another embodiment, an artificial glycine or alanine is positioned in the C-terminal extein either by altering the native sequence or by adding an additional amino acid residue onto the N-terminus of the native sequence. In this embodiment, the native amino acid sequence of the protein will be altered by one amino acid after polyprotein processing. In further embodiments, other modifications useful in the present invention are described in US 7026526.

The DNA sequence of the Pyrococcus species GB-D DNA Polymerase intein is SEQ ID NO:1 of US Patent No. 7,026,526. The N-terminal extein junction point is the "aac" sequence (nucleotides 1-3 of SEQ ID NO:1) and encodes an asparagine residue. The splicing sites in the native GB-D DNA Polymerase precursor protein follow nucleotide 3 and nucleotide 1614 in SEQ ID NO:1. The C-terminal extein junction point is the "agc" sequence (nucleotides 1615-1617 of SEQ ID NO:1), which encodes a serine residue. Mutation of the C-terminal extein serine to an alanine or glycine forms a modified intein splicing element that is capable of promoting excision of the polyprotein but not ligation of the extein units.

The DNA sequence of the *Mycobacterium xenopi* GyrA minimal intein is SEQ ID NO:2 of US Patent 7,026,526. The N-terminal extein junction point is the "tac" sequence (nucleotides 1-3 of SEQ ID NO:2) and encodes a tyrosine residue. The splicing sites in the precursor protein follow nucleotide 3 and nucleotide 597 of SEQ ID NO:2. The C-terminal extein junction point is the "acc" sequence (nucleotides 598-600 of SEQ ID NO:2) and encodes a threonine residue. Mutation of the C-terminal extein threonine to an alanine or glycine forms a modified intein splicing element that promotes excision of the polyprotein but does not ligate the extein units.

**2A systems**

Turning now to the 2A protease processing embodiment of the present invention, the activity of 2A may involve ribosomal skipping between codons which prevents formation of peptide bonds (de Felipe et al. 2000. Human Gene Therapy 11:1921-1931; Donnelly et al. 2001. J. Gen. Virol. 82:1013-1025), although it has been considered that the domain acts more like an autolytic enzyme (Ryan et al. 1989. Virology 173:35-45). Studies in which the Foot and Mouth Disease Virus (FMDV) 2A coding region was cloned into expression vectors and transfected into target cells have established that FMDV 2A cleavage of artificial reporter polyproteins is efficient in a broad range of heterologous expression systems (wheat-germ lysate and transgenic tobacco plant (Halpin et al., U.S. Pat. No. 5,846,767 (1998) and Halpin et al. 1999. The Plant Journal 17:453-459); Hs 683 human glioma cell line (de Felipe et al. 1999. Gene Therapy 6:198-208; hereinafter referred to as "de Felipe II"); rabbit reticulocyte lysate and human HTK-143 cells (Ryan et al. 1994. EMBO J. 13:928-933); and insect cells (Roosien et al. 1990. J. Gen. Virol. 71:1703-1711). The FMDV 2A-mediated cleavage of a heterologous polyprotein for a biologically relevant molecule has been shown for IL-12 (p40/p35 heterodimer; Chaplin et al. 1999. J. Interferon Cytokine Res. 19:235-241). In transfected COS-7 cells, FMDV 2A mediated the cleavage of a p40-2A-p35 polyprotein into biologically functional p40 and p35 subunits having activities associated with IL-12.

The FMDV 2A sequence has been incorporated into expression vectors, alone or combined with different IRES sequences to construct bicistronic, tricistronic and tetracistronic vectors. The efficiency of 2A-mediated gene expression in animals was demonstrated by Furler (2001) using recombinant adeno-associated viral (AAV) vectors encoding α-synuclein and EGFP or Cu/Zn superoxide dismutase (SOD-1) and EGFP linked via the FMDV 2A sequence. EGFP and α-synuclein were expressed at substantially higher levels from vectors which included a 2A sequence relative to corresponding IRES-based vectors, while SOD-1 was expressed at comparable or slightly higher levels.

The DNA sequence encoding a self-processing cleavage site is exemplified by viral sequences derived from a picornavirus, including but not limited to an entero-, rhino-, cardio-, aphtho- or Foot-and-Mouth Disease Virus (FMDV). In a preferred embodiment, the self-processing cleavage site coding sequence is derived from a FMDV. Self-processing cleavage sites include but are not limited to 2A and 2A-like domains (Donnelly et al. 2001. J. Gen. Virol. 82:1027-1041, incorporated by reference in its entirety).

Alternatively, a protease recognition site can be substituted for the self-processing site. Suitable protease and cognate recognitions sites include, without limitation, furin, RXR/K-R (SEQ ID NO:1); VP4 of IPNV, S/TXA-S/AG (SEQ ID NO:2); Tobacco etch virus (TEV) protease, EXXYXQ-G (SEQ ID NO:3); 3C protease of rhinovirus, LEVLFQ-GP (SEQ ID NO:4); PC5/6 protease; PACE protease, LPC/PC7 protease; enterokinase, DDDDK-X (SEQ ID NO:5); Factor Xa protease IE/DGR-X (SEQ ID NO:6); thrombin, LVPR-GS (SEQ ID NO:7); genenase I, PGAAH-Y (SEQ ID NO:8); and MMP protease; an internally cleavable signal peptide, an example of which is the internally cleavable signal peptide of influenza C virus (Pekosz A. 1998. Proc. Natl. Acad. Sci. USA 95: I13233-13238) (MGRMAMKWLVVIICFSITSQPASA, SEQ ID NO:11). The protease can be provided in trans or in cis as part of the polyprotein, such that it is encoded within the same transcription and separated from the remainder of the primary translation product, for example, by a self-processing site or protease recognition site.

As more and more antibody therapeutics become approved for clinical applications, there has been steady improvement in the methods for manufacturing these therapeutic proteins over the last 20 years (Wurm, FM, 2004, "Production of recombinant protein therapeutics in cultivated mammalian cells," Nat. Biotechnol. 22(11): 1393). However, still more efficient and reliable production methods are desired by the industry. Some desirable features include higher levels of antibody secretion into the culture media, improved genetic stability of manufacturing cell lines, and greater speed in the generation of cell lines.

In our search for more efficient methods for producing therapeutic antibodies, we have developed methods for expressing antibody heavy chain and light chain from a single open reading frame. In one such method, an intein coding sequence is used to separate the antibody heavy and light chain genes within a single open reading frame (sORF). Advantages offered by such a sORF antibody expression technology include the ability to manipulate gene dosage ratios for heavy and light chains, the proximity of heavy and light chain polypeptides for multi-subunit assembly in ER, and the potential for high efficiency protein secretion.

Other technology for expressing monoclonal antibodies in mammalian cells involves introducing the heavy and the light chain genes in two separate ORFs, each with its own promoter and regulatory sequences. Promoter interference is a concern associated with this method. An alternative method to introduce the antibody heavy and light chain coding sequences into the expression cell lines is to use internal ribosomal entry site (IRES) to separate the antibody heavy and light chain coding sequences. This method has not been widely used because of the decreased efficiency in translating the coding sequence downstream of the IRES sequence. Recently, a method that uses a sequence encoding the foot-and-mouth virus peptide (2A peptide) to separate the coding sequences for antibody heavy and light chain has been described (Fang et.al. 2005. Nat. Biotechnol. 23(5):584-90). In this method the antibody heavy and light chain and the 2A peptide are transcribed as a single mRNA. However, the antibody heavy and light chain polypeptides are cleaved before they enter the endoplasmic reticulum (ER). In addition, two non-native amino acids are left at the C-terminus of the heavy chain after the cleavage/separation of the heavy and light chains. The intein expression system of the present invention is fundamentally different. It differs from the 2A method in that the heavy and light chain polypeptide are translated and brought into ER as a single polyprotein. Advantageously, it is not necessary for non-native amino acids to be included in the mature antibody molecules.

The following descriptions are all in the context of the antibody-production vectors comprising expression cassettes as follows: Promoter - Secretion signal - heavy chain -wt intein such as p. horikoshii Pol I intein - secretion signal - light chain - polyA; Promoter - Secretion signal - heavy chain -modified intein such as p. horikoshii Pol I intein - light chain - polyA; Promoter - Secretion signal - heavy chain - Pol modified intein such as p. horikoshii Pol I intein - secretion signal - light chain - Pol modified intein such as p. horikoshii Pol I intein - Secretion signal - light chain - polyA; Promoter - Secretion signal - heavy chain -wt or modified intein such as p. horikoshii Pol I intein - modified secretion signal - light chain - polyA; Promoter - Secretion signal - light chain -wt or modified intein such as P. horikoshii Pol I intein - modified secretion signal - heavy chain - polyA; Promoter - Secretion signal - heavy chain -wt or modified intein such as p. horikoshii Pol I intein - modified secretion signal - light chain - wt or modified intein such as P. horikoshii Pol I intein-modified secretion signal - light chain - polyA; Promoter - Secretion signal - heavy chain -Furin cleavage site - modified intein such as P. horikoshii Pol I intein - Furin Cleavage site - secretion signal - Light Chain - polyA; and Promoter - heavy chain - Furin cleavage site - modified intein such as P. horikoshii Pol I intein - Furin Cleavage site - Light Chain - Furin Cleavage site - modified intein such as P. horikoshii Pol I intein - Furin cleavage site - light chain - polyA. In further constructs, a modified Psp-GBD Pol intein is used.

The specifically exemplified polyprotein described here makes use of the P. horikoshii Pol I intein that was fused in frame with the D2E7 heavy chain and light chain before and after it respectively. The amino acid that was in the -1 position was a lysine and the amino acid that was in the +1 position was a Methionine, the first amino acid of the light chain signal peptide. The use of methionine at the +1 position allowed for abolishment of splicing, the joint of the heavy and light chains, as we have demonstrated in the latter sections, with an understanding that a nucleophilic amino acid residue such as serine, cysteine, or threonine is needed at the +1 position to allow for splicing. In addition to wt inteins, mutations that change the last amino acid asparagine and the second to last histidine can be used as these mutations generally abolish splicing and preserve cleavage at the N-terminal splicing junction (Mills, 2004; Xu, 1996, Chong, 1997). Alternatively mutations that change the 1^{st} amino acid of the intein can also be used, as such mutations generally abolishes splicing, preserve the cleavage at the C-terminal splicing junction, and either abolish or preserve attenuated cleavage at the N-terminal splicing junction (Nichols, 2004; Evans, 1999, and Xu, 1996). For example, this has been demonstrated to "completely block splicing and inhibit the formation of the branched intermediate, resulting in the cleavage at both splice junctions" (Xu, M.Q., EMBO vol. 15:5146-5153).

In an alternative version of the polypeptide, inclusion of the furin cleavage site allows alteration of the junction sequence with subsequent excision via furin cleavage during secretion. The wildtype sequence for the intein is given in Table 9. In the DNA polymerase I of Pyrococcus spp. GB-D, the cleavage/splice junctions are RQRAIKILAN/S (SEQ ID NO:138) (N terminal) and HN/SYYGYYGYAK (SEQ ID NO:139) (C terminal). Desirably, the endonuclease coding region is excised by HindIII cleavage. The cleavage, splicing and endonuclease functions are dissociated from one another and this endonuclease region can be substituted with a small linker to create mini-inteins that are still capable of cleavage and splicing (Telenti et al. 1997. J. Bacteriol. 179:6378-6382). It is noted that at least one yeast intein functions in mammalian cells (Mootz et al. 2003. J. Am. Chem. Soc. 125:10561-10569). See Tables 8A and 8B for the coding and amino acid sequences of a D2E7 (immunoglobulin) intein construct; Table 8C provides the complete nucleotide sequence of a D2E7 intein construct expression vector. A fusion construction is described that encodes the heavy chain of D2E7 (Humira - registered trademark for adalimumab) fused to the modified Psp Pol1 intein which is itself fused to the coding region for D2E7 light chain. The light chain sequence can be duplicated, with an intein, signal peptide or protease cleavage site(s) separating it from the remainder of the polyprotein. In this embodiment the mature heavy chain is preceded by the heavy chain secretion signal. The intein has been altered as described above, the serine 1 being changed to a threonine and the internal Hind III fragment excised to remove the endonuclease activity. The intein is fused in-frame to the mature D2E7 light chain region. An alternate embodiment would include the light chain secretion signal 5' of the mature light chain. See Figs. 10 and 11 for schematic representation of the D2E7 intein construct and expression vector and Tables 8A-8C for the nucleotide sequences of the expression construct and the complete expression vector and the amino acid sequence of the D2E7 intein construct.

**Signal peptides and signal peptidases**

The signal hypothesis, wherein proteins contain information within their amino acid sequences for protein targeting to the membrane, has been known for more than thirty years. Milstein and co-workers discovered that the light chain of IgG from myeloma cells was synthesized in a higher molecular weight form and was converted to its mature form when endoplasmic reticulum vesicles (microsomes) were added to the translation system, and proposed a model based on these results in which microsomes contain a protease that converts the precursor protein form to the mature form by removing the amino-terminal extension peptide. The signal hypothesis was soon expanded to include distinct targeting sequences within proteins localized to different intracellular membranes, such as the mitochondria and chloroplast. These distinct targeting sequences were later found to be cleaved from the exported protein by specific signal peptidases (SPases).

There are at least three distinct SPases involved in cleaving signal peptides in bacteria. SPase I can process nonlipoprotein substrates that are exported by the SecYEG pathway or the twin arginine translocation (Tat) pathway. Lipoproteins that are exported by the Sec pathway are cleaved by SPase II. SPase IV cleaves type IV prepilins and prepilin-like proteins that are components of the type II secretion apparatus.

In eukaryotes, proteins that are targeted to the endoplasmic reticulum (ER) membrane are mediated by signal peptides that target the protein either cotranslationally or post-translationally to the Sec61 translocation machinery. The ER signal peptides have features similar to those of their bacterial counterparts. The ER signal peptides are cleaved from the exported protein after export into the ER lumen by the signal peptidase complex (SPC). The signal peptides that sort proteins to different locations within the eukaryotic cell have to be distinct because these cells contain many different membranous and aqueous compartments. Proteins that are targeted to the ER often contain cleavable signal sequences. Amazingly, many artificial peptides can function as translocation signals. The most important key feature is believed to be hydrophobicity above a certain threshold. ER signal peptides have a higher content of leucine residues than do bacterial signal peptides. The signal recognition particle (SRP) binds to cleavable signal peptides after they emerge from the ribosome. The SRP is required for targeting the nascent protein to the ER membrane. After translocation of the protein to the ER lumen, the exported protein is processed by the SPC. Another embodiment takes advantage of signal (leader) peptide processing enzymes which occur naturally in eukaryotic cells. In eukaryotes, proteins that are targeted to the endoplasmic reticulum (ER) membrane are mediated by signal peptides that target the protein either cotranslationally or post-translationally to the Sec61 translocation machinery. The ER signal peptides are cleaved from the exported protein after export into the ER lumen by the signal peptidase complex (SPC). Most of known ER signal peptides are either N-terminal cleavable or internally uncleavable. Recently, a number of viral polyproteins such as those found in the hepatitis C virus, hantavirus, flavivirus, rubella virus, and influenza C virus were found to contain internal signal peptides that are most likely cleaved by the ER SPC. These studies on the maturation of viral polyproteins show that SPC can cleave not only amino-terminally located signal peptides, but also after internal signal peptides.

The presenilin-type aspartic protease signal peptide peptidase (SPP) cleaves signal peptides within their transmembrane region. SPP is essential for generation of signal peptide-derived HLA-E epitopes in humans. Recently, a number of viral polyproteins such as those found in the hepatitis C virus, hantavirus, flavivirus, rubella virus, and influenza C virus were found to contain internal signal peptides that are most likely cleaved by the ER SPC. Mutagenesis of the predicted signal peptidase substrate specificity elements may thus block viral infectivity. These studies on the maturation of polyproteins are also very interesting because they show that SPC can cleave not only amino-terminally located signal peptides, but also after internal signal peptides. Signal peptidases are well known in the art. See, for example, Paetzel M. 2002. Chem. Rev. 102(12): 4549; Pekosz A. 1998. Proc. Natl. Acad. Sci. USA. 95:13233-13238; Marius K. 2002. Molecular Cell 10:735-744; Okamoto K. 2004. J. Virol. 78:6370-6380, Vol. 78; Martoglio B. 2003. Human Molecular Genetics 12: R201-R206; and Xia W. 2003. J. Cell Sci. 116:2839-2844.

Proteins that are targeted to the endoplasmic reticulum (ER) membrane are mediated by signal peptides that target the protein either cotranslationally or post-translationally to the Sec61 translocation machinery. The ER signal peptides are cleaved from the exported protein after export into the ER lumen by the signal peptidase complex (SPC). Most of known ER signal peptides are either N-terminal cleavable or internally uncleavable. Recently, a number of viral polyproteins such as those found in the hepatitis C virus, hantavirus, flavivirus, rubella virus, and influenza C virus were found to contain internal signal peptides that are most likely cleaved by the ER SPC. These studies on the maturation of viral polyproteins show that SPC can cleave not only amino-terminally located signal peptides, but also after internal signal peptides.

This invention utilizes internal cleavable signal peptides for expression of a polypeptide in a single transcript. The single transcribed polypeptide is then cleaved by SPC, leaving individual peptides separately or individual peptides being assembled into a protein. The methods of the present invention are applicable to the expression of immunoglobulin heavy chain and light chain in a single transcribed polypeptide, followed by cleavage, then assembly into a mature immunoglobulin. This technology is applicable to polypeptide cytokines, growth factors, or a variety of other proteins, for example, IL-12p40 and IL-12p35 in a single transcribed polypeptide and then assembly into IL-12, or IL-12p40 and IL-23p19 in a single transcribed polypeptide and then assembly into IL-23.

The signal peptidase approach is applicable to mammalian expression vectors which result in the expression of functional antibody or other processed product from a precursor or polyprotein. In the case of the antibody, it is produced from the vector as a polyprotein containing both heavy and light chains, with an intervening sequence between heavy chain and light chain being an internal cleavable signal peptide. This internal cleavable signal peptide can be cleaved by ER-residing proteases, mainly signal peptidases, presenilin or presenilin-like proteases, leaving heavy and light chains to fold and assemble to give a functional molecule, and desirably it is secreted. In addition to the internal cleavable signal peptide derived from hepatitis C virus, other internal cleavable sequences which can be cleaved by ER-residing proteases can be substituted thereof. Similarly, the practice of the invention need not be limited to host cells in which signal peptidase effects cleavage, but it also includes proteases including, but not limited to, presenilin, presenilin-like protease, and other proteases for processing polypeptides. Those proteases have been reviewed in the cited articles, among others.

In addition, the present invention is not limited to the expression of immunoglobulin heavy and light chains, but it also includes other polypeptides and polyproteins expressed in single transcripts followed by internal signal peptide cleavage to release each individual peptide or protein. These proteins may or may not assemble together in the mature product.

Also within the scope of the present invention are expression constructs in which the individual polypeptides are present in alternate orders, i.e., "Peptide 1-internal cleavable signal peptide- peptide 2" or "Peptide 2-internal cleavable signal peptide- peptide 1 ". This invention further includes expression of more than two peptides linked by internal cleavable signal peptides, such as "Peptide 1-internal cleavable signal peptide- peptide 2- internal cleavable signal peptide- peptide 3", and so on.

In addition, this invention applies to expression of both type I and type II transmembrane proteins and to the addition of other protease cleavage sites surrounding expression constructs. One example is to add a furin or PC5/6 cleavage site after an immunoglobulin heavy chain to facilitate the cleaving off of additional amino acid residues at the carboxyl-terminal of heavy chain peptide, e.g., "Heavy chain - furin cleavage site - internal cleavable signal peptide - Light chain". The present invention also includes more than one internal cleavable signal peptide separately or in tandem, for example, "Heavy chain - furin cleavage site - internal cleavable signal peptide - internal cleavable signal peptide - Light chain". Further, this invention includes situations where there is maintenance or removal of self signal peptides of heavy chain and light chain, such as "HC signal peptide - Heavy chain - furin cleavage site - internal cleavable signal peptide - LC signal peptide - Light chain".

The following descriptions are in the context of antibody-production vectors, some of which are described elsewhere herein. Vector designs include but are not limited to the following.

Table of vector designs.

| |
|---|
| Promoter - Secretion signal - heavy chain - internal cleavable signal peptide - secretion signal - light chain - polyA; |
| Promoter - Secretion signal - heavy chain - internal cleavable signal peptide - light chain - polyA; |
| Promoter - Secretion signal - heavy chain - internal cleavable signal peptide - secretion signal - light chain - internal cleavable signal peptide - Secretion signal - light chain -polyA; |
| Promoter - Secretion signal - heavy chain -Furin cleavage site - internal cleavable signal peptide - Furin Cleavage site - secretion siqnal - Light Chain - polyA; and |
| Promoter - heavy chain -Furin cleavage site - internal cleavable signal peptide - Furin Cleavage site - Light Chain - Furin Cleavage site - internal cleavable signal peptide -Furin cleavage site - light chain - polyA. |

A specific example of a fusion construct encodes the heavy chain of D2E7 (Humira/adalimumab) fused to internal cleavable signal peptide which is itself fused to the coding region for D2E7 light chain. In this embodiment the mature heavy chain is preceded by the heavy chain secretion signal. The internal cleavable signal peptide sequence is derived from Influenza C virus. A furin cleavage site is included in the carboxyl terminus of heavy chain. To minimize the affect on the mature antibody, the third to last amino residue of heavy chain is mutated from proline to arginine to create a furin cleavage site. An alternate embodiment would include the light chain secretion signal 5' of the mature light chain. See Tables 9A-9C. The minimal internal cleavable signal peptide sequence from Influenza C virus (MGRMAMKWLVVIICFSITSQPASA, SEQ ID NO:11) is used in the example. A longer sequence may also be used to enhance the cleavage efficiency. See GenBank accession number AB126196. A variety of nucleotide sequence encoding the same amino acid sequence can also be used.

This invention can further utilize internal cleavable signal peptides for maturation of one or more polypeptides within a polyprotein encoded within a single transcript. The single transcribed polypeptide is then cleaved by SPC, leaving individual peptides separately or individual peptides being assembled into a protein. This invention is applicable to express immunoglobulin heavy chain and light chain in a single transcribed polypeptide and then assembly into a mature immunoglobulin. This invention is applicable to express polypeptide cytokines, growth factors, or a variety of other proteins for example to express IL-12p40 and IL-12p35 in a single transcribed polypeptide and then assembly into IL-12, or IL-12p40 and IL-23p19 in a single transcribed polypeptide and then assembly into IL-23.

Positional subcloning of a 2A sequence or other protease or signal peptidase cleavage (recognition) site between two or more heterologous DNA sequences for the inventive vector construct allows the delivery and expression of two or more genes through a single expression vector. Preferably, self processing cleavage sites such as FMDV 2A sequences or protease recognition sequences provide a unique means to express and deliver from a single viral vector, two or multiple proteins, polypeptides or peptides which can be individual parts of, for example, an antibody, heterodimeric receptor or heterodimeric protein.

FMDV 2A is a polyprotein region which functions in the FMDV genome to direct a single cleavage at its own C-terminus, thus functioning in cis. The FMDV 2A domain is typically reported to be about nineteen amino acids in length (LLNFDLLKLAGDVESNPGP, SEQ ID NO:12; TLNFDLLKLAGDVESNPGP, SEQ ID NO:13; Ryan et al. 1991. J. Gen. Virol. 72:2727-2732), however oligopeptides of as few as fourteen amino acid residues (LLKLAGDVESNPGP, SEQ ID NO:14) have been shown to mediate cleavage at the 2A C-terminus in a fashion similar to its role in the native FMDV polyprotein processing.

Variations of the 2A sequence have been studied for their ability to mediate efficient processing of polyproteins (Donnelly et al. 2001). Homologues and variants of a 2A sequence are included within the scope of the invention and include but are not limited to the following sequences: QLLNFDLLKLAGDVESNPGP, SEQ ID NO:15; NFDLLKLAGDVESNPGPFF, SEQ ID NO:16; LLKLAGDVESNPGP, SEQ ID NO:17; NFDLLKLAGDVESNPGP, SEQ ID NO:18; APVKQTLNFDLLKLAGDVESNPGP, SEQ ID NO:19; VTELLYRMKRAETYCPRPLLAIHPTEARHKQKIVAPVKQTLNFDLLKLAGDVESNPG P, SEQ ID NO:20; LLAIHPTEARHKQKIVAPVKQTLNFDLLKLAGDVESNPGP, SEQ ID NO:141; and EARHKQKIVAPVKQTLNFDLLKLAGDVESNPGP, SEQ ID NO:142.

2A sequences and variants thereof can be used to make vectors expressing self-processing polyproteins, including any vector (plasmid or virus based) which includes the coding sequences for proteins or polypeptides linked via self-processing cleavage sites or other protease cleavage sites such that the individual proteins are expressed in the appropriate molar ratios and/or amounts following the cleavage of the polyprotein due to the presence of the self-processing or other cleavage site. These proteins may be heterologous to the vector itself, to each other or to the self-processing cleavage site, e.g., FMDV, thus the self-processing cleavage sites for use in practicing the invention do not discriminate between heterologous proteins and coding sequences derived from the same source as the self-processing cleavage site, in the ability to function or mediate cleavage.

In one embodiment, the FMDV 2A sequence included in a vector according to the invention encodes amino acid residues comprising LLNFDLLKLAGDVESNPGP (SEQ ID NO:12). Alternatively, a vector according to the invention may encode amino acid residues for other 2A-like regions as discussed in Donnelly et al. 2001. J. Gen. Virol. 82:1027-1041 and including, but not limited to, a 2A-like domain from picornavirus, insect virus, Type C rotavirus, trypanosome repeated sequences or the bacterium, *Thermatoga maritima.*

The invention contemplates use of nucleic acid sequence variants that encodes a 2A or 2A-like peptide sequence, such as a nucleic acid coding sequence for a 2A or 2A-like polypeptide which has a different codon for one or more of the amino acids relative to that of the parent nucleotide. Such variants are specifically contemplated and encompassed by the present invention. Sequence variants of 2A peptides and polypeptides are included within the scope of the invention as well. Similarly, proteases supplied in cis or in trans can mediate proteolytic processing via cognate protease recognition (cleavage) sites between the regions of the polyprotein.

In further experiments with intein-antibody expression constructs, we have demonstrated that the *Pyrococcus horikoshii* Pol I intein-mediated protein splicing reaction can take place in mammalian (293E) cells, in ER, and in the context of an antibody (D2E7) heavy and light chain amino acid sequences. For the purpose of using this type of reaction in antibody expression in a single open reading frame (sORF) format, we demonstrated that this reaction can take place in mammalian cells (293E), in ER, and in the context of antibody heavy and antibody light chain amino acid sequences using two constructs, pTT3-HcintLC1 aa-p.hori and pTT3-HcintLC3aa-p.hori. See Tables 11A and 12 A.

These constructs were made on the PTT3 vector backbone. This vector has an Epstein Barr virus (EBV) origin of replication, which allows for its episomal amplification in transfected 293E cells (cells that express Epstein-Barr virus nuclear antigen 1) in suspension culture (Durocher, 2002 , "High level and high-throughput recombinant protein production by transient transfection of suspension-growing human 293-EBNA1 cells, Nucleic Acids Research 30(2):E9). Each vector had one ORF, transcriptionally expressed under the regulatory control of a CMV promoter. In the ORF, a P. horikoshii Poll intein was inserted in frame between the D2E7 heavy and light chains, each having a signal peptide (SP). The pTT3-HcintLC1 aa-p.hori and pTT3-HcintLC3aa -p.hori constructs had 1 native extein amino acid, or 3 native extein amino acids on the either side of the intein, separating the D2E7 antibody heavy and light chain sequences from the intein sequence. These constructs were introduced into 293E cells through transient transfection. Both the culture supernatant and cell pellet samples were analyzed.

Cell pellet samples were lysed under conditions that allow separation of the cytosolic and intracellular membrane fractions. Both of these fractions were analyzed using western blots (WB) with either an anti-heavy chain or an anti-kappa light chain antibody. On these blots we saw the expression of 4 protein species corresponding to a tri-partite form as in the construct's ORF (130 kDa), a fusion of H and L, which was derived from a splicing event (80 kDa), an antibody heavy chain (50 kDa), and an antibody light chain (25 kDa). The first 2 protein species were detected by both the anti-heavy chain and the anti-light chain antibodies, the heavy chain was detected only by the anti-heavy chain antibody, and the light chain was detected by only the anti-light chain antibody. The presence of the 80 kDa protein species, which was detected by both the heavy and the light chain antibodies in both of these constructs, demonstrated that a protein splicing event had taken place. Furthermore, all four protein species were predominantly present in the sub-cellular membrane fraction, which contained endoplasmic reticulum (ER). This indicated that the heavy chain signal peptide (encoded at the beginning of the ORF) had directed the entire polypeptide into ER, where the splicing reaction had taken place. Without wishing to be bound by any particular theory, it is believed that the free heavy and light chain polypeptides were likely to be the result of cleavages at the N-terminal and the C-terminal splicing junctions, resulting from incomplete splicing.

Cell pellet samples were also used for total RNA extraction and Northern blot analysis using both an antibody heavy chain probe and an antibody light chain probe. Northern blot analysis revealed a tripartite mRNA (3.4 kb) in these sORF constructs, which was hybridized with both the heavy chain probe and the light chain probe, but not the mRNA for a separate heavy chain or a light chain. In contrast, in the cell pellet samples that expressed the D2E7 antibody using the conventional approach, that is, introducing the antibody heavy and the light chains from two separate ORFs carried in two pTT3 vectors, mRNAs for the heavy (1.4 kb) and the L chain (0.7kb) were detected using the heavy chain or light chain probes respectively. No tripartite mRNA was detected in these control cell pellets.

The above described data demonstrate that using constructs containing a single ORF (D2E7 heavy chain-P. horikoshi intein-D2E7 light chain), a single mRNA containing all 3 proteins was transcribed. This tripartite message was translated into a tripartite polypeptide, and co-translationally imported into ER, directed by the heavy chain signal peptide present at the N-terminus of the tripartite polyprotein. With this construct, the intein-mediated protein splicing reaction took place inside the ER. This suggested that intein-mediated reactions could be used in the expression of antibodies, as well as other multi-subunit secreted proteins, i.e., those proteins that need to go through the secretory pathway in order to be folded and properly post-translationally modified.

Culture supernatants were also analyzed. Both Western Blot and ELISA allow detection of antibody secreted from expression of the pTT3-HcintL.C1aa-p.hori construct. These studies are discussed in more detail herein below; the amount of secreted antibody expression has been increased through both point mutations and the mutation within the sequence encoding the light chain signal peptide.

Mutations designed to inhibit intein-mediated ligation but preserve the cleavage reactions at either the N-terminal or the C-terminal splicing junctions resulted in increased levels of antibody secretion.

With the goal of enhanced efficiency of antibody secretion, three types of point mutations were designed and tested. The first type of mutation was in the codon of the first serine residue of the C-terminal extein; these constructs had Ser to Met (S>M) changes (construct pTT3-HcintLC-p.hori, construct E, and construct A). The second type of mutation was at the coding for the first serine residue of the intein; such a construct had a Ser to Thr (S>T) change (construct E). The third type of mutation was in the codon for the histidine residue that was the second to last (penultimate) amino acid of the intein; these constructs had a His to Ala (H>A) substitution mutation (construct A and construct B). These mutations were introduced either alone or in combination. All the mutant constructs were designed to preserve the cleavage at either the N- or the C- terminal splicing junctions and reduce splicing of the released exteins, or both, according to reaction mechanisms described in the literature. As outlined below the secretion of D2E7 antibody is achieved using a number of these constructs.

In one experiment, these constructs were introduced into 293E cells through transient transfection, and after 7 days, the cultured supernatants were analyzed for IgG antibody titers by ELISA analysis. The antibody titers for constructs pTT3-HcintLC3aa-p.hori, pTT3-HcintLC1aa-p.hori, pTT3-HcintLC-p.hori, **E, A**, and **B** were 17.0+ 0.6,113.8 + 2.6, 225.8 + 10.0, 9.3 + 0.5, 161.7 + 4.4, and 48.2 + 1.0 ng/ml (average + s.d.), respectively.

These supernatant samples were also analyzed on SDS-PAGE gel under denaturing conditions, and blotted with an antibody against the human IgG heavy chain and an antibody against the human Kappa light chain. On these western blots the antibody heavy chain (∼ 50 kDa) and the antibody light chain (- 25 kDa) are clearly visible in the supernatants generated from constructs pTT3-HcintLC-p.hori and A, consistent with the rank order of IgG levels measured by ELISA.

Cell pellet samples from these transfections were also characterized using western blot analysis. A tripartite-polypeptide (∼ 130 kDa) along with the antibody heavy chain (-50kDa) and light chain (∼25 kDa) bands are seen in the cell pellets containing all the above-described constructs. Among these the constructs, pTT3-HcintLC-p.hori and construct A gave the strongest heavy chain and the light chain bands; therefore it was concluded that there was a correlation between level of intracellular free heavy and light chains and the assembled and secreted antibodies. The spliced product (- 80 kDa), that is the fusion between the antibody heavy chain and light chain, was present in cell pellets generated using construct pTT3-HcintLC3aa-p.hori and to a lesser extent in cell pellets generated from the construct pTT3-HcintLC1 aa-p.hori; it was absent in constructs pTT3-HcintLC-p.hori and constructs A, B, and E. This indicated that the level of protein splicing was inversely correlated with antibody secretion efficiency, consistent with the expectation that the joining of the antibody heavy and light chains would result in misfolding, based on the general knowledge about antibody structure, and this misfolding would consequently prevent secretion due to cellular mechanisms for degradation of misfolded proteins. Another protein species on these blots was intein-light chain fusion (80 kDa, recognized by the light chain antibody but not the heavy chain antibody), which resulted from a cleavage at the N-terminal splicing junction in the absence of any additional cleavages. This band was present in constructs **A**, **B**, **E**, pTT3-HcintLC3aa-p.hori, pTT3-HcintLC1 aa-p.hori, and mostly absent in constructs pTT3-HcintLC-p.hori and H, described herein. Therefore the presence of this protein species was also inversely related to the amount of antibody secretion. Finally, an intein band was also detected in these cell lysates using rabbit polyclonal antisera generated against a P. horikoshii peptide, conjugate to KLH.

We demonstrated that the D2E7 antibody secreted using the sORF construct pTT3-HcintLC-p.hori has the correct N-terminal sequences of the heavy and light chains, the correct heavy and light chain molecular weights and intact molecular weights.

The D2E7 antibody secreted using one of sORF construct pTT3-HcintLC-p.hori was purified by Protein A affinity chromatography and analyzed with respect to the N-terminal sequences of both its heavy chain and its light chains. The unambiguous results indicated that the N-terminal peptide sequence of the heavy chain was EVQLVESGGG (SEQ ID NO:21) and the N-terminal sequence of the light chain was DIQMTQSPSS (SEQ ID NO:22). Thus, using this construct, the cleavage sites used by the signal peptidase w DIQMTQSPSS ere the same as those used in the conventional, two ORF/two vector approach to DE27 antibody expression.

These data provided important scientific insights for the design of the next generation of constructs: the mammalian ER peptidase could recognize and accurately cleave a signal peptide in the newly synthesized polyprotein, even though there were some apparent requirements for its presentation (see herein below).

This purified antibody was analyzed by mass spectrometry, along with the D2E7 produced by the conventional manufacturing process. Under denaturing conditions, D2E7 light chain produced from the pTT3-HcintLC-p.hori construct yielded one single peak on the mass spectrum and its molecular weight (MW) was 23408.8, whereas the molecular weight (MW) of the D2E7 light chain produced from standard manufacturing process was 23409.7, in close agreement. Also under denaturing conditions, the D2E7 heavy chain produced from the pTT3-HcintLC-p.hori construct yielded one major peak and 2 minor peaks on the mass spectrum and their molecular weights (MW) were 50640.6, 50768.2, and 50802.4 respectively, where-as the molecular weights (MW) of the D2E7 heavy chain produced from standard manufacturing process were 50641.7, 50768.6, and 50804.1, respectively, again in close agreement. The 3 peaks correspond to the standard variations of the D2E7 heavy chain.

The intact molecular weights (MW) under native conditions for this D2E7 antibody produced from the pTT3-HcintLC-p.hori construct, along with the D2E7 antibody produced from the manufacturing process, were also determined using mass spectrometry. The D2E7 antibody produced from the pTT3-HcintLC-p.hori construct had 3 peaks, with MW of 148097.6, 148246.9, and 148413.1 respectively; the D2E7 antibody produced from the manufacturing process also had 3 peaks, with MW of 148096.0, 148252.3, and 148412.8, respectively.

These data demonstrated clearly that the D2E7 antibody produced from the pTT3-HcintLC-p.hori construct was identical in size to the D2E7 antibody produced from the conventional manufacturing process, under both the denaturing and native conditions. The ability to produce antibodies with completely authentic amino acid sequences as compared to the conventional manufacturing method is one of the advantages of antibody expression system of the present invention. Using the 2A system as described by Fang et.al. in Nature Biotechnology, 2005, for example, the antibody produced had 2 extra non-native amino acids at the C-terminus of its heavy chain, and this could not be avoided due to the nature of the cleavage.

We have also demonstrated that the D2E7 antibody produced using the pTT3-HcintLC-p.hori sORF construct had the same affinity for binding TNF as the D2E7 antibody produced from the manufacturing process. Real-time binding interactions between rhTNFa antagonists captured across a biosensor chip via immobilized goat anti-human IgG, and soluble rhTNFa were measured using a Biacore 3000 instrument (Pharmacia LKB Biotechnology, Uppsala, Sweden) according to the manufacturer's instructions and standard procedures. Briefly, rhTNFa aliquots were diluted into a HBS-EP (Biacore) buffer, and 150-µl aliquots were injected across the immobilized protein matrices at a flow rate of 25 ml/min. Equivalent concentration of analyte was simultaneously injected over an untreated reference surface to serve as blank sensorgrams for subtraction of bulk refractive index background. The sensor chip surface was regenerated between cycles with two 5-min injections of 10 mM Glycine, at 25 ml/min. The resultant experimental binding sensorgrams were then evaluated using the BIA evaluation 4.0.1 software to determine kinetic rate parameters. Datasets for each antagonist were fit to the 1:1 Langmuir model. For these studies, binding and dissociation data were analyzed under global fit analysis protocol while selecting fit locally for maximum analyte binding capacity (RU) or Rmax attribute. In this case, the software calculated a single dissociation constant (kd), association constant (ka), and affinity constant (Kd). The equilibrium dissociation constant is Kd = kd/ ka.The kinetic on-rate, the kinetic off rate, and the overall affinities were determined by using different TNFα concentrations in the range of 1 - 100 nM. The kinetic on-rate, kinetic off rate, and overall affinity for the D2E7 antibody produced from the construct pTT3-HcintLC-p.hori were 1.61 E+6 (M⁻¹s⁻¹), 5.69 E-5(s⁻¹), and 3.54E-11 (M) respectively; the kinetic on-rate, kinetic off rate, and overall affinity for the D2E7 antibody produced via the manufacturing process were 1.73E+6(M⁻¹s⁻¹), 6.72E-5(s⁻¹), and 3.89E-11 (M) respectively. Biacore analysis indicated that the D2E7 antibody produced using this sORF construct has similar affinity to TNF□ as the D2E7 antibody produced by the conventional manufacturing process.

Modification of Signal Peptide

We have demonstrated that in the sORF construct design, Heavychain-int-LightChain, the antibody secretion level was increased about 10 fold when the hydrophobicity of the light chain signal peptide sequence was reduced through site-directed mutagenesis.

We designed construct H, in which following the P. horikoshi intein sequence, the light chain signal peptide sequence was changed from "MDMRVPAQLLGLLLLWFPGSRC" (SEQ ID NO:23) to "MDMRVPAQLLG DE WFPGSRC" (SEQ ID NO:24). In the same type of transfection experiment as described above, the supernatant of cells which expressed this construct contained 2047 + 116 ng/ml antibody as measured by ELISA analysis. This level of antibody secretion is similar to that described using the 2A technology (1.6 µg/ml). Western blot analysis of this supernatant showed strong bands corresponding to the antibody heavy chain and the antibody light chain.

In a control experiment, this same light chain signal peptide mutation was introduced into a vector for expressing this antibody using the conventional approach (expressing the antibody heavy and light chains from two separate open reading frames in two separate vectors). In this construct, the change in SEQ ID NO:23 to provide SEQ ID NO:24 abolished antibody secretion as expected because the hydrophobic region is important for targeting to the signal recognition particle (SRP) complex on the ER and directing the entrance into the translocon, in the conventional construct design. This verified that in the sORF construct design, the targeting function of the light chain signal peptide is dispensable, even though it can be recognized and cleaved by the ER signal peptidase, consistent with the hypothesis that the entire ORF had entered into the ER as directed by the heavy chain signal peptide at the beginning of the ORF.

The D2E7 antibody secreted using sORF construct H was purified by Protein A affinity chromatography and analyzed with respect to the N-terminal sequence of its light chain. The N-terminal peptide sequence of the light chain was MDMRVPAQLL (SEQ ID NO:26) (without ambiguity), which represented the un-cleaved signal peptide. Even though the literature suggests that the H region of a mammalian ER signal peptide functions primarily in targeting to (SRP) complex and directing the translocation through the translocon, our data suggested that the hydrophobic (H) region of the signal peptide also plays a role in recognition and cleavage by signal peptidase.

We have demonstrated that D2E7 antibodies secreted using both the pTT3-HcintLC-p.hori construct and the construct H were biologically active in cell-based assays. The D2E7 antibody produced using construct pTT3-HcintLC-p.hori and construct H were purified and tested in their ability to neutralize TNFa induced cytotoxicity in L929 cells. This assay was carried out essentially as described in US 6090382 (see Example 4 therein). Human recombinant TNFa causes cytotoxicity in murine L929 cells and was used in this assay. As D2E7, an anti-TNFa antibody, can neutralize this cytotoxicity, L929 assay is one of the cell based assays that can be used to evaluate the biological activity of a particular D2E7 antibody preparation. When analyzed using this assay D2E7 produced from both the pTT3-HcintLC-p.hori construct and the construct H neutralized TNFa induced cytotoxicity. Their IC50 values were similar to that by D2E7 produced from standard manufacturing process.

We have investigated additional constructs with different designs in the light chain signal peptide area. To identify the optimal sORF construct design that would allow for high antibody secretion efficiency, we have designed several additional constructs that varied the region around the C-terminal splicing site and the following signal peptide. Construct J determined "MDMRVPAQWFPGSRC" (SEQ ID NO:25) following the last N of the intein instead of the "MDMRVPAQLLG DE WFPGSRC" (SEQ ID NO:24) of the H construct, which further removed the hydrophobic region inside this signal peptide while preserving the C-terminal region as well as signal peptidase cleavage site. Construct K directed expression of the mature light chain sequence directly following the last N of the intein. Construct L directed expression of "MDMRVPAQLLGLLLLWFPGSGG" (SEQ ID NO:27) following the last N of the intein instead of "MDMRVPAQLLGLLLLWFPGSRC" (SEQ ID NO:23) as in construct pTT3-HcintLC-p.hori, which changed the -1 and -2 amino acids before the cleavage site by the signal peptidase.

In an experiment, these constructs were introduced into 293E cells through transient transfections, and after 7 days, the cultured supernatants were analyzed for IgG antibody titers by ELISA analysis. The antibody titers for constructs **H, J, K,** and **L** were 2328.5+ 79.9, 1289.7 + 129.6, 139.3 + 4.7, and 625.0 + 20.6 ng/ml (average + s.d.), respectively.

The cell pellet samples from these transfections were also analyzed by western blot analysis. All constructs had the tripartite polypeptide band (∼130 kDa), the heavy chain band (∼50kDa), and the light chain band (∼25 kDa) described previously, and none had detectable spliced product (80 kDa and recognized by both the heavy chain and the light chain antibody). Among this group of constructs, the construct **K** produced the most distinctive western blot (WB) pattern in that it produced only a very small amount of the intracellular light chain, and instead it produced the protein species corresponding to intein-light chain fusion, a product of one cleavage event at the N-terminal splice junction. This protein species was absent with the other constructs in this group. The construct **K** differed from the other constructs in two aspects: it did not have a cleavage site by the signal peptidase, and it had an aspartic acid, instead of a methionine or a serine, as the 1 st amino acid residue of the C-terminal extein. Either or both of these features could have prevented the cleavage at the area between the intein and the antibody light chain, resulting in decreased antibody secretion.

The D2E7 antibody secreted using the sORF construct **J** and **L** were purified by Protein A affinity chromatography and analyzed for the N-terminal sequences of their light chain. This analysis indicated that the N-terminal peptide sequence of the light chain produced by construct **J** was MDMRVPAQLL, which represented the un-cleaved signal peptide; whereas the N-terminal peptide sequence of the light chain produced by construct **L** was DIQMTQSPSS, which represented the mature light chain after correct signal peptide cleavage. Therefore, construct L represent a design that gave increased antibody secretion (0.6 -1 ug/ml in different transient transfections) compared to the construct pTT3-HcintLC-p.hori, and its light chain had the correct N-terminal sequence at the same time.

We explored mechanisms of expressing assembled antibody from sORF constructs using inteins and methods for further increasing antibody secretion levels. Intracellular samples of cells tranfected with most of the sORF constructs described contained two antibody light chain species corresponding to the un-processed and processed light chains. In cell transfected with either the positive control constructs or the pTT3-HcintLC-p.hori construct only the processed light chain was secreted, indicating that un-processed light chains that have attached wild type light chain signal peptides could not be assembled and secreted. In contrast, the un-processed light chains from the H and the J constructs were able to be assembled and secreted; both had mutated signal peptides. The extent of the light chain signal peptide processing, as seen in the distributions of the intracellular light chain polypeptide between the un-processed and processed forms, varies depending on the construct. Compared to construct pTT3-HcintLC-p.hori, the construct L had an increased amount of processed light chain, and this has translated into increased antibody secretion.

Based on the above experimental data one way to increase antibody secretion from the sORF constructs is to improve processing efficiency of the light chain signal peptide. This is performed by systematically testing mutations in both the hydrophobic region as well as in the area around the cleavage site, and by testing signal peptides of different length. This can also be done by screening in yeast for peptide sequences that can be cleaved efficiently in this presentation, and by doing similar screenings in CHO cells.

Another method that can be used to increase the antibody secretion level from the sORF constructs is to test different 5' and 3' untranslated regions (UTRs) to increase the stability of the tripartite mRNA, as these mRNAs are larger than traditional mRNAs coding for the antibody heavy and light chains separately.

Another method for increase the antibody secretion level from the sORF constructs is to generate and select stable CHO or NS0 cell line and amplify using either DHFR or GS to increase the recombinant gene copy numbers. The antibody secretion level is independently increased by changing the location of the recombinant genes from episomal (transient) to genomic (stable). It is also enhanced by increasing copy number, and/or by manipulating 5' and 3' UTRs, promoter and enhancer sequences. Vectors expressing dihydrofolate reductase (dhfr) are transfected into dhfr-deficient cell lines. Cell lines with higher vector copy numbers are selected using methotrexate, a competitive inhibitor of dhfr (Kaufman, R.J. and Sharp, P.A. J Mol. Biol. (1982) 159:601-621). As a further independent alternative, expression vectors carrying the cytomegalovirus promoter enhancer in conjunction with a glutamine synthetase selectable marker are employed to increase expression (Bebbington, C.R. (1991) Methods 2:138-145). In addition to increasing the recombinant gene copy numbers, the cellular lineages that are particularly amenable for the processing from sORF construct designs are also selected in this process.

Using modified inteins containing insertions

For the purpose of tracking intracellular intein proteins that have been separated from the D2E7 heavy chain and light chain polypeptides, we have made 4 constructs that introduced a Histidine tag at amino acid sequence positions FRKVR! RGRG(! Represents insertion sites, - HT1), and EGKR ! IPEF (-HT2), in both constructs pTT3- LcintHC-p.hori and construct H. These 2 positions in the P.horikoshi intein was hypothesized to be loops that can tolerate inserts while maintaining its 3-dimentional structure and therefore its function. In one experiment, after 4 days of incubation following transfection of 293E cells, the culture supernatants were analyzed for IgG antibody titers by ELISA analysis. The antibody titers for constructs pTT3- LcintHC-p.hori-HT1, pTT3- LcintHC-p.hori-HT2, construct H-HT1, construct H-HT2, and construct H were 78.3+3.2, 67.3+0.6, 663.0+15.5, 402.7+5.5, 747.0+22.5 ng/ml (average + s.d.), respectively. Use of P.horikoshii intein with insertions at both of the 2 locations have allowed the secretion of assembled antibody. In particular, the use of the intein with an internal inserted tag at the 1 st position gave similar antibody secretion level as compared to using intein without any insertion.

The above data demonstrates that sORF construct designs of the present invention include use of modified inteins that contain an internal tag. A variety of tags are known in the art. Tags of the present invention include but are not limited to fluorescent tags and chemiluminescent tags. Using such constructs, the amount of polyprotein expressed can be monitored using fluorescent detection in individual cells. In addition, these cells can be sorted according to the level of protein expression using FACS. The use of such tags are particularly useful in stable cell line generations as this allows the selection of high producing cells or cell lines through FACS analysis. As taught in the present invention, full length inteins have been observed in the cell lysate after their being auto-cleaved from the flanking antibody heavy and light chains. This provides bases for the detections of fluorescent labeled inteins and their use in stable cell line generation. Tags can also be used in purification of proteins.

From the data presented above, we have learned that the *P. horikoshii* Pol I intein-mediated protein splicing reaction can take place in 293E cells, in ER, and in the context of antibody (as specifically exemplified by D2E7) heavy and light chain amino acid sequences. Point substitution mutations such as S>M at the first amino acid of the C-terminal extein and H>A at the penultimate amino acid of the intein increased the levels of secreted antibody. Reducing the hydrophobicity of the H region of the light chain signal peptide, such as in constructs H and J, produced even higher levels of antibody secretion. The antibody secretion level in a construct that lacks the light chain signal peptide is relatively low, and this appeared to be due to less efficient cleavage at the C-terminal splicing junction. Two approaches are used to increase the efficiency of this cleavage. The first uses an amino acid other than the Aspartic Acid at the +1 position. Also several constructs described here used methionine at the +1 position and gave efficient cleavage at the C-terminal splicing junction. A second approach for increasing the efficiency of this cleavage is to alter the spacing between the C-terminal cleavage site and the light chain globular structure with the use of a linker, optionally followed by a different type of cleavage site such as those described in this disclosure.

While various constructs comprising the *P. horikoshii* intein and the DE27 antibody have been described and tested, other inteins and intein-like proteins (including hedgehog and related family) are used in sORF designs of the invention, e.g., incorporated between antibody heavy and light chains. Other multiple subunit proteins (including two-subunit proteins and proteins with more than two subunits) are substituted for the heavy and light proteins of antibody as well.

In addition to the *P. horikoshii* Poll intein constructs described herein above, we have designed analogous constructs using Sce.VMA intein and Ssp. dnaE mini intein: pTT3-Hc-VMAint-LC-0aa, pTT3-Hc-VMAint-LC-1aa, pTT3-Hc-VMAint-LC-3aa, pTT3-Hc-Ssp-GA-int-LC-0aa, pTT3-Hc- Ssp-GA-int-LC-1 aa, and pTT3-Hc- Ssp-GA-int-LC-3aa. These constructs were transfected into 293E cells, and supernatant and cell pellet samples were analyzed.

In one experiment, after 7 days of incubation following transfection of 293E cells, the culture supernatants were analyzed for IgG antibody titers by ELISA analysis. The antibody titers for constructs pTT3-Hc-VMAint-LC-0aa, pTT3-Hc-VMAint-LC-1aa, pTT3-Hc-VMAint-LC-3aa, pTT3-HC-Ssp-GA-int-LC-0aa, pTT3-HC-Ssp-GA-int-LC-1aa, and pTT3-HC-Ssp-GA-int-LC-3aa were 9.0± 3.5, 12.0 ± 0.0, 39.7 ± 1.2, 90.0 ± 2.0, 38.7 ± 1.5, and 32 ± 2.6 ng/ml (average ± s.d.), respectively.

Cell pellet samples from these transfections were also analyzed by western blot analysis. The tripartite polypeptides were observed in all of these samples. In addition, the heavy chain polypeptide was observed in constructs pTT3-Hc-VMAint-LC-Oaa, pTT3-HC-Ssp-GA-int-LC-0aa, pTT3-HC-Ssp-GA-int-LC-1 aa, and pTT3-HC-Ssp-GA-int-LC-3aa; and the light chain polypeptide was observed in pTT3-HC-Ssp-GA-int-LC-0aa, pTT3-HC-Ssp-GA-int-LC-1 aa, and pTT3-HC-Ssp-GA-int-LC-3aa.

The results of those experiments indicated that inteins, as a class of proteins, can be used successfully in sORF protein expression strategies as we described. Furthermore, bacterial intein-like (BIL) domains and hedgehog (Hog) auto-processing domains, the other 2 members of the Hog/intein (HINT) superfamily besides intein, are applicable in similar construct designs to those described herein.

Additionally, because endonuclease regions that are present in many inteins, including the *P. horikoshii* Poll intein and the Sce.VMA intein, are not useful in the present gene expression strategy, the endonuclease domain can be deleted and replace with a small linker to create "mini-inteins".

These engineered mini-inteins are also useful in the described construct designs, and they present the advantage that the intein coding region is significantly smaller, thus allowing for a larger sequence encoding the polypeptides of interest and/or greater ease of handling the recombinant DNA molecules.

One concern associated with the use of self-processing peptides, such as 2A or 2A-like sequences or protease recognition sequences is that the C or N termini of the one or more of the polypeptide chains contain(s) amino acids derived from the self-processing peptide, i.e. 2A-derived amino acid residues, or protease recognition sequence, depending on the position cleaved and the relative position of the particular chain within the primary translation product. These amino acid residues are "foreign" to the host and may elicit an immune response when the recombinant protein is expressed or delivered in vivo (i.e., expressed from a viral or non-viral vector in the context of gene therapy or administered as an in vitro-produced recombinant protein). In addition, if not removed, 2A-derived or protease site-derived amino acid residues may interfere with protein secretion in producer cells and/or alter protein conformation, resulting in a less than optimal expression level and/or reduced biological activity of the recombinant protein.

Gene expression constructs, engineered such that an additional proteolytic cleavage site is provided between a polypeptide coding sequence and the self processing cleavage site (i.e., a 2A-sequence) or other protease cleavage site as a means for removal of remaining self processing cleavage site derived amino acid residues following cleavage can be used in the practice of the present invention.

Examples of additional proteolytic cleavage sites are furin cleavage sites with the consensus sequence RXK(R)R (SEQ ID NO:1), which can be cleaved by endogenous subtilisin-like proteases, such as furin and other serine proteases within the protein secretion pathway. US Patent Publication 2005/0042721 shows that the 2A residues at the N terminus of the first protein can be efficiently removed by introducing a furin cleavage site RAKR between the first polypeptide and the 2A sequence. In addition, use of a plasmid containing a 2A sequence and a furin cleavage site adjacent to the 2A site was shown to result in a higher level of protein expression than a plasmid containing the 2A sequence alone. This improvement provides a further advantage in that when 2A residues are removed from the N-terminus of the protein, longer 2A- or 2A like sequences or other self-processing sequences can be used. Such longer self-processing sequences such as 2A- or 2A like sequences may facilitate better equimolar expression of two or more polypeptides by way of a single promoter. Still further increased in immunoglobulin expression are achieved when the immunoglobulin light chain coding sequence is present twice and the heavy chain coding sequence is present only once in the polyprotein.

It is advantageous to employ antibodies or analogues thereof with fully human characteristics. These reagents avoid the undesired immune responses induced by antibodies or analogues originating from non-human species. To address possible host immune responses to amino acid residues derived from self-processing peptides, the coding sequence for a proteolytic cleavage site may be inserted (using standard methodology known in the art) between the coding sequence for the first protein and the coding sequence for the self-processing peptide so as to remove the self-processing peptide sequence from the expressed polypeptide, i.e. the antibody. This finds particular utility in therapeutic or diagnostic antibodies for use in vivo.

Any additional proteolytic cleavage site known in the art which can be expressed using recombinant DNA technology vectors may be employed in practicing the invention. Exemplary additional proteolytic cleavage sites which can be inserted between a polypeptide or protein coding sequence and a self processing cleavage sequence (such as a 2A sequence) include, but are not limited to a Furin cleavage site, RXK(R)R (SEQ ID NO:1); a Factor Xa cleavage site, IE(D)GR (SEQ ID NO:6); Signal peptidase I cleavage site, e.g. LAGFATVAQA (SEQ ID NO:28); and thrombin cleavage site, LVPRGS (SEQ ID NO:7).

As an alternative to the IRES, furin, 2A and intein approaches to the expression of more than one mature protein from a single open reading frame, the present invention also provides for protein processing using a hedgehog protein domain positioned within a polyprotein between first and second protein portions. we designed a single open reading frame for expressing antibody heavy chain and light chain with a hedgehog autoprocessing domain to separate the antibody heavy and light chain genes. In cells that carry such an ORF, a single mRNA that consists of at least one antibody heavy chain, one antibody light chain, and one hedgehog autoprocessing domain is transcribed and used to generate a corresponding polyprotein. Post-translationally, the hedgehog autoprocessing domain mediates the separation of the antibody heavy and light chains.

The hedgehog family of proteins contains conserved signaling molecules that act as morphogens in different developmental systems, and are involved in a wide range of human diseases (Kalderon, D. 2005. Biochem Soc Trans. Dec;33(Pt 6):1509-12). Hedgehog proteins have 2 structural domains, a N-terminal domain (Hh-N) that functions in cell signaling, and a C-terminal domain (Hh-C) that catalyzes a post-translational autoprocessing event that cleaves between these 2 domains, adds a cholesterol moiety to the C-terminus of the N-terminal domain, and thereby activates the signaling molecule. (Traci et al. 1997. Cell, 91, 85-97).

Advantages offered by such a sORF antibody expression technology include the ability to manipulate gene dosage ratios for heavy and light chains, the proximity of heavy and light chain polypeptides for multi-subunit assembly in ER, and the potential for high efficiency protein secretion.

The Hh-C protein domains can be used to catalyze an autoprocessing reaction in ER that result in a post-translational cleavage between the antibody heavy chain polypeptide and the Hh-C polypeptide in the single open reading frame construct design described below.

Hedgehog family of proteins has a N-terminal signaling domain and a C-terminal autoprocessing domain. Their C-terminal autoprocessing domains cleave themselves from the N-terminal domains, and add to their C-termini a cholesterol moiety through a 2-step reaction mechanism (Porter et al. 1996. Science. 274(5285):255-9). In addition to cholesterol, other nucleophiles such as DTT or glutathione also stimulate the autoprocessing (Lee et al. 1994. Science, 266, 1528-1537). As the cleavage reaction is catalyzed by the C-terminal autoprocessing domain, a similar cleavage reaction takes place when the N-terminal signaling domain of the hedgehog protein is replaced by an antibody heavy chain or light chain polypeptide. This reaction can be used to separate the antibody heavy and light chains contained within a polyprotein encoded by single open reading frame.

First the antibody expression is tested in a transient expression system and for this purpose, constructs are made on a PTT3 vector backbone. This vector has EBV origin of replication, which allows for its episomal amplification in transfected 293E cells (cells that express Epstein-Barr virus nuclear antigen 1) in suspension culture (Durocher et al. 2002). Each vector has a single open reading frame, driven by a CMV promoter. In one construct design, pTT3-HC-Hh-C25-LC, the entire C-terminal domain of the sonic hedgehog protein from *Drosophila melanogaster* was inserted in frame between the D2E7 heavy and light chains, each of which had a signal peptide (SP). These constructs are introduced into 293E cells through transient transfection. Both the cultured supernatants and cell pellet sample are analyzed.

Cell pellet samples are lysed under conditions that allow separation of the cytosolic and intracellular membrane fractions. Both of these fractions are analyzed using immunoblots techniques with either an anti-heavy chain or an anti- kappa light chain antibody. On these blots protein species are observed include the poly protein (HC-Hh-C25-LC), Hh-C25-LC, and the separate heavy (HC) and light chains (LC). The presence of the latter 3 protein species confirm that the autoprocessing reaction has taken place. The free heavy chain is generated from the cleavage catalyzed by the Hh-C protein domain; the free light chain polypeptides are the results of a cleavage by the signal peptidase. The segregation of protein species in the sub-cellular membrane fraction that contained endoplasmic reticulum (ER) suggest that the heavy chain signal peptide at the beginning of our ORF had directed the entire ORF into ER, where the cleavage reaction takes place.

These cell pellet samples are also subjected to total RNA extraction and Northern blot analysis using both an antibody heavy chain-specific probe and an antibody light chain-specific probe. On these northern blots observations of a tripartite mRNA that hybridizes to both the heavy chain probe and the light chain probe confirms the sORF nature of the construct design. In contrast, in the cell pellet samples that expressed the D2E7 antibody using the conventional approach, that is, introducing the antibody heavy and the light chains from two separate ORFs carried in two pTT3 vectors, mRNAs for the heavy (1.4 kb) and the L chain (0.7kb) have been detected using the heavy chain or light chain probes respectively.

These experiments demonstrate that using constructs containing a single ORF (D2E7 heavy chain- Hh-C25 -D2E7 light chain), a single mRNA containing all 3 proteins is transcribed. This tripartite message is translated into a tripartite polypeptide, and co-translationally imported into ER, directed by the heavy chain signal peptide present at the beginning of the ORF. This indicates that Hh-C protein domain is useful for the expression of antibodies, as well as of other multi-subunit secreted proteins and/or other proteins that need to go through the secretory pathways in order to be folded and properly post-translationally modified.

In addition to the cell pellets the cultured supernatants are analyzed, using both western blots and ELISA, for secreted antibodies, as discussed herein. Constructs using deleted hh-C25 can be tested to compare efficiencies of polyprotein processing and antibody secretion level.

It has been shown that deletion of the C-terminal 63 amino acid from the Hh-C25 protein domain yielded a protein domain, Hh-C17, which can catalyze protein processing but not the cholesterol addition. Hh-C17 expressed well as a recombinant protein and its crystal structure has been determined (Traci et al. 1997. supra). Therefore, in another construct design, pTT3-HC-C17 -LC, this truncated protein domain was inserted between the D2E7 antibody heavy and light chains.

In the homology alignment of hedgehog proteins and inteins, which we have tested in similar construct designs as described in detail herein, the last 8 amino acids are extensions beyond the last predicted β-sheet secondary structure, and they may or may not contribute to the efficiency of the auto-processing. Therefore, an additional construct, pTT3-HC-C17sc -LC, is also tested.

These constructs are introduced into 293E cells through transient transfection, and after 7 days, the cultured supernatants can be analyzed for IgG antibody titers by ELISA analysis. The antibody titers for pTT3-HC-C25 -LC, pTT3-HC-C17 -LC, pTT3-HC-C17sc -LC, and pTT3-HC-C17hn-LC are 0.038, 0.042, 0.040 and 0.046 ug/ml respectively.

These supernatant samples are also analyzed on SDS-PAGE gels (denaturing conditions), and blotted with antibody specific for the human IgG heavy chain and an antibody specific for the human Kappa light chain. On these western blots the antibody heavy chain (- 50 kDa) and the antibody light chain (- 25 kDa) proteins can be observed and correlated with IgG levels measured by ELISA.

The cell pellet samples from these transfections are also analyzed by western blot analysis. The presence and relative density of the four protein species described can be compared among different constructs to determine the protein processing efficiencies afforded by each of the construct designs.

In another class of self-processing proteins, inteins, the last two amino acids tend to be HisAsn. In the process of protein-splicing catalyzed by inteins the Asn undergoes a cyclization, assisted by the His, which results in a cleavage of a peptide bond between the intein and its C-terminal flanking polypeptide. In contrast to inteins, hedgehog auto-processing proteins do not in nature have a C-terminal flanking polypeptide and they do not have a conserved Asn at this position of the polypeptide. In one construct design, pTT3-HC-C17hn -LC, we have introduced His-Asn at this position, replacing Ser-Cys. Without wishing to be bound by theory, the engineered cleavage site at this position makes the separation between the hedgehog auto-processing protein and the antibody light chain in this particular construct design more efficient. The efficiency of antibody secretion is tested as described above.

Antibodies produced through sORF constructs containing hedgehog auto-processing protein are characterized. The D2E7 antibody secreted using the above sORF construct are purified by Protein A affinity chromatography and analyzed for the N-terminal sequences of both its heavy chain and its light chain. These purified antibodies are analyzed by mass spectrometry as previously described, along with the D2E7 produced from the standard manufacturing process, under the denaturing conditions. Using mass spectrometry the intact molecular weights (MW) under native conditions are determined for the D2E7 antibody produced from these constructs, along with the D2E7 antibody produced from the manufacturing process.

The binding between D2E7 antibody and human TNFα is analyzed using Biacore as described before. The kinetic on-rate, kinetic off rate, and overall affinities are determined by using different TNFα concentrations in the range of 1 - 100 nM.

The present invention contemplates the use of any of a variety of vectors for introduction of constructs comprising the coding sequence for two or more polypeptides or proteins and a self processing cleavage sequence into cells. Numerous examples of gene expression vectors are known in the art and may be of viral or non-viral origin. Non-viral gene delivery methods which may be employed in the practice of the invention include but are not limited to plasmids, liposomes, nucleic acid/liposome complexes, cationic lipids and the like.

**Viral vectors**

Viral and other vectors can efficiently transduce cells and introduce their own DNA into a host cell. In generating recombinant viral vectors, non-essential genes are replaced with expressible sequences encoding proteins or polypeptides of interest. Exemplary vectors include but are not limited to viral and non-viral vectors, such a retroviral vector (including lentiviral vectors), adenoviral (Ad) vectors including replication competent, replication deficient and gutless forms thereof, adeno-associated virus (AAV) vectors, simian virus 40 (SV-40) vectors, bovine papilloma vectors, Epstein-Barr vectors, herpes vectors, vaccinia vectors, Moloney murine leukemia vectors, Harvey murine sarcoma virus vectors, murine mammary tumor virus vectors, Rous sarcoma virus vectors and nonviral plasmids. Baculovirus vectors are well known and are suitable for expression in insect cells. A plethora of vectors suitable for expression in mammalian or other eukaryotic cells are well known to the art, and many are commercially available. Commercial sources include, without limitation, Stratagene, La Jolla, CA; Invitrogen, Carlsbad, CA; Promega, Madison, WI and Sigma-Aldrich, St. Louis, MO. Many vector sequences are available through GenBank, and additional information concerning vectors is available on the internet via the Riken BioSource Center.

The vector typically comprises an origin of replication and the vector may or may not in addition comprise a "marker" or "selectable marker" function by which the vector can be identified and selected. While any selectable marker can be used, selectable markers for use in recombinant vectors are generally known in the art and the choice of the proper selectable marker will depend on the host cell. Examples of selectable marker genes which encode proteins that confer resistance to antibiotics or other toxins include, but are not limited to ampicillin, methotrexate, tetracycline, neomycin (Southern et al. 1982. J Mol Appl Genet. 1:327-41), mycophenolic acid (Mulligan et al. 1980. Science 209:1422-7), puromycin, zeomycin, hygromycin (Sugden et al. 1985. Mol Cell Biol. 5:410-3), dihydrofolate reductase, glutamine synthetase, and G418. As will be understood by those of skill in the art, expression vectors typically include an origin of replication, a promoter operably linked to the coding sequence or sequences to be expressed, as well as ribosome binding sites, RNA splice sites, a polyadenylation site, and transcriptional terminator sequences, as appropriate to the coding sequence(s) being expressed.

Reference to a vector or other DNA sequences as "recombinant" merely acknowledges the operable linkage of DNA sequences which are not typically operably linked as isolated from or found in nature. Regulatory (expression and/or control) sequences are operatively linked to a nucleic acid coding sequence when the expression and/or control sequences regulate the transcription and, as appropriate, translation of the nucleic acid sequence. Thus expression and/or control sequences can include promoters, enhancers, transcription terminators, a start codon (i.e., ATG) 5' to the coding sequence, splicing signals for introns and stop codons.

Adenovirus gene therapy vectors are known to exhibit strong transient expression, excellent titer, and the ability to transduce dividing and non-dividing cells in vivo (Hitt et al. 2000. Adv in Virus Res 55:479-505). The recombinant Ad vectors of the instant invention comprise a packaging site enabling the vector to be incorporated into replication-defective Ad virions; the coding sequence for two or more polypeptides or proteins of interest, e.g., heavy and light chains of an immunoglobulin of interest; and a sequence encoding a self-processing cleavage site alone or in combination with an additional proteolytic cleavage site. Other elements necessary or helpful for incorporation into infectious virions, include the 5' and 3' Ad ITRs, the E2 genes, portions of the E4 gene and optionally the E3 gene.

Replication-defective Ad virions encapsulating the recombinant Ad vectors are made by standard techniques known in the art using Ad packaging cells and packaging technology. Examples of these methods may be found, for example, in US Patent No. 5,872,005. The coding sequence for two or more polypeptides or proteins of interest is commonly inserted into adenovirus in the deleted E3 region of the virus genome. Preferred adenoviral vectors for use in practicing the invention do not express one or more wild-type Ad gene products, e.g., E1a, E1b, E2, E3, and E4. Preferred embodiments are virions that are typically used together with packaging cell lines that complement the functions of E1, E2A, E4 and optionally the E3 gene regions. See, e.g. US Patent Nos. 5,872,005, 5,994,106, 6,133,028 and 6,127,175.

Thus, as used herein, "adenovirus" and "adenovirus particle" refer to the virus itself or derivatives thereof and cover all serotypes and subtypes and both naturally occurring and recombinant forms, except where indicated otherwise. Such adenoviruses may be wild type or may be modified in various ways known in the art or as disclosed herein. Such modifications include modifications to the adenovirus genome that is packaged in the particle in order to make an infectious virus. Such modifications include deletions known in the art, such as deletions in one or more of the E1a, E1b, E2a, E2b, E3, or E4 coding regions. Exemplary packaging and producer cells are derived from 293, A549 or HeLa cells. Adenovirus vectors are purified and formulated using standard techniques known in the art.

Adeno-associated virus (AAV) is a helper-dependent human parvovirus which is able to infect cells latently by chromosomal integration. Because of its ability to integrate chromosomally and its nonpathogenic nature, AAV has significant potential as a human gene therapy vector. For use in practicing the present invention rAAV virions may be produced using standard methodology, known to those of skill in the art and are constructed such that they include, as operatively linked components in the direction of transcription, control sequences including transcriptional initiation and termination sequences, and the coding sequence(s) of interest. More specifically, the recombinant AAV vectors of the instant invention comprise a packaging site enabling the vector to be incorporated into replication-defective AAV virions; the coding sequence for two or more polypeptides or proteins of interest, e.g., heavy and light chains of an immunoglobulin of interest; a sequence encoding a self-processing cleavage site alone or in combination with one or more additional proteolytic cleavage sites. AAV vectors for use in practicing the invention are constructed such that they also include, as operatively linked components in the direction of transcription, control sequences including transcriptional initiation and termination sequences. These components are flanked on the 5' and 3' end by functional AAV ITR sequences. By "functional AAV ITR sequences" is meant that the ITR sequences function as intended for the rescue, replication and packaging of the AAV virion.

Recombinant AAV vectors are also characterized in that they are capable of directing the expression and production of selected recombinant polypeptide or protein products in target cells. Thus, the recombinant vectors comprise at least all of the sequences of AAV essential for encapsidation and the physical structures for infection of the recombinant AAV (rAAV) virions. Hence, AAV ITRs for use in expression vectors need not have a wild-type nucleotide sequence (e.g., as described in Kotin. 1994. Hum. Gene Ther. 5:793-801), and may be altered by the insertion, deletion or substitution of nucleotides or the AAV ITRs may be derived from any of several AAV serotypes. Generally, an AAV vector can be any vector derived from an adeno-associated virus serotype known to the art.

Typically, an AAV expression vector is introduced into a producer cell, followed by introduction of an AAV helper construct, where the helper construct includes AAV coding regions capable of being expressed in the producer cell and which complement AAV helper functions absent in the AAV vector. The helper, construct may be designed to down regulate the expression of the large Rep proteins (Rep78 and Rep68), typically by mutating the start codon following p5 from ATG to ACG, as described in US Patent No. 6,548,286, incorporated by reference herein. This is followed by introduction of helper virus and/or additional vectors into the producer cell, wherein the helper virus and/or additional vectors provide accessory functions capable of supporting efficient rAAV virus production. The producer cells are then cultured to produce rAAV. These steps are carried out using standard methodology. Replication-defective AAV virions encapsulating the recombinant AAV vectors of the instant invention are made by standard techniques known in the art using AAV packaging cells and packaging technology. Examples of these methods may be found, for example, in US Patent Nos. 5,436,146; 5,753,500, 6,040,183, 6,093,570 and 6,548,286, incorporated by reference herein in their entireties. Further compositions and methods for packaging are described in Wang et al. (US Patent Publication 2002/0168342), also incorporated by reference herein in its entirety, and include those techniques within the knowledge of those of skill in the art.

In practicing the invention, host cells for producing rAAV or other vector expression vector virions include mammalian cells, insect cells, microorganisms and yeast. Host cells can also be packaging cells in which the AAV (or other) rep and cap genes are stably maintained in the host cell or producer cells in which the AAV vector genome is stably maintained and packaged. Exemplary packaging and producer cells are derived from 293, A549 or HeLa cells. AAV vectors are purified and formulated using standard techniques known in the art. Additional suitable host cells (depending on the vector) include Chinese Hamster Ovary (CHO) cells, CHO dihydrofolate reductase deficient variants such as CHO DX B11 or CHO DG44 cells (see, e.g., Urlaub and Chasin. 1980. Proc. Natl. Acad. Sci. 77:4216-4220), PerC.6 cells (Jones et al. 2003. Biotechnol. Prog. 19:163-168) or Sp/20 mouse myeloma cells (Coney et al. 1994. Cancer Res. 54:2448-2455).

**Retrioviral vectors**

Retroviral vectors are also a common tool for gene delivery (Miller. 1992. Nature 357: 455-460). Retroviral vectors and more particularly lentiviral vectors may be used in practicing the present invention. Accordingly, the term "retrovirus" or "retroviral vector", as used herein is meant to include "lentivirus" and "lentiviral vectors" respectively. Retroviral vectors have been tested and found to be suitable delivery vehicles for the stable introduction of genes of interest into the genome of a broad range of target cells. The ability of retroviral vectors to deliver unrearranged, single copy transgenes into cells makes retroviral vectors well suited for transferring genes into cells. Further, retroviruses enter host cells by the binding of retroviral envelope glycoproteins to specific cell surface receptors on the host cells. Consequently, pseudotyped retroviral vectors in which the encoded native envelope protein is replaced by a heterologous envelope protein that has a different cellular specificity than the native envelope protein (e.g., binds to a different cell-surface receptor as compared to the native envelope protein) may also find utility in practicing the present invention. The ability to direct the delivery of retroviral vectors encoding one or more target protein coding sequences to specific target cells is desirable in practice of the present invention.

The present invention provides retroviral vectors which include e.g., retroviral transfer vectors comprising one or more transgene sequences and retroviral packaging vectors comprising one or more packaging elements. In particular, the present invention provides pseudotyped retroviral vectors encoding a heterologous or functionally modified envelope protein for producing pseudotyped retrovirus.

The core sequence of the retroviral vectors of the present invention may be readily derived from a wide variety of retroviruses, including for example, B, C, and D type retroviruses as well as spumaviruses and lentiviruses (see RNA Tumor Viruses, Second Edition, Cold Spring Harbor Laboratory, 1985). An example of a retrovirus suitable for use in the compositions and methods of the present invention includes, but is not limited to, lentivirus. Other retroviruses suitable for use in the compositions and methods of the present invention include, but are not limited to, Avian Leukosis Virus, Bovine Leukemia Virus, Murine Leukemia Virus, Mink-Cell Focus-inducing Virus, Murine Sarcoma Virus, Reticuloendotheliosis virus and Rous Sarcoma Virus. Particularly preferred Murine Leukemia Viruses include 4070A and 1504A (Hartley and Rowe. 1976. J. Virol. 19:19-25), Abelson (ATCC No. VR-999), Friend (ATCC No. VR-245), Graffi, Gross (ATCC No. VR-590), Kirsteni Harvey Sarcoma Virus and Rauscher (ATCC No. VR-998), and Moloney Murine Leukemia Virus (ATCC No. VR-190). Such retroviruses may be readily obtained from depositories or collections such as the American Type Culture Collection (ATCC; Manassas, VA), or isolated from known sources using commonly available techniques. Others are available commercially.

A retroviral vector sequence of the present invention can be derived from a lentivirus. A preferred lentivirus is a human immunodeficiency virus, e.g., type 1 or 2 (i.e., HIV-1 or HIV-2, wherein HIV-1 was formerly called lymphadenopathy associated virus 3 (HTLV-III) and acquired immune deficiency syndrome (AIDS)-related virus (ARV)), or another virus related to HIV-1 or HIV-2 that has been identified and associated with AIDS or AIDS-like disease. Other lentivirus include, a sheep Visna/maedi virus, a feline immunodeficiency virus (FIV), a bovine lentivirus, simian immunodeficiency virus (SIV), an equine infectious anemia virus (EIAV), and a caprine arthritis-encephalitis virus (CAEV).

Suitable genera and strains of retroviruses are well known in the art (see, e.g., Fields Virology, Third Edition, edited by B. N. Fields et al. 1996. Lippincott-Raven Publishers, see e.g., Chapter 58, Retroviridae: The Viruses and Their Replication, Classification, pages 1768-1771, including Table 1, incorporated herein by reference). Retroviral packaging systems for generating producer cells and producer cell lines that produce retroviruses, and methods of making such packaging systems are also known in the art.

Typical packaging systems comprise at least two packaging vectors: a first packaging vector which comprises a first nucleotide sequence comprising a gag, a pol, or gag and pol genes; and a second packaging vector which comprises a second nucleotide sequence comprising a heterologous or functionally modified envelope gene. The retroviral elements can be derived from a lentivirus, such as HIV. The vectors can lack a functional tat gene and/or functional accessory genes (vif, vpr, vpu, vpx, nef). The system can further comprise a third packaging vector with a nucleotide sequence comprising a rev gene. The packaging system can be provided in the form of a packaging cell that contains the first, second, and, optionally, third nucleotide sequences.

The invention is applicable to a variety of expression systems, especially those with eukaryotic cells, and advantageously mammalian cells. Where native proteins are glycosylated, it is preferred that the expression system be one which will provide native-like glycosylation to the expressed proteins.

Lentiviruses share several structural virion proteins in common, including the envelope glycoproteins SU (gp120) and TM (gp41), which are encoded by the env gene; CA (p24), MA (p17) and NC (p7-11), which are encoded by the gag gene; and RT, PR and IN encoded by the pol gene. HIV-1 and HIV-2 contain accessory and other proteins involved in regulation of synthesis and processing virus RNA and other replicative functions. The accessory proteins, encoded by the vif, vpr, vpu/vpx, and nef genes, can be omitted (or inactivated) from the recombinant system. In addition, tat and rev can be omitted or inactivated, e.g., by mutation or deletion.

First generation lentiviral vector packaging systems provide separate packaging constructs for gag/pol and env, and typically employ a heterologous or functionally modified envelope protein for safety reasons. In second generation lentiviral vector systems, the accessory genes, vif, vpr, vpu and nef, are deleted or inactivated. Third generation lentiviral vector systems are those from which the *tat* gene has been deleted or otherwise inactivated (e.g., via mutation).

Compensation for the regulation of transcription normally provided by tat can be provided by the use of a strong constitutive promoter, such as the human cytomegalovirus immediate early (HCAAV-IE) enhancer/promoter. Other promoters/enhancers can be selected based on strength of constitutive promoter activity, specificity for target tissue (e.g., a liver-specific promoter), or other factors relating to desired control over expression, as is understood in the art. For example, in some embodiments, it is desirable to employ an inducible promoter such as *tet* to achieve controlled expression. The gene encoding *rev* can be provided on a separate expression construct, such that a typical third generation lentiviral vector system will involve four plasmids: one each for gagpol, rev, envelope and the transfer vector. Regardless of the generation of packaging system employed, gag and pol can be provided on a single construct or on separate constructs.

Typically, the packaging vectors are included in a packaging cell, and are introduced into the cell via transfection, transduction or infection. Methods for transfection, transduction or infection are well known by those of skill in the art. A retroviral transfer vector of the present invention can be introduced into a packaging cell line, via transfection, transduction or infection, to generate a producer cell or cell line. The packaging vectors of the present invention can be introduced into human cells or cell lines by standard methods including, e.g., calcium phosphate transfection, lipofection or electroporation. In some embodiments, the packaging vectors are introduced into the cells together with a dominant selectable marker, such as neo, dihydrofolate reductase (DHFR), glutamine synthetase or ADA, followed by selection in the presence of the appropriate drug and isolation of clones. A selectable marker gene can be linked physically to genes encoded by the packaging vector.

Stable cell lines, wherein the packaging functions are configured to be expressed by a suitable packaging cell, are known. For example, see US Patent No. 5,686,279; and Ory et al. 1996. Proc. Natl. Acad. Sci. 93:11400-11406, which describe packaging cells. Further description of stable cell line production can be found in Dull et al. 1998. J. Virol. 72(11):8463-8471; and in Zufferey et al. 1998. J. Virol. 72:9873-9880.

Zufferey et al. 1997. Nat. Biotechnol. 15:871-75, teach a lentiviral packaging plasmid wherein sequences 3' of pol including the HIV-1 envelope gene are deleted. The construct contains tat and rev sequences and the 3' LTR is replaced with poly A sequences. The 5' LTR and psi sequences are replaced by another promoter, such as one which is inducible. For example, a CMV promoter or derivative thereof can be used.

The packaging vectors may contain additional changes to the packaging functions to enhance lentiviral protein expression and to enhance safety. For example, all of the HIV sequences upstream of gag can be removed. Also, sequences downstream of the envelope can be removed. Moreover, steps can be taken to modify the vector to enhance the splicing and translation of the RNA.

Optionally, a conditional packaging system is used, such as that described by Dull et al. 1998. supra. Also preferred is the use of a self-inactivating vector (SIN), which improves the biosafety of the vector by deletion of the HIV-1 long terminal repeat (LTR) as described, for example, by Zufferey et al. 1998. J. Virol. 72:9873-9880. Inducible vectors can also be used, such as through a tetracycline-inducible LTR.

**Promoters**

The vectors of the invention typically include heterologous control sequences, which include, but are not limited to, constitutive promoters, such as the cytomegalovirus (CMV) immediate early promoter, the RSV LTR, the MOMLV LTR, and the PGK promoter; tissue or cell type specific promoters including mTTR, TK, HBV, hAAT, regulatable or inducible promoters, enhancers, etc.

Useful promoters include the LSP promoter (III et al. 1997. Blood Coagul. Fibrinolysis 8S2:23-30), the EF1-alpha promoter (Kim et al. 1990. Gene 91 (2):217-23) and Guo et al. 1996. Gene Ther. 3(9):802-10). Most preferred promoters include the elongation factor 1-alpha (EF1 a) promoter, a phosphoglycerate kinase-1 (PGK) promoter, a cytomegalovirus immediate early gene (CMV) promoter, chimeric liver-specific promoters (LSPs), a cytomegalovirus enhancer/chicken beta-actin (CAG) promoter, a tetracycline responsive promoter (TRE), a transthyretin promoter (TTR), an simian virus 40 (SV40) promoter and a CK6 promoter. An advantageous promoter useful in the practice of the present invention is the adenovirus major late promoter (Berkner and Sharp. 1985. Nucl. Acids Res. 13:841-857). The sequence of a specifically exemplified expression vector employing the adenovirus major late promoter is provided herein below. The sequences of these and numerous additional promoters are known in the art. The relevant sequences may be readily obtained from public databases and incorporated into vectors for use in practicing the present invention.

A particular preferred promoter in the practice of the present invention is the Adenovirus major late promoter. An expression cassette can comprise, in the 5' to 3' direction, an adenovirus major late promoter, a tripartite leader sequence operably to a first coding sequence for a protein of interest or protein chain of interest, a sequence encoding a self processing sequence or protease cleavage sequence, a second coding sequence for a protein or protein chain of interest, and optionally a sequence encoding a self processing sequence or protease cleavage sequence, followed by a third coding sequence for a protein or protein chain of interest. All of these coding sequences are covalently joined and in the same reading frame such that translation is not terminated within the polyprotein coding sequence. During protein synthesis or after completion of the synthesis of the polypeptide self processing or proteolytic processing cleaves the polyprotein into the appropriate protein chains or proteins. In the case of immunoglobulin synthesis, the coding sequence for light chain is present twice within the polyprotein coding sequence. Advantageously, leader sequence coding regions can be associated with the protein or protein chain sequences; processing by signal peptidases can have the added benefit of removing certain residual amino acid residues at the N-termini of proteins downstream of processing sites. Components for immunoglobulin heavy chain are Met, protein initiation methionine; HC, heavy chain; LC, light chain, SPPC, self-processing or protease cleavage site. Expression constructs for immunoglobulin synthesis can include the following: Met-protease-SPPC- HC leader sequence-HC-SPPC-LC leader sequence-LC-SPPC-LC leader sequence-LC; Met-protease-SPPC-LC leader sequence-LC-SPPC-LC leader sequence-LC-SPPC-HC leader sequence-HC; Met-protease-SPPC- LC leader sequence-LC-SPPC-HC leader sequence-HC-SPPC-LC leader sequence-LC; HC leader sequence-HC-SPPC-LC leader sequence-LC-SPPC-LC leader sequence-LC; LC leader sequence-LC-SPPC-HC leader sequence-HC-SPPC--LC leader sequence-LC; LC leader sequence-LC-SPPC-LC leader sequence-LC-SPPC-HC leader sequence-HC; Met-protease-SPPC-HC leader-HC-SPPC-LC leader-LC.

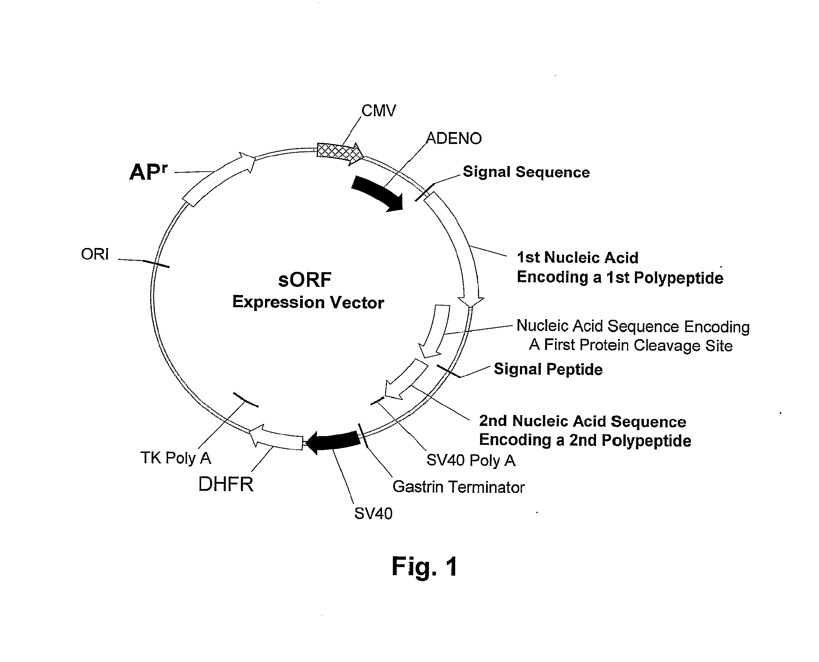

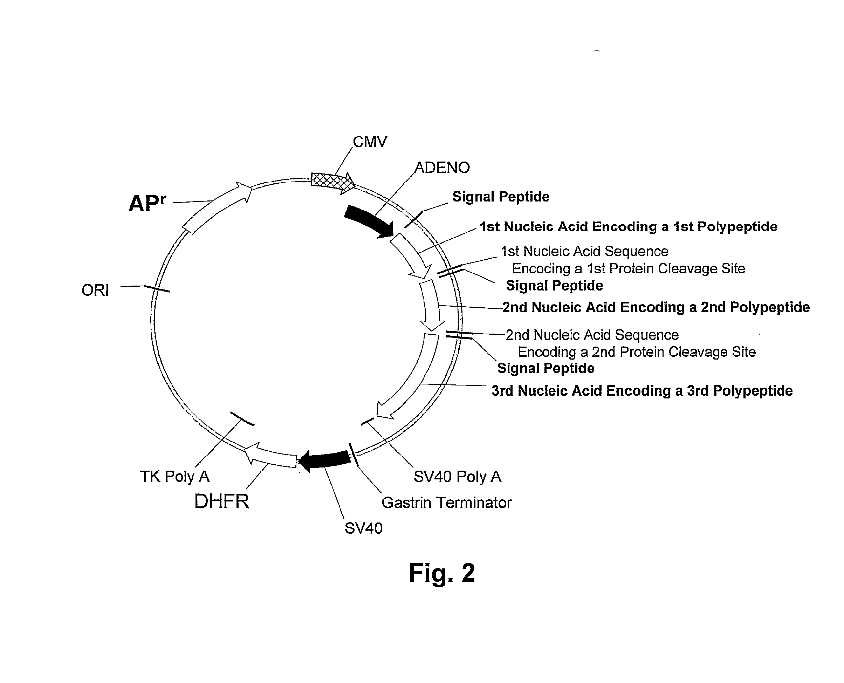

A specifically exemplified polyprotein coding sequence (product Met-HC leader-HC-engineered furin site-TEV cleavage site-TEV Nia protease-TEV cleavage site-LC leader-LC is schematically shown in Fig. 1, and schematic of the expression vector for the expression of this construct is shown in Fig. 2. Anti-TNFα (D2E7) is an exemplary antibody with respect to its HC and LC sequences. The LC leader sequence may not be required for the production of a therapeutic antibody. The SPPS is a TEV protease recognition site, and there is a furin site encoded 5' to the TEV site. Furin cleavage after TEV cleavage restores the "correct" C terminal lysine residue to the heavy chain. The complete DNA sequence of the D2E7-TEV expression vector is shown in Table 1.

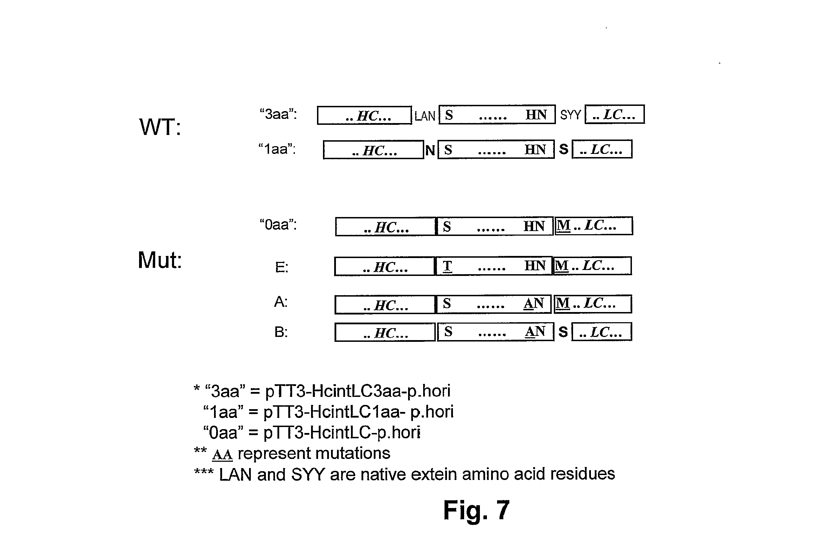

A specifically exemplified D2E7 polyprotein expression construct (D2E7-Lc-LC-HC) encoding a tandem repeat of the LC and cleaved using the 2A protease sequence as cleavage sites has been designed. The D2E7 light chain C termini have been modified to add the Furin cleavage sites. This results in a Glu to Arg change in the (normally) penultimate amino acid and the addition of a lysine to the C-terminus. By placing the two LC sequences 5' to the HC, the two LC copies maintain the same amino acid sequence. The complete nucleotide sequence of the expression vector is shown in Table 6C, and the amino acid sequence and coding sequence of the polyprotein are shown in Tables 6B and 6A, respectively. See also SEQ ID NOs:29-31. A schematic expression vector map is shown in Fig. 7.

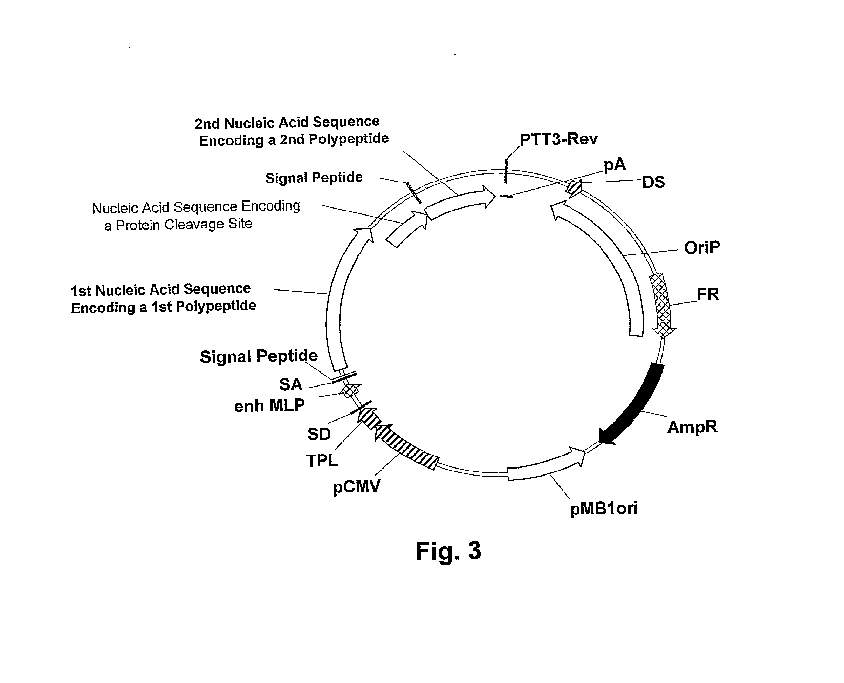

Another specifically exemplified polyprotein (and its coding sequence) is that of ABT-007-TEV; see Tables 2B and 2A, respectively. See SEQ ID NOs:33 and 32. This recombinant antibody specifically binds to erythropoietin receptor (EpoR). The complete sequence of the expression vector encoding the engineered ABT-007-TEV polyprotein is shown in Table 2C (SEQ ID NO:35. See also SEQ ID NO:34. The schematic representation of the vector is shown in Fig. 3.

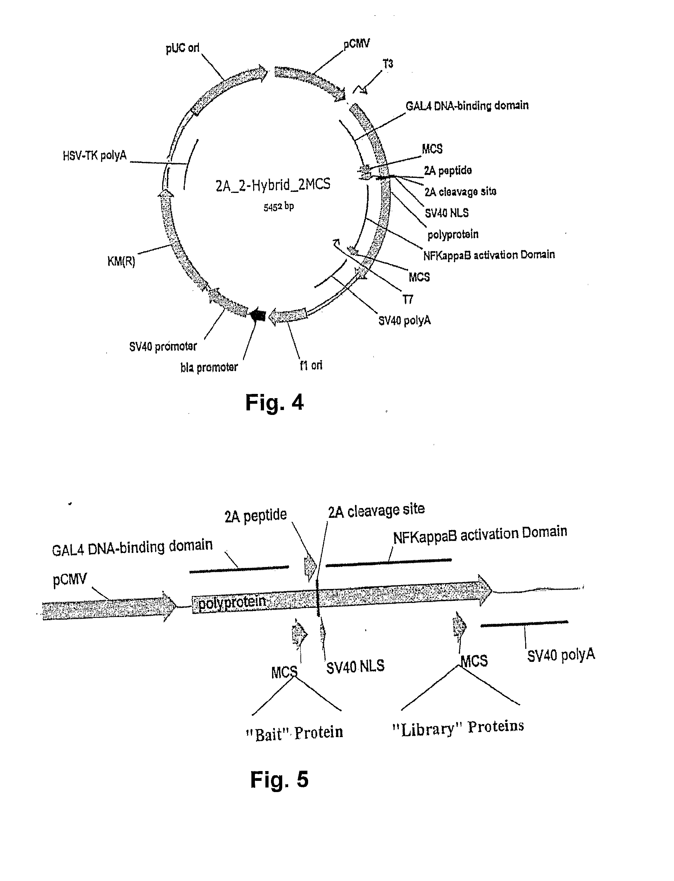

An additional specifically exemplified polyprotein and its coding sequence is that of ABT-874-TEV; see Tables 3B and 3A, respectively. This antibody specifically binds to interleukin-12. The schematic representation of the expression vector is shown in Fig. 4. See also SEQ ID NOs:35-37.

Yet another specifically exemplified polyprotein (and its coding sequence) is that of EL246-GG-TEV; see Tables 4B and 4A. The antibody encoded therein specifically binds to E/L selectin. The expression vector is provided in schematic form in Fig. 5. See also SEQ, ID NOs:38-40.

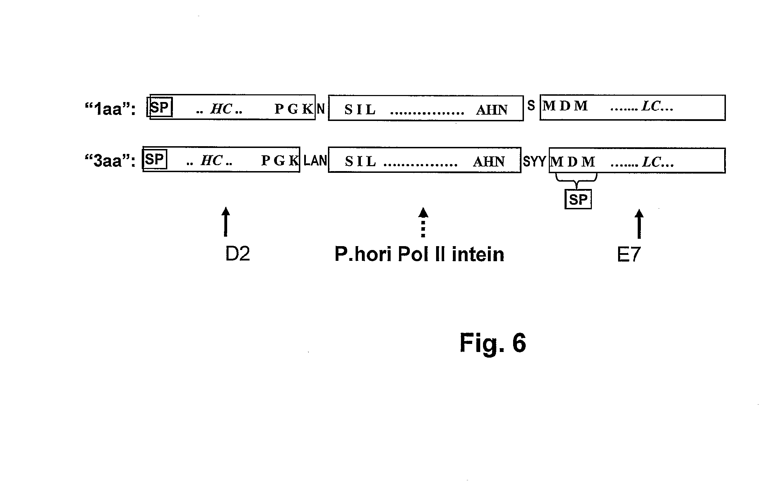

ABT-325-TEV is an engineered antibody with binding specificity for interleukin-18. The coding and amino acid sequences of the polyprotein are given in Tables 5A and 5B, respectively, and the complete expression vector sequence is provided in Table 5C. The expression vector for its synthesis is shown in Fig. 6. See also SEQ ID NOs:41-43.

Also provided is a TEV protease with its nuclear localization signal (NLS) removed (TEV NLS-). The TEV or TEV(NLS-) protease can also be expressed in cells transiently or stably as part of a separate vector or separate transcript. The TEV(NLS-) protein may be anchored to the ER or to the ribosome by including an ER anchor sequence or by fusing to a small ribosome binding protein, respectively at the previous NLS portion.

While the present application contains discussion of proteolytic cleavage of precursor proteins and polyproteins during synthesis or in the cell after synthesis, it is understood that the polyproteins and precursor proteins (proproteins) can be achieved after collection of those proteins with the use of appropriate protease(s) in vitro.

Within the scope of the present invention, particular expressed antibodies (immunoglobulins) can include, inter alia, those which specifically bind tumor necrosis factor (engineered antibody corresponding to and/or derived from HUMIRA/D2E7; trademark for adalimumab of Abbott Biotechnology Ltd., Hamilton, Bermuda); interleukin-12 (engineered antibody derived from ABT-874); interleukin-18 (engineered antibody derived from ABT-325); recombinant erythropoietin receptor (engineered antibody derived from ABT-007); interleukin-18 (engineered antibody derived from ABT-325); or E/L selectin (engineered antibody derived from EL246-GG). Coding and amino acid sequences of the engineered polyproteins are shown in Tables 1-5. Further antibodies which are suitable to the present invention include, e.g., Remicade (infliximab); Rituxan/Mabthera (rituximab); Herceptin (trastuzumab); Avastin (bevacizumab);Synagis (palivizumab); Erbitux (cetuximab); Reopro (abciximab); Orthoclone OKT3 (muromonab-CD3); Zenapax (daclizumab); Simulect (basiliximab); Mylotarg (gemtuzumab); Campath (alemtuzumab); Zevalin (ibritumomab); Xolair (omalizumab); Bexxar (tositumomab); and Raptiva (efalizumab); wherein generally a trademark-brand name is followed by a respective generic name in parentheses. Additional suitable proteins include, e.g., one or more of epoetin alfa, epoetin beta, etanercept, darbepoetin alfa, filgrastim, interferon beta 1 a, interferon beta 1b, interferon alfa-2b, insulin glargine, somatropin, teriparatide, follitropin alfa, dornase, Factor VIII, Factor VII, Factor IX, imiglucerase, nesiritide, lenograstim, and Von Willebrand factor; wherein one or more generic designations may each correspond to one or more trademark-brand names of products. Other antibodies and proteins are suitable to the present invention as would be understood in the art..

The present invention also contemplates the controlled expression of the coding sequence for two or more polypeptides or proteins or proproteins of interest. Gene regulation systems are useful in the modulated expression of a particular gene or genes. In one exemplary approach, a gene regulation system or switch includes a chimeric transcription factor that has a ligand binding domain, a transcriptional activation domain and a DNA binding domain. The domains may be obtained from virtually any source and may be combined in any of a number of ways to obtain a novel protein. A regulatable gene system also includes a DNA response element which interacts with the chimeric transcription factor. This transcription regulatory element is located adjacent to the gene to be regulated.

Exemplary transcription regulation systems that may be employed in practicing the present invention include, for example, the Drosophila ecdysone system (Yao et al. 1996. Proc. Natl. Acad. Sci. 93:3346), the Bombyx ecdysone system (Suhr et al. 1998. Proc. Natl. Acad. Sci. 95:7999), the GeneSwitch (trademark of Valentis, The Woodlands, TX) synthetic progesterone receptor system which employs RU486 as the inducer (Osterwalder et al. 2001. Proc. Natl. Acad. Sci. USA 98(22):12596-601); the Tet and RevTet Systems (tetracycline regulated expression systems, trademarks of BD Biosciences Clontech, Mountain View, CA), which employ small molecules, such as tetracycline (Tc) or analogues, e.g. doxycycline, to regulate (turn on or off) transcription of the target (Knott et al. 2002. Biotechniques 32(4):796, 798, 800); ARIAD Regulation Technology (Ariad, Cambridge, MA) which is based on the use of a small molecule to bring together two intracellular molecules, each of which is linked to either a transcriptional activator or a DNA binding protein. When these components come together, transcription of the gene of interest is activated. Ariad has a system based on homodimerization and a system based on heterodimerization (Rivera et al. 1996. Nature Med. 2(9):1028-1032; Ye et al. 2000. Science 283:88-91).

The expression vector constructs of the invention comprising nucleic acid sequences encoding antibodies or fragments thereof or other heterologous proteins or pro-proteins in the form of self-processing or protease-cleaved recombinant polypeptides may be introduced into cells in vitro, ex vivo or in vivo for delivery of foreign, therapeutic or transgenes to cells, e.g., somatic cells, or in the production of recombinant polypeptides by vector-transduced cells.

**Host Cells and Delivery of Vectors**

The vector constructs of the present invention may be introduced into suitable cells in vitro or ex vivo using standard methodology known in the art. Such techniques include, e.g., transfection using calcium phosphate, microinjection into cultured cells (Capecchi. 1980. Cell 22:479-488), electroporation (Shigekawa et al. 1988. BioTechnology 6:742-751), liposome-mediated gene transfer (Mannino et al. 1988. BioTechnology 6:682-690), lipid-mediated transduction (Feigner et al. 1987. Proc. Natl. Acad. Sci. USA 84:7413-7417), and nucleic acid delivery using high-velocity microprojectiles (Klein et al. 1987. Nature 327:70-73).

For in vitro or ex vivo expression, any cell effective to express a functional protein product may be employed. Numerous examples of cells and cell lines used for protein expression are known in the art. For example, prokaryotic cells and insect cells may be used for expression. In addition, eukaryotic microorganisms, such as yeast may be used. The expression of recombinant proteins in prokaryotic, insect and yeast systems are generally known in the art and may be adapted for antibody or other protein expression using the compositions and methods of the present invention.

Examples of cells useful for expression further include mammalian cells, such as fibroblast cells, cells from non-human mammals such as ovine, porcine, murine and bovine cells, insect cells and the like. Specific examples of mammalian cells include, without limitation, COS cells, VERO cells, HeLa cells, Chinese hamster ovary (CHO) cells, CHO DX B11 cells, CHO DG44 cells, PerC.6 cells, Sp2/0 cells, 293 cells, NSO cells, 3T3 fibroblast cells, W138 cells, BHK cells, HEPG2 cells, and MDCK cells.

Host cells are cultured in conventional nutrient media, modified as appropriate for inducing promoters, selecting transformants, or amplifying the genes encoding the desired sequences. Mammalian host cells may be cultured in a variety of media. Commercially available media such as Ham's F10 (Sigma), Minimal Essential Medium (MEM), Sigma), RPMI 1640 (Sigma), and Dulbecco's Modified Eagle's Medium (DMEM), Sigma) are typically suitable for culturing host cells. A given medium is generally supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics, trace elements, and glucose or an equivalent energy source. Any other necessary supplements may also be included at appropriate concentrations as well known to those skilled in the art. The appropriate culture conditions for a particular cell line, such as temperature, pH and the like, are generally known in the art, with suggested culture conditions for culture of numerous cell lines, for example, in the ATCC Catalogue (available on the internet at "atcc.org/SearchCatalogs/AllCollections.cfm" or as instructed by commercial suppliers.

The expression vectors may be administered in vivo via various routes (e.g., intradermally, intravenously, intratumorally, into the brain, intraportally, intraperitoneally, intramuscularly, into the bladder etc.), to deliver multiple genes connected via a self processing cleavage sequence to express two or more proteins or polypeptides in animal models or human subjects. Dependent upon the route of administration, the therapeutic proteins elicit their effect locally (in brain or bladder) or systemically (other routes of administration). The use of tissue specific promoters 5' to the open reading frame(s) results in tissue specific expression of the proteins or polypeptides encoded by the entire open reading frame.

Various methods that introduce a recombinant expression vector carrying a transgene into target cells in vitro, ex vivo or in vivo have been previously described and are well known in the art. The present invention provides for therapeutic methods, vaccines, and cancer therapies by infecting targeted cells with the recombinant vectors containing the coding sequence for two or more proteins or polypeptides of interest, and expressing the proteins or polypeptides in the targeted cell.

For example, in vivo delivery of the recombinant vectors of the invention may be targeted to a wide variety of organ types including, but not limited to brain, liver, blood vessels, muscle, heart, lung and skin.

In the case of ex vivo gene transfer, the target cells are removed from the host and genetically modified in the laboratory using recombinant vectors of the present invention and methods well known in the art.

The recombinant vectors of the invention can be administered using conventional modes of administration including but not limited to the modes described above. The recombinant vectors of the invention may be in a variety of formulations which include but are not limited to liquid solutions and suspensions, microvesicles, liposomes and injectable or infusible solutions. The preferred form depends upon the mode of administration and the therapeutic application.

Advantages of the present inventive recombinant expression vector constructs of the invention in immunoglobulin or other biologically active protein production in vivo include administration of a single vector for long-term and sustained antibody expression in patients; in vivo expression of an antibody or fragment thereof (or other biologically active protein) having full biological activities; and the natural posttranslational modifications of the antibody generated in human cells. Desirably, the expressed protein is identical to or sufficiently identical to a naturally occurring protein so that immunological responses are not triggered where the expressed protein is administered to on multiple occasions or expressed continually in a patient in need of said protein.

The recombinant vector constructs of the present invention find further utility in the in vitro production of recombinant antibodies and other biologically active proteins for use in therapy or in research. Methods for recombinant protein production are well known in the art and may be utilized for expression of recombinant antibodies using the self processing cleavage site or other protease cleavage site-containing vector constructs described herein.

In one aspect, the invention provides methods for producing a recombinant immunoglobulin or fragment thereof, by introducing an expression vector such as described above into a cell to obtain a transfected cell, wherein the vector comprises in the 5' to 3' direction: a promoter operably linked to the coding sequences for immunoglobulin heavy and two light chains or fragment thereof, a self processing sequence such as a 2A or 2A-like sequence or protease cleavage site between each of said chains. It is appreciated that the coding sequence for either the immunoglobulin heavy chain or the coding sequence for the immunoglobulin light chain may be 5' to the 2A sequence (i.e. first) in a given vector construct. Alternatively, the protease cognate to the protease cleavage site can be expressed as part of the polyprotein so that it is either self-processed from the remainder of the polyprotein or proteolytically cleaved by a separate (or the same) protease. Other multichain proteins or other proteins (such as those from the two- or three-hybrid systems) can be expressed in processed, active form by substituting the relevant coding sequences, interspersed by self-processing sites or protease recognition sites also correctly sized, separate proteins are produced.

The two (and other) hybrid system approach has been used to screen cDNA libraries for previously unrecognized binding partners to a know ligand or subunit of a protein complex. With appropriate variations to this system, proteins or subunits which inhibit, compete or disrupt binding in a known complex can also be identified. Although the two (and other) hybrid systems have been applied to a variety of scientific inquiries, these systems can be inefficient because of the significance frequency of false positive or false negative results. Those false signals have been at least in some instances, attributed to an imbalance in the relative expression of the "bait" protein relative to candidate binding partner proteins or candidate disrupter proteins. An additional advantage of the strategy of the present invention is that only one plasmid is transfected or transformed into the host cell, and only a single selection is needed for that plasmid, instead of two selections in the binary vector two hybrid schemes. The approach can also be adapted for use in three hybrid systems. For discussions of the two hybrid systems, see Toby and Golemis. 2001. Methods 24:201-217; Vidal and Legrain. 1999. Nucl. Acids Res. 27:919-929; Drees, B. 1999. Curr. Op. Chem. Biol. 3:64-70; and Fields and Song. 1989. Nature 340:245-246. Fig. 9 shows a schematic representation of a polyprotein/self-processing or protease cleavage expression strategy for bait and prey proteins (or candidate prey proteins), and Fig. 8 shows a vector containing an expression cassette for bait and prey protein production using this approach. The vector expression cassette is structured to translate the bait protein first as a GAL4::bait::2A peptide fusion, which is self processed after the translation of the 2A peptide. The second open reading frame (ORF) is an NFkappaB::library fusion protein. Engineering of the bait protein into MCS1 requires an in-frame translation into the 2A self-processing peptide sequence. Engineering of an expression library in the downstream MCS2 is less critical.

The strategy provided herein can be similarly adapted to the expression of proteins that are expressed as pro-forms that are processed to the mature, active form by proteolytic cleavage, thus providing compositions and methods for recombinant expression. Examples of such proteins include, but are not limited to interleukins 1 and 18 (IL-1 and IL-18) insulin, among others. IL-1 and IL-18 are produced in the cytoplasm of inflammatory cells. These molecules lack a traditional secretion signal and must be cleaved by a protease in order to be secreted as the biologically active form. IL-1 is processed to the mature form by interleukin converting enzyme (ICE). Pro-IL-18 is converted to mature IL-18 by caspases. Production of these molecules in recombinant form is difficult because the cells frequently used as hosts do not express the proteases needed to produce biologically active mature forms of these proteins. Expression of these cytokines without the pro domains leads to inactive molecules and/or low levels of production. The present invention provides primary translation products which contain an engineered self processing site (e.g., 2A sequence) or an inserted protease cleavage site between the pro domain and the amino acid of the mature polypeptide, without the need to express a potentially toxic protease in parallel with the protein of interest.

In a related aspect, the invention provides a method for producing a recombinant immunoglobulin or fragment thereof, by introducing an expression vector such as described above into a cell, wherein the vector further comprises an additional proteolytic cleavage site between the first and second immunoglobulin coding sequences. A preferred additional proteolytic cleavage site is a furin cleavage site with the consensus sequence RXK/R-R (SEQ ID NO:1). For a discussion, see US Patent Publication 2005/0003482A1.

In one exemplary aspect of the invention, vector introduction or administration to a cell is followed by one or more of the following steps: culturing the transfected cell under conditions for selecting a cell and expressing the polyprotein or proprotein; measuring expression of the immunoglobulin or the fragment thereof or other protein(s); and collecting the immunoglobulin or the fragment thereof or other protein(s).

Another aspect of the invention provides a cell for expressing a recombinant immunoglobulin or a fragment thereof or other protein(s) or protein of interest, wherein the cell comprises an expression vector for the expression of two or more immunoglobulin chains or fragments thereof or other proprotein or proteins, a promoter operably linked to a first coding sequence for an immunoglobulin or other chain or fragment thereof, a self processing or other cleavage coding sequence, such as a 2A or 2A-like sequence or a protease recognition site, and a second coding sequence for an immunoglobulin or other chain or a fragment thereof, wherein the self processing cleavage sequence or protease recognition site coding sequence is inserted between the first and the second coding sequences. In a related aspect, the cell comprises an expression vector as described above wherein the expression vector further comprises an additional proteolytic cleavage site between the first and second immunoglobulin or other coding sequences of interest. A preferred additional proteolytic cleavage site is a furin cleavage site with the consensus sequence RXR/K-R (SEQ ID NO:1).

As used herein, "the coding sequence for a first chain of an immunoglobulin molecule or a fragment thereof" refers to a nucleic acid sequence encoding a protein molecule including, but not limited to a light chain or heavy chain for an antibody or immunoglobulin, or a fragment thereof.

As used herein, a "the coding sequence for a second chain of an immunoglobulin molecule or a fragment thereof" refers to a nucleic acid sequence encoding a protein molecule including, but not limited to a light chain or heavy chain for an antibody or immunoglobulin, or a fragment thereof. It is understood, in one aspect of the present invention, that improved expression results when there are two copies of the immunoglobulin light chain coding sequence per copy of the heavy chain coding sequence.

The sequence encoding the first or second chain for an antibody or immunoglobulin or a fragment thereof includes a heavy chain or a fragment thereof derived from an IgG, IgM, IgD, IgE or IgA. As broadly stated, the sequence encoding the chain for an antibody or immunoglobulin or a fragment thereof also includes the light chain or a fragment thereof from an IgG, IgM, IgD, IgE or IgA. Genes for whole antibody molecules as well as modified or derived forms thereof, include, e.g., other antigen recognition molecules fragments like Fab, single chain Fv (scFv) and F(ab')₂. The antibodies and fragments can be animal-derived, human-mouse chimeric, humanized, altered by Deimmunisation™ (Biovation Ltd), altered to change affinity for Fc receptors, or fully human. Desirably, the antibody or other recombinant protein does not elicit an immune response in a human or animal to which it is administered.

The antibodies can be bispecific and include, but are not limited to, diantibodies, quadroma, mini-antibodies, ScBs antibodies and knobs-into-holes antibodies.

The production and recovery of the antibodies themselves can be achieved in various ways well known in the art (Harlow et al. 1988. Antibodies, A Laboratory Manual, Cold Spring Harbor Laboratory. Other proteins of interest are collected and/or purified and/or used according to methods well known to the art.

In practicing the invention, the production of an antibody or variant (analogue) thereof using recombinant DNA technology can be achieved by culturing a modified recombinant host cell under culture conditions appropriate for the growth of the host cell and the expression of the coding sequences. In order to monitor the success of expression, the antibody levels with respect to the antigen may be monitored using standard techniques such as ELISA, RIA and the like. The antibodies are recovered from the culture supernatant using standard techniques known in the art. Purified forms of these antibodies can, of course, be readily prepared by standard purification techniques including but not limited to, affinity chromatography via protein A, protein G or protein L columns, or with respect to the particular antigen, or even with respect to the particular epitope of the antigen for which specificity is desired. Antibodies can also be purified with conventional chromatography, such as an ion exchange or size exclusion column, in conjunction with other technologies, such as ammonia sulfate precipitation and size-limited membrane filtration. Where expression systems are designed to include signal peptides, the resulting antibodies are secreted into the culture medium or supernatant; however, intracellular production is also possible.

The production and selection of antigen-specific, fully human monoclonal antibodies from mice engineered with human Ig loci, has previously been described (Jakobovits et al. 1998. Advanced Drug Delivery Reviews 31:33-42; Mendez et al. 1997. Nature Genetics 15: 146-156; Jakobovits et al. 1995. Curr Opin Biotechnol 6: 561-566; Green et al. 1994. Nature Genetics Vol. 7:13-21).

High level expression of therapeutic monoclonal antibodies has been achieved in the milk of transgenic goats, and it has been shown that antigen binding levels are equivalent to that of monoclonal antibodies produced using conventional cell culture technology. This method is based on development of human therapeutic proteins in the milk of transgenic animals, which carry genetic information allowing them to express human therapeutic proteins in their milk. Once they are produced, these recombinant proteins can be efficiently purified from milk using standard technology. See e.g., Pollock et al. 1999. J. Immunol. Meth. 231:147-157 and Young et al. 1998. Res Immunol. 149(6): 609-610. Animal milk, egg white, blood, urine, seminal plasma and silk worm cocoons from transgenic animals have demonstrated potential as sources for production of recombinant proteins at an industrial scale (Houdebine L M. 2002. Curr Opin Biotechnol 13:625-629; Little et al. 2000. Immunol Today, 21(8):364-70; and Gura T. 2002. Nature, 417:584-5860. The invention contemplates use of transgenic animal expression systems for expression of a recombinant an antibody or variant (analogue) or other protein(s) of interest thereof using the self-processing cleavage site-encoding and/or protease recognition site vectors of the invention.

Production of recombinant proteins in plants has also been successfully demonstrated including, but not limited to, potatoes, tomatoes, tobacco, rice, and other plants transformed by Agrobacterium infection, biolistic transformation, protoplast transformation, and the like. Recombinant human GM-CSF expression in the seeds of transgenic tobacco plants and expression of antibodies including single-chain antibodies in plants has been demonstrated. See, e.g., Streaffield and Howard. 2003. Int. J. Parasitol. 33:479-93; Schillberg et al. 2003. Cell Mol Life Sci. 60:433A5; Pogue et al. 2002. Annu. Rev. Phytopathol. 40:45-74; and McCormick et al. 2003. J Immunological Methods, 278:95-104. The invention contemplates use of transgenic plant expression systems for expression of a recombinant immunoglobulin or fragment thereof or other protein(s) of interest using the protease cleavage site or self-processing cleavage site-encoding vectors of the invention.

Baculovirus vector expression systems in conjunction with insect cells are also gaining ground as a viable platform for recombinant protein production. Baculovirus vector expression systems have been reported to provide advantages relative to mammalian cell culture such as ease of culture and higher expression levels. See, e.g., Ghosh et al. 2002. Mol Ther. 6:5-11, and Ikonomou et al. 2003. Appl Microbiol Biotechnol. 62:1-20. The invention further contemplates use of baculovirus vector expression systems for expression of a recombinant immunoglobulin or fragment thereof using the self-processing cleavage site-encoding vectors of the invention. Baculovirus vectors and suitable host cells are well known to the art and commercially available.

Yeast-based systems may also be employed for expression of a recombinant immunoglobulin or fragment thereof or other protein(s) of interest, including two- or three-hybrid systems, using the self-processing cleavage site-encoding vectors of the invention. See, e.g., US Patent No. 5,643,745, incorporated by reference herein.

It is understood that the expression cassettes and vectors and recombinant host cells of the present invention which comprise the coding sequences for a self-processing peptide alone or in combination with additional coding sequences for a proteolytic cleavage site find utility in the expression of recombinant immunoglobulins or fragments thereof, proproteins, biologically active proteins and protein components of two- and three-hybrid systems, in any protein expression system, a number of which are known in the art and examples of which are described herein. One of skill in the art may easily adapt the vectors of the invention for use in any protein expression system.

When a compound, construct or composition is claimed, it should be understood that compounds, constructs and compositions known in the art including those taught in the references disclosed herein are not intended to be included. When a Markush group or other grouping is used herein, all individual members of the group and all combinations and subcombinations possible from within the group the group are intended to be individually included in the disclosure.

**Example 1. Expression of Immunoglobulins with Intein-Mediated Processing**

A strategy for the efficient expression of antibody molecules is via polyprotein expression, wherein an intein is located between the heavy and light chains, with modification of the intein sequence and/or junction sequences such that there is release of the component proteins without ligation of the N-terminal and C-terminal proteins. Within such constructs, there can be one copy of each of the relevant heavy and light chains, or the light chain can be duplicated, or there can be multiple copies of both heavy and light chains, provided that functional cleavage sequence is provided to promote separation of each immunoglobulin-derived protein within the polyprotein. The intein strategy can be employed more than once or a different proteolytic processing sequence or enzyme can be positioned at at least one terminus of an immunoglobulin derived protein.

The intein from Pyrococcus horikoshii has been incorporated into a construct as briefly described above and has been shown to successfully produce correctly processed and fully functional D2E7 antibody. Additional inteins tested are from Saccharomyces cerevisiae and Synechocystis spp. Strain PCC6803 and have been shown to produce secreted antibody via ELISA.

PCR Amplification and subcloning of the *Pyrococcus horikoshii* Pho Pol I intein:

The following oligonucleotides were used for the amplification of the p. horikoshii Pho Pol I intein (NCBI/protein accession # 059610, the GenBank accession # for the entire DNA Polymerase I DNA sequence is BA000001.2:1686361..1690068 as taken from the entire genomic sequence for *P*. *horikoshii*) using genomic DNA as template and Platinum Taq Hi Fidelity DNA Polymerase Supermix (Invitrogen, Carlsbad, CA). Genomic DNA was purchased from ATCC.

| | |
|---|---|
| *P*. *horikoshii* int-5' | AGCATTTTACCAGATGAATGGCTCCC (SEQ ID NO:52) |
| *P*. *horikoshii* int-3' | AACGAGGAAGTTCTCATTATCCTCAAC (SEQ ID NO:53) |

PCR was run according to the following program:

| Step | 1 | 2 | 3 | 4 | 5 | 6 | 7 | 8 |
|---|---|---|---|---|---|---|---|---|
| Temp | 94°C | 94°C | 55°C | 72°C | Go to step 2 (34 times) | 72°C | 4°C | End |
| Time | 2min | 1 min | 1 min | 2min | | 5min | hold | |

The PCR product was subcloned into pCR2.1-TOPO (Invitrogen) and the insert was sequenced and proven correct. At this time it was realized that there was sequence missing from the 3' end of the intein due to a printout error. The missing sequence was then filled in during subsequent PCR reactions to link the intein to heavy and light chain of D2E7.

Oligonucleotide primers were designed in order to generate the fusion of D2E7 Heavy Chain - Intein - D2E7 Light Chain. Primers were designed so that PCR product could be used as primers in subsequent PCR reactions.

| Item | Sequence | SEQ ID NO: |
|---|---|---|
| HC-intein-5' | AGCCTCTCCCTGTCTCCGGGTAAA- AGCATTTTACCAGATGAATG | 54 |
| Revised LC-intein-3' | GGGCGGGCACGCGCATGTCCAT- GTTGTGTGCGTAAAGTAGTC | 55 |
| HC-intein(1aa)-5' | AGCCTCTCCCTGTCTCCGGGTAAA-AAC- AGCATTTTACCAGATGAATG | 56 |
| Revised LC-intein(1aa)-3' | GGGCGGGCACGCGCATGTCCAT-ACT- GTTGTGTGCGTAAAGTAGTC | 57 |
| HC-intein(3aa)-5' | AGCCTCTCCCTGTCTCCGGGTAAA-TTAGCAAAC- AGCATTTTACCAGATGAATG | 58 |
| Revised LC-intein(3aa)-3' | GGGCGGGCACGCGCATGTCCAT-GTAATAACT- GTTGTGTGCGTAAAGTAGTC | 59 |
| HC-Srfl-5' | TGCCCGGCGCCACC- ATGGAGTTTGGGCTGAGCTGG | 60 |
| LC-BamHl-3' | TGGATCC-CCGCGGCCGCTCA- ACACTCTCCCCTGTTGAAGCTC | 61 |

Code for sequences to illustrate components:
**Heavy chain sequence (bold red)-**Light chain sequence (underlined)-P.horikoshi intein sequence (plain Arial)-**P.horikoshi extein sequence (bold underlined blue) Srfl recognition sequence GCCCGGGC (double underline) green** BamHI recognition sequence GGATCC (violet dashed underline) Stop codon, TCA (Times New Roman. Olive) **Kozak**

PCR Amplification and assembly of D2E7 Heavy Chain - Intein - D2E7 Light Chain fusion: Using the pCR2.1-TOPO-p.horikoshii intein clone generated above as template, PCR was performed using the primers *P. horikoshii* int-5' and revised P.hori-3' to restore the proper 3' end to the intein. The polymerase used was Pful DNA Polymerase to avoid the A-tailing that occurs with Platinum Taq.
PCR was run according to the following program:

| Step | 1 | 2 | 3 | 4 | 5 | 6 | 7 | 8 |
|---|---|---|---|---|---|---|---|---|
| Temp | 94°C | 94°C | 55°C | 72°C | Go to step 2 (34 times) | 72°C | 4°C | End |
| Time | 2min | 1 min | 1 min | 2min | | 5min | hold | |

The PCR amplification product was gel purified using the Qiaquick Gel Extraction kit (Qiagen, Valencia, CA). This product was used as template in the next set of reactions.

Three sets of PCR reactions were performed to generate intein coding sequences with varied numbers of extein residues 5' and 3' of the intein coding sequence. The extein codons come from the native DNA polymerase gene in *P*. *horik*oshii which this intein is naturally part of. Primers were used as follows: Set 1 introduces zero extein sequence (HC-intein-5' and Revised LC-intein-3'), Set 2 introduces one amino acid (3 base pairs) at both ends of the intein (HC-intein(1aa)-5' and Revised LC-intein(1aa)-3') and Set 3 introduces three amino acids (9 base pairs) at both ends of the intein (HC-intein(3aa)-5' and Revised LC-intein(3aa)-3').

The PCR program was the same as given above. PCR products were gel purified using the Qiaquick Gel Extraction kit (Qiagen). These products were used as primers in the next set of reactions.

Three sets of PCR reactions were performed to generate the fusion of D2E7 Heavy Chain to intein, with 0, 1 or 3 extein amino acids in between. The template for the reactions is the D2E7 Heavy Chain DNA. The PCR products described above were used as the 3' primers, respectively, and HC-Srfl-5' was used as the 5' primer in all reactions. Pful DNA Polymerase was used.
PCR was run according to the following program:

| Step | 1 | 2 | 3 | 4 | 5 | 6 | 7 | 8 |
|---|---|---|---|---|---|---|---|---|
| Temp | 94°C | 94°C | 50°C | 72°C | Go to step 2 (39 times) | 72°C | 4°C | End |
| Time | 2min | 1 min | 1 min | 3min | | 5min | hold | |

PCR product was gel purified using the Qiaquick Gel Extraction kit (Qiagen). This product was used as primers in the next set of reactions.

Three sets of PCR reactions were performed to generate the fusion of D2E7 Heavy Chain - intein to D2E7 Light Chain, with 0, 1 or 3 extein amino acids in between. The template for the reactions is the D2E7 Light Chain DNA. The PCR products described directly above were used as the 5' primers, respectively, and LC-BamHI-3' was used as the 3' primer in all reactions. Pful DNA Polymerase was used.
PCR was run according to the following program:

| Step | 1 | 2 | 3 | 4 | 5 | 6 | 7 | 8 |
|---|---|---|---|---|---|---|---|---|
| Temp | 94°C | 94°ºC | 55°C | 72°C | Go to step 2 (39 times) | 72°C | 4°C | End |
| Time | 2min | 1 min | 1 min | 5min | | 5min | hold | |

The PCR product produced was diffuse and sparse when run on a gel. These reactions were directly used as template in the final round of PCR, using HC-Srfl-5' and LC-BamHI-3' as primers. Pful DNA Polymerase was used. The same PCR program was used as set forth above. PCR products were gel purified using the Qiaquick Gel Extraction kit (Qiagen).

The purified PCR products described above were subcloned into pCR-BluntII-TOPO (Invitrogen) using the Zero Blunt TOPO PCR Cloning Kit (Invitrogen). Clones were sequenced to verify that the constructs exhibited the expected nucleic acid sequences. Correct clones were found for each type of product. The D2E7 Heavy Chain - intein - D2E7 Light Chain cassette was excised from pCR-Bluntll-TOPO using Srfl and NotI and subcloned into pTT3 restricted with the same enzymes and gel purified.

Three Expression Constructs for D2E7 Heavy Chain - intein - D2E7 Light Chain, utilizing the *P. horikoshii* intein were designed: pTT3-HcintLC-p.hori (See Fig. 14 for plasmid map); pTT3-HcintLC1aa-p.hori; and pTT3-HcintLC3aa-p.hori.

Table 10A. Nucleotide sequence of pTT3-HcintLC-p.hori (SEQ ID NO:62)

| |
|---|
| 5'- gcggccgctcgaggccggcaaggccggatcccccgacctcgacctctggctaataaaggaaatttattttcattgcaa tagtgtgttggaattttttgtgtctctcactcggaaggacatatgggagggcaaatcatttggtcgagatccctcggagatc tctagctagaggatcgatccccgccccggacgaactaaacctgactacgacatctctgccccttcttcgcggggcagt gcatgtaatcccttcagttggttggtacaacttgccaactgggccctgttccacatgtgacacggggggggaccaaac acaaaggggttctctgactgtagttgacatccttataaatggatgtgcacatttgccaacactgagtggctttcatcctgg agcagactttgcagtctgtggactgcaacacaacattgcctttatgtgtaactcttggctgaagctcttacaccaatgctg ggggacatgtacctcccaggggcccaggaagactacgggaggctacaccaacgtcaatcagaggggcctgtgta gctaccgataagcggaccctcaagagggcattagcaatagtgtttataaggcccccttgttaaccctaaacgggtagc atatgcttcccgggtagtagtatatactatccagactaaccctaattcaatagcatatgttacccaacgggaagcatatg |
| ctatcgaattagggttagtaaaagggtcctaaggaacagcgatatctcccaccccatgagctgtcacggttttatttaca tggggtcaggattccacgagggtagtgaaccattttagtcacaagggcagtggctgaagatcaaggagcgggcagt gaactctcctgaatcttcgcctgcttcttcattctccttcgtttagctaatagaataactgctgagttgtgaacagtaaggtgt atgtgaggtgctcgaaaacaaggtttcaggtgacgcccccagaataaaatttggacggggggttcagtggtggcatt gtgctatgacaccaatataaccctcacaaaccccttgggcaataaatactagtgtaggaatgaaacattctgaatatct ttaacaatagaaatccatggggtggggacaagccgtaaagactggatgtccatctcacacgaatttatggctatgggc aacacataatcctagtgcaatatgatactggggttattaagatgtgtcccaggcagggaccaagacaggtgaaccat gttgttacactctatttgtaacaaggggaaagagagtggacgccgacagcagcggactccactggttgtctctaacac ccccgaaaattaaacggggctccacgccaatggggcccataaacaaagacaagtggccactcttttttttgaaattgt ggagtgggggcacgcgtcagcccccacacgccgccctgcggttttggactgtaaaataagggtgtaataacttggct gattgtaaccccgctaaccactgcggtcaaaccacttgcccacaaaaccactaatggcaccccggggaatacctgc ataagtaggtgggcgggccaagataggggcgcgattgctgcgatctggaggacaaattacacacacttgcgcctga gcgccaagcacagggttgttggtcctcatattcacgaggtcgctgagagcacggtgggctaatgttgccatgggtagc atatactacccaaatatctggatagcatatgctatcctaatctatatctgggtagcataggctatcctaatctatatctgggt agcatatgctatcctaatctatatctgggtagtatatgctatcctaatttatatctgggtagcataggctatcctaatctatatc tgggtagcatatgctatcctaatctatatctgggtagtatatgctatcctaatctgtatccgggtagcatatgctatcctaata gagattagggtagtatatgctatcctaatttatatctgggtagcatatactacccaaatatctggatagcatatgctatccta atctatatctgggtagcatatgctatcctaatctatatctgggtagcataggctatcctaatctatatctgggtagcatatgct atcctaatctatatctgggtagtatatgctatcctaatttatatctgggtagcataggctatcctaatctatatctgggtagcat atgctatcctaatctatatctgggtagtatatgctatcctaatctgtatccgggtagcatatgctatcctcatgataagctgtc aaacatgagaattttcttgaagacgaaagggcctcgtgatacgcctatttttataggttaatgtcatgataataatggtttct tagacgtcaggtggcacttttcggggaaatgtgcgcggaacccctatttgtttatttttctaaatacattcaaatatgtatcc gctcatgagacaataaccctgataaatgcttcaataatattgaaaaaggaagagtatgagtattcaacatttccgtgtc gcccttattcccttttttgcggcattttgccttcctgtttttgctcacccagaaacgctggtgaaagtaaaagatgctgaagat cagttgggtgcacgagtgggttacatcgaactggatctcaacagcggtaagatccttgagagttttcgccccgaagaa cgttttccaatgatgagcacttttaaagttctgctatgtggcgcggtattatcccgtgttgacgccgggcaagagcaactc ggtcgccgcatacactattctcagaatgacttggttgagtactcaccagtcacagaaaagcatcttacggatggcatga cagtaagagaattatgcagtgctgccataaccatgagtgataacactgcggccaacttacttctgacaacgatcgga ggaccgaaggagctaaccgcttttttgcacaacatgggggatcatgtaactcgccttgatcgttgggaaccggagctg aatgaagccataccaaacgacgagcgtgacaccacgatgcctgcagcaatggcaacaacgttgcgcaaactatta actggcgaactacttactctagcttcccggcaacaattaatagactggatggaggcggataaagttgcaggaccactt ctgcgctcggcccttccggctggctggtttattgctgataaatctggagccggtgagcgtgggtctcgcggtatcattgca gcactggggccagatggtaagccctcccgtatcgtagttatctacacgacggggagtcaggcaactatggatgaacg aaatagacagatcgctgagataggtgcctcactgattaagcattggtaactgtcagaccaagtttactcatatatacttta gattgatttaaaacttcatttttaatttaaaaggatctaggtgaagatcctttttgataatctcatgaccaaaatcccttaacg tgagttttcgttccactgagcgtcagaccccgtagaaaagatcaaaggatcttcttgagatcctttttttctgcgcgtaatct gctgcttgcaaacaaaaaaaccaccgctaccagcggtggtttgtttgccggatcaagagctaccaactctttttccgaa ggtaactggcttcagcagagcgcagataccaaatactgttcttctagtgtagccgtagttaggccaccacttcaagaac tctgtagcaccgcctacatacctcgctctgctaatcctgttaccagtggctgctgccagtggcgataagtcgtgtcttacc gggttggactcaagacgatagttaccggataaggcgcagcggtcgggctgaacggggggttcgtgcacacagccc agcttggagcgaacgacctacaccgaactgagatacctacagcgtgagctatgagaaagcgccacgcttcccgaa gggagaaaggcggacaggtatccggtaagcggcagggtcggaacaggagagcgcacgagggagcttccaggg ggaaacgcctggtatctttatagtcctgtcgggtttcgccacctctgacttgagcgtcgatttttgtgatgctcgtcagggg gcggagcctatggaaaaacgccagcaacgcggcctttttacggttcctggccttttgctggccttttgctcacatgttcttc ctgcgttatcccctgattctgtggataaccgtattaccgcctttgagtgagctgataccgctcgccgcagccgaacgacc gagcgcagcgagtcagtgagcgaggaagcggaagagcgcccaatacgcaaaccgcctctccccgcgcgttggc cgattcattaatgcagctggcacgacaggtttcccgactggaaagcgggcagtgagcgcaacgcaattaatgtgagt tagctcactcattaggcaccccaggctttacactttatgcttccggctcgtatgttgtgtggaattgtgagcggataacaatt tcacacaggaaacagctatgaccatgattacgccaagctctagctagaggtcgaccaattctcatgtttgacagcttat catcgcagatccgggcaacgttgttgccattgctgcaggcgcagaactggtaggtatggaagatctatacattgaatc |
| aatattggcaattagccatattagtcattggttatatagcataaatcaatattggctattggccattgcatacgttgtatctat atcataatatgtacatttatattggctcatgtccaatatgaccgccatgttgacattgattattgactagttattaatagtaatc aattacggggtcattagttcatagcccatatatggagttccgcgttacataacttacggtaaatggcccgcctggctgac cgcccaacgacccccgcccattgacgtcaataatgacgtatgttcccatagtaacgccaatagggactttccattgac gtcaatgggtggagtatttacggtaaactgcccacttggcagtacatcaagtgtatcatatgccaagtccgccccctatt gacgtcaatgacggtaaatggcccgcctggcattatgcccagtacatgaccttacgggactttcctacttggcagtaca tctacgtattagtcatcgctattaccatggtgatgcggttttggcagtacaccaatgggcgtggatagcggtttgactcac ggggatttccaagtctccaccccattgacgtcaatgggagtttgttttggcaccaaaatcaacgggactttccaaaatgt cgtaataaccccgccccgttgacgcaaatgggcggtaggcgtgtacggtgggaggtctatataagcagagctcgttt agtgaaccgtcagatcctcactcttccgcatcgctgtctgcgagggccagctgttgggctcgcggttgaggacaaac tcttcgcggtctttccagtactcttggatcggaaacccgtcggcctccgaacggtactccgccaccgagggacctgag cgagtccgcatcgaccggatcggaaaacctctcgagaaaggcgtctaaccagtcacagtcgcaaggtaggctgag caccgtggcgggcggcagcgggtggcggtcggggttgtttctggcggaggtgctgctgatgatgtaattaaagtaggc ggtcttgagacggcggatggtcgaggtgaggtgtggcaggcttgagatccagctgttggggtgagtactccctctcaa aagcgggcattacttctgcgctaagattgtcagtttccaaaaacgaggaggatttgatattcacctggcccgatctggcc atacacttgagtgacaatgacatccactttgcctttctctccacaggtgtccactcccaggtccaagtttgggcgccacc atggagtttgggctgagctggctttttcttgtcgcgattttaaaaggtgtccagtgt- gaggtgcagctggtggagtctgggggaggcttggtacagcccggcaggtccctgagactctcctgtgcggcctctgg attcacctttgatgattatgccatgcactgggtccggcaagctccagggaagggcctggaatgggtctcagctatcactt ggaatagtggtcacatagactatgcggactctgtggagggccgattcaccatctccagagacaacgccaagaactc cctgtatctgcaaatgaacagtctgagagctgaggatacggccgtatattactgtgcgaaagtctcgtaccttagcacc gcgtcctcccttgactattggggccaaggtaccctggtcaccgtctcgagtgcgtcgaccaagggcccatcggtcttcc ccctggcaccctcctccaagagcacctctgggggcacagcggccctgggctgcctggtcaaggactacttccccga accggtgacggtgtcgtggaactcaggcgccctgaccagcggcgtgcacaccttcccggctgtcctacagtcctcag gactctactccctcagcagcgtggtgaccgtgccctccagcagcttgggcacccagacctacatctgcaacgtgaatc acaagcccagcaacaccaaggtggacaagaaagttgagcccaaatcttgtgacaaaactcacacatgcccaccg tgcccagcacctgaactcctggggggaccgtcagtcttcctcttccccccaaaacccaaggacaccctcatgatctcc cggacccctgaggtcacatgcgtggtggtggacgtgagccacgaagaccctgaggtcaagttcaactggtacgtgg acggcgtggaggtgcataatgccaagacaaagccgcgggaggagcagtacaacagcacgtaccgtgtggtcag cgtcctcaccgtcctgcaccaggactggctgaatggcaaggagtacaagtgcaaggtctccaacaaagccctccca gcccccatcgagaaaaccatctccaaagccaaagggcagccccgagaaccacaggtgtacaccctgcccccatc ccgggatgagctgaccaagaaccaggtcagcctgacctgcctggtcaaaggcttctatcccagcgacatcgccgtg gagtgggagagcaatgggcagccggagaacaactacaagaccacgcctcccgtgctggactccgacggctccttc ttcctctacagcaagctcaccgtggacaagagcaggtggcagcaggggaacgtcttctcatgctccgtgatgcatga ggctctgcacaaccactacacgcagaagagcctctccctgtctccgggtaaa- agcattttaccagatgaatggctcccaattgttgaaaatgaaaaagttcgattcgtaaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat gatcccagttaaaggtagaaagaatttcttcaaagggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct |
| gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaac- atggacatgcgcgtgcccgcccagctgctgggcctgctgctgctgtggttccccggctcgcgatgcgacatccagatg acccagtctccatcctccctgtctgcatctgtaggggacagagtcaccatcacttgtcgggcaagtcagggcatcaga aattacttagcctggtatcagcaaaaaccagggaaagcccctaagctcctgatctatgctgcatccactttgcaatcag gggtcccatctcggttcagtggcagtggatctgggacagatttcactctcaccatcagcagcctacagcctgaagatgt tgcaacttattactgtcaaaggtataaccgtgcaccgtatacttttggccaggggaccaaggtggaaatcaaacgtac ggtggctgcaccatctgtcttcatcttcccgccatctgatgagcagttgaaatctggaactgcctctgttgtgtgcctgctg aataacttctatcccagagaggccaaagtacagtggaaggtggataacgccctccaatcgggtaactcccaggaga gtgtcacagagcaggacagcaaggacagcacctacagcctcagcagcaccctgacgctgagcaaagcagacta cgagaaacacaaagtctacgcctgcgaagtcacccatcagggcctgagctcgcccgtcacaaagagcttcaacag gggagagtgt -3' |

Table 10B. Amino Acid Sequence of the open reading frame in pTT3-HcintLC-p.hori (SEQ ID NO:63)

| |
|---|
| |

Text/font symbol code for sequences:
pTT3 Vector -Heavy Chain - Intein - Light Chain

In the following 2 constructs, the only difference from the construct above is the inclusion of extein sequences native to *P. horikoshii* (underlined). The sequences shown are from the end of the D2E7 heavy chain coding region (last 9 base pairs as shown in red) to the 5' end of the D2E7 light chain coding region (first 9 base pairs as shown in pink, on a separate line)

Table 11 A. pTT3-HcintLC1aa-p.hori partial coding sequence (SEQ ID NO:64)

| |
|---|
| 5'-ccgggtaaa- aacagcattttaccagatgaatggctcccaattgttgaaaatgaaaaagttcgattcgtaaaattggagacttcatag atagggagattgaggaaaacgctgagagagtgaagagggatggtgaaactgaaattctagaggttaaagatcttaa agccctttccttcaatagagaaacaaaaaagagcgagctcaagaaggtaaaggccctaattagacaccgctattca gggaaggtttacagcattaaactaaagtcagggagaaggatcaaaataacctcaggtcatagtctgttctcagtaaa aaatggaaagctagttaaggtcaggggagatgaactcaagcctggtgatctcgttgtcgttccaggaaggttaaaact tccagaaagcaagcaagtgctaaatctcgttgaactactcctgaaattacccgaagaggagacatcgaacatcgta atgatgatcccagttaaaggtagaaagaatttcttcaaaggatgctcaaaacattatactggatcttcggggaggga gaaaggccaagaaccgcagggcgctatctcaagcatcttgaaagattaggatacgttaagctcaagagaagaggc tgtgaagttctcgactgggagtcacttaagaggtacaggaagctttacgagaccctcattaagaacctgaaatataac ggtaatagcagggcatacatggttgaatttaactctctcagggatgtagtgagcttaatgccaatagaagaacttaagg agtggataattggagaacctaggggtcctaagataggtaccttcattgatgtagatgattcatttgcaaagctcctaggtt actacataagtagcggagatgtagagaaagatagggtgaagttccacagtaaagatcaaaacgttctcgaggatat agcgaaacttgccgagaagttatttggaaaggtgaggagaggaagaggatatattgaggtatcagggaaaattagc catgccatatttagagttttagcggaaggtaagagaattccagagttcatcttcacatccccaatggatattaaggtagc cttccttaagggactcaacggtaatgctgaagaattaacgttctccactaagagtgagctattagttaaccagcttatcct tctcctgaactccattggagtttcggatataaagattgaacatgagaaaggggtttacagagtttacataaataagaag gaatcctccaatggggatatagtacttgatagcgtcgaatctatcgaagttgaaaaatacgagggctacgtttatgatct aagtgttgaggataatgagaacttcctcgttggcttcggactactttacgcacacaacagt- atggacatg -3' |

Table 11 B. pTT3-HcintLC1 aa-p.hori partial amino acid sequence showing 4 amino acids upstream of the heavy chain and four amino acids downstream of the intein (SEQ ID NO:65)

| |
|---|
| Pgknsilpdewlpivenekvrfvkigdfidreieenaervkrdgeteilevkdlkalsfnretkkselkkvkalirhrysgkv ysiklksgrrikitsghslfsvkngklvkvrgdelkpgdlvvvpgrlklpeskqvlnlvelllklpeeetsnivmmipvkgrk nffkgmlktlywifgegerprtagrylkhlerlgyvklkrrgcevldweslkryrklyetliknlkyngnsraymvefnslrdv vslmpieelkewiigeprgpkigtfidvddsfakllgyyissgdvekdrvkfhskdqnvlediaklaeklfgkvrrgrgyie vsgkishaifrvlaegkripefiftspmdikvaflkglngnaeeltfstksellvnqlilllnsigvsdikiehekgvyrvyinkk essngdivldsvesievekyegyvydlsvednenflvgfgllyahn-s-mdm |

Heavy Chain 3' sequence-Intein-Extein-Light Chain 5' sequence

Table 12A. pTT3-HcintLC3aa-p.hori partial coding sequence (SEQ ID NO:66)

| |
|---|
| 5'- ccgggtaaa-ttagcaaac- agcattttaccagatgaatggctcccaattgttgaaaatgaaaaagttcgattcgtaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat gatcccagttaaaggtagaaagaatttcttcaaaggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg |
| ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaac-agttattac-atggacatg-3' |

Table 12B. pTT3-HcintLC3aa-p.hori partial amino acid sequence showing intein and flanking sequences (SEQ ID NO:67)

| |
|---|
| Pgk-lan- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyahn-syy-mdm |

Heavy Chain 3' sequence-Intein-Extein-Light Chain 5' sequence

Primers used for constructs A, B, E, H, I, J, K, and L were:
YKF1:GGACTACTTTACGCAGCCAACATGGACATGC (SEQ ID NO:68)
YKR1:GCATGTCCATGTTGGCTGCGTAAAGTAGTCC (SEQ ID NO:69)
YKF2:GGACTACTTTACGCAGCCAACAGTATGGACATGC (SEQ ID NO:70)
YKR2:GCATGTCCATACTGTTGGCTGCGTAAAGTAGTCC (SEQ ID NO:71)
YKF3:GGTGAGGAGAGGAAGAGG (SEQ ID NO:72)
YKR3:CCAGAGGTCGAGGTCG (SEQ ID NO:73)
YKF4:CGGCGTGGAGGTGC (SEQ ID NO:74)
YKR4:CAACAATTGGGAGCCATTCATCTGGTAAAATGGTTTTACCCGGAG (SEQ ID NO:75)
YKF5: CCGCCCAGCTGCTGGGCGACGAGTGGTTCCCCGGCTCGCG (SEQ ID NO:76)
YKR5:Cgcgagccggggaaccactcgtcgcccagcagctgggcgg (SEQ ID NO:77)
YKF6: tgagcggccgctcga (SEQ ID NO:78)
YKR6: gttgtgtgcgtaaag (SEQ ID NO:79)
YKF7: agcattttaccagat (SEQ ID NO:80)
YKR7:ggtggcgcccaaact (SEQ ID NO:81)
YKF8: ctttacgcacacaacatggacatgcgcgtg (SEQ ID NO:82)
YKR8:tcgagcggccgctcaacactctcccct (SEQ ID NO:83)
YKF9: agtttgggcgccaccatggagtttgggctg (SEQ ID NO:84)
YKR9:atctggtaaaatgcttttacccggagacag (SEQ ID NO:85)
YKF10: agtttgggcgccaccatggacatgcgcgtg (SEQ ID NO:86)
YKR10: atctggtaaaatgctacactctcccctgttg (SEQ ID NO:87)
YKF11: ctttacgcacacaacatggagtttgggctg (SEQ ID NO:88)
YKR11: tcgagcggccgctcatttacccggagacag (SEQ ID NO:89)
YKF12: cgccaagctctagc (SEQ ID NO:90)
YKR12: ggtcgaggtcgggg (SEQ ID NO:91)
YKF13: acatgcgcgtgcccgcccagtggttccccggctcgcgatg (SEQ ID NO:92)
YKR13:catcgcgagccggggaaccactgggcgggcacgcgcatgt (SEQ ID NO:93)
YKF14: ctttacgcacacaacgacatccagatgacc (SEQ ID NO:94)
YKR14:ggtcatctggatgtcgttgtgtgcgtaaag (SEQ ID NO:95)
YKF15: tggttccccggctcgGgaGgcgacatccagatgacc (SEQ ID NO:96)
YKR15: ggtcatctggatgtcgcctcccgagccggggaacca (SEQ ID NO:97)

To prepare Construct A, plasmid pTT3 HC-int-LC P.hori was used as template 2 and overlapping DNA fragments were amplified using mutagenesis primer YKF1 and primer YKR3, and mutagenesis primer YKR1 with primer YKF3, respectively. A DNA fragment linking the above 2 fragments was generated by PCR amplification using the mixture of the above 2 PCR fragments as template, and primers YKF3 and YKR3. This PCR fragment is then cut with restriction enzymes EcoR I and Not I, and cloned into pTT3 HC-int-LC P.hori cut with the same restriction enzymes.

Construct B was generated in a similar manner as for construct A, except that mutagenesis primers YKF2 and YKR2 were used in place of YKF1 and YKR1, and plasmid pTT3 HC-int-LC-1aa P.hori was used as the PCR template in the place of plasmid pTT3 HC-int-LC P.hori, and pTT3 HC-int-LC P.hori vector was used as the backbone for cloning.

To prepare Construct E, a DNA fragment was amplified using plasmid pTT3 HC-int-LC-1 aa P.hori as template, and primer YKF4 and mutagenesis primer YKR4. This PCR fragment was cut with Sac II and Mfe I, and cloned into pTT3 HC-int-LC P.hori cut with the same restriction enzymes.

For Construct H, pTT3 HC-int-LC P.hori was used as template 2, and overlapping fragments were amplified using mutagenesis primer YKF5 and primer YKR3 for one fragment and primer F3 and mutagenesis primer R5 for the other. A second round of PCR amplification was carried out using the above 2 fragments as templates and primers YKF3 and YKR3. This fragment was digested with restriction enzymes EcoR I and Not I, and cloned into pTT3 HC-int-LC P.hori cut with the same enzymes.

To prepare Construct J, pTT3 HC-int-LC P.hori was used as template 2, and overlapping fragments were amplified using mutagenesis primer YKF13 and primer YKR3 for one fragment and primer F3 and mutagenesis primer R13 for the other. A second round of PCR amplification was carried out using the above 2 fragments as templates and primers YKF3 and YKR3. This fragment was cut with restriction enzymes EcoR I and Not I and cloned into pTT3 HC-int-LC P.hori cut with the same enzymes.

For Construct K, pTT3 HC-int-LC P.hori served as template 2. Overlapping fragments were amplified using mutagenesis primer YKF14 and primer YKR3 for one fragment and primer F3 and mutagenesis primer R14 for the other. A second round of PCR amplification was carried out using the above 2 fragments as templates and primers YKF3 and YKR3. This fragment was digested with restriction enzymes EcoR I and Not I, and cloned into pTT3 HC-int-LC P.hori cut with the same enzymes.

To make Constructs L, Using pTT3 HC-int-LC P.hori was used as template 2, and overlapping fragments were amplified using mutagenesis primer YKF15 and primer YKR3 for one fragment and primer F3 and mutagenesis primer R15 for the other. A second round of PCR amplification was carried out using the above 2 fragments as templates and primers YKF3 and YKR3. This fragment was digested with restriction enzymes EcoR I and Not I, and cloned into pTT3 HC-int-LC P.hori cut with the same enzymes.

The nucleotide sequences of all constructs were verified. All constructs have the same sequence as pTT3 HC-int-LC P.hori except for the sequences between the last codons of the D2E7 heavy chain (encoding PGK) and the first codons of the D2E7 light chain mature sequence (encoding DIQ). Sequences in this region, which include wt or mutant intein in conjunction with wt or mutant light chain signal sequence, are provided for all the constructs as below.

Table 13A. Partial coding sequence of construct A (SEQ ID NO:98)

| |
|---|
| Ccgggtaaa- agcattttaccagatgaatggctcccaattgttgaaaatgaaaaagttcgattcgtaaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat |
| gatcccagttaaaggtagaaagaatttcttcaaaggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcagccaacatggacatgcgcgtgcccgcccagct gctgggcctgctgctgctgtggttccccggctcgcgatgc-gacatccag |

Table 13B. Partial amino acid sequence showing intein and flanking sequences in construct A (SEQ ID NO:99)

| |
|---|
| Pgk- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyaanmdmrvpaqllglllwfpgsrc-diq |

Table 14A. Partial coding sequence in construct B (SEQ ID NO:100)

| |
|---|
| Ccgggtaaa- agcattttaccagatgaatggctcccaattgttgaaaatgaaaaagttcgattcgtaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat gatcccagttaaaggtagaaagaatttcttcaaagggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcagccaacagtatggacatgcgcgtgcccgccca gctgctgggcctgctgctgctgtggttccccggctcgcgatgc-gacatccag |

Table 14B. Partial amino acid sequence in construct B (SEQ ID NO:101)

| |
|---|
| Pgk- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyaansmdmrvpaqllglllwfpgsrc-diq |

Table 15A. Partial coding sequence in construct E (SEQ ID NO:102)

| |
|---|
| Ccgggtaaa- accattttaccagatgaatggctcccaattgttgaaaatgaaaaagttcgattcgtaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat gatcccagttaaaggtagaaagaatttcttcaaagggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaacagtatggacatgcgcgtgcccgccca gctgctgggcctgctgctgctgtggttccccggctcgcgatgc-gacatccag |

Table 15B. Partial amino acid sequence in construct E (SEQ ID NO:103)

| |
|---|
| Pgk- tilpdewlpivenekvrfvkigdfidreieenaervkrdgeteilevkdlkalsfnretkkselkkvkalirhrysgkvysiklk sgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyahnsmdmrvpaqllglllwfpgsrc-diq |

Table 16A. Partial coding sequence in construct H (SEQ ID NO:104)

| |
|---|
| Ccgggtaaa- agcattttaccagatgaatggctcccaattgttgaaaatgaaaaagttcgattcgtaaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca |
| gaaagcaagcaagtgctaaatctcgttgaactactcctgaaattacccgaagaggagacatcgaacatcgtaatgat gatcccagttaaaggtagaaagaatttcttcaaaggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaacatggacatgcgcgtgcccgcccagct gctgggcgacgagtggttccccggctcgcgatgc-gacatccag |

Table 16B. Partial amino acid sequence in construct H (SEQ ID NO:105)

| |
|---|
| Pgk- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyahnmdmrvpaqllgdewfpgsrc-diq |

Table 17A. Partial coding sequence in construct J (SEQ ID NO:106)

| |
|---|
| Ccgggtaaa- agcattttaccagatgaatggctcccaattgttgaaaatgaaaaagttcgattcgtaaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat gatcccagttaaaggtagaaagaatttcttcaaagggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaacatggacatgcgcgtgcccgcccagtg gttccccggctcgcgatgc-gacatccag |

Table 17B. Partial amino acid sequence in construct J (SEQ ID NO:107)

| |
|---|
| Pgk- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyahnmdmrvpaqwfpgsrc-diq |

Table 18A. Partial coding sequence in construct K (SEQ ID NO:108)

| |
|---|
| Ccgggtaaa- agcatttaccagatgaatggctcccaattgttgaaaatgaaaaagttcgattcgtaaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca gaaagcaagcaagtgctaaatctcgttgaactactcctgaaattacccgaagaggagacatcgaacatcgtaatgat gatcccagttaaaggtagaaagaatttcttcaaagggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaac-gacatccag |

Table 18B. Partial amino acid sequence in construct K (SEQ ID NO:109)

| |
|---|
| Pgk- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyahn-diq |

Table 19A. Partial coding sequence in construct L (SEQ ID NO:110)

| |
|---|
| Ccgggtaaa- agcattttaccagatgaatggctcccaattgttgaaaatgaaaaagttcgattcgtaaaattggagacttcatagatag ggagattgaggaaaacgctgagagagtgaagagggatggtgaaactgaaattctagaggttaaagatcttaaagc cctttccttcaatagagaaacaaaaaagagcgagctcaagaaggtaaaggccctaattagacaccgctattcaggg aaggtttacagcattaaactaaagtcagggagaaggatcaaaataacctcaggtcatagtctgttctcagtaaaaaat ggaaagctagttaaggtcaggggagatgaactcaagcctggtgatctcgttgtcgttccaggaaggttaaaacttcca |
| gaaagcaagcaagtgctaaatctcgttgaactactcctgaaattacccgaagaggagacatcgaacatcgtaatgat gatcccagttaaaggtagaaagaatttcttcaaaggatgctcaaaacattatactggatcttcggggagggagaaa ggccaagaaccgcagggcgctatctcaagcatcttgaaagattaggatacgttaagctcaagagaagaggctgtga agttctcgactgggagtcacttaagaggtacaggaagctttacgagaccctcattaagaacctgaaatataacggtaa tagcagggcatacatggttgaatttaactctctcagggatgtagtgagcttaatgccaatagaagaacttaaggagtgg ataattggagaacctaggggtcctaagataggtaccttcattgatgtagatgattcatttgcaaagctcctaggttactac ataagtagcggagatgtagagaaagatagggtgaagttccacagtaaagatcaaaacgttctcgaggatatagcga aacttgccgagaagttatttggaaaggtgaggagaggaagaggatatattgaggtatcagggaaaattagccatgc catatttagagttttagcggaaggtaagagaattccagagttcatcttcacatccccaatggatattaaggtagccttcctt aagggactcaacggtaatgctgaagaattaacgttctccactaagagtgagctattagttaaccagcttatccttctcct gaactccattggagtttcggatataaagattgaacatgagaaaggggtttacagagtttacataaataagaaggaatc ctccaatggggatatagtacttgatagcgtcgaatctatcgaagttgaaaaatacgagggctacgtttatgatctaagtg ttgaggataatgagaacttcctcgttggcttcggactactttacgcacacaacatggacatgcgcgtgcccgcccagct gctgggcctgctgctgctgtggttccccggctcgggaggc-gacatccag |

Table 19B. Partial amino acid sequence in construct L (SEQ ID NO:111)

| |
|---|
| Pgk- silpdewlpivenekvrfvkigdfidreieenaervkrdgeteilevkdlkalsfnretkkselkkvkalirhrysgkvysikl ksgrrikitsghslfsvkngklvkvrgdelkpgdlvvvpgrlklpeskqvlnlvelllklpeeetsnivmmipvkgrknffkg mlktlywifgegerprtagrylkhlerlgyvklkrrgcevldweslkryrklyetliknlkyngnsraymvefnslrdvvslm pieelkewiigeprgpkigtfidvddsfakllgyyissgdvekdrvkfhskdqnvlediaklaeklfgkvrrgrgyievsgki shaifrvlaegkripefiftspmdikvaflkglngnaeeltfstksellvnqlilllnsigvsdikiehekgvyrvyinkkessng divldsvesievekyegyvydlsvednenflvgfgllyahnmdmrvpaqllglllwfpgsgg-diq |

Heavy Chain 3' sequence-Intein + light chain signal peptide sequence-Light Chain mature sequence

The following oligonucleotides were used for the amplification of the *Saccharomyces cerevisiae* VMA intein (GenBank accession #AB093499) using genomic DNA as template and Pfu-I Hi Fidelity DNA Polymerase (Stratagene). Genomic DNA was prepared from a culture of *Saccharomyces cerevisiae* using the Yeast-Geno-DNA-Template kit (G Biosciences, cat. #786-134).

| | |
|---|---|
| Sce VMA intein 5': | TGCTTTGCCAAGGGTACCAATGTTTT (SEQ ID NO:112) |
| Sce VMA intein 3' | ATTATGGACGACAACCTGGTTGGCAA (SEQ ID NO:113) |

PCR run according to the following program:

| Step | 1 | 2 | 3 | 4 | 5 | 6 | 7 | 8 |
|---|---|---|---|---|---|---|---|---|
| Temp | 94ºC | 94ºC | 55ºC | 72ºC | Go to step 2 (39 times) | 72ºC | 4ºC | End |
| Time | 2min | 1 min | 1 min | 2min | | 5min | hold | |

The PCR product was used as template using the following pairs of primers to produce 0aa, 1 aa or 3aa versions of the intein as for the *P. horikoshii intein* constructs. Pfu-I Hi Fidelity DNA Polymerase (Stratagene) used. 
Sce-5'-Sap
CCGCAGAAGAGCCTCTCCCTGTCTCCGGGTAAATGCTTTGCCAAGGGTA
CCAATGTTTT (SEQ ID NO:114)
 
Sce-5'-1aa-Sap
CCGCAGAAGAGCCTCTCCCTGTCTCCGGGTAAAGGGTGCTTTGCCAAGG
GTACCAATGTTTT (SEQ ID NO:115)
 
Sce-5'-3aa-Sap
CCGCAGAAGAGCCTCTCCCTGTCTCCGGGTAAATATGTCGGGTGCTTTG
CCAAGGGTACCAATGTTTT (SEQ ID NO:116)
 
Sce-3'-Van911
CAGCAGGCCCAGCAGCTGGGCGGGCACGCGCATGTCCATATTATGGAC
GACAACCTGGTTGGCAA (SEQ ID NO:117)
 
Sce-3'-1aa-Van911
CAGCAGGCCCAGCAGCTGGGCGGGCACGCGCATGTCCATGCAATTATG
GACGACAACCTGGTTGGCAA (SEQ ID NO:118)
 
Sce-3'-3aa-Van911
CAGCAGGCCCAGCAGCTGGGCGGGCACGCGCATGTCCATTTCTCCGCA
ATTATGGACGACAACCTGGTTGGCAA (SEQ ID NO:119)
 PCR was run using the same program provided above. The PCR product from each reaction type was subcloned into pCR-BluntII-TOPO (Invitrogen) and the insert of each type was sequenced and proven correct.

Oligonucleotide primers were designed in order to generate the fusion of D2E7 Heavy Chain - Intein - D2E7 Light Chain by way of homologous recombination into the pTT3-HcintLC p. horikoshii construct in *E. coli.* By engineering a 40 base pair overhang between PCR generated vector (containing pTT3 vector, heavy chain and light chain regions but not the *P. horikoshii* intein) and the VMA intein insert, the two DNAs can be mixed and transformed into *E. coli* without the benefit of ligation, resulting in *E. coli* homologous recombination of the two fragments into pTT3-HC-VMAint-LC in the 0aa, 1 aa and 3aa versions.
VMA homologous recombination primers:
VMA-HR5': CCACTACACGCAGAAGAGCCTCTCCCTGTCTCCGGGTAAA (SEQ ID NO:120)
VMA-HR3': GCAGCAGGCCCAGCAGCTGGGCGGGCACGCGCATGTCCAT (SEQ ID NO:121)
pTT3-HcintLC homologous recombination primers:
pTT3int-HR5': ATGGACATGCGCGTGCCCGCCCAGCTGCTGGGCCTGCTGC (SEQ ID NO:122)
pTT3int-HR3': TTTACCCGGAGACAGGGAGAGGCTCTTCTGCGTGTAGTGGT (SEQ ID NO:123)
PCR for intein was run on the following program: Pfu-I Hi Fidelity DNA Polymerase (Stratagene) used.

| Step | 1 | 2 | 3 | 4 | 5 | 6 | 7 | 8 |
|---|---|---|---|---|---|---|---|---|
| Temp | 94°C | 94°C | 60°C | 72°C | Go to step 2 (34 times) | 72°C | 4°C | End |
| Time | 2min | 1 min | 1 min | 1.5min | | | 5min | hold |

PCR for the vector was run per the following program: Platinum Taq Hi Fidelity Supermix (Invitrogen) used.

| Step | 1 | 2 | 3 | 4 | 5 | 6 | 7 | 8 |
|---|---|---|---|---|---|---|---|---|
| Temp | 94°C | 94°C | 60°C | 68°C | Go to step 2 (24 times) | 68°C | 4°C | End |
| Time | 2min | 30sec | 30sec | 10min | | 5min | hold | |

To effect homologous recombination of the VMA intein into pTT3-HcintLC the following strategy was employed. PCR products were gel purified, and each was eluted into 50 µl elution buffer using a Qiaquick Gel Extraction kit (Qiagen). 3 µl of the vector PCR product was mixed in an eppendorf tube, and 3 µl of the desired VMA intein PCR product was added (either 0aa, 1 aa or 3aa in separate tubes). Each mixture was transformed into *E. coli,* and the cells were then plated onto LB + Ampicillin plates and incubated at 37C overnight. Colonies were grown to 2ml cultures, plasmid DNA was prepared using Wizard Prep Kits (Promega) and analyzed by restriction endonuclease digestion and agarose gel electrophoresis. Clones that produced the correct restriction pattern were analyzed with respect to DNA sequence.

Three Expression Constructs for D2E7 Heavy Chain - intein - D2E7 Light Chain, utilizing the *S. cerevisiae* VMA intein, were created: pTT3-Hc-VMAint-LC-0aa; pTT3-Hc-VMAint-LC-1 aa; and pTT3-Hc-VMAint-LC-3aa. See also Fig. 15 for a plasmid map.

Table 20. Sequence of entire plasmid pTT3-D2E7 Heavy Chain - intein-D2E7 Light Chain (SEQ ID NO:124)

| |
|---|
| 5'- gcggccgctcgaggccggcaaggccggatcccccgacctcgacctctggctaataaaggaaatttattttcattgcaa tagtgtgttggaattttttgtgtctctcactcggaaggacatatgggagggcaaatcatttggtcgagatccctcggagatc tctagctagaggatcgatccccgccccggacgaactaaacctgactacgacatctctgccccttcttcgcggggcagt gcatgtaatcccttcagttggttggtacaacttgccaactgggccctgttccacatgtgacacggggggggaccaaac acaaaggggttctctgactgtagttgacatccttataaatggatgtgcacatttgccaacactgagtggctttcatcctgg agcagactttgcagtctgtggactgcaacacaacattgcctttatgtgtaactcttggctgaagctcttacaccaatgctg ggggacatgtacctcccaggggcccaggaagactacgggaggctacaccaacgtcaatcagaggggcctgtgta gctaccgataagcggaccctcaagagggcattagcaatagtgtttataaggccccttgttaaccctaaacgggtagc atatgcttcccgggtagtagtatatactatccagactaaccctaattcaatagcatatgttacccaacgggaagcatatg ctatcgaattagggttagtaaaagggtcctaaggaacagcgatatctcccaccccatgagctgtcacggttttatttaca tggggtcaggattccacgagggtagtgaaccattttagtcacaagggcagtggctgaagatcaaggagcgggcagt gaactctcctgaatcttcgcctgcttcttcattctccttcgtttagctaatagaataactgctgagttgtgaacagtaaggtgt atgtgaggtgctcgaaaacaaggtttcaggtgacgcccccagaataaaatttggacggggggttcagtggtggcatt gtgctatgacaccaatataaccctcacaaaccccttgggcaataaatactagtgtaggaatgaaacattctgaatatct ttaacaatagaaatccatggggtggggacaagccgtaaagactggatgtccatctcacacgaatttatggctatgggc aacacataatcctagtgcaatatgatactggggttattaagatgtgtcccaggcagggaccaagacaggtgaaccat gttgttacactctatttgtaacaaggggaaagagagtggacgccgacagcagcggactccactggttgtctctaacac ccccgaaaattaaacggggctccacgccaatggggcccataaacaaagacaagtggccactcttttttttgaaattgt ggagtgggggcacgcgtcagcccccacacgccgccctgcggttttggactgtaaaataagggtgtaataacttggct gattgtaaccccgctaaccactgcggtcaaaccacttgcccacaaaaccactaatggcaccccggggaatacctgc ataagtaggtgggcgggccaagataggggcgcgattgctgcgatctggaggacaaattacacacacttgcgcctga gcgccaagcacagggttgttggtcctcatattcacgaggtcgctgagagcacggtgggctaatgttgccatgggtagc atatactacccaaatatctggatagcatatgctatcctaatctatatctgggtagcataggctatcctaatctatatctgggt agcatatgctatcctaatctatatctgggtagtatatgctatcctaatttatatctgggtagcataggctatcctaatctatatc tgggtagcatatgctatcctaatctatatctgggtagtatatgctatcctaatctgtatccgggtagcatatgctatcctaata gagattagggtagtatatgctatcctaatttatatctgggtagcatatactacccaaatatctggatagcatatgctatccta atctatatctgggtagcatatgctatcctaatctatatctgggtagcataggctatcctaatctatatctgggtagcatatgct atcctaatctatatctgggtagtatatgctatcctaatttatatctgggtagcataggctatcctaatctatatctgggtagcat atgctatcctaatctatatctgggtagtatatgctatcctaatctgtatccgggtagcatatgctatcctcatgataagctgtc aaacatgagaattttcttgaagacgaaagggcctcgtgatacgcctatttttataggttaatgtcatgataataatggtttct tagacgtcaggtggcacttttcggggaaatgtgcgcggaacccctatttgtttatttttctaaatacattcaaatatgtatcc gctcatgagacaataaccctgataaatgcttcaataatattgaaaaaggaagagtatgagtattcaacatttccgtgtc gcccttattcccttttttgcggcattttgccttcctgtttttgctcacccagaaacgctggtgaaagtaaaagatgctgaagat cagttgggtgcacgagtgggttacatcgaactggatctcaacagcggtaagatccttgagagttttcgccccgaagaa cgttttccaatgatgagcacttttaaagttctgctatgtggcgcggtattatcccgtgttgacgccgggcaagagcaactc ggtcgccgcatacactattctcagaatgacttggttgagtactcaccagtcacagaaaagcatcttacggatggcatga cagtaagagaattatgcagtgctgccataaccatgagtgataacactgcggccaacttacttctgacaacgatcgga ggaccgaaggagctaaccgcttttttgcacaacatgggggatcatgtaactcgccttgatcgttgggaaccggagctg aatgaagccataccaaacgacgagcgtgacaccacgatgcctgcagcaatggcaacaacgttgcgcaaactatta actggcgaactacttactctagcttcccggcaacaattaatagactggatggaggcggataaagttgcaggaccactt ctgcgctcggcccttccggctggctggtttattgctgataaatctggagccggtgagcgtgggtctcgcggtatcattgca gcactggggccagatggtaagccctcccgtatcgtagttatctacacgacggggagtcaggcaactatggatgaacg aaatagacagatcgctgagataggtgcctcactgattaagcattggtaactgtcagaccaagtttactcatatatacttta gattgatttaaaacttcatttttaatttaaaaggatctaggtgaagatcctttttgataatctcatgaccaaaatcccttaacg tgagttttcgttccactgagcgtcagaccccgtagaaaagatcaaaggatcttcttgagatcctttttttctgcgcgtaatct |
| gctgcttgcaaacaaaaaaaccaccgctaccagcggtggtttgtttgccggatcaagagctaccaactctttttccgaa ggtaactggcttcagcagagcgcagataccaaatactgttcttctagtgtagccgtagttaggccaccacttcaagaac tctgtagcaccgcctacatacctcgctctgctaatcctgttaccagtggctgctgccagtggcgataagtcgtgtcttacc gggttggactcaagacgatagttaccggataaggcgcagcggtcgggctgaacggggggttcgtgcacacagccc agcttggagcgaacgacctacaccgaactgagatacctacagcgtgagctatgagaaagcgccacgcttcccgaa gggagaaaggcggacaggtatccggtaagcggcagggtcggaacaggagagcgcacgagggagcttccaggg ggaaacgcctggtatctttatagtcctgtcgggtttcgccacctctgacttgagcgtcgatttttgtgatgctcgtcagggg ggcggagcctatggaaaaacgccagcaacgcggcctttttacggttcctggccttttgctggccttttgctcacatgttcttc ctgcgttatcccctgattctgtggataaccgtattaccgcctttgagtgagctgataccgctcgccgcagccgaacgacc gagcgcagcgagtcagtgagcgaggaagcggaagagcgcccaatacgcaaaccgcctctccccgcgcgttggc cgattcattaatgcagctggcacgacaggtttcccgactggaaagcgggcagtgagcgcaacgcaattaatgtgagt tagctcactcattaggcaccccaggctttacactttatgcttccggctcgtatgttgtgtggaattgtgagcggataacaatt tcacacaggaaacagctatgaccatgattacgccaagctctagctagaggtcgaccaattctcatgtttgacagcttat catcgcagatccgggcaacgttgttgccattgctgcaggcgcagaactggtaggtatggaagatctatacattgaatc aatattggcaattagccatattagtcattggttatatagcataaatcaatattggctattggccattgcatacgttgtatctat atcataatatgtacatttatattggctcatgtccaatatgaccgccatgttgacattgattattgactagttattaatagtaatc aattacggggtcattagttcatagcccatatatggagttccgcgttacataacttacggtaaatggcccgcctggctgac cgcccaacgacccccgcccattgacgtcaataatgacgtatgttcccatagtaacgccaatagggactttccattgac gtcaatgggtggagtatttacggtaaactgcccacttggcagtacatcaagtgtatcatatgccaagtccgccccctatt gacgtcaatgacggtaaatggcccgcctggcattatgcccagtacatgaccttacgggactttcctacttggcagtaca tctacgtattagtcatcgctattaccatggtgatgcggttttggcagtacaccaatgggcgtggatagcggtttgactcac ggggatttccaagtctccaccccattgacgtcaatgggagtttgttttggcaccaaaatcaacgggactttccaaaatgt cgtaataaccccgccccgttgacgcaaatgggcggtaggcgtgtacggtgggaggtctatataagcagagctcgttt agtgaaccgtcagatcctcactctcttccgcatcgctgtctgcgagggccagctgttgggctcgcggttgaggacaaac tcttcgcggtctttccagtactcttggatcggaaacccgtcggcctccgaacggtactccgccaccgagggacctgag cgagtccgcatcgaccggatcggaaaacctctcgagaaaggcgtctaaccagtcacagtcgcaaggtaggctgag caccgtggcgggcggcagcgggtggcggtcggggttgtttctggcggaggtgctgctgatgatgtaattaaagtaggc ggtcttgagacggcggatggtcgaggtgaggtgtggcaggcttgagatccagctgttggggtgagtactccctctcaa aagcgggcattacttctgcgctaagattgtcagtttccaaaaacgaggaggatttgatattcacctggcccgatctggcc atacacttgagtgacaatgacatccactttgcctttctctccacaggtgtccactcccaggtccaagtttgggcgccacc atggagtttgggctgagctggctttttcttgtcgcgattttaaaaggtgtccagtgt- gaggtgcagctggtggagtctgggggaggcttggtacagcccggcaggtccctgagactctcctgtgcggcctctgg attcacctttgatgattatgccatgcactgggtccggcaagctccagggaagggcctggaatgggtctcagctatcactt ggaatagtggtcacatagactatgcggactctgtggagggccgattcaccatctccagagacaacgccaagaactc cctgtatctgcaaatgaacagtctgagagctgaggatacggccgtatattactgtgcgaaagtctcgtaccttagcacc gcgtcctcccttgactattggggccaaggtaccctggtcaccgtctcgagtgcgtcgaccaagggcccatcggtcttcc ccctggcaccctcctccaagagcacctctgggggcacagcggccctgggctgcctggtcaaggactacttccccga accggtgacggtgtcgtggaactcaggcgccctgaccagcggcgtgcacaccttcccggctgtcctacagtcctcag gactctactccctcagcagcgtggtgaccgtgccctccagcagcttgggcacccagacctacatctgcaacgtgaatc acaagcccagcaacaccaaggtggacaagaaagttgagcccaaatcttgtgacaaaactcacacatgcccaccg tgcccagcacctgaactcctggggggaccgtcagtcttcctcttccccccaaaacccaaggacaccctcatgatctcc cggacccctgaggtcacatgcgtggtggtggacgtgagccacgaagaccctgaggtcaagttcaactggtacgtgg acggcgtggaggtgcataatgccaagacaaagccgcgggaggagcagtacaacagcacgtaccgtgtggtcag cgtcctcaccgtcctgcaccaggactggctgaatggcaaggagtacaagtgcaaggtctccaacaaagccctccca gcccccatcgagaaaaccatctccaaagccaaagggcagccccgagaaccacaggtgtacaccctgcccccatc ccgggatgagctgaccaagaaccaggtcagcctgacctgcctggtcaaaggcttctatcccagcgacatcgccgtg gagtgggagagcaatgggcagccggagaacaactacaagaccacgcctcccgtgctggactccgacggctccttc ttcctctacagcaagctcaccgtggacaagagcaggtggcagcaggggaacgtcttctcatgctccgtgatgcatga ggctctgcacaaccactacacgcagaagagcctctccctgtctccgggtaaa- tgctttgccaagggtaccaatgtttaatggcggatggtctattgaatgtattgaaacattgaggttggtaataaggtca |
| tgggtaaagatggcagacctcgtgaggtaattaaattgcccagaggaagagaaactatgtacagcgtcgtgcagaa aagtcagcacagagcccacaaaagtgactcaagtcgtgaagtgccagaattactcaagtttacgtgtaatgcgaccc atgagttggttgttagaacacctcgtagtgtccgccgtttgtctcgtaccattaagggtgtcgaatattttgaagttattactttt gagatgggccaaaagaaagcccccgacggtagaattgttgagcttgtcaaggaagtttcaaagagctacccaatat ctgaggggcctgagagagccaacgaattagtagaatcctatagaaaggcttcaaataaagcttatttgagtggacta ttgaggccagagatctttctctgttgggttcccatgttcgtaaagctacctaccagacttacgctccaattctttatgagaat gaccactttttcgactacatgcaaaaaagtaagtttcatctcaccattgaaggtccaaaagtacttgcttatttacttggttt atggattggtgatggattgtctgacagggcaacttttcggttgattccagagatacttcttgatggaacgtgttactgaat atgctgaaaagttgaatttgtgcgccgagtataaggacagaaaagaaccacaagttgccaaaactgttaatttgtact ctaaagttgtcagaggtaatggtattcgcaataatcttaatactgagaatccattatgggacgctattgttggcttaggatt cttgaaggacggtgtcaaaaatattccttctttcttgtctacggacaatatcggtactcgtgaaacatttcttgctggtctaat tgattctgatggctatgttactgatgagcatggtattaaagcaacaataaagacaattcatacttctgtcagagatggtttg gtttcccttgctcgttctttaggcttagtagtctcggttaacgcagaacctgctaaggttgacatgaatggcaccaaacata aaattagttatgctatttatatgtctggtggagatgttttgcttaacgttctttcgaagtgtgccggctctaaaaaattcaggc ctgctcccgccgctgcttttgcacgtgagtgccgcggatttatttcgagttacaagaattgaaggaagacgattattatg ggattactttatctgatgattctgatcatcagtttttgcttgccaaccaggttgtcgtccataat- atggacatgcgcgtgcccgcccagctgctgggcctgctgctgctgtggttccccggctcgcgatgcgacatccagatg acccagtctccatcctccctgtctgcatctgtaggggacagagtcaccatcacttgtcgggcaagtcagggcatcaga aattacttagcctggtatcagcaaaaaccagggaaagcccctaagctcctgatctatgctgcatccactttgcaatcag gggtcccatctcggttcagtggcagtggatctgggacagatttcactctcaccatcagcagcctacagcctgaagatgt tgcaacttattactgtcaaaggtataaccgtgcaccgtatacttttggccaggggaccaaggtggaaatcaaacgtac ggtggctgcaccatctgtcttcatcttcccgccatctgatgagcagttgaaatctggaactgcctctgttgtgtgcctgctg aataacttctatcccagagaggccaaagtacagtggaaggtggataacgccctccaatcgggtaactcccaggaga gtgtcacagagcaggacagcaaggacagcacctacagcctcagcagcaccctgacgctgagcaaagcagacta cgagaaacacaaagtctacgcctgcgaagtcacccatcagggcctgagctcgcccgtcacaaagagcttcaacag gggagagtgt -3' |

pTT3 Vector-Heavy Chain-intein-Light Chain

In the following constructs, the only difference from the construct above is the inclusion of extein sequences native to *S. cerevisiae* (shown in blue). The sequences shown are from the end of the D2E7 heavy chain coding region (last 9 base pairs as shown in red) to the 5' end of the D2E7 light chain coding region (first 9 base pairs as shown in pink)

Table 21. Partial coding sequence in pTT3-HC-VMAint-LC-1 aa (SEQ ID NO:125)

| |
|---|
| 5’-ccgggtaaa-ggg- tgctttgccaagggtaccaatgttttaatggcggatgggtctattgaatgtattgaaaacattgaggttggtaataaggtca tgggtaaagatggcagacctcgtgaggtaattaaattgcccagaggaagagaaactatgtacagcgtcgtgcagaa aagtcagcacagagcccacaaaagtgactcaagtcgtgaagtgccagaattactcaagtttacgtgtaatgcgaccc atgagttggttgttagaacacctcgtagtgtccgccgtttgtctcgtaccattaagggtgtcgaatattttgaagttattactttt gagatgggccaaaagaaagcccccgacggtagaattgttgagcttgtcaaggaagtttcaaagagctacccaatat ctgaggggcctgagagagccaacgaattagtagaatcctatagaaaggcttcaaataaagcttatttgagtggacta ttgaggccagagatctttctctgttgggttcccatgttcgtaaagctacctaccagacttacgctccaattctttatgagaat gaccactttttcgactacatgcaaaaaagtaagtttcatctcaccattgaaggtccaaaagtacttgcttatttacttggttt |
| atggattggtgatggattgtctgacagggcaacttttcggttgattccagagatacttcttgatggaacgtgttactgaat atgctgaaaagttgaatttgtgcgccgagtataaggacagaaaagaaccacaagttgccaaaactgttaatttgtact ctaaagttgtcagaggtaatggtattcgcaataatcttaatactgagaatccattatgggacgctattgttggcttaggatt cttgaaggacggtgtcaaaaatattccttctttcttgtctacggacaatatcggtactcgtgaaacatttcttgctggtctaat tgattctgatggctatgttactgatgagcatggtattaaagcaacaataaagacaattcatacttctgtcagagatggtttg gtttcccttgctcgttctttaggcttagtagtctcggttaacgcagaacctgctaaggttgacatgaatggcaccaaacata aaattagttatgctatttatatgtctggtggagatgttttgcttaacgttctttcgaagtgtgccggctctaaaaaattcaggc ctgctcccgccgctgcttttgcacgtgagtgccgcggatttatttcgagttacaagaattgaaggaagacgattattatg ggattactttatctgatgattctgatcatcagtttttgcttgccaaccaggttgtcgtccataat-tgc-atggacatg-3' |

Heavy Chain 3' sequence- Intein-Extein-Light Chain 5' sequence

Table 22. pTT3-HC-VMAint-LC-3aa (SEQ ID NO:126)

| |
|---|
| ccgggtaaatatgtcgggtgctttgccaagggtaccaatgttttaatggcggatgggtctattgaatgtattgaaaacatt gaggttggtaataaggtcatgggtaaagatggcagacctcgtgaggtaattaaattgcccagaggaagagaaactat gtacagcgtcgtgcagaaaagtcagcacagagcccacaaaagtgactcaagtcgtgaagtgccagaattactcaa gtttacgtgtaatgcgacccatgagttggttgttagaacacctcgtagtgtccgccgtttgtctcgtaccattaagggtgtc gaatattttgaagttattacttttgagatgggccaaaagaaagcccccgacggtagaattgttgagcttgtcaaggaagt ttcaaagagctacccaatatctgaggggcctgagagagccaacgaattagtagaatcctatagaaaggcttcaaata aagcttattttgagtggactattgaggccagagatctttctctgttgggttcccatgttcgtaaagctacctaccagacttac gctccaattctttatgagaatgaccactttttcgactacatgcaaaaaagtaagtttcatctcaccattgaaggtccaaaa gtacttgcttatttacttggtttatggattggtgatggattgtctgacagggcaactttttcggttgattccagagatacttctttg atggaacgtgttactgaatatgctgaaaagttgaatttgtgcgccgagtataaggacagaaaagaaccacaagttgc caaaactgttaatttgtactctaaagttgtcagaggtaatggtattcgcaataatcttaatactgagaatccattatgggac gctattgttggcttaggattcttgaaggacggtgtcaaaaatattccttctttcttgtctacggacaatatcggtactcgtgaa acatttcttgctggtctaattgattctgatggctatgttactgatgagcatggtattaaagcaacaataaagacaattcata cttctgtcagagatggtttggtttcccttgctcgttctttaggcttagtagtctcggttaacgcagaacctgctaaggttgaca tgaatggcaccaaacataaaattagttatgctatttatatgtctggtggagatgttttgcttaacgttctttcgaagtgtgccg gctctaaaaaattcaggcctgctcccgccgctgcttttgcacgtgagtgccgcggatttatttcgagttacaagaattga aggaagacgattattatgggattactttatctgatgattctgatcatcagtttttgcttgccaaccaggttgtcgtccataattg cggagaaatggacatg |

Heavy Chain 3' sequence-Intein-Extein-Light Chain 5' sequence

Synechocystis spp. Strain PCC6803 DnaE intein: Synthesis, PCR amplification and cloning

The Synechocystis spp. Strain PCC6803 DnaE intein is a naturally split intein (NCBI accession #s S76958 and S75328). We have linked the N'terminal and C-terminal halves of this intein as one open reading frame by having it synthetically synthesized. The coding sequence for the desired protein sequence was codon-optimized for expression in CHO cells (www.geneart.com). The resulting nucleotide sequence is given in Table 23.

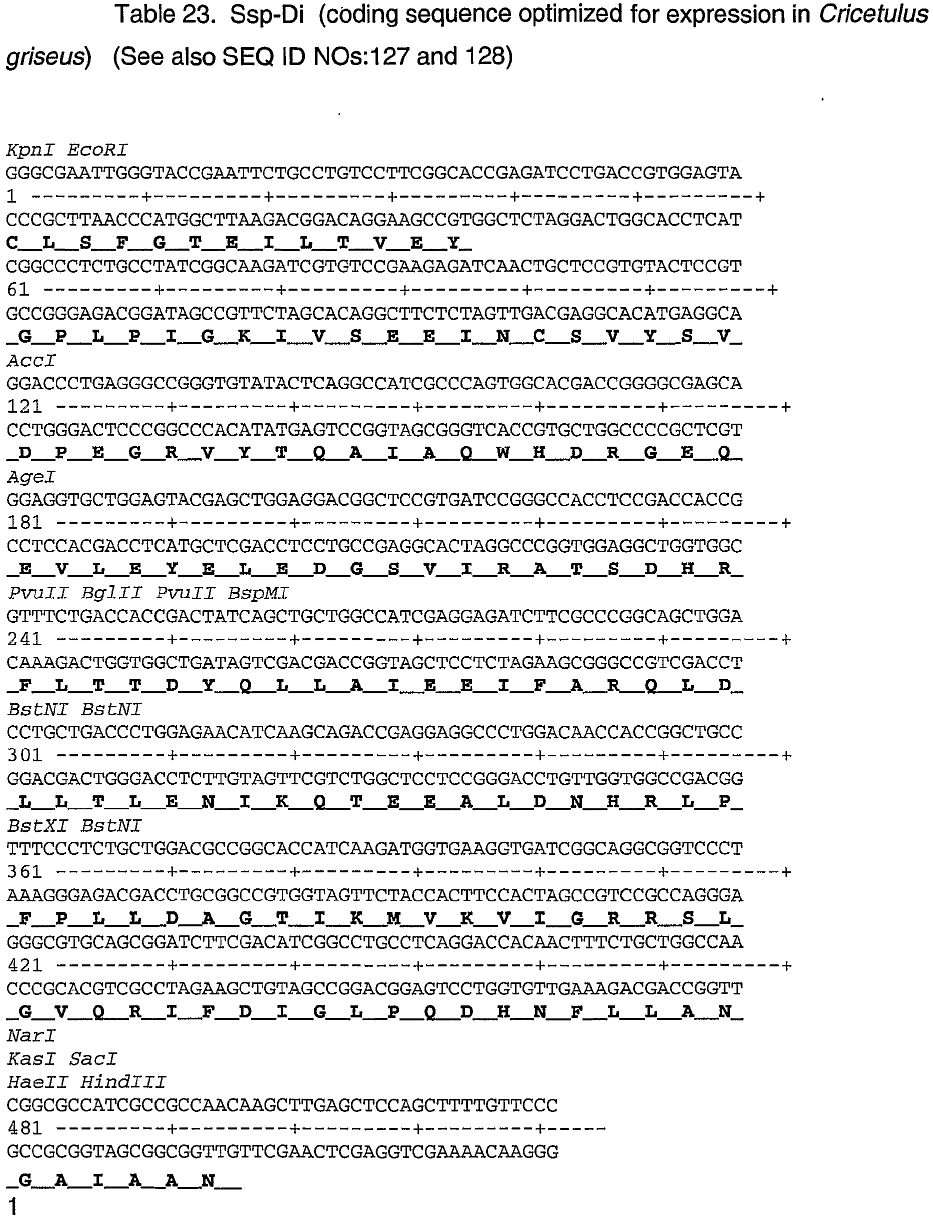

The following oligonucleotides were used for the amplification of the Synechocystis spp. Strain PCC6803 DnaE intein using the synthetic DNA above as template and Platinum Taq Hi Fidelity Supermix (Invitrogen). These primers also introduce extein sequences to generate the 0aa, 1 aa and 3aa versions, as well as sequences for the homologous recombination of the PCR product into the pTT3-HcintLC vector as done with the S. cerevisiae VMA intein: 
Ssp-geneart-5' HR:
CCACTACACGCAGAAGAGCCTCTCCCTGTCTCCGGGTAAATGCCTGTCC
TTCGGCACCGAG (SEQ ID NO:129)
 
Ssp-geneart-3'-HR:
GCAGCAGGCCCAGCAGCTGGGCGGCACGCGCATGTCCATGTTGGCG
GCGATGGCGCCGTTGGCC (SEQ ID NO:130)
 
Ssp-GA-1aa-5'-HR:
CCACTACACGCAGAAGAGCCTCTCCCTGTCTCCGGGTAAATATTGCCTG
TCCTTCGGCACCGAG (SEQ ID NO:131)
 
Ssp-GA-1aa-3'-HR:
GCAGCAGGCCCAGCAGCTGGGCGGCACGCGCATGTCCATACAGTTGG
CGGCGATGGCGCCGT (SEQ ID NO:132)
 
Ssp-GA-3aa-5'-HR:
CCACTACACGCAGAAGAGCCTCTCCCTGTCTCCGGGTAAAGCCGAGTAT
TGCCTGTCCTTCGGCACCGAG (SEQ ID NO:133)
 
Ssp-GA-3aa-3'-HR:
CCACTACACGCAGAAGAGCCTCTCCCTGTCTCCGGGTAAAGCCGAGTAT
TGCCTGTCCTTCGGCACCGAG (SEQ ID NO:134)
 PCR run on the following program:

| Step | 1 | 2 | 3 | 4 | 5 | 6 | 7 | 8 |
|---|---|---|---|---|---|---|---|---|
| Temp | 94°C | 94°C | 60°C | 68°C | Go to step 2 (34 times) | 68°C | 4°C | End |
| Time | 2min | 30sec | 30sec | 1 min | | 5min | hold | |

To obtain homologous recombination of the codon-optimized Synechocystis spp. Strain PCC6803 DnaE intein into pTT3-HcintLC, the following strategy was used. PCR products were gel purified and each eluted into 50ul elution buffer (Qiaquick Gel Extraction kit (Qiagen). 2 µl of the vector PCR product (same as used in the homologous recombination with the VMA intein) was mixed in an Eppendorf tube 2 µl of the desired Synechocystis spp. Strain PCC6803 DnaE intein PCR product (either 0aa, 1 aa or 3aa in separate tubes). The nucleic acids are then transformed into *E. coli* and plated onto LB + Ampicillin plates and then incubated at 37ºC overnight. Colonies were grown to 2 ml cultures, prepped for DNA using the Wizard prep kit (Promega) and assayed by restriction endonuclease digestion and agarose gel electrophoresis. Clones that produce the correct restriction pattern are analyzed with respect to DNA sequence to confirm that the desired sequences are present.

Three Expression Constructs for D2E7 Heavy Chain - intein - D2E7 Light Chain, utilizing the Synechocystis spp. Strain PCC6803 DnaE intein were designed: pTT3-Hc.Ssp-GA-int-LC-0aa (See Fig. 16 for plasmid map); pTT3-Hc- Ssp-GA-int-LC-1 aa; and pTT3-Hc- Ssp-GA-int-LC-3aa.

Table 24. Sequence of entire plasmid pTT3-D2E7 Heavy Chain - Ssp-GA-intein - D2E7 Light Chain (SEQ ID NO:135)

| |
|---|
| 5'- gcggccgctcgaggccggcaaggccggatccccgacctcgacctctggctaataaaggaaatttattttcattgcaa tagtgtgttggaattttttgtgtctctcactcggaaggacatatgggagggcaaatcatttggtcgagatccctcggagatc tctagctagaggatcgatccccgccccggacgaactaaacctgactacgacatctctgccccttcttcgcggggcagt gcatgtaatcccttcagttggttggtacaacttgccaactgggccctgttccacatgtgacacggggggggaccaaac acaaaggggttctctgactgtagttgacatccttataaatggatgtgcacatttgccaacactgagtggctttcatcctgg agcagactttgcagtctgtggactgcaacacaacattgcctttatgtgtaactcttggctgaagctcttacaccaatgctg ggggacatgtacctcccaggggcccaggaagactacgggaggctacaccaacgtcaatcagaggggcctgtgta gctaccgataagcggaccctcaagagggcattagcaatagtgtttataaggccccttgttaaccctaaacgggtagc atatgcttcccgggtagtagtatatactatccagactaaccctaattcaatagcatatgttacccaacgggaagcatatg ctatcgaattagggttagtaaaagggtcctaaggaacagcgatatctcccaccccatgagctgtcacggttttattaca tggggtcaggattccacgagggtagtgaaccattttagtcacaagggcagtggctgaagatcaaggagcgggcagt gaactctcctgaatcttcgcctgcttcttcattctccttcgtttagctaatagaataactgctgagttgtgaacagtaaggtgt atgtgaggtgctcgaaaacaaggtttcaggtgacgcccccagaataaaatttggacggggggttcagtggtggcatt gtgctatgacaccaatataaccctcacaaaccccttgggcaataaatactagtgtaggaatgaaacattctgaatatct ttaacaatagaaatccatggggtggggacaagccgtaaagactggatgtccatctcacacgaatttatggctatgggc aacacataatcctagtgcaatatgatactggggttattaagatgtgtcccaggcagggaccaagacaggtgaaccat gttgttacactctatttgtaacaaggggaaagagagtggacgccgacagcagcggactccactggttgtctctaacac ccccgaaaattaaacggggctccacgccaatggggcccataaacaaagacaagtggccactcttttttttgaaattgt ggagtgggggcacgcgtcagcccccacacgccgccctgcggttttggactgtaaaataagggtgtaataacttggct gattgtaaccccgctaaccactgcggtcaaaccacttgcccacaaaaccactaatggcaccccggggaatacctgc ataagtaggtgggcgggccaagataggggcgcgattgctgcgatctggaggacaaattacacacacttgcgcctga gcgccaagcacagggttgttggtcctcatattcacgaggtcgctgagagcacggtgggctaatgttgccatgggtagc atatactacccaaatatctggatagcatatgctatcctaatctatatctgggtagcataggctatcctaatctatatctgggt agcatatgctatcctaatctatatctgggtagtatatgctatcctaatttatatctgggtagcataggctatcctaatctatatc tgggtagcatatgctatcctaatctatatctgggtagtatatgctatcctaatctgtatccgggtagcatatgctatcctaata gagattagggtagtatatgctatcctaatttatatctgggtagcatatactacccaaatatctggatagcatatgctatccta atctatatctgggtagcatatgctatcctaatctatatctgggtagcataggctatcctaatctatatctgggtagcatatgct atcctaatctatatctgggtagtatatgctatcctaatttatatctgggtagcataggctatcctaatctatatctgggtagcat atgctatcctaatctatatctgggtagtatatgctatcctaatctgtatccgggtagcatatgctatcctcatgataagctgtc aaacatgagaattttcttgaagacgaaaggcctcgtgatacgcctatttttataggttaatgtcatgataataatggtttct tagacgtcaggtggcacttttcggggaaatgtgcgcggaacccctatttgtttatttttctaaatacattcaaatatgtatcc gctcatgagacaataaccctgataaatgcttcaataatattgaaaaaggaagagtatgagtattcaacatttccgtgtc gcccttattcccttttttgcggcattttgccttcctgtttttgctcacccagaaacgctggtgaaagtaaaagatgctgaagat |
| cagttgggtgcacgagtgggttacatcgaactggatctcaacagcggtaagatccttgagagttttcgccccgaagaa cgttttccaatgatgagcacttttaaagttctgctatgtggcgcggtattatcccgtgttgacgccgggcaagagcaactc ggtcgccgcatacactattctcagaatgacttggttgagtactcaccagtcacagaaaagcatcttacggatggcatga cagtaagagaattatgcagtgctgccataaccatgagtgataacactgcggccaacttacttctgacaacgatcgga ggaccgaaggagctaaccgcttttttgcacaacatgggggatcatgtaactcgccttgatcgttgggaaccggagctg aatgaagccataccaaacgacgagcgtgacaccacgatgcctgcagcaatggcaacaacgttgcgcaaactatta actggcgaactacttactctagcttcccggcaacaattaatagactggatggaggcggataaagttgcaggaccactt ctgcgctcggcccttccggctggctggtttattgctgataaatctggagccggtgagcgtgggtctcgcggtatcattgca gcactggggccagatggtaagccctcccgtatcgtagttatctacacgacggggagtcaggcaactatggatgaacg aaatagacagatcgctgagataggtgcctcactgattaagcattggtaactgtcagaccaagtttactcatatatactta gattgatttaaaacttcatttttaatttaaaaggatctaggtgaagatcctttttgataatctcatgaccaaaatcccttaacg tgagttttcgttccactgagcgtcagaccccgtagaaaagatcaaaggatcttcttgagatcctttttttctgcgcgtaatct gctgcttgcaaacaaaaaaaccaccgctaccagcggtggtttgtttgccggatcaagagctaccaactctttttccgaa ggtaactggcttcagcagagcgcagataccaaatactgttcttctagtgtagccgtagttaggccaccacttcaagaac tctgtagcaccgcctacatacctcgctctgctaatcctgttaccagtggctgctgccagtggcgataagtcgtgtcttacc gggttggactcaagacgatagttaccggataaggcgcagcggtcgggctgaacggggggttcgtgcacacagccc agcttggagcgaacgacctacaccgaactgagatacctacagcgtgagctatgagaaagcgccacgcttcccgaa gggagaaaggcggacaggtatccggtaagcggcagggtcggaacaggagagcgcacgagggagcttccaggg ggaaacgcctggtatctttatagtcctgtcgggtttcgccacctctgacttgagcgtcgatttttgtgatgctcgtcagggg ggcggagcctatggaaaaacgccagcaacgcggcctttttacggttcctggccttttgctggccttttgctcacatgttcttc ctgcgttatcccctgattctgtggataaccgtattaccgcctttgagtgagctgataccgctcgccgcagccgaacgacc gagcgcagcgagtcagtgagcgaggaagcggaagagcgcccaatacgcaaaccgcctctccccgcgcgttggc cgattcattaatgcagctggcacgacaggtttcccgactggaaagcgggcagtgagcgcaacgcaattaatgtgagt tagctcactcattaggcaccccaggctttacactttatgcttccggctcgtatgttgtgtggaattgtgagcggataacaatt tcacacaggaaacagctatgaccatgattacgccaagctctagctagaggtcgaccaattctcatgtttgacagcttat catcgcagatccgggcaacgttgttgccattgctgcaggcgcagaactggtaggtatggaagatctatacattgaatc aatattggcaattagccatattagtcattggttatatagcataaatcaatattggctattggccattgcatacgttgtatctat atcataatatgtacatttatattggctcatgtccaatatgaccgccatgttgacattgattattgactagttattaatagtaatc aattacggggtcattagttcatagcccatatatggagttccgcgttacataacttacggtaaatggcccgcctggctgac cgcccaacgacccccgcccattgacgtcaataatgacgtatgttcccatagtaacgccaatagggactttccattgac gtcaatgggtggagtatttacggtaaactgcccacttggcagtacatcaagtgtatcatatgccaagtccgccccctatt gacgtcaatgacggtaaatggcccgcctggcattatgcccagtacatgaccttacgggactttcctacttggcagtaca tctacgtattagtcatcgctattaccatggtgatgcggttttggcagtacaccaatgggcgtggatagcggtttgactcac ggggatttccaagtctccaccccattgacgtcaatgggagtttgttttggcaccaaaatcaacgggactttccaaaatgt cgtaataaccccgccccgttgacgcaaatgggcggtaggcgtgtacggtgggaggtctatataagcagagctcgttt agtgaaccgtcagatcctcactctcttccgcatcgctgtctgcgagggccagctgttgggctcgcggttgaggacaaac tcttcgcggtctttccagtactcttggatcggaaacccgtcggcctccgaacggtactccgccaccgagggacctgag cgagtccgcatcgaccggatcggaaaacctctcgagaaaggcgtctaaccagtcacagtcgcaaggtaggctgag caccgtggcgggcggcagcgggtggcggtcggggttgtttctggcggaggtgctgctgatgatgtaattaaagtaggc ggtcttgagacggcggatggtcgaggtgaggtgtggcaggcttgagatccagctgttggggtgagtactccctctcaa aagcgggcattacttctgcgctaagattgtcagtttccaaaaacgaggaggatttgatattcacctggcccgatctggcc atacacttgagtgacaatgacatccactttgcctttctctccacaggtgtccactcccaggtccaagtttgggcgccacc atggagtttgggctgagctggctttttcttgtcgcgattttaaaaggtgtccagtgt- gaggtgcagctggtggagtctgggggaggcttggtacagcccggcaggtccctgagactctcctgtgcggcctctgg attcacctttgatgattatgccatgcactgggtccggcaagctccagggaagggcctggaatgggtctcagctatcactt ggaatagtggtcacatagactatgcggactctgtggagggccgattcaccatctccagagacaacgccaagaactc cctgtatctgcaaatgaacagtctgagagctgaggatacggccgtatattactgtgcgaaagtctcgtaccttagcacc gcgtcctcccttgactattggggccaaggtaccctggtcaccgtctcgagtgcgtcgaccaagggcccatcggtcttcc ccctggcaccctcctccaagagcacctctgggggcacagcggccctgggctgcctggtcaaggactacttccccga accggtgacggtgtcgtggaactcaggcgccctgaccagcggcgtgcacaccttcccggctgtcctacagtcctcag |
| gactctactccctcagcagcgtggtgaccgtgccctccagcagcttgggcacccagacctacatctgcaacgtgaatc acaagcccagcaacaccaaggtggacaagaaagttgagcccaaatcttgtgacaaaactcacacatgcccaccg tgcccagcacctgaactcctggggggaccgtcagtcttcctcttccccccaaaacccaaggacaccctcatgatctcc cggacccctgaggtcacatgcgtggtggtggacgtgagccacgaagaccctgaggtcaagttcaactggtacgtgg acggcgtggaggtgcataatgccaagacaaagccgcgggaggagcagtacaacagcacgtaccgtgtggtcag cgtcctcaccgtcctgcaccaggactggctgaatggcaaggagtacaagtgcaaggtctccaacaaagccctccca gcccccatcgagaaaaccatctccaaagccaaagggcagccccgagaaccacaggtgtacaccctgcccccatc ccgggatgagctgaccaagaaccaggtcagcctgacctgcctggtcaaaggcttctatcccagcgacatcgccgtg gagtgggagagcaatgggcagccggagaacaactacaagaccacgcctcccgtgctggactccgacggctccttc ttcctctacagcaagctcaccgtggacaagagcaggtggcagcaggggaacgtcttctcatgctccgtgatgcatga ggctctgcacaaccactacacgcagaagagcctctccctgtctccgggtaaa- tgcctgtccttcggcaccgagatcctgaccgtggagtacggccctctgcctatcggcaagatcgtgtccgaagagatc aactgctccgtgtactccgtggaccctgagggccgggtgtatactcaggccatcgcccagtggcacgaccggggcg agcaggaggtgctggagtacgagctggaggacggctccgtgatccgggccacctccgaccaccggtttctgaccac cgactatcagctgctggccatcgaggagatcttcgcccggcagctggacctgctgaccctggagaacatcaagcag accgaggaggccctggacaaccaccggctgcctttccctctgctggacgccggcaccatcaagatggtgaaggtga tcggcaggcggtccctgggcgtgcagcggatcttcgacatcggcctgcctcaggaccacaactttctgctggccaac ggcgccatcgccgccaac- atggacatgcgcgtgcccgcccagctgctgggcctgctgctgctgtggttccccggctcgcgatgcgacatccagatg acccagtctccatcctccctgtctgcatctgtaggggacagagtcaccatcacttgtcgggcaagtcagggcatcaga aattacttagcctggtatcagcaaaaaccagggaaagcccctaagctcctgatctatgctgcatccactttgcaatcag gggtcccatctcggttcagtggcagtggatctgggacagatttcactctcaccatcagcagcctacagcctgaagatgt tgcaacttattactgtcaaaggtataaccgtgcaccgtatacttttggccaggggaccaaggtggaaatcaaacgtac ggtggctgcaccatctgtcttcatcttcccgccatctgatgagcagttgaaatctggaactgcctctgttgtgtgcctgctg aataacttctatcccagagaggccaaagtacagtggaaggtggataacgccctccaatcgggtaactcccaggaga gtgtcacagagcaggacagcaaggacagcacctacagcctcagcagcaccctgacgctgagcaaagcagacta cgagaaacacaaagtctacgcctgcgaagtcacccatcagggcctgagctcgcccgtcacaaagagcttcaacag gggagagtgt -3' |

pTT3 Vector-Heavy Chain-Intein-Light Chain

In the following constructs, the only difference from the construct above is the inclusion of extein sequences native to *Synechocystis spp.* Strain PCC6803 (shown in blue). The sequences shown are from the end of the D2E7 heavy chain coding region (last 9 base pairs as shown in red) to the 5' end of the D2E7 light chain coding region (first 9 base pairs as shown in pink).

Table 25. pTT3-HC-Ssp-GA-int-LC-1 aa, relevant portion of coding sequence (SEQ ID NO:136)

| |
|---|
| Ccgggtaaa-tatt- gcctgtccttcggcaccgagatcctgaccgtggagtacggccctctgcctatcggcaagatcgtgtccgaagagatca actgctccgtgtactccgtggaccctgagggccgggtgtatactcaggccatcgcccagtggcacgaccggggcga gcaggaggtgctggagtacgagctggaggacggctccgtgatccgggccacctccgaccaccggtttctgaccacc gactatcagctgctggccatcgaggagatcttcgcccggcagctggacctgctgaccctggagaacatcaagcaga ccgaggaggccctggacaaccaccggctgcctttccctctgctggacgccggcaccatcaagatggtgaaggtgat |
| cggcaggcggtccctgggcgtgcagcggatcttcgacatcggcctgcctcaggaccacaactttctgctggccaacg gcgccatcgccgccaac-tgt-atggacatg |

pTT3 Vector-Heavy Chain-Intein-Light Chain

Table 26. pTT3-HC-Ssp-GA-int-LC-3aa - relevant portion of coding sequence (SEQ ID NO:137)

| |
|---|
| Ccgggtaaa-gccgagtatt- gcctgtccttcggcaccgagatcctgaccgtggagtacggccctctgcctatcggcaagatcgtgtccgaagagatca actgctccgtgtactccgtggaccctgagggccgggtgtatactcaggccatcgcccagtggcacgaccggggcga gcaggaggtgctggagtacgagctggaggacggctccgtgatccgggccacctccgaccaccggtttctgaccacc gactatcagctgctggccatcgaggagatcttcgcccggcagctggacctgctgaccctggagaacatcaagcaga ccgaggaggccctggacaaccaccggctgcctttccctctgctggacgccggcaccatcaagatggtgaaggtgat cggcaggcggtccctgggcgtgcagcggatcttcgacatcggcctgcctcaggaccacaactttctgctggccaacg gcgccatcgccgccaac-tgtttcaac-atggacatg |

pTT3 Vector-Heavy Chain-intein-Light Chain

In addition, tables 8A-8C provide relevant sequences for a D2E7 intein fusion protein, expression vector and coding sequence using the mutated (Serine to Threonine) Pyrococcus Ssp. GBD Pol intein.

Table 8A. Coding Sequence of D2E7 Intein Fusion Protein (SEQ ID NO:48)

| |
|---|
| ATGGAGTTTGGGCTGAGCTGGCTTTTTCTTGTCGCGATTTTAAAAGGTGTCCAGTGTGAGGTGCAGCTGGTGGAGTCTGG GGGAGGCTTGGTACAGCCCGGCAGGTCCCTGAGACTCTCCTGTGCGGCCTCTGGATTCACCTTTGATGATTATGCCATGC ACTGGGTCCGGCAAGCTCCAGGGAAGGGCCTGGAATGGGTCTCAGCTATCACTTGGAATAGTGGTCACATAGACTATGCG GACTCTGTGGAGGGCCGATTCACCATCTCCAGAGACAACGCCAAGAACTCCCTGTATCTGCAAATGAACAGTCTGAGAGC TGAGGATACGGCCGTATATTACTGTGCGAAAGTCTCGTACCTTAGCACCGCGTCCTCCCTTGACTATTGGGGCCAAGGTAC CCTGGTCACCGTCTCGAGTGCGTCGACCAAGGGCCCATCGGTCTTCCCCCTGGCACCCTCCTCCAAGAGCACCTCTGGG GGCACAGCGGCCCTGGGCTGCCTGGTCAAGGACTACTTCCCCGAACCGGTGACGGTGTCGTGGAACTCAGGCGCCCTGA CCAGCGGCGTGCACACCTTCCCGGCTGTCCTACAGTCCTCAGGACTCTACTCCCTCAGCAGCGTGGTGACCGTGCCCTC CAGCAGCTTGGGCACCCAGACCTACATCTGCAACGTGAATCACAAGCCCAGCAACACCAAGGTGGACAAGAAAGTTGAGC CCAAATCTTGTGACAAAACTCACACATGCCCACCGTGCCCAGCACCTGAACTCCTGGGGGGACCGTCAGTCTTCCTCTTC CCCCCAAAACCCAAGGACACCCTCATGATCTCCCGGACCCCTGAGGTCACATGCGTGGTGGTGGACGTGAGCCACGAAG ACCCTGAGGTCAAGTTCAACTGGTACGTGGACGGCGTGGAGGTGCATAATGCCAAGACAAAGCCGCGGGAGGAGCAGTA CAACAGCACGTACCGTGTGGTCAGCGTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAAGGGCAGCCCCGAGAACCACAGGTGTA CACCCTGCCCCCATCCCGGGATGAGCTGACCAAGAACCAGGTCAGCCTGACCTGCCTGGTCAAAGGCTTCTATCCCAGC GACATCGCCGTGGAGTGGGAGAGCAATGGGCAGCCGGAGAACAACTACAAGACCACGCCTCCCGTGCTGGACTCCGACG GCTCCTTCTTCCTCTACAGCAAGCTCACCGTGGACAAGAGCAGGTGGCAGCAGGGGAACGTCTTCTCATGCTCCGTGATG CATGAGGCTCTGCACAACCACTACACGCAGAAGAGCCTCTCCCTGTCTCCGGGTAAAACCATTTTACCGGAAGAATGGGTT CCACTAATTAAAAACGGTAAAGTTAAGATATTCCGCATTGGGGACTTCGTTGATGGACTTATGAAGGCGAACCAAGGAAAA GTGAAGAAAACGGGGGATACAGAAGTTTTAGAAGTTGCAGGAATTCATGCGTTTTCCTTTGACAGGAAGTCCAAGAAGGCC CGTGTAATGGCAGTGAAAGCCGTGATAAGACACCGTTATTCCGGAAATGTTTATAGAATAGTCTTAAACTCTGGTAGAAAAA TAACAATAACAGAAGGGCATAGCCTATTTGTCTATAGGAACGGGGATCTCGTTGAGGCAACTGGGGAGGATGTCAAAATTG GGGATCTTCTTGCAGTTCCAAGATCAGTAAACCTACCAGAGAAAAGGGAACGCTTGAATATTGTTGAACTTCTTCTGAATCT CTCACCGGAAGAGACAGAAGATATAATACTTACGATTCCAGTTAAAGGCAGAAAGAACTTCTTCAAGGGAATGTTGAGAAC ATTACGTTGGATTTTTGGTGAGGAAAAGAGAGTAAGGACAGCGAGCCGCTATCTAAGACACCTTGAAAATCTCGGATACAT |
| AAGGTTGAGGAAAATTGGATACGACATCATTGATAAGGAGGGGCTTGAGAAATATAGAACGTTGTACGAGAAACTTGTTGA TGTTGTCCGCTATAATGGCAACAAGAGAGAGTATTTAGTTGAATTTAATGCTGTCCGGGACGTTATCTCACTAATGCCAGAG GAAGAACTGAAGGAATGGCGTATTGGAACTAGAAATGGATTCAGAATGGGTACGTTCGTAGATATTGATGAAGATTTTGCC AAGCTTGGATACGATAGCGGAGTCTACAGGGTTTATGTAAACGAGGAACTTAAGTTTACGGAATACAGAAAGAAAAAGAAT GTATATCACTCTCACATTGTTCCAAAGGATATTCTCAAAGAAACTTTTGGTAAGGTCTTCCAGAAAAATATAAGTTACAAGAA ATTTAGAGAGCTTGTAGAAAATGGAAAACTTGACAGGGAGAAAGCCAAACGCATTGAGTGGTTACTTAACGGAGATATAGT CCTAGATAGAGTCGTAGAGATTAAGAGAGAGTACTATGATGGTTACGTTTACGATCTAAGTGTCGATGAAGATGAGAATTTC CTTGCTGGCTTTGGATTCCTCTATGCACATAATGACATCCAGATGACCCAGTCTCCATCCTCCCTGTCTGCATCTGTAGGG GACAGAGTCACCATCACTTGTCGGGCAAGTCAGGGCATCAGAAATTACTTAGCCTGGTATCAGCAAAAACCAGGGAAAGC CCCTAAGCTCCTGATCTATGCTGCATCCACTTTGCAATCAGGGGTCCCATCTCGGTTCAGTGGCAGTGGATCTGGGACAG ATTTCACTCTCACCATCAGCAGCCTACAGCCTGAAGATGTTGCAACTTATTACTGTCAAAGGTATAACCGTGCACCGTATAC TTTTGGCCAGGGGACCAAGGTGGAAATCAAACGTACGGTGGCTGCACCATCTGTCTTCATCTTCCCGCCATCTGATGAGC AGTTGAAATCTGGAACTGCCTCTGTTGTGTGCCTGCTGAATAACTTCTATCCCAGAGAGGCCAAAGTACAGTGGAAGGTGG ATAACGCCCTCCAATCGGGTAACTCCCAGGAGAGTGTCACAGAGCAGGACAGCAAGGACAGCACCTACAGCCTCAGCAG CACCCTGACGCTGAGCAAAGCAGACTACGAGAAACACAAAGTCTACGCCTGCGAAGTCACCCATCAGGGCCTGAGCTCGC CCGTCACAAAGAGCTTCAACAGGGGAGAGTGTTGA |

Table 8B. Amino Acid Sequence of D2E7 Intein Fusion Construct (SEQ ID NO:49)

| |
|---|
| MEFGLSWLFLVAILKGVQCEVQLVESGGGLVQPGRSLRLSCAASGFTFDDYAMHWVRQAPGKGLEWVSAITWNSGHIDYADS VEGRFTISRDNAKNSLYLQMNSLRAEDTAVYYCAKVSYLSTASSLDYWGQGTLVTVSSASTKGPSVFPLAPSSKSTSGGTAALG CLVKDYFPEPVTVSWNSGALTSGVHTFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQVSLTCLVKGFYPSDIAVEWESNGQPENNYKTTP PVLDSDGSFFLYSKLTVDKSRWQQGNVFSCSVMHEALHNHYTQKSLSLSPGKTILPEEWVPLIKNGKVKIFRIGDFVDGLMKAN QGKVKKTGDTEVLEVAGIHAFSFDRKSKKARVMAVKAVIRHRYSGNVYRIVLNSGRKITITEGHSLFVYRNGDLVEATGEDVKIGD LLAVPRSVNLPEKRERLNIVELLLNLSPEETEDIILTIPVKGRKNFFKGMLRTLRWIFGEEKRVRTASRYLRHLENLGYIRLRKIGYDI IDKEGLEKYRTLYEKLVDVVRYNGNKREYLVEFNAVRDVISLMPEEELKEWRIGTRNGFRMGTFVDIDEDFAKLGYDSGVYRVYV NEELKFTEYRKKKNVYHSHIVPKDILKETFGKVFQKNISYKKFRELVENGKLDREKAKRIEWLLNGDIVLDRVVEIKREYYDGYVY DLSVDEDENFLAGFGFLYAHNDIQMTQSPSSLSASVGDRVTITCRASQGIRNYLAWYQQKPGKAPKLLIYAASTLQSGVPSRFS GSGSGTDFTLTISSLQPEDVATYYCQRYNRAPYTFGQGTKVEIKRTVAAPSVFIFPPSDEQLKSGTASVVCLLNNFYPREAKVQW KVDNALQSGNSQESVTEQDSKDSTYSLSSTLTLSKADYEKHKVYACEVTHQGLSSPVTKSFNRGEC* |

Table 8C. Complete Nucleotide Sequence of Expression Vector for the D2E7 Intein Fusion Construct (SECT ID NO:50)

| |
|---|
| GAAGTTCCTATTCCGAAGTTCCTATTCTCTAGACGTTACATAACTTACGGTAAATGGCCCGCCTG GCTGACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGGCAGTAC ATCAAGTGTATCATATGCCAAGTACGCCCCCTATTGACGTCAATGACGGTAAATGGCCCGCCTG GCATTATGCCCAGTACATGACCTTATGGGACTTTCCTACTTGGCAGTACATCTACGTATTAGTCAT CGCTATTACCATGGTGATGCGGTTTTGGCAGTACATCAATGGGCGTGGATAGCGGTTTGACTCA CGGGGATTTCCAAGTCTCCACCCCATTGACGTCAATGGGAGTTTGTTTTGGCACCAAAATCAACG GGACTTTCCAAAATGTCGTAACAACTCCGCCCCAATGACGCAAATGGGCAGGGAATTCGAGCTC GGTACTCGAGCGGTGTTCCGCGGTCCTCCTCGTATAGAAACTCGGACCACTCTGAGACGAAGGC TCGCGTCCAGGCCAGCACGAAGGAGGCTAAGTGGGAGGGGTAGCGGTCGTTGTCCACTAGGG GGTCCACTCGCTCCAGGGTGTGAAGACACATGTCGCCCTCTTCGGCATCAAGGAAGGTGATTGG TTTATAGGTGTAGGCCACGTGACCGGGTGTTCCTGAAGGGGGGCTATAAAAGGGGGTGGGGGC GCGTTCGTCCTCACTCTCTTCCGCATCGCTGTCTGCGAGGGCCAGCTGTTGGGCTCGCGGTTGA GGACAAACTCTTCGCGGTCTTTCCAGTACTCTTGGATCGGAAACCCGTCGGCCTCCGAACGGTA CTCCGCCACCGAGGGACCTGAGCGAGTCCGCATCGACCGGATCGGAAAACCTCTCGACTGTTG GGGTGAGTACTCCCTCTCAAAAGCGGGCATGACTTCTGCGCTAAGATTGTCAGTTTCCAAAAACG AGGAGGATTTGATATTCACCTGGCCCGCGGTGATGCCTTTGAGGGTGGCCGCGTCCATCTGGTC AGAAAAGACAATCTTTTTGTTGTCAAGCTTGAGGTGTGGCAGGCTTGAGATCTGGCCATACACTT GAGTGACAATGACATCCACTTTGCCTTTCTCTCCACAGGTGTCCACTCCCAGGTCCAACCGGAAT |
| TGTACCCGCGGCCAGAGCTTGCCCGGGCGCCACCATGGAGTTTGGGCTGAGCTGGCTTTTTCTT GTCGCGATTTTAAAAGGTGTCCAGTGTGAGGTGCAGCTGGTGGAGTCTGGGGGAGGCTTGGTA CAGCCCGGCAGGTCCCTGAGACTCTCCTGTGCGGCCTCTGGATTCACCTTTGATGATTATGCCA TGCACTGGGTCCGGCAAGCTCCAGGGAAGGGCCTGGAATGGGTCTCAGCTATCACTTGGAATA GTGGTCACATAGACTATGCGGACTCTGTGGAGGGCCGATTCACCATCTCCAGAGACAACGCCAA GAACTCCCTGTATCTGCAAATGAACAGTCTGAGAGCTGAGGATACGGCCGTATATTACTGTGCGA AAGTCTCGTACCTTAGCACCGCGTCCTCCCTTGACTATTGGGGCCAAGGTACCCTGGTCACCGT CTCGAGTGCGTCGACCAAGGGCCCATCGGTCTTCCCCCTGGCACCCTCCTCCAAGAGCACCTCT GGGGGCACAGCGGCCCTGGGCTGCCTGGTCAAGGACTACTTCCCCGAACCGGTGACGGTGTC GTGGAACTCAGGCGCCCTGACCAGCGGCGTGCACACCTTCCCGGCTGTCCTACAGTCCTCAGG ACTCTACTCCCTCAGCAGCGTGGTGACCGTGCCCTCCAGCAGCTTGGGCACCCAGACCTACATC TGCAACGTGAATCACAAGCCCAGCAACACCAAGGTGGACAAGAAAGTTGAGCCCAAATCTTGTG ACAAAACTCACACATGCCCACCGTGCCCAGCACCTGAACTCCTGGGGGGACCGTCAGTCTTCCT CTTCCCCCCAAAACCCAAGGACACCCTCATGATCTCCCGGACCCCTGAGGTCACATGCGTGGTG GTGGACGTGAGCCACGAAGACCCTGAGGTCAAGTTCAACTGGTACGTGGACGGCGTGGAGGTG CATAATGCCAAGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTCTCCAACAAAG CCCTCCCAGCCCCCATCGAGAAAACCATCTCCAAAGCCAAAGGGCAGCCCCGAGAACCACAGG TGTACACCCTGCCCCCATCCCGGGATGAGCTGACCAAGAACCAGGTCAGCCTGACCTGCCTGG TCAAAGGCTTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAGCAAGCTCAC CGTGGACAAGAGCAGGTGGCAGCAGGGGAACGTCTTCTCATGCTCCGTGATGCATGAGGCTCT GCACAACCACTACACGCAGAAGAGCCTCTCCCTGTCTCCGGGTAAACCATTTTACCGGAAGAA TGGGTTCCACTAATTAAAAACGGTAAAGTTAAGATATTCCGCATTGGGGACTTCGTTGATGGACT TATGAAGGCGAACCAAGGAAAAGTGAAGAAAACGGGGGATACAGAAGTTTTAGAAGTTGCAGGA ATTCATGCGTTTTCCTTTGACAGGAAGTCCAAGAAGGCCCGTGTAATGGCAGTGAAAGCCGTGAT AAGACACCGTTATTCCGGAAATGTTTATAGAATAGTCTTAAACTCTGGTAGAAAAATAACAATAAC AGAAGGGCATAGCCTATTTGTCTATAGGAACGGGGATCTCGTTGAGGCAACTGGGGAGGATGTC AAAATTGGGGATCTTCTTGCAGTTCCAAGATCAGTAAACCTACCAGAGAAAAGGGAACGCTTGAA TATTGTTGAACTTCTTCTGAATCTCTCACCGGAAGAGACAGAAGATATAATACTTACGATTCCAGT TAAAGGCAGAAAGAACTTCTTCAAGGGAATGTTGAGAACATTACGTTGGATTTTTGGTGAGGAAA AGAGAGTAAGGACAGCGAGCCGCTATCTAAGACACCTTGAAAATCTCGGATACATAAGGTTGAG GAAAATTGGATACGACATCATTGATAAGGAGGGGCTTGAGAAATATAGAACGTTGTACGAGAAAC TTGTTGATGTTGTCCGCTATAATGGCAACAAGAGAGAGTATTTAGTTGAATTTAATGCTGTCCGG GACGTTATCTCACTAATGCCAGAGGAAGAACTGAAGGAATGGCGTATTGGAACTAGAAATGGATT CAGAATGGGTACGTTCGTAGATATTGATGAAGATTTTGCCAAGCTTGGATACGATAGCGGAGTCT ACAGGGTTTATGTAAACGAGGAACTTAAGTTTACGGAATACAGAAAGAAAAAGAATGTATATCACT CTCACATTGTTCCAAAGGATATTCTCAAAGAAACTTTTGGTAAGGTCTTCCAGAAAAATATAAGTT ACAAGAAATTTAGAGAGCTTGTAGAAAATGGAAAACTTGACAGGGAGAAAGCCAAACGCATTGAG TGGTTACTTAACGGAGATATAGTCCTAGATAGAGTCGTAGAGATTAAGAGAGAGTACTATGATGG TTACGTTTACGATCTAAGTGTCGATGAAGATGAGAATTTCCTTGCTGGCTTTGGATTCCTCTATGC ACATAATGACATCCAGATGACCCAGTCTCCATCCTCCCTGTCTGCATCTGTAGGGGACAGAGTCA CCATCACTTGTCGGGCAAGTCAGGGCATCAGAAATTACTTAGCCTGGTATCAGCAAAAACCAGG GAAAGCCCCTAAGCTCCTGATCTATGCTGCATCCACTTTGCAATCAGGGGTCCCATCTCGGTTCA GTGGCAGTGGATCTGGGACAGATTTCACTCTCACCATCAGCAGCCTACAGCCTGAAGATGTTGC AACTTATTACTGTCAAAGGTATAACCGTGCACCGTATACTTTTGGCCAGGGGACCAAGGTGGAAA TCAAACGTACGGTGGCTGCACCATCTGTCTTCATCTTCCCGCCATCTGATGAGCAGTTGAAATCT GGAACTGCCTCTGTTGTGTGCCTGCTGAATAACTTCTATCCCAGAGAGGCCAAAGTACAGTGGA AGGTGGATAACGCCCTCCAATCGGGTAACTCCCAGGAGAGTGTCACAGAGCAGGACAGCAAGG ACAGCACCTACAGCCTCAGCAGCACCCTGACGCTGAGCAAAGCAGACTACGAGAAACACAAAGT CTACGCCTGCGAAGTCACCCATCAGGGCCTGAGCTCGCCCGTCACAAAGAGCTTCAACAGGGG AGAGTGTTGAGCGGCCGCGTTTAAACTGAATGAGCGCGTCCATCCAGACATGATAAGATACATT GATGAGTTTGGACAAACCACAACTAGAATGCAGTGAAAAAAATGCTTTATTTGTGAAATTTGTGAT GCTATTGCTTTATTTGTAACCATTATAAGCTGCAATAAACAAGTTAACAACAACAATTGCATTCATT TTATGTTTCAGGTTCAGGGGGAGGTGTGGGAGGTTTTTTAAAGCAAGTAAAACCTCTACAAATGT GGTATGGCTGATTATGATCCGGCTGCCTCGCGCGTTTCGGTGATGACGGTGAAAACCTCTGACA CATGCAGCTCCCGGAGACGGTCACAGCTTGTCTGTAAGCGGATGCCGGGAGCAGACAAGCCCG TCAGGGCGCGTCAGCGGGTGTTGGCGGGTGTCGGGGCGCAGCCATGACCGGTCGACGGCGCG CCTTTTTTTTTAATTTTTATTTTATTTTATTTTTGACGCGCCGAAGGCGCGATCTGAGCTCGGTACA GCTTGGCTGTGGAATGTGTGTCAGTTAGGGTGTGGAAAGTCCCCAGGCTCCCCAGCAGGCAGA |
| AGTATGCAAAGCATGCATCTCAATTAGTCAGCAACCAGGTGTGGAAAGTCCCCAGGCTCCCCAG CAGGCAGAAGTATGCAAAGCATGCATCTCAATTAGTCAGCAACCATAGTCCCGCCCCTAACTCC GCCCATCCCGCCCCTAACTCCGCCCAGTTCCGCCCATTCTCCGCCCCATGGCTGACTAATTTTT TTATTTATGCAGAGGCCGAGGCCGCCTCGGCCTCTGAGCTATTCCAGAAGTAGTGAGGAGGCTT TTTTGGAGGCCTAGGCTTTTGCAAAAAGCTCCTCGAGGAACTGAAAAACCAGAAAGTTAACTGGT AAGTTTAGTCTTTTTGTCTTTTATTTCAGGTCCCGGATCCGGTGGTGGTGCAAATCAAAGAACTGC TCCTCAGTGGATGTTGCCTTTACTTCTAGGCCTGTACGGAAGTGTTACTTCTGCTCTAAAAGCTG CGGAATTGTACCCGCGGCCTAATACGACTCACTATAGGGACTAGTATGGTTCGACCATTGAACTG CATCGTCGCCGTGTCCCAAAATATGGGGATTGGCAAGAACGGAGACCTACCCTGGCCTCCGCTC AGGAACGAGTTCAAGTACTTCCAAAGAATGACCACAACCTCTTCAGTGGAAGGTAAACAGAATCT GGTGATTATGGGTAGGAAAACCTGGTTCTCCATTCCTGAGAAGAATCGACCTTTAAAGGACAGAA TTAATATAGTTCTCAGTAGAGAACTCAAAGAACCACCACGAGGAGCTCATTTTCTTGCCAAAAGTT TAGATGATGCCTTAAGACTTATTGAACAACCGGAATTGGCAAGTAAAGTAGACATGGTTTGGATA GTCGGAGGCAGTTCTGTTTACCAGGAAGCCATGAATCAACCAGGCCACCTCAGACTCTTTGTGA CAAGGATCATGCAGGAATTTGAAAGTGACACGTTTTTCCCAGAAATTGATTTGGGGAAATATAAA CTTCTCCCAGAATACCCAGGCGTCCTCTCTGAGGTCCAGGAGGAAAAAGGCATCAAGTATAAGT TTGAAGTCTACGAGAAGAAAGACTAAGCGGCCGAGCGCGCGGATCTGGAAACGGGAGATGGGG GAGGCTAACTGAAGCACGGAAGGAGACAATACCGGAAGGAACCCGCGCTATGACGGCAATAAA AAGACAGAATAAAACGCACGGGTGTTGGGTCGTTTGTTCATAAACGCGGGGTTCGGTCCCAGGG CTGGCACTCTGTCGATACCCCACCGAGACCCCATTGGGGCCAATACGCCCGCGTTTCTTCCTTT TCCCCACCCCACCCCCCAAGTTCGGGTGAAGGCCCAGGGCTCGCAGCCAACGTCGGGGCGGC AGGCCCTGCCATAGCCACTGGCCCCGTGGGTTAGGGACGGGGTCCCCCATGGGGAATGGTTTA TGGTTCGTGGGGGTTATTATTTTGGGCGTTGCGTGGGGTCTGGAGATCCCCCGGGCTGCAGGA ATTCCGTTACATTACTTACGGTAAATGGCCCGCCTGGCTGACCGCCCAACGACCCCCGCCCATT GACGTCAATAATGACGTATGTTCCCATAGTAACGCCAATAGGGACTTTCCATTGACGTCAATGGG TGGAGTATTTACGGTAAACTGCCCACTTGGCAGTACATCAAGTGTATCATATGCCAAGTACGCCC CCTATTGACGTCAATGACGGTAAATGGCCCGCCTGGCATTATGCCCAGTACATGACCTTATGGG ACTTTCCTACTTGGCAGTACATCTACGTATTAGTCATCGCTATTACCATGGTGATGCGGTTTTGGC AGTACATCAATGGGCGTGGATAGCGGTTTGACTCACGGGGATTTCCAAGTCTCCACCCCATTGA CGTCAATGGGAGTTTGTTTTGGCACCAAAATCAACGGGACTTTCCAAAATGTCGTAACAACTCCG CCCCATTGACGCAAAGGGCGGAATTCGAGCTCGGTACTCGAGCGGTGTTCCGCGGTCCTCC TCGTATAGAAACTCGGACCACTCTGAGACGAAGGCTCGCGTCCAGGCCAGCACGAAGGAGGCT AAGTGGGAGGGGTAGCGGTCGTTGTCCACTAGGGGGTCCACTCGCTCCAGGGTGTGAAGACAC ATGTCGCCCTCTTCGGCATCAAGGAAGGTGATTGGTTTATAGGTGTAGGCCACGTGACCGGGTG TTCCTGAAGGGGGGCTATAAAAGGGGGTGGGGGCGCGTTCGTCCTCACTCTCTTCCGCATCGC TGTCTGCGAGGGCCAGCTGTTGGGCTCGCGGTTGAGGACAAACTCTTCGCGGTCTTTCCAGTAC TCTTGGATCGGAAACCCGTCGGCCTCCGAACGGTACTCCGCCACCGAGGGACCTGAGCGAGTC CGCATCGACCGGATCGGAAAACCTCTCGACTGTTGGGGTGAGTACTCCCTCTCAAAAGCGGGCA TGACTTCTGCGCTAAGATTGTCAGTTTCCAAAAACGAGGAGGATTTGATATTCACCTGGCCCGCG GTGATGCCTTTGAGGGTGGCCGCGTCCATCTGGTCAGAAAAGACAATCTTTTTGTTGTCAAGCTT GAGGTGTGGCAGGCTTGAGATCTGGCCATACACTTGAGTGACAATGACATCCACTTTGCCTTTCT CTCCACAGGTGTCCACTCCCAGGTCCAACCGGAATTGTACCCGCGGCCAGAGCTTGCGGGCGC CACCGCGGCCGCGGGGATCCAGACATGATAAGATACATTGATGAGTTTGGACAAACCACAACTA GAATGCAGTGAAAAAAATGCTTTATTTGTGAAATTTGTGATGCTATTGCTTTATTTGTAACCATTAT AAGCTGCAATAAACAAGTTAACAACAACAATTGCATTCATTTTATGTTTCAGGTTCAGGGGGAGGT GTGGGAGGTTTTTTCGGATCCTCTTGGCGTAATCATGGTCATAGCTGTTTCCTGTGTGAAATTGT TATCCGCTCACAATTCCACACAACATACGAGCCGGAAGCATAAAGTGTAAAGCCTGGGGTGCCT AATGAGTGAGCTAACTCACATTAATTGCGTTGCGCTCACTGCCCGCTTTCCAGTCGGGAAACCTG TCGTGCCAGCTGCATTAATGAATCGGCCAACGCGCGGGGAAAGGCGGTTTGCGTATTGGGCGC TCTTCCGCTTCCTCGCTCACTGACTCGCTGCGCTCGGTCGTTCGGCTGCGGCGAGCGGTATCAG CTCACTCAAAGGCGGTAATACGGTTATCCACAGAATCAGGGGATAACGCAGGAAAGAACATGTG AGCAAAAGGCCAGCAAAAGGCCAGGAACCGTAAAAAGGCCGCGTTGCTGGCGTTCTTCCATAG GCTCCGCCCCCCTGACGAGCATCACAAAAATCGACGCTCAAGTCAGAGGTGGCGAAACCCGAC AGGACTATAAAGATACCAGGCGTTTCCCCCTGGAAGCTCCCTCGTGCGCTCTCCTGTTCCGACC CTGCCGCTTACCGGATACCTGTCCGCCTTTCTCCCTTCGGGAAGCGTGGCGCTTTCTCATAGCT CACGCTGTAGGTATCTCAGTTCGGTGTAGGTCGTTCGCTCCAAGCTGGGCTGTGTGCACGAACC CCCCGTTCAGCCCGACCGCTGCGCCTTATCCGGTAACTATCGTCTTGAGTCCAACCCGGTAAGA CACGACTTATCGCCACTGGCAGCAGCCACTGGTAACAGGATTAGCAGAGCGAGGTATGTAGGC GGTGCTACAGAGTTCTTGAAGTGGTGGCCTAACTACGGCTACACTAGAAGAACAGTATTTGGTAT CTGCGCTCTGCTGAAGCCAGTTACCTTCGGAAAAAGAGTTGGTAGCTCTTGATCCGGCAAACAA |
| ACCACCGCTGGTAGCGGTGGTTTTTTTGTTTGCAAGCAGCAGATTACGCGCAGAAAAAAAGGAT CTCAAGAAGATCCTTTGATCTTTTCTACGGGGTCTGACGCTCAGTGGAACGAAAACTCACGTTAA GGGATTTTGGTCATGAGATTATCAAAAAGGATCTTCACCTAGATCCTTTTAATTAAAAATGAAGT TTTAAATCAATCTAAAGTATATATGAGTAAACTTGGTCTGACAGTTACCAATGCTTAATCAGTGAG GCACCTATCTCAGCGATCTGTCTATTTCGTTCATCCATAGTTGCCTGACTCCCCGTCGTGTAGAT AACTACGATACGGGAGGGCTTACCATCTGGCCCCAGTGCTGCAATGATACCGCGAGACCCACG CTCACCGGCTCCAGATTTATCAGCAATAAACCAGCCAGCCGGAAGGGCCGAGCGCAGAAGTGG TCCTGCAACTTTATCCGCCTCCATCCAGTCTATTAATTGTTGCCGGGAAGCTAGAGTAAGTAGTT CGCCAGTTAATAGTTTGCGCAACGTTGTTGCCATTGCTACAGGCATCGTGGTGTCACGCTCGTC GTTTGGTATGGCTTCATTCAGCTCCGGTTCCCAACGATCAAGGCGAGTTACATGATCCCCCATGT TGTGCAAAAAAGCGGTTAGCTCCTTCGGTCCTCCGATCGTTGTCAGAAGTAAGTTGGCCGCAGT GTTATCACTCATGGTTATGGCAGCACTGCATAATTCTCTTACTGTCATGCCATCCGTAAGATGCTT TTCTGTGACTGGTGAGTACTCAACCAAGTCATTCTGAGAATAGTGTATGCGGCGACCGAGTTGCT CTTGCCCGGCGTCAATACGGGATAATACCGCGCCACATAGCAGAACTTTAAAAGTGCTCATCATT GGAAAACGTTCTTCGGGGCGAAAACTCTCAAGGATCTTACCGCTGTTGAGATCCAGTTCGATGTA ACCCACTCGTGCACCCAACTGATCTTCAGCATCTTTTACTTTCACCAGCGTTTCTGGGTGAGCAA AAACAGGAAGGCAAAATGCCGCAAAAAAGGGAATAAGGGCGACACGGAAATGTTGAATACTCAT ACTCTTCCTTTTTCAATATTATTGAAGCATTTATCAGGGTTATTGTCTCATGAGCGGATACATATTT GAATGTATTTAGAAAAATAAACAAATAGGGGTTCCGCGCACATTTCCCCGAAAAGTGCCACCTGA CGTCTAAGAAACCATTATTATCATGACATTAACCTATAAAAATAGGCGTATCACGAGGCCCTTTCG TCTCGCGCGTTTCGGTGATGACGGTGAAAACCTCTGACACATGCAGCTCCCGGAGACGGTCACA GCTTGTCTGTAAGCGGATGCCGGGAGCAGACAAGCCCGTCAGGGCGCGTCAGCGGGTGTTGG CGGGTGTCGGGGCTGGCTTAACTATGCGGCATCAGAGCAGATTGTACTGAGAGTGCACCATATG CGGTGTGAAATACCGCACAGATGCGTAAGGAGAAAATACCGCATCAGGCGCCATTCGCCATTCA GGCTGCGCAACTGTTGGGAAGGGCGATCGGTGCGGGCCTCTTCGCTATTACGCCAGCTGGCGA AAGGGGGATGTGCTGCAAGGCGATTAAGTTGGGTAACGCCAGGGTTTTCCCAGTTACGACGTTG TAAAACGACGGCCAGTGAATT |

Table 9. Amino acid sequence of the native Psp-GBD Pol intein sequence with limited flanking sequence information (NCBI Accession No. AAA67132.1) (SEQ ID NO:51)

| |
|---|
| N/SILPEEWVPLIKNGKVKIFRIGDFVDGLMKANQGKVKKTGDTEVLEVAGIHAFSFD RKSKKARVMAVKAVIRHRYSGNVYRIVLNSGRKITITEGHSLFVYRNGDLVEATGED VKIGDLLAVPRSVNLPEKRERLNIVELLLNLSPEETEDIILTIPVKGRKNFFKGMLRTL RWIFGEEKRVRTASRYLRHLENLGYIRLRKIGYDIIDKEGLEKYRTLYEKLVDVVRYN GNKREYLVEFNAVRDVISLMPEEELKEWRIGTRNGFRMGTFVDIDEDFAKLLGYYV SEGSARKWKNQTGGWSYTVRLYNENDEVLDDMEHLAKKFFGKVKRGKNYVEIPK KMAYIIFESLCGTLAENKRVPEVIFTSSKGVRWAFLEGYFIGDGDVHPSKRVRLSTK SELLVNGLVLLLNSLGVSAIKLGYDSGVYRVYVNEELKFTEYRKKKNVYHSHIVPKDI LKETFGKVFQKNISYKKFRELVENGKLDREKAKRIEWLLNGDIVLDRVVEIKREYYD GYVYDLSVDEDENFLAGFGFLYAHN/SYYGYYGYA |

/ represents splice junction, and underlined amino acids represent intein sequences, the remainder represents extein sequence information.

**Example 2. Construction of Immunoglobulin Polyprotein Sequences and Vectors with *Drosophila melanogaster* Hedgehog auto processing domain, C17 and C25 sequences**

A further strategy for the efficient expression of antibody molecules is polyprotein expression, wherein an Hedgehog domain is located between the heavy and light chains, with modification of the Hedgehog domain sequence and/or junction sequences such that there is release of the component proteins without cholesterol addition to the N-terminal protein. Within such constructs, there can be one copy of each of the relevant heavy and light chains, or the light chain can be duplicated to provide at least two light chains, or there can be multiple copies of both heavy and light chains, provided that a functional cleavage sequence is provided to promote separation of each immunoglobulin-derived protein within the polyprotein. A particular cleavage site strategy (e.g., the Hedgehog domain) can be employed more than once, or for multiple cleavage sites each can be independent. Thus a different proteolytic processing sequence or enzyme can be positioned relative to at least one terminus of an immunoglobulin or immunoglobulin-derived protein.

The following oligonucleotides were used for the amplification of the *Drosophila melanogaster* Hedgehog C-terminal auto processing domain (Hh-C), sequences Hh-C17, Hh-C17 truncations (and one with mutation) and Hh-C25 (GenBank accession #L02793.1) using genomic DNA as template and Platinum Taq Hi Fidelity PCR Supermix (Invitrogen). Genomic DNA was prepared from a frozen vial of Drosophila D.Mel-2 cells (Invitrogen, cat. #10831-014).
C17-5': TGCTTCACGCCGGAGAGCAC (SEQ ID NO:141)
C17-full-3' ATTATGGACGACAACCTGGTTGGCAA (SEQ ID NO:142)
C25-actual-3': ATCGTGGCGCCAGCTCTGCG (SEQ ID NO:143)
C17-3': GCAACTGGCGGCCACCGAGT (SEQ ID NO:144)
C17-scya-3': CGCATAGCAACTGGCGGCCA (SEQ ID NO:145)
C17-sc/hn-3': GTTGTGGGCGGCCACCGAGT (SEQ ID NO:146)
PCR run on the following program:

| Step | 1 | 2 | 3 | 4 | 5 | 6 | 7 | 8 |
|---|---|---|---|---|---|---|---|---|
| Temp | 94°C | 94°C | 55°C | 68°C | Go to step 2 (34 times) | 68°C | 4°C | End |
| Time | 2min | 1 min | 1 min | 2.5min | | | 5min | hold |

Oligonucleotide primers were designed to generate the fusion of D2E7 Heavy Chain - Hh-C - D2E7 Light Chain by way of homologous recombination into the pTT3-HcintLC p. horikoshii construct in *E. coli.* By engineering a 40 base pair overhang between PCR generated vector (containing pTT3 vector, heavy chain and light chain regions but not the *P. horikoshii* intein) and the Hh-C domain inserts, the two DNA fragments are mixed and transformed into *E*. *coli* without the benefit of ligation, resulting in *E. coli* homologous recombination of the two fragments into pTT3-HC-Hh-C-LC (in various versions as the initial PCR products dictate).
Hh-C domain homologous recombination primers:

C17-HR5':
CCACTACACGCAGAAGAGCCTCTCCCTGTCTCCGGGTAAATGCTTCACG
CCGGAGAGCAC (SEQ ID NO:147)

C17-full-HR-3':
GCAGCAGGCCCAGCAGCTGGGCGGCACGCGCATGTCCATGCACTGG
CTGTTGATCACCG (SEQ ID NO:148)

C25-actual-HR-3':
GCAGCAGGCCCAGCAGCTGGGCGGGCACGCGCATGTCCATATCGTGGC
GCCAGCTCTGCG (SEQ ID NO:149)

C17-HR3':
GCAGCAGGCCCAGCAGCTGGGCGGGCACGCGCATGTCCATGCAACTGG
CGGCCACCGAGT (SEQ ID NO:150)

C17-scya-HR-3':
GCAGCAGGCCCAGCAGCTGGGCGGGCACGCGCATGTCCATCGCATAGC
AACTGGCGGCCA (SEQ ID NO:151)

C17-sc/hn-HR-3':
GCAGCAGGCCCAGCAGCTGGGCGGGCACGCGCATGTCCATGTTGTGGG
CGGCCACCGAGT (SEQ ID NO:152)

pTT3-HcintLC homologous recombination primers:
pTT3int-HR5': ATGGACATGCGCGTGCCCGCCCAGCTGCTGGGCCTGCTGC (SEQ ID NO:153)
pTT3int-HR3': TTTACCCGGAGACAGGGAGAGGCTCTTCTGCGTGTAGTGGT (SEQ ID NO:154)
PCR for Hh-C domain run on the following program: Pfu-I Hi Fidelity DNA Polymerase (Stratagene) used.

| Step | 1 | 2 | 3 | 4 | 5 | 6 | 7 | 8 |
|---|---|---|---|---|---|---|---|---|
| Temp | 94°C | 94°C | 60°C | 72°C | Go to step 2 (34 times) | 72°C | 4°C | End |
| Time | 2min | 1 min | 1 min | 1.5min | | | 5min | hold |

PCR for the vector run on the following program: Platinum Taq Hi Fidelity Supermix (Invitrogen) used.

| Step | 1 | 2 | 3 | 4 | 5 | 6 | 7 | 8 |
|---|---|---|---|---|---|---|---|---|
| Temp | 94°C | 94°C | 60°C | 68°C | Go to step 2 (24 times) | 68°C | 4°C | End |
| Time | 2min | 30sec | 30sec | 10min | | 5min | hold | |

To achieve homologous recombination of Hh-C domains into pTT3-HcintLC, the following strategy was employed. PCR products were gel purified and each eluted into 50 µl elution buffer (Qiaquick Gel Extraction kit, Qiagen). 3 µl of the vector PCR product was mixed in an eppendorf tube 3 µl of the desired Hint domain PCR product (various versions). The PCR amplification products were transformed into *E. coli* and plated onto LB + Ampicillin plates, incubated at 37ºC overnight, and colonies were grown to 2 ml cultures, plasmid DNA was extracted using the Wizard prep kit (Promega) and the DNA samples were assayed by restriction endonuclease digestion and agarose gel electrophoresis. Clones that produced the correct restriction pattern were analyzed with respect to DNA sequence to confirm that the desired sequence had been produced.

Five expression constructs for D2E7 Heavy Chain - Hh-C - D2E7 Light Chain expression, utilizing the Drosophila melanogaster Hedgehog C-terminal auto-processing domain, were designed: pTT3-HC-Hh-C17-LC; pTT3-HC- Hh-C17-SC-LC; pTT3-HC- Hh-C17-HN -LC; and pTT3-HC-Hh-C25-LC.

Table 27. Sequence of entire plasmid pTT3-D2E7 Heavy Chain -Hh-C17- D2E7 Light Chain (SEQ ID NO:155)

| |
|---|
| 5'- gcggccgctcgaggccggcaaggccggatcccccgacctcgacctctggctaataaaggaaatttattttcattgcaa tagtgtgttggaattttttgtgtctctcactcggaaggacatatgggagggcaaatcatttggtcgagatccctcggagatc tctagctagaggatcgatccccgccccggacgaactaaacctgactacgacatctctgccccttcttcgcggggcagt gcatgtaatcccttcagttggttggtacaacttgccaactgggccctgttccacatgtgacacggggggggaccaaac acaaaggggttctctgactgtagttgacatccttataaatggatgtgcacatttgccaacactgagtggctttcatcctgg agcagactttgcagtctgtggactgcaacacaacattgcctttatgtgtaactcttggctgaagctcttacaccaatgctg ggggacatgtacctcccaggggcccaggaagactacgggaggctacaccaacgtcaatcagaggggcctgtgta gctaccgataagcggaccctcaagagggcattagcaatagtgtttataaggccccttgttaaccctaaacgggtagc atatgcttcccgggtagtagtatatactatccagactaaccctaattcaatagcatatgttacccaacgggaagcatatg ctatcgaattagggttagtaaaagggtcctaaggaacagcgatatctcccaccccatgagctgtcacggttttattaca tggggtcaggattccacgagggtagtgaaccattttagtcacaagggcagtggctgaagatcaaggagcgggcagt gaactctcctgaatcttcgcctgcttcttcattctccttcgtttagctaatagaataactgctgagttgtgaacagtaaggtgt atgtgaggtgctcgaaaacaaggtttcaggtgacgcccccagaataaaatttggacggggggttcagtggtggcatt gtgctatgacaccaatataaccctcacaaaccccttgggcaataaatactagtgtaggaatgaaacattctgaatatct ttaacaatagaaatccatggggtggggacaagccgtaaagactggatgtccatctcacacgaatttatggctatgggc aacacataatcctagtgcaatatgatactggggttattaagatgtgtcccaggcagggaccaagacaggtgaaccat gttgttacactctatttgtaacaaggggaaagagagtggacgccgacagcagcggactccactggttgtctctaacac ccccgaaaattaaacggggctccacgccaatggggcccataaacaaagacaagtggccactcttttttttgaaattgt ggagtgggggcacgcgtcagcccccacacgccgccctgcggttttggactgtaaaataagggtgtaataacttggct gattgtaaccccgctaaccactgcggtcaaaccacttgcccacaaaaccactaatggcaccccggggaatacctgc ataagtaggtgggcgggccaagataggggcgcgattgctgcgatctggaggacaaattacacacacttgcgcctga gcgccaagcacagggttgttggtcctcatattcacgaggtcgctgagagcacggtgggctaatgttgccatgggtagc atatactacccaaatatctggatagcatatgctatcctaatctatatctgggtagcataggctatcctaatctatatctgggt agcatatgctatcctaatctatatctgggtagtatatgctatcctaatttatatctgggtagcataggctatcctaatctatatc tgggtagcatatgctatcctaatctatatctgggtagtatatgctatcctaatctgtatccgggtagcatatgctatcctaata gagattagggtagtatatgctatcctaatttatatctgggtagcatatactacccaaatatctggatagcatatgctatccta atctatatctgggtagcatatgctatcctaatctatatctgggtagcataggctatcctaatctatatctgggtagcatatgct atcctaatctatatctgggtagtatatgctatcctaatttatatctgggtagcataggctatcctaatctatatctgggtagcat atgctatcctaatctatatctgggtagtatatgctatcctaatctgtatccgggtagcatatgctatcctcatgataagctgtc aaacatgagaattttcttgaagacgaaagggcctcgtgatacgcctatttttataggttaatgtcatgataataatggtttct tagacgtcaggtggcacttttcggggaaatgtgcgcggaacccctatttgtttatttttctaaatacattcaaatatgtatcc gctcatgagacaataaccctgataaatgcttcaataatattgaaaaaggaagagtatgagtattcaacatttccgtgtc gcccttattcccttttttgcggcattttgccttcctgtttttgctcacccagaaacgctggtgaaagtaaaagatgctgaagat cagttgggtgcacgagtgggttacatcgaactggatctcaacagcggtaagatccttgagagttttcgccccgaagaa cgttttccaatgatgagcacttttaaagttctgctatgtggcgcggtattatcccgtgttgacgccgggcaagagcaactc ggtcgccgcatacactattctcagaatgacttggttgagtactcaccagtcacagaaaagcatcttacggatggcatga cagtaagagaattatgcagtgctgccataaccatgagtgataacactgcggccaacttacttctgacaacgatcgga ggaccgaaggagctaaccgcttttttgcacaacatgggggatcatgtaactcgccttgatcgttgggaaccggagctg aatgaagccataccaaacgacgagcgtgacaccacgatgcctgcagcaatggcaacaacgttgcgcaaactatta actggcgaactacttactctagcttcccggcaacaattaatagactggatggaggcggataaagttgcaggaccactt ctgcgctcggcccttccggctggctggtttattgctgataaatctggagccggtgagcgtgggtctcgcggtatcattgca gcactggggccagatggtaagccctcccgtatcgtagttatctacacgacggggagtcaggcaactatggatgaacg aaatagacagatcgctgagataggtgcctcactgattaagcattggtaactgtcagaccaagtttactcatatatacttta gattgatttaaaacttcatttttaatttaaaaggatctaggtgaagatcctttttgataatctcatgaccaaaatcccttaacg tgagttttcgttccactgagcgtcagaccccgtagaaaagatcaaaggatcttcttgagatcctttttttctgcgcgtaatct gctgcttgcaaacaaaaaaaccaccgctaccagcggtggtttgtttgccggatcaagagctaccaactctttttccgaa ggtaactggcttcagcagagcgcagataccaaatactgttcttctagtgtagccgtagttaggccaccacttcaagaac tctgtagcaccgcctacatacctcgctctgctaatcctgttaccagtggctgctgccagtggcgataagtcgtgtcttacc gggttggactcaagacgatagttaccggataaggcgcagcggtcgggctgaacggggggttcgtgcacacagccc |
| agcttggagcgaacgacctacaccgaactgagatacctacagcgtgagctatgagaaagcgccacgcttcccgaa gggagaaaggcggacaggtatccggtaagcggcagggtcggaacaggagagcgcacgagggagcttccaggg ggaaacgcctggtatctttatagtcctgtcgggtttcgccacctctgacttgagcgtcgatttttgtgatgctcgtcagggg gcggagcctatggaaaaacgccagcaacgcggcctttttacggttcctggccttttgctggccttttgctcacatgttcttc ctgcgttatcccctgattctgtggataaccgtattaccgcctttgagtgagctgataccgctcgccgcagccgaacgacc gagcgcagcgagtcagtgagcgaggaagcggaagagcgcccaatacgcaaaccgcctctccccgcgcgttggc cgattcattaatgcagctggcacgacaggtttcccgactggaaagcgggcagtgagcgcaacgcaattaatgtgagt tagctcactcattaggcaccccaggctttacactttatgcttccggctcgtatgttgtgtggaattgtgagcggataacaatt tcacacaggaaacagctatgaccatgattacgccaagctctagctagaggtcgaccaattctcatgtttgacagcttat catcgcagatccgggcaacgttgttgccattgctgcaggcgcagaactggtaggtatggaagatctatacattgaatc aatattggcaattagccatattagtcattggttatatagcataaatcaatattggctattggccattgcatacgttgtatctat atcataatatgtacatttatattggctcatgtccaatatgaccgccatgttgacattgattattgactagttattaatagtaatc aattacggggtcattagttcatagcccatatatggagttccgcgttacataacttacggtaaatggcccgcctggctgac cgcccaacgacccccgcccattgacgtcaataatgacgtatgttcccatagtaacgccaatagggactttccattgac gtcaatgggtggagtatttacggtaaactgcccacttggcagtacatcaagtgtatcatatgccaagtccgccccctatt gacgtcaatgacggtaaatggcccgcctggcattatgcccagtacatgaccttacgggactttcctacttggcagtaca tctacgtattagtcatcgctattaccatggtgatgcggttttggcagtacaccaatgggcgtggatagcggtttgactcac ggggatttccaagtctccaccccattgacgtcaatgggagtttgttttggcaccaaaatcaacgggactttccaaaatgt cgtaataaccccgccccgttgacgcaaatgggcggtaggcgtgtacggtgggaggtctatataagcagagctcgttt agtgaaccgtcagatcctcactctcttccgcatcgctgtctgcgagggccagctgttgggctcgcggttgaggacaaac tcttcgcggtctttccagtactcttggatcggaaacccgtcggcctccgaacggtactccgccaccgagggacctgag cgagtccgcatcgaccggatcggaaaacctctcgagaaaggcgtctaaccagtcacagtcgcaaggtaggctgag caccgtggcgggcggcagcgggtggcggtcggggttgtttctggcggaggtgctgctgatgatgtaattaaagtaggc ggtcttgagacggcggatggtcgaggtgaggtgtggcaggcttgagatccagctgttggggtgagtactccctctcaa aagcgggcattacttctgcgctaagattgtcagtttccaaaaacgaggaggatttgatattcacctggcccgatctggcc atacacttgagtgacaatgacatccactttgcctttctctccacaggtgtccactcccaggtccaagtttgggcgccacc atggagtttgggctgagctggctttttcttgtcgcgattttaaaaggtgtccagtgt- gaggtgcagctggtggagtctgggggaggcttggtacagcccggcaggtccctgagactctcctgtgcggcctctgg attcacctttgatgattatgccatgcactgggtccggcaagctccagggaagggcctggaatgggtctcagctatcactt ggaatagtggtcacatagactatgcggactctgtggagggccgattcaccatctccagagacaacgccaagaactc cctgtatctgcaaatgaacagtctgagagctgaggatacggccgtatattactgtgcgaaagtctcgtaccttagcacc gcgtcctcccttgactattggggccaaggtaccctggtcaccgtctcgagtgcgtcgaccaagggcccatcggtcttcc ccctggcaccctcctccaagagcacctctgggggcacagcggccctgggctgcctggtcaaggactacttccccga accggtgacggtgtcgtggaactcaggcgccctgaccagcggcgtgcacaccttcccggctgtcctacagtcctcag gactctactccctcagcagcgtggtgaccgtgccctccagcagcttgggcacccagacctacatctgcaacgtgaatc acaagcccagcaacaccaaggtggacaagaaagttgagcccaaatcttgtgacaaaactcacacatgcccaccg tgcccagcacctgaactcctggggggaccgtcagtcttcctcttccccccaaaacccaaggacaccctcatgatctcc cggacccctgaggtcacatgcgtggtggtggacgtgagccacgaagaccctgaggtcaagttcaactggtacgtgg acggcgtggaggtgcataatgccaagacaaagccgcgggaggagcagtacaacagcacgtaccgtgtggtcag cgtcctcaccgtcctgcaccaggactggctgaatggcaaggagtacaagtgcaaggtctccaacaaagccctccca gcccccatcgagaaaaccatctccaaagccaaagggcagccccgagaaccacaggtgtacaccctgcccccatc ccgggatgagctgaccaagaaccaggtcagcctgacctgcctggtcaaaggcttctatcccagcgacatcgccgtg gagtgggagagcaatgggcagccggagaacaactacaagaccacgcctcccgtgctggactccgacggctccttc ttcctctacagcaagctcaccgtggacaagagcaggtggcagcaggggaacgtcttctcatgctccgtgatgcatga ggctctgcacaaccactacacgcagaagagcctctccctgtctccgggtaaa- tgcttcacgccggagagcacagcgctgctggagagtggagtccggaagccgctcggcgagctctctatcggagatc gtgttttgagcatgaccgccaacggacaggccgtctacagcgaagtgatcctcttcatggaccgcaacctcgagcag atgcaaaactttgtgcagctgcacacggacggtggagcagtgctcacggtgacgccggctcacctggttagcgtttgg cagccggagagccagaagctcacgtttgtgtttgcggatcgcatcgaggagaagaaccaggtgctcgtacgggatg tggagacgggcgagctgaggccccagcgagtcgtcaaggtgggcagtgtgcgcagtaagggcgtggtcgcgccg |
| ctgacccgcgagggcaccattgtggtcaactcggtggccgccagttgctatgcggtgatcaacagccagtcg- atggacatgcgcgtgcccgcccagctgctgggcctgctgctgctgtggttccccggctcgcgatgcgacatccagatg acccagtctccatcctccctgtctgcatctgtaggggacagagtcaccatcacttgtcgggcaagtcagggcatcaga aattacttagcctggtatcagcaaaaaccagggaaagcccctaagctcctgatctatgctgcatccactttgcaatcag gggtcccatctcggttcagtggcagtggatctgggacagatttcactctcaccatcagcagcctacagcctgaagatgt tgcaacttattactgtcaaaggtataaccgtgcaccgtatacttttggccaggggaccaaggtggaaatcaaacgtac ggtggctgcaccatctgtcttcatcttcccgccatctgatgagcagttgaaatctggaactgcctctgttgtgtgcctgctg aataacttctatcccagagaggccaaagtacagtggaaggtggataacgccctccaatcgggtaactcccaggaga gtgtcacagagcaggacagcaaggacagcacctacagcctcagcagcaccctgacgctgagcaaagcagacta cgagaaacacaaagtctacgcctgcgaagtcacccatcagggcctgagctcgcccgtcacaaagagcttcaacag gggagagtgt -3' |

pTT3 Vector-Heavy Chain-Hh-C17-Light Chain

In the following constructs, the only difference from the construct above is the truncation of the C17 region, with the result that cholesterol transferred activity is ablated. The sequences shown are from the end of the D2E7 heavy chain coding region (last 9 base pairs of the HC coding sequence, first line of table) to the 5' end of the D2E7 light chain coding region (first 9 base pairs of LC coding sequence, last line of table).

Table 28. Partial coding sequence of plasmid pTT3-HC-C17-sc-LC (SEQ ID NO:156)

| |
|---|
| Ccgggtaaa- tgcttcacgccggagagcacagcgctgctggagagtggagtccggaagccgctcggcgagctctctatcggagatc gtgttttgagcatgaccgccaacggacaggccgtctacagcgaagtgatcctcttcatggaccgcaacctcgagcag atgcaaaactttgtgcagctgcacacggacggtggagcagtgctcacggtgacgccggctcacctggttagcgtttgg cagccggagagccagaagctcacgtttgtgtttgcggatcgcatcgaggagaagaaccaggtgctcgtacgggatg tggagacgggcgagctgaggccccagcgagtcgtcaaggtgggcagtgtgcgcagtaagggcgtggtcgcgccg ctgacccgcgagggcaccattgtggtcaactcggtggccgccagttgc-atggacatg |

Heavy Chain 3' sequence-Hh-C17-Light Chain 5' sequence

In the following construct, the only difference from construct pTT3-HC-C17-sc-LC above is the mutation of the last two amino acids in the hedgehog C17 region from SC to HN (underlined). The sequences shown are from the end of the D2E7 heavy chain coding region (last 9 base pairs of HC coding sequence, first line of table) to the 5' end of the D2E7 light chain coding region (last line of table).

Table 29. Partial coding sequence from plasmid pTT3-HC-C17-hn-LC (SEQ ID NO:157)

| |
|---|
| ccgggtaaa- tgcttcacgccggagagcacagcgctgctggagagtggagtccggaagccgctcggcgagctctctatcggagatc gtgttttgagcatgaccgccaacggacaggccgtctacagcgaagtgatcctcttcatggaccgcaacctcgagcag atgcaaaactttgtgcagctgcacacggacggtggagcagtgctcacggtgacgccggctcacctggttagcgtttgg cagccggagagccagaagctcacgtttgtgtttgcggatcgcatcgaggagaagaaccaggtgctcgtacgggatg tggagacgggcgagctgaggccccagcgagtcgtcaaggtgggcagtgtgcgcagtaagggcgtggtcgcgccg ctgacccgcgagggcaccattgtggtcaactcggtggccgcccacaac-atggacatg |

Heavy Chain 3' sequence-Hh-C17-Mutation-Light Chain 5' sequence

In the following construct, the full C25 region of the Hint domain is used, rather than the C17. The sequences shown are from the end of the D2E7 heavy chain coding region (last 9 base pairs of HC coding sequence, first line of table) to the 5' end of the D2E7 light chain coding region (first 9 base pairs of LC coding sequence, last line of table)

Table 29B. Partial coding sequence from pTT3-HC-C25-Hint-LC (SEQ ID NO:158)

| |
|---|
| ccgggtaaa- tgcttcacgccggagagcacagcgctgctggagagtggagtccggaagccgctcggcgagctctctatcggagatc gtgttttgagcatgaccgccaacggacaggccgtctacagcgaagtgatcctcttcatggaccgcaacctcgagcag atgcaaaactttgtgcagctgcacacggacggtggagcagtgctcacggtgacgccggctcacctggttagcgtttgg cagccggagagccagaagctcacgtttgtgtttgcggatcgcatcgaggagaagaaccaggtgctcgtacgggatg tggagacgggcgagctgaggccccagcgagtcgtcaaggtgggcagtgtgcgcagtaagggcgtggtcgcgccg ctgacccgcgagggcaccattgtggtcaactcggtggccgccagttgctatgcggtgatcaacagccagtcgctggc ccactggggactggctcccatgcgcctgctgtccacgctggaggcgtggctgcccgccaaggagcagttgcacagtt cgccgaaggtggtgagctcggcgcagcagcagaatggcatccattggtatgccaatgcgctctacaaggtcaagga ctacgttctgccgcagagctggcgccacgat- atggacatg |
| [Heavy Chain 3' sequence-Hh-C25 domain-Light Chain 5' sequence] (SEQ ID NO: 140) |
| Amino acid sequence of Hh-C25 and related constructs (down arrow indicates cleavage site; ↓: Hh-C17 ↓: Hh-C17sc): cftpestallesgvrkplgelsigdrvlsmtangqavysevilfmdrnleqmqnfvqlhtdggavltvtpahlvsvwqpe sqkltfvfadrieeknqvlvrdvetgelrpqrvvkvgsvrskgvvapltregtivvnsvaasc ↓ ya vinsqs↓lahwglapmrllstleawlpakeqlhsspkvvssaqqqngihwyanalykvkdyvlpqswrhd |

**Example 3. Antibody Expression with TEV Recognition Sequence for Proteolytic Processing**

Constructs and expression vectors are generated to direct the expression of antibodies specific for tumor necrosis factor-α, interleukin-12, interleukin-18 and erythropoietin receptor, with a TEV recognition sequence between the immunoglobulin heavy and light chain sequence segments that comprise the antibody of interest. Preferably, constructs include expression vectors comprising an adenovirus major late promoter and cytomegalovirus enhancer directing transcription of the antibody heavy chain of interest which is preceeded by an in-frame leader sequence. The heavy chain coding sequence is linked to an in-frame furin cleavage site and a TEV recognition sequence (E-P-V-Y-F-Q-G) followed by the coding region for the nuclear-localization-region-deleted TEV protease (Ceriani et al. (1998) Plant Molec Biol. 36:239), followed by a second TEV recognition sequence. The second TEV recognition sequence is linked in-frame to the leader sequence for the antibody light chain linked to the coding region for the antibody light chain of interest and stop codon. The coding region is followed by a polyadenylation signal.Relevant sequences are provided herein below.

Table 1. D2E7 (Humira/adalimumab) TEV Expression Vector Complete DNA Sequence (SEQ ID NO:44)

| |
|---|
| GAAGTTCCTATTCCGAAGTTCCTATTCTCTAGACGTTACATAACTTACGGTAAATGGCCCGCCTGG CTGACCGCCCAACGACCCCCGCCCATTGACGTCAATAATGACGTATGTTCCCATAGTAACGCCAAT AGGGACTTTCCATTGACGTCAATGGGTGGAGTATTTACGGTAAACTGCCCACTTGGCAGTACATCA AGTGTATCATATGCCAAGTACGCCCCCTATTGACGTCAATGACGGTAAATGGCCCGCCTGGCATTA TGCCCAGTACATGACCTTATGGGACTTTCCTACTTGGCAGTACATCTACGTATTAGTCATCGCTATT ACCATGGTGATGCGGTTTTGGCAGTACATCAATGGGCGTGGATAGCGGTTTGACTCACGGGGATTT CCAAGTCTCCACCCCATTGACGTCAATGGGAGTTTGTTTTGGCACCAAAATCAACGGGACTTTCCA AAATGTCGTAACAACTCCGCCCCAATGACGCAAATGGGCAGGGAATTCGAGCTCGGTACTCGAGC GGTGTTCCGCGGTCCTCCTCGTATAGAAACTCGGACCACTCTGAGACGAAGGCTCGCGTCCAGGCC AGCACGAAGGAGGCTAAGTGGGAGGGGTAGCGGTCGTTGTCCACTAGGGGGTCCACTCGCTCCAG GGTGTGAAGACACATGTCGCCCTCTTCGGCATCAAGGAAGGTGATTGGTTTATAGGTGTAGGCCAC GTGACCGGGTGTTCCTGAAGGGGGGCTATAAAAGGGGGTGGGGGCGCGTTCGTCCTCACTCTCTTC CGCATCGCTGTCTGCGAGGGCCAGCTGTTGGGCTCGCGGTTGAGGACAAACTCTTCGCGGTCTTTC CAGTACTCTTGGATCGGAAACCCGTCGGCCTCCGAACGGTACTCCGCCACCGAGGGACCTGAGCG AGTCCGCATCGACCGGATCGGAAAACCTCTCGACTGTTGGGGTGAGTACTCCCTCTCAAAAGCGG GCATGACTTCTGCGCTAAGATTGTCAGTTTCCAAAAACGAGGAGGATTTGATATTCACCTGGCCCG CGGTGATGCCTTTGAGGGTGGCCGCGTCCATCTGGTCAGAAAAGACAATCTTTTTGTTGTCAAGCT TGAGGTGTGGCAGGCTTGAGATCTGGCCATACACTTGAGTGACAATGACATCCACTTTGCCTTTCT CTCCACAGGTGTCCACTCCCAGGTCCAACCGGAATTGTACCCGCGGCCAGAGCTTGCCCGGGCGCC ACCATGGAGTTTGGGCTGAGCTGGCTTTTTCTTGTCGCGATTTTAAAAGGTGTCCAGTGTGAGGTG CAGCTGGTGGAGTCTGGGGGAGGCTTGGTACAGCCCGGCAGGTCCCTGAGACTCTCCTGTGCGGC CTCTGGATTCACCTTTGATGATTATGCCATGCACTGGGTCCGGCAAGCTCCAGGGAAGGGCCTGGA ATGGGTCTCAGCTATCACTTGGAATAGTGGTCACATAGACTATGCGGACTCTGTGGAGGGCCGATT CACCATCTCCAGAGACAACGCCAAGAACTCCCTGTATCTGCAAATGAACAGTCTGAGAGCTGAGG ATACGGCCGTATATTACTGTGCGAAAGTCTCGTACCTTAGCACCGCGTCCTCCCTTGACTATTGGG GCCAAGGTACCCTGGTCACCGTCTCGAGTGCGTCGACCAAGGGCCCATCGGTCTTCCCCCTGGCAC CCTCCTCCAAGAGCACCTCTGGGGGCACAGCGGCCCTGGGCTGCCTGGTCAAGGACTACTTCCCCG AACCGGTGACGGTGTCGTGGAACTCAGGCGCCCTGACCAGCGGCGTGCACACCTTCCCGGCTGTCC TACAGTCCTCAGGACTCTACTCCCTCAGCAGCGTGGTGACCGTGCCCTCCAGCAGCTTGGGCACCC AGACCTACATCTGCAACGTGAATCACAAGCCCAGCAACACCAAGGTGGACAAGAAAGTTGAGCCC AAATCTTGTGACAAAACTCACACATGCCCACCGTGCCCAGCACCTGAACTCCTGGGGGGACCGTC AGTCTTCCTCTTCCCCCCAAAACCCAAGGACACCCTCATGATCTCCCGGACCCCTGAGGTCACATG CGTGGTGGTGGACGTGAGCCACGAAGACCCTGAGGTCAAGTTCAACTGGTACGTGGACGGCGTGG |
| AGGTGCATAATGCCAAGACAAAGCCGCGGGAGGAGCAGTACAACAGCACGTACCGTGTGGTCAG CGTCCTCACCGTCCTGCACCAGGACTGGCTGAATGGCAAGGAGTACAAGTGCAAGGTCTCCAACA AAGCCCTCCCAGCCCCCATCGAGAAAACCATCTCCAAAGCCAAAGGGCAGCCCCGAGAACCACAG GTGTACACCCTGCCCCCATCCCGGGATGAGCTGACCAAGAACCAGGTCAGCCTGACCTGCCTGGTC AAAGGCTTCTATCCCAGCGACATCGCCGTGGAGTGGGAGAGCAATGGGCAGCCGGAGAACAACTA CAAGACCACGCCTCCCGTGCTGGACTCCGACGGCTCCTTCTTCCTCTACAGCAAGCTCACCGTGGA CAAGAGCAGGTGGCAGCAGGGGAACGTCTTCTCATGCTCCGTGATGCATGAGGCTCTGCACAACC ACTACACGCAGAAGAGCCTCTCCCTGTCTAGGGGTAAACGCGAACCAGTTTATTTCCAGGGGAGCT TGTTTAAGGGGCCGCGTGATTATAACCCAATATCGAGTGCCATTTGTCATCTAACGAATGAATCTG ATGGGCACACAACATCGTTGTATGGTATTGGTTTTGGCCCTTTCATCATCACAAACAAGCATTTGTT TAGAAGAAATAATGGTACACTGTTAGTTCAATCACTACATGGTGTGTTCAAGGTAAAGAATACCAC AACTTTGCAACAACACCTCATTGATGGGAGGGACATGATGCTCATTCGCATGCCTAAGGATTTCCC ACCATTTCCTCAAAAGCTGAAATTCAGAGAGCCACAAAGGGAAGAGCGCATATGTCTTGTGACAA CCAACTTCCAAACTAAGAGCATGTCTAGCATGGTTTCAGATACTAGTTGCACATTCCCTTCATCTG ATGGTATATTCTGGAAACATTGGATTCAGACCAAGGATGGGCACTGTGGTAGCCCGTTGGTGTCAA CTAGAGATGGGTTTATTGTTGGTATACACTCAGCATCAAATTTCACCAACACAAACAATTATTTTA CAAGTGTGCCGAAAGACTTCATGGATTTATTGACAAATCAAGAGGCGCAGCAATGGGTTAGTGGT TGGCGATTGAATGCTGACTCAGTGTTATGGGGAGGCCACAAAGTTTTCATGAGCAAACCTGAAGA ACCCTTTCAGCCAGTCAAAGAAGCAACTCAACTCATGAGTGAATTAGTCTACTCGCAAGGGATGG ACATGCGCGTGCCCGCCCAGCTGCTGGGCCTGCTGCTGCTGTGGTTCCCCGGCTCGCGATGCGACA TCCAGATGACCCAGTCTCCATCCTCCCTGTCTGCATCTGTAGGGGACAGAGTCACCATCACTTGTC GGGCAAGTCAGGGCATCAGAAATTACTTAGCCTGGTATCAGCAAAAACCAGGGAAAGCCCCTAAG CTCCTGATCTATGCTGCATCCACTTTGCAATCAGGGGTCCCATCTCGGTTCAGTGGCAGTGGATCTG GGACAGATTTCACTCTCACCATCAGCAGCCTACAGCCTGAAGATGTTGCAACTTATTACTGTCAAA GGTATAACCGTGCACCGTATACTTTTGGCCAGGGGACCAAGGTGGAAATCAAACGTACGGTGGCT GCACCATCTGTCTTCATCTTCCCGCCATCTGATGAGCAGTTGAAATCTGGAACTGCCTCTGTTGTGT GCCTGCTGAATAACTTCTATCCCAGAGAGGCCAAAGTACAGTGGAAGGTGGATAACGCCCTCCAA TCGGGTAACTCCCAGGAGAGTGTCACAGAGCAGGACAGCAAGGACAGCACCTACAGCCTCAGCAG CACCCTGACGCTGAGCAAAGCAGACTACGAGAAACACAAAGTCTACGCCTGCGAAGTCACCCATC AGGGCCTGAGCTCGCCCGTCACAAAGAGCTTCAACAGGGGAGAGTGTTGAGCGGCCGCGTTTAAA CTGAATGAGCGCGTCCATCCAGACATGATAAGATACATTGATGAGTTTGGACAAACCACAACTAG AATGCAGTGAAAAAAATGCTTTATTTGTGAAATTTGTGATGCTATTGCTTTATTTGTAACCATTATA AGCTGCAATAAACAAGTTAACAACAACAATTGCATTCATTTTATGTTTCAGGTTCAGGGGGAGGTG TGGGAGGTTTTTTAAAGCAAGTAAAACCTCTACAAATGTGGTATGGCTGATTATGATCCGGCTGCC TCGCGCGTTTCGGTGATGACGGTGAAAACCTCTGACACATGCAGCTCCCGGAGACGGTCACAGCTT GTCTGTAAGCGGATGCCGGGAGCAGACAAGCCCGTCAGGGCGCGTCAGCGGGTGTTGGCGGGTGT CGGGGCGCAGCCATGACCGGTCGACGGCGCGCCTTTTTTTTTAATTTTTATTTTATTTTATTTTTGAC GCGCCGAAGGCGCGATCTGAGCTCGGTACAGCTTGGCTGTGGAATGTGTGTCAGTTAGGGTGTGG AAAGTCCCCAGGCTCCCCAGCAGGCAGAAGTATGCAAAGCATGCATCTCAATTAGTCAGCAACCA GGTGTGGAAAGTCCCCAGGCTCCCCAGCAGGCAGAAGTATGCAAAGCATGCATCTCAATTAGTCA GCAACCATAGTCCCGCCCCTAACTCCGCCCATCCCGCCCCTAACTCCGCCCAGTTCCGCCCATTCTC CGCCCCATGGCTGACTAATTTTTTTTATTTATGCAGAGGCCGAGGCCGCCTCGGCCTCTGAGCTATT CCAGAAGTAGTGAGGAGGCTTTTTTGGAGGCCTAGGCTTTTGCAAAAAGCTCCTCGAGGAACTGA AAAACCAGAAAGTTAACTGGTAAGTTTAGTCTTTTTGTCTTTTATTTCAGGTCCCGGATCCGGTGGT GGTGCAAATCAAAGAACTGCTCCTCAGTGGATGTTGCCTTTACTTCTAGGCCTGTACGGAAGTGTT ACTTCTGCTCTAAAAGCTGCGGAATTGTACCCGCGGCCTAATACGACTCACTATAGGGACTAGTAT GGTTCGACCATTGAACTGCATCGTCGCCGTGTCCCAAAATATGGGGATTGGCAAGAACGGAGACC TACCCTGGCCTCCGCTCAGGAACGAGTTCAAGTACTTCCAAAGAATGACCACAACCTCTTCAGTGG AAGGTAAACAGAATCTGGTGATTATGGGTAGGAAAACCTGGTTCTCCATTCCTGAGAAGAATCGA CCTTTAAAGGACAGAATTAATATAGTTCTCAGTAGAGAACTCAAAGAACCACCACGAGGAGCTCA TTTTCTTGCCAAAAGTTTAGATGATGCCTTAAGACTTATTGAACAACCGGAATTGGCAAGTAAAGT AGACATGGTTTGGATAGTCGGAGGCAGTTCTGTTTACCAGGAAGCCATGAATCAACCAGGCCACC TCAGACTCTTTGTGACAAGGATCATGCAGGAATTTGAAAGTGACACGTTTTTCCCAGAAATTGATT TGGGGAAATATAAACTTCTCCCAGAATACCCAGGCGTCCTCTCTGAGGTCCAGGAGGAAAAAGGC ATCAAGTATAAGTTTGAAGTCTACGAGAAGAAAGACTAAGCGGCCGAGCGCGCGGATCTGGAAAC GGGAGATGGGGGAGGCTAACTGAAGCACGGAAGGAGACAATACCGGAAGGAACCCGCGCTATGA CGGCAATAAAAAGACAGAATAAAACGCACGGGTGTTGGGTCGTTTGTTCATAAACGCGGGGTTCG GTCCCAGGGCTGGCACTCTGTCGATACCCCACCGAGACCCCATTGGGGCCAATACGCCCGCGTTTC TTCCTTTTCCCCACCCCACCCCCCAAGTTCGGGTGAAGGCCCAGGGCTCGCAGCCAACGTCGGGGC GGCAGGCCCTGCCATAGCCACTGGCCCCGTGGGTTAGGGACGGGGTCCCCCATGGGGAATGGTTT |
| ATGGTTCGTGGGGGTTATTATTTGGGCGTTGCGTGGGGTCTGGAGATCCCCCGGCTGCAGGAAT TCCGTTACATTACTTACGGTAAATGGCCCGCCTGGCTGACCGCCCAACGACCCCCGCCCATTGACG TCAATAATGACGTATGTTCCCATAGTAACGCCAATAGGGACTTTCCATTGACGTCAATGGGTGGAG TATTTACGGTAAACTGCCCACTTGGCAGTACATCAAGTGTATCATATGCCAAGTACGCCCCCTATT GACGTCAATGACGGTAAATGGCCCGCCTGGCATTATGCCCAGTACATGACCTTATGGGACTTTCCT ACTTGGCAGTACATCTACGTATTAGTCATCGCTATTACCATGGTGATGCGGTTTTGGCAGTACATC AATGGGCGTGGATAGCGGTTTGACTCACGGGGATTTCCAAGTCTCCACCCCATTGACGTCAATGGG AGTTTGTTTTGGCACCAAAATCAACGGGACTTTCCAAAATGTCGTAACAACTCCGCCCCATTGACG CAAAAGGGCGGAATTCGAGCTCGGTACTCGAGCGGTGTTCCGCGGTCCTCCTCGTATAGAAACTC GGACCACTCTGAGACGAAGGCTCGCGTCCAGGCCAGCACGAAGGAGGCTAAGTGGGAGGGGTAG CGGTCGTTGTCCACTAGGGGGTCCACTCGCTCCAGGGTGTGAAGACACATGTCGCCCTCTTCGGCA TCAAGGAAGGTGATTGGTTTATAGGTGTAGGCCACGTGACCGGGTGTTCCTGAAGGGGGGCTATA AAAGGGGGTGGGGGCGCGTTCGTCCTCACTCTCTTCCGCATCGCTGTCTGCGAGGGCCAGCTGTTG GGCTCGCGGTTGAGGACAAACTCTTCGCGGTCTTTCCAGTACTCTTGGATCGGAAACCCGTCGGCC TCCGAACGGTACTCCGCCACCGAGGGACCTGAGCGAGTCCGCATCGACCGGATCGGAAAACCTCT CGACTGTTGGGGTGAGTACTCCCTCTCAAAAGCGGGCATGACTTCTGCGCTAAGATTGTCAGTTTC CAAAAACGAGGAGGATTTGATATTCACCTGGCCCGCGGTGATGCCTTTGAGGGTGGCCGCGTCCAT CTGGTCAGAAAAGACAATCTTTTTGTTGTCAAGCTTGAGGTGTGGCAGGCTTGAGATCTGGCCATA CACTTGAGTGACAATGACATCCACTTTGCCTTTCTCTCCACAGGTGTCCACTCCCAGGTCCAACCG GAATTGTACCCGCGGCCAGAGCTTGCGGGCGCCACCGCGGCCGCGGGGATCCAGACATGATAAGA TACATTGATGAGTTTGGACAAACCACAACTAGAATGCAGTGAAAAAAATGCTTTATTTGTGAAATT TGTGATGCTATTGCTTTATTTGTAACCATTATAAGCTGCAATAAACAAGTTAACAACAACAATTGC ATTCATTTTATGTTTCAGGTTCAGGGGGAGGTGTGGGAGGTTTTTTCGGATCCTCTTGGCGTAATCA TGGTCATAGCTGTTTCCTGTGTGAAATTGTTATCCGCTCACAATTCCACACAACATACGAGCCGGA AGCATAAAGTGTAAAGCCTGGGGTGCCTAATGAGTGAGCTAACTCACATTAATTGCGTTGCGCTCA CTGCCCGCTTTCCAGTCGGGAAACCTGTCGTGCCAGCTGCATTAATGAATCGGCCAACGCGCGGGG AAAGGCGGTTTGCGTATTGGGCGCTCTTCCGCTTCCTCGCTCACTGACTCGCTGCGCTCGGTCGTTC GGCTGCGGCGAGCGGTATCAGCTCACTCAAAGGCGGTAATACGGTTATCCACAGAATCAGGGGAT AACGCAGGAAAGAACATGTGAGCAAAAGGCCAGCAAAAGGCCAGGAACCGTAAAAAGGCCGCGT TGCTGGCGTTCTTCCATAGGCTCCGCCCCCCTGACGAGCATCACAAAAATCGACGCTCAAGTCAGA GGTGGCGAAACCCGACAGGACTATAAAGATACCAGGCGTTTCCCCCTGGAAGCTCCCTCGTGCGC TCTCCTGTTCCGACCCTGCCGCTTACCGGATACCTGTCCGCCTTTCTCCCTTCGGGAAGCGTGGCGC TTTCTCATAGCTCACGCTGTAGGTATCTCAGTTCGGTGTAGGTCGTTCGCTCCAAGCTGGGCTGTGT GCACGAACCCCCCGTTCAGCCCGACCGCTGCGCCTTATCCGGTAACTATCGTCTTGAGTCCAACCC GGTAAGACACGACTTATCGCCACTGGCAGCAGCCACTGGTAACAGGATTAGCAGAGCGAGGTATG TAGGCGGTGCTACAGAGTTCTTGAAGTGGTGGCCTAACTACGGCTACACTAGAAGAACAGTATTTG GTATCTGCGCTCTGCTGAAGCCAGTTACCTTCGGAAAAAGAGTTGGTAGCTCTTGATCCGGCAAAC AAACCACCGCTGGTAGCGGTGGTTTTTTTGTTTGCAAGCAGCAGATTACGCGCAGAAAAAAAGGA TCTCAAGAAGATCCTTTGATCTTTTCTACGGGGTCTGACGCTCAGTGGAACGAAAACTCACGTTAA GGGATTTTGGTCATGAGATTATCAAAAAGGATCTTCACCTAGATCCTTTTAATTAAAAATGAAGT TTTAAATCAATCTAAAGTATATATGAGTAAACTTGGTCTGACAGTTACCAATGCTTAATCAGTGAG GCACCTATCTCAGCGATCTGTCTATTTCGTTCATCCATAGTTGCCTGACTCCCCGTCGTGTAGATAA CTACGATACGGGAGGGCTTACCATCTGGCCCCAGTGCTGCAATGATACCGCGAGACCCACGCTCA CCGGCTCCAGATTTATCAGCAATAAACCAGCCAGCCGGAAGGGCCGAGCGCAGAAGTGGTCCTGC AACTTTATCCGCCTCCATCCAGTCTATTAATTGTTGCCGGGAAGCTAGAGTAAGTAGTTCGCCAGT TAATAGTTTGCGCAACGTTGTTGCCATTGCTACAGGCATCGTGGTGTCACGCTCGTCGTTTGGTATG GCTTCATTCAGCTCCGGTTCCCAACGATCAAGGCGAGTTACATGATCCCCCATGTTGTGCAAAAAA GCGGTTAGCTCCTTCGGTCCTCCGATCGTTGTCAGAAGTAAGTTGGCCGCAGTGTTATCACTCATG GTTATGGCAGCACTGCATAATTCTCTTACTGTCATGCCATCCGTAAGATGCTTTTCTGTGACTGGTG AGTACTCAACCAAGTCATTCTGAGAATAGTGTATGCGGCGACCGAGTTGCTCTTGCCCGGCGTCAA TACGGGATAATACCGCGCCACATAGCAGAACTTTAAAAGTGCTCATCATTGGAAAACGTTCTTCGG GGCGAAAACTCTCAAGGATCTTACCGCTGTTGAGATCCAGTTCGATGTAACCCACTCGTGCACCCA ACTGATCTTCAGCATCTTTTACTTTCACCAGCGTTTCTGGGTGAGCAAAAACAGGAAGGCAAAATG CCGCAAAAAAGGGAATAAGGGCGACACGGAAATGTTGAATACTCATACTCTTCCTTTTTCAATATT ATTGAAGCATTTATCAGGGTTATTGTCTCATGAGCGGATACATATTTGAATGTATTTAGAAAAATA AACAAATAGGGGTTCCGCGCACATTTCCCCGAAAAGTGCCACCTGACGTCTAAGAAACCATTATTA TCATGACATTAACCTATAAAAATAGGCGTATCACGAGGCCCTTTCGTCTCGCGCGTTTCGGTGATG ACGGTGAAAACCTCTGACACATGCAGCTCCCGGAGACGGTCACAGCTTGTCTGTAAGCGGATGCC GGGAGCAGACAAGCCCGTCAGGGCGCGTCAGCGGGTGTTGGCGGGTGTCGGGGCTGGCTTAACTA TGCGGCATCAGAGCAGATTGTACTGAGAGTGCACCATATGCGGTGTGAAATACCGCACAGATGCG |
| TAAGGAGAAAATACCGCATCAGGCGCCATTCGCCATTCAGGCTGCGCAACTGTTGGGAAGGGCGA TCGGTGCGGGCCTCTTCGCTATTACGCCAGCTGGCGAAAGGGGGATGTGCTGCAAGGCGATTAAG TTGGGTAACGCCAGGGTTTTCCCAGTTACGACGTTGTAAAACGACGGCCAGTGAATT |

Table 2A. ABT-007 TEV Construct: Coding Sequence for Polyprotein (SEQ ID NO:32)

| |
|---|
| ATGGAGTTTGGGCTGAGCTGGCTTTTTCTTGTCGCGATTTTAAAAGGTGTCCAGTGTCAGGTGCAGCTGCAGGAGTCGGG CCCAGGACTGGTGAAGCCTTCGGAGACCCTGTCCCTCACCTGCACTGTCTCTGGTGCCTCCATCAGTAGTTACTACTGGA GCTGGATCCGGCAGCCCCCAGGGAAGGGACTGGAGTGGATTGGGTATATCGGGGGGGAGGGGAGCACCAACTACAACC CCTCCCTCAAGAGTCGAGTCACCATATCAGTAGACACGTCCAAGAACCAGTTCTCCCTGAAGCTGAGGTCTGTGACCGCT GCGGACACGGCCGTGTATTACTGTGCGAGAGAGCGACTGGGGATCGGGGACTACTGGGGCCAGGGAACCCTGGTCACC GTCTCCTCAGCGTCGACCAAGGGCCCATCGGTCTTCCCCCTGGCGCCCTGCTCTAGAAGCACCTCCGAGAGCACAGCGG CCCTGGGCTGCCTGGTCAAGGACTACTTCCCCGAACCGGTGACGGTGTCGTGGAACTCAGGCGCTCTGACCAGCGGCGT GCACACCTTCCCAGCTGTCCTGCAGTCCTCAGGACTCTACTCCCTCAGCAGCGTGGTGACCGTGCCCTCCAGCAACTTCG GCACCCAGACCTACACATGCAACGTAGATCACAAGCCCAGCAACACCAAGGTGGACAAGACAGTTGAGCGCAAATGTTGT GTCGAGTGCCCACCGTGCCCAGCACCACCTGTGGCAGGACCGTCAGTCTTCCTCTTCCCCCCAAAACCCAAGGACACCCT CATGATCTCCCGGACCCCTGAGGTCACGTGCGTGGTGGTGGACGTGAGCCACGAAGACCCCGAGGTCCAGTTCAACTGG TACGTGGACGGCGTGGAGGTGCATAATGCCAAGACAAAGCCACGGGAGGAGCAGTTCAACAGCACGTTCCGTGTGGTCA GCGTCCTCACCGTTGTGCACCAGGACTGGCTGAACGGCAAGGAGTACAAGTGCAAGGTCTCCAACAAAGGCCTCCCAGC CCCCATCGAGAAAACCATCTCCAAAACCAAAGGGCAGCCCCGAGAACCACAGGTGTACACCCTGCCCCCATCCCGGGAG GAGATGACCAAGAACCAGGTCAGCCTGACCTGCCTGGTCAAAGGCTTCTACCCCAGCGACATCGCCGTGGAGTGGGAGA GCAATGGGCAGCCGGAGAACAACTACAAGACCACACCTCCCATGCTGGACTCCGACGGCTCCTTCTTCCTCTACAGCAAG CTCACCGTGGACAAGAGCAGGTGGCAGCAGGGGAACGTCTTCTCATGCTCCGTGATGCATGAGGCTCTGCACAACCACTA CACGCAGAAGAGCCTCTCCCTGTCTAGGGGTAAACGCAACCAGTTTATTTCCAGGGGAGCTTGTTTAAGGGGCCGCGTG ATTATAACCCAATATCGAGTGCCATTTGTCATCTAACGAATGAATCTGATGGGCACACAACATCGTTGTATGGTATTGGTTTT GGCCCTTTCATCATCACAAACAAGCATTTGTTTAGAAGAAATAATGGTACACTGTTAGTTCAATCACTACATGGTGTGTTCAA GGTAAAGAATACCACAACTTTGCAACAACACCTCATTGATGGGAGGGACATGATGCTCATTCGCATGCCTAAGGATTTCCC ACCATTTCCTCAAAAGCTGAAATTCAGAGAGCCACAAAGGGAAGAGCGCATATGTCTTGTGACAACCAACTTCCAAACTAA GAGCATGTCTAGCATGGTTTCAGATACTAGTTGCACATTCCCTTCATCTGATGGTATATTCTGGAAACATTGGATTCAGACC AAGGATGGGCACTGTGGTAGCCCGTTGGTGTCAACTAGAGATGGGTTTATTGTTGGTATACACTCAGCATCAAATTTCACC AACACAAACAATTATTTTACAAGTGTGCCGAAAGACTTCATGGATTTATTGACAAATCAAGAGGCGCAGCAATGGGTTAGTG GTTGGCGATTGAATGCTGACTCAGTGTTATGGGGAGGCCACAAAGTTTTCATGAGCAAACCTGAAGAACCCTTTCAGCCAG TCAAAGAAGCAACTCAACTCATGAGTGAATTAGTCTACTCGCAAGGGATGCGCGTGCCCGCCCAGCTGCTGGGCCTGCTG CTGCTGTGGTTCCCCGGCTCGCGATGCGACATCCAGCTGACCCAATCTCCATCCTCCCTGTCTGCATCTGTAGGAGACAG AGTCACCATCACTTGCCGGGCAAGTCAGGGCATTAGAAATGATTTAGGCTGGTATCAGCAGAAACCAGGGAAAGCCCCTA AGCGCCTGATCTATGCTGCATCCAGTTTGCAAAGTGGGGTCCCATCAAGGTTCAGCGGCAGTGGATCTGGGACAGAATTC ACTCTCACAATCAGCAGCCTGCAGCCTGAAGATTTTGCAACTTATTACTGTCTACAGCATAATACTTACCCTCCGACGTTCG GCCAAGGGACCAAGGTGGAAATCAAACGTACGGTGGCTGCACCATCTGTCTTCATCTTCCCGCCATCTGATGAGCAGTTG AAATCTGGAACTGCCTCTGTTGTGTGCCTGCTGAATAACTTCTATCCCAGAGAGGCCAAAGTACAGTGGAAGGTGGATAAC GCCCTCCAATCGGGTAACTCCCAGGAGAGTGTCACAGAGCAGGACAGCAAGGACAGCACCTACAGCCTCAGCAGCACCC TGACGCTGAGCAAAGCAGACTACGAGAAACACAAAGTCTACGCCTGCGAAGTCACCCATCAGGGCCTGAGCTCGCCCGTC ACAAAGAGCTTCAACAGGGGAGAGTGTTGA |

Table 2B. ABT-007 TEV Polyprotein Amino Acid Sequence (SEQ ID NO:33)

| |
|---|
| |
| * |

Table 2C. Complete ABT-007 TEV Construct Expression Vector Sequence (SEQ ID NO:34)

| |
|---|
| GAAGTTCCTATTCCGAAGTTCCTATTCTCTAGACGTTACATAACTTACGGTAAATGGCCCGCCTG GCTGACCGCCCAACGACCCCCGCCCATTGACGTCAATAATGACGTATGTTCCCATAGTAACGCC AATAGGGACTTTCCATTGACGTCAATGGGTGGAGTATTTACGGTAAACTGCCCACTTGGCAGTAC ATCAAGTGTATCATATGCCAAGTACGCCCCCTATTGACGTCAATGACGGTAAATGGCCCGCCTG GCATTATGCCCAGTACATGACCTTATGGGACTTTCCTACTTGGCAGTACATCTACGTATTAGTCAT CGCTATTACCATGGTGATGCGGTTTTGGCAGTACATCAATGGGCGTGGATAGCGGTTTGACTCA CGGGGATTTCCAAGTCTCCACCCCATTGACGTCAATGGGAGTTTGTTTTGGCACCAAAATCAACG GGACTTTCCAAAATGTCGTAACAACTCCGCCCCAATGACGCAAATGGGCAGGGAATTCGAGCTC GGTACTCGAGCGGTGTTCCGCGGTCCTCCTCGTATAGAAACTCGGACCACTCTGAGACGAAGGC TCGCGTCCAGGCCAGCACGAAGGAGGCTAAGTGGGAGGGGTAGCGGTCGTTGTCCACTAGGG GGTCCACTCGCTCCAGGGTGTGAAGACACATGTCGCCCTCTTCGGCATCAAGGAAGGTGATTGG TTTATAGGTGTAGGCCACGTGACCGGGTGTTCCTGAAGGGGGGCTATAAAAGGGGGTGGGGGC GCGTTCGTCCTCACTCTCTTCCGCATCGCTGTCTGCGAGGGCCAGCTGTTGGGCTCGCGGTTGA GGACAAACTCTTCGCGGTCTTTCCAGTACTCTTGGATCGGAAACCCGTCGGCCTCCGAACGGTA CTCCGCCACCGAGGGACCTGAGCGAGTCCGCATCGACCGGATCGGAAAACCTCTCGACTGTTG GGGTGAGTACTCCCTCTCAAAAGCGGGCATGACTTCTGCGCTAAGATTGTCAGTTTCCAAAAACG AGGAGGATTTGATATTCACCTGGCCCGCGGTGATGCCTTTGAGGGTGGCCGCGTCCATCTGGTC AGAAAAGACAATCTTTTTGTTGTCAAGCTTGAGGTGTGGCAGGCTTGAGATCTGGCCATACACTT GAGTGACAATGACATCCACTTTGCCTTTCTCTCCACAGGTGTCCACTCCCAGGTCCAACCGGAAT TGTACCCGCGGCCAGAGCTTGCCCGGGCGCCACCATGGAGTTTGGGCTGAGCTGGCTTTTTCTT GTCGCGATTTTAAAAGGTGTCCAGTGTCAGGTGCAGCTGCAGGAGTCGGGCCCAGGACTGGTG AAGCCTTCGGAGACCCTGTCCCTCACCTGCACTGTCTCTGGTGCCTCCATCAGTAGTTACTACTG GAGCTGGATCCGGCAGCCCCCAGGGAAGGGACTGGAGTGGATTGGGTATATCGGGGGGGAGG GGAGCACCAACTACAACCCCTCCCTCAAGAGTCGAGTCACCATATCAGTAGACACGTCCAAGAA CCAGTTCTCCCTGAAGCTGAGGTCTGTGACCGCTGCGGACACGGCCGTGTATTACTGTGCGAGA GAGCGACTGGGGATCGGGGACTACTGGGGCCAGGGAACCCTGGTCACCGTCTCCTCAGCGTC GACCAAGGGCCCATCGGTCTTCCCCCTGGCGCCCTGCTCTAGAAGCACCTCCGAGAGCACAGC GGCCCTGGGCTGCCTGGTCAAGGACTACTTCCCCGAACCGGTGACGGTGTCGTGGAACTCAGG CGCTCTGACCAGCGGCGTGCACACCTTCCCAGCTGTCCTGCAGTCCTCAGGACTCTACTCCCTC AGCAGCGTGGTGACCGTGCCCTCCAGCAACTTCGGCACCCAGACCTACACATGCAACGTAGATC ACAAGCCCAGCAACACCAAGGTGGACAAGACAGTTGAGCGCAAATGTTGTGTCGAGTGCCCACC GTGCCCAGCACCACCTGTGGCAGGACCGTCAGTCTTCCTCTTCCCCCCAAAACCCAAGGACACC CTCATGATCTCCCGGACCCCTGAGGTCACGTGCGTGGTGGTGGACGTGAGCCACGAAGACCCC GAGGTCCAGTTCAACTGGTACGTGGACGGCGTGGAGGTGCATAATGCCAAGACAAAGCCACGG GAGGAGCAGTTCAACAGCACGTTCCGTGTGGTCAGCGTCCTCACCGTTGTGCACCAGGACTGG CTGAACGGCAAGGAGTACAAGTGCAAGGTCTCCAACAAAGGCCTCCCAGCCCCCATCGAGAAAA CCATCTCCAAAACCAAAGGGCAGCCCCGAGAACCACAGGTGTACACCCTGCCCCCATCCCGGG AGGAGATGACCAAGAACCAGGTCAGCCTGACCTGCCTGGTCAAAGGCTTCTACCCCAGCGACAT CGCCGTGGAGTGGGAGAGCAATGGGCAGCCGGAGAACAACTACAAGACCACACCTCCCATGCT GGACTCCGACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACGCAGAAGAGC CTCTCCCTGTCTAGGGGTAAACGCGAACCAGTTTATTTCCAGGGGAGCTTGTTTAAGGGGCCGC |
| GTGATTATAACCCAATATCGAGTGCCATTTGTCATCTAACGAATGAATCTGATGGCACACAACAT CGTTGTATGGTATTGGTTTTGGCCCTTTCATCATCACAAACAAGCATTTGTTTAGAAGAAATAATG GTACACTGTTAGTTCAATCACTACATGGTGTGTTCAAGGTAAAGAATACCACAACTTTGCAACAAC ACCTCATTGATGGGAGGGACATGATGCTCATTCGCATGCCTAAGGATTTCCCACCATTTCCTCAA AAGCTGAAATTCAGAGAGCCACAAAGGGAAGAGCGCATATGTCTTGTGACAACCAACTTCCAAAC TAAGAGCATGTCTAGCATGGTTTCAGATACTAGTTGCACATTCCCTTCATCTGATGGTATATTCTG GAAACATTGGATTCAGACCAAGGATGGGCACTGTGGTAGCCCGTTGGTGTCAACTAGAGATGGG TTTATTGTTGGTATACACTCAGCATCAAATTTCACCAACACAAACAATTATTTTACAAGTGTGCCGA AAGACTTCATGGATTTATTGACAAATCAAGAGGCGCAGCAATGGGTTAGTGGTTGGCGATTGAAT GCTGACTCAGTGTTATGGGGAGGCCACAAAGTTTTCATGAGCAAACCTGAAGAACCCTTTCAGC CAGTCAAAGAAGCAACTCAACTCATGAGTGAATTAGTCTACTCGCAAGGGATGCGCGTGCCCGC CCAGCTGCTGGGCCTGCTGCTGCTGTGGTTCCCCGGCTCGCGATGCGACATCCAGCTGACCCA ATCTCCATCCTCCCTGTCTGCATCTGTAGGAGACAGAGTCACCATCACTTGCCGGGCAAGTCAG GGCATTAGAAATGATTTAGGCTGGTATCAGCAGAAACCAGGGAAAGCCCCTAAGCGCCTGATCT ATGCTGCATCCAGTTTGCAAAGTGGGGTCCCATCAAGGTTCAGCGGCAGTGGATCTGGGACAGA ATTCACTCTCACAATCAGCAGCCTGCAGCCTGAAGATTTTGCAACTTATTACTGTCTACAGCATAA TACTTACCCTCCGACGTTCGGCCAAGGGACCAAGGTGGAAATCAAACGTACGGTGGCTGCACCA TCTGTCTTCATCTTCCCGCCATCTGATGAGCAGTTGAAATCTGGAACTGCCTCTGTTGTGTGCCT GCTGAATAACTTCTATCCCAGAGAGGCCAAAGTACAGTGGAAGGTGGATAACGCCCTCCAATCG GGTAACTCCCAGGAGAGTGTCACAGAGCAGGACAGCAAGGACAGCACCTACAGCCTCAGCAGC ACCCTGACGCTGAGCAAAGCAGACTACGAGAAACACAAAGTCTACGCCTGCGAAGTCACCCATC AGGGCCTGAGCTCGCCCGTCACAAAGAGCTTCAACAGGGGAGAGTGTTGAGCGGCCGCGTTTA AACTGAATGAGCGCGTCCATCCAGACATGATAAGATACATTGATGAGTTTGGACAAACCACAACT AGAATGCAGTGAAAAAAATGCTTTATTTGTGAAATTTGTGATGCTATTGCTTTATTTGTAACCATTA TAAGCTGCAATAAACAAGTTAACAACAACAATTGCATTCATTTTATGTTTCAGGTTCAGGGGGAGG TGTGGGAGGTTTTTTAAAGCAAGTAAAACCTCTACAAATGTGGTATGGCTGATTATGATCCGGCT GCCTCGCGCGTTTCGGTGATGACGGTGAAAACCTCTGACACATGCAGCTCCCGGAGACGGTCA CAGCTTGTCTGTAAGCGGATGCCGGGAGCAGACAAGCCCGTCAGGGCGCGTCAGCGGGTGTTG GCGGGTGTCGGGGCGCAGCCATGACCGGTCGACGGCGCGCCTTTTTTTTTAATTTTTATTTTATT TTATTTTTGACGCGCCGAAGGCGCGATCTGAGCTCGGTACAGCTTGGCTGTGGAATGTGTGTCA GTTAGGGTGTGGAAAGTCCCCAGGCTCCCCAGCAGGCAGAAGTATGCAAAGCATGCATCTCAAT TAGTCAGCAACCAGGTGTGGAAAGTCCCCAGGCTCCCCAGCAGGCAGAAGTATGCAAAGCATG CATCTCAATTAGTCAGCAACCATAGTCCCGCCCCTAACTCCGCCCATCCCGCCCCTAACTCCGC CCAGTTCCGCCCATTCTCCGCCCCATGGCTGACTAATTTTTTTTATTTATGCAGAGGCCGAGGCC GCCTCGGCCTCTGAGCTATTCCAGAAGTAGTGAGGAGGCTTTTTTGGAGGCCTAGGCTTTTGCA AAAAGCTCCTCGAGGAACTGAAAAACCAGAAAGTTAACTGGTAAGTTTAGTCTTTTTGTCTTTTAT TTCAGGTCCCGGATCCGGTGGTGGTGCAAATCAAAGAACTGCTCCTCAGTGGATGTTGCCTTTA CTTCTAGGCCTGTACGGAAGTGTTACTTCTGCTCTAAAAGCTGCGGAATTGTACCCGCGGCCTAA TACGACTCACTATAGGGACTAGTATGGTTCGACCATTGAACTGCATCGTCGCCGTGTCCCAAAAT ATGGGGATTGGCAAGAACGGAGACCTACCCTGGCCTCCGCTCAGGAACGAGTTCAAGTACTTCC AAAGAATGACCACAACCTCTTCAGTGGAAGGTAAACAGAATCTGGTGATTATGGGTAGGAAAACC TGGTTCTCCATTCCTGAGAAGAATCGACCTTTAAAGGACAGAATTAATATAGTTCTCAGTAGAGAA CTCAAAGAACCACCACGAGGAGCTCATTTTCTTGCCAAAAGTTTAGATGATGCCTTAAGACTTATT GAACAACCGGAATTGGCAAGTAAAGTAGACATGGTTTGGATAGTCGGAGGCAGTTCTGTTTACCA GGAAGCCATGAATCAACCAGGCCACCTCAGACTCTTTGTGACAAGGATCATGCAGGAATTTGAAA GTGACACGTTTTTCCCAGAAATTGATTTGGGGAAATATAAACTTCTCCCAGAATACCCAGGCGTC CTCTCTGAGGTCCAGGAGGAAAAAGGCATCAAGTATAAGTTTGAAGTCTACGAGAAGAAAGACTA AGCGGCCGAGCGCGCGGATCTGGAAACGGGAGATGGGGGAGGCTAACTGAAGCACGGAAGGA GACAATACCGGAAGGAACCCGCGCTATGACGGCAATAAAAAGACAGAATAAAACGCACGGGTGT TGGGTCGTTTGTTCATAAACGCGGGGTTCGGTCCCAGGGCTGGCACTCTGTCGATACCCCACCG AGACCCCATTGGGGCCAATACGCCCGCGTTTCTTCCTTTTCCCCACCCCACCCCCCAAGTTCGG GTGAAGGCCCAGGGCTCGCAGCCAACGTCGGGGCGGCAGGCCCTGCCATAGCCACTGGCCCC GTGGGTTAGGGACGGGGTCCCCCATGGGGAATGGTTTATGGTTCGTGGGGGTTATTATTTTGGG CGTTGCGTGGGGTCTGGAGATCCCCCGGGCTGCAGGAATTCCGTTACATTACTTACGGTAAATG GCCCGCCTGGCTGACCGCCCAACGACCCCCGCCCATTGACGTCAATAATGACGTATGTTCCCAT AGTAACGCCAATAGGGACTTTCCATTGACGTCAATGGGTGGAGTATTTACGGTAAACTGCCCACT TGGCAGTACATCAAGTGTATCATATGCCAAGTACGCCCCCTATTGACGTCAATGACGGTAAATGG CCCGCCTGGCATTATGCCCAGTACATGACCTTATGGGACTTTCCTACTTGGCAGTACATCTACGT ATTAGTCATCGCTATTACCATGGTGATGCGGTTTTGGCAGTACATCAATGGGCGTGGATAGCGGT TTGACTCACGGGGATTTCCAAGTCTCCACCCCATTGACGTCAATGGGAGTTTGTTTTGGCACCAA |
| AATCAACGGGACTTTCCAAAATGTCGTAACAACTCCGCCCCATTGACGCAAAGGGCGGGAATT CGAGCTCGGTACTCGAGCGGTGTTCCGCGGTCCTCCTCGTATAGAAACTCGGACCACTCTGAGA CGAAGGCTCGCGTCCAGGCCAGCACGAAGGAGGCTAAGTGGGAGGGGTAGCGGTCGTTGTCC ACTAGGGGGTCCACTCGCTCCAGGGTGTGAAGACACATGTCGCCCTCTTCGGCATCAAGGAAG GTGATTGGTTTATAGGTGTAGGCCACGTGACCGGGTGTTCCTGAAGGGGGGCTATAAAAGGGG GTGGGGGCGCGTTCGTCCTCACTCTCTTCCGCATCGCTGTCTGCGAGGGCCAGCTGTTGGGCT CGCGGTTGAGGACAAACTCTTCGCGGTCTTTCCAGTACTCTTGGATCGGAAACCCGTCGGCCTC CGAACGGTACTCCGCCACCGAGGGACCTGAGCGAGTCCGCATCGACCGGATCGGAAAACCTCT CGACTGTTGGGGTGAGTACTCCCTCTCAAAAGCGGGCATGACTTCTGCGCTAAGATTGTCAGTTT CCAAAAACGAGGAGGATTTGATATTCACCTGGCCCGCGGTGATGCCTTTGAGGGTGGCCGCGTC CATCTGGTCAGAAAAGACAATCTTTTTGTTGTCAAGCTTGAGGTGTGGCAGGCTTGAGATCTGGC CATACACTTGAGTGACAATGACATCCACTTTGCCTTTCTCTCCACAGGTGTCCACTCCCAGGTCC AACCGGAATTGTACCCGCGGCCAGAGCTTGCGGGCGCCACCGCGGCCGCGGGGATCCAGACA TGATAAGATACATTGATGAGTTTGGACAAACCACAACTAGAATGCAGTGAAAAAAATGCTTTATTT GTGAAATTTGTGATGCTATTGCTTTATTTGTAACCATTATAAGCTGCAATAAACAAGTTAACAACAA CAATTGCATTCATTTTATGTTTCAGGTTCAGGGGGAGGTGTGGGAGGTTTTTTCGGATCCTCTTG GCGTAATCATGGTCATAGCTGTTTCCTGTGTGAAATTGTTATCCGCTCACAATTCCACACAACATA CGAGCCGGAAGCATAAAGTGTAAAGCCTGGGGTGCCTAATGAGTGAGCTAACTCACATTAATTG CGTTGCGCTCACTGCCCGCTTTCCAGTCGGGAAACCTGTCGTGCCAGCTGCATTAATGAATCGG CCAACGCGCGGGGAAAGGCGGTTTGCGTATTGGGCGCTCTTCCGCTTCCTCGCTCACTGACTC GCTGCGCTCGGTCGTTCGGCTGCGGCGAGCGGTATCAGCTCACTCAAAGGCGGTAATACGGTT ATCCACAGAATCAGGGGATAACGCAGGAAAGAACATGTGAGCAAAAGGCCAGCAAAAGGCCAG GAACCGTAAAAAGGCCGCGTTGCTGGCGTTCTTCCATAGGCTCCGCCCCCCTGACGAGCATCAC AAAAATCGACGCTCAAGTCAGAGGTGGCGAAACCCGACAGGACTATAAAGATACCAGGCGTTTC CCCCTGGAAGCTCCCTCGTGCGCTCTCCTGTTCCGACCCTGCCGCTTACCGGATACCTGTCCGC CTTTCTCCCTTCGGGAAGCGTGGCGCTTTCTCATAGCTCACGCTGTAGGTATCTCAGTTCGGTGT AGGTCGTTCGCTCCAAGCTGGGCTGTGTGCACGAACCCCCCGTTCAGCCCGACCGCTGCGCCT TATCCGGTAACTATCGTCTTGAGTCCAACCCGGTAAGACACGACTTATCGCCACTGGCAGCAGC CACTGGTAACAGGATTAGCAGAGCGAGGTATGTAGGCGGTGCTACAGAGTTCTTGAAGTGGTGG CCTAACTACGGCTACACTAGAAGAACAGTATTTGGTATCTGCGCTCTGCTGAAGCCAGTTACCTT CGGAAAAAGAGTTGGTAGCTCTTGATCCGGCAAACAAACCACCGCTGGTAGCGGTGGTTTTTTT GTTTGCAAGCAGCAGATTACGCGCAGAAAAAAAGGATCTCAAGAAGATCCTTTGATCTTTTCTAC GGGGTCTGACGCTCAGTGGAACGAAAACTCACGTTAAGGGATTTTGGTCATGAGATTATCAAAAA GGATCTTCACCTAGATCCTTTTAATTAAAAATGAAGTTTTAAATCAATCTAAAGTATATATGAGTA AACTTGGTCTGACAGTTACCAATGCTTAATCAGTGAGGCACCTATCTCAGCGATCTGTCTATTTC GTTCATCCATAGTTGCCTGACTCCCCGTCGTGTAGATAACTACGATACGGGAGGGCTTACCATCT GGCCCCAGTGCTGCAATGATACCGCGAGACCCACGCTCACCGGCTCCAGATTTATCAGCAATAA ACCAGCCAGCCGGAAGGGCCGAGCGCAGAAGTGGTCCTGCAACTTTATCCGCCTCCATCCAGT CTATTAATTGTTGCCGGGAAGCTAGAGTAAGTAGTTCGCCAGTTAATAGTTTGCGCAACGTTGTT GCCATTGCTACAGGCATCGTGGTGTCACGCTCGTCGTTTGGTATGGCTTCATTCAGCTCCGGTT CCCAACGATCAAGGCGAGTTACATGATCCCCCATGTTGTGCAAAAAAGCGGTTAGCTCCTTCGG TCCTCCGATCGTTGTCAGAAGTAAGTTGGCCGCAGTGTTATCACTCATGGTTATGGCAGCACTGC ATAATTCTCTTACTGTCATGCCATCCGTAAGATGCTTTTCTGTGACTGGTGAGTACTCAACCAAGT CATTCTGAGAATAGTGTATGCGGCGACCGAGTTGCTCTTGCCCGGCGTCAATACGGGATAATAC CGCGCCACATAGCAGAACTTTAAAAGTGCTCATCATTGGAAAACGTTCTTCGGGGCGAAAACTCT CAAGGATCTTACCGCTGTTGAGATCCAGTTCGATGTAACCCACTCGTGCACCCAACTGATCTTCA GCATCTTTTACTTTCACCAGCGTTTCTGGGTGAGCAAAAACAGGAAGGCAAAATGCCGCAAAAAA GGGAATAAGGGCGACACGGAAATGTTGAATACTCATACTCTTCCTTTTTCAATATTATTGAAGCAT TTATCAGGGTTATTGTCTCATGAGCGGATACATATTTGAATGTATTTAGAAAAATAAACAAATAGG GGTTCCGCGCACATTTCCCCGAAAAGTGCCACCTGACGTCTAAGAAACCATTATTATCATGACAT TAACCTATAAAAATAGGCGTATCACGAGGCCCTTTCGTCTCGCGCGTTTCGGTGATGACGGTGAA AACCTCTGACACATGCAGCTCCCGGAGACGGTCACAGCTTGTCTGTAAGCGGATGCCGGGAGC AGACAAGCCCGTCAGGGCGCGTCAGCGGGTGTTGGCGGGTGTCGGGGCTGGCTTAACTATGC GGCATCAGAGCAGATTGTACTGAGAGTGCACCATATGCGGTGTGAAATACCGCACAGATGCGTA AGGAGAAAATACCGCATCAGGCGCCATTCGCCATTCAGGCTGCGCAACTGTTGGGAAGGGCGA TCGGTGCGGGCCTCTTCGCTATTACGCCAGCTGGCGAAAGGGGGATGTGCTGCAAGGCGATTA AGTTGGGTAACGCCAGGGTTTTCCCAGTTACGACGTTGTAAAACGACGGCCAGTGAATT |

Table 3A. Coding Sequence for ABT-874 (J695) TEV Polyprotein (SEQ ID NO:35)

| |
|---|
| ATGGAGTTTGGGCTGAGCTGGCTTTTTCTTGTCGCGATTTTAAAAGGTGTCCAGTGTCAGGTGCAGCTGGTGGAGTCTGG GGGAGGCGTGGTCCAGCCTGGGAGGTCCCTGAGACTCTCCTGTGCAGCGTCTGGATTCACCTTCAGTAGCTATGGCATG CACTGGGTCCGCCAGGCTCCAGGCAAGGGGCTGGAGTGGGTGGCATTTATACGGTATGATGGAAGTAATAAATACTATGC AGACTCCGTGAAGGGCCGATTCACCATCTCCAGAGACAATTCCAAGAACACGCTGTATCTGCAGATGAACAGCCTGAGAG CTGAGGACACGGCTGTGTATTACTGTAAGACCCATGGTAGCCATGACAACTGGGGCCAAGGGACAATGGTCACCGTCTCT TCAGCGTCGACCAAGGGCCCATCGGTCTTCCCCCTGGCACCCTCCTCCAAGAGCACCTCTGGGGGCACAGCGGCCCTGG GCTGCCTGGTCAAGGACTACTTCCCCGAACCGGTGACGGTGTCGTGGAACTCAGGCGCCCTGACCAGCGGCGTGCACAC CTTCCCGGCTGTCCTACAGTCCTCAGGACTCTACTCCCTCAGCAGCGTGGTGACCGTGCCCTCCAGCAGCTTGGGCACCC AGACCTACATCTGCAACGTGAATCACAAGCCCAGCAACACCAAGGTGGACAAGAAAGTTGAGCCCAAATCTTGTGACAAAA CTCACACATGCCCACCGTGCCCAGCACCTGAACTCCTGGGGGGACCGTCAGTCTTCCTCTTCCCCCCAAAACCCAAGGAC ACCCTCATGATCTCCCGGACCCCTGAGGTCACATGCGTGGTGGTGGACGTGAGCCACGAAGACCCTGAGGTCAAGTTCAA CTGGTACGTGGACGGCGTGGAGGTGCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGTGCAAGGTCTCCAACAAAGCCCTCCC AGCCCCCATCGAGAAAACCATCTCCAAAGCCAAAGGGCAGCCCCGAGAACCACAGGTGTACACCCTGCCCCCATCCCGC GAGGAGATGACCAAGAACCAGGTCAGCCTGACCTGCCTGGTCAAAGGCTTCTATCCCAGCGACATCGCCGTGGAGTGGG AGAGCAATGGGCAGCCGGAGAACAACTACAAGACCACGCCTCCCGTGCTGGACTCCGACGGCTCCTTCTTCCTCTACAGC AAGCTCACCGTGGACAAGAGCAGGTGGCAGCAGGGGAACGTCTTCTCATGCTCCGTGATGCATGAGGCTCTGCACAACC ACTACACGCAGAAGAGCCTCTCCCTGTCTAGGGGTAAACGCGAACCAGTTTATTTCCAGGGGAGCTTGTTTAAGGGGCCG CGTGATTATAACCCAATATCGAGTGCCATTTGTCATCTAACGAATGAATCTGATGGGCACACAACATCGTTGTATGGTATTG GTTTTGGCCCTTTCATCATCACAAACAAGCATTTGTTTAGAAGAAATAATGGTACACTGTTAGTTCAATCACTACATGGTGTG TTCAAGGTAAAGAATACCACAACTTTGCAACAACACCTCATTGATGGGAGGGACATGATGCTCATTCGCATGCCTAAGGAT TTCCCACCATTTCCTCAAAAGCTGAAATTCAGAGAGCCACAAAGGGAAGAGCGCATATGTCTTGTGACAACCAACTTCCAA ACTAAGAGCATGTCTAGCATGGTTTCAGATACTAGTTGCACATTCCCTTCATCTGATGGTATATTCTGGAAACATTGGATTC AGACCAAGGATGGGCACTGTGGTAGCCCGTTGGTGTCAACTAGAGATGGGTTTATTGTTGGTATACACTCAGCATCAAATT TCACCAACACAAACAATTATTTTACAAGTGTGCCGAAAGACTTCATGGATTTATTGACAAATCAAGAGGCGCAGCAATGGGT TAGTGGTTGGCGATTGAATGCTGACTCAGTGTTATGGGGAGGCCACAAAGTTTTCATGAGCAAACCTGAAGAACCCTTTCA GCCAGTCAAAGAAGCAACTCAACTCATGAGTGAATTAGTCTACTCGCAAGGGATGACTTGGACCCCACTCCTCTTCCTCAC CCTCCTCCTCCACTGCACAGGAAGCTTATCCCAGTCTGTGCTGACTCAGCCCCCCTCAGTGTCTGGGGCCCCCGGGCAGA GAGTCACCATCTCTTGTTCTGGAAGCAGATCCAACATCGGCAGTAATACTGTAAAGTGGTATCAGCAGCTCCCAGGAACGG CCCCCAAACTCCTCATCTATTACAATGATCAGCGGCCCTCAGGGGTCCCTGACCGATTCTCTGGATCCAAGTCTGGCACCT CAGCCTCCCTCGCCATCACTGGGCTCCAGGCTGAAGACGAGGCTGACTATTACTGCCAGTCATATGACAGATACACCCAC CCCGCCCTGCTCTTCGGAACTGGGACCAAGGTCACAGTACTAGGTCAGCCCAAGGCTGCCCCCTCGGTCACTCTGTTCCC GCCCTCCTCTGAGGAGCTTCAAGCCAACAAGGCCACACTGGTGTGTCTCATAAGTGACTTCTACCCGGGAGCCGTGACAG TGGCCTGGAAGGCAGATAGCAGCCCCGTCAAGGCGGGAGTGGAGACCACCACACCCTCCAAACAAAGCAACAACAAGTA CGCGGCCAGCAGCTACCTGAGCCTGACGCCTGAGCAGTGGAAGTCCCACAGAAGCTACAGCTGCCAGGTCACGCATGAA GGGAGCACCGTGGAGAAGACAGTGGCCCCTACAGAATGTTCATGA |

Table 3B. Amino Acid Sequence of ABT-874 (J695) TEV Polyprotein (SEQ ID NO:36)

| |
|---|
| * |

Table 3C. Complete Nucleotide Sequence of ABT-874 (J695) TEV Expression Vector (SEQ ID NO:37)

| |
|---|
| GAAGTTCCTATTCCGAAGTTCCTATTCTCTAGACGTTACATAACTTACGGTAAATGGCCCGCCTGG CTGACCGCCCAACGACCCCCGCCCATTGACGTCAATAATGACGTATGTTCCCATAGTAACGCCAAT AGGGACTTTCCATTGACGTCAATGGGTGGAGTATTTACGGTAAACTGCCCACTTGGCAGTACATCA AGTGTATCATATGCCAAGTACGCCCCCTATTGACGTCAATGACGGTAAATGGCCCGCCTGGCATTA TGCCCAGTACATGACCTTATGGGACTTTCCTACTTGGCAGTACATCTACGTATTAGTCATCGCTATT ACCATGGTGATGCGGTTTTGGCAGTACATCAATGGGCGTGGATAGCGGTTTGACTCACGGGGATTT CCAAGTCTCCACCCCATTGACGTCAATGGGAGTTTGTTTTGGCACCAAAATCAACGGGACTTTCCA AAATGTCGTAACAACTCCGCCCCAATGACGCAAATGGGCAGGGAATTCGAGCTCGGTACTCGAGC GGTGTTCCGCGGTCCTCCTCGTATAGAAACTCGGACCACTCTGAGACGAAGGCTCGCGTCCAGGCC AGCACGAAGGAGGCTAAGTGGGAGGGGTAGCGGTCGTTGTCCACTAGGGGGTCCACTCGCTCCAG GGTGTGAAGACACATGTCGCCCTCTTCGGCATCAAGGAAGGTGATTGGTTTATAGGTGTAGGCCAC GTGACCGGGTGTTCCTGAAGGGGGGCTATAAAAGGGGGTGGGGGCGCGTTCGTCCTCACTCTCTTC CGCATCGCTGTCTGCGAGGGCCAGCTGTTGGGCTCGCGGTTGAGGACAAACTCTTCGCGGTCTTTC CAGTACTCTTGGATCGGAAACCCGTCGGCCTCCGAACGGTACTCCGCCACCGAGGGACCTGAGCG AGTCCGCATCGACCGGATCGGAAAACCTCTCGACTGTTGGGGTGAGTACTCCCTCTCAAAAGCGG GCATGACTTCTGCGCTAAGATTGTCAGTTTCCAAAAACGAGGAGGATTTGATATTCACCTGGCCCG CGGTGATGCCTTTGAGGGTGGCCGCGTCCATCTGGTCAGAAAAGACAATCTTTTTGTTGTCAAGCT TGAGGTGTGGCAGGCTTGAGATCTGGCCATACACTTGAGTGACAATGACATCCACTTTGCCTTTCT CTCCACAGGTGTCCACTCCCAGGTCCAACCGGAATTGTACCCGCGGCCAGAGCTTGCCCGGGCGCC ACCATGGAGTTTGGGCTGAGCTGGCTTTTTCTTGTCGCGATTTTAAAAGGTGTCCAGTGTCAGGTG CAGCTGGTGGAGTCTGGGGGAGGCGTGGTCCAGCCTGGGAGGTCCCTGAGACTCTCCTGTGCAGC GTCTGGATTCACCTTCAGTAGCTATGGCATGCACTGGGTCCGCCAGGCTCCAGGCAAGGGGCTGGA GTGGGTGGCATTTATACGGTATGATGGAAGTAATAAATACTATGCAGACTCCGTGAAGGGCCGAT TCACCATCTCCAGAGACAATTCCAAGAACACGCTGTATCTGCAGATGAACAGCCTGAGAGCTGAG GACACGGCTGTGTATTACTGTAAGACCCATGGTAGCCATGACAACTGGGGCCAAGGGACAATGGT. CACCGTCTCTTCAGCGTCGACCAAGGGCCCATCGGTCTTCCCCCTGGCACCCTCCTCCAAGAGCAC CTCTGGGGGCACAGCGGCCCTGGGCTGCCTGGTCAAGGACTACTTCCCCGAACCGGTGACGGTGTC GTGGAACTCAGGCGCCCTGACCAGCGGCGTGCACACCTTCCCGGCTGTCCTACAGTCCTCAGGACT CTACTCCCTCAGCAGCGTGGTGACCGTGCCCTCCAGCAGCTTGGGCACCCAGACCTACATCTGCAA CGTGAATCACAAGCCCAGCAACACCAAGGTGGACAAGAAAGTTGAGCCCAAATCTTGTGACAAAA CTCACACATGCCCACCGTGCCCAGCACCTGAACTCCTGGGGGGACCGTCAGTCTTCCTCTTCCCCC CAAAACCCAAGGACACCCTCATGATCTCCCGGACCCCTGAGGTCACATGCGTGGTGGTGGACGTG AGCCACGAAGACCCTGAGGTCAAGTTCAACTGGTACGTGGACGGCGTGGAGGTGCATAATGCCAA GACAAAGCCGCGGGAGGAGCAGTACAACAGCACGTACCGTGTGGTCAGCGTCCTCACCGTCCTGC ACCAGGACTGGCTGAATGGCAAGGAGTACAAGTGCAAGGTCTCCAACAAAGCCCTCCCAGCCCCC ATCGAGAAAACCATCTCCAAAGCCAAAGGGCAGCCCCGAGAACCACAGGTGTACACCCTGCCCCC ATCCCGCGAGGAGATGACCAAGAACCAGGTCAGCCTGACCTGCCTGGTCAAAGGCTTCTATCCCA GCGACATCGCCGTGGAGTGGGAGAGCAATGGGCAGCCGGAGAACAACTACAAGACCACGCCTCC CGTGCTGGACTCCGACGGCTCCTTCTTCCTCTACAGCAAGCTCACCGTGGACAAGAGCAGGTGGCA GCAGGGGAACGTCTTCTCATGCTCCGTGATGCATGAGGCTCTGCACAACCACTACACGCAGAAGA GCCTCTCCCTGTCTAGGGGTAAACGCGAACCAGTTTATTCCAGGGGAGCTTGTTTAAGGGGCCGC GTGATTATAACCCAATATCGAGTGCCATTTGTCATCTAACGAATGAATCTGATGGGCACACAACAT CGTTGTATGGTATTGGTTTTGGCCCTTTCATCATCACAAACAAGCATTTGTTTAGAAGAAATAATG GTACACTGTTAGTTCAATCACTACATGGTGTGTTCAAGGTAAAGAATACCACAACTTTGCAACAAC ACCTCATTGATGGGAGGGACATGATGCTCATTCGCATGCCTAAGGATTTCCCACCATTTCCTCAAA AGCTGAAATTCAGAGAGCCACAAAGGGAAGAGCGCATATGTCTTGTGACAACCAACTTCCAAACT AAGAGCATGTCTAGCATGGTTTCAGATACTAGTTGCACATTCCCTTCATCTGATGGTATATTCTGGA AACATTGGATTCAGACCAAGGATGGGCACTGTGGTAGCCCGTTGGTGTCAACTAGAGATGGGTTT ATTGTTGGTATACACTCAGCATCAAATTTCACCAACACAAACAATTATTTTACAAGTGTGCCGAAA GACTTCATGGATTTATTGACAAATCAAGAGGCGCAGCAATGGGTTAGTGGTTGGCGATTGAATGCT GACTCAGTGTTATGGGGAGGCCACAAAGTTTTCATGAGCAAACCTGAAGAACCCTTTCAGCCAGTC AAAGAAGCAACTCAACTCATGAGTGAATTAGTCTACTCGCAAGGGATGACTTGGACCCCACTCCTC TTCCTCACCCTCCTCCTCCACTGCACAGGAAGCTTATCCCAGTCTGTGCTGACTCAGCCCCCCTCAG TGTCTGGGGCCCCCGGGCAGAGAGTCACCATCTCTTGTTCTGGAAGCAGATCCAACATCGGCAGTA ATACTGTAAAGTGGTATCAGCAGCTCCCAGGAACGGCCCCCAAACTCCTCATCTATTACAATGATC |
| AGCGGCCCTCAGGGGTCCTGACCGATTCTCTGGATCCAAGTCTGGCACCTCAGCCTCCCTCGCCA TCACTGGGCTCCAGGCTGAAGACGAGGCTGACTATTACTGCCAGTCATATGACAGATACACCCACC CCGCCCTGCTCTTCGGAACTGGGACCAAGGTCACAGTACTAGGTCAGCCCAAGGCTGCCCCCTCGG TCACTCTGTTCCCGCCCTCCTCTGAGGAGCTTCAAGCCAACAAGGCCACACTGGTGTGTCTCATAA GTGACTTCTACCCGGGAGCCGTGACAGTGGCCTGGAAGGCAGATAGCAGCCCCGTCAAGGCGGGA GTGGAGACCACCACACCCTCCAAACAAAGCAACAACAAGTACGCGGCCAGCAGCTACCTGAGCCT GACGCCTGAGCAGTGGAAGTCCCACAGAAGCTACAGCTGCCAGGTCACGCATGAAGGGAGCACCG TGGAGAAGACAGTGGCCCCTACAGAATGTTCATGAGCGGCCGCGTTTAAACTGAATGAGCGCGTC CATCCAGACATGATAAGATACATTGATGAGTTTGGACAAACCACAACTAGAATGCAGTGAAAAAA ATGCTTTATTTGTGAAATTTGTGATGCTATTGCTTTATTTGTAACCATTATAAGCTGCAATAAACAA GTTAACAACAACAATTGCATTCATTTTATGTTTCAGGTTCAGGGGGAGGTGTGGGAGGTTTTTTAA AGCAAGTAAAACCTCTACAAATGTGGTATGGCTGATTATGATCCGGCTGCCTCGCGCGTTTCGGTG ATGACGGTGAAAACCTCTGACACATGCAGCTCCCGGAGACGGTCACAGCTTGTCTGTAAGCGGAT GCCGGGAGCAGACAAGCCCGTCAGGGCGCGTCAGCGGGTGTTGGCGGGTGTCGGGGCGCAGCCAT GACCGGTCGACGGCGCGCCTTTTTTTTAATTTTATTTTATTTTATTTTTGACGCGCCGAAGGCGC GATCTGAGCTCGGTACAGCTTGGCTGTGGAATGTGTGTCAGTTAGGGTGTGGAAAGTCCCCAGGCT CCCCAGCAGGCAGAAGTATGCAAAGCATGCATCTCAATTAGTCAGCAACCAGGTGTGGAAAGTCC CCAGGCTCCCCAGCAGGCAGAAGTATGCAAAGCATGCATCTCAATTAGTCAGCAACCATAGTCCC GCCCCTAACTCCGCCCATCCCGCCCCTAACTCCGCCCAGTTCCGCCCATTCTCCGCCCCATGGCTGA CTAATTTTTTTTATTTATGCAGAGGCCGAGGCCGCCTCGGCCTCTGAGCTATTCCAGAAGTAGTGA GGAGGCTTTTTTGGAGGCCTAGGCTTTTGCAAAAAGCTCCTCGAGGAACTGAAAAACCAGAAAGT TAACTGGTAAGTTTAGTCTTTTTGTCTTTTATTTCAGGTCCCGGATCCGGTGGTGGTGCAAATCAAA GAACTGCTCCTCAGTGGATGTTGCCTTTACTTCTAGGCCTGTACGGAAGTGTTACTTCTGCTCTAAA AGCTGCGGAATTGTACCCGCGGCCTAATACGACTCACTATAGGGACTAGTATGGTTCGACCATTGA ACTGCATCGTCGCCGTGTCCCAAAATATGGGGATTGGCAAGAACGGAGACCTACCCTGGCCTCCG CTCAGGAACGAGTTCAAGTACTTCCAAAGAATGACCACAACCTCTTCAGTGGAAGGTAAACAGAA TCTGGTGATTATGGGTAGGAAAACCTGGTTCTCCATTCCTGAGAAGAATCGACCTTTAAAGGACAG AATTAATATAGTTCTCAGTAGAGAACTCAAAGAACCACCACGAGGAGCTCATTTTCTTGCCAAAAG TTTAGATGATGCCTTAAGACTTATTGAACAACCGGAATTGGCAAGTAAAGTAGACATGGTTTGGAT AGTCGGAGGCAGTTCTGTTTACCAGGAAGCCATGAATCAACCAGGCCACCTCAGACTCTTTGTGAC AAGGATCATGCAGGAATTTGAAAGTGACACGTTTTTCCCAGAAATTGATTTGGGGAAATATAAACT TCTCCCAGAATACCCAGGCGTCCTCTCTGAGGTCCAGGAGGAAAAAGGCATCAAGTATAAGTTTG AAGTCTACGAGAAGAAAGACTAAGCGGCCGAGCGCGCGGATCTGGAAACGGAGATGGGGGAGG CTAACTGAAGCACGGAAGGAGACAATACCGGAAGGAACCCGCGCTATGACGGCAATAAAAAGAC AGAATAAAACGCACGGGTGTTGGGTCGTTTGTTCATAAACGCGGGGTTCGGTCCCAGGGCTGGCA CTCTGTCGATACCCCACCGAGACCCCATTGGGGCCAATACGCCCGCGTTTCTTCCTTTTCCCCACCC CACCCCCCAAGTTCGGGTGAAGGCCCAGGGCTCGCAGCCAACGTCGGGGCGGCAGGCCCTGCCAT AGCCACTGGCCCCGTGGGTTAGGGACGGGGTCCCCCATGGGGAATGGTTTATGGTTCGTGGGGGTT ATTATTTTGGGCGTTGCGTGGGGTCTGGAGATCCCCCGGGCTGCAGGAATTCCGTTACATTACTTA CGGTAAATGGCCCGCCTGGCTGACCGCCCAACGACCCCCGCCCATTGACGTCAATAATGACGTATG TTCCCATAGTAACGCCAATAGGGACTTTCCATTGACGTCAATGGGTGGAGTATTTACGGTAAACTG CCCACTTGGCAGTACATCAAGTGTATCATATGCCAAGTACGCCCCCTATTGACGTCAATGACGGTA AATGGCCCGCCTGGCATTATGCCCAGTACATGACCTTATGGGACTTTCCTACTTGGCAGTACATCT ACGTATTAGTCATCGCTATTACCATGGTGATGCGGTTTTGGCAGTACATCAATGGGCGTGGATAGC GGTTTGACTCACGGGGATTTCCAAGTCTCCACCCCATTGACGTCAATGGGAGTTTGTTTTGGCACC AAAATCAACGGGACTTTCCAAAATGTCGTAACAACTCCGCCCCATTGACGCAAATGGGCGGGAAT TCGAGCTCGGTACTCGAGCGGTGTTCCGCGGTCCTCCTCGTATAGAAACTCGGACCACTCTGAGAC GAAGGCTCGCGTCCAGGCCAGCACGAAGGAGGCTAAGTGGGAGGGGTAGCGGTCGTTGTCCACTA GGGGGTCCACTCGCTCCAGGGTGTGAAGACACATGTCGCCCTCTTCGGCATCAAGGAAGGTGATT GGTTTATAGGTGTAGGCCACGTGACCGGGTGTTCCTGAAGGGGGGCTATAAAAGGGGGTGGGGGC GCGTTCGTCCTCACTCTCTTCCGCATCGCTGTCTGCGAGGGCCAGCTGTTGGGCTCGCGGTTGAGG ACAAACTCTTCGCGGTCTTTCCAGTACTCTTGGATCGGAAACCCGTCGGCCTCCGAACGGTACTCC GCCACCGAGGGACCTGAGCGAGTCCGCATCGACCGGATCGGAAAACCTCTCGACTGTTGGGGTGA GTACTCCCTCTCAAAAGCGGGCATGACTTCTGCGCTAAGATTGTCAGTTTCCAAAAACGAGGAGGA TTTGATATTCACCTGGCCCGCGGTGATGCCTTTGAGGGTGGCCGCGTCCATCTGGTCAGAAAAGAC AATCTTTTTGTTGTCAAGCTTGAGGTGTGGCAGGCTTGAGATCTGGCCATACACTTGAGTGACAAT GACATCCACTTTGCCTTTCTCTCCACAGGTGTCCACTCCCAGGTCCAACCGGAATTGTACCCGCGG CCAGAGCTTGCGGGCGCCACCGCGGCCGCGGGGATCCAGACATGATAAGATACATTGATGAGTTT GGACAAACCACAACTAGAATGCAGTGAAAAAAATGCTTTATTTGTGAAATTTGTGATGCTATTGCT TTATTTGTAACCATTATAAGCTGCAATAAACAAGTTAACAACAACAATTGCATTCATTTTATGTTTC |
| AGGTTCAGGGGGAGGTGTGGGAGGTTTTTCGGATCCTCTTGGCGTAATCATGGTCATAGCTGTTT CCTGTGTGAAATTGTTATCCGCTCACAATTCCACACAACATACGAGCCGGAAGCATAAAGTGTAAA GCCTGGGGTGCCTAATGAGTGAGCTAACTCACATTAATTGCGTTGCGCTCACTGCCCGCTTTCCAG TCGGGAAACCTGTCGTGCCAGCTGCATTAATGAATCGGCCAACGCGCGGGGAAAGGCGGTTTGCG TATTGGGCGCTCTTCCGCTTCCTCGCTCACTGACTCGCTGCGCTCGGTCGTTCGGCTGCGGCGAGCG GTATCAGCTCACTCAAAGGCGGTAATACGGTTATCCACAGAATCAGGGGATAACGCAGGAAAGAA CATGTGAGCAAAAGGCCAGCAAAAGGCCAGGAACCGTAAAAAGGCCGCGTTGCTGGCGTTCTTCC ATAGGCTCCGCCCCCCTGACGAGCATCACAAAAATCGACGCTCAAGTCAGAGGTGGCGAAACCCG ACAGGACTATAAAGATACCAGGCGTTTCCCCCTGGAAGCTCCCTCGTGCGCTCTCCTGTTCCGACC CTGCCGCTTACCGGATACCTGTCCGCCTTTCTCCCTTCGGGAAGCGTGGCGCTTTCTCATAGCTCAC GCTGTAGGTATCTCAGTTCGGTGTAGGTCGTTCGCTCCAAGCTGGGCTGTGTGCACGAACCCCCCG TTCAGCCCGACCGCTGCGCCTTATCCGGTAACTATCGTCTTGAGTCCAACCCGGTAAGACACGACT TATCGCCACTGGCAGCAGCCACTGGTAACAGGATTAGCAGAGCGAGGTATGTAGGCGGTGCTACA GAGTTCTTGAAGTGGTGGCCTAACTACGGCTACACTAGAAGAACAGTATTTGGTATCTGCGCTCTG CTGAAGCCAGTTACCTTCGGAAAAAGAGTTGGTAGCTCTTGATCCGGCAAACAAACCACCGCTGG TAGCGGTGGTTTTTTTGTTTGCAAGCAGCAGATTACGCGCAGAAAAAAAGGATCTCAAGAAGATC CTTTGATCTTTTCTACGGGGTCTGACGCTCAGTGGAACGAAAACTCACGTTAAGGGATTTTGGTCA TGAGATTATCAAAAAGGATCTTCACCTAGATCCTTTTAATTAAAAATGAAGTTTTAAATCAATCT AAAGTATATATGAGTAAACTTGGTCTGACAGTTACCAATGCTTAATCAGTGAGGCACCTATCTCAG CGATCTGTCTATTTCGTTCATCCATAGTTGCCTGACTCCCCGTCGTGTAGATAACTACGATACGGGA GGGCTTACCATCTGGCCCCAGTGCTGCAATGATACCGCGAGACCCACGCTCACCGGCTCCAGATTT ATCAGCAATAAACCAGCCAGCCGGAAGGGCCGAGCGCAGAAGTGGTCCTGCAACTTTATCCGCCT CCATCCAGTCTATTAATTGTTGCCGGGAAGCTAGAGTAAGTAGTTCGCCAGTTAATAGTTTGCGCA ACGTTGTTGCCATTGCTACAGGCATCGTGGTGTCACGCTCGTCGTTTGGTATGGCTTCATTCAGCTC CGGTTCCCAACGATCAAGGCGAGTTACATGATCCCCCATGTTGTGCAAAAAAGCGGTTAGCTCCTT CGGTCCTCCGATCGTTGTCAGAAGTAAGTTGGCCGCAGTGTTATCACTCATGGTTATGGCAGCACT GCATAATTCTCTTACTGTCATGCCATCCGTAAGATGCTTTTCTGTGACTGGTGAGTACTCAACCAAG TCATTCTGAGAATAGTGTATGCGGCGACCGAGTTGCTCTTGCCCGGCGTCAATACGGGATAATACC GCGCCACATAGCAGAACTTTAAAAGTGCTCATCATTGGAAAACGTTCTTCGGGGCGAAAACTCTCA AGGATCTTACCGCTGTTGAGATCCAGTTCGATGTAACCCACTCGTGCACCCAACTGATCTTCAGCA TCTTTTACTTTCACCAGCGTTTCTGGGTGAGCAAAAACAGGAAGGCAAAATGCCGCAAAAAAGGG AATAAGGGCGACACGGAAATGTTGAATACTCATACTCTTCCTTTTTCAATATTATTGAAGCATTTAT CAGGGTTATTGTCTCATGAGCGGATACATATTTGAATGTATTTAGAAAAATAAACAAATAGGGGTT CCGCGCACATTTCCCCGAAAAGTGCCACCTGACGTCTAAGAAACCATTATTATCATGACATTAACC TATAAAAATAGGCGTATCACGAGGCCCTTTCGTCTCGCGCGTTTCGGTGATGACGGTGAAAACCTC TGACACATGCAGCTCCCGGAGACGGTCACAGCTTGTCTGTAAGCGGATGCCGGGAGCAGACAAGC CCGTCAGGGCGCGTCAGCGGGTGTTGGCGGGTGTCGGGGCTGGCTTAACTATGCGGCATCAGAGC AGATTGTACTGAGAGTGCACCATATGCGGTGTGAAATACCGCACAGATGCGTAAGGAGAAAATAC CGCATCAGGCGCCATTCGCCATTCAGGCTGCGCAACTGTTGGGAAGGGCGATCGGTGCGGGCCTCT TCGCTATTACGCCAGCTGGCGAAAGGGGGATGTGCTGCAAGGCGATTAAGTTGGGTAACGCCAGG GTTTTCCCAGTCACGACGTTGTAAAACGACGGCCAGTGAATT |

Table 4A. Nucleic Acid Sequence Encoding EL246 GG (Anti-E/L Selectin) TEV Polyprotein (SEQ ID NO:38)

| |
|---|
| ATGGAGTTTGGGCTGAGCTGGCTTTTTCTTGTCGCGATTTTAAAAGGTGTCCAGTGCGAGGTGCA GCTGGTGCAGTCTGGAGCAGAGGTGAAAAAGCCCGGGGAGTCTCTGAAGATCTCCTGTAAGGG GTCCGGATACGCATTCAGTAGTTCCTGGATCGGCTGGGTGCGCCAGATGCCCGGGAAAGGCCT GGAGTGGATGGGGCGGATTTATCCTGGAGATGGAGATACTAACTACAATGGGAAGTTCAAGGGC CAGGTCACCATCTCAGCCGACAAGTCCATCAGCACCGCCTACCTGCAGTGGAGCAGCCTGAAG GCTAGCGACACCGCCATGTATTACTGTGCGAGAGCGCGCGTGGGATCCACGGTCTATGATGGTT ACCTCTATGCAATGGACTACTGGGGTCAAGGTACCTCAGTCACCGTCTCCTCAGCGTCGACCAA GGGCCCATCGGTCTTCCCCCTGGCACCCTCCTCCAAGAGCACCTCTGGGGGCACAGCGGCCCT GGGCTGCCTGGTCAAGGACTACTTCCCCGAACCGGTGACGGTGTCGTGGAACTCAGGCGCCCT GACCAGCGGCGTGCACACCTTCCCGGCTGTCCTACAGTCCTCAGGACTCTACTCCCTCAGCAGC GTGGTGACCGTGCCCTCCAGCAGCTTGGGCACCCAGACCTACATCTGCAACGTGAATCACAAGC CCAGCAACACCAAGGTGGACAAGAAAGTTGAGCCCAAATCTTGTGACAAAACTCACACATGCCC |
| ACCGTGCCCAGCACCTGAAGCCGCGGGGGGACCGTCAGTCTTCCTCTTCCCCCCAAAACCCAA GGACACCCTCATGATCTCCCGGACCCCTGAGGTCACATGCGTGGTGGTGGACGTGAGCCACGA AGACCCTGAGGTCAAGTTCAACTGGTACGTGGACGGCGTGGAGGTGCATAATGCCAAGACAAAG CCGCGGGAGGAGCAGTACAACAGCACGTACCGTGTGGTCAGCGTCCTCACCGTCCTGCACCAG GACTGGCTGAATGGCAAGGAGTACAAGTGCAAGGTCTCCAACAAAGCCCTCCCAGCCCCCATCG AGAAAACCATCTCCAAAGCCAAAGGGCAGCCCCGAGAACCACAGGTGTACACCCTGCCCCCATC CCGCGAGGAGATGACCAAGAACCAGGTCAGCCTGACCTGCCTGGTCAAAGGCTTCTATCCCAG CGACATCGCCGTGGAGTGGGAGAGCAATGGGCAGCCGGAGAACAACTACAAGACCACGCCTCC CGTGCTGGACTCCGACGGCTCCTTCTTCCTCTACAGCAAGCTCACCGTGGACAAGAGCAGGTGG CAGCAGGGGAACGTCTTCTCATGCTCCGTGATGCATGAGGCTCTGCACAACCACTACACGCAGA AGAGCCTCTCCCTGTCTAGGGGTAAACGCGAACCAGTTTATTTCCAGGGGAGCTTGTTTAAGGG GCCGCGTGATTATAACCCAATATCGAGTGCCATTTGTCATCTAACGAATGAATCTGATGGGCACA CAACATCGTTGTATGGTATTGGTTTTGGCCCTTTCATCATCACAAACAAGCATTTGTTTAGAAGAA ATAATGGTACACTGTTAGTTCAATCACTACATGGTGTGTTCAAGGTAAAGAATACCACAACTTTGC AACAACACCTCATTGATGGGAGGGACATGATGCTCATTCGCATGCCTAAGGATTTCCCACCATTT CCTCAAAAGCTGAAATTCAGAGAGCCACAAAGGGAAGAGCGCATATGTCTTGTGACAACCAACTT CCAAACTAAGAGCATGTCTAGCATGGTTTCAGATACTAGTTGCACATTCCCTTCATCTGATGGTAT ATTCTGGAAACATTGGATTCAGACCAAGGATGGGCACTGTGGTAGCCCGTTGGTGTCAACTAGA GATGGGTTTATTGTTGGTATACACTCAGCATCAAATTTCACCAACACAAACAATTATTTTACAAGT GTGCCGAAAGACTTCATGGATTTATTGACAAATCAAGAGGCGCAGCAATGGGTTAGTGGTTGGC GATTGAATGCTGACTCAGTGTTATGGGGAGGCCACAAAGTTTTCATGAGCAAACCTGAAGAACCC TTTCAGCCAGTCAAAGAAGCAACTCAACTCATGAGTGAATTAGTCTACTCGCAAGGGATGGACAT GCGCGTGCCCGCCCAGCTGCTGGGCCTGCTGCTGCTGTGGTTCCCCGGCTCGCGATGCGACAT CGTGATGACCCAGTCTCCAGACTCCCTGGCTGTGTCTCTGGGCGAGAGGGCCACCATCAACTG CAAGTCCAGTCAGAGCCTTTCATATAGAAGCAATCAAAAGAACTCGTTGGCCTGGTACCAGCAGA AACCAGGACAGCCTCCTAAGCTGCTCATTTACTGGGCTAGCACTAGGGAATCTGGGGTCCCTGA CCGATTCAGTGGATCCGGGTCTGGGACAGATTTCACTCTCACCATCAGCAGCCTGCAGGCTGAA GATGTGGCAGTTTATTACTGTCACCAATATTATAGCTATCCGTACACGTTCGGAGGGGGGACCAA GGTGGAAATTAAACGTACGGTGGCTGCACCATCTGTCTTCATCTTCCCGCCATCTGATGAGCAGT TGAAATCTGGAACTGCCTCTGTTGTGTGCCTGCTGAATAACTTCTATCCCAGAGAGGCCAAAGTA CAGTGGAAGGTGGATAACGCCCTCCAATCGGGTAACTCCCAGGAGAGTGTCACAGAGCAGGAC AGCAAGGACAGCACCTACAGCCTCAGCAGCACCCTGACGCTGAGCAAAGCAGACTACGAGAAA CACAAAGTCTACGCCTGCGAAGTCACCCATCAGGGCCTGAGCTCGCCCGTCACAAAGAGCTTCA ACAGGGGAGAGTGTTGA |

Table 4B. Amino Acid Sequence of EL246 GG (Anti-E/L Selectin) TEV Polyprotein (SEQ ID NO:39)

| |
|---|
| * |

Table 4C. Complete Nucleotide Sequence for EL246 GG (Anti-E/L Selectin) TEV Polyprotein Expression Vector (SEQ ID NO:40)

| |
|---|
| GAAGTTCCTATTCCGAAGTTCCTATTCTCTAGACGTTACATAACTTACGGTAAATGGCCCGCCTGG CTGACCGCCCAACGACCCCCGCCCATTGACGTCAATAATGACGTATGTTCCCATAGTAACGCCAAT AGGGACTTTCCATTGACGTCAATGGGTGGAGTATTTACGGTAAACTGCCCACTTGGCAGTACATCA AGTGTATCATATGCCAAGTACGCCCCCTATTGACGTCAATGACGGTAAATGGCCCGCCTGGCATTA TGCCCAGTACATGACCTTATGGGACTTTCCTACTTGGCAGTACATCTACGTATTAGTCATCGCTATT ACCATGGTGATGCGGTTTTGGCAGTACATCAATGGGCGTGGATAGCGGTTTGACTCACGGGGATTT CCAAGTCTCCACCCCATTGACGTCAATGGGAGTTTGTTTTGGCACCAAAATCAACGGGACTTTCCA AAATGTCGTAACAACTCCGCCCCAATGACGCAAATGGGCAGGGAATTCGAGCTCGGTACTCGAGC GGTGTTCCGCGGTCCTCCTCGTATAGAAACTCGGACCACTCTGAGACGAAGGCTCGCGTCCAGGCC AGCACGAAGGAGGCTAAGTGGGAGGGGTAGCGGTCGTTGTCCACTAGGGGGTCCACTCGCTCCAG GGTGTGAAGACACATGTCGCCCTCTTCGGCATCAAGGAAGGTGATTGGTTTATAGGTGTAGGCCAC GTGACCGGGTGTTCCTGAAGGGGGGCTATAAAAGGGGGTGGGGGCGCGTTCGTCCTCACTCTCTTC CGCATCGCTGTCTGCGAGGGCCAGCTGTTGGGCTCGCGGTTGAGGACAAACTCTTCGCGGTCTTTC CAGTACTCTTGGATCGGAAACCCGTCGGCCTCCGAACGGTACTCCGCCACCGAGGGACCTGAGCG AGTCCGCATCGACCGGATCGGAAAACCTCTCGACTGTTGGGGTGAGTACTCCCTCTCAAAAGCGG GCATGACTTCTGCGCTAAGATTGTCAGTTTCCAAAAACGAGGAGGATTTGATATTCACCTGGCCCG CGGTGATGCCTTTGAGGGTGGCCGCGTCCATCTGGTCAGAAAAGACAATCTTTTTGTTGTCAAGCT TGAGGTGTGGCAGGCTTGAGATCTGGCCATACACTTGAGTGACAATGACATCCACTTTGCCTTTCT CTCCACAGGTGTCCACTCCCAGGTCCAACCGGAATTGTACCCGCGGCCAGAGCTTGCCCGGGCGCC ACCATGGAGTTTGGGCTGAGCTGGCTTTTTCTTGTCGCGATTTTAAAAGGTGTCCAGTGCGAGGTG CAGCTGGTGCAGTCTGGAGCAGAGGTGAAAAAGCCCGGGGAGTCTCTGAAGATCTCCTGTAAGGG GTCCGGATACGCATTCAGTAGTTCCTGGATCGGCTGGGTGCGCCAGATGCCCGGGAAAGGCCTGG AGTGGATGGGGCGGATTTATCCTGGAGATGGAGATACTAACTACAATGGGAAGTTCAAGGGCCAG GTCACCATCTCAGCCGACAAGTCCATCAGCACCGCCTACCTGCAGTGGAGCAGCCTGAAGGCTAG CGACACCGCCATGTATTACTGTGCGAGAGCGCGCGTGGGATCCACGGTCTATGATGGTTACCTCTA TGCAATGGACTACTGGGGTCAAGGTACCTCAGTCACCGTCTCCTCAGCGTCGACCAAGGGCCCATC GGTCTTCCCCCTGGCACCCTCCTCCAAGAGCACCTCTGGGGGCACAGCGGCCCTGGGCTGCCTGGT CAAGGACTACTTCCCCGAACCGGTGACGGTGTCGTGGAACTCAGGCGCCCTGACCAGCGGCGTGC ACACCTTCCCGGCTGTCCTACAGTCCTCAGGACTCTACTCCCTCAGCAGCGTGGTGACCGTGCCCT CCAGCAGCTTGGGCACCCAGACCTACATCTGCAACGTGAATCACAAGCCCAGCAACACCAAGGTG GACAAGAAAGTTGAGCCCAAATCTTGTGACAAAACTCACACATGCCCACCGTGCCCAGCACCTGA AGCCGCGGGGGGACCGTCAGTCTTCCTCTTCCCCCCAAAACCCAAGGACACCCTCATGATCTCCCG GACCCCTGAGGTCACATGCGTGGTGGTGGACGTGAGCCACGAAGACCCTGAGGTCAAGTTCAACT GGTACGTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGGAGTACA AGTGCAAGGTCTCCAACAAAGCCCTCCCAGCCCCCATCGAGAAAACCATCTCCAAAGCCAAAGGG CAGCCCCGAGAACCACAGGTGTACACCCTGCCCCCATCCCGCGAGGAGATGACCAAGAACCAGGT CAGCCTGACCTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTCTTCCTCT ACAGCAAGCTCACCGTGGACAAGAGCAGGTGGCAGCAGGGGAACGTCTTCTCATGCTCCGTGATG CATGAGGCTCTGCACAACCACTACACGCAGAAGAGCCTCTCCCTGTCTAGGGGTAAACGCGAACC AGTTTATTTCCAGGGGAGCTTGTTTAAGGGGCCGCGTGATTATAACCCAATATCGAGTGCCATTTG TCATCTAACGAATGAATCTGATGGGCACACAACATCGTTGTATGGTATTGGTTTTGGCCCTTTCATC ATCACAAACAAGCATTTGTTTAGAAGAAATAATGGTACACTGTTAGTTCAATCACTACATGGTGTG TTCAAGGTAAAGAATACCACAACTTTGCAACAACACCTCATTGATGGGAGGGACATGATGCTCATT CGCATGCCTAAGGATTTCCCACCATTTCCTCAAAAGCTGAAATTCAGAGAGCCACAAAGGGAAGA GCGCATATGTCTTGTGACAACCAACTTCCAAACTAAGAGCATGTCTAGCATGGTTTCAGATACTAG TTGCACATTCCCTTCATCTGATGGTATATTCTGGAAACATTGGATTCAGACCAAGGATGGGCACTG TGGTAGCCCGTTGGTGTCAACTAGAGATGGGTTTATTGTTGGTATACACTCAGCATCAAATTTCAC CAACACAAACAATTATTTTACAAGTGTGCCGAAAGACTTCATGGATTTATTGACAAATCAAGAGGC GCAGCAATGGGTTAGTGGTTGGCGATTGAATGCTGACTCAGTGTTATGGGAGGCCACAAAGTTTT CATGAGCAAACCTGAAGAACCCTTTCAGCCAGTCAAAGAAGCAACTCAACTCATGAGTGAATTAG TCTACTCGCAAGGGATGGACATGCGCGTGCCCGCCCAGCTGCTGGGCCTGCTGCTGCTGTGGTTCC CCGGCTCGCGATGCGACATCGTGATGACCCAGTCTCCAGACTCCCTGGCTGTGTCTCTGGGCGAGA GGGCCACCATCAACTGCAAGTCCAGTCAGAGCCTTTCATATAGAAGCAATCAAAAGAACTCGTTG GCCTGGTACCAGCAGAAACCAGGACAGCCTCCTAAGCTGCTCATTTACTGGGCTAGCACTAGGGA ATCTGGGGTCCCTGACCGATTCAGTGGATCCGGGTCTGGGACAGATTTCACTCTCACCATCAGCAG CCTGCAGGCTGAAGATGTGGCAGTTTATTACTGTCACCAATATTATAGCTATCCGTACACGTTCGG AGGGGGGACCAAGGTGGAAATTAAACGTACGGTGGCTGCACCATCTGTCTTCATCTTCCCGCCATC TGATGAGCAGTTGAAATCTGGAACTGCCTCTGTTGTGTGCCTGCTGAATAACTTCTATCCCAGAGA |
| GGCCAAAGTACAGTGGAAGGTGGATAACGCCCTCCAATCGGGTAACTCCCAGGAGAGTGTCACAG AGCAGGACAGCAAGGACAGCACCTACAGCCTCAGCAGCACCCTGACGCTGAGCAAAGCAGACTA CGAGAAACACAAAGTCTACGCCTGCGAAGTCACCCATCAGGGCCTGAGCTCGCCCGTCACAAAGA GCTTCAACAGGGGAGAGTGTTGAGCGGCCGCGTTTAAACTGAATGAGCGCGTCCATCCAGACATG ATAAGATACATTGATGAGTTTGGACAAACCACAACTAGAATGCAGTGAAAAAAATGCTTTATTTGT GAAATTTGTGATGCTATTGCTTTATTTGTAACCATTATAAGCTGCAATAAACAAGTTAACAACAAC AATTGCATTCATTTTATGTTTCAGGTTCAGGGGGAGGTGTGGGAGGTTTTTTAAAGCAAGTAAAAC CTCTACAAATGTGGTATGGCTGATTATGATCCGGCTGCCTCGCGCGTTTCGGTGATGACGGTGAAA ACCTCTGACACATGCAGCTCCCGGAGACGGTCACAGCTTGTCTGTAAGCGGATGCCGGGAGCAGA CAAGCCCGTCAGGGCGCGTCAGCGGGTGTTGGCGGGTGTCGGGGCGCAGCCATGACCGGTCGACG GCGCGCCTTTTTTTTAATTTTTATTTTATTTTATTTTTGACGCGCCGAAGGCGCGATCTGAGCTCGG TACAGCTTGGCTGTGGAATGTGTGTCAGTTAGGGTGTGGAAAGTCCCCAGGCTCCCCAGCAGGCA GAAGTATGCAAAGCATGCATCTCAATTAGTCAGCAACCAGGTGTGGAAAGTCCCCAGGCTCCCCA GCAGGCAGAAGTATGCAAAGCATGCATCTCAATTAGTCAGCAACCATAGTCCCGCCCCTAACTCC GCCCATCCCGCCCCTAACTCCGCCCAGTTCCGCCCATTCTCCGCCCCATGGCTGACTAATTTTTTT ATTTATGCAGAGGCCGAGGCCGCCTCGGCCTCTGAGCTATTCCAGAAGTAGTGAGGAGGCTTTTT GGAGGCCTAGGCTTTTGCAAAAAGCTCCTCGAGGAACTGAAAAACCAGAAAGTTAACTGGTAAGT TTAGTCTTTTTGTCTTTTATTTCAGGTCCCGGATCCGGTGGTGGTGCAAATCAAAGAACTGCTCCTC AGTGGATGTTGCCTTTACTTCTAGGCCTGTACGGAAGTGTTACTTCTGCTCTAAAAGCTGCGGAATT GTACCCGCGGCCTAATACGACTCACTATAGGGACTAGTATGGTTCGACCATTGAACTGCATCGTCG CCGTGTCCCAAAATATGGGGATTGGCAAGAACGGAGACCTACCCTGGCCTCCGCTCAGGAACGAG TTCAAGTACTTCCAAAGAATGACCACAACCTCTTCAGTGGAAGGTAAACAGAATCTGGTGATTATG GGTAGGAAAACCTGGTTCTCCATTCCTGAGAAGAATCGACCTTTAAAGGACAGAATTAATATAGTT CTCAGTAGAGAACTCAAAGAACCACCACGAGGAGCTCATTTTCTTGCCAAAAGTTTAGATGATGCC TTAAGACTTATTGAACAACCGGAATTGGCAAGTAAAGTAGACATGGTTTGGATAGTCGGAGGCAG TTCTGTTTACCAGGAAGCCATGAATCAACCAGGCCACCTCAGACTCTTTGTGACAAGGATCATGCA GGAATTTGAAAGTGACACGTTTTTCCCAGAAATTGATTTGGGGAAATATAAACTTCTCCCAGAATA CCCAGGCGTCCTCTCTGAGGTCCAGGAGGAAAAAGGCATCAAGTATAAGTTTGAAGTCTACGAGA AGAAAGACTAAGCGGCCGAGCGCGCGGATCTGGAAACGGGAGATGGGGGAGGCTAACTGAAGCA CGGAAGGAGACAATACCGGAAGGAACCCGCGCTATGACGGCAATAAAAAGACAGAATAAAACGC ACGGGTGTTGGGTCGTTTGTTCATAAACGCGGGGTTCGGTCCCAGGGCTGGCACTCTGTCGATACC CCACCGAGACCCCATTGGGGCCAATACGCCCGCGTTTCTTCCTTTTCCCCACCCCACCCCCCAAGTT CGGGTGAAGGCCCAGGGCTCGCAGCCAACGTCGGGGCGGCAGGCCCTGCCATAGCCACTGGCCCC GTGGGTTAGGGACGGGGTCCCCCATGGGGAATGGTTTATGGTTCGTGGGGGTTATTATTTTGGGCG TTGCGTGGGGTCTGGAGATCCCCCGGGCTGCAGGAATTCCGTTACATTACTTACGGTAAATGGCCC GCCTGGCTGACCGCCCAACGACCCCCGCCCATTGACGTCAATAATGACGTATGTTCCCATAGTAAC GCCAATAGGGACTTTCCATTGACGTCAATGGGTGGAGTATTTACGGTAAACTGCCCACTTGGCAGT ACATCAAGTGTATCATATGCCAAGTACGCCCCCTATTGACGTCAATGACGGTAAATGGCCCGCCTG GCATTATGCCCAGTACATGACCTTATGGGACTTTCCTACTTGGCAGTACATCTACGTATTAGTCATC GCTATTACCATGGTGATGCGGTTTTGGCAGTACATCAATGGGCGTGGATAGCGGTTTGACTCACGG GGATTTCCAAGTCTCCACCCCATTGACGTCAATGGGAGTTTGTTTTGGCACCAAAATCAACGGGAC TTTCCAAAATGTCGTAACAACTCCGCCCCATTGACGCAAATGGGCGGGAATTCGAGCTCGGTACTC GAGCGGTGTTCCGCGGTCCTCCTCGTATAGAAACTCGGACCACTCTGAGACGAAGGCTCGCGTCCA GGCCAGCACGAAGGAGGCTAAGTGGGAGGGGTAGCGGTCGTTGTCCACTAGGGGGTCCACTCGCT CCAGGGTGTGAAGACACATGTCGCCCTCTTCGGCATCAAGGAAGGTGATTGGTTTATAGGTGTAGG CCACGTGACCGGGTGTTCCTGAAGGGGGGCTATAAAAGGGGGTGGGGGCGCGTTCGTCCTCACTC TCTTCCGCATCGCTGTCTGCGAGGGCCAGCTGTTGGGCTCGCGGTTGAGGACAAACTCTTCGCGGT CTTTCCAGTACTCTTGGATCGGAAACCCGTCGGCCTCCGAACGGTACTCCGCCACCGAGGGACCTG AGCGAGTCCGCATCGACCGGATCGGAAAACCTCTCGACTGTTGGGGTGAGTACTCCCTCTCAAAA GCGGGCATGACTTCTGCGCTAAGATTGTCAGTTTCCAAAAACGAGGAGGATTTGATATTCACCTGG CCCGCGGTGATGCCTTTGAGGGTGGCCGCGTCCATCTGGTCAGAAAAGACAATCTTTTTGTTGTCA AGCTTGAGGTGTGGCAGGCTTGAGATCTGGCCATACACTTGAGTGACAATGACATCCACTTTGCCT TTCTCTCCACAGGTGTCCACTCCCAGGTCCAACCGGAATTGTACCCGCGGCCAGAGCTTGCGGGCG CCACCGCGGCCGCGGGGATCCAGACATGATAAGATACATTGATGAGTTTGGACAAACCACAACTA GAATGCAGTGAAAAAAATGCTTTATTTGTGAAATTTGTGATGCTATTGCTTTATTTGTAACCATTAT AAGCTGCAATAAACAAGTTAACAACAACAATTGCATTCATTTTATGTTTCAGGTTCAGGGGGAGGT GTGGGAGGTTTTTTCGGATCCTCTTGGCGTAATCATGGTCATAGCTGTTTCCTGTGTGAAATTGTTA TCCGCTCACAATTCCACACAACATACGAGCCGGAAGCATAAAGTGTAAAGCCTGGGGTGCCTAAT GAGTGAGCTAACTCACATTAATTGCGTTGCGCTCACTGCCCGCTTTCCAGTCGGGAAACCTGTCGT GCCAGCTGCATTAATGAATCGGCCAACGCGCGGGGAAAGGCGGTTTGCGTATTGGGCGCTCTTCC |
| GCTTCCTCGCTCACTGACTCGCTGCGCTCGGTCGTTCGGCTGCGGCGAGCGGTATCAGCTCACTCA AAGGCGGTAATACGGTTATCCACAGAATCAGGGGATAACGCAGGAAAGAACATGTGAGCAAAAG GCCAGCAAAAGGCCAGGAACCGTAAAAAGGCCGCGTTGCTGGCGTTCTTCCATAGGCTCCGCCCC CCTGACGAGCATCACAAAAATCGACGCTCAAGTCAGAGGTGGCGAAACCCGACAGGACTATAAAG ATACCAGGCGTTTCCCCCTGGAAGCTCCCTCGTGCGCTCTCCTGTTCCGACCCTGCCGCTTACCGGA TACCTGTCCGCCTTTCTCCCTTCGGGAAGCGTGGCGCTTTCTCATAGCTCACGCTGTAGGTATCTCA GTTCGGTGTAGGTCGTTCGCTCCAAGCTGGGCTGTGTGCACGAACCCCCCGTTCAGCCCGACCGCT GCGCCTTATCCGGTAACTATCGTCTTGAGTCCAACCCGGTAAGACACGACTTATCGCCACTGGCAG CAGCCACTGGTAACAGGATTAGCAGAGCGAGGTATGTAGGCGGTGCTACAGAGTTCTTGAAGTGG TGGCCTAACTACGGCTACACTAGAAGAACAGTATTTGGTATCTGCGCTCTGCTGAAGCCAGTTACC TTCGGAAAAAGAGTTGGTAGCTCTTGATCCGGCAAACAAACCACCGCTGGTAGCGGTGGTTTTTTT GTTTGCAAGCAGCAGATTACGCGCAGAAAAAAAGGATCTCAAGAAGATCCTTTGATCTTTTCTACG GGGTCTGACGCTCAGTGGAACGAAAACTCACGTTAAGGGATTTTGGTCATGAGATTATCAAAAAG GATCTTCACCTAGATCCTTTTAATTAAAAATGAAGTTTTAAATCAATCTAAAGTATATATGAGTA AACTTGGTCTGACAGTTACCAATGCTTAATCAGTGAGGCACCTATCTCAGCGATCTGTCTATTTCGT TCATCCATAGTTGCCTGACTCCCCGTCGTGTAGATAACTACGATACGGGAGGGCTTACCATCTGGC CCCAGTGCTGCAATGATACCGCGAGACCCACGCTCACCGGCTCCAGATTTATCAGCAATAAACCA GCCAGCCGGAAGGGCCGAGCGCAGAAGTGGTCCTGCAACTTTATCCGCCTCCATCCAGTCTATTAA TTGTTGCCGGGAAGCTAGAGTAAGTAGTTCGCCAGTTAATAGTTTGCGCAACGTTGTTGCCATTGC TACAGGCATCGTGGTGTCACGCTCGTCGTTTGGTATGGCTTCATTCAGCTCCGGTTCCCAACGATCA AGGCGAGTTACATGATCCCCCATGTTGTGCAAAAAAGCGGTTAGCTCCTTCGGTCCTCCGATCGTT GTCAGAAGTAAGTTGGCCGCAGTGTTATCACTCATGGTTATGGCAGCACTGCATAATTCTCTTACT GTCATGCCATCCGTAAGATGCTTTTCTGTGACTGGTGAGTACTCAACCAAGTCATTCTGAGAATAG TGTATGCGGCGACCGAGTTGCTCTTGCCCGGCGTCAATACGGGATAATACCGCGCCACATAGCAG AACTTTAAAAGTGCTCATCATTGGAAAACGTTCTTCGGGGCGAAAACTCTCAAGGATCTTACCGCT GTTGAGATCCAGTTCGATGTAACCCACTCGTGCACCCAACTGATCTTCAGCATCTTTTACTTTCACC AGCGTTTCTGGGTGAGCAAAAACAGGAAGGCAAAATGCCGCAAAAAAGGGAATAAGGGCGACAC GGAAATGTTGAATACTCATACTCTTCCTTTTTCAATATTATTGAAGCATTTATCAGGGTTATTGTCT CATGAGCGGATACATATTTGAATGTATTTAGAAAAATAAACAAATAGGGGTTCCGCGCACATTTCC CCGAAAAGTGCCACCTGACGTCTAAGAAACCATTATTATCATGACATTAACCTATAAAAATAGGC GTATCACGAGGCCCTTTCGTCTCGCGCGTTTCGGTGATGACGGTGAAAACCTCTGACACATGCAGC TCCCGGAGACGGTCACAGCTTGTCTGTAAGCGGATGCCGGGAGCAGACAAGCCCGTCAGGGCGCG TCAGCGGGTGTTGGCGGGTGTCGGGGCTGGCTTAACTATGCGGCATCAGAGCAGATTGTACTGAG AGTGCACCATATGCGGTGTGAAATACCGCACAGATGCGTAAGGAGAAAATACCGCATCAGGCGCC ATTCGCCATTCAGGCTGCGCAACTGTTGGGAAGGGCGATCGGTGCGGGCCTCTTCGCTATTACGCC AGCTGGCGAAAGGGGGATGTGCTGCAAGGCGATTAAGTTGGGTAACGCCAGGGTTTTCCCAGTTA CGACGTTGTAAAACGACGGCCAGTGAATT |

Table 5A. Coding Sequence for ABT-325 TEV Polyprotein (SEQ ID NO:41)

| |
|---|
| ATGGAGTTTGGGCTGAGCTGGCTTTTCCTTGTCGCGATTTTAAAAGGTGTCCAGTGTGAGGTGCAGCTGGTGCAGTCTGG AACAGAGGTGAAAAAACCCGGGGAGTCTCTGAAGATCTCCTGTAAGGGTTCTGGATACACTGTTACCAGTTACTGGATCGG CTGGGTGCGCCAGATGCCCGGGAAAGGCCTGGAGTGGATGGGATTCATCTATCCTGGTGACTCTGAAACCAGATACAGTC CGACCTTCCAAGGCCAGGTCACCATCTCAGCCGACAAGTCCTTCAATACCGCCTTCCTGCAGTGGAGCAGTCTAAAGGCC TCGGACACCGCCATGTATTACTGTGCGCGAGTCGGCAGTGGCTGGTACCCTTATACTTTTGATATCTGGGGCCAAGGGAC AATGGTCACCGTCTCTTCAGCGTCGACCAAGGGCCCATCGGTCTTCCCCCTGGCACCCTCCTCCAAGAGCACCTCTGGGG GCACAGCGGCCCTGGGCTGCCTGGTCAAGGACTACTTCCCCGAACCGGTGACGGTGTCGTGGAACTCAGGCGCCCTGAC CAGCGGCGTGCACACCTTCCCGGCTGTCCTACAGTCCTCAGGACTCTACTCCCTCAGCAGCGTGGTGACCGTGCCCTCCA GCAGCTTGGGCACCCAGACCTACATCTGCAACGTGAATCACAAGCCCAGCAACACCAAGGTGGACAAGAAAGTTGAGCCC AAATCTTGTGACAAAACTCACACATGCCCACCGTGCCCAGCACCTGAAGCCGCGGGGGGACCGTCAGTCTTCCTCTTCCC CCCAAAACCCAAGGACACCCTCATGATCTCCCGGACCCCTGAGGTCACATGCGTGGTGGTGGACGTGAGCCACGAAGAC CCTGAGGTCAAGTTCAACTGGTACGTGGACGGCGTGGAGGTGCATAATGCCAAGACAAAGCCGCGGGAGGAGCAGTACA ACAGCACGTACCGTGTGGTCAGCGTCCTCACCGTCCTGCACCAGGACTGGCTGAATGGCAAGGAGTACAAGTGCAAGGT CTCCAACAAAGCCCTCCCAGCCCCCATCGAGAAAACCATCTCCAAAGCCAAAGGGCAGCCCCGAGAACCACAGGTGTACA CCCTGCCCCCATCCCGCGAGGAGATGACCAAGAACCAGGTCAGCCTGACCTGCCTGGTCAAAGGCTTCTATCCCAGCGA CATCGCCGTGGAGTGGGAGAGCAATGGGCAGCCGGAGAACAACTACAAGACCACGCCTCCCGTGCTGGACTCCGACGG CTCCTTCTTCCTCTACAGCAAGCTCACCGTGGACAAGAGCAGGTGGCAGCAGGGGAACGTCTTCTCATGCTCCGTGATGC |
| ATGAGGCTCTGCACAACCACTACACGCAGAAGAGCCTCTCCCTGTCTAGGGGTAAACGCAACCAGTTTATTTCCAGGGG AGCTTGTTTAAGGGGCCGCGTGATTATAACCCAATATCGAGTGCCATTTGTCATCTAACGAATGAATCTGATGGGCACACA ACATCGTTGTATGGTATTGGTTTTGGCCCTTTCATCATCACAAACAAGCATTTGTTTAGAAGAAATAATGGTACACTGTTAGT TCAATCACTACATGGTGTGTTCAAGGTAAAGAATACCACAACTTTGCAACAACACCTCATTGATGGGAGGGACATGATGCT CATTCGCATGCCTAAGGATTTCCCACCATTTCCTCAAAAGCTGAAATTCAGAGAGCCACAAAGGGAAGAGCGCATATGTCT TGTGACAACCAACTTCCAAACTAAGAGCATGTCTAGCATGGTTTCAGATACTAGTTGCACATTCCCTTCATCTGATGGTATA TTCTGGAAACATTGGATTCAGACCAAGGATGGGCACTGTGGTAGCCCGTTGGTGTCAACTAGAGATGGGTTTATTGTTGGT ATACACTCAGCATCAAATTTCACCAACACAAACAATTATTTTACAAGTGTGCCGAAAGACTTCATGGATTTATTGACAAATCA AGAGGCGCAGCAATGGGTTAGTGGTTGGCGATTGAATGCTGACTCAGTGTTATGGGGAGGCCACAAAGTTTTCATGAGCA AACCTGAAGAACCCTTTCAGCCAGTCAAAGAAGCAACTCAACTCATGAGTGAATTAGTCTACTCGCAAGGGATGGAAGCCC CAGCGCAGCTTCTCTTCCTCCTGCTACTCTGGCTCCCAGATACCACTGGAGAAATAGTGATGACGCAGTCTCCAGCCACC CTGTCTGTGTCTCCAGGGGAAAGAGCCACCCTCTCCTGCAGGGCCAGTGAGAGTATTAGCAGCAACTTAGCCTGGTACCA GCAGAAACCTGGCCAGGCTCCCAGGCTCTTCATCTATACTGCATCCACCAGGGCCACTGATATCCCAGCCAGGTTCAGTG GCAGTGGGTCTGGGACAGAGTTCACTCTCACCATCAGCAGCCTGCAGTCTGAAGATTTTGCAGTTTATTACTGTCAGCAGT ATAATAACTGGCCTTCGATCACCTTCGGCCAAGGGACACGACTGGAGATTAAACGAACTGTGGCTGCACCATCTGTCTTCA TCTTCCCGCCATCTGATGAGCAGTTGAAATCTGGAACTGCTAGCGTTGTGTGCCTGCTGAATAACTTCTATCCCAGAGAGG CCAAAGTACAGTGGAAGGTGGATAACGCCCTCCAATCGGGTAACTCCCAGGAGAGTGTCACAGAGCAGGACAGCAAGGA CAGCACCTACAGCCTCAGCAGCACCCTGACGCTGAGCAAAGCAGACTACGAGAAACACAAAGTCTACGCCTGCGAAGTCA CCCATCAGGGCCTGAGCTCGCCCGTCACAAAGAGCTTCAACAGGGGAGAGTGTTGA |

Table 5B. ABT-325 TEV Polyprotein Amino Acid Sequence (SEQ ID NO:42)

| |
|---|
| MEFGLSWLFLVAILKGVQCEVQLVQSGTEVKKPGESLKISCKGSGYTVTSYWIGWVRQMPGKGLEWMGFIYPGDSETRYSPTF QGQVTISADKSFNTAFLQWSSLKASDTAMYYCARVGSGWYPYTFDIWGQGTMVTVSSASTKGPSVFPLAPSSKSTSGGTAALG CLVKDYFPEPVTVSWNSGALTSGVHTFPAVLQSSGLYSLSSVVTVPSSSLGTQTYICNVNHKPSNTKVDKKVEPKSCDKTHTCP PCPAPEAAGGPSVFLFPPKPKDTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPENNYKTT PPVLDSDGSFFLYSKLTVDKSRWQQGNVFSCSVMHEALHNHYTQKSLSLSRGKREPVYFQGSLFKGPRDYNPISSAICHLTNES DGHTTSLYGIGFGPFIITNKHLFRRNNGTLLVQSLHGVFKVKNTTTLQQHLIDGRDMMLIRMPKDFPPFPQKLKFREPQREERICL VTTNFQTKSMSSMVSDTSCTFPSSDGIFWKHWIQTKDGHCGSPLVSTRDGFIVGIHSASNFTNTNNYFTSVPKDFMDLLTNQEA QQWVSGWRLNADSVLWGGHKVFMSKPEEPFQPVKEATQLMSELVYSQGMEAPAQLLFLLLLWLPDTTGEIVMTQSPATLSVS PGERATLSCRASESISSNLAWYQQKPGQAPRLFIYTASTRATDIPARFSGSGSGTEFTLTISSLQSEDFAVYYCQQYNNWPSITF GQGTRLEIKRTVAAPSVFIFPPSDEQLKSGTASVVCLLNNFYPREAKVQWKVDNALQSGNSQESVTEQDSKDSTYSLSSTLTLS KADYEKHKVYACEVTHQGLSSPVTKSFNRGEC* |

Table 5C. Nucleotide Sequence of Complete ABT-325 TEV Polyprotein Expression Vector (SEQ ID NO:43)

| |
|---|
| GAAGTTCCTATTCCGAAGTTCCTATTCTCTAGACGTTACATAACTTACGGTAAATGGCCCGCCTGGCTGACCGCCCAACGA CCCCCGCCCATTGACGTCAATAATGACGTATGTTCCCATAGTAACGCCAATAGGGACTTTCCATTGACGTCAATGGGTGGA GTATTTACGGTAAACTGCCCACTTGGCAGTACATCAAGTGTATCATATGCCAAGTACGCCCCCTATTGACGTCAATGACGG TAAATGGCCCGCCTGGCATTATGCCCAGTACATGACCTTATGGGACTTTCCTACTTGGCAGTACATCTACGTATTAGTCATC GCTATTACCATGGTGATGCGGTTTTGGCAGTACATCAATGGGCGTGGATAGCGGTTTGACTCACGGGGATTTCCAAGTCTC CACCCCATTGACGTCAATGGGAGTTTGTTTTGGCACCAAAATCAACGGGACTTTCCAAAATGTCGTAACAACTCCGCCCCA ATGACGCAAATGGGCAGGGAATTCGAGCTCGGTACTCGAGCGGTGTTCCGCGGTCCTCCTCGTATAGAAACTCGGACCAC TCTGAGACGAAGGCTCGCGTCCAGGCCAGCACGAAGGAGGCTAAGTGGGAGGGGTAGCGGTCGTTGTCCACTAGGGGG TCCACTCGCTCCAGGGTGTGAAGACACATGTCGCCCTCTTCGGCATCAAGGAAGGTGATTGGTTTATAGGTGTAGGCCAC GTGACCGGGTGTTCCTGAAGGGGGGCTATAAAAGGGGGTGGGGGCGCGTTCGTCCTCACTCTCTTCCGCATCGCTGTCT GCGAGGGCCAGCTGTTGGGCTCGCGGTTGAGGACAAACTCTTCGCGGTCTTTCCAGTACTCTTGGATCGGAAACCCGTCG GCCTCCGAACGGTACTCCGCCACCGAGGGACCTGAGCGAGTCCGCATCGACCGGATCGGAAAACCTCTCGACTGTTGGG GTGAGTACTCCCTCTCAAAAGCGGGCATGACTTCTGCGCTAAGATTGTCAGTTTCCAAAAACGAGGAGGATTTGATATTCA CCTGGCCCGCGGTGATGCCTTTGAGGGTGGCCGCGTCCATCTGGTCAGAAAAGACAATCTTTTTGTTGTCAAGCTTGAGG TGTGGCAGGCTTGAGATCTGGCCATACACTTGAGTGACAATGACATCCACTTTGCCTTTCTCTCCACAGGTGTCCACTCCC AGGTCCAACCGGAATTGTACCCGCGGCCAGAGCTTGCCCGGGCGCCACCATGGAGTTTGGGCTGAGCTGGCTTTTCCTT GTCGCGATTTTAAAAGGTGTCCAGTGTGAGGTGCAGCTGGTGCAGTCTGGAACAGAGGTGAAAAAACCCGGGGAGTCTCT GAAGATCTCCTGTAAGGGTTCTGGATACACTGTTACCAGTTACTGGATCGGCTGGGTGCGCCAGATGCCCGGGAAAGGCC |
| TGGAGTGGATGGGATTCATCTATCCTGGTGACTCTGAAACCAGATACAGTCCGACCTTCCAAGGCCAGGTCACCATCTCAG CCGACAAGTCCTTCAATACCGCCTTCCTGCAGTGGAGCAGTCTAAAGGCCTCGGACACCGCCATGTATTACTGTGCGCGA GTCGGCAGTGGCTGGTACCCTTATACTTTTGATATCTGGGGCCAAGGGACAATGGTCACCGTCTCTTCAGCGTCGACCAA GGGCCCATCGGTCTTCCCCCTGGCACCCTCCTCCAAGAGCACCTCTGGGGGCACAGCGGCCCTGGGCTGCCTGGTCAAG GACTACTTCCCCGAACCGGTGACGGTGTCGTGGAACTCAGGCGCCCTGACCAGCGGCGTGCACACCTTCCCGGCTGTCC TACAGTCCTCAGGACTCTACTCCCTCAGCAGCGTGGTGACCGTGCCCTCCAGCAGCTTGGGCACCCAGACCTACATCTGC AACGTGAATCACAAGCCCAGCAACACCAAGGTGGACAAGAAAGTTGAGCCCAAATCTTGTGACAAAACTCACACATGCCCA CCGTGCCCAGCACCTGAAGCCGCGGGGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGCACGTACCGTGTGGTCAGCGTCCTCA CCGTCCTGCACCAGGACTGGCTGAATGGCAAGGAGTACAAGTGCAAGGTCTCCAACAAAGCCCTCCCAGCCCCCATCGA GAAAACCATCTCCAAAGCCAAAGGGCAGCCCCGAGAACCACAGGTGTACACCCTGCCCCCATCCCGCGAGGAGATGACC AAGAACCAGGTCAGCCTGACCTGCCTGGTCAAAGGCTTCTATCCCAGCGACATCGCCGTGGAGTGGGAGAGCAATGGGC AGCCGGAGAACAACTACAAGACCACGCCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAGGCTCTGCACAACCACTACACGCAGAA GAGCCTCTCCCTGTCTAGGGGTAAACGCGAACCAGTTTATTTCCAGGGGAGCTTGTTTAAGGGGCCGCGTGATTATAACC CAATATCGAGTGCCATTTGTCATCTAACGAATGAATCTGATGGGCACACAACATCGTTGTATGGTATTGGTTTTGGCCCTTT CATCATCACAAACAAGCATTTGTTTAGAAGAAATAATGGTACACTGTTAGTTCAATCACTACATGGTGTGTTCAAGGTAAAGA ATACCACAACTTTGCAACAACACCTCATTGATGGGAGGGACATGATGCTCATTCGCATGCCTAAGGATTTCCCACCATTTCC TCAAAAGCTGAAATTCAGAGAGCCACAAAGGGAAGAGCGCATATGTCTTGTGACAACCAACTTCCAAACTAAGAGCATGTC TAGCATGGTTTCAGATACTAGTTGCACATTCCCTTCATCTGATGGTATATTCTGGAAACATTGGATTCAGACCAAGGATGGG CACTGTGGTAGCCCGTTGGTGTCAACTAGAGATGGGTTTATTGTTGGTATACACTCAGCATCAAATTTCACCAACACAAACA ATTATTTTACAAGTGTGCCGAAAGACTTCATGGATTTATTGACAAATCAAGAGGCGCAGCAATGGGTTAGTGGTTGGCGATT GAATGCTGACTCAGTGTTATGGGGAGGCCACAAAGTTTTCATGAGCAAACCTGAAGAACCCTTTCAGCCAGTCAAAGAAGC AACTCAACTCATGAGTGAATTAGTCTACTCGCAAGGGATGGAAGCCCCAGCGCAGCTTCTCTTCCTCCTGCTACTCTGGCT CCCAGATACCACTGGAGAAATAGTGATGACGCAGTCTCCAGCCACCCTGTCTGTGTCTCCAGGGGAAAGAGCCACCCTCT CCTGCAGGGCCAGTGAGAGTATTAGCAGCAACTTAGCCTGGTACCAGCAGAAACCTGGCCAGGCTCCCAGGCTCTTCATC TATACTGCATCCACCAGGGCCACTGATATCCCAGCCAGGTTCAGTGGCAGTGGGTCTGGGACAGAGTTCACTCTCACCAT CAGCAGCCTGCAGTCTGAAGATTTTGCAGTTTATTACTGTCAGCAGTATAATAACTGGCCTTCGATCACCTTCGGCCAAGG GACACGACTGGAGATTAAACGAACTGTGGCTGCACCATCTGTCTTCATCTTCCCGCCATCTGATGAGCAGTTGAAATCTGG AACTGCTAGCGTTGTGTGCCTGCTGAATAACTTCTATCCCAGAGAGGCCAAAGTACAGTGGAAGGTGGATAACGCCCTCC AATCGGGTAACTCCCAGGAGAGTGTCACAGAGCAGGACAGCAAGGACAGCACCTACAGCCTCAGCAGCACCCTGACGCT GAGCAAAGCAGACTACGAGAAACACAAAGTCTACGCCTGCGAAGTCACCCATCAGGGCCTGAGCTCGCCCGTCACAAAGA GCTTCAACAGGGGAGAGTGTTGAGCGGCCGCGTTTAAACTGAATGAGCGCGTCCATCCAGACATGATAAGATACATTGAT GAGTTTGGACAAACCACAACTAGAATGCAGTGAAAAAAATGCTTTATTTGTGAAATTTGTGATGCTATTGCTTTATTTGTAAC CATTATAAGCTGCAATAAACAAGTTAACAACAACAATTGCATTCATTTTATGTTTCAGGTTCAGGGGGAGGTGTGGGAGGTT TTTTAAAGCAAGTAAAACCTCTACAAATGTGGTATGGCTGATTATGATCCGGCTGCCTCGCGCGTTTCGGTGATGACGGTG AAAACCTCTGACACATGCAGCTCCCGGAGACGGTCACAGCTTGTCTGTAAGCGGATGCCGGGAGCAGACAAGCCCGTCA GGGCGCGTCAGCGGGTGTTGGCGGGTGTCGGGGCGCAGCCATGACCGGTCGACGGCGCGCCTTTTTTTTTAATTTTTATT TTATTTTATTTTTGACGCGCCGAAGGCGCGATCTGAGCTCGGTACAGCTTGGCTGTGGAATGTGTGTCAGTTAGGGTGTGG AAAGTCCCCAGGCTCCCCAGCAGGCAGAAGTATGCAAAGCATGCATCTCAATTAGTCAGCAACCAGGTGTGGAAAGTCCC CAGGCTCCCCAGCAGGCAGAAGTATGCAAAGCATGCATCTCAATTAGTCAGCAACCATAGTCCCGCCCCTAACTCCGCCC ATCCCGCCCCTAACTCCGCCCAGTTCCGCCCATTCTCCGCCCCATGGCTGACTAATTTTTTTTATTTATGCAGAGGCCGAG GCCGCCTCGGCCTCTGAGCTATTCCAGAAGTAGTGAGGAGGCTTTTTTGGAGGCCTAGGCTTTTGCAAAAAGCTCCTCGA GGAACTGAAAAACCAGAAAGTTAACTGGTAAGTTTAGTCTTTTTGTCTTTTATTTCAGGTCCCGGATCCGGTGGTGGTGCAA ATCAAAGAACTGCTCCTCAGTGGATGTTGCCTTTACTTCTAGGCCTGTACGGAAGTGTTACTTCTGCTCTAAAAGCTGCGG AATTGTACCCGCGGCCTAATACGACTCACTATAGGGACTAGTATGGTTCGACCATTGAACTGCATCGTCGCCGTGTCCCAA AATATGGGGATTGGCAAGAACGGAGACCTACCCTGGCCTCCGCTCAGGAACGAGTTCAAGTACTTCCAAAGAATGACCAC AACCTCTTCAGTGGAAGGTAAACAGAATCTGGTGATTATGGGTAGGAAAACCTGGTTCTCCATTCCTGAGAAGAATCGACC TTTAAAGGACAGAATTAATATAGTTCTCAGTAGAGAACTCAAAGAACCACCACGAGGAGCTCATTTTCTTGCCAAAAGTTTA GATGATGCCTTAAGACTTATTGAACAACCGGAATTGGCAAGTAAAGTAGACATGGTTTGGATAGTCGGAGGCAGTTCTGTT TACCAGGAAGCCATGAATCAACCAGGCCACCTCAGACTCTTTGTGACAAGGATCATGCAGGAATTTGAAAGTGACACGTTT TTCCCAGAAATTGATTTGGGGAAATATAAACTTCTCCCAGAATACCCAGGCGTCCTCTCTGAGGTCCAGGAGGAAAAAGGC ATCAAGTATAAGTTTGAAGTCTACGAGAAGAAAGACTAAGCGGCCGAGCGCGCGGATCTGGAAACGGGAGATGGGGGAG GCTAACTGAAGCACGGAAGGAGACAATACCGGAAGGAACCCGCGCTATGACGGCAATAAAAAGACAGAATAAAACGCACG GGTGTTGGGTCGTTTGTTCATAAACGCGGGGTTCGGTCCCAGGGCTGGCACTCTGTCGATACCCCACCGAGACCCCATTG GGGCCAATACGCCCGCGTTTCTTCCTTTTCCCCACCCCACCCCCCAAGTTCGGGTGAAGGCCCAGGGCTCGCAGCCAAC GTCGGGGCGGCAGGCCCTGCCATAGCCACTGGCCCCGTGGGTTAGGGACGGGGTCCCCCATGGGGAATGGTTTATGGT TCGTGGGGGTTATTATTTTGGGCGTTGCGTGGGGTCTGGAGATCCCCCGGGCTGCAGGAATTCCGTTACATTACTTACGG TAAATGGCCCGCCTGGCTGACCGCCCAACGACCCCCGCCCATTGACGTCAATAATGACGTATGTTCCCATAGTAACGCCA |
| ATAGGGACTTTCCATTGACGTCAATGGGTGGAGTATTTACGGTAAACTGCCCACTTGGCAGTACATCAAGTGTATCATATG CCAAGTACGCCCCCTATTGACGTCAATGACGGTAAATGGCCCGCCTGGCATTATGCCCAGTACATGACCTTATGGGACTTT CCTACTTGGCAGTACATCTACGTATTAGTCATCGCTATTACCATGGTGATGCGGTTTTGGCAGTACATCAATGGGCGTGGA TAGCGGTTTGACTCACGGGGATTTCCAAGTCTCCACCCCATTGACGTCAATGGGAGTTTGTTTTGGCACCAAAATCAACGG GACTTTCCAAAATGTCGTAACAACTCCGCCCCATTGACGCAAAGGGCGGAATTCGAGCTCGGTACTCGAGCGGTGTTC CGCGGTCCTCCTCGTATAGAAACTCGGACCACTCTGAGACGAAGGCTCGCGTCCAGGCCAGCACGAAGGAGGCTAAGTG GGAGGGGTAGCGGTCGTTGTCCACTAGGGGGTCCACTCGCTCCAGGGTGTGAAGACACATGTCGCCCTCTTCGGCATCA AGGAAGGTGATTGGTTTATAGGTGTAGGCCACGTGACCGGGTGTTCCTGAAGGGGGGCTATAAAAGGGGGTGGGGGCGC GTTCGTCCTCACTCTCTTCCGCATCGCTGTCTGCGAGGGCCAGCTGTTGGGCTCGCGGTTGAGGACAAACTCTTCGCGGT CTTTCCAGTACTCTTGGATCGGAAACCCGTCGGCCTCCGAACGGTACTCCGCCACCGAGGGACCTGAGCGAGTCCGCAT CGACCGGATCGGAAAACCTCTCGACTGTTGGGGTGAGTACTCCCTCTCAAAAGCGGGCATGACTTCTGCGCTAAGATTGT CAGTTTCCAAAAACGAGGAGGATTTGATATTCACCTGGCCCGCGGTGATGCCTTTGAGGGTGGCCGCGTCCATCTGGTCA GAAAAGACAATCTTTTTGTTGTCAAGCTTGAGGTGTGGCAGGCTTGAGATCTGGCCATACACTTGAGTGACAATGACATCC ACTTTGCCTTTCTCTCCACAGGTGTCCACTCCCAGGTCCAACCGGAATTGTACCCGCGGCCAGAGCTTGCGGGCGCCACC GCGGCCGCGGGGATCCAGACATGATAAGATACATTGATGAGTTTGGACAAACCACAACTAGAATGCAGTGAAAAAAATGCT TTATTTGTGAAATTTGTGATGCTATTGCTTTATTTGTAACCATTATAAGCTGCAATAAACAAGTTAACAACAACAATTGCATTC ATTTTATGTTTCAGGTTCAGGGGGAGGTGTGGGAGGTTTTTTCGGATCCTCTTGGCGTAATCATGGTCATAGCTGTTTCCT GTGTGAAATTGTTATCCGCTCACAATTCCACACAACATACGAGCCGGAAGCATAAAGTGTAAAGCCTGGGGTGCCTAATGA GTGAGCTAACTCACATTAATTGCGTTGCGCTCACTGCCCGCTTTCCAGTCGGGAAACCTGTCGTGCCAGCTGCATTAATGA ATCGGCCAACGCGCGGGGAAAGGCGGTTTGCGTATTGGGCGCTCTTCCGCTTCCTCGCTCACTGACTCGCTGCGCTCGG TCGTTCGGCTGCGGCGAGCGGTATCAGCTCACTCAAAGGCGGTAATACGGTTATCCACAGAATCAGGGGATAACGCAGGA AAGAACATGTGAGCAAAAGGCCAGCAAAAGGCCAGGAACCGTAAAAAGGCCGCGTTGCTGGCGTTTTTCCATAGGCTCCG CCCCCCTGACGAGCATCACAAAAATCGACGCTCAAGTCAGAGGTGGCGAAACCCGACAGGACTATAAAGATACCAGGCGT TTCCCCCTGGAAGCTCCCTCGTGCGCTCTCCTGTTCCGACCCTGCCGCTTACCGGATACCTGTCCGCCTTTCTCCCTTCG GGAAGCGTGGCGCTTTCTCATAGCTCACGCTGTAGGTATCTCAGTTCGGTGTAGGTCGTTCGCTCCAAGCTGGGCTGTGT GCACGAACCCCCCGTTCAGCCCGACCGCTGCGCCTTATCCGGTAACTATCGTCTTGAGTCCAACCCGGTAAGACACGACT TATCGCCACTGGCAGCAGCCACTGGTAACAGGATTAGCAGAGCGAGGTATGTAGGCGGTGCTACAGAGTTCTTGAAGTGG TGGCCTAACTACGGCTACACTAGAAGAACAGTATTTGGTATCTGCGCTCTGCTGAAGCCAGTTACCTTCGGAAAAAGAGTT GGTAGCTCTTGATCCGGCAAACAAACCACCGCTGGTAGCGGTGGTTTTTTTGTTTGCAAGCAGCAGATTACGCGCAGAAAA AAAGGATCTCAAGAAGATCCTTTGATCTTTTCTACGGGGTCTGACGCTCAGTGGAACGAAAACTCACGTTAAGGGATTTTG GTCATGAGATTATCAAAAAGGATCTTCACCTAGATCCTTTTAAATTAAAAATGAAGTTTTAAATCAATCTAAAGTATATATGAG TAAACTTGGTCTGACAGTTACCAATGCTTAATCAGTGAGGCACCTATCTCAGCGATCTGTCTATTTCGTTCATCCATAGTTG CCTGACTCCCCGTCGTGTAGATAACTACGATACGGGAGGGCTTACCATCTGGCCCCAGTGCTGCAATGATACCGCGAGAC CCACGCTCACCGGCTCCAGATTTATCAGCAATAAACCAGCCAGCCGGAAGGGCCGAGCGCAGAAGTGGTCCTGCAACTTT ATCCGCCTCCATCCAGTCTATTAATTGTTGCCGGGAAGCTAGAGTAAGTAGTTCGCCAGTTAATAGTTTGCGCAACGTTGTT GCCATTGCTACAGGCATCGTGGTGTCACGCTCGTCGTTTGGTATGGCTTCATTCAGCTCCGGTTCCCAACGATCAAGGCG AGTTACATGATCCCCCATGTTGTGCAAAAAAGCGGTTAGCTCCTTCGGTCCTCCGATCGTTGTCAGAAGTAAGTTGGCCGC AGTGTTATCACTCATGGTTATGGCAGCACTGCATAATTCTCTTACTGTCATGCCATCCGTAAGATGCTTTTCTGTGACTGGT GAGTACTCAACCAAGTCATTCTGAGAATAGTGTATGCGGCGACCGAGTTGCTCTTGCCCGGCGTCAATACGGGATAATACC GCGCCACATAGCAGAACTTTAAAAGTGCTCATCATTGGAAAACGTTCTTCGGGGCGAAAACTCTCAAGGATCTTACCGCTG TTGAGATCCAGTTCGATGTAACCCACTCGTGCACCCAACTGATCTTCAGCATCTTTTACTTTCACCAGCGTTTCTGGGTGAG CAAAAACAGGAAGGCAAAATGCCGCAAAAAAGGGAATAAGGGCGACACGGAAATGTTGAATACTCATACTCTTCCTTTTTC AATATTATTGAAGCATTTATCAGGGTTATTGTCTCATGAGCGGATACATATTTGAATGTATTTAGAAAAATAAACAAATAGGG GTTCCGCGCACATTTCCCCGAAAAGTGCCACCTGACGTCTAAGAAACCATTATTATCATGACATTAACCTATAAAAATAGGC GTATCACGAGGCCCTTTCGTCTCGCGCGTTTCGGTGATGACGGTGAAAACCTCTGACACATGCAGCTCCCGGAGACGGTC ACAGCTTGTCTGTAAGCGGATGCCGGGAGCAGACAAGCCCGTCAGGGCGCGTCAGCGGGTGTTGGCGGGTGTCGGGGC TGGCTTAACTATGCGGCATCAGAGCAGATTGTACTGAGAGTGCACCATATGCGGTGTGAAATACCGCACAGATGCGTAAG GAGAAAATACCGCATCAGGCGCCATTCGCCATTCAGGCTGCGCAACTGTTGGGAAGGGCGATCGGTGCGGGCCTCTTCG CTATTACGCCAGCTGGCGAAAGGGGGATGTGCTGCAAGGCGATTAAGTTGGGTAACGCCAGGGTTTTCCCAGTTACGACG TTGTAAAACGACGGCCAGTGAATT |

Table 6A. Coding Sequence for D2E7 LC-LC-HC Polyprotein Construct (SEQ ID NO:29)

| |
|---|
| ATGGACATGCGCGTGCCCGCCCAGCTGCTGGGCCTGCTGCTGCTGTGGTTCCCCGGCTCGCGATGCGACATCCAGATGA CCCAGTCTCCATCCTCCCTGTCTGCATCTGTAGGGGACAGAGTCACCATCACTTGTCGGGCAAGTCAGGGCATCAGAAAT TACTTAGCCTGGTATCAGCAAAAACCAGGGAAAGCCCCTAAGCTCCTGATCTATGCTGCATCCACTTTGCAATCAGGGGTC CCATCTCGGTTCAGTGGCAGTGGATCTGGGACAGATTTCACTCTCACCATCAGCAGCCTACAGCCTGAAGATGTTGCAACT |
| TATTACTGTCAAAGGTATAACCGTGCACCGTATACTTTTGGCCAGGGGACCAAGGTGGAAATCAAACGTACGGTGGCTGCA CCATCTGTCTTCATCTTCCCGCCATCTGATGAGCAGTTGAAATCTGGAACTGCCTCTGTTGTGTGCCTGCTGAATAACTTCT ATCCCAGAGAGGCCAAAGTACAGTGGAAGGTGGATAACGCCCTCCAATCGGGTAACTCCCAGGAGAGTGTCACAGAGCA GGACAGCAAGGACAGCACCTACAGCCTCAGCAGCACCCTGACGCTGAGCAAAGCAGACTACGAGAAACACAAAGTCTAC GCCTGCGAAGTCACCCATCAGGGCCTGAGCTCGCCCGTCACAAAGAGCTTCAACAGGGGAAGGTGTAAGAGACTTCTCAA GTTGGCAGGAGACGTTGAGTCCAACCCTGGGCCCATGGACATGCGCGTGCCCGCCCAGCTGCTGGGCCTGCTGCTGCTG TGGTTCCCCGGCTCGCGATGCGACATCCAGATGACCCAGTCTCCATCCTCCCTGTCTGCATCTGTAGGGGACAGAGTCAC CATCACTTGTCGGGCAAGTCAGGGCATCAGAAATTACTTAGCCTGGTATCAGCAAAAACCAGGGAAAGCCCCTAAGCTCCT GATCTATGCTGCATCCACTTTGCAATCAGGGGTCCCATCTCGGTTCAGTGGCAGTGGATCTGGGACAGATTTCACTCTCAC CATCAGCAGCCTACAGCCTGAAGATGTTGCAACTTATTACTGTCAAAGGTATAACCGTGCACCGTATACTTTTGGCCAGGG GACCAAGGTGGAAATCAAACGTACGGTGGCTGCACCATCTGTCTTCATCTTCCCGCCATCTGATGAGCAGTTGAAATCTGG AACTGCCTCTGTTGTGTGCCTGCTGAATAACTTCTATCCCAGAGAGGCCAAAGTACAGTGGAAGGTGGATAACGCCCTCCA ATCGGGTAACTCCCAGGAGAGTGTCACAGAGCAGGACAGCAAGGACAGCACCTACAGCCTCAGCAGCACCCTGACGCTG AGCAAAGCAGACTACGAGAAACACAAAGTCTACGCCTGCGAAGTCACCCATCAGGGCCTGAGCTCGCCCGTCACAAAGAG CTTCAACAGGGGAAGGTGTAAGAGACTTCTCAAGTTGGCAGGAGACGTTGAGTCCAACCCTGGGCCCATGGAGTTTGGGC TGAGCTGGCTTTTTCTTGTCGCGATTTTAAAAGGTGTCCAGTGTGAGGTGCAGCTGGTGGAGTCTGGGGGAGGCTTGGTA CAGCCCGGCAGGTCCCTGAGACTCTCCTGTGCGGCCTCTGGATTCACCTTTGATGATTATGCCATGCACTGGGTCCGGCA AGCTCCAGGGAAGGGCCTGGAATGGGTCTCAGCTATCACTTGGAATAGTGGTCACATAGACTATGCGGACTCTGTGGAGG GCCGATTCACCATCTCCAGAGACAACGCCAAGAACTCCCTGTATCTGCAAATGAACAGTCTGAGAGCTGAGGATACGGCC GTATATTACTGTGCGAAAGTCTCGTACCTTAGCACCGCGTCCTCCCTTGACTATTGGGGCCAAGGTACCCTGGTCACCGTC TCGAGTGCGTCGACCAAGGGCCCATCGGTCTTCCCCCTGGCACCCTCCTCCAAGAGCACCTCTGGGGGCACAGCGGCCC TGGGCTGCCTGGTCAAGGACTACTTCCCCGAACCGGTGACGGTGTCGTGGAACTCAGGCGCCCTGACCAGCGGCGTGCA CACCTTCCCGGCTGTCCTACAGTCCTCAGGACTCTACTCCCTCAGCAGCGTGGTGACCGTGCCCTCCAGCAGCTTGGGCA CCCAGACCTACATCTGCAACGTGAATCACAAGCCCAGCAACACCAAGGTGGACAAGAAAGTTGAGCCCAAATCTTGTGAC AAAACTCACACATGCCCACCGTGCCCAGCACCTGAACTCCTGGGGGGACCGTCAGTCTTCCTCTTCCCCCCAAAACCCAA GGACACCCTCATGATCTCCCGGACCCCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGGGAGGAGCAGTACAACAGCACGTACC GTGTGGTCAGCGTCCTCACCGTCCTGCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAACCACAGGTGTACACCCTGCCCCCATC CCGGGATGAGCTGACCAAGAACCAGGTCAGCCTGACCTGCCTGGTCAAAGGCTTCTATCCCAGCGACATCGCCGTGGAG TGGGAGAGCAATGGGCAGCCGGAGAACAACTACAAGACCACGCCTCCCGTGCTGGACTCCGACGGCTCCTTCTTCCTCTA CAGCAAGCTCACCGTGGACAAGAGCAGGTGGCAGCAGGGGAACGTCTTCTCATGCTCCGTGATGCATGAGGCTCTGCAC AACCACTACACGCAGAAGAGCCTCTCCCTGTCTCCGGGTAAATGA |

Table 6B. D2E7 LC-LC-HC Polyprotein Amino Acid Sequence (SEQ ID NO:30)

| |
|---|
| * |

Table 6C. Complete Nucleotide Sequence of the D2E7 LC-LC-HC Polyprotein Expression Vector DNA Sequence(SEQ ID NO:31)

| |
|---|
| GAAGTTCCTATTCCGAAGTTCCTATTCTCTAGACGTTACATAACTTACGGTAAATGGCCCGCCTGGCTGACCGCCCAACGA CCCCCGCCCATTGACGTCAATAATGACGTATGTTCCCATAGTAACGCCAATAGGGACTTTCCATTGACGTCAATGGGTGGA GTATTTACGGTAAACTGCCCACTTGGCAGTACATCAAGTGTATCATATGCCAAGTACGCCCCCTATTGACGTCAATGACGG |
| TAAATGGCCCGCCTGGCATTATGCCCAGTACATGACCTTATGGGACTTTCCTACTTGGCAGTACATCTACGTATTAGTCATC GCTATTACCATGGTGATGCGGTTTTGGCAGTACATCAATGGGCGTGGATAGCGGTTTGACTCACGGGGATTTCCAAGTCTC CACCCCATTGACGTCAATGGGAGTTTGTTTTGGCACCAAAATCAACGGGACTTTCCAAAATGTCGTAACAACTCCGCCCCA ATGACGCAAATGGGCAGGGAATTCGAGCTCGGTACTCGAGCGGTGTTCCGCGGTCCTCCTCGTATAGAAACTCGGACCAC TCTGAGACGAAGGCTCGCGTCCAGGCCAGCACGAAGGAGGCTAAGTGGGAGGGGTAGCGGTCGTTGTCCACTAGGGGG TCCACTCGCTCCAGGGTGTGAAGACACATGTCGCCCTCTTCGGCATCAAGGAAGGTGATTGGTTTATAGGTGTAGGCCAC GTGACCGGGTGTTCCTGAAGGGGGGCTATAAAAGGGGGTGGGGGCGCGTTCGTCCTCACTCTCTTCCGCATCGCTGTCT GCGAGGGCCAGCTGTTGGGCTCGCGGTTGAGGACAAACTCTTCGCGGTCTTTCCAGTACTCTTGGATCGGAAACCCGTCG GCCTCCGAACGGTACTCCGCCACCGAGGGACCTGAGCGAGTCCGCATCGACCGGATCGGAAAACCTCTCGACTGTTGGG GTGAGTACTCCCTCTCAAAAGCGGGCATGACTTCTGCGCTAAGATTGTCAGTTTCCAAAAACGAGGAGGATTTGATATTCA CCTGGCCCGCGGTGATGCCTTTGAGGGTGGCCGCGTCCATCTGGTCAGAAAAGACAATCTTTTTGTTGTCAAGCTTGAGG TGTGGCAGGCTTGAGATCTGGCCATACACTTGAGTGACAATGACATCCACTTTGCCTTTCTCTCCACAGGTGTCCACTCCC AGGTCCAACCGGAATTGTACCCGCGGCCAGAGCTTGCCCGGGCGCCACCATGGACATGCGCGTGCCCGCCCAGCTGCT GGGCCTGCTGCTGCTGTGGTTCCCCGGCTCGCGATGCGACATCCAGATGACCCAGTCTCCATCCTCCCTGTCTGCATCTG TAGGGGACAGAGTCACCATCACTTGTCGGGCAAGTCAGGGCATCAGAAATTACTTAGCCTGGTATCAGCAAAAACCAGGG AAAGCCCCTAAGCTCCTGATCTATGCTGCATCCACTTTGCAATCAGGGGTCCCATCTCGGTTCAGTGGCAGTGGATCTGG GACAGATTTCACTCTCACCATCAGCAGCCTACAGCCTGAAGATGTTGCAACTTATTACTGTCAAAGGTATAACCGTGCACC GTATACTTTTGGCCAGGGGACCAAGGTGGAAATCAAACGTACGGTGGCTGCACCATCTGTCTTCATCTTCCCGCCATCTGA TGAGCAGTTGAAATCTGGAACTGCCTCTGTTGTGTGCCTGCTGAATAACTTCTATCCCAGAGAGGCCAAAGTACAGTGGAA GGTGGATAACGCCCTCCAATCGGGTAACTCCCAGGAGAGTGTCACAGAGCAGGACAGCAAGGACAGCACCTACAGCCTC AGCAGCACCCTGACGCTGAGCAAAGCAGACTACGAGAAACACAAAGTCTACGCCTGCGAAGTCACCCATCAGGGCCTGA GCTCGCCCGTCACAAAGAGCTTCAACAGGGGAAGGTGTAAGAGACTTCTCAAGTTGGCAGGAGACGTTGAGTCCAACCCT GGGCCCATGGACATGCGCGTGCCCGCCCAGCTGCTGGGCCTGCTGCTGCTGTGGTTCCCCGGCTCGCGATGCGACATC CAGATGACCCAGTCTCCATCCTCCCTGTCTGCATCTGTAGGGGACAGAGTCACCATCACTTGTCGGGCAAGTCAGGGCAT CAGAAATTACTTAGCCTGGTATCAGCAAAAACCAGGGAAAGCCCCTAAGCTCCTGATCTATGCTGCATCCACTTTGCAATC AGGGGTCCCATCTCGGTTCAGTGGCAGTGGATCTGGGACAGATTTCACTCTCACCATCAGCAGCCTACAGCCTGAAGATG TTGCAACTTATTACTGTCAAAGGTATAACCGTGCACCGTATACTTTTGGCCAGGGGACCAAGGTGGAAATCAAACGTACGG TGGCTGCACCATCTGTCTTCATCTTCCCGCCATCTGATGAGCAGTTGAAATCTGGAACTGCCTCTGTTGTGTGCCTGCTGA ATAACTTCTATCCCAGAGAGGCCAAAGTACAGTGGAAGGTGGATAACGCCCTCCAATCGGGTAACTCCCAGGAGAGTGTC ACAGAGCAGGACAGCAAGGACAGCACCTACAGCCTCAGCAGCACCCTGACGCTGAGCAAAGCAGACTACGAGAAACACA AAGTCTACGCCTGCGAAGTCACCCATCAGGGCCTGAGCTCGCCCGTCACAAAGAGCTTCAACAGGGGAAGGTGTAAGAG ACTTCTCAAGTTGGCAGGAGACGTTGAGTCCAACCCTGGGCCCATGGAGTTTGGGCTGAGCTGGCTTTTTCTTGTCGCGA TTTTAAAAGGTGTCCAGTGTGAGGTGCAGCTGGTGGAGTCTGGGGGAGGCTTGGTACAGCCCGGCAGGTCCCTGAGACT CTCCTGTGCGGCCTCTGGATTCACCTTTGATGATTATGCCATGCACTGGGTCCGGCAAGCTCCAGGGAAGGGCCTGGAAT GGGTCTCAGCTATCACTTGGAATAGTGGTCACATAGACTATGCGGACTCTGTGGAGGGCCGATTCACCATCTCCAGAGAC AACGCCAAGAACTCCCTGTATCTGCAAATGAACAGTCTGAGAGCTGAGGATACGGCCGTATATTACTGTGCGAAAGTCTCG TACCTTAGCACCGCGTCCTCCCTTGACTATTGGGGCCAAGGTACCCTGGTCACCGTCTCGAGTGCGTCGACCAAGGGCCC ATCGGTCTTCCCCCTGGCACCCTCCTCCAAGAGCACCTCTGGGGGCACAGCGGCCCTGGGCTGCCTGGTCAAGGACTAC TTCCCCGAACCGGTGACGGTGTCGTGGAACTCAGGCGCCCTGACCAGCGGCGTGCACACCTTCCCGGCTGTCCTACAGT CCTCAGGACTCTACTCCCTCAGCAGCGTGGTGACCGTGCCCTCCAGCAGCTTGGGCACCCAGACCTACATCTGCAACGTG AATCACAAGCCCAGCAACACCAAGGTGGACAAGAAAGTTGAGCCCAAATCTTGTGACAAAACTCACACATGCCCACCGTG CCCAGCACCTGAACTCCTGGGGGGACCGTCAGTCTTCCTCTTCCCCCCAAAACCCAAGGACACCCTCATGATCTCCCGGA CCCCTGAGGTCACATGCGTGGTGGTGGACGTGAGCCACGAAGACCCTGAGGTCAAGTTCAACTGGTACGTGGACGGCGT GGAGGTGCATAATGCCAAGACAAAGCCGCGGGAGGAGCAGTACAACAGCACGTACCGTGTGGTCAGCGTCCTCACCGTC CTGCACCAGGACTGGCTGAATGGCAAGGAGTACAAGTGCAAGGTCTCCAACAAAGCCCTCCCAGCCCCCATCGAGAAAAC CATCTCCAAAGCCAAAGGGCAGCCCCGAGAACCACAGGTGTACACCCTGCCCCCATCCCGGGATGAGCTGACCAAGAAC CAGGTCAGCCTGACCTGCCTGGTCAAAGGCTTCTATCCCAGCGACATCGCCGTGGAGTGGGAGAGCAATGGGCAGCCGG AGAACAACTACAAGACCACGCCTCCCGTGCTGGACTCCGACGGCTCCTTCTTCCTCTACAGCAAGCTCACCGTGGACAAG AGCAGGTGGCAGCAGGGGAACGTCTTCTCATGCTCCGTGATGCATGAGGCTCTGCACAACCACTACACGCAGAAGAGCCT CTCCCTGTCTCCGGGTAAATGAGAATTAGTCTACTCGCAAGGGGCGGCCGCGTTTAAACTGAATGAGCGCGTCCATCCAG ACATGATAAGATACATTGATGAGTTTGGACAAACCACAACTAGAATGCAGTGAAAAAAATGCTTTATTTGTGAAATTTGTGAT GCTATTGCTTTATTTGTAACCATTATAAGCTGCAATAAACAAGTTAACAACAACAATTGCATTCATTTTATGTTTCAGGTTCAG GGGGAGGTGTGGGAGGTTTTTTAAAGCAAGTAAAACCTCTACAAATGTGGTATGGCTGATTATGATCCGGCTGCCTCGCG CGTTTCGGTGATGACGGTGAAAACCTCTGACACATGCAGCTCCCGGAGACGGTCACAGCTTGTCTGTAAGCGGATGCCGG GAGCAGACAAGCCCGTCAGGGCGCGTCAGCGGGTGTTGGCGGGTGTCGGGGCGCAGCCATGACCGGTCGACGGCGCG CCTTTTTTTTTAATTTTTATTTTATTTTATTTTTGACGCGCCGAAGGCGCGATCTGAGCTCGGTACAGCTTGGCTGTGGAATG TGTGTCAGTTAGGGTGTGGAAAGTCCCCAGGCTCCCCAGCAGGCAGAAGTATGCAAAGCATGCATCTCAATTAGTCAGCA ACCAGGTGTGGAAAGTCCCCAGGCTCCCCAGCAGGCAGAAGTATGCAAAGCATGCATCTCAATTAGTCAGCAACCATAGT CCCGCCCCTAACTCCGCCCATCCCGCCCCTAACTCCGCCCAGTTCCGCCCATTCTCCGCCCCATGGCTGACTAATTTTTTT TATTTATGCAGAGGCCGAGGCCGCCTCGGCCTCTGAGCTATTCCAGAAGTAGTGAGGAGGCTTTTTTGGAGGCCTAGGCT |
| TTTGCAAAAAGCTCCTCGAGGAACTGAAAAACCAGAAAGTTAACTGGTAAGTTTAGTCTTTTTGTCTTTTATTTCAGGTCCCG GATCCGGTGGTGGTGCAAATCAAAGAACTGCTCCTCAGTGGATGTTGCCTTTACTTCTAGGCCTGTACGGAAGTGTTACTT CTGCTCTAAAAGCTGCGGAATTGTACCCGCGGCCTAATACGACTCACTATAGGGACTAGTATGGTTCGACCATTGAACTGC ATCGTCGCCGTGTCCCAAAATATGGGGATTGGCAAGAACGGAGACCTACCCTGGCCTCCGCTCAGGAACGAGTTCAAGTA CTTCCAAAGAATGACCACAACCTCTTCAGTGGAAGGTAAACAGAATCTGGTGATTATGGGTAGGAAAACCTGGTTCTCCAT TCCTGAGAAGAATCGACCTTTAAAGGACAGAATTAATATAGTTCTCAGTAGAGAACTCAAAGAACCACCACGAGGAGCTCA TTTTCTTGCCAAAAGTTTAGATGATGCCTTAAGACTTATTGAACAACCGGAATTGGCAAGTAAAGTAGACATGGTTTGGATA GTCGGAGGCAGTTCTGTTTACCAGGAAGCCATGAATCAACCAGGCCACCTCAGACTCTTTGTGACAAGGATCATGCAGGA ATTTGAAAGTGACACGTTTTTCCCAGAAATTGATTTGGGGAAATATAAACTTCTCCCAGAATACCCAGGCGTCCTCTCTGAG GTCCAGGAGGAAAAAGGCATCAAGTATAAGTTTGAAGTCTACGAGAAGAAAGACTAAGCGGCCGAGCGCGCGGATCTGGA AACGGGAGATGGGGGAGGCTAACTGAAGCACGGAAGGAGACAATACCGGAAGGAACCCGCGCTATGACGGCAATAAAAA GACAGAATAAAACGCACGGGTGTTGGGTCGTTTGTTCATAAACGCGGGGTTCGGTCCCAGGGCTGGCACTCTGTCGATAC CCCACCGAGACCCCATTGGGGCCAATACGCCCGCGTTTCTTCCTTTTCCCCACCCCACCCCCCAAGTTCGGGTGAAGGCC CAGGGCTCGCAGCCAACGTCGGGGCGGCAGGCCCTGCCATAGCCACTGGCCCCGTGGGTTAGGGACGGGGTCCCCCAT GGGGAATGGTTTATGGTTCGTGGGGGTTATTATTTTGGGCGTTGCGTGGGGTCTGGAGATCCCCCGGGCTGCAGGAATTC CGTTACATTACTTACGGTAAATGGCCCGCCTGGCTGACCGCCCAACGACCCCCGCCCATTGACGTCAATAATGACGTATGT TCCCATAGTAACGCCAATAGGGACTTTCCATTGACGTCAATGGGTGGAGTATTTACGGTAAACTGCCCACTTGGCAGTACA TCAAGTGTATCATATGCCAAGTACGCCCCCTATTGACGTCAATGACGGTAAATGGCCCGCCTGGCATTATGCCCAGTACAT GACCTTATGGGACTTTCCTACTTGGCAGTACATCTACGTATTAGTCATCGCTATTACCATGGTGATGCGGTTTTGGCAGTAC ATCAATGGGCGTGGATAGCGGTTTGACTCACGGGGATTTCCAAGTCTCCACCCCATTGACGTCAATGGGAGTTTGTTTTGG CACCAAAATCAACGGGACTTTCCAAAATGTCGTAACAACTCCGCCCCATTGACGCAAATGGGCGGGAATTCGAGCTCGGT ACTCGAGCGGTGTTCCGCGGTCCTCCTCGTATAGAAACTCGGACCACTCTGAGACGAAGGCTCGCGTCCAGGCCAGCAC GAAGGAGGCTAAGTGGGAGGGGTAGCGGTCGTTGTCCACTAGGGGGTCCACTCGCTCCAGGGTGTGAAGACACATGTCG CCCTCTTCGGCATCAAGGAAGGTGATTGGTTTATAGGTGTAGGCCACGTGACCGGGTGTTCCTGAAGGGGGGCTATAAAA GGGGGTGGGGGCGCGTTCGTCCTCACTCTCTTCCGCATCGCTGTCTGCGAGGGCCAGCTGTTGGGCTCGCGGTTGAGGA CAAACTCTTCGCGGTCTTTCCAGTACTCTTGGATCGGAAACCCGTCGGCCTCCGAACGGTACTCCGCCACCGAGGGACCT GAGCGAGTCCGCATCGACCGGATCGGAAAACCTCTCGACTGTTGGGGTGAGTACTCCCTCTCAAAAGCGGGCATGACTTC TGCGCTAAGATTGTCAGTTTCCAAAAACGAGGAGGATTTGATATTCACCTGGCCCGCGGTGATGCCTTTGAGGGTGGCCG CGTCCATCTGGTCAGAAAAGACAATCTTTTTGTTGTCAAGCTTGAGGTGTGGCAGGCTTGAGATCTGGCCATACACTTGAG TGACAATGACATCCACTTTGCCTTTCTCTCCACAGGTGTCCACTCCCAGGTCCAACCGGAATTGTACCCGCGGCCAGAGCT TGCGGGCGCCACCGCGGCCGCGGGGATCCAGACATGATAAGATACATTGATGAGTTTGGACAAACCACAACTAGAATGCA GTGAAAAAAATGCTTTATTTGTGAAATTTGTGATGCTATTGCTTTATTTGTAACCATTATAAGCTGCAATAAACAAGTTAACAA CAACAATTGCATTCATTTTATGTTTCAGGTTCAGGGGGAGGTGTGGGAGGTTTTTTCGGATCCTCTTGGCGTAATCATGGTC ATAGCTGTTTCCTGTGTGAAATTGTTATCCGCTCACAATTCCACACAACATACGAGCCGGAAGCATAAAGTGTAAAGCCTG GGGTGCCTAATGAGTGAGCTAACTCACATTAATTGCGTTGCGCTCACTGCCCGCTTTCCAGTCGGGAAACCTGTCGTGCC AGCTGCATTAATGAATCGGCCAACGCGCGGGGAAAGGCGGTTTGCGTATTGGGCGCTCTTCCGCTTCCTCGCTCACTGAC TCGCTGCGCTCGGTCGTTCGGCTGCGGCGAGCGGTATCAGCTCACTCAAAGGCGGTAATACGGTTATCCACAGAATCAGG GGATAACGCAGGAAAGAACATGTGAGCAAAAGGCCAGCAAAAGGCCAGGAACCGTAAAAAGGCCGCGTTGCTGGCGTTC TTCCATAGGCTCCGCCCCCCTGACGAGCATCACAAAAATCGACGCTCAAGTCAGAGGTGGCGAAACCCGACAGGACTATA AAGATACCAGGCGTTTCCCCCTGGAAGCTCCCTCGTGCGCTCTCCTGTTCCGACCCTGCCGCTTACCGGATACCTGTCCG CCTTTCTCCCTTCGGGAAGCGTGGCGCTTTCTCATAGCTCACGCTGTAGGTATCTCAGTTCGGTGTAGGTCGTTCGCTCCA AGCTGGGCTGTGTGCACGAACCCCCCGTTCAGCCCGACCGCTGCGCCTTATCCGGTAACTATCGTCTTGAGTCCAACCCG GTAAGACACGACTTATCGCCACTGGCAGCAGCCACTGGTAACAGGATTAGCAGAGCGAGGTATGTAGGCGGTGCTACAGA GTTCTTGAAGTGGTGGCCTAACTACGGCTACACTAGAAGAACAGTATTTGGTATCTGCGCTCTGCTGAAGCCAGTTACCTT CGGAAAAAGAGTTGGTAGCTCTTGATCCGGCAAACAAACCACCGCTGGTAGCGGTGGTTTTTTTGTTTGCAAGCAGCAGAT TACGCGCAGAAAAAAAGGATCTCAAGAAGATCCTTTGATCTTTTCTACGGGGTCTGACGCTCAGTGGAACGAAAACTCACG TTAAGGGATTTTGGTCATGAGATTATCAAAAAGGATCTTCACCTAGATCCTTTTAAATTAAAAATGAAGTTTTAAATCAATCTA AAGTATATATGAGTAAACTTGGTCTGACAGTTACCAATGCTTAATCAGTGAGGCACCTATCTCAGCGATCTGTCTATTTCGT TCATCCATAGTTGCCTGACTCCCCGTCGTGTAGATAACTACGATACGGGAGGGCTTACCATCTGGCCCCAGTGCTGCAAT GATACCGCGAGACCCACGCTCACCGGCTCCAGATTTATCAGCAATAAACCAGCCAGCCGGAAGGGCCGAGCGCAGAAGT GGTCCTGCAACTTTATCCGCCTCCATCCAGTCTATTAATTGTTGCCGGGAAGCTAGAGTAAGTAGTTCGCCAGTTAATAGTT TGCGCAACGTTGTTGCCATTGCTACAGGCATCGTGGTGTCACGCTCGTCGTTTGGTATGGCTTCATTCAGCTCCGGTTCCC AACGATCAAGGCGAGTTACATGATCCCCCATGTTGTGCAAAAAAGCGGTTAGCTCCTTCGGTCCTCCGATCGTTGTCAGAA GTAAGTTGGCCGCAGTGTTATCACTCATGGTTATGGCAGCACTGCATAATTCTCTTACTGTCATGCCATCCGTAAGATGCTT TTCTGTGACTGGTGAGTACTCAACCAAGTCATTCTGAGAATAGTGTATGCGGCGACCGAGTTGCTCTTGCCCGGCGTCAAT ACGGGATAATACCGCGCCACATAGCAGAACTTTAAAAGTGCTCATCATTGGAAAACGTTCTTCGGGGCGAAAACTCTCAAG GATCTTACCGCTGTTGAGATCCAGTTCGATGTAACCCACTCGTGCACCCAACTGATCTTCAGCATCTTTTACTTTCACCAGC GTTTCTGGGTGAGCAAAAACAGGAAGGCAAAATGCCGCAAAAAAGGGAATAAGGGCGACACGGAAATGTTGAATACTCAT ACTCTTCCTTTTTCAATATTATTGAAGCATTTATCAGGGTTATTGTCTCATGAGCGGATACATATTTGAATGTATTTAGAAAAA TAAACAAATAGGGGTTCCGCGCACATTTCCCCGAAAAGTGCCACCTGACGTCTAAGAAACCATTATTATCATGACATTAACC |
| TATAAAAATAGGCGTATCACGAGGCCCTTTCGTCTCGCGCGTTTCGGTGATGACGGTGAAAACCTCTGACACATGCAGCTC CCGGAGACGGTCACAGCTTGTCTGTAAGCGGATGCCGGGAGCAGACAAGCCCGTCAGGGCGCGTCAGCGGGTGTTGGC GGGTGTCGGGGCTGGCTTAACTATGCGGCATCAGAGCAGATTGTACTGAGAGTGCACCATATGCGGTGTGAAATACCGCA CAGATGCGTAAGGAGAAAATACCGCATCAGGCGCCATTCGCCATTCAGGCTGCGCAACTGTTGGGAAGGGCGATCGGTG CGGGCCTCTTCGCTATTACGCCAGCTGGCGAAAGGGGGATGTGCTGCAAGGCGATTAAGTTGGGTAACGCCAGGGTTTTC CCAGTTACGACGTTGTAAAACGACGGCCAGTGAATT |

**Example 4. Expression of antibody as polyprotein with internal cleavable signal peptide construct.**

Further embodiments are created of coding sequences, expression vectors, and methods for the expression of an antibody. A primary expression construct comprises a polyprotein with an internal cleavable signal peptide, so that expression and subsequent cleavage results in the formation of a multi-chain (e.g., two-chain) antibody molecule.

Table 7A. Coding Sequence for D2E7 internal cleavable signal peptide construct (SEQ ID NO:45)

| |
|---|
| atggagtttgggctgagctggctttttcttgtcgcgattttaaaaggtgtccagtgtgaggtgcagctggtggagtctgggggaggcttg gtacagcccggcaggtccctgagactctcctgtgcggcctctggattcacctttgatgattatgccatgcactgggtccggcaagctcc agggaagggcctggaatgggtctcagctatcacttggaatagtggtcacatagactatgcggactctgtggagggccgattcaccatct ccagagacaacgccaagaactccctgtatctgcaaatgaacagtctgagagctgaggatacggccgtatattactgtgcgaaagtctc gtaccttagcaccgcgtcctcccttgactattggggccaaggtaccctggtcaccgtctcgagtgcgtcgaccaagggcccatcggtct tccccctggcaccctcctccaagagcacctctgggggcacagcggccctgggctgcctggtcaaggactacttccccgaaccggtg acggtgtcgtggaactcaggcgccctgaccagcggcgtgcacaccttcccggctgtcctacagtcctcaggactctactccctcagca gcgtggtgaccgtgccctccagcagcttgggcacccagacctacatctgcaacgtgaatcacaagcccagcaacaccaaggtggac aagaaagttgagcccaaatcttgtgacaaaactcacacatgcccaccgtgcccagcacctgaactcctggggggaccgtcagtcttcc tcttccccccaaaacccaaggacaccctcatgatctcccggacccctgaggtcacatgcgtggtggtggacgtgagccacgaagacc ctgaggtcaagttcaactggtacgtggacggcgtggaggtgcataatgccaagacaaagccgcgggaggagcagtacaacagcac gtaccgtgtggtcagcgtcctcaccgtcctgcaccaggactggctgaatggcaaggagtacaagtgcaaggtctccaacaaagccct cccagcccccatcgagaaaaccatctccaaagccaaagggcagccccgagaaccacaggtgtacaccctgcccccatcccgggat gagctgaccaagaaccaggtcagcctgacctgcctggtcaaaggcttctatcccagcgacatcgccgtggagtgggagagcaatgg gcagccggagaacaactacaagaccacgcctcccgtgctggactccgacggctccttcttcctctacagcaagctcaccgtggacaa gagcaggtggcagcaggggaacgtcttctcatgctccgtgatgcatgaggctctgcacaaccactacacgcagaagagcctctccct gtctaggggtaaacgcatgggacgaatggcaatgaaatggttagttgttataatatgtttctctataacaagtcaacctgcttctgctatgg acatgcgcgtgcccgcccagctgctgggcctgctgctgctgtggttccccggctcgcgatgcgacatccagatgacccagtctccatc ctccctgtctgcatctgtaggggacagagtcaccatcacttgtcgggcaagtcagggcatcagaaattacttagcctggtatcagcaa aaaccagggaaagcccctaagctcctgatctatgctgcatccactttgcaatcaggggtcccatctcggttcagtggcagtggatctggg acagatttcactctcaccatcagcagcctacagcctgaagatgttgcaacttattactgtcaaaggtataaccgtgcaccgtatacttttgg ccaggggaccaaggtggaaatcaaacgtacggtggctgcaccatctgtcttcatcttcccgccatctgatgagcagttgaaatctggaa ctgcctctgttgtgtgcctgctgaataacttctatcccagagaggccaaagtacagtggaaggtggataacgccctccaatcgggtaact cccaggagagtgtcacagagcaggacagcaaggacagcacctacagcctcagcagcaccctgacgctgagcaaagcagactacg agaaacacaaagtctacgcctgcgaagtcacccatcagggcctgagctcgcccgtcacaaagagcttcaacaggggagagtgttga |

Table 7B. Amino Acid Sequence of the D2E7 Internal Cleavable Signal Peptide Polyprotein (SEQ ID NO:46)

| |
|---|
| MEFGLSWLFLVAILKGVQCEVQLVESGGGLVQPGRSLRLSCAASGFTFDDYAMHWVRQAPGKGLE WVSAITWNSGHIDYADSVEGRFTISRDNAKNSLYLQMNSLRAEDTAVYYCAKVSYLSTASSLDYWGQ GTLVTVSSASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHEALHNHYTQKSLSLS RGKRMGRMAMKWLVVIICFSITSQPASAMDMRVPAQLLGLLLLWFPGSRCDIQMTQSPSSLSASVGD RVTITCRASQGIRNYLAWYQQKPGKAPKLLIYAASTLQSGVPSRFSGSGSGTDFTLTISSLQPEDVAT YYCQRYNRAPYTFGQGTKVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLSSPVTKSFNRGEC* |

Table 7C. Complete D2E7 Internal Cleavable Signal Peptide Polyprotein Expression Vector DNA Sequence (SEQ ID NO:47)

| |
|---|
| gaagttcctattccgaagttcctattctctagacgttacataacttacggtaaatggcccgcctggctgaccgcccaacg acccccgcccattgacgtcaataatgacgtatgttcccatagtaacgccaatagggactttccattgacgtcaatgggt ggagtatttacggtaaactgcccacttggcagtacatcaagtgtatcatatgccaagtacgccccctattgacgtcaatg acggtaaatggcccgcctggcattatgcccagtacatgaccttatgggactttcctacttggcagtacatctacgtattag tcatcgctattaccatggtgatgcggttttggcagtacatcaatgggcgtggatagcggtttgactcacggggatttccaa gtctccaccccattgacgtcaatgggagtttgttttggcaccaaaatcaacgggactttccaaaatgtcgtaacaactcc gccccaatgacgcaaatgggcagggaattcgagctcggtactcgagcggtgttccgcggtcctcctcgtatagaaac tcggaccactctgagacgaaggctcgcgtccaggccagcacgaaggaggctaagtgggaggggtagcggtcgtt gtccactagggggtccactcgctccagggtgtgaagacacatgtcgccctcttcggcatcaaggaaggtgattggtta taggtgtaggccacgtgaccgggtgttcctgaagggggctataaaagggggtgggggcgcgttcgtcctcactctct tccgcatcgctgtctgcgagggccagctgttgggctcgcggttgaggacaaactcttcgcggtctttccagtactcttgg atcggaaacccgtcggcctccgaacggtactccgccaccgagggacctgagcgagtccgcatcgaccggatcgg aaaacctctcgactgttggggtgagtactccctctcaaaagcgggcatgacttctgcgctaagattgtcagtttccaaaa acgaggaggatttgatattcacctggcccgcggtgatgcctttgagggtggccgcgtccatctggtcagaaaagaca atctttttgttgtcaagcttgaggtgtggcaggcttgagatctggccatacacttgagtgacaatgacatccactttgccttt ctctccacaggtgtccactcccaggtccaaccggaattgtacccgcggccagagcttgcccgggcgccaccatgga gtttgggctgagctggctttttcttgtcgcgattttaaaaggtgtccagtgtgaggtgcagctggtggagtctgggggagg cttggtacagcccggcaggtccctgagactctcctgtgcggcctctggattcacctttgatgattatgccatgcactgggt ccggcaagctccagggaagggcctggaatgggtctcagctatcacttggaatagtggtcacatagactatgcggact ctgtggagggccgattcaccatctccagagacaacgccaagaactccctgtatctgcaaatgaacagtctgagagct gaggatacggccgtatattactgtgcgaaagtctcgtaccttagcaccgcgtcctcccttgactattggggccaaggta ccctggtcaccgtctcgagtgcgtcgaccaagggcccatcggtcttccccctggcaccctcctccaagagcacctctg ggggcacagcggccctgggctgcctggtcaaggactacttccccgaaccggtgacggtgtcgtggaactcaggcgc cctgaccagcggcgtgcacaccttcccggctgtcctacagtcctcaggactctactccctcagcagcgtggtgaccgt gccctccagcagcttgggcacccagacctacatctgcaacgtgaatcacaagcccagcaacaccaaggtggaca agaaagttgagcccaaatcttgtgacaaaactcacacatgcccaccgtgcccagcacctgaactcctggggggacc gtcagtcttcctcttccccccaaaacccaaggacaccctcatgatctcccggacccctgaggtcacatgcgtggtggtg gacgtgagccacgaagaccctgaggtcaagttcaactggtacgtggacggcgtggaggtgcataatgccaagaca aagccgcgggaggagcagtacaacagcacgtaccgtgtggtcagcgtcctcaccgtcctgcaccaggactggctg aatggcaaggagtacaagtgcaaggtctccaacaaagccctcccagcccccatcgagaaaaccatctccaaagc caaagggcagccccgagaaccacaggtgtacaccctgcccccatcccgggatgagctgaccaagaaccaggtc agcctgacctgcctggtcaaaggcttctatcccagcgacatcgccgtggagtgggagagcaatgggcagccggag |
| aacaactacaagaccacgcctcccgtgctggactccgacggctccttcttcctctacagcaagctcaccgtggacaa gagcaggtggcagcaggggaacgtcttctcatgctccgtgatgcatgaggctctgcacaaccactacacgcagaag agcctctccctgtctaggggtaaacgcatggacgaatggcaatgaaatggttagttgttataatatgtttctctataaca agtcaacctgcttctgctatggacatgcgcgtgcccgcccagctgctgggcctgctgctgctgtggttccccggctcgcg atgcgacatccagatgacccagtctccatcctccctgtctgcatctgtaggggacagagtcaccatcacttgtcggca agtcagggcatcagaaattacttagcctggtatcagcaaaaaccagggaaagcccctaagctcctgatctatgctgc atccactttgcaatcaggggtcccatctcggttcagtggcagtggatctgggacagatttcactctcaccatcagcagcc tacagcctgaagatgttgcaacttattactgtcaaaggtataaccgtgcaccgtatacttttggccaggggaccaaggt ggaaatcaaacgtacggtggctgcaccatctgtcttcatcttcccgccatctgatgagcagttgaaatctggaactgcct ctgttgtgtgcctgctgaataacttctatcccagagaggccaaagtacagtggaaggtggataacgccctccaatcgg gtaactcccaggagagtgtcacagagcaggacagcaaggacagcacctacagcctcagcagcaccctgacgctg agcaaagcagactacgagaaacacaaagtctacgcctgcgaagtcacccatcagggcctgagctcgcccgtcac aaagagcttcaacaggggagagtgttgagcggccgcgtttaaactgaatgagcgcgtccatccagacatgataaga tacattgatgagtttggacaaaccacaactagaatgcagtgaaaaaaatgctttatttgtgaaatttgtgatgctattgcttt atttgtaaccattataagctgcaataaacaagttaacaacaacaattgcattcattttatgtttcaggttcagggggaggt gtgggaggttttttaaagcaagtaaaacctctacaaatgtggtatggctgattatgatccggctgcctcgcgcgtttcggt gatgacggtgaaaacctctgacacatgcagctcccggagacggtcacagcttgtctgtaagcggatgccgggagca gacaagcccgtcagggcgcgtcagcgggtgttggcgggtgtcggggcgcagccatgaccggtcgacggcgcgcc ttttttttaattttattttattttatttttgacgcgccgaaggcgcgatctgagctcggtacagcttggctgtggaatgtgtgtca gttagggtgtggaaagtccccaggctccccagcaggcagaagtatgcaaagcatgcatctcaattagtcagcaacc aggtgtggaaagtccccaggctccccagcaggcagaagtatgcaaagcatgcatctcaattagtcagcaaccatag tcccgcccctaactccgcccatcccgcccctaactccgcccagttccgcccattctccgccccatggctgactaatttttt tatttatgcagaggccgaggccgcctcggcctctgagctattccagaagtagtgaggaggcttttttggaggcctaggc ttttgcaaaaagctcctcgaggaactgaaaaaccagaaagttaactggtaagtttagtctttttgtcttttatttcaggtccc ggatccggtggtggtgcaaatcaaagaactgctcctcagtggatgttgcctttacttctaggcctgtacggaagtgttact tctgctctaaaagctgcggaattgtacccgcggcctaatacgactcactatagggactagtatggttcgaccattgaact gcatcgtcgccgtgtcccaaaatatggggattggcaagaacggagacctaccctggcctccgctcaggaacgagtt caagtacttccaaagaatgaccacaacctcttcagtggaaggtaaacagaatctggtgattatgggtaggaaaacct ggttctccattcctgagaagaatcgacctttaaaggacagaattaatatagttctcagtagagaactcaaagaaccac cacgaggagctcattttcttgccaaaagtttagatgatgccttaagacttattgaacaaccggaattggcaagtaaagta gacatggtttggatagtcggaggcagttctgtttaccaggaagccatgaatcaaccaggccacctcagactctttgtga caaggatcatgcaggaatttgaaagtgacacgtttttcccagaaattgatttggggaaatataaacttctcccagaatac ccaggcgtcctctctgaggtccaggaggaaaaaggcatcaagtataagtttgaagtctacgagaagaaagactaag cggccgagcgcgcggatctggaaacgggagatgggggaggctaactgaagcacggaaggagacaataccgga aggaacccgcgctatgacggcaataaaaagacagaataaaacgcacgggtgttgggtcgtttgttcataaacgcgg ggttcggtcccagggctggcactctgtcgataccccaccgagaccccattggggccaatacgcccgcgtttcttcctttt ccccaccccaccccccaagttcgggtgaaggcccagggctcgcagccaacgtcggggcggcaggccctgccata gccactggccccgtgggttagggacggggtcccccatggggaatggtttatggttcgtgggggttattattttgggcgttg cgtggggtctggagatcccccgggctgcaggaattccgttacattacttacggtaaatggcccgcctggctgaccgcc caacgacccccgcccattgacgtcaataatgacgtatgttcccatagtaacgccaatagggactttccattgacgtcaa tgggtggagtatttacggtaaactgcccacttggcagtacatcaagtgtatcatatgccaagtacgccccctattgacgt caatgacggtaaatggcccgcctggcattatgcccagtacatgaccttatgggactttcctacttggcagtacatctacg tattagtcatcgctattaccatggtgatgcggttttggcagtacatcaatgggcgtggatagcggtttgactcacggggatt tccaagtctccaccccattgacgtcaatgggagtttgttttggcaccaaaatcaacgggactttccaaaatgtcgtaaca actccgccccattgacgcaaatgggcggaattcgagctcggtactcgagcggtgttccgcggtcctcctcgtataga aactcggaccactctgagacgaaggctcgcgtccaggccagcacgaaggaggctaagtgggaggggtagcggtc gttgtccactaggggggtccactcgctccagggtgtgaagacacatgtcgccctcttcggcatcaaggaaggtgattggt ttataggtgtaggccacgtgaccgggtgttcctgaagggggctataaaaggggggtgggggcgcgttcgtcctcactc tcttccgcatcgctgtctgcgagggccagctgttgggctcgcggttgaggacaaactcttcgcggtctttccagtactcttg gatcggaaacccgtcggcctccgaacggtactccgccaccgagggacctgagcgagtccgcatcgaccggatcg |
| gaaaacctctcgactgttggggtgagtactccctctcaaaagcgggcatgacttctgcgctaagattgtcagtttccaaa aacgaggaggatttgatattcacctggcccgcggtgatgcctttgagggtggccgcgtccatctggtcagaaaagac aatctttttgttgtcaagcttgaggtgtggcaggcttgagatctggccatacacttgagtgacaatgacatccactttgcctt tctctccacaggtgtccactcccaggtccaaccggaattgtacccgcggccagagcttgcgggcgccaccgcggcc gcggggatccagacatgataagatacattgatgagtttggacaaaccacaactagaatgcagtgaaaaaaatgcttt atttgtgaaatttgtgatgctattgctttatttgtaaccattataagctgcaataaacaagttaacaacaacaattgcattcat tttatgtttcaggttcagggggaggtgtgggaggttttttcggatcctcttggcgtaatcatggtcatagctgtttcctgtgtga aattgttatccgctcacaattccacacaacatacgagccggaagcataaagtgtaaagcctggggtgcctaatgagt gagctaactcacattaattgcgttgcgctcactgcccgctttccagtcgggaaacctgtcgtgccagctgcattaatgaa tcggccaacgcgcggggagaggcggtttgcgtattgggcgctcttccgcttcctcgctcactgactcgctgcgctcggt cgttcggctgcggcgagcggtatcagctcactcaaaggcggtaatacggttatccacagaatcaggggataacgca ggaaagaacatgtgagcaaaaggccagcaaaaggccaggaaccgtaaaaaggccgcgttgctggcgtttttcca taggctccgcccccctgacgagcatcacaaaaatcgacgctcaagtcagaggtggcgaaacccgacaggactata aagataccaggcgtttccccctggaagctccctcgtgcgctctcctgttccgaccctgccgcttaccggatacctgtccg cctttctcccttcgggaagcgtggcgctttctcatagctcacgctgtaggtatctcagttcggtgtaggtcgttcgctccaag ctgggctgtgtgcacgaaccccccgttcagcccgaccgctgcgccttatccggtaactatcgtcttgagtccaacccgg taagacacgacttatcgccactggcagcagccactggtaacaggattagcagagcgaggtatgtaggcggtgctac agagttcttgaagtggtggcctaactacggctacactagaagaacagtatttggtatctgcgctctgctgaagccagtta ccttcggaaaaagagttggtagctcttgatccggcaaacaaaccaccgctggtagcggtggtttttttgtttgcaagcag cagattacgcgcagaaaaaaaggatctcaagaagatcctttgatcttttctacggggtctgacgctcagtggaacgaa aactcacgttaagggattttggtcatgagattatcaaaaaggatcttcacctagatccttttaaattaaaaatgaagtttta aatcaatctaaagtatatatgagtaaacttggtctgacagttaccaatgcttaatcagtgaggcacctatctcagcgatct gtctatttcgttcatccatagttgcctgactccccgtcgtgtagataactacgatacgggagggcttaccatctggcccca gtgctgcaatgataccgcgagacccacgctcaccggctccagatttatcagcaataaaccagccagccggaaggg ccgagcgcagaagtggtcctgcaactttatccgcctccatccagtctattaattgttgccgggaagctagagtaagtagt tcgccagttaatagtttgcgcaacgttgttgccattgctacaggcatcgtggtgtcacgctcgtcgtttggtatggcttcattc agctccggttcccaacgatcaaggcgagttacatgatcccccatgttgtgcaaaaaagcggttagctccttcggtcctc cgatcgttgtcagaagtaagttggccgcagtgttatcactcatggttatggcagcactgcataattctcttactgtcatgcc atccgtaagatgcttttctgtgactggtgagtactcaaccaagtcattctgagaatagtgtatgcggcgaccgagttgctc ttgcccggcgtcaatacgggataataccgcgccacatagcagaactttaaaagtgctcatcattggaaaacgttcttcg gggcgaaaactctcaaggatcttaccgctgttgagatccagttcgatgtaacccactcgtgcacccaactgatcttcag catcttttactttcaccagcgtttctgggtgagcaaaaacaggaaggcaaaatgccgcaaaaaagggaataagggc gacacggaaatgttgaatactcatactcttcctttttcaatattattgaagcatttatcagggttattgtctcatgagcggata catatttgaatgtatttagaaaaataaacaaataggggttccgcgcacatttccccgaaaagtgccacctgacgtctaa gaaaccattattatcatgacattaacctataaaaataggcgtatcacgaggccctttcgtctcgcgcgtttcggtgatgac ggtgaaaacctctgacacatgcagctcccggagacggtcacagcttgtctgtaagcggatgccgggagcagacaa gcccgtcagggcgcgtcagcgggtgttggcgggtgtcggggctggcttaactatgcggcatcagagcagattgtact gagagtgcaccatatgcggtgtgaaataccgcacagatgcgtaaggagaaaataccgcatcaggcgccattcgcc attcaggctgcgcaactgttgggaagggcgatcggtgcgggcctcttcgctattacgccagctggcgaaagggggat gtgctgcaaggcgattaagttgggtaacgccagggttttcccagttacgacgttgtaaaacgacggccagtgaatt |

**Materials and Methods:**

Transfection of described constructs into 293-6E cells is carried out as follows. The cells used are HEK293-6E cells in exponential growth phase (0.8 to 1.5x10⁶ cells/ml), which cells have been passaged in culture less than 30 times; the cultures are inoculated into fresh growth medium to a concentration of 3 x 10⁵ cells/ml, every three or four days. Growth medium is FreeStyle™ 293 Expression Medium (GIBCO™ Cat. No. 12338-018, Invitrogen, Carlsbad, CA) supplemented with Geneticin (G418) 25ug/ml (GIBCO™ Cat. No. 10131-027) and 0.1% Pluronic F-68 (surfactant, GIBCO™ Cat. No. 24040-032). Transfection Medium is FreeStyle™ 293 Expression Medium (GIBCO™ Cat. No. 12338-018) with a final concentration of 10mM HEPES Buffer Solution ml (GIBCO™ Cat. No. 15630-080). For transfection, the vector DNA of choice is added to achieve a concentration of 1 µg (Heavy Chain + Light Chain)/ml Subject to change based on optimization experiments. PEI (polyethylenimine), linear, 25 kDa, 1mg/ml sterile stock solution, pH 7.0 (Polysciences, Inc., Warrington, PA) is added as a transfection mediator, with a DNA:PEI ratio of 1:2. The Feeding Medium used is Tryptone N1 Medium (TN1 powder from Organotechnie France, Cat No. 19554, available through TekniScience Inc. Tel# 1-800-267-9799). 5% w/v stock solution in FreeStyle™ 293 Expression Medium is added to a final concentration of 0.5%. Standard laboratory equipment is generally used. A Cedex Cell Counting System is employed (Innovatis, Bielefeld, Germany).

Each small-scale transfection is carried out in a 125ml Erlenmeyer flask as follows. An aliquot of 20ml of fresh culture medium is inoculated with 1x10⁶ cells/ml of viable cells. (Note: For larger volumes, culture should be 20-25% of nominal capacity of vessel, e.g. 100ml culture in 500ml flask). Cultures are then placed in a 37°C incubator with a humidified atmosphere of 5% CO₂ with 130 rpm rotation speed.

The DNA-PEI complex preparation is made by warming transfection medium to 37°C in a water bath, thawing at room temperature frozen PEI stock and DNA solutions (stored at -20°C). The amounts of DNA and PEI used are based on the total volume of culture being transfected. A 20ml culture with 2.5 ml DNA/PEI complex and 2.5ml Tn1 requires a total of 25 µg DNA and 50 µg PEI. DNA:PEI complexes (e.g., for ten transfections) are formed by combining a 12.5ml of transfection medium to tube A to which has been added a solution containing the DNA vector of choice to a final concentration of 10 µg/ml and 12.5 ml of transfection medium to PEI has been added (20 µg/ml, final conc.). The PEI mixture is mixed by vortexing about 10 seconds prior to mixing with the DNA solution. After combining the PEI and DNA mixtures, the combination is mixed by vortexing for 10 seconds. Then the mixture is allowed to stand at room temperature for 15 minutes (but not more than 20 minutes). 2.5ml of the DNA:PEI complex solution is added per 20 ml HEK-6E cells. The 5% TN1 supplement is added to a final concentration of 0.5% to each flask about 20 to 24 hours after transfection.

Cell density and viability are determined on day 4 and day 7. Cell pellets are collected from 2 ml aliquot of culture) for Western analysis and Northern Blot analysis on day 4. Pellets are frozen at -80ºC until analyzed. Cells are harvested by centrifugation at 1000 rpm (10 min) 7 days after transfection, and supernatants are filtered using pre-filter papers and a Corning 0.22 µm CA Filter system. Supernatant samples are also stored at 80°C until analyzed, for example using ELISA assays.

For Northern Blot Analysis, total RNA is isolated from transiently transfected 293-6E cells as follows. Frozen cell pellets are thawed on ice. RNA is purified using the Qiagen Rneasy Mini Kit (Qiagen, cat. #74104), according to the manufacturer's instructions.

Formaldehyde/agarose gel preparation is as follows. 2 grams of agarose (Ambion, cat. #9040) is boiled in 161.3 ml distilled water. 4 ml 1 M MOPS (Morpholinopropanesulfonic acid) PH 7.0, 1 ml 1M NaOAc, 0.4 ml 0.5 M EDTA are added and the mixture is cooled to 60ºC. Then 33.3 ml 37% Formaldehyde (J.T. Baker, cat #2106-01) is added, and the molten agarose solution is mixed gently. The gel is poured and allowed to solidify in a fume hood.

Running buffer is prepared by mixing 30 ml 1M MOPS, pH 7.0, 7 ml 1M NaOAc, 3 ml 0.5M EDTA and DEPC (diethylpyrocarbonate) treated dH₂O to 1.5.

RNA samples are prepared by mixing 3 parts formaldehyde load dye (Ambion, cat. #8552) with 1 part RNA. 3 to 5 µg of RNA is run per lane. The RNA molecular weight markers used is from the 0.5-10 Kb RNA Ladder (Invitrogen, cat. #15623-200). Samples are heated at 65ºC for 5 minutes to denature and chill on ice. Then 0.5 µl 10 µg/µl Ethidium Bromide (Pierce, cat. #17898) is added to each sample. Each sample is spun briefly to pellet liquid.

Gel electrophoresis is carried out as follows. The formaldehyde/agarose gel is covered with running buffer, samples are loaded and then run at 150V for 2 hours in a fume hood. Bands are viewed using ultraviolet transillumination and photographed for a permanent record.

Capillary transfer is done by soaking the gel in several changes of DEPC-treated dH₂O for five minutes to remove formaldehyde. The gel is then soaked in 50 mM NaOH, 10 mM NaCl for 20 minutes at room temperature to further denature any double-stranded RNA. The gel is rinsed once in DEPC-treated dH₂O and then soaked in 20X SSC (175.3g NaCl; 88.2g Sodium Citrate; pH to -7.0 with 10M NaOH, volume adjusted to 1 L) for 20 minutes at room temperature to neutralize. Hybond-N+ membrane (Amersham Biosciences, cat #RPN303B) is soaked and cut to the same size as the gel, in DEPC-treated dH₂O to wet. 3M filter paper (Whatman cat#3030917) is cut to the same size as the gel and the membrane. The transfer system is assembled by placing a layer of 3M paper on a solid support over a reservoir of 20X SSC so that the paper wicks the 20X SSC through the layers to be assembled on top. The gel is placed on this wick, the Hybond-N+ membrane, 3 sheets of 3M paper cut to size, and a thick stack of Gel Blot Paper (Schleicher & Schuell, cat. #10427920). A flat support is placed on top of the stack, and weight is added (usually a liter bottle of water), if needed, to insure efficient capillary transfer. Plastic wrap is used to cover any of the reservoir exposed to air to prevent evaporation. The transfer is allowed to proceed overnight at room temperature. Then the transfer system is disassembled and the blot is soaked in 6X SSC to remove any agarose. The membrane is allowed to air dry and exposed to UV to crosslink the blot.

DNA probe templates are the coding region for heavy and light chain of D2E7. 100 ng of the desired template is labeled with Alkaline Phosphate using the AlkPhos Direct Labeling Reagents kit (alkaline phosphatase labeling system, Amersham Biosciences, cat. #RPN3680) according to the manufacturer's instructions. Prehybridization and hybridization steps were performed using the same kit as for labeling (contains hybridization buffer). Membranes were prehybridized for at least 1 hour at 65ºC in a hybridization oven, the probe was boiled and added directly to prehybridization buffer/blot. Hybridization took place overnight at 65ºC in a hybridization oven. The hybridization solution was decanted, and the membrane was washed briefly with 2X SSC to remove hybridization solution, then washed twice with 2X SSC, 0.1 % SDS at 65ºC for 15 minutes each, and finally washed twice with 0.1X SSC, 0.1% SDS at 65ºC for 15 minutes each time. To visualize bands on the membrane, chemiluminescence was used. Blots were overlaid with CDP-Star Detection Reagent (alkaline phosphatase-dependent production of a photope from a 1,2-dioxetane substrate, Amersham Biosciences, cat. #RPN3682), for 5 minutes at room temperature. Excess reagent was drained from blots and they were then encased in plastic sheet protectors. Blots were exposed to Kodak Biomax MR film (x ray film, Kodak, cat. #8952855), starting for 10 seconds for up to 10 minutes. Films were developed using the Kodak M35A X-OMAT Processor (x ray developer/processor).

Cell pellet samples for western blotting were prepared as follows. For the analysis of intracellular antibody expression, cells were lysed in NP 40 Lysis buffer (50 mM Tris-HCl, pH 7.5, 150 mM NaCl, 1 % NP40 (octylphenolpoly(ethyleneglycolether)), 5 mM BME, and protease inhibitors cocktail III), with incubation on ice for 10 min. The fractions for membranes and insoluble proteins are collected by centrifugation at 16,000 rpm for 30 min using a microcentrifuge. The supernatant, designated the soluble intracellular, or cytosolic fraction, was used for gel analysis, with the addition of SDS loading buffer with DTT. The pellets were suspended with equal volume of lysis buffer, and SDS gel loading buffer with DTT was added. Culture supernatant samples were prepared for western blotting as follows. Culture supernatants were either concentrated using Centricon Ultra (ultrafiltration device, Millipore), with a MW cut off of 30,000 daltons, or used directly for western blotting. For immunoblotting (western analysis), samples were resolved on NUPAGE 4-12 % Bis-Tris (polyacrylamide) gels and transferred to PVDF membrane using standard methods. The membranes were incubated for 1 h in blocking solution (PBS with 0.05 % Tween 20 (polyoxyethylene sorbitan monolaurate) and 5 % dry milk), washed, incubated with polyclonal rabbit anti-human IgG/HRP or polyclonal rabbit anti-human kappa light chain/HRP, from DakoCytomation (Denmark), at 1:1000 dilution in PBST buffer, and then washed again in three changes of PBST at room temperature. ECL Plus Western Blotting Detection (chemiluminescent and chemifluorescent detection) System from GE/Amersham Biosciences (Piscataway, NJ) was used for detection.

ELISA assays were carried out using standard methods, using Goat Anti-Human IgG, UNLB and Goat Anti-Human IgG/HRP from Southern Biotech (Birmingham, AL), 2% milk in PBS as blotting buffer, K-Blue (3,3', 5,5' tetramethylbenzidine and hydrogen peroxide (H₂O₂, Neogen, Lansing, MI) as substrate. Plates were read with Spectramax microplate reader at 650 nM primary wavelength and 490 nm reference wavelength.

The secreted antibody was affinity purified with standard methods using Protein A Agarose beads from Invitrogen (Carlsbad, CA), Immuno Pure (A) IgG Binding Buffer from Pierce, PBS, pH 7.4 as wash buffer, and 0.1 M Acetic Acid/150 mM NaCl, pH 3.5 as elution buffer (neutralized using 1 M Tris pH 9.5).

Determination of intact molecular weight. Intact molecular weights of the D2E7 samples produced from construct pTT3 HC-int-LC P.hori were analyzed by LC-MS. An 1100 capillary HPLC system (Agilent SN DE 14900659) with a protein microtrap (Michrom Bioresources, Inc. cat. 004/25109/03) was used to desalt and introduce samples into the Q Star Pulsar i mass spectrometer (Applied Biosystems, SN K1820202). To elute the samples, a gradient was run with buffer A (0.08% FA, 0.02% TFA in HPLC water) and buffer B (0.08% FA and 0.02% TFA in acetonitrile), at a flow rate of 50 µL/min, for 15 minutes.

Determination of light chain and heavy chain molecular weight. Native D2E7 samples produced from construct pTT3 HC-int-LC P.hori were were analyzed by LC-MS. Reduction of the disulfide bonds that linked light chains and heavy chains together was conducted in 20 mM DTT at 37°C for 30 minutes. An 1100 capillary HPLC system (Agilent SN DE 14900659) with a PLRP-S column (Michrom Bioresources, Inc. 8 µm, 4000 A, 1.0 x 150 mm, P/N 901-00911-00) was used to separate light chains from heavy chains and introduce them into the Q Star Pulsar i mass spectrometer (Applied Biosystems, SN K1820202). The column was heated at 60ºC. An HPLC gradient, which was run with buffer A (0.08% FA, 0.02% TFA in HPLC water) and buffer B (0.08% FA and 0.02% TFA in acetonitrile), at a flow rate of 50 µL/min, was run for 60 minutes to elute the samples.

Restriction endonucleases were from New England Biolabs (Beverly, MA). Custom oligonucleotides, DNA polymerases, DNA ligases, and *E*. *coli* strains used for cloning were from Invitrogen (Carlsbad, CA). Protease inhibitor cocktail III was from Calbiochem (La Jolla, CA). Qiagen (Valencia, CA) products were used for DNA isolation and purification.

STATEMENTS REGARDING INCORPORATION BY REFERENCE AND VARIATIONS

All references mentioned throughout this application, for example patent documents including issued or granted patents or equivalents; patent application publications; unpublished patent applications; and non-patent literature documents or other source material; are hereby incorporated by reference herein in their entireties, as though individually incorporated by reference. In the event of any inconsistency between cited references and the disclosure of the present application, the disclosure herein takes precedence. Some references provided herein are incorporated by reference to provide information, e.g., details concerning sources of starting materials, additional starting materials, additional reagents, additional methods of synthesis, additional methods of analysis, additional biological materials, additional cells, and additional uses of the invention.

All patents and publications mentioned herein are indicative of the levels of skill of those skilled in the art to which the invention pertains. References cited herein can indicate the state of the art as of their publication or filing date, and it is intended that this information can be employed herein, if needed, to exclude specific embodiments that are in the qualifying prior art. For example, when compositions of matter are claimed herein, it should be understood that compounds known and available as qualifying prior art relative to Applicant's invention, including compounds for which an enabling disclosure is provided in the references cited herein, are not intended to be included in the composition of matter claims herein.

Any appendix or appendices hereto are incorporated by reference as part of the specification and/or drawings.

Where the terms "comprise", "comprises", "comprised", or "comprising" are used herein, they are to be interpreted as specifying the presence of the stated features, integers, steps, or components referred to, but not to preclude the presence or addition of one or more other feature, integer, step, component, or group thereof. Thus as used herein, comprising is synonymous with including, containing, having, or characterized by, and is inclusive or open-ended. As used herein, "consisting of" excludes any element, step, or ingredient, etc. not specified in the claim description. As used herein, "consisting essentially of" does not exclude materials or steps that do not materially affect the basic and novel characteristics of the claim (e.g., relating to the active ingredient). In each instance herein any of the terms "comprising", "consisting essentially of" and "consisting of" may be replaced with either of the other two terms, thereby disclosing separate embodiments and/or scopes which are not necessarily coextensive. The invention illustratively described herein suitably may be practiced in the absence of any element or elements or limitation or limitations not specifically disclosed herein.

Whenever a range is disclosed herein, e.g., a temperature range, time range, composition or concentration range, or other value range, etc., all intermediate ranges and subranges as well as all individual values included in the ranges given are intended to be included in the disclosure. This invention is not to be limited by the embodiments disclosed, including any shown in the drawings or exemplified in the specification, which are given by way of example or illustration and not of limitation. It will be understood that any subranges or individual values in a range or subrange that are included in the description herein can be excluded from the claims herein.

The invention has been described with reference to various specific and/or preferred embodiments and techniques. However, it should be understood that many variations and modifications may be made while remaining within the spirit and scope of the invention. It will be apparent to one of ordinary skill in the art that compositions, methods, devices, device elements, materials, procedures and techniques other than those specifically described herein can be employed in the practice of the invention as broadly disclosed herein without resort to undue experimentation; this can extend, for example, to starting materials, biological materials, reagents, synthetic methods, purification methods, analytical methods, assay methods, and biological methods other than those specifically exemplified. All art-known functional equivalents of the foregoing (e.g., compositions, methods, devices, device elements, materials, procedures and techniques, etc.) described herein are intended to be encompassed by this invention. The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments, preferred embodiments, and optional features, modification and variation of the concepts herein disclosed may be resorted to by those skilled in the art, and that such modifications and variations are considered to be within the scope of this invention as defined by the appended claims.

ADDITIONAL REFERENCES

US 6258562, US 6090382; US 6455275; EP1080206B1; WO 9960135; US 5912167; US 5162601; WO 199521249A1; US 5149783; US 5955072; US 5532142; US 20040224391; US 6537806; US 5846767; US 20030099932; WO 9958663; US 20030157641; US 2003048306A2; US 6114146; US 6060273; US 5925565; US 20040241821; WO 2003100021 A2; WO 2003100022A2; US 20040265955; US 20050003482; US 20050042721; WO 2005017149; WO 2004113493; US 20050136035; WO 2004108893; US 6692736; US 20050147962; US 6331415; US 6632637; US 20040063186; US 7026526; US 6365377; WO 2005123915; US 5665567; WO 9741241A1; EP 0701616B1; US 20060010506; WO 2006048459; US 6852510; WO 2005072129; US 5648254; US 6908751; US 20050221429; WO 2005071088; WO 2005108585; WO 2005085456; US 7029876; US 6638762; US 6544780; US 5519164; WO 2003031630; US 6294353; WO 2005047512; US 7052905; US 7018833; US 20020034814; US 20040126883; US 20050002907; US 20050112095; US 20050214258; EP 0598029.

Mathys S et al., 1999, Gene 231(1-2):1-13, Characterization of a self-splicing mini-intein and its conversion into autocatalytic N- and C-terminal cleavage elements: facile production of protein building blocks for protein ligation.

## Claims

1. An expression vector for expressing a recombinant protein, comprising a coding sequence for a polyprotein, wherein the polyprotein comprises at least a first and a second protein segment, wherein said protein segments are separated by a protein cleavage site therebetween, wherein the protein cleavage site comprises a self processing peptide cleavage sequence, a signal peptide cleavage sequence, or a protease cleavage sequence; and wherein said coding sequence is expressible in a host cell and is cleaved within the host cell.

2. The expression vector of claim 1, wherein said protein cleavage site comprises an intein segment or modified intein segment, wherein the intein segment or modified intein segment permits proteolytic cleavage of the between the first protein segment and the intein or modified intein segment and between the intein or modified intein segment and the second protein segment but not ligation of said first protein segment to said second protein segment, wherein the first and second protein segments are of mammalian origin and capable of extracellular secretion.

3. The expression vector of claim 1 or 2, further comprising a signal peptide nucleic acid sequence encoding a signal peptide cleavage site, operably positioned before a first nucleic acid sequence encoding the first protein segment or a second nucleic acid sequence encoding the second protein segment.

4. The expression vector of claim 3, wherein the signal peptide nucleic acid sequence is positioned before the second nucleic acid sequence encoding the second protein segment.

5. A host cell comprising an expression vector of any one of claims 1-4.

6. A method for producing a recombinant protein or a plurality of proteins, comprising culturing a host cell comprising the vector of any one of claims 1-4 in a culture medium under conditions sufficient to allow expression of a vector protein, and recovering and/or purifying said vector protein or plurality of proteins.
